# EUROPEAN PATENT SPECIFICATION

(11) **EP 2 831 053 B1**
(45) Date of publication and mention of the grant of the patent: **22.06.2016**
(21) Application number: 13724175.8
(22) Date of filing: 25.03.2013
(51) Int. Cl.: C07D 307/80, A61K 31/343, A61P 3/10, C07D 405/12, C07D 405/14, C07D 407/12, C07D 409/12, C07D 413/12, C07D 413/14, C07D 417/12

(54) **NEW INDANYLOXYDIHYDROBENZOFURANYLACETIC ACID DERIVATIVES AND THEIR USE AS GPR40 RECEPTOR AGONISTS**
NEUE INDANYLOXYDIHYDROBENZOFURANYLESSIGSÄURE-DERIVATE UND IHRE VERWENDUNG ALS GPR40 RECEPTOR AGONISTEN
NOUVEAUX DÉRIVÉS D'ACIDE INDANYLOXYDIHYDROBENZOFURANYLACETIQUE ET LEUR UTILISATION EN TANT QU'AGONISTES DU RÉCÉPTEUR GPR40

(30) Priority: 26.03.2012 EP 12161240
(43) Date of publication of application: 04.02.2015
(73) Proprietor: Boehringer Ingelheim International GmbH, 55216 Ingelheim am Rhein (DE)
(72) Inventor: ECKHARDT, Matthias, 55216 Ingelheim Am Rhein (DE); FRATTINI, Sara, 55216 Ingelheim Am Rhein (DE); HAMPRECHT, Dieter, 55216 Ingelheim Am Rhein (DE); HIMMELSBACH, Frank, 55216 Ingelheim Am Rhein (DE); LANGKOPF, Elke, 55216 Ingelheim Am Rhein (DE); LINGARD, Iain, 55216 Ingelheim Am Rhein (DE); PETERS, Stefan, 55216 Ingelheim Am Rhein (DE); WAGNER, Holger, 55216 Ingelheim Am Rhein (DE)
(74) Representative: Simon, Elke Anna Maria
(86) International application number: PCT/EP2013/056313
(87) International publication number: WO 2013/144098

(56) References cited:
- EP-A1- 1 559 422
- EP-A1- 1 630 152
- WO-A1-2010/143733
- WO-A1-2012/072691
- WO-A2-2008/001931
- NEGORO, NOBUYUKI ET AL: "Identification of Fused-Ring Alkanoic Acids with Improved Pharmacokinetic Profiles that Act as G Protein-Coupled Receptor 40/Free Fatty Acid Receptor 1 Agonists", JOURNAL OF MEDICINAL CHEMISTRY, CODEN: JMCMAR; ISSN: 0022-2623, vol. 55, no. 4, 16 January 2012 (2012-01-16), pages 1538-1552, XP002696495,
- MIKAMI, SATOSHI ET AL: "Discovery of Phenylpropanoic Acid Derivatives Containing Polar Functionalities as Potent and Orally Bioavailable G Protein-Coupled Receptor 40 Agonists for the Treatment of Type 2 Diabetes", JOURNAL OF MEDICINAL CHEMISTRY, CODEN: JMCMAR; ISSN: 0022-2623, vol. 55, no. 8, 19 March 2012 (2012-03-19) , pages 3756-3776, XP002696496,

## Description

### Field of the invention

The present invention relates to novel indanyloxydihydrobenzofuranylacetic acids, that are agonists of the G-protein coupled receptor 40 (GPR40, also known as free fatty acid receptor FFAR 1), to processes for their preparation, to pharmaceutical compositions containing these compounds and to their medical use for the prophylaxis and/or treatment of metabolic diseases, such as diabetes, more specifically type 2 diabetes mellitus, and conditions associated with the disease, including insulin resistance, obesity, cardiovascular disease and dyslipidemia.

### Background of the Invention

Metabolic diseases are diseases caused by an abnormal metabolic process and may either be congenital due to an inherited enzyme abnormality or acquired due to a disease of an endocrine organ or failure of a metabolically important organ such as the liver or the pancreas.

Diabetes mellitus is a disease state or process derived from multiple causative factors and is defined as a chronic hyperglycemia associated with resulting damages to organs and dysfunctions of metabolic processes. Depending on its etiology, one differentiates between several forms of diabetes, which are either due to an absolute (lacking or decreased insulin secretion) or to a relative lack of insulin. Diabetes mellitus Type I (IDDM, insulin-dependent diabetes mellitus) generally occurs in adolescents under 20 years of age. It is assumed to be of auto-immune etiology, leading to an insulitis with the subsequent destruction of the beta cells of the islets of Langerhans which are responsible for the insulin synthesis. In addition, in latent autoimmune diabetes in adults (LADA; Diabetes Care. 8: 1460-1467, 2001) beta cells are being destroyed due to autoimmune attack. The amount of insulin produced by the remaining pancreatic islet cells is too low, resulting in elevated blood glucose levels (hyperglycemia). Diabetes mellitus Type II generally occurs at an older age. It is above all associated with a resistance to insulin in the liver and the skeletal muscles, but also with a defect of the islets of Langerhans. High blood glucose levels (and also high blood lipid levels) in turn lead to an impairment of beta cell function and to an increase in beta cell apoptosis.

Persistent or inadequately controlled hyperglycemia is associated with a wide range of pathologies. Diabetes is a very disabling disease, because today's common antidiabetic drugs do not control blood sugar levels well enough to completely prevent the occurrence of high and low blood sugar levels. Out of range blood sugar levels are toxic and cause long-term complications for example retinopathy, renopathy, neuropathy and peripheral vascular disease. There is also a host of related conditions, such as obesity, hypertension, stroke, heart disease and hyperlipidemia, for which persons with diabetes are substantially at risk.

Obesity is associated with an increased risk of follow-up diseases such as cardiovascular diseases, hypertension, diabetes, hyperlipidemia and an increased mortality. Diabetes (insulin resistance) and obesity are part of the "metabolic syndrome" which is defined as the linkage between several diseases (also referred to as syndrome X, insulin-resistance syndrome, or deadly quartet). These often occur in the same patients and are major risk factors for development of diabetes type II and cardiovascular disease. It has been suggested that the control of lipid levels and glucose levels is required to treat diabetes type II, heart disease, and other occurrences of metabolic syndrome (see e.g., Diabetes 48: 1836-1841, 1999; JAMA 288: 2209-2716, 2002).

The free fatty acid receptor GPR40 (also referred to as either FFAR, FFAR1, or FFA1) is a cell-surface receptor and a member of the gene superfamily of G-protein coupled receptors, which was first identified as a so-called orphan receptor, i.e. a receptor without a known ligand, based on the predicted presence of seven putative transmembrane regions in the corresponding protein (Sawzdargo et al. (1997) Biochem. Biophys. Res. Commun. 239: 543-547). GPR40 is found to be highly expressed in several particular cell types: the pancreatic β cells and insulin-secreting cell lines, as well as in enteroendocrine cells, taste cells, and is reported to be expressed in immune cells, splenocytes, and in the human and monkey brain. Meanwhile, fatty acids of varying chain lengths are thought to represent the endogenous ligands for GPR40, activation of which is linked primarily to the modulation of the Gq family of intra-cellular signaling G proteins and concomitant induction of elevated calcium levels, although activation of Gs- and Gi-proteins to modulate intracellular levels of cAMP have also been reported. GPR40 is activated especially by long-chain FFA, particularly oleate, as well as the PPAR-gamma agonist rosiglitazone.

It has been recognized that the fatty acids that serve as activators for GPR40 augment the elevated plasma glucose-induced secretion of insulin through GPR40 receptors that are expressed in the insulin secreting cells (Itoh et al. (2003) Nature 422: 173-176; Briscoe et al. (2003) J. Biol. Chem. 278: 11303-11311; Kotarsky et al. (2003) Biochem. Biophys. Res. Commun. 301: 406-410). Despite initial controversy, the use of GPR40 agonist appears to be the appropriate for increasing insulin release for the treatment of diabetes (see e.g. Diabetes 2008, 57, 2211; J. Med. Chem. 2007, 50, 2807). Typically, long term diabetes therapy leads to the gradual diminution of islet activity, so that after extended periods of treatment Type 2 diabetic patients need treatment with daily insulin injections instead. GPR40 agonists may have the potential to restore or preserve islet function, therefore, GPR40 agonists may be beneficial also in that that they may delay or prevent the diminution and loss of islet function in a Type 2 diabetic patient.

It is well established that the incretins GLP-1 (glucagon-like peptide- 1) and GIP (glucose-dependent insulinotropic peptide; also known as gastric inhibitory peptide) stimulate insulin secretion and are rapidly inactivated in vivo by DPP-4. These peptidyl hormones are secreted by endocrine cells that are located in the epithelium of the small intestine. When these endocrine cells sense an increase in the concentration of glucose in the lumen of the digestive tract, they act as the trigger for incretin release. Incretins are carried through the circulation to beta cells in the pancreas and cause the beta cells to secrete more insulin in anticipation of an increase of blood glucose resulting from the digesting meal. Further studies indicating that the GPR40 modulatory role on the release of incretins from the enteroendocrine cells, including CCK, GLP-1, GIP, PYY, and possibly others, suggest that GPR40 modulators may contribute to enhanced insulin release from the pancreatic beta cells also indirectly by e.g. a synergistic effect of GLP-1 and possibly GIP on the insulin release, and the other release incretins may also contribute to an overall beneficial contribution of GPR40 modulation on metabolic diseases. The indirect contributions of GPR40 modulation on insulin release through the elevation of plasma levels of incretins may be further augmented by the coadministration of inhibitors of the enzymes responsible for the incretin degradation, such as inhibitors of DPP-4.

Insulin imbalances lead to conditions such as type II diabetes mellitus, a serious metabolic disease. The modulation of the function of GPR40 in modulating insulin secretion indicates the therapeutic agents capable of modulating GPR40 function could be useful for the treatment of disorders such as diabetes and conditions associated with the disease, including insulin resistance, obesity, cardiovascular disease and dyslipidemia.

### Object of the present invention

The object of the present invention is to provide new compounds, hereinafter described as compounds of formula (I), in particular new 2,3-indanyloxydihydrobenzofuranylacetic acids, which are active with regard to the G-protein-coupled receptor GPR40, notably are agonists of the G-protein-coupled receptor GPR40.

A further object of the present invention is to provide new compounds, in particular new indanyloxydihydrobenzofuranylacetic acids, which have an activating effect on the G-protein-coupled receptor GPR40 *in vitro* and/or *in vivo* and possess suitable pharmacological and pharmacokinetic properties to use them as medicaments.

A further object of the present invention is to provide effective GPR40 agonists, in particular for the treatment of metabolic disorders, for example diabetes, dyslipidemia and/or obesity.

A further object of the present invention is to provide a pharmaceutical composition comprising at least one compound according to the invention.

A further object of the present invention is to provide a combination of at least one compound according to the invention with one or more additional therapeutic agents.

Further objects of the present invention become apparent to the one skilled in the art by the description hereinbefore and in the following and by the examples.

GPR40 modulators are known in the art, for example, the compounds disclosed in WO 2004/041266 (EP 1 559 422), WO 2007/033002, WO 2009/157418 EP 1 630 152, WO 2008/001931, WO 2010/143733, WO 2012/072691, N. Negoro et al. (J. Med. Chem. 2012, 55, 1538-1552) and S. Mikami et al. (J. Med. Chem. 2012, 55, 3756-3776). The indanyloxydihydrobenzofuranylacetic acids of the present invention may provide several advantages, such as enhanced potency, high metabolic and/or chemical stability, high selectivity and tolerability, enhanced solubility, and the possibility to form stable salts.

### Summary of the Invention

In a first aspect the invention relates to a compound of formula I 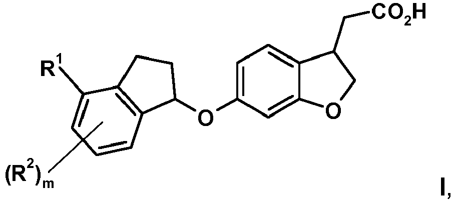 wherein
- R¹: is selected from the group R¹-G1 consisting of a phenyl ring, a tetrazolyl ring, and a 5- or 6-membered heteroaromatic ring which contains 1, 2, or 3 heteroatoms independently of each other selected from =N-, -NH-, -O- and -S-;
wherein optionally a second ring is annulated to said phenyl or heteroaromatic ring,
wherein said second ring is 5- or 6-membered, unsaturated or aromatic and may contain 1, 2, or 3 heteroatoms independently of each other selected from =N-, -NH-, -O- and -S- with the proviso that only up to two of the heteroatoms are O and S and no O-O, S-S, and S-O bond is formed, and wherein in said second ring independently of the presence of heteroatoms 1 or 2 CH₂ groups may be replaced by -C(=O)-, -S(=O)- or -S(=O)₂-, and
wherein said phenyl ring, tetrazolyl ring, heteroaromatic ring, annulated phenyl ring, and annulated heteroaromatic ring are substituted with one group R³; and
wherein each of the phenyl ring, tetrazolyl ring, heteroaromatic ring, annulated phenyl ring, and annulated heteroaromatic ring is optionally additionally substituted with 1 to 4 groups independently selected from R⁴; and
wherein in said heteroaromatic ring and/or said second ring the H-atom in one or more NH groups, if present, is replaced by R^{N} or R³;
- R²: is selected from the group R²-G3 consisting of F, Cl, Br, H₂FC-, F₃C-, NC-, and F₃C-O-.;
- R³: is selected from the group R³-G1 consisting of
C₁₋₆-alkyl, C₂₋₆-alkenyl, C₂₋₆-alkinyl, C₃₋₆-cycloalkyl-, C₁₋₄-alkyl-NH-, (C₁₋₄-alkyl)₂N-, C₁₋₆-alkyl-O-, C₃₋₆-cycloalkyl-O-, C₁₋₄-alkyl-S-, C₁₋₄-alkyl-S(=O)-, and C₁₋₄-alkyl-S(=O)₂,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from C₁₋₄-alkyl-C(=O)-, heterocyclyl-C(=O)-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, HO₂C-, C₁₋₄-alkyl-O-C(=O)-, C₃₋₆-cycloalkyl-O-C(=O)-, heterocyclyl-O-C(=O)-, -NHR^{N}, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, heterocyclyl-O-, phenyl-O-, heteroaryl-O-, C₃₋₆-cycloalkyl-S-, heterocyclyl-S-, phenyl-S-, heteroaryl-S-, C₃₋₆-cycloalkyl-S(=O)-, heterocyclyl-S(=O)-, phenyl-S(=O)-, heteroaryl-S(=O)-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups mentioned is optionally substituted with 1 to 3 groups independently selected from R⁵ and optionally substituted with 1 or more F atoms; and
wherein each phenyl and heteroaryl group is optionally substituted with 1 to 5 substituents independently selected from R⁶;
wherein heterocyclyl is selected from
a cyclobutyl group wherein 1 CH₂ group is replaced by -NH- or -O-,
a saturated or partially unsaturated C₅₋₇cycloalkyl group wherein 1 CH₂ group is replaced by -C(=O)-, -NH-, -O-, -S(=O)- or -S(=O)₂- and/or 1 CH group by N;
a saturated or partially unsaturated C₅₋₇cycloalkyl group wherein 1 CH₂ group is replaced by -NH- or -O-, a second CH₂ group is replaced by -NH-, -C(=O)-, -S(=O)- or -S(=O)₂- and/or 1 CH group is replaced by N; and
a saturated or partially unsaturated C₅₋₇cycloalkyl group wherein 2 CH₂ groups are replaced by -NH- or 1 CH₂ group by -NH- and the other by -O-and a third CH₂ group is replaced by -C(=O)-, -S(=O)- or -S(=O)₂- and/or 1 CH group by N;
wherein heteroaryl is selected from
a tetrazolyl ring, and a 5- or 6-membered heteroaromatic ring which contains 1, 2, or 3 heteroatoms independently of each other selected from =N-, -NH-, -O-, and -S-, wherein in heteroaromatic groups containing a -HC=N- unit this group is optionally replaced by -NH-C(=O)-;
wherein in heteroaryl and heterocyclyl rings with one or more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, C₃₋₄-alkenyl, C₃₋₄-alkinyl, H₂N-, C₁₋₄-alkyl-NH-, (C₁₋4-alkyl)₂N-, C₁₋₄-alkyl-O-, C₁₋₄-alkyl-S-, C₁₋₄-alkyl-S(=O)-, and C₁₋₄-alkyl-S(=O)₂;
- R⁴: is selected from the group R⁴-G1 consisting of F, Cl, Br, I, CN, -OH, C₁₋₄-alkyl, C₃₋₆-cycloalkyl-, HO-C₁₋₄-alkyl, C₁₋₄-alkyl-O-C₁₋₄-alkyl, -NR^{N}H, C₁₋₄-alkyl-NR^{N}-, G₁₋₄-alkyl-O-, C₃₋₆-cycloalkyl-O-, C₁₋₄-alkyl-O-C₁₋₄-alkyl-O-, C₁₋₄-alkyl-S-, C₁₋₄-alkyl-S(=O)-, and C₁₋₄-alkyl-S(=O)₂-,
wherein any alkyl and cycloalkyl group or sub-group within the groups mentioned is optionally substituted with 1 or more F atoms;

- R⁵: is selected from the group R⁵-G1 consisting of Cl, Br, I, C₁₋₄-alkyl-, CN, C₃₋₆-cycloalkyl-, heterocyclyl-C(=O)-, H₂N-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, HO-C(=O)-, C₁₋₄-alkyl-O-C(=O)-, -NHR^{N}, C₁₋₄-alkyl-NR^{N}-, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, phenyl-O-, heteroaryl-O-, C₁₋₄-alkyl-S-, C₃₋₆-cycloalkyl-S-, heterocyclyl-S-, phenyl-S-, heteroaryl-S-, C₁₋₄-alkyl-S(=O)-, C₃₋₆-cycloalkyl-S(=O)-, heterocyclyl-S(=O)-, phenyl-S(=O)-, heteroaryl-S(=O)-, C₁₋₄-alkyl-S(=O)₂-, C₃₋₆-cycloalkyl-S(=O)₂-, heterocyclyl-S(=O)₂-, phenyl-S(=O)₂-, heteroaryl-S(=O)₂-, H₂N-S(=O)₂-, C₁₋₄-alkyl-NR^{N}-S(=O)₂-, heterocyclyl, phenyl, and heteroaryl,
wherein any alkyl, cycloalkyl and heterocyclyl group or sub-group within the groups mentioned is optionally substituted with 1 or more F atoms and optionally substituted with 1 or 2 groups independently selected from H₃C-, HO-, H₃C-O-, and -CN;
wherein heterocyclyl is selected from
a cyclobutyl group wherein 1 CH₂ group is replaced by -NR^{N}- or -O-,
a saturated or partially unsaturated C₅₋₇cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₇cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₇cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from
a tetrazolyl ring, and a 5- or 6-membered heteroaromatic ring which contains 1, 2, or 3 heteroatoms independently of each other selected from =N-, -NH-, -O-, and -S-, wherein in heteroaromatic groups containing a -HC=N- unit this group is optionally replaced by -NR^{N}-C(=O)-, and wherein in heteroaromatic rings with one ore more NH groups each of them is replaced by NR^{N}, and each heteroaryl group is optionally substituted with 1 to 3 substituents independently selected from F, Cl, -CH₃, -CN, and -O-CH₃;
- R⁶: is selected from the group R⁶-G1 consisting of F, Cl, Br, I, CN, C₁₋₄-alkyl, C₃₋₆-cycloalkyl-, HO-C₁₋₄-alkyl-, C₁₋₄-alkyl-O-C₁₋₄-alkyl-, R^{N}HN-, C₁₋₄-alkyl-O-, -S(=O)-C₁₋₄-alkyl, and S(=O)2-C₁₋₄-alkyl,
wherein any alkyl and cycloalkyl group or sub-group within the groups mentioned is optionally substituted with one or more F atoms;
- R^{N}: is independently of each other selected from the group R^{N}-G1 consisting of H, C₁₋₄-alkyl, C₁₋₄-alkyl-C(=O)-, C₁₋₄-alkyl-NH-C(=O)-, C₁₋₄-alkyl-N(C₁₋₄-alkyl)-C(=O)-, C₁₋₄-alkyl-O-C(=O)-, and C₁₋₄-alkyl-S(=O)₂-; and
- m: is 1 or 2;
wherein in any definition mentioned hereinbefore and if not specified otherwise, any alkyl group or sub-group may be straight-chained or branched,
the isoforms, tautomers, stereoisomers, metabolites, prodrugs, solvates, hydrates, and the salts thereof, particularly the physiologically acceptable salts thereof with inorganic or organic acids or bases, or the combinations thereof.

The expression "optionally substituted with 1 or more F atoms" means that none or one up to successively all H atoms bound to carbon atoms of the respective group or submoiety may be replaced by F atoms, preferably 1 to 5 H atoms or, more preferred, 1 to 3 H atoms may be replaced by F atoms.

The extension -Gn used within the definitions is meant to identify genus n of the respective substituent. For example, R¹-G1 defines genus 1 of the substituent R¹.

In a further aspect this invention relates to a pharmaceutical composition, comprising one or more compounds of general formula **I** or one or more pharmaceutically acceptable salts thereof according to the invention, optionally together with one or more inert carriers and/or diluents.

According to another aspect of the invention, there is provided a compound of the general formula **I** or a pharmaceutically acceptable salt thereof for use in a therapeutic method for treating a metabolic disease or disorder, such as diabetes, dyslipidemia and/or obesity, as described hereinbefore and hereinafter.

In a further aspect this invention relates to a compound of the general formula **I** or a pharmaceutically acceptable salt thereof for use in combination with one or more additional therapeutic agents in the treatment of metabolic diseases or conditions associated therewith, such as diabetes, insulin resitance, cardiovascular disease, dyslipidemia and/or obesity.

In a further aspect this invention relates to a pharmaceutical composition which comprises a compound according to general formula **I** or a pharmaceutically acceptable salt thereof and one or more additional therapeutic agents, optionally together with one or more inert carriers and/or diluents.

Other aspects of the invention become apparent to the one skilled in the art from the specification and the experimental part as described hereinbefore and hereinafter.

### Detailed Description

Unless otherwise stated, the groups, residues, and substituents, particularly R¹, R², R³, R⁴, R⁵, R⁶, R^{N}, and m are defined as above and hereinafter. If residues, substituents, or groups occur several times in a compound, as for example R^{N}, they may have the same or different meanings. Some preferred meanings of individual groups and substituents of the compounds according to the invention will be given hereinafter. Any and each of these definitions may be combined with each other.

### R¹:

### R¹-G1:

The group R¹ is preferably selected from the group R¹-G1 as defined hereinbefore.

### R¹-G2:

According to one embodiment the group R¹ is selected from the group R¹-G2 consisting of a phenyl ring, a tetrazolyl ring, a 5-membered heteroaromatic ring which contains 2 or 3 heteroatoms independently of each other selected from =N-, -NH-, -O- and -S- with the proviso that not more than one heteroatom is -O- or -S-, and a 6-membered heteroaromatic ring which contains 1 or 2 =N- atoms;
wherein optionally a second ring is annulated to said phenyl ring and 5- and 6-membered heteroaromatic rings, wherein said second ring is 5- or 6-membered, unsaturated or aromatic and may contain 1 or 2 heteroatoms independently of each other selected from =N-, -NH-, -O- and -S- with the proviso that no O-O, S-S, and SO bond is formed, and wherein in said second ring independently of the presence of heteroatoms 1 or 2 -CH₂- groups may be replaced by -C(=O)- or -S(=O)₂-, and
wherein in said heteroaromatic ring and/or said second ring the H-atom in one or more NH groups, if present, is replaced by R^{N} or R³, and
wherein each of said phenyl ring, tetrazolyl ring, heteroaromatic rings, annulated phenyl ring, and annulated heteroaromatic rings is substituted with one group R³ and optionally additionally substituted with 1 or 2 substituents independently selected from R⁴.

### R¹-G2a:

According to one embodiment the group R¹ is selected from the group R¹-G2a consisting of a phenyl ring, a tetrazolyl ring, a 5-membered heteroaromatic ring which contains 2 or 3 heteroatoms independently of each other selected from =N-, -NH-, -O- and -S-, and a 6-membered heteroaromatic ring which contains 1 or 2 =N- atoms;
wherein in said 5-membered heteroaromatic ring the H-atom in one or more NH groups is replaced with R^{N} or R³, and
wherein each of said phenyl ring, tetrazolyl ring, and heteroaromatic rings is substituted with one group R³ and optionally additionally substituted with 1 or 2 substituents independently selected from R⁴.

### R¹-G2b:

According to one embodiment the group R¹ is selected from the group R¹-G2b consisting of a phenyl ring, a 5-membered heteroaromatic ring which contains 2 or 3 heteroatoms independently of each other selected from =N-, -NH-, -O- and -S-, and a 6-membered heteroaromatic ring which contains 1 or 2 =N- atoms, wherein a second 5- or 6-membered, unsaturated or aromatic ring is annulated to said phenyl ring and 5- and 6-membered heteroaromatic rings, which may contain 1 or 2 heteroatoms independently of each other selected from =N-, -NH-, -O- and -S- with the proviso that no O-O, S-S, and S-O bond is formed, and wherein in said second ring 1 or 2 -CH₂- groups may be replaced by -C(=O)- or -S(=O)₂-, and
wherein in said heteroaromatic rings and said second rings the H-atom in one or more NH groups, if present, is replaced by R^{N} or R³, and
wherein each annulated phenyl ring and annulated heteroaromatic ring is substituted with one group R³ and optionally additionally substituted with 1 or 2 substituents independently selected from R⁴.

### R¹-G3:

According to one embodiment the group R¹ is selected from the group R¹-G3 consisting of: 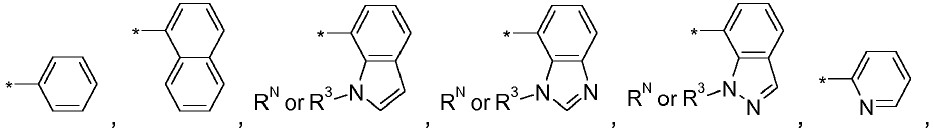 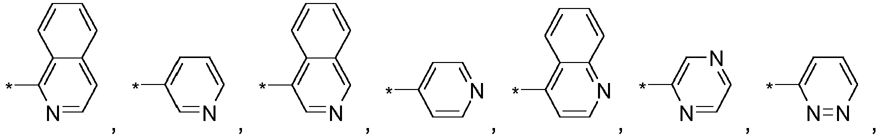 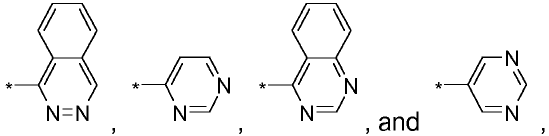 wherein each group is substituted with one group R³ and optionally additionally substituted with 1 or 2 groups independently selected from R⁴; and

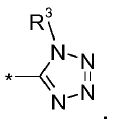

### R¹-G3a:

According to one embodiment the group R¹ is selected from the group R¹-G3a consisting of 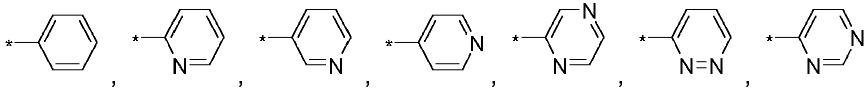 and 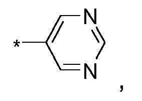 wherein each group is substituted with one group R³ and optionally additionally substituted with 1 or 2 groups independently selected from R⁴.

### R¹-G3b:

According to one embodiment the group R¹ is selected from the group R¹-G3b consisting of

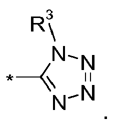

### R¹-G3c:

In another embodiment the group R¹ is selected from the group R¹-G4 consisting of 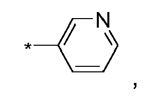 which is substituted with one group R³ and optionally additionally substituted with 1 or 2 groups independently selected from R⁴.

### R¹-G4:

In another embodiment the group R¹ is selected from the group R¹-G4 consisting of 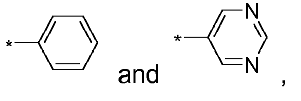 wherein each group is substituted with one group R³ and optionally additionally substituted with 1 or 2 groups independently selected from R⁴.

### R¹-G4a:

In another embodiment the group R¹ is selected from the group R¹-G4a consisting of 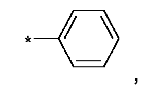 which is substituted with one group R³ and optionally additionally substituted with 1 or 2 groups independently selected from R⁴.

### R¹-G4b:

In another embodiment the group R¹ is selected from the group R¹-G4b consisting of 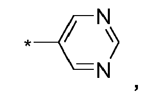 which is substituted with one group R³ and optionally additionally substituted with 1 or 2 groups independently selected from R⁴.

### R¹-G5:

In another embodiment the group R¹ is selected from the group R¹-G5 consisting of

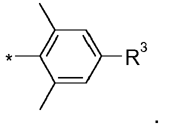

### R¹-G5a:

In another embodiment the group R¹ is selected from the group R¹-G5a consisting of

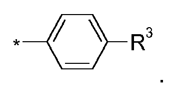

### R²:

### R²-G3:

In one embodiment the group R² is selected from the group R²-G3 consisting of F, Cl, Br, H₂FC-, F₃C-, NC-, and F₃C-O-.

### R²-G4:

In another embodiment the group R² is selected from the group R²-G4 consisting of F, Cl, and NC-.

### R²-G4a:

In another embodiment the group R² is selected from the group R²-G4a consisting of F, Cl, F₃C-, and NC-.

### R²-G4b:

In another embodiment the group R² is selected from the group R²-G4b consisting of F.

### R²-G4c:

In another embodiment the group R² is selected from the group R²-G4c consisting of F₃C-.

### R³:

### R³-G1:

The group R³ is preferably selected from the group R³-G1 as defined hereinbefore.

### R³-G2:

In another embodiment the group R³ is selected from the group R³-G2 consisting of C₁₋₄-alkyl, C₃₋₆-cycloalkyl-, C₁₋₄-alkyl-O-, C₃₋₆-cycloalkyl-O-, C₁₋₄-alkyl-S(=O)-, and C₁₋₄-alkyl-S(=O)₂-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from C₁₋₄-alkyl-C(=O)-, heterocyclyl-C(=O)-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, heterocyclyl-O-, phenyl-O-, heteroaryl-O-, C₃₋₆-cycloalkyl-S(=O)-, heterocyclyl-S(=O)-, phenyl-S(=O)-, heteroaryl-S(=O)-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups mentioned is optionally substituted with 1 to 3 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₆-cycloalkyl group wherein 1 CH₂ group is replaced by -C(=O)-, -NH-, -O-, -S(=O)- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)-, -S(=O)- or -S(=O)₂- and/or 1 CH group is replaced by N; and
a saturated or mono-unsaturated C₅₋₆-cycloalkyl group wherein 2 CH₂ groups are replaced by -NH- or 1 CH₂ group by -NH- and the other by -O- and a third CH₂ group is replaced by -C(=O)-, -S(=O)- or -S(=O)₂- and/or 1 CH group by N;
wherein heteroaryl is selected from
a tetrazolyl ring, a pyridin-2-onyl ring, a 5-membered heteroaromatic ring which contains 1, 2, or 3 heteroatoms independently of each other selected from =N-, -NH-, O, and S, and a 6-membered heteroaromatic ring which contains 1 or 2 =N- atom,
and wherein in heteroaryl and heterocyclyl rings with one ore more NH groups each of them is replaced by NR^{N} or NR⁵,
with the proviso that R³ in total cannot be C₁₋₄-alkyl-, C₁₋₄-alkyl-O-C₁₋₄-alkyl, HO-C₁₋₄-alkyl, C₁₋₄-alkyl-O-, C₁₋₄-alkyl-S(=O)-, and C₁₋₄-alkyl-S(=O)₂.

### R³-G2a:

In another embodiment the group R³ is selected from the group R³-G2a consisting of C₁₋₄-alkyl, C₃₋₆-cycloalkyl-, C₁₋₄-alkyl-O-, C₃₋₆-cycloalkyl-O-, C₁₋₄-alkyl-S(=O)-, and C₁₋₄-alkyl-S(=O)₂-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from
C₁₋₄-alkyl-C(=O)-, heterocyclyl-C(=O)-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, heterocyclyl-O-, phenyl-O-, heteroaryl-O-, C₃₋₆-cycloalkyl-S(=O)-, heterocyclyl-S(=O)-, phenyl-S(=O)-, heteroaryl-S(=O)-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups mentioned is optionally substituted with 1 to 3 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₇cycloalkyl group wherein 1 CH₂ group is replaced by -C(=O)-, -NH-, -O-, -S(=O)- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)-, -S(=O)- or -S(=O)₂- and/or 1 CH group is replaced by N; and
a saturated or mono-unsaturated C₅₋₆-cycloalkyl group wherein 2 CH₂ groups are replaced by -NH- or 1 CH₂ group by -NH- and the other by -O- and a third CH₂ group is replaced by -C(=O)-, -S(=O)- or -S(=O)₂- and/or 1 CH group by N;
wherein heteroaryl is selected from
a tetrazolyl ring, a 5-membered heteroaromatic ring which contains 1, 2, or 3 heteroatoms independently of each other selected from =N-, -NH-, O, and S, and a 6-membered heteroaromatic ring which contains 1 or 2 =N- atom and wherein
a -HC=N- unit is optionally replaced by -NH-C(=O)-; and
wherein in heteroaryl and heterocyclyl rings with one ore more NH groups each of them is replaced by NR^{N} or NR⁵,
with the proviso that R³ in total cannot be C₁₋₄-alkyl-, C₁₋₄-alkyl-O-C₁₋₄-alkyl, HO-C₁₋₄-alkyl, C₁₋₄-alkyl-O-, C₁₋₄-alkyl-S(=O)-, and C₁₋₄-alkyl-S(=O)2.

### R³-G3:

In another embodiment the group R³ is selected from the group R³-G3 consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from H₃C-C(=O)-, heterocyclyl-C(=O)-, H₂N-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl, and heteroaryl,
wherein each alkyl, cycloalkyl, and heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or -S(=O)₂- and/or 1 CH group is replaced by N; and
wherein heteroaryl is selected from
a pyridin-2-onyl ring, a 5-membered heteroaromatic ring which contains 1, 2, or 3 heteroatoms independently of each other selected from =N-, -NH-, O, and S, and
a 6-membered heteroaromatic ring which contains 1 or 2 =N- atoms,
and wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-.

### R³-G3a:

In another embodiment the group R³ is selected from the group R³-G3a consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 to 3 F atoms;
or from
C₃-alkyl-S(=O)₂- substituted with 1 HO- or H₃C-O- group; H₃C-C(=O)-, heterocyclyl-C(=O)-, H₂N-C(=O)-, tetrahydropyran-4-yl-NH-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl and heteroaryl,
wherein each alkyl, cycloalkyl, and heterocyclyl group or sub-group within the groups mentioned is optionally substituted with 1 or 2 groups independently selected from R⁵ and optionally substituted with 1 to 3 F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₇cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or -S(=O)₂-and/or 1 CH group is replaced by N; and
wherein heteroaryl is selected from
tetrazolyl, a 5-membered heteroaromatic ring which contains 1, 2 or 3 heteroatoms independently of each other selected from =N-, -NH-, O, and S, and a 6-membered heteroaromatic ring which contains 1 or 2 =N- atoms and wherein a -HC=N- unit is optionally replaced by -NH-C(=O)-; and
wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-.

### R³-G4:

In another embodiment the group R³ is selected from the group R³-G4 consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 group selected from R⁵ and optionally substituted with 1 or 2 H₃C- group;
or from H₃C-C(=O)-, heterocyclyl-C(=O)-, H₂N-C(=O)-, H₃C-NR^{N}-C(=O)-, heterocyclyl-O-, heteroaryl-O-, H₂N-S(=O)₂-, heterocyclyl, phenyl, and heteroaryl,
wherein each heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 or 2 substituents independently selected from R⁶;
wherein heterocyclyl is selected from
a cyclobutyl group wherein 1 CH₂ group is replaced by -NH- or -O-;
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)- or-S(=O)₂- and/or 1 CH group is replaced by N; and
wherein heteroaryl is selected from
a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-.

### R³-G4a:

In another embodiment the group R³ is selected from the group R³-G4a consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 group selected from R⁵ and optionally substituted with 1 or 2 H₃C- groups;
or from
C₅-alkyl-O- substituted with 2 HO- groups;
C₃-alkyl-S(=O)₂- substituted with 1 HO- or H₃C-O- group; and
H₃C-C(=O)-, heterocyclyl-C(=O)-, H₂N-C(=O)-, HO-(H₃C)₂C-CH₂-NH-C(=O)-, tetrahydropyran-4-yl-NH-C(=O)-, H₃C-NR^{N}-C(=O)-, H₃C-S(=O)₂-NH-, heterocyclyl-O-, phenyl-O-, heteroaryl-O-, H₂N-S(=O)₂-, heterocyclyl, phenyl and heteroaryl,
wherein each heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 or 2 substituents independently selected from R⁶;
wherein heterocyclyl is selected from
a cyclobutyl group wherein 1 CH₂ group is replaced by -NH- or -O-;
a saturated or mono-unsaturated C₅₋₇cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)- or -S(=O)₂- and/or 1 CH group is replaced by N; and
wherein heteroaryl is selected from
tetrazolyl, a 5-membered heteroaromatic ring which contains 1 to 3 heteroatoms independently of each other selected from =N-, -NH-, -O- and -S-, and a 6-membered heteroaromatic ring which contains 1 or 2 =N- atoms and wherein a - HC=N- unit is optionally replaced by -NH-C(=O)-; and
wherein in heteroaryl and heterocyclyl rings with one or more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-.

### R³-G5:

According to another embodiment the group R³ is selected from the group R³-G5 consisting of
cyclopropyl substituted with 1 NC- group;
C₁₋₄-alkyl-O- optionally substituted with 1 or 2 H₃C- groups but necessarily substituted with 1 group selected from cyclopropyl, NC-, heterocyclyl-C(=O)-, H₂N-C(=O)-, H₃CNH-C(=O)-, (H₃C)₂N-C(=O)-, (H₃C)₂N-, HO-, C₁₋₂-alkyl-O-, C₁₋₂-alkyl-O-C₂-alkyl-O-, H₃C-S(=O)-, H₃C-S(=O)₂-, heterocyclyl, and heteroaryl;
wherein each heterocyclyl group and subgroup is selected from the group consisting of azetidinyl, oxetanyl, pyrrolidinyl, pyrrolidin-2-onyl, tetrahydrofuranyl, sulfolanyl, piperidinyl, tetrahydropyranyl, 1,1-dioxo-tetrahydrothiopyranyl, morpholinyl, and oxazolidin-2-onyl, each of which optionally substituted with 1 H₃C- group and wherein a NH group, if present, optionally is replaced by C₁₋₃-alkyl-S(=O)₂-N; and
wherein heteroaryl is selected from the group consisting of imidazolyl, pyrazolyl, oxazolyl, and pyridin-2-onyl, wherein a NH group, if present, optionally is replaced by N-CH₃ and each of which is optionally substituted with 1 H₃C- group; C₄₋₅-cycloalkyl-O- substituted with -N(CH₃)S(=O)₂CH₃ or -OH;
H₃C-C(=O)-, H₂N-C(=O)-, H₃C-NH-C(=O)-, (H₃C)₂N-C(=O)-, morpholin-4-yl-C(=O)-, 3,6-dihydropyranyl, 1-methanesulfonyl-1,2,3,6-tetrahydropyridinyl, pyrrolidin-2-onyl; azetidinyloxy and pyrrolidinyloxy, wherein in each of both the NH group is replaced by NR^{N};
tetrahydrofuranyloxy, tetrahydropyranyloxy, 1,1-dioxo-tetrahydrothiopyranyloxy, H₂N-S(=O)₂-; and
phenyl, pyrazolyl, oxazolyl, isoxazolyl, and pyrimidinyl, wherein a NH group, if present, optionally is replaced by N-CH₃.

### R³-G5a:

According to another embodiment the group R³ is selected from the group R³-G5a consisting of
C₅-alkyl substituted with 1 HO- group;
C₂₋₃-alkyl substituted with 1 group selected from H₃C-C(=O)-NH-, H₃C-S(=O)₂-NH-and H₃C-S(=O)₂-;
cyclopropyl substituted with 1 NC- group;
(H₃C)₃C-CH₂-O-; C₅-alkyl-O- substituted with 2 HO- groups;
cyclopropyl-CH₂-O- and cyclohexyl-CH₂-O-, each of both cycloalkyl groups is substituted with 1 HO- group and optionally substituted with 1 or 2 F atoms; C₁₋₄-alkyl-O- optionally substituted with 1 or 2 H₃C- groups and necessarily substituted with 1 group selected from cyclopropyl, NC-, heterocyclyl-C(=O)-, H₂N-C(=O)-, H₃CNH-C(=O)-, (H₃C)₂N-C(=O)-, (H₃C)₂N-, H₃C-C(=O)-NH-, (H₃C)₃C-O-C(=O)-NH-, H₃C-S(=O)₂-NH-, HO-, C₁₋₂-alkyl-O-, C₁₋₂-alkyl-O-C₂-alkyl-O-, H₃C-S(=O)-, H₃C-S(=O)₂-, heterocyclyl, phenyl and heteroaryl;
wherein each heterocyclyl group and subgroup is selected from the group consisting of azetidinyl, oxetanyl, pyrrolidinyl, pyrrolidin-2-onyl, tetrahydrofuranyl, sulfolanyl, 1,1-dioxo-isothiazolidinyl, piperidinyl, tetrahydropyranyl, 1,1-dioxo-tetrahydrothiopyranyl, morpholin-4-yl and oxazolidin-2-on-3-yl, each of which is optionally substituted with 1 group selected from H₃C- and HO- group, and wherein a NH group, if present, optionally is replaced by C₁₋₃-alkyl-S(=O)2-N; and
wherein heteroaryl is selected from the group consisting of imidazolyl, pyrazolyl, oxazolyl, pyridinyl and pyridin-2-onyl, wherein a NH group, if present, optionally is replaced by N-CH₃ and each heteroaryl is optionally substituted with 1 H₃C- or 1 H₃C-O- group;
C₄₋₅-cycloalkyl-O- substituted with 1 H₃C-S(=O)₂-N(CH₃)- or 1 HO- group and optionally substituted with 1 H₃C- group;
azetidinyloxy, pyrrolidinyloxy, pyrrolidin-2-onyloxy, piperidinyloxy and 1,1-dioxo-[1,2]thiazinanyloxy, in each of which the NH group is optionally replaced by N-CH₃ or N-S(=O)₂-CH₃;
tetrahydrofuranyloxy, tetrahydropyranyloxy, 1,1-dioxo-tetrahydrothiopyranyloxy and phenyloxy;
H₃C-C(=O)-, H₂N-C(=O)-, H₃C-NH-C(=O)-, HO-(H₃C)₂C-CH₂-NH-C(=O)-, (H₃C)₂N-C(=O)-, tetrahydropyran-4-yl-NH-C(=O)-, morpholin-4-yl-C(=O)-, pyrrolidinyl, tetrahydrofuranyl, piperidinyl, morpholin-4-yl, [1,4]oxazepan-4-yl, tetrahydropyranyl, 3,6-dihydropyranyl, 1-methanesulfonyl-1,2,3,6-tetrahydropyridinyl, pyrrolidin-2-onyl, 6-oxo-3,6-d ihydro-pyran-4-yl;
H₃C-S(=O)₂-NH-, H₂N-S(=O)₂-;
C₃-alkyl-S(=O)₂- substituted with 1 HO- or H₃C-O- group; and
phenyl, furanyl, imidazolyl, pyrazolyl, oxazolyl, isoxazolyl, thiazolyl, triazolyl, oxadiazolyl, tetrazolyl, pyridinyl, pyrimidinyl, pyridazinyl, pyrazinyl, pyridin-2-onyl, pyrimidin-2-onyl, pyrimidin-4-onyl and pyridazin-3-onyl, wherein a NH group, if present, optionally is replaced by N-CH₃, N-CH₂-C(CH₃)₂-OH or N-C(CH₃)₂-CH₂-OH, and which are optionally substituted with 1 H₃C- group and optionally substituted with 1 group selected from -CH₃, -CH₂-CH₃, -CH(CH₃)₂, cyclopropyl, -CF₃, -C(CH₃)₂-OH, -O-CH₃ and -CH₂-C(CH₃)₂-OH.

### R³-G6:

According to another embodiment the group R³ is selected from the group R³-G6 consisting of

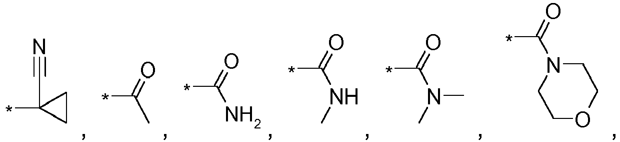

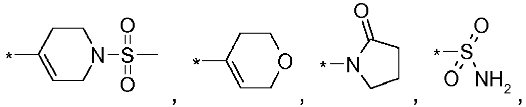

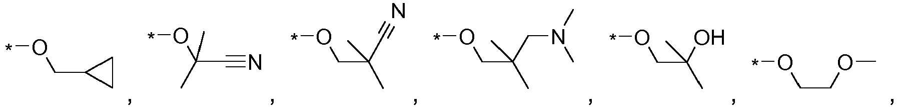

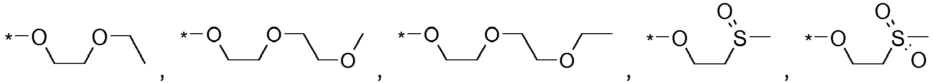

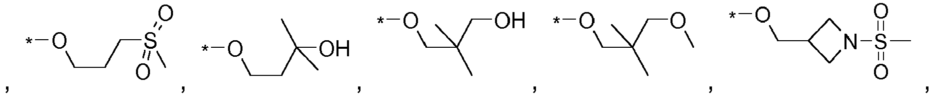

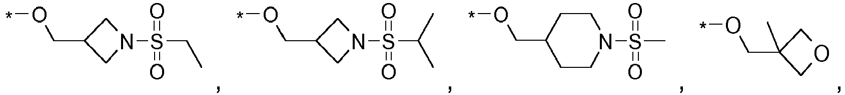

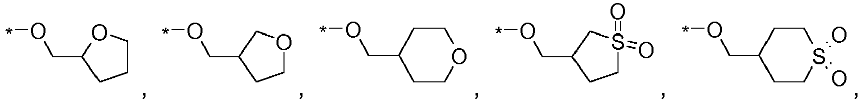

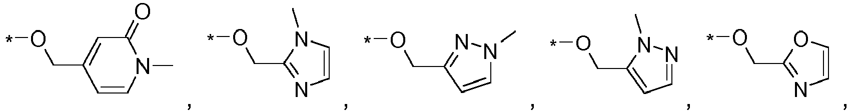

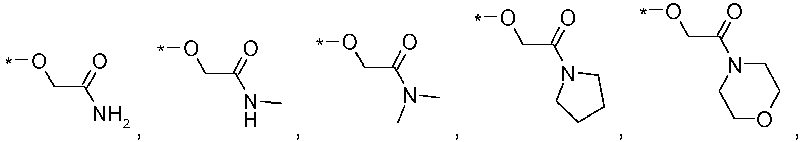

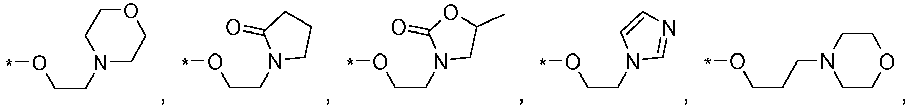

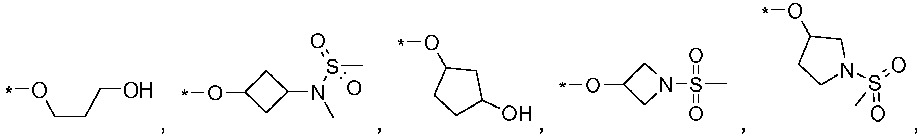

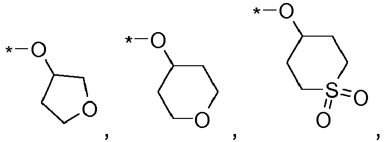

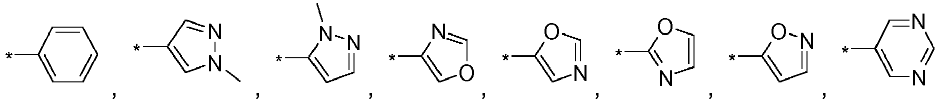

### R³-G6a:

According to another embodiment the group R³ is selected from the group R³-G6a consisting of

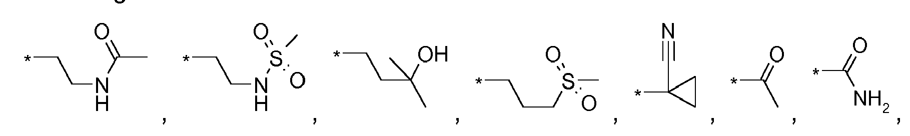

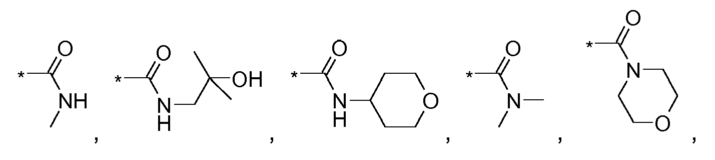

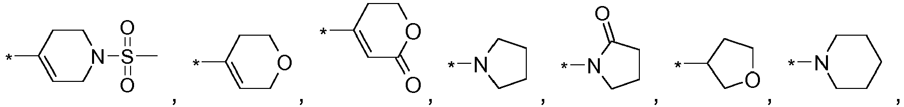

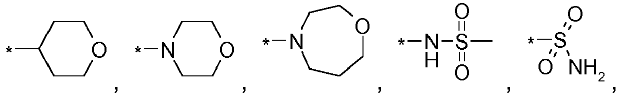

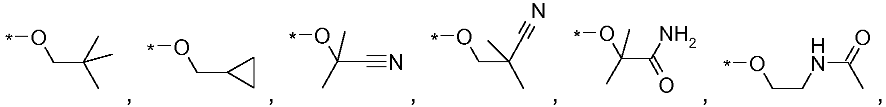

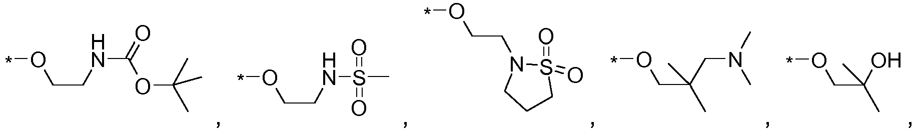

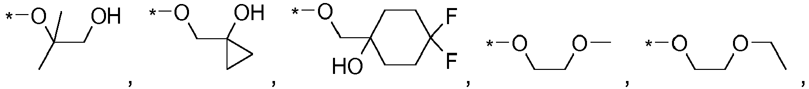

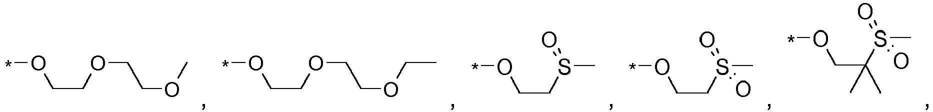

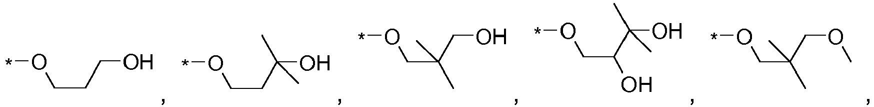

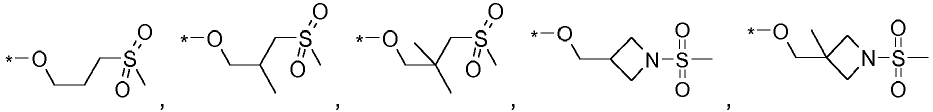

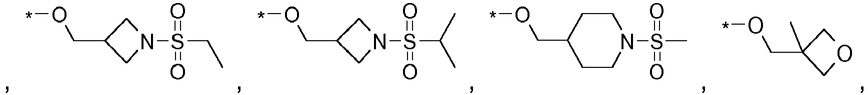

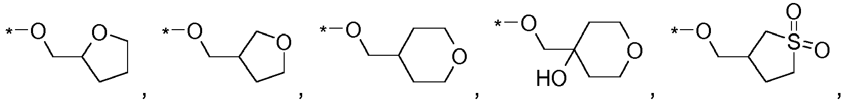

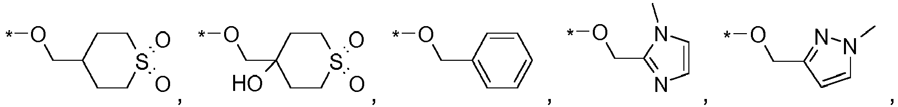

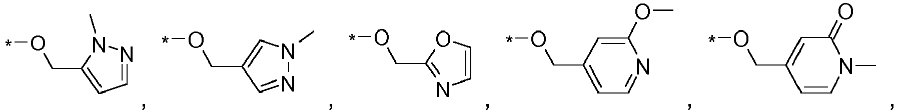

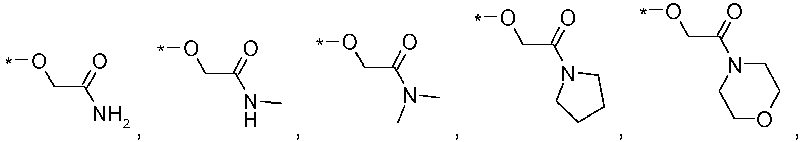

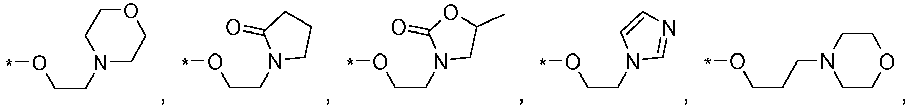

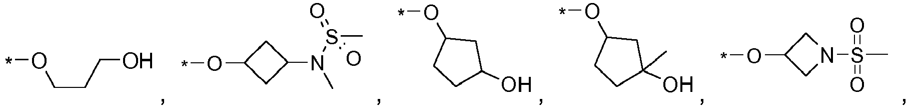

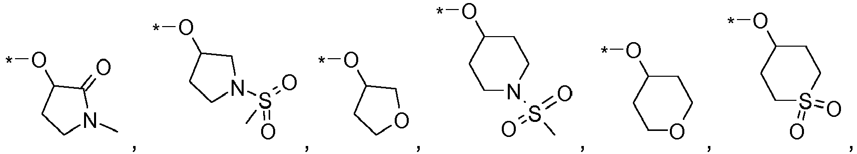

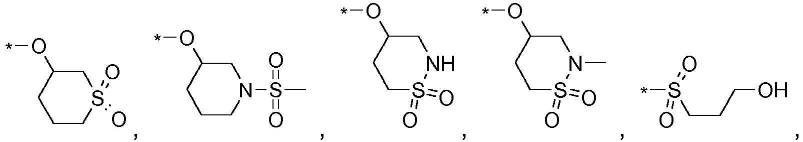

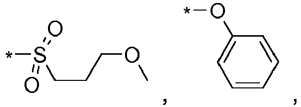

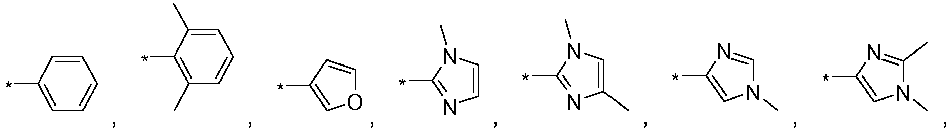

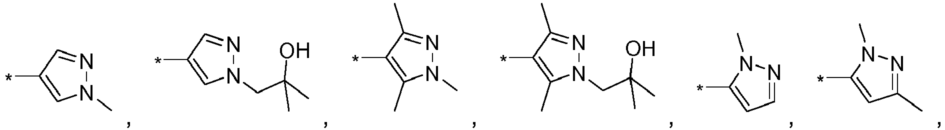

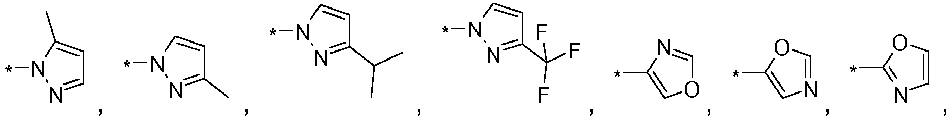

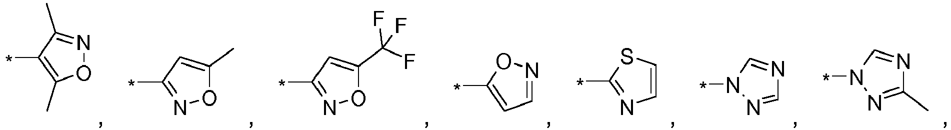

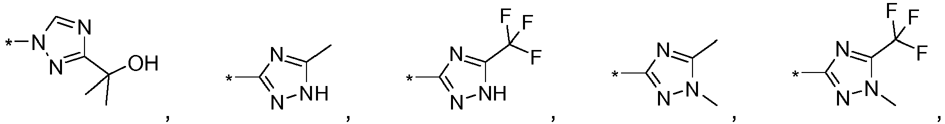

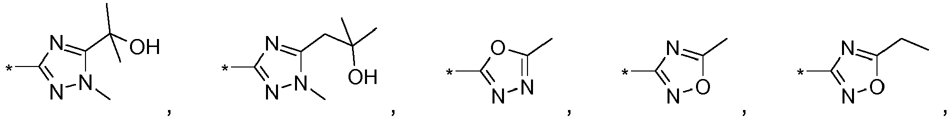

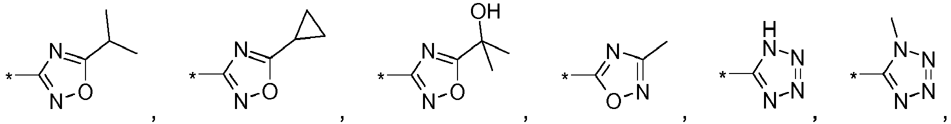

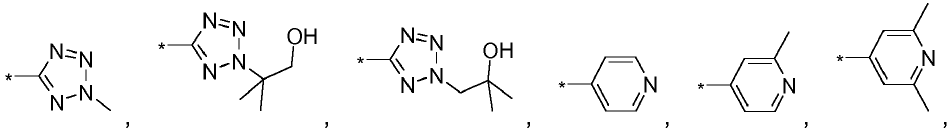

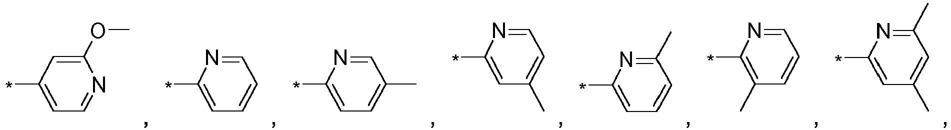

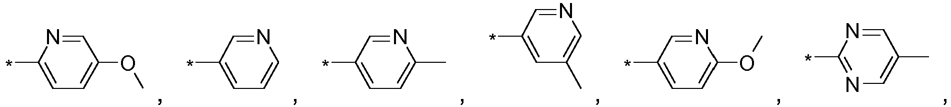

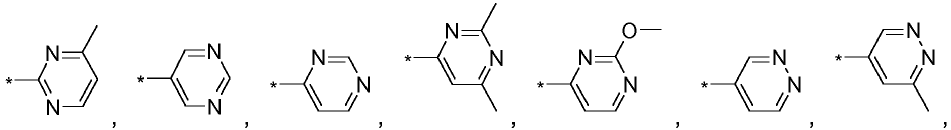

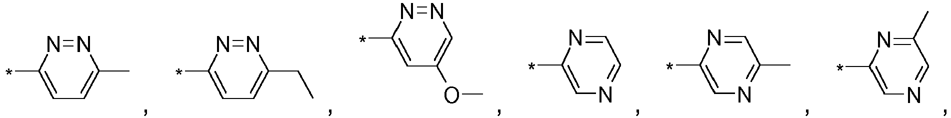

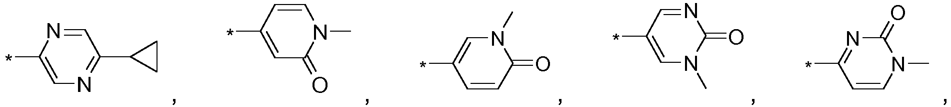

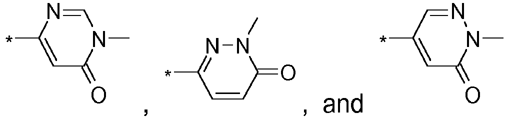

### R⁴

### R⁴-G1:

The group R⁴ is preferably selected from the group R⁴-G1 as defined hereinbefore.

### R⁴-G2:

In another embodiment the group R⁴ is selected from the group R⁴-G2 consisting of F, Cl, Br, CN, C₁₋₃-alkyl, C₃₋₄-cycloalkyl-, HO-C₁₋₃-alkyl, C₁₋₃-alkyl-O-C₁₋₃-alkyl, -NR^{N}H, C₁₋₄-alkyl-O-, C₃₋₅-cycloalkyl-O-, H₃C-S(=O)-, H₃C-S(=O)₂-, wherein any alkyl and cycloalkyl group is optionally substituted with 1 or more F atoms.

### R⁴-G3:

In another embodiment the group R⁴ is selected from the group R⁴-G3 consisting of F, Cl, CN, -CH₃, -CF₃, isopropyl, cyclopropyl, H₃C-O-CH₂-, H₃C-O-, and F₃C-O-.

### R⁴-G4:

In another embodiment the group R⁴ is selected from the group R⁴-G4 consisting of CH₃.

### R⁴-G4a:

In another embodiment the group R⁴ is selected from the group R⁴-G4a consisting of F and CH₃.

### R⁵

### R⁵-G1:

The group R⁵ is preferably selected from the group R⁵-G1 as defined hereinbefore.

### R⁵-G2:

In one embodiment the group R⁵ is selected from the group R⁵-G2 consisting of Cl, C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₃₋₆-cycloalkyl-NR^{N}-C(=O)-, heterocyclyl-NR^{N}-C(=O)-, heteroaryl-NR^{N}-C(=O)-, -NH₂, C₁₋₄-alkyl-NR^{N}-, C₁₋₄-alkyl-C(=O)NR^{N}-, C₃₋₆-cycloalkyl-C(=O)NR^{N}-, heterocyclyl-C(=O)NR^{N}-, heteroaryl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, phenyl-O-, heteroaryl-O-, C₁₋₄-alkyl-S(=O)-, C₃₋₆-cycloalkyl-S(=O)-, C₁₋₄-alkyl-S(=O)2-, C₃₋₆-cycloalkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a tetrazolyl ring, a pyridin-2-onyl ring, a 5-membered heteroaromatic ring which contains 1, 2, or 3 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 to 3 substituents independently selected from F, Cl, -CH₃, -CN, and -O-CH₃.

### R⁵-G2a:

In one embodiment the group R⁵ is selected from the group R⁵-G2a consisting of Cl, C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₃₋₆-cycloalkyl-NR^{N}-C(=O)-, heterocyclyl-NR^{N}-C(=O)-, heteroaryl-NR^{N}-C(=O)-, -NH₂, C₁₋₄-alkyl-NR^{N}-, -NHC(=O)-O-C(CH₃)₃, C₁₋₄-alkyl-C(=O)NR^{N}-, C₃₋₆-cycloalkyl-C(=O)NR^{N}-, heterocyclyl-C(=O)NR^{N}-, heteroaryl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, phenyl-O-, heteroaryl-O-, C₁₋₄-alkyl-S(=O)-, C₃₋₆-cycloalkyl-S(=O)-, C₁₋₄-alkyl-S(=O)₂-, C₃₋₆-cycloalkyl-S(=O)₂-, heterocyclyl, phenyl and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a tetrazolyl ring, a pyridin-2-onyl ring, a 5-membered heteroaromatic ring which contains 1, 2, or 3 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 to 3 substituents independently selected from F, Cl, -CH₃, -CN, and -O-CH₃.

### R⁵-G3:

In another embodiment the group R⁵ is selected from the group R⁵-G3 consisting of C₁₋₄-alkyl-, -CN, C₃₋₆-cycloalkyl-, heterocyclyl-C(=O)-, H₂N-C(=O)-, C1₋4-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, H₃C-O-, and -CN,
wherein heterocyclyl is selected from
a cyclobutyl group wherein 1 CH₂ group is replaced by -NR^{N}- or -O-,
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from
a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 or 2 substituents independently selected from F, Cl, -CH₃, -CN, and -O-CH₃.

### R⁵-G3a:

In another embodiment the group R⁵ is selected from the group R⁵-G3a consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, -NHC(=O)-O-C(CH₃)₃, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, phenyl and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O- and -CN,
wherein heterocyclyl is selected from
a cyclobutyl group wherein 1 CH₂ group is replaced by -NR^{N}- or -O-,
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N; and wherein heteroaryl is selected from
a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 or 2 substituents independently selected from F, Cl, -CH₃, -CN, and -O-CH₃.

### R⁵-G4:

In another embodiment the group R⁵ is selected from the group R⁵-G4 consisting of C₁₋₂-alkyl-, -CN, C₃₋₅-cycloalkyl-, heterocyclyl-C(=O)-, H₂N-C(=O)-, -C(=O)NHCH₃, -C(=O)N(CH₃)₂, -N(CH₃)₂, H₃C-C(=O)NH-, H₃C-C(=O)NCH₃-, H₃C-S(=O)₂NH-, H₃C-S(=O)₂NCH₃-, -OH, C₁₋₃-alkyl-O-, C₁₋₂-alkyl-O-C₂-alkyl-O-, C₃₋₅-cycloalkyl-O-, heterocyclyl-O-, H₃C-S(=O)-, H₃C-S(=O)₂-, heterocyclyl, and heteroaryl,
wherein heterocyclyl is selected from
an azetidinyl, oxetanyl, a pyrrolidinyl, pyrrolidin-2-onyl, tetrahydrofuranyl, sulfolanyl, piperidinyl, tetrahydropyranyl, 1,1-dioxo-tetrahydrothiopyranyl, morpholinyl, and an oxazolidin-2-onyl ring, wherein each of these rings optionally is substituted with 1 CH₃ group and wherein an NH group, if present, optionally is replaced with NS(=O)₂-C₁₋₃-alkyl;
and wherein heteroaryl is selected from
a pyridin-2-onyl, imidazolyl, pyrazolyl, and oxazolyl ring, wherein in a heteroaryl group with a NH group this unit optionally is replaced by a N-CH₃ group.

### R⁵-G4a:

In another embodiment the group R⁵ is selected from the group R⁵-G4a consisting of C₁₋₂-alkyl-, -CN, 2-hydroxy-isobutyl, C₃₋₅-cycloalkyl, 1-hydroxy-cyclopropyl, 4,4-difluoro-1-hydroxy-cyclohex-1-yl, heterocyclyl-C(=O)-, H₂N-C(=O)-, -C(=O)NHCH₃, -C(=O)N(CH₃)₂, -N(CH₃)₂, H₃C-C(=O)NH-, H₃C-C(=O)NCH₃-, H₃C-S(=O)₂NH-, H₃C-S(=O)₂NCH₃-, -NHC(=O)-O-C(CH₃)₃, -OH, C₁₋₃-alkyl-O-, C₁₋₂-alkyl-O-C₂-alkyl-O-, C₃₋₅-cycloalkyl-O-, heterocyclyl-O-, H₃C-S(=O)-, H₃C-S(=O)₂-, heterocyclyl, phenyl, and heteroaryl,
wherein heterocyclyl is selected from
an azetidinyl, oxetanyl, a pyrrolidinyl, pyrrolidin-2-onyl, tetrahydrofuranyl, sulfolanyl, piperidinyl, tetrahydropyranyl, 1,1-dioxo-tetrahydrothiopyranyl, morpholinyl, 1,1-dioxo-isothiazolidin-2-yl and an oxazolidin-2-onyl ring, wherein each of these rings optionally is substituted with 1 CH₃ or 1 OH group and wherein an NH group, if present, optionally is replaced with NS(=O)₂-C₁₋₃-alkyl;
and wherein heteroaryl is selected from
pyridin-2-onyl, pyridinyl, imidazolyl, pyrazolyl, and oxazolyl ring, each of which is optionally substituted with a H₃C-O- group and wherein a heteroaryl group with a NH group this unit optionally is replaced by a N-CH₃ group.

### R⁵-G5:

According to another embodiment the group R⁵ is selected from the group R⁵-G5 consisting of -CH₃, cyclopropyl, -CN, -C(=O)NH₂, -C(=O)NHCH₃, -C(=O)N(CH₃)₂, -N(CH₃)₂, -N(CH₃)S(=O)₂CH₃, -OH, -O-CH₃, -O-CH₂CH₃, -O-CH₂CH₂-O-CH₃, -O-CH₂CH₂-O-CH₂CH₃, -S(=O)CH₃, -S(=O)₂CH₃,

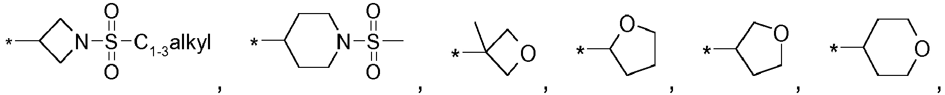

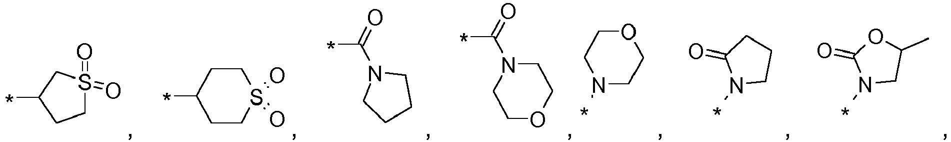

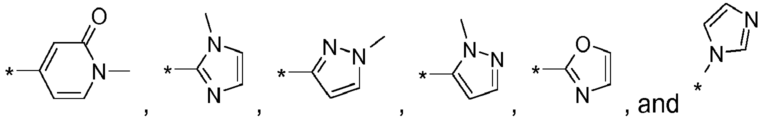

### R⁵-G5a:

According to another embodiment the group R⁵ is selected from the group R⁵-G5a consisting of -CH₃, cyclopropyl, -CN, -C(=O)NH₂, -C(=O)NHCH₃, -C(=O)N(CH₃)₂, -N(CH₃)₂, -NHC(=O)CH₃, -NHS(=O)₂CH₃, N(CH₃)S(=O)₂CH₃, NHC(=O)-O-C(CH₃)₃, -OH, -O-CH₃, -O-CH₂CH₃, -O-CH₂CH₂-O-CH₃, -O-CH₂CH₂-O-CH₂CH₃, -S(=O)CH₃, -S(=O)₂CH₃,

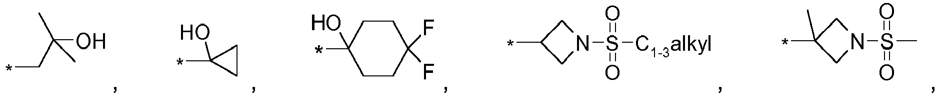

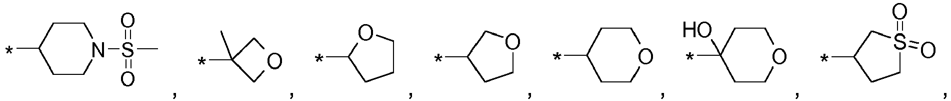

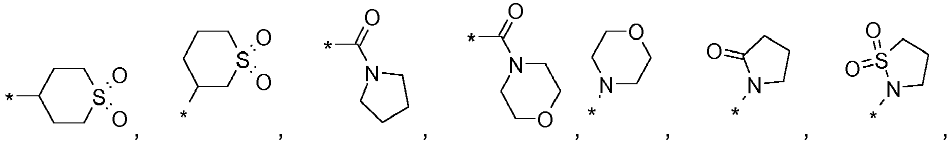

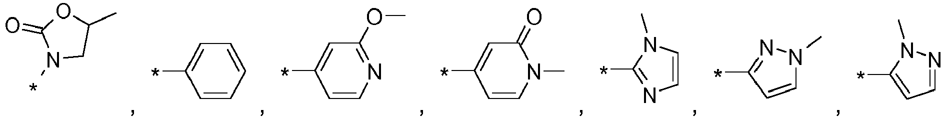

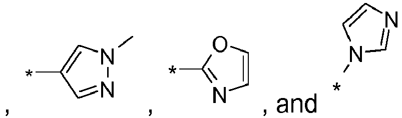

### R⁶

### R⁶-G1:

The group R⁶ is preferably selected from the group R⁶-G1 as defined hereinbefore.

### R⁶-G2:

In one embodiment the group R⁶ is selected from the group R⁶-G2 consisting of F, Cl, -CN, C₁₋₃-alkyl, cyclopropyl, HO-C₁₋₃-alkyl-, H₃C-O-C₁₋₃-alkyl-, H₃C-O-, -S(=O)CH₃, and -S(=O)₂-CH₃, wherein any alkyl and cycloalkyl group or sub-group within the groups mentioned is optionally substituted with one or more F atoms.

### R⁶-G2a:

In one embodiment the group R⁶ is selected from the group R⁶-G2a consisting of F, Cl, -CN, C₁₋₃-alkyl, cyclopropyl, HO-C₁₋₄-alkyl-, H₃C-O-C₁₋₄-alkyl-, H₃C-O-, -S(=O)CH₃, and -S(=O)₂-CH₃, wherein any alkyl and cycloalkyl group or sub-group within the groups mentioned is optionally substituted with one or more F atoms.

### R⁶-G3:

In another embodiment the group R⁶ is selected from the group R⁶-G3 consisting of F, Cl, -CN, -CH₃, -CF₃, -OCH₃, -OCF₃, -S(=O)CH₃, and -S(=O)₂-CH₃.

### R⁶-G3a:

In another embodiment the group R⁶ is selected from the group R⁶-G3a consisting of F, Cl, C₁₋₃-alkyl, cyclopropyl, HO-C₁₋₄-alkyl-, -CN, -CF₃, -OCH₃, -OCF₃, -S(=O)CH₃, and -S(=O)₂-CH₃.

### R⁶-G4:

In another embodiment the group R⁶ is selected from the group R⁶-G4 consisting of F, -CH₃, CN, and -OCH₃.

### R⁶-G4a:

In another embodiment the group R⁶ is selected from the group R⁶-G4a consisting of F, -CH₃, -CH₂-CH₃, -CH(CH₃)₂, cyclopropyl, HO-C(CH₃)₂-, HO-CH₂-C(CH₃)₂-, HO-C(CH₃)₂-CH₂-, -CF₃, CN, and -OCH₃.

### R^{N}

### R^{N}-G1:

The group R^{N} is preferably selected from the group R^{N}-G1 as defined hereinbefore.

### R^{N}-G2:

In another embodiment the group R^{N} is selected from the group R^{N}-G2 consisting of H, C₁₋₃-alkyl, H₃C-C(=O)-, and C₁₋₃-alkyl-S(=O)₂-.

### R^{N}-G3:

In another embodiment the group R^{N} is selected from the group R^{N}-G3 consisting of H, H₃C-, H₃C-C(=O)-, and C₁₋₃-alkyl-S(=O)₂-.

### m:

According to another embodiment the index m is 1 or 2.

According to another embodiment the index m is 1.

According to another embodiment the index m is 2.

Examples of preferred subgeneric embodiments (E) according to the present invention are set forth in the following table, wherein each substituent group of each embodiment is defined according to the definitions set forth hereinbefore and wherein all other substituents of the formula **I** are defined according to the definitions set forth hereinbefore:

| **E** | **R¹-** | **R²-** | **R³-** | **R⁴-** | **R⁵-** | **R⁶-** | **R^{N}-** | **m** |
|---|---|---|---|---|---|---|---|---|
| **E-3** | **R¹-G3a** | **R²-G3** | **R³-G2** | **R⁴-G3** | **R⁵-G3** | **R⁶-G3** | **R^{N}-G3** | **1,2** |
| **E-4** | **R¹-G3a** | **R²-G3** | **R³-G3** | **R⁴-G3** | **R⁵-G3** | **R⁶-G3** | **R^{N}-G3** | **1,2** |
| **E-5** | **R¹-G4a** | **R²-G3** | **R³-G2** | **R⁴-G3** | **R⁵-G3** | **R⁶-G3** | **R^{N}-G3** | **1,2** |
| **E-6** | **R¹-G4a** | **R²-G3** | **R³-G3** | **R⁴-G3** | **R⁵-G3** | **R⁶-G3** | **R^{N}-G3** | **1,2** |
| **E-7** | **R¹-G4a** | **R²-G3** | **R³-G4** | **R⁴-G3** | **R⁵-G3** | **R⁶-G3** | **R^{N}-G3** | **1,2** |
| **E-8** | **R¹-G4a** | **R²-G3** | **R³-G4** | **R⁴-G3** | **R⁵-G3** | **R⁶-G4** | **R^{N}-G3** | **1,2** |
| **E-9** | **R¹-G4a** | **R²-G3** | **R³-G4** | **R⁴-G3** | **R⁵-G4** | **R⁶-G4** | **R^{N}-G3** | **1,2** |
| **E-10** | **R¹-G4a** | **R²-G4** | **R³-G4** | **R⁴-G3** | **R⁵-G5** | **R⁶-G4** | **R^{N}-G3** | **1,2** |
| **E-11** | **R¹-G5** | **R²-G3** | **R³-G4** | **-** | **R⁵-G4** | **R⁶-G4** | **R^{N}-G3** | **1,2** |
| **E-12** | **R¹-G5** | **R²-G3** | **R³-G4** | **-** | **R⁵-G3** | **R⁶-G3** | **R^{N}-G3** | **1,2** |
| **E-13** | **R¹-G5** | **R²-G4** | **R³-G4** | **-** | **R⁵-G3** | **R⁶-G3** | **R^{N}-G2** | **1,2** |
| **E-14** | **R¹-G5** | **R²-G4** | **R³-G4** | **-** | **R⁵-G3** | **R⁶-G3** | **R^{N}-G3** | **1,2** |
| **E-15** | **R¹-G5** | **R²-G4** | **R³-G4** | **-** | **R⁵-G3** | **R⁶-G4** | **R^{N}-G3** | **1,2** |
| **E-16** | **R¹-G5** | **R²-G4** | **R³-G4** | **-** | **R⁵-G4** | **R⁶-G4** | **R^{N}-G3** | **1,2** |
| **E-17** | **R¹-G5** | **R²-G4** | **R³-G3** | **-** | **R⁵-G5** | **R⁶-G4** | **R^{N}-G3** | **1,2** |
| **E-18** | **R¹-G5** | **R²-G4** | **R³-G4** | **-** | **R⁵-G5** | **R⁶-G4** | **R^{N}-G3** | **1,2** |
| **E-19** | **R¹-G5** | **R²-G4** | **R³-G5** | **-** | **-** | **-** | **-** | **1,2** |
| **E-20** | **R¹-G5** | **R²-G4** | **R³-G6** | **-** | **-** | **-** | **-** | **1,2** |
| **E-21** | **R¹-G4a** | **R²-G4a** | **R³-G2a** | **R⁴-G3** | **R⁵-G2a** | **R⁶-G2a** | **R^{N}-G3** | **1** |
| **E-22** | **R¹-G4a** | **R²-G4a** | **R³-G3a** | **R⁴-G3** | **R⁵-G3a** | **R⁶-G3a** | **R^{N}-G3** | **1** |
| **E-23** | **R¹-G5a** | **R²-G4a** | **R³-G3a** | **-** | **R⁵-G3a** | **R⁶-G3a** | **R^{N}-G3** | **1** |
| **E-24** | **R¹-G5a** | **R²-G4a** | **R³-G4a** | **-** | **R⁵-G4a** | **R⁶-G3a** | **R^{N}-G3** | **1** |
| **E-25** | **R¹-G5a** | **R²-G4a** | **R³-G5a** | **-** | **-** | **-** | **-** | **1** |
| **E-26** | **R¹-G5a** | **R²-G4a** | **R³-G6a** | **-** | **-** | **-** | **-** | **1** |
| **E-27** | **R¹-G5** | **R²-G4a** | **R³-G1** | **-** | **R⁵-G1** | **R⁶-G1** | **R^{N}-G3** | **1** |
| **E-28** | **R¹-G5** | **R²-G4a** | **R³-G2a** | **-** | **R⁵-G2a** | **R⁶-G2a** | **R^{N}-G3** | **1** |
| **E-29** | **R¹-G5** | **R²-G4a** | **R³-G2a** | **-** | **R⁵-G3a** | **R⁶-G3a** | **R^{N}-G3** | **1** |
| **E-30** | **R¹-G5** | **R²-G4a** | **R³-G3a** | **-** | **R⁵-G3a** | **R⁶-G3a** | **R^{N}-G3** | **1** |
| **E-31** | **R¹-G5** | **R²-G4a** | **R³-G3a** | **-** | **R⁵-G4a** | **R⁶-G3a** | **R^{N}-G3** | **1** |
| **E-32** | **R¹-G5** | **R²-G4a** | **R³-G4a** | **-** | **R⁵-G4a** | **R⁶-G3a** | **R^{N}-G3** | **1** |
| **E-33** | **R¹-G5** | **R²-G4a** | **R³-G4a** | **-** | **R⁵-G5a** | **R⁶-G4a** | **R^{N}-G3** | **1** |
| **E-34** | **R¹-G5** | **R²-G4a** | **R³-G5a** | **-** | **-** | **-** | **-** | **1** |
| **E-35** | **R¹-G5** | **R²-G4a** | **R³-G6a** | **-** | **-** | **-** | **-** | **1** |

The following preferred embodiments of compounds of the formula I are described using generic formulas (1.1) to (1.3), wherein any tautomers, solvates, hydrates and salts thereof, in particular the pharmaceutically acceptable salts thereof, are encompassed.

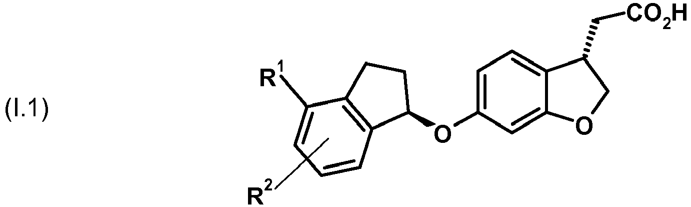

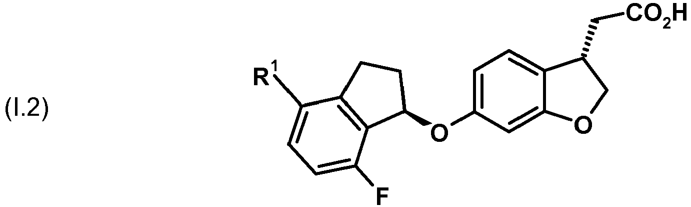

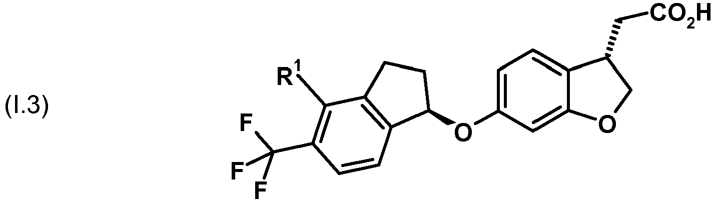

Preferred are those compounds of formula I, wherein
R¹ is selected from the group consisting of 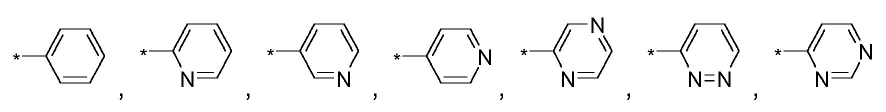 and 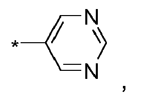 wherein each group is substituted with one group R³ and optionally additionally substituted with 1 or 2 groups independently selected from R⁴;
R² is selected from the group consisting of F, Cl, Br, H₂FC-, F₃C-, NC-, and F₃C-O-;
R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from H₃C-C(=O)-, heterocyclyl-C(=O)-, H₂N-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl, and heteroaryl,
wherein each alkyl, cycloalkyl, and heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-;
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or -S(=O)₂- and/or 1 CH group is replaced by N; and
wherein heteroaryl is selected from a pyridin-2-onyl ring, a 5-membered heteroaromatic ring which contains 1, 2, or 3 heteroatoms independently of each other selected from =N-, -NH-, O, and S, and a 6-membered heteroaromatic ring which contains 1 or 2 =N- atoms,
and wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁴ is selected from the group consisting of F, Cl, CN, -CH₃, -CF₃, isopropyl, cyclopropyl, H₃C-O-CH₂-, H₃C-O-, and F₃C-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}-, or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore moreNH groups each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, Cl, -CN, -CH₃, -CF₃, -OCH₃, -OCF₃, -S(=O)CH₃, and -S(=O)₂-CH₃;
R^{N} is selected from the group consisting of H, H₃C-, H₃C-C(=O)-, and C₁₋₃-alkyl-S(=O)₂-; and
m is 1 or 2; and the pharmaceutically acceptable salts thereof.

More preferred are those compounds of formula I, wherein
R¹ is 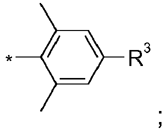 R² is selected from the group consisting of F, Cl, and NC-;
R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 group selected from R⁵ and optionally substituted with 1 or 2 H₃C- group;
or from H₃C-C(=O)-, heterocyclyl-C(=O)-, H₂N-C(=O)-, H₃C-NR^{N}-C(=O)-, heterocyclyl-O-, heteroaryl-O-, H₂N-S(=O)₂-, heterocyclyl, phenyl, and heteroaryl,
wherein each heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 or 2 substituents independently selected from R⁶;
wherein heterocyclyl is selected from
a cyclobutyl group wherein 1 CH₂ group is replaced by -NH- or -O-;
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)- or -S(=O)₂- and/or 1 CH group is replaced by N; and
wherein heteroaryl is selected from a 5-membered heteroaromatic ring which contains 1 or 2 heteroatoms independently of each other selected from =N-, -NH-, -O-, and -S-, and a 6-membered heteroaromatic ring which contains 1 or 2 =N-atoms, and wherein in heteroaryl and heterocyclyl rings with one ore more NH group each of them is replaced with NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}-, or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH group each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, -CH₃, CN, and -OCH₃;
R^{N} is selected from the group consisting of H, H₃C-, H₃C-C(=O)-, and C₁₋₃-alkyl-S(=O)₂-; and
m is 1 or 2; and the pharmaceutically acceptable salts thereof.

Preferred are those compounds of formula (I.2), wherein
R¹ is 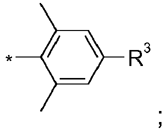 R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 to 3 F atoms;
or selected from
C₃-alkyl-S(=O)₂- substituted with 1 HO- or H₃C-O- group;
H₃C-C(=O)-, heterocyclyl-C(=O)-, H₂N-C(=O)-, tetrahydropyran-4-yl-NH-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl and heteroaryl,
wherein each alkyl, cycloalkyl, and heterocyclyl group or sub-group within the groups mentioned is optionally substituted with 1 or 2 groups independently selected from R⁵ and optionally substituted with 1 to 3 F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₇-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or -S(=O)₂- and/or 1 CH group is replaced by N; and
wherein heteroaryl is selected from
tetrazolyl, a 5-membered heteroaromatic ring which contains 1, 2 or 3 heteroatoms independently of each other selected from =N-, -NH-, O, and S, and a 6-membered heteroaromatic ring which contains 1 or 2 =N- atoms and wherein a -HC=N- unit is optionally replaced by -NH-C(=O)-; and
wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, -NHC(=O)-O-C(CH₃)₃, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, phenyl and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O- and -CN,
wherein heterocyclyl is selected from
a cyclobutyl group wherein 1 CH₂ group is replaced by -NR^{N}- or -O-,
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from
a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, Cl, C₁₋₃-alkyl, cyclopropyl, HO-C₁₋₄-alkyl-, -CN, -CF₃, -OCH₃, -OCF₃, -S(=O)CH₃, and -S(=O)₂-CH₃;
R^{N} is selected from the group consisting of H, H₃C-, H₃C-C(=O)-, and C₁₋₃-alkyl-S(=O)₂-;
and the pharmaceutically acceptable salts thereof.

Preferred are those compounds of formula (I.3), wherein
R¹ is 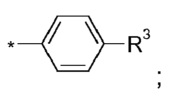 R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 to 3 F atoms;
or selected from
C₃-alkyl-S(=O)₂- substituted with 1 HO- or H₃C-O- group;
H₃C-C(=O)-, heterocyclyl-C(=O)-, H₂N-C(=O)-, tetrahydropyran-4-yl-NH-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl and heteroaryl,
wherein each alkyl, cycloalkyl, and heterocyclyl group or sub-group within the groups mentioned is optionally substituted with 1 or 2 groups independently selected from R⁵ and optionally substituted with 1 to 3 F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₇-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or -S(=O)₂- and/or 1 CH group is replaced by N; and
wherein heteroaryl is selected from
tetrazolyl, a 5-membered heteroaromatic ring which contains 1, 2 or 3 heteroatoms independently of each other selected from =N-, -NH-, O, and S, and a 6-membered heteroaromatic ring which contains 1 or 2 =N- atoms and wherein a -HC=N- unit is optionally replaced by -NH-C(=O)-; and
wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, -NHC(=O)-O-C(CH₃)₃, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, C₁₋₄-alkyl-S(=O)-, C₁₋₄-alkyl-S(=O)₂-, heterocyclyl, phenyl and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O- and -CN,
wherein heterocyclyl is selected from
a cyclobutyl group wherein 1 CH₂ group is replaced by -NR^{N}- or -O-,
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from
a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, Cl, C₁₋₃-alkyl, cyclopropyl, HO-C₁₋₄-alkyl-, -CN, -CF₃, -OCH₃, -OCF₃, -S(=O)CH₃, and -S(=O)₂-CH₃;
R^{N} is selected from the group consisting of H, H₃C-, H₃C-C(=O)-, and C₁₋₃-alkyl-S(=O)₂-;
and the pharmaceutically acceptable salts thereof.

Particularly preferred compounds, including their tautomers and stereoisomers, the salts thereof, or any solvates or hydrates thereof, are described in the experimental section hereinafter.

The compounds according to the invention and their intermediates may be obtained using methods of synthesis which are known to the one skilled in the art and described in the literature of organic synthesis. Preferably the compounds are obtained analogously to the methods of preparation explained more fully hereinafter, in particular as described in the experimental section. In some cases the sequence adopted in carrying out the reaction schemes may be varied. Variants of these reactions that are known to the skilled man but are not described in detail here may also be used. The general processes for preparing the compounds according to the invention will become apparent to the skilled man on studying the schemes that follow. Starting compounds are commercially available or may be prepared by methods that are described in the literature or herein, or may be prepared in an analogous or similar manner. Before the reaction is carried out any corresponding functional groups in the compounds may be protected using conventional protecting groups. These protecting groups may be cleaved again at a suitable stage within the reaction sequence using methods familiar to the skilled man.

The compounds of the invention **I** are preferably accessed from a precursor **1** that bears the carboxylic acid function in a protected or masked form as sketched in Scheme 1; R¹, R², and m have the meanings as defined hereinbefore and hereinafter. Suited precursor groups for the carboxylic acid may be, e.g., a carboxylic ester, a carboxylic amide, cyano, an olefin, oxazole, or a thiazole. All these groups have been transformed into the carboxylic acid function by different means which are described in the organic chemistry literature and are known to the one skilled in the art. The preferred precursor group is a C₁₋₄-alkyl or benzyl carboxylate, each of which may be additionally mono- or polysubstituted with fluorine, methyl, and/or methoxy. These ester groups may be hydrolysed with an acid, such as hydrochloric acid or sulfuric acid, or an alkali metal hydroxide, such as lithium hydroxide, sodium hydroxide, or potassium hydroxide, to yield the carboxylic acid function; the hydrolysis is preferably conducted in aqueous solvents, such as water and tetrahydrofuran, 1,4-dioxane, alcohol, e.g. methanol, ethanol, and isopropanol, or dimethyl sulfoxide, at 0 to 120 °C. A tert-butyl ester is preferably cleaved under acidic conditions, e.g. trifluoroacetic acid or hydrochloric acid, in a solvent such as dichloromethane, 1,4-dioxane, isopropanol, or ethyl acetate. A benzyl ester is advantageously cleaved using hydrogen in the presence of a transition metal, preferably palladium on carbon.

Benzyl esters bearing electron donating groups, such as methoxy groups, on the aromatic ring may also be removed under oxidative conditions; ceric ammonium nitrate (CAN) or 2,3-dichloro-5,6-dicyanoquinone (DDQ) are two commonly used reagents for this approach.

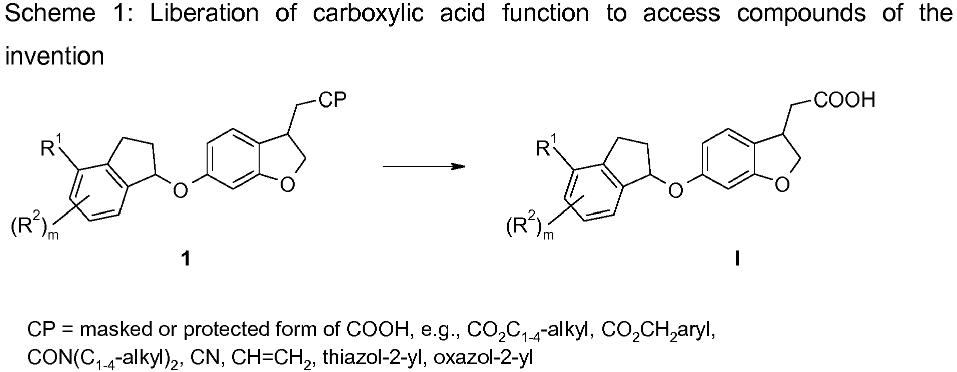

Compound **1**, in turn, may be obtained from indane **2**, which bears a leaving group, and phenol **3**, which is decorated with the carboxylic acid precursor group (Scheme 2); R¹, R², and m in Scheme 2 have the meanings as defined hereinbefore and hereinafter. The leaving group LG in **2** is replaced with the O in **3** via a nucleophilic substitution; suited LG may be Cl, Br, I, methylsulfonyloxy, phenylsulfonyloxy, p-tolylsulfonyloxy, and trifluoromethylsulfonyloxy. The reaction is usually carried out in the presence of a base, such as triethylamine, ethyldiisopropylamine, 1,8-diazabicyclo[5.4.0]undecene, carbonates, e.g. Li₂CO₃, Na₂CO₃, K₂CO₃, and Cs₂CO₃, hydroxides, e.g. LiOH, NaOH, and KOH, alcoholates, e.g. NaOMe, NaOEt, and KOtBu, hydrides, e.g. NaH and KH, amides, e.g. NaNH₂, KN(SiMe₃)₂, and LiN(iPr)₂, and oxides, e.g. CaO and Ag₂O. Additives, such as silver salts, e.g. AgNO₃, AgOSO₂CF₃, and Ag₂CO₃, crown ethers, e.g. 12-crown-4, 15-crown-5, and 18-crown-6, hexamethylphosphorus triamide (HMPT), and 1,3-dimethyl-3,4,5,6-dihydro-2-pyrimidinone (DMPU), may be beneficial or even essential for the reaction to proceed. Preferred solvents are dimethylsulfoxide, N,N-dimethylformamide, N,N-dimethylacetamide, N-methylpyrrolidinone, acetonitrile, acetone, 1,4-dioxane, tetrahydrofuran, alcohol, e.g. ethanol or isopropanol, water, or mixtures thereof, while not all of the solvents can be combined with each additive and base mentioned above. Suited reaction temperatures range from -20 to 140 °C.

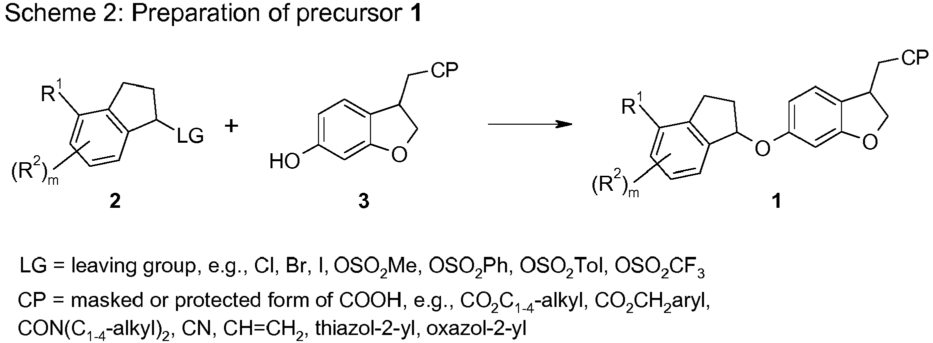

An alternative reaction to combine building blocks **2** and **3** is the Mitsunobu reaction or variations thereof (Scheme 3); R¹, R², and m in Scheme 3 have the meanings as defined hereinbefore and hereinafter. The reaction is usually conducted with a phosphine and an azodicarboxylic ester or amide in tetrahydrofuran, 1,4-dioxane, diethyl ether, toluene, benzene, dichloromethane, or mixtures thereof, at -30 to 100 °C. Phosphines often used are triphenylphosphine and tributylphosphine which are commonly combined with dimethyl azodicarboxylate, diethyl azodicarboxylate, diisopropyl azodicarboxylate, di-(4-chlorobenzyl) azodicarboxylate, dibenzyl azodicarboxylate, di-tert-butyl azodicarboxylate, azodicarboxylic acid bis-(dimethylamide), azodicarboxylic acid dipiperidide, or azodicarboxylic acid dimorpholide.

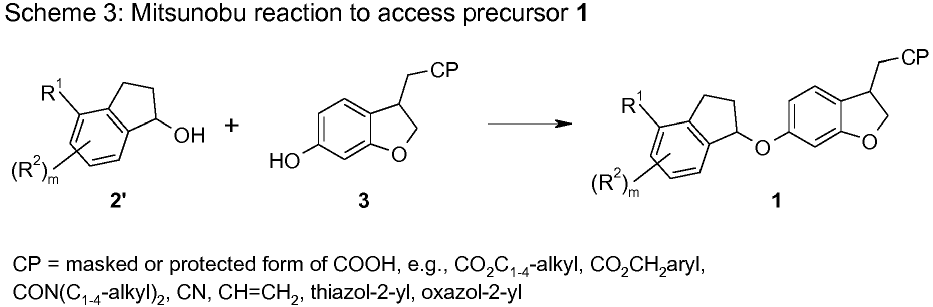

Intermediate **2**' is conveniently obtained from indanone **4** which, in turn, may be prepared from phenylpropionic acid derivative **5** (Scheme 4); R¹, R², and m in Scheme 4 have the meanings as defined hereinbefore and hereinafter. For the intramolecular acylation (Friedel-Crafts acylation), **5**→**4**, a considerable number of approaches has been reported. The reaction may be performed starting with a carboxylic acid, carboxylic ester, carboxylic anhydride, carboxylic chloride or fluoride, or a nitrile using a Lewis acid as catalyst. The following Lewis acids are some of the more often used ones: hydrobromic acid, hydroiodic acid, hydrochloric acid, sulfuric acid, phosphoric acid, P₄O₁₀, trifluoroacetic acid, methanesulfonic acid, toluenesulfonic acid, trifluoromethanesulfonic acid, ClSO₃H, Sc(OSO₂CF₃)₃, Tb(OSO₂CF₃)₃, SnCl₄, FeCl₃, AlBr₃, AlCl₃, SbCl₅, BCl₃, BF₃, ZnCl₂, montmorillonites, POCl₃, and PCl₅. The reaction may be conducted, e.g., in dichloromethane, 1,2-dichloroethane, nitrobenzene, chlorobenzene, carbon disulfide, mixtures thereof, or without an additional solvent in an excess of the Lewis acid, at 0 to 180 °C. Carboxylic acids are preferably reacted in polyphosphoric acid at 0 to 120 °C, while carboxylic chlorides are preferably reacted with AlCl₃ in dichloromethane or 1,2-dichloroethane at 0 to 80 °C.
The subsequent reduction of the keto group in Scheme 4 is a standard transformation in organic synthesis, which may be accomplished with lithium borohydride, sodium borohydride, lithium aluminum hydride, or diisobutylaluminum hydride. While sodium borohydride is employed in aqueous or alcoholic solution at 0 to 60 °C, the other reducing agents mentioned are preferably used in inert solvents, such as tetrahydrofuran, diethyl ether, dichloromethane, and toluene, at -80 to 60 °C.
The reduction of the keto group may also be conducted in a stereoselective fashion providing the alcohol in enantiomerically enriched or pure form. Suited chiral reducing agents are boranes combined with an enantiomerically pure [1,3,2]oxazaborol (Corey-Bakshi-Shibata reaction or Corey-Itsuno reaction) or formic acid, formates, hydrogen, or silanes in the presence of an enantiomerically pure transition metal catalyst. Typical reaction conditions for the former approach are borane (complexed with, e.g., dimethyl sulfide) and (*R*)- or (*S*)-3,3-diphenyl-1-methyltetrahydro-1 H,3H-pyrrolo[1,2-c][1,3,2]oxazaborol in, e.g., dichloromethane, toluene, methanol, tetrahydrofuran, or mixtures thereof, at 0 to 60 °C. Using a chiral transition metal catalyst, such as a ruthenium complex, e.g. chloro{[(1S,2S)-(-)-2-amino-1,2-diphenylethyl](4-toluenesulfonyl)-amido}-(mesitylene)ruthenium(II), may deliver the hydroxy compound with high enantiomeric excess using, e.g., formic acid in the presence of a base, e.g. triethylamine, in dichloromethane, at -20 to 60 °C. 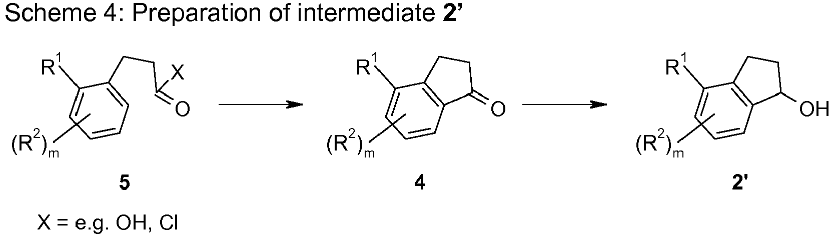 Alternatively, indanone **4** can be synthesized as described in Scheme 5; R¹, R², and m have the meanings as defined hereinbefore and hereinafter. Starting with benzene **6** and 3-halo-propionic acid or a derivative thereof or acrylic acid or a derivative thereof the required indanone **4** may be obtained via the combination of a Friedel-Crafts alkylation and acylation reaction in one pot or two separate reactions (eq. 1.)).
These reactions are catalyzed by a Lewis acid, such as triflic acid, sulfuric acid, phosphoric acid, AlCl₃, ZnCl₂, and phosphorus pentoxide, and preferably conducted without additional solvent in an excess of the Lewis acid or in dichloromethane, 1,2-dichloroethane, cyclohexane, or carbon disulfide, at 0 to 140 °C. A preferred combination comprises compound **6,** 3-chloro-propionyl chloride, and AlCl₃ in dichloromethane or 1,2-dichlorethane at 20 to 80 °C.
Starting with ethynylbenzene **7** indanone **4** is accessible by a transition metal catalyzed reaction with carbon monoxide (eq. 2.)). Rhodium is a preferred catalyst basis which is combined with a phosphine, e.g. triphenylphosphine, and a base, e.g. triethylamine, and used in a solvent, preferably tetrahydrofuran, at high carbon monoxide pressure, preferably 50 to 150 bar, at 150 to 200 °C (see e.g. J. Org. Chem. 1993, 58, 5386-92).
Combination of 2-halo or pseudo-halo substituted styrene **8** and carbon monoxide in the presence of a transition metal also allows the preparation of indanone **4** (eq. 3.)). Palladium catalysts are preferred and used with carbon monoxide or molybdenum hexacarbonyl as carbon monoxide source. Preferred solvents are N,N-dimethylformamide, N,N-dimethylacetamide, N-methylpyrrolidone, and 1,4-dioxane which are preferably employed at 20 to 150 °C by conventional heating or microwave irradiation. Pyridine and tetrabutylammonium chloride are preferred additives for this transformation (see e.g. J. Am. Chem. Soc. 2003, 125, 4804-7 and J. Org. Chem. 2005, 70, 346-9).

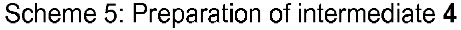

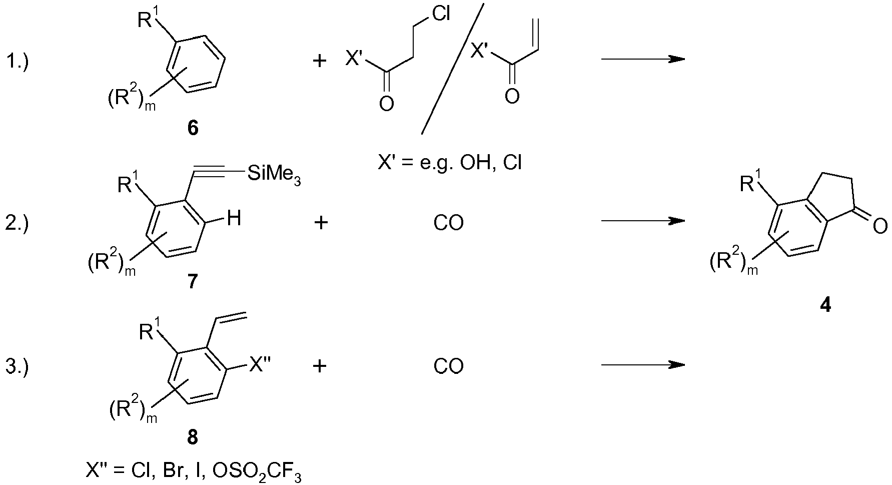

Common synthetic routes to building block **3** are summarized in Scheme 6. 2-lodo or bromo ether **9** can be transformed into indane **3** via addition of an *in situ* generated carbon anion or radical to the double bond and subsequent trapping of the cyclic anion by a proton and the cyclic radical by a hydride source (eq. 1.)). nBuLi, iPrMgCl, iPrMgCl*LiCl, Mg, Mg*LiCl, Zn, and Zn*LiCl are preferred reagents to convert the C-I or C-Br bond into a C-M bond (M = Li, Mgl, Znl etc.) with sufficient nucleophilicity to add to the double bond. The reactions with lithium and magnesium reagents are preferably conducted in hexanes, tetrahydrofuran, diethyl ether, 1,2-dimethoxyethane, toluene, or mixtures thereof, at -100 to 60 °C. Zn is preferably used in tetrahydrofuran, dimethyl sulfoxide, N-methylpyrrolidinone, or mixtures thereof, at 0 to 100 °C. Frequently employed reaction conditions for the radical pathway are, e.g., tributyltin hydride, azobisisobutyronitrile, in benzene, at 60 to 100 °C (see e.g. J. Org. Chem. 1987, 52, 4072-8); and NaH₃B(CN) in N,N-dimethylformamide under UV irradiation at 20 to 120 °C (see e.g. *Synlett* **2005,** 2248-50).
Combination of allyl ether **10** with carbon monoxide is another possibility to obtain indane **3** (equation 2.)). The reaction is preferably conducted with a palladium catalyst in the presence of carbon monoxide or molybdenum hexacarbonyl as carbon monoxide source (see e.g. Tetrahedron Lett. 2010, 51, 2102-5).
Starting from benzofuran **11** or dihydrobenzofuran **12** indane **3** is yielded after reduction of the double bond. Hydrogen is the preferred reducing agent which is mainly employed in combination with a transition metal catalyst, such as palladium on carbon, Raney nickel, and PtO₂. N,N-dimethylformamide, tetrahydrofuran, ethyl acetate, alcohol, e.g. methanol and ethanol, acetic acid, water, or mixtures thereof are preferably used as solvent, at hydrogen pressures of 1 to 100 bar, and temperatures of 20 to 120 °C. This reaction may also be carried out stereoselectively providing compound 3 in enantiomerically enriched or pure form.

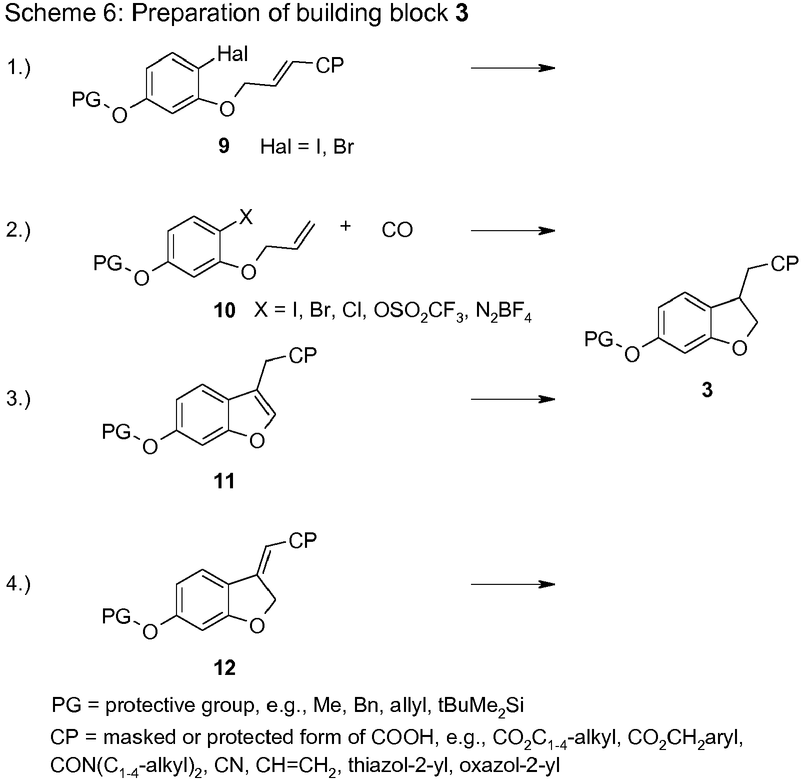

The syntheses of the starting compounds in Scheme 6 comprise standard procedures used in organic synthesis. Intermediate **11** can, for example, be prepared as described in Scheme **7.** Compound **11** may thus be obtained from compound **13** which, in turn, may be assembled from phenol **14** and ester **15.** The latter transformation may be achieved in the presence of a Lewis acid, e.g. sulfuric acid, ZrCl₄, InCl₃, methanesulfonic acid, p-toluenesulfonic acid, Hl, or amberlyst, in toluene, dichloromethane, acetic acid, ethanol, water, or without a solvent in an excess of the Lewis acid, at 0 to 120 °C. Transformation of compound **13** into intermediate **11** is preferably accomplished under basic conditions with sodium hydroxide in an aqueous solution at 0 to 100 °C.

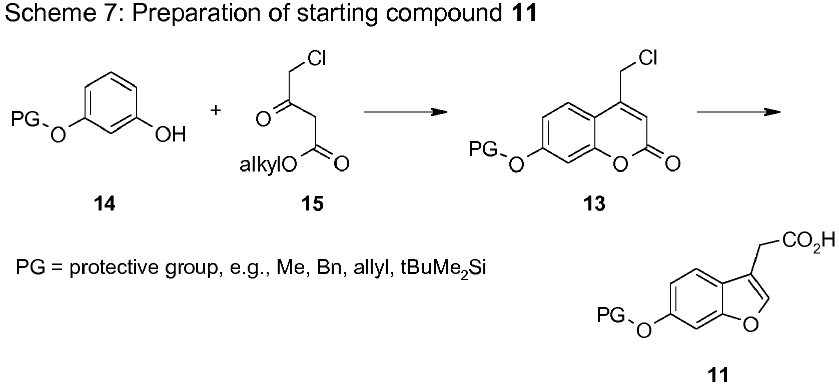

The synthetic routes presented may rely on the use of protecting groups. For example, potentially reactive groups present, such as hydroxy, carbonyl, carboxy, amino, alkylamino, or imino, may be protected during the reaction by conventional protecting groups which are cleaved again after the reaction. Suitable protecting groups for the respective functionalities and their removal are well known to the one skilled in the art and are described in the literature of organic synthesis.

The compounds of general formula I may be resolved into their enantiomers and/or diastereomers as mentioned below. Thus, for example, *cis*/*trans* mixtures may be resolved into their *cis* and *trans* isomers and racemic compounds may be separated into their enantiomers.

The *cis*/*trans* mixtures may be resolved, for example, by chromatography into the *cis* and *trans* isomers thereof. The compounds of general formula I which occur as racemates may be separated by methods known *per se* into their optical antipodes and diastereomeric mixtures of compounds of general formula I may be resolved into their diastereomers by taking advantage of their different physico-chemical properties using methods known *per se,* e.g. chromatography and/or fractional crystallization; if the compounds obtained thereafter are racemates, they may be resolved into the enantiomers as mentioned below.

The racemates are preferably resolved by column chromatography on chiral phases or by crystallization from an optically active solvent or by reacting with an optically active substance which forms salts or derivatives such as esters or amides with the racemic compound. Salts may be formed with enantiomerically pure acids for basic compounds and with enantiomerically pure bases for acidic compounds. Diastereomeric derivatives are formed with enantiomerically pure auxiliary compounds, e.g. acids, their activated derivatives, or alcohols. Separation of the diastereomeric mixture of salts or derivatives thus obtained may be achieved by taking advantage of their different physico-chemical properties, e.g. differences in solubility; the free antipodes may be released from the pure diastereomeric salts or derivatives by the action of suitable agents. Optically active acids commonly used for such a purpose as well as optically active alcohols applicable as auxiliary residues are known to those skilled in the art.

As mentioned above, the compounds of formula I may be converted into salts, particularly for pharmaceutical use into the pharmaceutically acceptable salts. As used herein, "pharmaceutically acceptable salts" refer to derivatives of the disclosed compounds wherein the parent compound is modified by making acid or base salts thereof.

The compounds according to the invention are advantageously also obtainable using the methods described in the examples that follow, which may also be combined for this purpose with methods known to the skilled man from the literature.

### Terms and definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

The terms "compound(s) according to this invention", "compound(s) of formula (I)", "compound(s) of the invention" and the like denote the compounds of the formula (I) according to the present invention including their tautomers, stereoisomers and mixtures thereof and the salts thereof, in particular the pharmaceutically acceptable salts thereof, and the solvates and hydrates of such compounds, including the solvates and hydrates of such tautomers, stereoisomers and salts thereof.

The terms "treatment" and "treating" embrace both preventative, i.e. prophylactic, or therapeutic, i.e. curative and/or palliative, treatment. Thus the terms "treatment" and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compounds and compositions of the present invention may be used for instance as therapeutic treatment over a period of time as well as for chronic therapy. In addition the terms "treatment" and "treating" comprise prophylactic treatment, i.e. a treatment of patients at risk to develop a condition mentioned hereinbefore, thus reducing said risk.

When this invention refers to patients requiring treatment, it relates primarily to treatment in mammals, in particular humans.

The term "therapeutically effective amount" means an amount of a compound of the present invention that (i) treats or prevents the particular disease or condition, (ii) attenuates, ameliorates, or eliminates one or more symptoms of the particular disease or condition, or (iii) prevents or delays the onset of one or more symptoms of the particular disease or condition described herein.

The terms "modulated" or "modulating", or "modulate(s)", as used herein, unless otherwise indicated, refer to the activation of the G-protein-coupled receptor GPR40 with one or more compounds of the present invention.

The terms "mediated" or "mediating" or "mediate", as used herein, unless otherwise indicated, refer to the (i) treatment, including prevention of the particular disease or condition, (ii) attenuation, amelioration, or elimination of one or more symptoms of the particular disease or condition, or (iii) prevention or delay of the onset of one or more symptoms of the particular disease or condition described herein.

The term "substituted" as used herein, means that any one or more hydrogens on the designated atom, radical or moiety is replaced with a selection from the indicated group, provided that the atom's normal valence is not exceeded, and that the substitution results in an acceptably stable compound.

In the groups, radicals, or moieties defined below, the number of carbon atoms is often specified preceding the group, for example, C₁-₆-alkyl means an alkyl group or radical having 1 to 6 carbon atoms. In general, for groups comprising two or more subgroups, the last named subgroup is the radical attachment point, for example, the substituent "aryl-C₁-₃-alkyl-" means an aryl group which is bound to a C₁-₃-alkyl- group, the latter of which is bound to the core or to the group to which the substituent is attached.

In case a compound of the present invention is depicted in form of a chemical name and as a formula in case of any discrepancy the formula shall prevail.

An asterisk may be used in sub-formulas to indicate the bond which is connected to the core molecule as defined.

The numeration of the atoms of a substituent starts with the atom which is closest to the core or to the group to which the substituent is attached.
For example, the term "3-carboxypropyl-group" represents the following substituent: 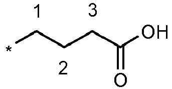 wherein the carboxy group is attached to the third carbon atom of the propyl group. The terms "1-methylpropyl-", "2,2-dimethylpropyl-" or "cyclopropylmethyl-" group represent the following groups:

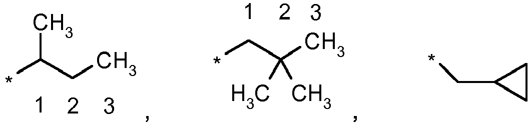

The asterisk may be used in sub-formulas to indicate the bond which is connected to the core molecule as defined.

In a definition of a group the term "wherein each X, Y and Z group is optionally substituted with" and the like denotes that each group X, each group Y and each group Z either each as a separate group or each as part of a composed group may be substituted as defined. For example a definition "R^{ex} denotes H, C₁₋₃-alkyl, C₃₋₆-cycloalkyl, C₃₋₆-cycloalkyl-C₁₋₃-alkyl or C₁₋₃-alkyl-O-, wherein each alkyl group is optionally substituted with one or more L^{ex}." or the like means that in each of the beforementioned groups which comprise the term alkyl, i.e. in each of the groups C₁₋₃-alkyl, C₃₋₆-cycloalkyl-C₁₋₃-alkyl and C₁₋₃-alkyl-O-, the alkyl moiety may be substituted with L^{eX} as defined.

Unless specifically indicated, throughout the specification and the appended claims, a given chemical formula or name shall encompass tautomers and all stereo-, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salts" refer to derivatives of the disclosed compounds wherein the parent compound is modified by making pharmaceutically acceptable acid or base salts thereof.

Salts of other acids than those mentioned above which for example are useful for purifying or isolating the compounds of the present invention (e.g. trifluoro acetate salts) also comprise a part of the invention.

The term halogen generally denotes fluorine, chlorine, bromine and iodine.

The term "C₁₋ₙ-alkyl", wherein n is an integer from 1 to n, either alone or in combination with another radical denotes an acyclic, saturated, branched or linear hydrocarbon radical with 1 to n C atoms. For example the term C₁₋₅-alkyl embraces the radicals H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-.

The term "C₁₋ₙ-alkylene" wherein n is an integer 1 to n, either alone or in combination with another radical, denotes an acyclic, straight or branched chain divalent alkyl radical containing from 1 to n carbon atoms. For example the term C₁₋₄-alkylene includes -(CH₂)-, -(CH₂-CH₂)-, -(CH(CH₃))-, -(CH₂-CH₂-CH₂)-, -(C(CH₃)₂)-, -(CH(CH₂CH₃))-, -(CH(CH₃)-CH₂)-, -(CH₂-CH(CH₃))-, -(CH₂-CH₂-CH₂-CH₂)-, -(CH₂-CH₂-CH(CH₃))-, -(CH(CH₃)-CH₂-CH₂)-, -(CH₂-CH(CH₃)-CH₂)-, -(CH₂-C(CH₃)₂)-, -(C (CH₃)₂-CH₂)-, -(CH(CH₃)-CH(CH₃))-, -(CH₂-CH(CH₂CH₃))-, -(CH(CH₂CH₃)-CH₂)- , -(CH(CH₂CH₂CH₃))- , -(CHCH(CH₃)₂)- and -C(CH₃)(CH₂CH₃)-.

The term "C₂₋ₙ-alkenyl", is used for a group as defined in the definition for "C₁₋ₙ-alkyl" with at least two carbon atoms, if at least two of those carbon atoms of said group are bonded to each other by a double bond. For example the term C₂₋₃-alkenyl includes -CH=CH₂, -CH=CH-CH₃, -CH₂-CH=CH₂.

The term "C₂₋ₙ-alkenylene" is used for a group as defined in the definition for "C₁₋ₙ-alkylene" with at least two carbon atoms, if at least two of those carbon atoms of said group are bonded to each other by a double bond. For example the term C₂₋₃-alkenylene includes -CH=CH-, -CH=CH-CH₂-, -CH₂-CH=CH-.

The term "C₂₋ₙ-alkynyl", is used for a group as defined in the definition for "C₁₋ₙ-alkyl" with at least two carbon atoms, if at least two of those carbon atoms of said group are bonded to each other by a triple bond. For example the term C₂₋₃-alkynyl includes -C≡CH, -C≡C-CH₃, -CH₂-C≡CH.

The term "C₂₋ₙ-alkynylene" is used for a group as defined in the definition for "C₁₋ₙ-alkylene" with at least two carbon atoms, if at least two of those carbon atoms of said group are bonded to each other by a triple bond. For example the term C₂₋₃-alkynylene includes -C≡C-, -C≡C-CH₂-, -CH₂-C≡C-.

The term "C₃₋ₙ-carbocyclyl" as used either alone or in combination with another radical, denotes a monocyclic, bicyclic or tricyclic, saturated or unsaturated hydrocarbon radical with 3 to n C atoms. The hydrocarbon radical is preferably nonaromatic. Preferably the 3 to n C atoms form one or two rings. In case of a bicyclic or tricyclic ring system the rings may be attached to each other via a single bond or may be fused or may form a spirocyclic or bridged ring system. For example the term C₃₋₁₀-carbocyclyl includes C₃₋₁₀-cylcoalkyl, C₃₋₁₀-cycloalkenyl, octahydropentalenyl, octahydroindenyl, decahydronaphthyl, indanyl, tetrahydronaphthyl. Most preferably the term C₃₋ₙ-carbocyclyl denotes C₃₋ₙ-cylcoalkyl, in particular C₃₋₇-cycloalkyl.

The term "C₃₋ₙ-cycloalkyl", wherein n is an integer 4 to n, either alone or in combination with another radical denotes a cyclic, saturated, unbranched hydrocarbon radical with 3 to n C atoms. The cyclic group may be mono-, bi-, tri- or spirocyclic, most preferably monocyclic. Examples of such cycloalkyl groups include cyclopropyl, cyclobutyl, cyclopentyl, cyclohexyl, cycloheptyl, cyclooctyl, cyclononyl, cyclododecyl, bicyclo[3.2.1.]octyl, spiro[4.5]decyl, norpinyl, norbonyl, norcaryl, adamantyl, etc.

The term "C₃₋ₙ-cycloalkenyl", wherein n is an integer 3 to n, either alone or in combination with another radical, denotes a cyclic, unsaturated but nonaromatic, unbranched hydrocarbon radical with 3 to n C atoms, at least two of which are bonded to each other by a double bond. For example the term C₃₋₇-cycloalkenyl includes cyclopropenyl, cyclobutenyl, cyclopentenyl, cyclopentadienyl, cyclohexenyl, cyclohexadienyl, cycloheptenyl, cycloheptadienyl and cycloheptatrienyl.

The term "heterocyclyl" is intended to include all the possible isomeric forms. Examples of such groups include azetidinyl, oxetanyl, pyrrolidinyl, tetrahydrofuranyl, piperidinyl, tetrahydropyranyl, azepanyl, piperazinyl, morpholinyl, tetrahydrofuranonyl, tetrahydropyranonyl, pyrrolidinonyl, piperidinonyl, piperazinonyl, morpholinonyl.

Unless otherwise specified, the term "heterocyclyl" includes the following exemplary structures which are not depicted as radicals as each form may be attached through a covalent bond to any atom so long as appropriate valences are maintained:

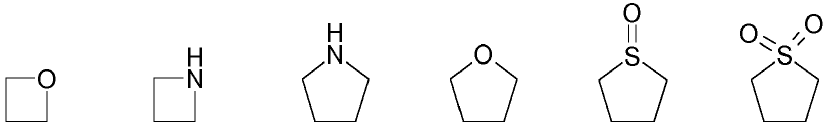

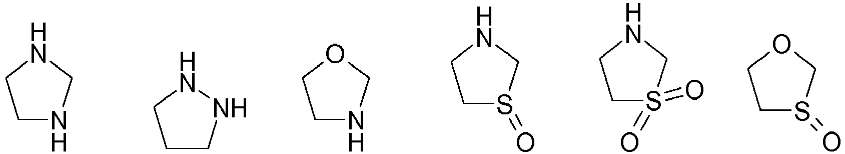

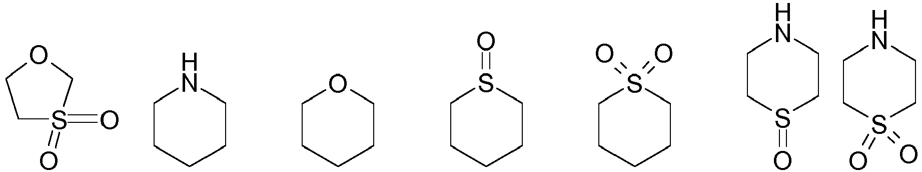

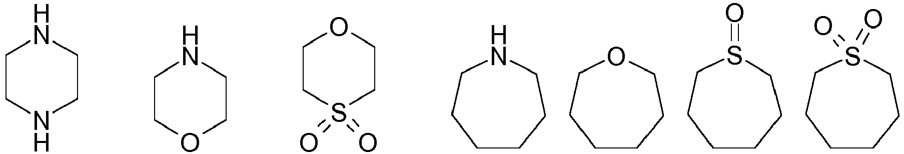

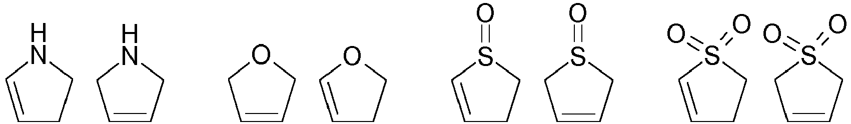

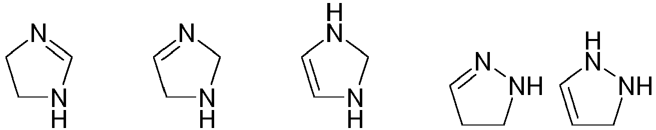

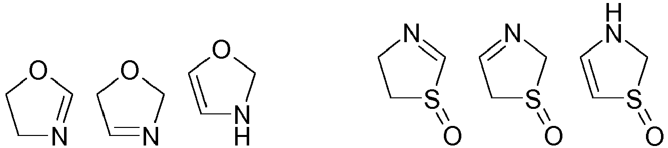

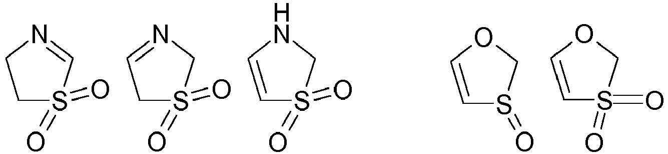

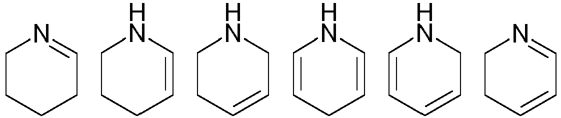

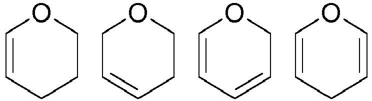

The term "heteroaryl" is intended to include all the possible isomeric forms.

Unless specified otherwise, the term "heteroaryl" includes the following exemplary structures which are not depicted as radicals as each form may be attached through a covalent bond to any atom so long as appropriate valences are maintained:

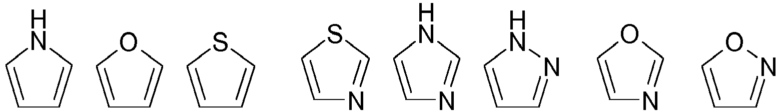

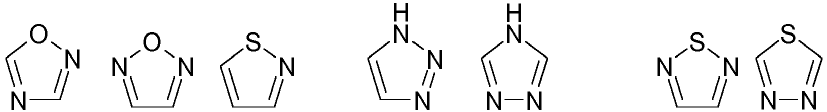

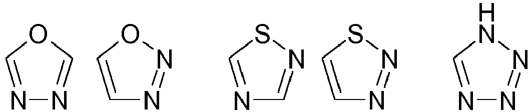

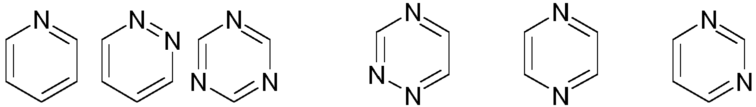

Many of the terms given above may be used repeatedly in the definition of a formula or group and in each case have one of the meanings given above, independently of one another.

### Pharmacological Activity

The activity of the compounds of the invention may be demonstrated using the following assays.

### Assay I:

IP₁ accumulation measurements using the IPOne assay system - 1321 N1 cells stably expressing human GPR40 receptor (Euroscreen, Belgium) are seeded 24 h before the assay in black clear-bottom collagen-coated 384-well plates in culture medium containing 10% FCS, 1% Na-Pyruvate and 400 µg/mL G418. IP₁ is assayed according to the Manufacturer's description (Cisbio Bioassays, France). In brief, the assay is started by substitution of the culture medium by stimulation buffer (Hepes 10 mM, CaCl₂ 1 mM, MgCl₂ 0.5 mM, KCI 4.2 mM, NaCl 146 mM and glucose 5.5 mM, pH 7.4) without LiCl. Cells are stimulated for 1 hour at 37 °C, 10% CO₂ by addition of the compounds that are diluted in stimulation buffer containing LiCl yielding a final LiCl concentration of 50 mM. Assays are stopped by adding HTRF-conjugates (IP1-d2 and Anti-IP1 cryptate Tb) and lysis buffer, provided by the manufacturer. After an incubation time of 1 hour at room temperature plates are measured using an EnVision™, Perkin Elmer. The obtained fluorescence ratios at 665/615 nM are then used to calculate the pEC₅₀ values using GraphPad Prism 5 (Graphpad Software Inc, USA) by interpolation using an IP₁ reference curve and subsequent sigmoidal curve fitting allowing for a variable hill slope.

The compounds according to the invention typically have EC₅₀ values in the range from about 1 nM to about 10 µM, preferably less than 1 µM, more preferably less than 100 nM.

EC₅₀ values for compounds according to the invention determined in Assay I are shown in the following table. The number of the compound corresponds to the number of the Example in the experimental section.

| Example | EC₅₀ [nM] | Example | EC₅₀ [nM] | Example | EC₅₀ [nM] | Example | EC₅₀ [nM] |
|---|---|---|---|---|---|---|---|
| 1 | 211 | 2 | 64 | 3 | 568 | 4 | 52 |
| 5 | 52 | 6 | 38 | 7 | 17 | 8 | 30 |
| 9 | 7 | 10 | 85 | 11 | 51 | 12 | 4 |
| 13 | 5 | 14 | 68 | 15 | 6 | 16 | 5 |
| 17 | 17 | 18 | 11 | 19 | 912 | 20 | 279 |
| 21 | 92 | 22 | 684 | 23 | 19 | 24 | 22 |
| 25 | 6 | 26 | 15 | 27 | 50 | 28 | 68 |
| 29 | 9 | 30 | 4 | 31 | 9 | 32 | 2 |
| 33 | 18 | 34 | 17 | 35 | 9 | 36 | 5 |
| 37 | 16 | 38 | 63 | 39 | 12 | 40 | 5 |
| 41 | 5 | 42 | 12 | 43 | 4 | 44 | 4 |
| 45 | 3 | 46 | 9 | 47 | 8 | 48 | 17 |
| 49 | 10 | 50 | 3 | 51 | 35 | 52 | 17 |
| 53 | 9 | 54 | 6 | 55 | 11 | 56 | 7 |
| 57 | 17 | 58 | 19 | 59 | 12 | 60 | 11 |
| 61 | 57 | 62 | 52 | 63 | 26 | 64 | 9 |
| 65 | 58 | 66 | 36 | 67 | 35 | 68 | 1514 |
| 69 | 2917 | 70 | 36 | 71 | 20 | 72 | 23 |
| 73 | 81 | 74 | 43 | 75 | 9 | 76 | 211 |
| 77 | 15 | 78 | 6 | 79 | 5 | 80 | 17 |
| 81 | 17 | 82 | 51 | 83 | 25 | 84 | 118 |
| 85 | 15 | 86 | 26 | 87 | 27 | 88 | 355 |
| 89 | 23 | 90 | 117 | 91 | 26 | 92 | 51 |
| 93 | 11 | 94 | 38 | 62s | 1223 | 62c | 76 |
| 62d | 8 | 98 | 34 | 190 | 8 | 193 | 2 |
| 197 | 3 | | | | | | |

### Assay II:

IP₁ accumulation measurements using the IPOne assay system - 1321 N1 cells stably expressing human GPR40 receptor (Euroscreen, Belgium) are seeded 24 h before the assay in white 384-well plates in culture medium containing 10% FCS, 1% Na-Pyruvate and 400 µg/mL G418. IP₁ is assayed according to the manufacturer's description (Cisbio Bioassays, France). In brief, the assay is started by substitution of the culture medium by stimulation buffer (Hepes 10 mM, CaCl₂ 1 mM, MgCl₂ 0.5 mM, KCI 4.2 mM, NaCl 146 mM, glucose 5.5 mM and LiCl 50 mM, pH 7.4). Cells are stimulated for 1 h at 37 °C, 5% CO₂ by addition of the compounds that are diluted in stimulation buffer containing LiCl. Assays are stopped by adding HTRF-conjugates (IP1-d2 and Anti-IP1 cryptate Tb) and lysis buffer, provided by the manufacturer. After an incubation time of 1 h at room temperature plates are measured using an EnVision™, Perkin Elmer. The obtained fluorescence ratios at 665/615 nM are then used to calculate the pEC₅₀ values using Assay Explorer 3.3 Software (Accelrys, Inc.) by interpolation using an IP₁ reference curve and subsequent sigmoidal curve fitting allowing for a variable hill slope.

The compounds according to the invention typically have EC₅₀ values in the range from about 1 nM to about 10 µM, preferably less than 1 µM, more preferably less than 100 nM.

EC₅₀ values for compounds according to the invention determined in Assay II are shown in the following table. The number of the compound corresponds to the number of the Example in the experimental section.

| Example | EC₅₀ [nM] | Example | EC₅₀ [nM] | Example | EC₅₀ [nM] | Example | EC₅₀ [nM] |
|---|---|---|---|---|---|---|---|
| 48a | 93 | 48b | 19 | 48c | 117 | 62a | 8 |
| 62b | 6 | 62e | 13 | 62f | 18 | 62g | 11 |
| 62h | 22 | 62i | 10 | 62j | 17 | 62k | 9 |
| 62l | 7 | 62m | 8 | 62n | 14 | 62o | 10 |
| 62p | 20 | 62q | 7 | 62t | 13 | 62u | 25 |
| 62w | 43 | 62aa | 21 | 62ab | 21 | 62ac | 6 |
| 62ad | 12 | 62ae | 13 | 62af | 6 | 62ag | 43 |
| 62ah | 13 | 62ai | 14 | 62aj | 27 | 62ak | 14 |
| 62al | 129 | 62am | 5 | 62an | 3 | 62ao | 7 |
| 62ap | 2 | 62aq | 3 | 62ar | 7 | 62as | 23 |
| 62at | 5 | 62au | 17 | 62aw | 6 | 62ax | 6 |
| 62ay | 19 | 62az | 29 | 62ba | 6 | 62bb | 29 |
| 62bc | 4 | 62be | 7 | 62bf | 5 | 62bg | 4 |
| 62bh | 6 | 62bi | 13 | 62bj | 8 | 62bk | 9 |
| 62bl | 21 | 62bm | 22 | 62bn | 23 | 62bo | 79 |
| 62bp | 22 | 62bq | 37 | 62br | 30 | 62bs | 50 |
| 62bt | 14 | 62bu | 9 | 62bw | 15 | 62bx | 19 |
| 62by | 5 | 62bz | 8 | 62ca | 10 | 62cb | 8 |
| 62cc | 17 | 62cd | 31 | 62ce | 29 | 62cf | 4 |
| 62cg | 12 | 62ch | 7 | 62ci | 8 | 62cj | 5 |
| 62ck | 6 | 62cl | 4 | 62cm | 8 | 62cn | 17 |
| 62co | 17 | 62cp | 9 | 62cq | 6 | 62cr | 2 |
| 62cs | 3 | 62ct | 4 | 62cu | 9 | 62cw | 5 |
| 62cx | 6 | 62cy | 19 | 62cz | 7 | 62da | 18 |
| 62db | 15 | 62dc | 7 | 62dd | 4 | 62de | 5 |
| 62df | 7 | 62dg | 5 | 62dh | 6 | 62di | 12 |
| 62dj | 11 | 62dk | 13 | 62dl | 5 | 62dm | 4 |
| 62dn | 5 | 62do | 5 | 62dp | 25 | 62dq | 9 |
| 62dr | 5 | 62ds | 5 | 62dt | 20 | 62du | 34 |
| 62dw | 5 | 62dx | 5 | 62dy | 8 | 62dz | 6 |
| 62ea | 9 | 62eb | 18 | 62ec | 13 | 62ed | 14 |
| 62ee | 11 | 62ef | 5 | 62eg | 10 | 62eh | 7 |
| 62ei | 8 | 62ej | 4 | 62ek | 8 | 95 | 8 |
| 96 | 10 | 97 | 37 | 99 | 36 | 100 | 5 |
| 101 | 7 | 102 | 10 | 103 | 8 | 104 | 60 |
| 105 | 28 | 106 | 23 | 107 | 13 | 108 | 40 |
| 109 | 17 | 110 | 27 | 111 | 10 | 112 | 4 |
| 113 | 5 | 114 | 137 | 115 | 18 | 116 | 7 |
| 117 | 11 | 118 | 8 | 119 | 82 | 120 | 12 |
| 121 | 7 | 122 | 14 | 123 | 6 | 124 | 9 |
| 125 | 7 | 126 | 8 | 127 | 24 | 128 | 3 |
| 129 | 9 | 130 | 45 | 131 | 3 | 132 | 9 |
| 133 | 5 | 134 | 3 | 135 | 5 | 136 | 4 |
| 137 | 82 | 138 | 3 | 139 | 20 | 140 | 5 |
| 141 | 3 | 142 | 4 | 143 | 21 | 144 | 279 |
| 145 | 58 | 146 | 3 | 147 | 2239 | 148 | 1429 |
| 149 | 7 | 150 | 582 | 151 | 59 | 152 | 4 |
| 154 | 8 | 155 | 351 | 156 | 4 | 157 | 5 |
| 158 | 1 | 159 | 2 | 160 | 2 | 161 | 3 |
| 162 | 2 | 163 | 2 | 164 | 1 | 165 | 2 |
| 166 | 2 | 167 | 2 | 168 | 7 | 169 | 339 |
| 170 | 4 | 171 | 6 | 172 | 48 | 173 | 288 |
| 174 | 4 | 175 | 5 | 176 | 2 | 177 | 2 |
| 178 | 2 | 179 | 3 | 180 | 7 | 181 | 3 |
| 182 | 2 | 183 | 9 | 184 | 2 | 185 | 2 |
| 186 | 3 | 187 | 16 | 188 | 3 | 189 | 17 |
| 191 | 16 | 192 | 7 | 194 | 13 | 195 | 12 |
| 196 | 6 | 198 | 19 | | | | |

In view of their ability to modulate the activity of the G-protein-coupled receptor GPR40, in particular an agonistic activity, the compounds of general formula I according to the invention, including the corresponding salts thereof, are theoretically suitable for the treatment of all those diseases or conditions which may be affected or which are mediated by the activation of the G-protein-coupled receptor GPR40.

Accordingly, the present invention relates to a compound of general formula I as a medicament.

Furthermore, the present invention relates to the use of a compound of general formula **I** or a pharmaceutical composition according to this invention for the treatment and/or prevention of metabolic diseases or conditions associated with the disease in a patient, preferably in a human.

According to one aspect the compounds and pharmaceutical compositions of the present invention are particularly suitable for treating diabetes mellitus, in particular Type 2 diabetes, Type 1 diabetes, complications of diabetes (such as e.g. retinopathy, nephropathy or neuropathies, diabetic foot, ulcers or macroangiopathies), metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

The compounds and pharmaceutical compositions of the present invention are also suitable for preventing beta-cell degeneration such as e.g. apoptosis or necrosis of pancreatic beta cells. The compounds and pharmaceutical compositions of the present invention are also suitable for improving or restoring the functionality of pancreatic cells, and also for increasing the number and size of pancreatic beta cells.

Therefore according to another aspect the invention relates to compounds of formula I and pharmaceutical compositions according to the invention for use in preventing, delaying, slowing the progression of and/or treating metabolic diseases, particularly in improving the glycaemic control and/or beta cell function in the patient.

In another aspect the invention relates to compounds of formula I and pharmaceutical compositions according to the invention for use in preventing, delaying, slowing the progression of and/or treating type 2 diabetes, overweight, obesity, complications of diabetes and associated pathological conditions.

In addition the compounds and pharmaceutical compositions according to the invention are suitable for use in one or more of the following therapeutic processes:
- for preventing, delaying, slowing the progression of or treating metabolic diseases, such as for example type 1 diabetes, type 2 diabetes, insufficient glucose tolerance, insulin resistance, hyperglycaemia, hyperlipidaemia, hypercholesterolaemia, dyslipidaemia, syndrome X, metabolic syndrome, obesity, high blood pressure, chronic systemic inflammation, retinopathy, neuropathy, nephropathy, atherosclerosis, endothelial dysfunction or bone-related diseases (such as osteoporosis, rheumatoid arthritis or osteoarthritis);
- for improving glycaemic control and/or reducing fasting plasma glucose, postprandial plasma glucose and/or the glycosylated haemoglobin HbA1c;
- for preventing, delaying, slowing or reversing the progression of disrupted glucose tolerance, insulin resistance and/or metabolic syndrome to type 2 diabetes;
- for preventing, delaying, slowing the progression of or treating a condition or a disease selected from among the complications of diabetes, such as for example retinopathy, nephropathy or neuropathies, diabetic foot, ulcers or macroangiopathies;
- for reducing weight or preventing weight gain or assisting weight loss;
- for preventing or treating the degradation of pancreatic beta cells and/or improving and/or restoring the functionality of pancreatic beta cells and/or restoring the functionality of pancreatic insulin secretion;
- for maintaining and/or improving insulin sensitivity and/or preventing or treating hyperinsulinaemia and/or insulin resistance.

In particular, the compounds and pharmaceutical compositions according to the invention are suitable for the treatment of obesity, diabetes (comprising type 1 and type 2 diabetes, preferably type 2 diabetes mellitus) and/or complications of diabetes (such as for example retinopathy, nephropathy or neuropathies, diabetic foot, ulcers or macroangiopathies).

The compounds according to the invention are most particularly suitable for treating type 2 diabetes mellitus.

The dose range of the compounds of general formula I applicable per day is usually from 0.001 to 10 mg per kg body weight, for example from 0.01 to 8 mg per kg body weight of the patient. Each dosage unit may conveniently contain from 0.1 to 1000 mg, for example 0.5 to 500 mg.

The actual therapeutically effective amount or therapeutic dosage will of course depend on factors known by those skilled in the art such as age and weight of the patient, route of administration and severity of disease. In any case the compound or composition will be administered at dosages and in a manner which allows a therapeutically effective amount to be delivered based upon patient's unique condition.

The compounds, compositions, including any combinations with one or more additional therapeutic agents, according to the invention may be administered by oral, transdermal, inhalative, parenteral or sublingual route. Of the possible methods of administration, oral or intravenous administration is preferred.

### Pharmaceutical Compositions

Suitable preparations for administering the compounds of formula I, optionally in combination with one or more further therapeutic agents, will be apparent to those with ordinary skill in the art and include for example tablets, pills, capsules, suppositories, lozenges, troches, solutions, syrups, elixirs, sachets, injectables, inhalatives and powders etc. Oral formulations, particularly solid forms such as e.g. tablets or capsules are preferred. The content of the pharmaceutically active compound(s) is advantageously in the range from 0.1 to 90 wt.-%, for example from 1 to 70 wt.-% of the composition as a whole.

Suitable tablets may be obtained, for example, by mixing one or more compounds according to formula **I** with known excipients, for example inert diluents, carriers, disintegrants, adjuvants, surfactants, binders and/or lubricants. The tablets may also consist of several layers. The particular excipients, carriers and/or diluents that are suitable for the desired preparations will be familiar to the skilled man on the basis of his specialist knowledge. The preferred ones are those that are suitable for the particular formulation and method of administration that are desired. The preparations or formulations according to the invention may be prepared using methods known *per se* that are familiar to the skilled man, such as for example by mixing or combining at least one compound of formula I according to the invention, or a pharmaceutically acceptable salt of such a compound, and one or more excipients, carriers and/or diluents.

### Combination Therapy

The compounds of the invention may further be combined with one or more, preferably one additional therapeutic agent. According to one embodiment the additional therapeutic agent is selected from the group of therapeutic agents useful in the treatment of diseases or conditions described hereinbefore, in particular associated with metabolic diseases or conditions such as for example diabetes mellitus, obesity, diabetic complications, hypertension, hyperlipidemia. Additional therapeutic agents which are suitable for such combinations include in particular those which for example potentiate the therapeutic effect of one or more active substances with respect to one of the indications mentioned and/or which allow the dosage of one or more active substances to be reduced.

Therefore a compound of the invention may be combined with one or more additional therapeutic agents selected from the group consisting of antidiabetic agents, agents for the treatment of overweight and/or obesity and agents for the treatment of high blood pressure, heart failure and/or atherosclerosis.

Antidiabetic agents are for example metformin, sulphonylureas, nateglinide, repaglinide, thiazolidinediones, PPAR-(alpha, gamma or alpha/gamma) agonists or modulators, alpha-glucosidase inhibitors, DPPIV inhibitors, SGLT2-inhibitors, insulin and insulin analogues, GLP-1 and GLP-1 analogues or amylin and amylin analogues, cycloset, 11β-HSD inhibitors. Other suitable combination partners are inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase, alpha2-antagonists, CCR-2 antagonists or glucokinase activators. One or more lipid lowering agents are also suitable as combination partners, such as for example HMG-CoA-reductase inhibitors, fibrates, nicotinic acid and the derivatives thereof, PPAR-(alpha, gamma or alpha/gamma) agonists or modulators, PPAR-delta agonists, ACAT inhibitors or cholesterol absorption inhibitors such as, bile acid-binding substances such as, inhibitors of ileac bile acid transport, MTP inhibitors, or HDL-raising compounds such as CETP inhibitors or ABC1 regulators.

Therapeutic agents for the treatment of overweight and/or obesity are for example antagonists of the cannabinoid1 receptor, MCH-1 receptor antagonists, MC4 receptor agonists, NPY5 or NPY2 antagonists, β3-agonists, leptin or leptin mimetics, agonists of the 5HT2c receptor.

Therapeutic agents for the treatment of high blood pressure, chronic heart failure and/or atherosclerosis are for example A-II antagonists or ACE inhibitors, ECE inhibitors, diuretics, β-blockers, Ca-antagonists, centrally acting antihypertensives, antagonists of the alpha-2-adrenergic receptor, inhibitors of neutral endopeptidase, thrombocyte aggregation inhibitors and others or combinations thereof are suitable. Angiotensin II receptor antagonists are preferably used for the treatment or prevention of high blood pressure and complications of diabetes, often combined with a diuretic such as hydrochlorothiazide.

The dosage for the combination partners mentioned above is usually 1/5 of the lowest dose normally recommended up to 1/1 of the normally recommended dose.

Preferably, compounds of the present invention and/or pharmaceutical compositions comprising a compound of the present invention optionally in combination with one or more additional therapeutic agents are administered in conjunction with exercise and/or a diet.

Therefore, in another aspect, this invention relates to a compound according to the invention in combination with one or more additional therapeutic agents described hereinbefore and hereinafter for use in the treatment of metabolic diseases or conditions associated with the disease, in particular diseases or conditions as described hereinbefore and hereinafter.

The use of the compound according to the invention in combination with the additional therapeutic agent may take place simultaneously or at staggered times.

The compound according to the invention and the one or more additional therapeutic agents may both be present together in one formulation, for example a tablet or capsule, or separately in two identical or different formulations, for example as a so-called kit-of-parts.

Consequently, in another aspect, this invention relates to a pharmaceutical composition which comprises a compound according to the invention and one or more additional therapeutic agents described hereinbefore and hereinafter, optionally together with one or more inert carriers and/or diluents.

Other features and advantages of the present invention will become apparent from the following more detailed Examples which illustrate, by way of example, the principles of the invention.

### Examples

Reference examples have been marked with an asterisk (*).

The terms "ambient temperature" and "room temperature" are used interchangeably and designate a temperature of about 20 °C.

### Preliminary remarks:

As a rule, ¹H-NMR and/or mass spectra have been obtained for the compounds prepared. The R_{f} values are determined using Merck silica gel 60 F₂₅₄ plates and UV light at 254 nm.

Analytical HPLC parameters employed for characterization of products (TFA denotes trifluoroacetic acid):

| | | | | |
|---|---|---|---|---|
| Method: | | 1 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [Methanol] | Flow [mL/min] | Temperature [°C] |
|---|---|---|---|---|
| 0.0 | 95 | 5 | 2.2 | 60 |
| 0.05 | 95 | 5 | 2.2 | 60 |
| 1.40 | 0 | 100 | 2.2 | 60 |
| 1.80 | 0 | 100 | 2.2 | 60 |

| | | | | |
|---|---|---|---|---|
| Method: | | 2 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [Methanol] | Flow [mL/min] | Temperature [°C] |
|---|---|---|---|---|
| 0.0 | 50 | 50 | 2.2 | 60 |
| 0.05 | 50 | 50 | 2.2 | 60 |
| 1.40 | 0 | 100 | 2.2 | 60 |
| 1.80 | 0 | 100 | 2.2 | 60 |

| | | | | |
|---|---|---|---|---|
| Method: | | 3 | | |
| Device: | | Agilent 1100 with DA and MS detector | | |
| Column: | | XBridge C18, 4,6 x 30 mm, 3.5 µm | | |
| Column Supplier: | | Waters | | |

| Gradient/Solvent Time [min] | % Solvent [H₂O,0.1 %HCOOH] | % Solvent [Methanol] | Flow [mL/min] | Temperature [°C] |
|---|---|---|---|---|
| 0.0 | 50 | 50 | 4 | 60 |
| 0.15 | 50 | 50 | 4 | 60 |
| 1.7 | 0 | 100 | 4 | 60 |
| 2.25 | 0 | 100 | 4 | 60 |

| | | | | |
|---|---|---|---|---|
| Method: | | 4 | | |
| Device: | | Agilent 1100 with DA and MS detector | | |
| Column: | | XBridge C18, 4,6 x 30 mm, 3.5 µm | | |
| Column Supplier: | | Waters | | |

| Gradient/Solvent Time [min] | % Solvent [H₂O,0.1%HCOOH] | % Solvent [Methanol] | Flow [mL/min] | Temperature [°C] |
|---|---|---|---|---|
| 0.0 | 95 | 5 | 4 | 60 |
| 0.15 | 95 | 5 | 4 | 60 |
| 1.7 | 0 | 100 | 4 | 60 |
| 2.25 | 0 | 100 | 4 | 60 |

| | | | | |
|---|---|---|---|---|
| Method: | | 5 | | |
| Device: | | Agilent 1100 with DA and MS detector | | |
| Column: | | XBridge C18, 4,6 x 30 mm, 3.5 µm | | |
| Column Supplier: | | Waters | | |

| Gradient/Solvent Time [min] | % Solvent [H₂O,0.1%NH₃] | % Solvent [Methanol] | Flow [mL/min] | Temperature [°C] |
|---|---|---|---|---|
| 0.0 | 50 | 50 | 4 | 60 |
| 0.15 | 50 | 50 | 4 | 60 |
| 1.7 | 0 | 100 | 4 | 60 |
| 2.25 | 0 | 100 | 4 | 60 |

| | | | | |
|---|---|---|---|---|
| Method: | | 6 | | |
| Device: | | Agilent 1100 with DA and MS detector | | |
| Column: | | XBridge C18, 4,6 x 30 mm, 3.5 µm | | |
| Column Supplier: | | Waters | | |

| Gradient/Solvent Time [min] | % Solvent [H₂O,0.1%NH₃] | % Solvent [Methanol] | Flow [mL/min] | Temperature [°C] |
|---|---|---|---|---|
| 0.0 | 95 | 5 | 4 | 60 |
| 0.15 | 95 | 5 | 4 | 60 |
| 1.7 | 0 | 100 | 4 | 60 |
| 2.25 | 0 | 100 | 4 | 60 |

| | | | | |
|---|---|---|---|---|
| Method: | | 7 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 8 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 50 | 50 | 2.2 | 60 |
| 0.20 | 50 | 50 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 9 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 10 | | |
| Device: | | Agilent 1200 with DA and MS detector | | |
| Column: | | Stable Bond, 3 x 30 mm, 1.8 µm | | |
| Column Supplier: | | Agilent | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 11 | | |
| Device: | | Agilent 1200 with DA and MS detector | | |
| Column: | | Sunfire, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 12 | | |
| Device: | | Agilent 1200 with DA and MS detector | | |
| Column: | | Stable Bond, 3 x 30 mm, 1.8 µm | | |
| Column Supplier: | | Agilent | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 50 | 50 | 2.2 | 60 |
| 0.20 | 50 | 50 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 13 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%NH₃] | % Solvent [CH₃CN] | Flow [mL/min] | Temperature [°C] |
|---|---|---|---|---|
| 0.00 | 50 | 50 | 2.2 | 60 |
| 0.20 | 50 | 50 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 14 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%NH₃] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 15 | | |
| Device: | | Agilent 1200 with DA and MS detector | | |
| Column: | | Sunfire C18, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 97 | 3 | 2.2 | 60 |
| 0.20 | 97 | 3 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 16 | | |
| Device: | | LC/MS Waters Alliance 2695 HPLC System DAD, Quattro Micro Triple quadrupole | | |
| Column: | | Gemini, C18 3 µm, 4.6 x 50 mm | | |

| Gradient/Solvent Time [min] | % Solvent [H₂O/CH₃CN 90:10,0.1 %TFA] | % Solvent [CH₃CN] | Flow [mL/min] | Temperature [°C] |
|---|---|---|---|---|
| 0.00 | 50 | 50 | 1.3 | 35 |
| 3.5 | 10 | 90 | 1.3 | 35 |
| 4.5 | 10 | 90 | 1.3 | 35 |
| 4.6 | 50 | 50 | 1.3 | 35 |

| | | | | |
|---|---|---|---|---|
| Method: | | 17 | | |
| Device: | | LC/MS Waters Acquity UPLC System DAD, SQD single quadrupole | | |
| Column: | | BEH C18, 1.7 µm, 2.1 x 50 mm | | |

| Gradient/Solvent Time [min] | % Solvent [H₂O/CH₃CN 90:10, 5 mM NH₄COOH] | % Solvent [CH₃CN/H₂O 90:10] | Flow [mL/min] | Temperature [°C] |
|---|---|---|---|---|
| 0.00 | 100 | 0 | 0.70 | 35 |
| 1.20 | 0 | 100 | 0.70 | 35 |
| 1.45 | 0 | 100 | 0.70 | 35 |
| 1.55 | 100 | 0 | 0.70 | 35 |
| 1.75 | 100 | 0 | 0.70 | 35 |

| | | | | |
|---|---|---|---|---|
| Method: | | 18 | | |
| Device: | | LC/MS Waters Acquity UPLC System DAD, SQD single quadrupole | | |
| Column: | | HSS C18, 1.8 µm, 2.1 x 50 mm | | |

| GradientJSolvent Time [min] | % Solvent [H₂O/CH₃CN 90:10,0.1%TFA] | % Solvent [CH₃CN/H₂O 90:10] | Flow [mL/min] | Temperature [°C] |
|---|---|---|---|---|
| 0.00 | 100 | 0 | 0.70 | 35 |
| 0.70 | 0 | 100 | 0.70 | 35 |
| 2.30 | 0 | 100 | 0.70 | 35 |
| 2.40 | 100 | 0 | 0.70 | 35 |
| 2.60 | 100 | 0 | 0.70 | 35 |

| | | | | |
|---|---|---|---|---|
| Method: | | 19 | | |
| Device: | | LC/MS Waters Acquity UPLC System DAD, SQD single quadrupole | | |
| Column: | | BEH C18, 1.7 µm, 2.1 x 50 mm | | |

| Gradient/Solvent Time [min] | % Solvent [H₂O/CH₃CN 90:10, 5 mM NH₄COOH] | % Solvent [CH₃CN/H₂O 90:10] | Flow [mL/min] | Temperature [°C] |
|---|---|---|---|---|
| 0.00 | 100 | 0 | 0.70 | 35 |
| 1.20 | 0 | 100 | 0.70 | 35 |
| 1.45 | 0 | 100 | 0.70 | 35 |
| 1.55 | 100 | 0 | 0.70 | 35 |
| 1.75 | 100 | 0 | 0.70 | 35 |

| | | | |
|---|---|---|---|
| Method: | | 20 | |
| Device: | | LC/MS ThermoFinnigan HPLC Surveyor DAD, MSQ single quadrupole | |
| Column: | | Synergi Hydro RP100A, 2.5 µm, 3 x 50 mm | |

| Gradient/Solvent Time [min] | % Solvent [H₂O/CH₃CN 90:10, 10 mM NH₄COOH] | % Solvent [CH₃CN/H₂O 90:10, 10 mM NH₄COOH] | Flow [mL/min] |
|---|---|---|---|
| 0.00 | 100 | 0 | 0.7 |
| 1.50 | 100 | 0 | 0.7 |
| 8.00 | 0 | 100 | 0.7 |
| 10.00 | 0 | 100 | 0.7 |
| 11.00 | 100 | 0 | 0.7 |
| 12.00 | 100 | 0 | 0.7 |

| | | | |
|---|---|---|---|
| Method: | | 20A | |
| Device: | | LC/MS ThermoFinnigan HPLC Surveyor DAD, LCQFleet Ion Trap | |
| Column: | | Simmetry Shield RP8, 5µm, 4,6 x 150 mm | |

| Gradient/Solvent Time [min] | % Solvent [H₂O/CH₃CN 90:10, 10 mM HCOOH] | % Solvent [CH₃CN/H₂O 90:10, 10 mM HCOOH] | Flow [mL/min] |
|---|---|---|---|
| 0.00 | 70 | 30 | 8.50 |
| 1.50 | 50 | 50 | 8.50 |
| 8.50 | 0 | 100 | 8.50 |
| 13.05 | 0 | 100 | 8.50 |
| 14.00 | 70 | 30 | 8.50 |
| 15 | 70 | 30 | 8.50 |

| | | | |
|---|---|---|---|
| Method: | | 20B | |
| Device: | | LC/MS ThermoFinnigan HPLC Surveyor DAD, MSQ single quadrupole | |
| Column: | | Synergi Hydro RP100A, 2,5 µm, 3 x 50 mm | |

| Gradient/Solvent Time [min] | % Solvent [H₂O/CH₃CN 90:10, 5 mM NH4COOH] | % Solvent [CH₃CN/H₂O 90:10] | Flow [mL/min] |
|---|---|---|---|
| 0.00 | 100 | 0 | 1.2 |
| 4.00 | 0 | 100 | 1.2 |
| 5.30 | 0 | 100 | 1.2 |
| 5.50 | 100 | 0 | 1.2 |
| 6.00 | 100 | 0 | 1.2 |

| | | | |
|---|---|---|---|
| Method: | | 20C | |
| Device: | | LC/MS ThermoFinnigan HPLC Surveyor DAD, MSQ single quadrupole | |
| Column: | | Synergi Hydro RP100A, 2,5 µm, 3 x 50 mm | |

| Gradient/Solvent Time [min] | % Solvent [H₂O/CH₃CN 90:10, 5 mM NH4COOH] | % Solvent [CH₃CN/H₂O 90:10] | Flow [mL/min] |
|---|---|---|---|
| 0.00 | 100 | 0 | 1.2 |
| 1.50 | 100 | 0 | 1.2 |
| 9.00 | 0 | 100 | 1.2 |
| 10.50 | 0 | 100 | 1.2 |
| 11.00 | 100 | 0 | 1.2 |
| 12.00 | 100 | 0 | 1.2 |

| | | | |
|---|---|---|---|
| Method: | | 20D | |
| Device: | | LC/MS ThermoFinnigan HPLC Surveyor DAD, LCQFleet Ion Trap | |
| Column: | | Xselect CSH, 2.5 µm, 4,6 x 50 mm | |

| Gradient/Solvent Time [min] | % Solvent [H₂O/CH₃CN 90:10, 10 mM HCOOH] | % Solvent [CH₃CN/H₂O 90:10, 10 mM HCOOH]] | Flow [mL/min] |
|---|---|---|---|
| 0.00 | 100 | 0 | 1.4 |
| 4.00 | 0 | 100 | 1.4 |
| 5.30 | 0 | 100 | 1.4 |
| 5.50 | 100 | 0 | 1.4 |
| 6.00 | 100 | 0 | 1.4 |
| 12.00 | 100 | 0 | 1.2 |

| | | | |
|---|---|---|---|
| Method: | | 20E | |
| Device: | | LC/MS ThermoFinnigan HPLC Surveyor DAD, LCQFleet Ion Trap | |
| Column: | | Xselect CSH, 2.5 µm, 4,6 x 50 mm | |

| Gradient/Solvent Time [min] | % Solvent [H₂O/CH₃CN 90:10, 10 mM HCOOH] | % Solvent [CH₃CN/H₂O 90:10, 10 mM HCOOH]] | Flow [mL/min] |
|---|---|---|---|
| 0.00 | 100 | 0 | 1.4 |
| 1.00 | 100 | 0 | 1.4 |
| 8.05 | 0 | 100 | 1.4 |
| 10.00 | 0 | 100 | 1.4 |
| 10.02 | 100 | 0 | 1.4 |
| 11.00 | 100 | 0 | 1.4 |

| | | | |
|---|---|---|---|
| Method: | | 20F | |
| Device: | | LC/MS Waters Alliance 2695 HPLC System DAD, Quattro Micro Triple | |
| Column: | | Zorbax Eclipse XDB-C18 3.5µm 4,6 x 50 mm, Temp 35° | |

| Gradient/Solvent Time [min] | % Solvent [H₂O/CH₃CN 90:10, 5 mM NH4COOH] | % Solvent [CH₃CN/H₂O 90:10, 10 mM HCOOH]] | Flow [mL/min] |
|---|---|---|---|
| 0.00 | 100 | 0 | 1.3 |
| 4.50 | 0 | 100 | 1.3 |
| 5.80 | 0 | 100 | 1.3 |
| 6.00 | 100 | 0 | 1.3 |

| | | | |
|---|---|---|---|
| Method: | | 21 | |
| Device: | | LC/MS ThermoFinnigan HPLC Surveyor DAD, LCQFleet Ion Trap | |
| Column: | | Symmetry Shield RP8, 5 µm, 4.6 x 150 mm | |

| Gradient/Solvent Time [min] | % Solvent [H₂O/CH₃CN 90:10, 0.1% HCOOH] | % Solvent [CH₃CN/H₂O 90:10, 0.1% HCOOH] | Flow [mL/min] |
|---|---|---|---|
| 0.00 | 95 | 5 | 1 |
| 1.50 | 95 | 5 | 1 |
| 11.05 | 5 | 95 | 1 |
| 13 | 5 | 95 | 1 |
| 13.03 | 95 | 5 | 1 |
| 15 | 95 | 5 | 1 |

| | | | | |
|---|---|---|---|---|
| Method: | | 22 | | |
| Device: | | Agilent 1200 with DA and MS detector | | |
| Column: | | Sunfire C18, 3 x 30 mm, 2.5 µm | | |
| Column Supplier: | | Waters | | |

| Gradient/Solvent Time [min] | % Solvent [H₂O,0.1%TFA] | % Solvent [MeOH] | Flow [ml/min] | Temperature [°C] |
|---|---|---|---|---|
| 0.00 | 95 | 5 | 1.8 | 60 |
| 0.25 | 95 | 5 | 1.8 | 60 |
| 1.70 | 0 | 100 | 1.8 | 60 |
| 1.75 | 0 | 100 | 2.5 | 60 |
| 1.90 | 0 | 100 | 2.5 | 60 |

| | | | | |
|---|---|---|---|---|
| Method: | | 23 | | |
| Device: | | Waters 1525 with DA and MS Detector | | |
| Column: | | Sunfire C18, 4.6 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [ml/min] | Temperature [°C] |
|---|---|---|---|---|
| 0.0 | 97 | 3 | 4 | 60 |
| 0.15 | 97 | 3 | 3 | 60 |
| 2.15 | 0 | 100 | 3.0 | 60 |
| 2.20 | 0 | 100 | 4.5 | 60 |
| 2.40 | 0 | 100 | 4,5 | 60 |

| | | | | |
|---|---|---|---|---|
| Method: | | 24 | | |
| Device: | | Agilent 1100 with DAD, CTC Autosampler and Waters MS Detector | | |
| Column: | | XBridge C18, 4.6 x 30 mm, 3.5 µm | | |
| Column Supplier: | | Waters | | |

| Gradient/Solvent Time [min] | % Solvent [H₂O,0.1%NH₄OH] | % Solvent [CH₃CN] | Flow [ml/min] | Temperature [°C] |
|---|---|---|---|---|
| 0.0 | 98.0 | 2.0 | 2.5 | 60.0 |
| 1.5 | 0.0 | 100.0 | 2.5 | 60.0 |
| 1.8 | 0.0 | 100.0 | 2.5 | 60.0 |

| Method: | | 25 | | |
|---|---|---|---|---|
| Device: | | LC/MS Waters Acquity UPLC System DAD, SQD single quadrupole | | |
| Column: | | HSS C18, 1.8 µm, 2.1 x 50 mm | | |
| Gradient/Solvent Time [min] | % Solvent [H₂O/CH₃CN 90:10,0.1 %TFA] | % Solvent [CH₃CN/H₂O 90:10] | Flow [ml/min] | Temperature [°C] |
| 0.00 | 100 | 0 | 0.70 | 35 |
| 1.20 | 0 | 100 | 0.70 | 35 |
| 1.45 | 0 | 100 | 0.70 | 35 |
| 1.55 | 100 | 0 | 0.70 | 35 |
| 1.75 | 100 | 0 | 0.70 | 35 |

| | | | | |
|---|---|---|---|---|
| Method: | | 26 | | |
| Device: | | Agilent 1200 with DA and MS detector | | |
| Column: | | XBridge C18, 3 x 30 mm, 2.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 50 | 50 | 2.2 | 60 |
| 0.20 | 50 | 50 | 2.2 | 60 |
| 1.20 | 0 | 100 | 2.2 | 60 |
| 1.25 | 0 | 100 | 3 | 60 |
| 1.40 | 0 | 100 | 3 | 60 |

| | | | | |
|---|---|---|---|---|
| Method: | | 27 | | |
| Device: | | Agilent 1100 with DAD, Waters Autosampler and MS Detector | | |
| Column: | | Sunfire C18, 4.6 x 30 mm, 3.5 µm | | |
| Column Supplier: | | Waters | | |

| Gradient/Solvent Time [min] | % Solvent [H₂O,0.1%TFA] | % Solvent [CH₃CN] | Flow [mL/min] | Temperature [°C] |
|---|---|---|---|---|
| 0.00 | 95 | 5 | 4 | 50 |
| 1.20 | 0 | 100 | 4 | 50 |
| 1.8 | 0 | 100 | 4 | 50 |

Analytical GC parameters employed for characterization of products:

| | |
|---|---|
| Method: | 1 |
| Device: | GC/MS Thermo Scientific TRACE GC ULTRA, DSQ II MS single quadrupole |
| Column: | Agilent DB-5MS, 25 m x 0.25 mm x 0.25 µm |
| Carrier gas: | Helium, 1 mL/min costant flow |
| Oven Program: | 50°C to 100°C in 10°C/min, to 200°C in 20°C/min, to 320°C in 30°C/min (hold 10 min) |
| Detection: | DSQ II MS single quadrupole |
| Ion source: | EI |
| Scan range: | 50- 450 amu |

Analytical chiral HPLC parameters employed for characterization of products

| | | | | |
|---|---|---|---|---|
| Method: | | 1a | | |
| Device: | | Agilent 1100 with DAD | | |
| Column: | | Daicel Chiralcel OJ-H | | |

| Solvent Time [min] | % Solvent [Hexane] | % Solvent [Ethanol] | Flow [mL/min] | Temperature [°C] |
|---|---|---|---|---|
| isocratic | 70 | 30 | 1.0 | 25 |

| | | | | |
|---|---|---|---|---|
| Method: | | 1b | | |
| Device: | | Agilent 1100 with DAD | | |
| Column: | | Daicel Chiralcel IA | | |

| Solvent Time [min] | % Solvent [Hexane] | % Solvent [Isopropanoll] | Flow [mL/min] | Temperature [°C] |
|---|---|---|---|---|
| isocratic | 70 | 30 | 1.0 | 25 |

| | | | | |
|---|---|---|---|---|
| Method: | | 1c | | |
| Device: | | Agilent 1100 with DAD | | |
| Column: | | Daicel Chiralcel AD-H | | |

| Solvent Time [min] | % Solvent [Hexane] | % Solvent [Isopropanoll] | Flow [mL/min] | Temperature [°C] |
|---|---|---|---|---|
| isocratic | 70 | 30 | 1.0 | 25 |

The Examples that follow are intended to illustrate the present invention without restricting it:

### Intermediate 1

### Methyl 2-((3S)-6-((1R)-4-(2-methyl-5-(3-(methylsulfonyl)propoxy)phenyl)-2,3-dihydro-1 H-inden-1-yloxy)-2,3-dihydrobenzofuran-3-yl)acetate

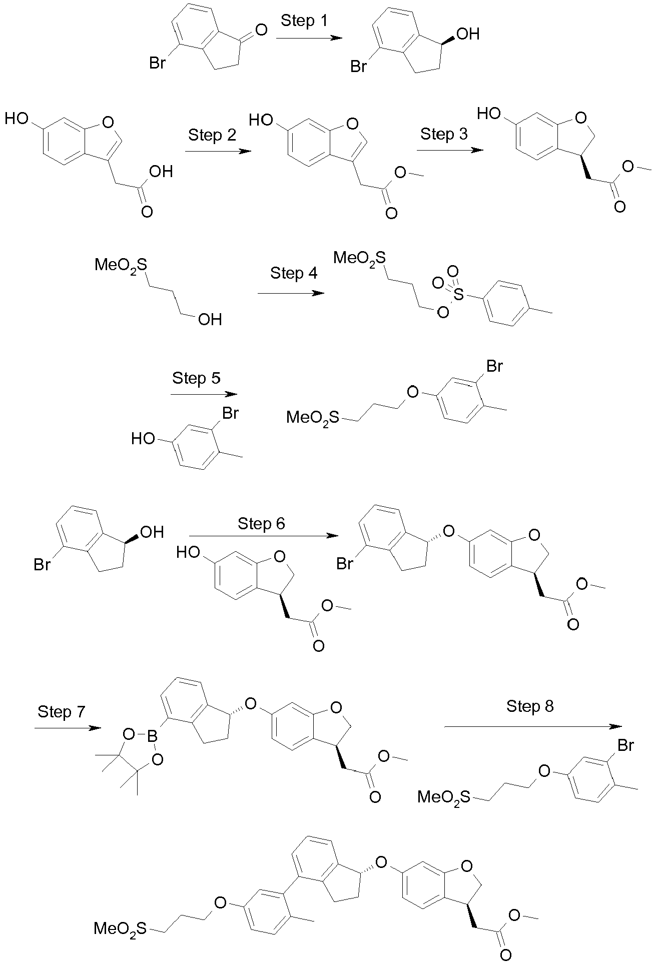

### Step 1: (S)-4-Bromo-2,3-dihydro-1H-inden-1-ol

Formic acid (3.2 mL) is added to a solution of triethylamine (10.5 mL) in dichloromethane (50 mL) chilled in an ice bath. 4-Bromo-2,3-dihydro-1H-inden-1-one (5 g) is added and the flask is purged with argon for 5 minutes. Chloro{[(1S,2S)-(-)-2-amino-1,2-diphenylethyl](4-toluenesulfonyl)amido}-(mesitylene)ruthenium(ll) (700 mg; alternatively, the catalyst is formed in situ from N-[(1S,2S)-2-amino-1,2-diphenylethyl]-4-methylbenzenesulfonamide and dichloro(p-cymene)-ruthenium(II) dimer) is added and the mixture is stirred at room temperature for 16 hours. Water is added and the resulting mixture is extracted with dichloromethane. The combined extract is washed with saturated aqueous NaHCO₃ solution and dried (MgSO₄). The solvent is evaporated and the residue is chromatographed on silica gel (cyclohexane/ethyl acetate 99:1→50:50) to give the title compound. Yield: 4.5 g; LC (method 1): t_{R} = 1.04 min; Mass spectrum (ESI⁺): m/z = 195 [M+H-H₂O]⁺.

### Step 2: Methyl 2-(6-hydroxybenzofuran-3-yl)acetate

A mixture of 2-(6-hydroxybenzofuran-3-yl)acetic acid (for preparation see WO 2008001931; 14.0 g), concentrated sulfuric acid (5 mL), and methanol (250 mL) is stirred at reflux temperature for 4 h. After cooling to room temperature, the mixture is concentrated. Ethyl acetate is added to the residue and the resulting mixture is washed with water, saturated aqueous NaHCO₃ solution, and brine and dried (MgSO₄). The solvent is evaporated and the residue is chromatographed on silica gel (cyclohexane/ethyl acetate 2:1→1:2) to give the title compound. Yield: 9.0 g; Mass spectrum (ESI⁺): m/z = 207 [M+H]⁺.

### Step 3: (S)-Methyl 2-(6-hydroxy-2,3-dihydrobenzofuran-3-yl)acetate

A mixture of methyl 2-(6-hydroxybenzofuran-3-yl)acetate (5.00 g), 10% palladium on carbon (0.50 g), and methanol (50 mL) is shaken under hydrogen atmosphere (3 bar) at room temperature for 3 h. The catalyst is separated by filtration and the filtrate is concentrated. The residue is chromatographed on silica gel (cyclohexane/ethyl acetate 4:1→1:1) to give methyl 2-(6-hydroxy-2,3-dihydrobenzofuran-3-yl)acetate as an oil that solidified upon standing. Yield: 2.60 g; Mass spectrum (ESI⁺): m/z = 209 [M+H]⁺.

The enantiomers are separated by SFC on chiral phase (column: Daicel ADH, 5 µm, 250 mm x 20 mm; eluent: scCO₂/(isopropanol+0.2% diethylamine) 80:20, 70 mL/min):
(S)-Methyl 2-(6-hydroxy-2,3-dihydrobenzofuran-3-yl)acetate: t_{R} = 2.33 min.
(R)-Methyl 2-(6-hydroxy-2,3-dihydrobenzofuran-3-yl)acetate: t_{R} = 2.75 min. Alternatively, the title compound may be obtained as described in WO 2008001931.

### Step 4: 3-(Methylsulfonyl)propyl 4-methylbenzenesulfonate

To a solution of 3-(methylsulfonyl)propan-1-ol (1.0 g) in dichloromethane (10 mL) and pyridine (1.5 mL) is added at 0°C p-toluene-sulfonylchloride (1.38 g) in portions. The mixture is stirred for 12 hours at room temperature, diluted with dichloromethane and washed with 1 M aqueous HCl solution and brine. After drying (MgSO₄) the solvent is evaporated to give the title compound. Yield: 1.6 g; LC (method 1): t_{R} = 0.82 min; Mass spectrum (ESI⁺): m/z = 293 [M+H]⁺.

### Step 5: 2-Bromo-1-methyl-4-(3-(methylsulfonyl)propoxy)benzene

To a solution of 3-bromo-4-methylphenol (200 mg) and K₂CO₃ (193 mg) in N,N-dimethylformamide (5 mL) is added 3-(methylsulfonyl)propyl 4-methylbenzenesulfonate (375 mg). The mixture is stirred for 12 hours at 90°C and then partitioned between water and ethyl acetate. The organic phase is washed with brine and dried (MgSO₄). The solvent is evaporated and the residue is chromatographed on silica gel (cyclohexane/ethyl acetate 80:20→20:80) to give the title compound. Yield: 268 mg; LC (method 1): t_{R} = 1.12 min; Mass spectrum (ESI⁺): m/z = 307 [M+H]⁺.

### Step 6: Methyl 2-((S)-6-((R)-4-bromo-2,3-dihydro-1H-inden-1-yloxy)-2,3-dihydrobenzofuran-3-yl)acetate

A solution of di-tert.-butyl azodicarboxylate (7.5 g) in tetrahydrofuran (15 mL) is added dropwise to a solution of (S)-methyl 2-(6-hydroxy-2,3-dihydrobenzofuran-3-yl)acetate (4.4 g), (S)-4-Bromo-2,3-dihydro-1H-inden-1-ol (4.5 g) and triphenylphosphine (8.3 g) in tetrahydrofuran (30 mL) at 5-10°C. The resulting solution is stirred for 12 hours diluted with ethyl acetate and then washed successively with water, saturated aqueous NaHCO₃ solution and brine. The organic phase is dried (MgSO₄) and concentrated. The residue is chromatographed on silica gel (cyclohexane/ethyl acetate 99:1→50:50) to give the title compound. Yield: 5.5 g; LC (method 1): t_{R} = 1.44 min; Mass spectrum (ESI⁺): m/z = 403 [M+H]⁺.

### Step 7: Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate

In a microwave vial methyl 2-((S)-6-((R)-4-bromo-2,3-dihydro-1H-inden-1-yloxy)-2,3-dihydrobenzofuran-3-yl)acetate (4.4 g), bis-(pinacolato)-diboron (3.7 g) and potassium acetate (2.8 g) are suspended in 1,4-dioxane and purged for 10 minutes with argon. [1,1'-Bis(diphenylphosphino)-ferrocene]-dichloropalladium-(II) (400 mg) is added, the vial is sealed and the mixture is stirred at 100°C for 2 hours. After cooling to room temperature the mixture is diluted with ethyl acetate and washed with saturated aqueous NH₄Cl solution and brine. The organic phase is dried (MgSO₄) and concentrated. The residue is chromatographed on silica gel (cyclohexane/ethyl acetate 99:1→60:40) to give the title compound. Yield: 2.3 g; LC (method 1): t_{R} = 1.48 min; Mass spectrum (ESI⁺): m/z = 451 [M+H]⁺.

### Step 8: Methyl 2-((3S)-6-((1R)-4-(2-methyl-5-(3-(methylsulfonyl)propoxy)phenyl)-2,3-dihydro-1H-inden-1-yloxy)-2,3-dihydrobenzofuran-3-yl)acetate

In a microwave vial methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (50 mg), 2-bromo-1-methyl-4-(3-(methylsulfonyl)propoxy)benzene (38 mg), K₂CO₃ (46 mg) and tetrabutyl-ammonium bromide (9 mg) are suspended in toluene (1 mL) and water (1 mL) and purged for 5 minutes with argon. Tetrakis-triphenylphosphine-palladium-(0) (26 mg) is added, the vial is sealed and the mixture is stirred at 120°C for 2 hours. After cooling to room temperature the mixture is diluted with ethyl acetate and washed with brine. The organic phase is dried (MgSO₄) and concentrated. The residue is chromatographed on silica gel (cyclohexane/ethyl acetate 99:1→50:50) to give the title compound. Yield: 34 mg; LC (method 1): t_{R} = 1.37 min; Mass spectrum (ESI⁺): m/z = 551 [M+H]⁺.

### Intermediate 2

### Methyl 2-((S)-6-((R)-4-(2,6-dimethyl-4-(3-methylsulfonyl)propoxy)phenyl-2,3-dihydro-1H-inden-1-yloxy)-2,3-dihydrobenzofuran-3-yl)acetate

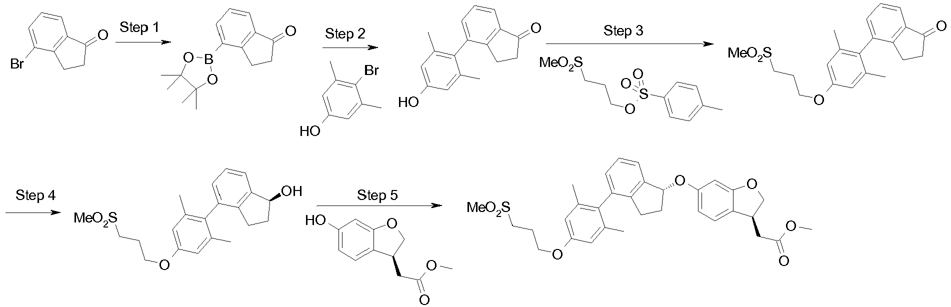

### Step 1: 4-(4,4,5,5-Tetramethyl-1,3,2-dioxaborolan-2-yl)-2,3-dihydro-1H-inden-1-one

The title compound is prepared from 4-bromo-2,3-dihydro-1H-inden-1-one following a procedure analogous to that described in Step 7 of Intermediate 1. LC (method 1): t_{R} = 1.26 min; Mass spectrum (ESI⁺): m/z = 259 [M+H]⁺.

### Step 2: 4-(4-Hydroxy-2,6-dimethylphenyl)-2,3-dihydro-1H-inden-1-one

In a microwave vial a mixture of 4-(4,4,5,5-tetramethyl-1,3,2-dioxaborolan-2-yl)-2,3-dihydro-1H-inden-1-one (610 mg), 4-bromo-3,5-dimethylphenol (475 mg), Na₂CO₃ (8 mL of a 1 M aqueous solution), toluene (8 mL) and ethanol (2.5 mL) is purged for 5 minutes with argon. Tetrakis-triphenylphosphine-palladium-(0) (140 mg) is added, the vial is sealed and the mixture is stirred at 110°C for 12 hours. After cooling to room temperature the mixture is diluted with diethylether and washed with saturated aqueous NH₄Cl and brine. The organic phase is dried (MgSO₄) and concentrated. The residue is chromatographed on silica gel (cyclohexane/ethyl acetate 90:10→60:40) to give the title compound. Yield: 210 mg; LC (method 1): t_{R} = 1.07 min; Mass spectrum (ESI⁺): m/z = 253 [M+H]⁺.

### Step 3: 4-(2,6-Dimethyl-4-(3-(methylsulfonyl)propoxy)phenyl)-2,3-dihydro-1H-inden-1-one

The title compound is prepared from 4-(4-hydroxy-2,6-dimethylphenyl)-2,3-dihydro-1H-inden-1-one following a procedure analogous to that described in Step 5 of Intermediate 1. The mixture is heated for 6 days at 90°C. LC (method 1): t_{R} = 1.12 min; Mass spectrum (ESI⁺): m/z = 373 [M+H]⁺.

### Step 4: (S)-4-(2,6-Dimethyl-4-(3-(methylsulfonyl)propoxy)phenyl)-2,3-dihydro-1H-inden-1-ol

The title compound is prepared from 4-(2,6-dimethyl-4-(3-(methylsulfonyl)propoxy)phenyl)-2,3-dihydro-1H-inden-1-one following a procedure analogous to that described in Step 1 of Intermediate 1. LC (method 1): t_{R} = 1.08 min; Mass spectrum (ESI⁺): m/z = 357 [M+H-H₂O]⁺.

### Step 5: Methyl 2-((S)-6-((R)-4-(2,6-dimethyl-4-(3-(methylsulfonyl)propoxy)phenyl)-2,3-dihydro-1 H-inden-1-yloxy)-2,3-dihydrobenzofuran-3-yl)acetate

The title compound is prepared from (S)-4-(2,6-dimethyl-4-(3-(methylsulfonyl)propoxy)phenyl)-2,3-dihydro-1H-inden-1-ol and (S)-methyl 2-(6-hydroxy-2,3-dihydrobenzofuran-3-yl)acetate following a procedure analogous to that described in Step 6 of Intermediate 1. The mixture is stirred for 1 hour. LC (method 1): t_{R} = 1.38 min; Mass spectrum (ESI⁺): m/z = 582 [M+NH₄]⁺.

### Intermediate 3

### Methyl 2-((3S)-6-((1R)-4-(1-phenyl-1H-tetrazol-5-yl)-2,3-dihydro-1H-inden-1-yloxy)-2,3-dihydrobenzofuran-3-yl)acetate

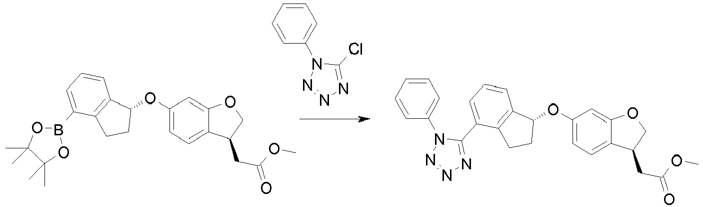

In a microwave vial methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (150 mg), 5-chloro-1-phenyl-1 H-tetrazole (66 mg) and Ba(OH)₂ (171 mg) are suspended in 1,4-dioxane (1.3 mL) and purged for 5 minutes with argon. Bis-(dibenzylideneacetone)-palladium(0) (19 mg) and 2-dicyclohexylphosphino-2',6'-dimethoxybiphenyl (27 mg), are added, the vial is sealed and the mixture is stirred at 100°C for 1 hour and at 120°C for 30 minutes. After cooling to room temperature the mixture is diluted with tetrahydrofuran, filtered and used directly in the next step (example 3).

### Intermediate 4

### Methyl 2-((3S)-6-((1R)-4-(2,6-dimethyl-3-(3-(methylsulfonyl)propoxy)phenyl-2,3-dihydro-1H-inden-1-yloxy)-2,3-dihydrobenzofuran-3-yl)acetate

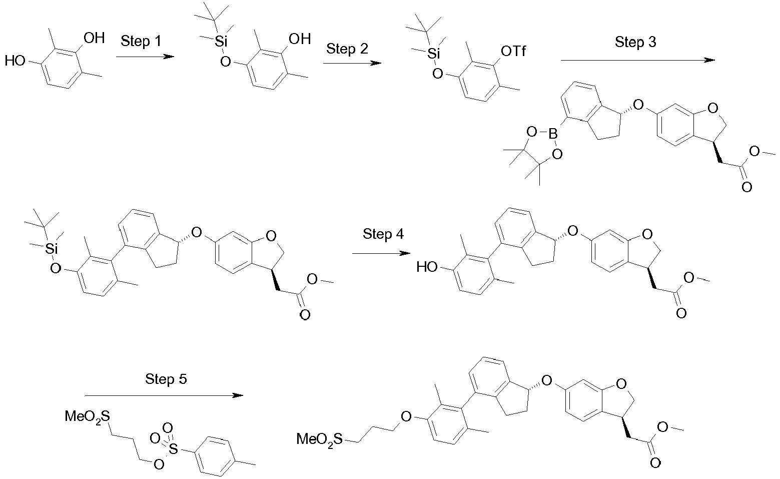

### Step 1: 3-(tert-Butyldimethylsilyloxy)-2,6-dimethylphenol

2,4-Dimethylbenzene-1,3-diol (1.5 g) and triethylamine (2.3 mL) are dissolved in dichloromethane (15 mL), cooled to 0°C and treated with tert-butylchlorodimethylsilane (1.7 g) and 4-dimethylaminopyridine (133 mg). The mixture is stirred for 12 hours at room temperature, diluted with dichloromethane and washed with 1 M hydrochloric acid, saturated aqueous NaHCO₃ solution and brine. After drying (MgSO₄) the solvents are evaporated in vacuo and the residue is chromatograped on silica gel (cyclohexane/ethyl acetate 99:1→60:40) to give the title compound. Yield: 2.1 g; LC (method 1): t_{R} = 1.44 min; Mass spectrum (ESI⁺): m/z = 253 [M+H]⁺.

### Step 2: 3-(tert-Butyldimethylsilyloxy)-2,6-dimethylphenyl trifluoromethane-sulfonate

3-(tert-Butyldimethylsilyloxy)-2,6-dimethylphenol (2.1 g) and 2,6-dimethylpyridine (lutidine) (2 mL) are dissolved in dichloromethane (20 mL) cooled to 0°C and treated with trifluoromethanesulfonic anhydride (1.6 mL). The mixture is stirred for 3 hours at room temperature, diluted with dichloromethane and washed with saturated aqueous NH₄CI solution and brine. After drying (MgS0₄) the solvents are evaporated in vacuo and the residue is chromatograped on silica gel (cyclohexane/ethyl acetate 99:1→70:30) to give the title compound. Yield: 2.6 g; LC (method 1): t_{R} = 1.60 min.

### Step 3: Methyl 2-((3S)-6-((1R)-4-(3-(tert-butyldimethylsilyloxy)-2,6-dimethylphenyl)-2,3-dihydro-1 H-inden-1-yloxy)-2,3-dihydrobenzofuran-3-yl)acetate

The title compound is prepared from 3-(tert-Butyldimethylsilyloxy)-2,6-dimethylphenyl trifluoromethanesulfonate and methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 8 of Intermediate 1. LC (method 1): t_{R} = 1.66 min; Mass spectrum (ESI⁺): m/z = 559 [M+H-H₂O]⁺.

### Step 4: Methyl 2-((3S)-6-((1R)-4-(3-hydroxy-2,6-dimethylphenyl)-2,3-dihydro-1H-inden-1-yloxy)-2,3-dihydrobenzofuran-3-yl)acetate

To a solution of methyl 2-((3S)-6-((1R)-4-(3-(tert-butyldimethylsilyloxy)-2,6-dimethylphenyl)-2,3-dihydro-1H-inden-1-yloxy)-2,3-dihydrobenzofuran-3-yl)acetate (95 mg) in tetrahydrofuran (3 mL) is added at 0°C tetrabutylammonium fluoride (700 µL of a 1 M solution in tetrahydrofuran). The mixture is stirred for 3 hours at room temperature, diluted with ethyl acetate and washed with water and brine. After drying (MgSO₄) the solvents are evaporated and the residue is chromatograped on silica gel (cyclohexane/ethyl acetate 99:1→50:50) to give the title compound. Yield: 33 mg; LC (method 1): t_{R} = 1.37 min; Mass spectrum (ESI⁺): m/z = 445 [M+H]⁺.

### Step 5: Methyl 2-((3S)-6-((1R)-4-(2,6-dimethyl-3-(3-(methylsulfonyl)propoxy)phenyl)-2,3-dihydro-1 H-inden-1-yloxy)-2,3-dihydrobenzofuran-3-yl)acetate

The title compound is prepared from methyl 2-((3S)-6-((1R)-4-(3-hydroxy-2,6-dimethylphenyl)-2,3-dihydro-1H-inden-1-yloxy)-2,3-dihydrobenzofuran-3-yl)acetate and 3-(methylsulfonyl)propyl 4-methylbenzenesulfonate following a procedure analogous to that described in Step 5 of Intermediate 1. LC (method 1): t_{R} = 1.36 min; Mass spectrum (ESI⁺): m/z = 565 [M+H-H₂O]⁺.

### Intermediate 5

### Methyl 2-((S)-6-((R)-4-(2,6-dimethyl-4-((R)-tetrahydrofuran-3-.phenyl-2-3-dihydro-1H-inden-1-yloxy)-2,3-dihydrozofuran-3-ylacetate

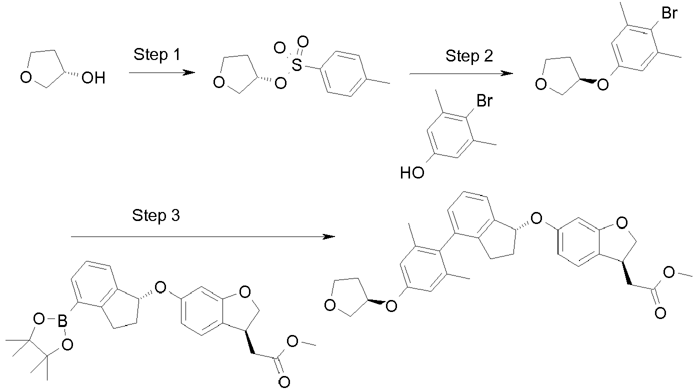

### Step 1: (S)-Tetrahydrofuran-3-yl 4-methylbenzenesulfonate

The title compound is prepared from (S)-tetrahydrofuran-3-ol following a procedure analogous to that described in Step 4 of Intermediate 1. Mass spectrum (ESI⁺): m/z = 243 [M+H]⁺.

### Step 2: (R)-3-(4-Bromo-3,5-dimethylphenoxy)tetrahydrofuran

The title compound is prepared from (S)-tetrahydrofuran-3-yl 4-methylbenzenesulfonate following a procedure analogous to that described in Step 5 of Intermediate 1. LC (method 1): t_{R} = 1.40 min; Mass spectrum (ESI⁺): m/z = 271 [M+H]⁺.

### Step 3: Methyl 2-((S)-6-((R)-4-(2,6-dimethyl-4-((R)-tetrahydrofuran-3-yloxy)phenyl)-2,3-dihydro-1H-inden-1-yloxy)-2,3-dihydrobenzofuran-3-yl)acetate

In a microwave vial methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (100 mg) and (R)-3-(4-bromo-3,5-dimethylphenoxy)tetrahydrofuran (66 mg) are suspended in N,N-dimethylformamide (2 mL) and Na₂CO₃ (390 µL of a 2M aqueous solution). The mixture is purged for 5 minutes with argon. [1,1'-Bis(diphenylphosphino)-ferrocene]-dichloropalladium dichloromethane complex (10 mg) is added, the vial is sealed and the mixture is stirred at 90°C for 2 hours. After cooling to room temperature the mixture is diluted with methanol and purified by HPLC on reversed phase. Yield: 35 mg; LC (method 2): t_{R} = 1.12 min; Mass spectrum (ESI⁺): m/z = 515 [M+H]⁺.

### Intermediate 6

### Methyl 2-((S)-6-((R)-4-(2,6-dimethyl-4-((S)-tetrahydrofuran-3-yloxy)phenyl-2,3-dihydro-1H-inden-1-yloxy)-2,3-dihydrobenzofuran-3-yl)acetate

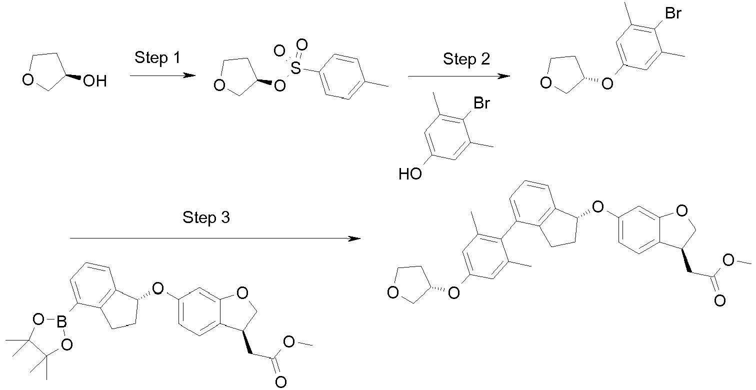

### Step 1: (R)-Tetrahydrofuran-3-yl 4-methylbenzenesulfonate

The title compound is prepared from (R)-tetrahydrofuran-3-ol following a procedure analogous to that described in Step 4 of Intermediate 1.The reaction is performed in the presence of a catalytic amount of 4-dimethylaminopyridine. Mass spectrum (ESI⁺): m/z = 243 [M+H]⁺.

### Step 2: (S)-3-(4-Bromo-3,5-dimethylphenoxy)tetrahydrofuran

The title compound is prepared from (R)-tetrahydrofuran-3-yl 4-methylbenzenesulfonate following a procedure analogous to that described in Step 5 of Intermediate 1. LC (method 1): t_{R} = 1.30 min; Mass spectrum (ESI⁺): m/z = 271 [M+H]⁺.

### Step 3: Methyl 2-((S)-6-((R)-4-(2,6-dimethyl-4-((S)-tetrahydrofuran-3-yloxy)phenyl)-2,3-dihydro-1H-inden-1-yloxy)-2,3-dihydrobenzofuran-3-yl)acetate

The title compound is prepared from 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate and (S)-3-(4-Bromo-3,5-dimethylphenoxy)tetrahydrofuran following a procedure analogous to that described in Step 8 of Intermediate 1. LC (method 1): t_{R} = 1.46 min; Mass spectrum (ESI⁺): m/z = 515 [M+H]⁺.

### Intermediate 7

### 4-(4-Bromo-3,5-dimethylphenoxy)-2-methylbutan-2-ol

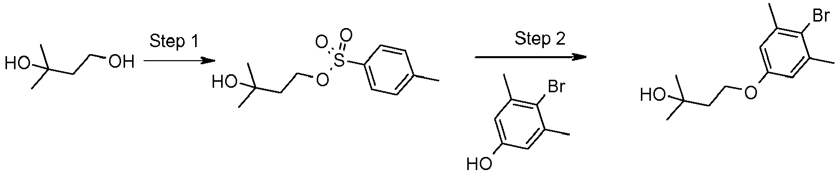

### Step 1: 3-Hydroxy-3-methylbutyl 4-methylbenzenesulfonate

The title compound is prepared from 3-methylbutane-1,3-diol and 4-methylbenzene-1-sulfonyl chloride following a procedure analogous to that described in Step 4 of Intermediate 1. The crude product is purified by chromatography on silica gel (cyclohexane/ethyl acetate 90:10→70:30). Mass spectrum (ESI⁺): m/z = 276 [M+NH₄]⁺.

### Step 2: 4-(4-Bromo-3,5-dimethylphenoxy)-2-methylbutan-2-ol

The title compound is prepared from 4-bromo-3,5-dimethylphenol and 3-hydroxy-3-methylbutyl 4-methylbenzenesulfonate following a procedure analogous to that described in Step 5 of Intermediate 1. LC (method 1): t_{R} = 1.35 min; Mass spectrum (ESI⁺): m/z = 269 [M+H-H₂O]⁺.

### Intermediate 8

### Methyl 2-((S)-6-((R)-4-(4-(2-methoxyethoxy)-2,6-dimethylphenyl)-2,3-dihydro-1H-inden-1-yloxy)-2,3-dihydrobenzofuran-3-yl)acetate

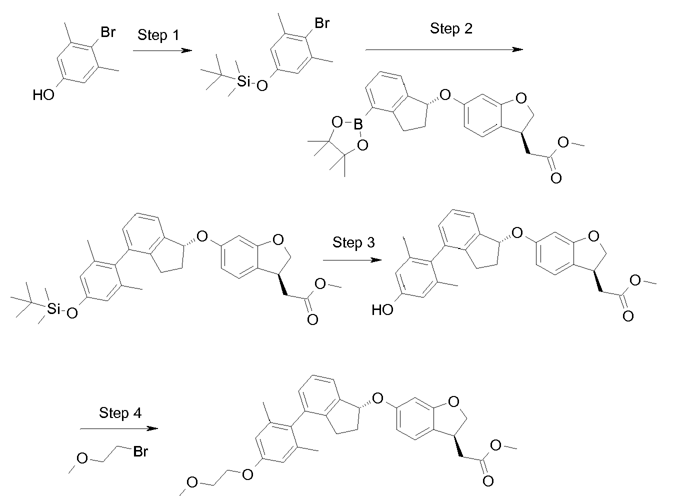

### Step 1: (4-Bromo-3,5-dimethylphenoxy)(tert-butyl)dimethylsilane

The title compound is prepared from 4-bromo-3,5-dimethylphenol and tert-butylchlorodimethylsilane following a procedure analogous to that described in Step 1 of Intermediate 4. LC (method 1): t_{R} = 1.62 min; Mass spectrum (ESI⁺): m/z = 315 [M+H]⁺.

### Step 2: Methyl 2-((S)-6-((R)-4-(4-(tert-butyldimethylsilyloxy)-2,6-dimethylphenyl)-2,3-dihydro-1H-inden-1-yloxy)-2,3-dihydrobenzofuran-3-yl)acetate

The title compound is prepared from (4-bromo-3,5-dimethylphenoxy)(tert-butyl)dimethylsilane and methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 8 of Intermediate 1. LC (method 1): t_{R} = 1.65 min; Mass spectrum (ESI⁺): m/z = 559 [M+H-H₂O]⁺.

### Step 3: Methyl 2-((S)-6-((R)-4-(4-hydroxy-2,6-dimethylphenyl)-2,3-dihydro-1H-inden-1-yloxy)-2,3-dihydrobenzofuran-3-yl)acetate

The title compound is prepared from methyl 2-((S)-6-((R)-4-(4-(tert-butyldimethylsilyloxy)-2,6-dimethylphenyl)-2,3-dihydro-1H-inden-1-yloxy)-2,3-dihydrobenzofuran-3-yl)acetate following a procedure analogous to that described in Step 4 of Intermediate 4. LC (method 1): t_{R} = 1.36 min; Mass spectrum (ESI⁺): m/z = 445 [M+H]⁺.

### Step 4: Methyl 2-((S)-6-((R)-4-(4-(2-methoxyethoxy)-2,6-dimethylphenyl)-2,3-dihydro-1 H-inden-1-yloxy)-2,3-dihydrobenzofuran-3-yl)acetate

To a solution of methyl 2-((S)-6-((R)-4-(4-hydroxy-2,6-dimethylphenyl)-2,3-dihydro-1 H-inden-1-yloxy)-2,3-dihydrobenzofuran-3-yl)acetate (37 mg) and K₂CO₃ (24 mg) in N,N-dimethylformamide (2 mL) is added 1-bromo-2-methoxyethane (10 µL). The mixture is stirred for 12 hours at room temperature. 1-Bromo-2-methoxyethane (10 µL) is added and the mixture is stirred for 4 hours at 50°C. After addition of 1-bromo-2-methoxyethane (10 µL) the mixture is stirred for 12 hours at 50°C, followed by addition of 1-bromo-2-methoxyethane (20 µL) and K₂CO₃ (12 mg). The mixture is stirred for further 5 hours at 50°C, then diluted with ethyl acetate and washed with water and brine. After drying (MgSO₄) the solvents arer evaporated and the residue is chromatographed on silica gel (cyclohexane/ethyl acetate 99:1→50:50) to give the title compound. Yield: 28 mg; LC (method 1): t_{R} = 1.47 min; Mass spectrum (ESI⁺): m/z = 503 [M+H]⁺.

### Intermediate 9

### Methyl 2-((S)-6-((R)-4-(2,6-dimethyl-4-((3-methyloxetan-3-yl)methoxy)phenyl)-2,3-dihydro-1H-inden-1-yloxy)-2,3-dihydrobenzofuran-3-yl)acetate

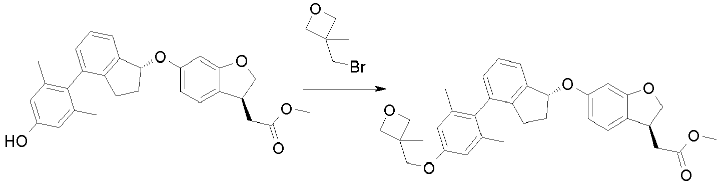

To a solution of methyl 2-((S)-6-((R)-4-(4-hydroxy-2,6-dimethylphenyl)-2,3-dihydro-1 H-inden-1-yloxy)-2,3-dihydrobenzofuran-3-yl)acetate (37 mg) and K₂CO₃ (24 mg) in N,N-dimethylformamide (2 mL) is added 3-(bromomethyl)-3-methyloxetane (17 mg). The mixture is stirred for 12 hours at room temperature. 1-Bromo-2-methoxyethane (10 mg) is added and the mixture is stirred for 4 hours at 50°C. The mixture is diluted with ethyl acetate and washed with water and brine. After drying (MgSO₄) the solvents are evaporated and the residue is chromatographed on silica gel (cyclohexane/ethyl acetate 80:20→20:80) to give the title compound. Yield: 33 mg; LC (method 1): t_{R} = 1.50 min; Mass spectrum (ESI⁺): m/z = 529 [M+H]⁺.

### Intermediate 10

### Methyl 2-((3S)-6-((1R)-4-(3-(2-methoxyethoxy)-2-methylphenyl)-2,3-dihydro-1H-inden-1-yloxy-2,3-ydrobenzofuran-3-yl)acetate

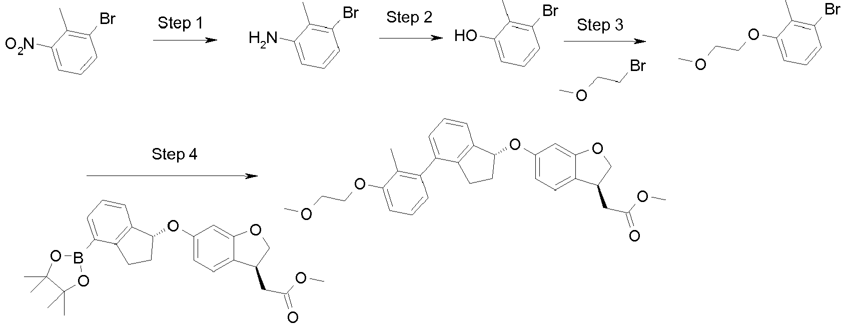

### Step 1: 3-Bromo-2-methylaniline

1-Bromo-2-methyl-3-nitrobenzene (4 g) is dissolved in ethanol (30 mL) and acetic acid (6 mL) and heated to gentle reflux. Iron powder (4.1 g) is added in small portions and the mixture is heated for 2 hours. After addition of 3 N hydrochloric acid (0.5 mL) iron powder (1 g) is cautiously added and the mixture is heated for 8 hours to reflux. After cooling to room temperature 2 N aqueous sodium hydroxide (50 mL) is added, the mixture is filtered over celite and the filter cake is rinsed with ethyl acetate. The organic phase is separated, washed with brine and dried (MgSO₄). Evaporation of the solvents gives the title compound. Yield: 3.1 g; TLC: r_{f} = 0.34 (silicagel, petrole ether/ethyl acetate 5:1).

### Step 2: 3-Bromo-2-methylphenol

At -5°C 3-bromo-2-methylaniline is dissolved in 1 M sulfuric acid (20 mL). A solution of sodium nitrite (1.4 g) in water (3 mL) is added dropwise and the mixture is stirred for 15 minutes. Then concentrated sulfuric acid (6.8 mL) is added dropwise and the mixture is heated for 1 hour to 100°C. The mixture is cooled to 0°C, diluted with water and extracted twice with diethylether. The combined organic phases are dried (MgSO₄) and concentrated. The residue is chromatographed on silica gel (cyclohexane/ethyl acetate 83:17→50:50) to give the title compound. Yield: 1.6 g; Mass spectrum (ESI⁻): m/z = 185 [M-H]⁻.

### Step 3: 1-Bromo-3-(2-methoxyethoxy)-2-methylbenzene

Under argon NaH (ca. 60% in mineral oil; 26 mg) is added to a cold (0°C) solution of 3-bromo-2-methylphenol (100 mg) in tetrahydrofuran (3 mL). The mixture is stirred for 10 minutes and then 1-bromo-2-methoxyethane (60 µL) is added. The mixture is then stirred for 12 hours at room temperature and 6 hours at 50°C. NaH (ca. 60% in mineral oil; 20 mg) and 1-bromo-2-methoxyethane (50 µL) are added successively and the mixture is heated to reflux for 8 hours. After cooling to room temperature the mixture is partitioned between water and ethyl acetate. The organic phase is dried (MgSO₄) and concentrated. The residue is chromatographed on silica gel (cyclohexane/ethyl acetate 95:5) to give the title compound. Yield: 74 mg; LC (method 3): t_{R} = 1.07 min; TLC: r_{f} = 0.40 (silicagel, petrole ether/ethyl acetate 9:1).

### Step 4: Methyl 2-((3S)-6-((1 R)-4-(3-(2-methoxyethoxy)-2-methylphenyl)-2,3-dihydro-1 H-inden-1-yloxy)-2,3-dihydrobenzofuran-3-yl)acetate

In a microwave vial methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (50 mg) and 1-bromo-3-(2-methoxyethoxy)-2-methylbenzene (33 mg) and K₃PO₄ (100 mg) are suspended in tetrahydrofuran (5 mL). The mixture is purged for 5 minutes with argon. [1,1'-Bis(diphenylphosphino)-ferrocene]-dichloropalladium dichloromethane complex (8 mg) is added, the vial is sealed and the mixture is stirred at 100°C for 1.5 hours. After cooling to room temperature the mixture is diluted with ethyl acetate and washed with brine. The organic phase is dried (MgSO₄) and concentrated. The residue is chromatographed on silica gel (cyclohexane/ethyl acetate 90:10→70:30) to give the title compound. Yield: 34 mg; LC (method 3): t_{R} = 1.59 min; Mass spectrum (ESI⁺): m/z = 489 [M+H]⁺.

### Intermediate 11

### Methyl 2-((3S)-6-((1R)-4-(3-(2-hydroxyethoxy)-2-methylphenyl)-2,3-dihydro-H-inden-1-yloxy-2,3-dihydrobenzofuran-3-yl)acetate

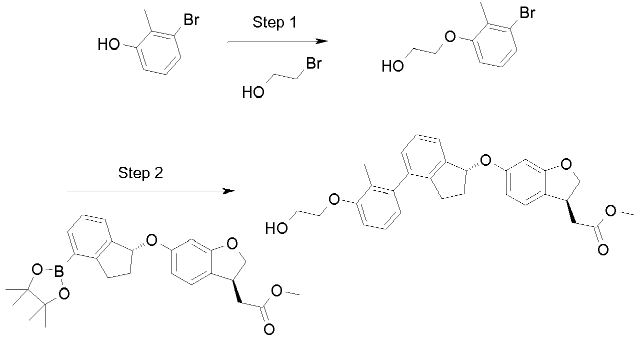

### Step 1: 2-(3-Bromo-2-methylphenoxy)ethanol

To a solution of 3-bromo-2-methylphenol (145 mg) and K₂CO₃ (200 mg) in N,N-dimethylformamide (3 mL) is added 2-bromoethanol (60 µL). The mixture is stirred for 2 hours at 80°C. 2-bromoethanol (350 µL) is added, the mixture is heated for 2 hours at 80°C and for 18 hours to 120°C. After cooling to room temperature the mixture is diluted with ethyl acetate and washed with water and brine. The organic phase is dried (MgSO₄) and concentrated. The residue is chromatographed on silica gel (cyclohexane/ethyl acetate 90:10→70:30) to give the title compound. Yield: 87 mg; TLC: r_{f} = 0.30 (silicagel, petrole ether/ethyl acetate 2:1).

### Step 2: Methyl 2-((3S)-6-((1 R)-4-(3-(2-hydroxyethoxy)-2-methylphenyl)-2,3-dihydro-1 H-inden-1-yloxy)-2,3-dihydrobenzofuran-3-yl)acetate

The title compound is prepared from methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate and 2-(3-bromo-2-methylphenoxy)ethanol following a procedure analogous to that described in Step 4 of Intermediate 10. LC (method 3): t_{R} = 1.41 min; Mass spectrum (ESI⁺): m/z = 475 [M+H]⁺.

### Intermediate 12

### Methyl 2-((S)-6-((R)-4-(2,6-dimethyl-4-((3-methyloxetan-3-yl)methoxy)phenyl)-7-fluoro-2,3-dihydro-1H-inden-1-yloxy-2,3-dihydrobenzofuran-3-yl)acetate

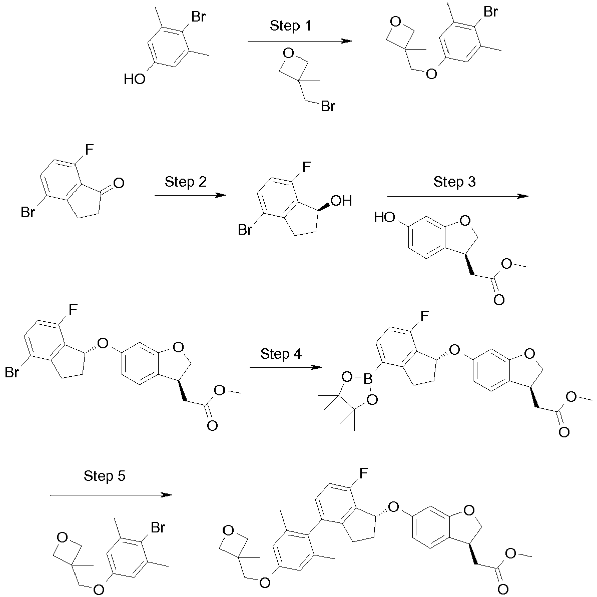

### Step 1: 3-((4-Bromo-3,5-dimethylphenoxy)methyl)-3-methyloxetane

To a solution of 4-bromo-3,5-dimethylphenol (3.0 g) and K₂CO₃ (5.5 g) in N,N-dimethylformamide (10 mL) is added 3-(bromomethyl)-3-methyloxetane (2.95 g). The mixture is stirred for 12 hours at room temperature and then partitioned between saturated aqueous NaHCO₃ solution and ethyl acetate. The aqueous phase is extracted with ethyl acetate and the combined organic phases are dried (MgSO₄). The solvent is evaporated and the residue is chromatographed on silica gel (cyclohexane/ethyl acetate 100:0→70:30) to give the title compound. Yield: 3.5 g; LC (method 4): t_{R} = 1.81 min; Mass spectrum (ESI⁺): m/z = 285 [M+H]⁺.

### Step 2: (S)-4-Bromo-7-fluoro-2,3-dihydro-1H-inden-1-ol

The title compound is prepared from 4-bromo-7-fluoro-2,3-dihydro-1H-inden-1-one following a procedure analogous to that described in Step 1 of Intermediate 1. LC (method 1): t_{R} = 1.04 min; Mass spectrum (ESI⁺): m/z = 213 [M+H-H₂O]⁺.

### Step 3: Methyl 2-((S)-6-((R)-4-bromo-7-fluoro-2,3-dihydro-1H-inden-1-yloxy)-2,3-dihydrobenzofuran-3-yl)acetate

A solution of di-tert.-butyl azodicarboxylate (18 g) in tetrahydrofuran (80 mL) is added dropwise over 45 minutes to a solution of (S)-methyl 2-(6-hydroxy-2,3-dihydrobenzofuran-3-yl)acetate (11 g), (S)-4-bromo-7-fluoro-2,3-dihydro-1H-inden-1-ol (11.95 g) and tributylphosphine (19.3 mL) in tetrahydrofuran (320 mL) at -10°C. The resulting solution is stirred for 30 minutes and then partitioned between saturated aqueous NaHCO₃ solution and dichloromethane. The aqueous phase is extracted with dichloromethane. The combined organic phases are dried (MgSO₄) and concentrated. The residue is chromatographed on silica gel (cyclohexane/ethyl acetate 90:10→70:30) to give the title compound. Yield: 10.35 g; LC (method 1): t_{R} = 1.41 min; Mass spectrum (ESI⁺): m/z = 421 [M+H]⁺.

### Step 4: Methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate

In a microwave vial methyl 2-((S)-6-((R)-4-bromo-7-fluoro-2,3-dihydro-1H-inden-1-yloxy)-2,3-dihydrobenzofuran-3-yl)acetate (7.0 g), bis-(pinacolato)-diboron (5.6 g) and potassium acetate (4.2 g) are suspended in 1,4-dioxane and purged for 10 minutes with argon. [1,1'-Bis(diphenylphosphino)-ferrocene]-dichloropalladium-(II) (600 mg) is added, the vial is sealed and the mixture is stirred at 100°C for 4 hours. After cooling to room temperature the mixture is partitioned between diethylether and saturated aqueous NH₄Cl solution. The organic phase is dried (MgSO₄) and concentrated. The residue is chromatographed on silica gel (cyclohexane/ethyl acetate 99:1→70:30) to give the title compound. Yield: 5.85 g; LC (method 1): t_{R} = 1.48 min; Mass spectrum (ESI⁺): m/z = 469 [M+H]⁺.

### Step 5: Methyl 2-((S)-6-((R)-4-(2,6-dimethyl-4-((3-methyloxetan-3-yl)methoxy)phenyl)-7-fluoro-2,3-dihydro-1H-inden-1-yloxy)-2,3-dihydrobenzofuran-3-yl)acetate

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (500 mg), 3-((4-bromo-3,5-dimethylphenoxy)methyl)-3-methyloxetane (450 mg), K₃PO₄ (450 mg) are suspended in toluene (2.5 mL) and water (250 µL) and purged for 10 minutes with argon. Palladium-(II)-acetate (12 mg) and dicyclohexyl(2',6'-dimethoxybiphenyl-2-yl)phosphine (S-Phos) (44 mg) are added, the vial is sealed and the mixture is stirred at 100°C for 4 hours. After cooling to room temperature the mixture is partitioned between diethylether and saturated aqueous NH₄Cl solution. The organic phase is dried (MgSO₄) and concentrated. The residue is chromatographed on silica gel (cyclohexane/ethyl acetate 90:10→60:40) to give the title compound. Yield: 420 mg; LC (method 4): t_{R} = 1.93 min; Mass spectrum (ESI⁺): m/z = 569 [M+Na]⁺.

### Intermediate 13

### Methyl 2-((3S)-6-((1R)-4-(2,6-dimethyl-4-((tetrahydrofuran-3-yl)methoxy)phenyl)-7-fluoro-2,3-dihydro-1H-inden-1-yloxy)-2,3-dihydrobenzofuran-3-yl)acetate

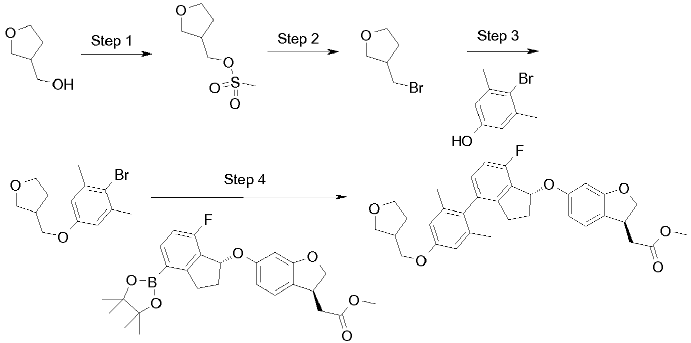

### Step 1: (Tetrahydrofuran-3-yl)methyl methanesulfonate

To a cooled (0°C) solution of (tetrahydrofuran-3-yl)methanol (3.0 mL) and triethylamine (5.7 mL) in dichloromethane (30 mL) is added methanesulfonyl chloride (3.1 mL). The mixture is stirred for 12 hours at room temperature. After cooling to 0°C triethylamine (1.3 mL) and methanesulfonyl chloride (0.7 mL) are added and the mixture is stirred for 12 hours at room temperature. The mixture is partitioned between dichloromethane and saturated aqueous NaHCO₃ solution and stirred vigorously for 30 minutes. The organic phase is separated, washed with brine and dried (MgSO₄). The solvent is evaporated to give the title compound. Yield: 5.6 g; TLC: r_{f} = 0.35 (silicagel, cyclohexane/ethyl acetate 1:1); Mass spectrum (ESI⁺): m/z = 181 [M+H]⁺.

### Step 2: 3-(Bromomethyl)tetrahydrofuran

To solution of (tetrahydrofuran-3-yl)methyl methanesulfonate (5.6 g) in acetone (80 mL) is added lithium bromide (13.6 g). The mixture is heated to reflux for 4 hours. Then the solvent is evaporated in vacuo and the residue is partitioned between dichloromethane and water. The organic phase is separated and dried (MgSO₄). The solvents are evaporated. The residue is dissolved in dichloromethane and filtered over a small plug of silica gel. Concentration gives the title compound. Yield: 3.9 g; TLC: r_{f} = 0.80 (silicagel, cyclohexane/ethyl acetate 1:1).

### Step3: 3-((4-Bromo-3,5-dimethylphenoxy)methyl)tetrahydrofuran

The title compound is prepared from 4-bromo-3,5-dimethylphenol and 3-(bromomethyl)tetrahydrofuran following a procedure analogous to that described in Step 5 of Intermediate 1. LC (method 1): t_{R} = 1.37 min; Mass spectrum (ESI⁺): m/z = 285 [M+H]⁺.

### Step 4: Methyl 2-((3S)-6-((1 R)-4-(2,6-dimethyl-4-((tetrahydrofuran-3-yl)methoxy)phenyl)-7-fluoro-2,3-dihydro-1H-inden-1-yloxy)-2,3-dihydrobenzofuran-3-yl)acetate

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (170 mg) and 3-((4-bromo-3,5-dimethylphenoxy)methyl)tetrahydrofuran (170 mg) and K₃PO₄ (290 mg) are suspended in tetrahydrofuran (10 mL). The mixture is purged for 5 minutes with argon. [1,1'-Bis(diphenylphosphino)-ferrocene]-dichloropalladium (27 mg) is added, the vial is sealed and the mixture is stirred at 100°C for 12 hours. After cooling to room temperature the mixture is diluted with diethylether and washed with saturated aqueous NH₄Cl solution. The organic phase is dried (MgSO₄) and concentrated. The residue is chromatographed on silica gel (cyclohexane/ethyl acetate 90:10→50:50) to give the title compound. Yield: 47 mg; LC (method 1): t_{R} = 1.48 min; Mass spectrum (ESI⁺): m/z = 569 [M+Na]⁺.

### Intermediate 14

### Methyl 2-((3S)-6-((1R)-4-(2-(1-cyanocyclopropyl)phenyl)-2,3-dihydro-1H-inden-1-yloxy)-2,3-dihydrobenzofuran-3-yl)acetate

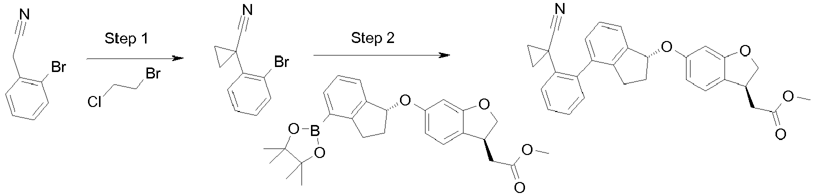

### Step 1: 1-(2-Bromophenyl)cyclopropanecarbonitrile

To a mixture of 2-(2-bromophenyl)acetonitrile (1.7 mL), 1-bromo-2-chloroethane (3.2 mL) and benzyltriethylammonium chloride (60 mg) is added dropwise at 0°C a solution of NaOH (10.6 g) in water (11 mL). The mixture is stirred for 15 minutes at 0°C and for 3 hours at 55°C. After cooling to room temperature the mixture is diluted with ethyl acetate and washed with water, 1 N hydrochloric acid, water and brine.

The organic phase is dried (MgSO₄) and concentrated to give the title compound. Yield: 2.9 g; LC (method 6): t_{R} = 1.44 min; Mass spectrum (ESI⁺): m/z = 222 [M+H]⁺. Step 2: Methyl 2-((3S)-6-((1R)-4-(2-(1-cyanocyclopropyl)phenyl)-2,3-dihydro-1H-inden-1-yloxy)-2,3-dihydrobenzofuran-3-yl)acetate

The title compound is prepared from methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate and 1-(2-bromophenyl)cyclopropanecarbonitrile following a procedure analogous to that described in Step 4 of Intermediate 13. The reaction temperature is 120°C. LC (method 4): t_{R} = 1.78 min; Mass spectrum (ESI⁺): m/z = 466 [M+H]⁺.

### Intermediate 15

### Methyl 2-((3S)-6-((1R)-4-(2,6-dimethyl-4-((tetrahydrofuran-2-yl)methoxy)phenyl)-7-fluoro-2,3-dihydro-1H-inden-1-yloxy)-2,3-dihydrobenzofuran-3-yl)acetate

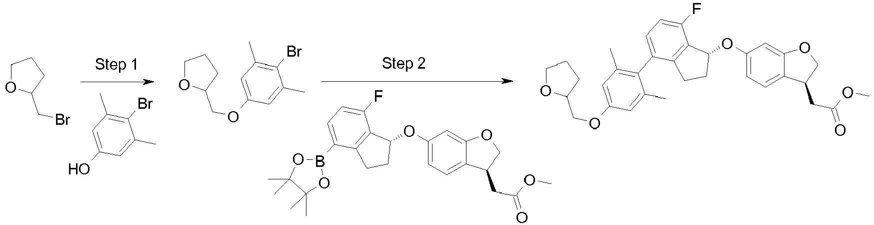

### Step 1: 2-((4-Bromo-3,5-dimethylphenoxy)methyl)tetrahydrofuran

The title compound is prepared from 4-bromo-3,5-dimethylphenol and 2-(bromomethyl)tetrahydrofuran following a procedure analogous to that described in Step 5 of Intermediate 1. LC (method 1): t_{R} = 1.37 min; Mass spectrum (ESI⁺): m/z = 285 [M+H]⁺.

### Step 2: Methyl 2-((3S)-6-((1R)-4-(2,6-dimethyl-4-((tetrahydrofuran-2-yl)methoxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-((4-bromo-3,5-dimethylphenoxy)methyl)-tetrahydrofuran following a procedure analogous to that described in Step 5 of Intermediate 12. LC (method 1): t_{R} = 1.48 min; Mass spectrum (ESI⁺): m/z = 569 [M+Na]⁺.

### Intermediate 16

### Methyl 2-((S)-6-((R)-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate

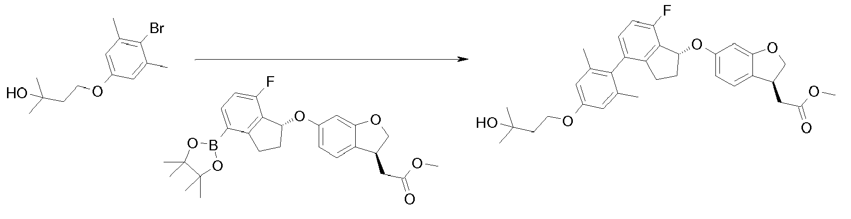

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenoxy)-2-methylbutan-2-ol following a procedure analogous to that described in Step 5 of Intermediate 12. LC (method 4): t_{R} = 1.90 min; Mass spectrum (ESI⁺): m/z = 571 [M+Na]⁺.

### Intermediate 17

### Methyl 2-((S)-6-((R)-6-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate

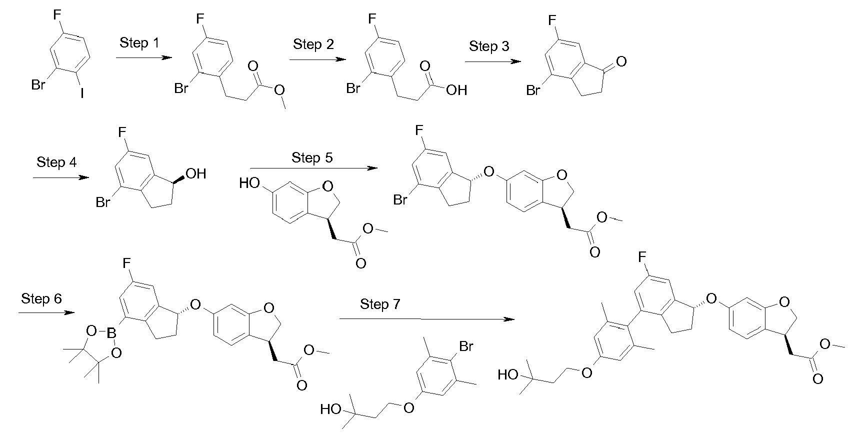

### Step 1: Methyl 3-(2-bromo-4-fluorophenyl)propanoate

To a solution of 2-bromo-4-fluoro-1-iodobenzene (3.1 g) and 3,3-dimethoxyprop-1-ene (1.8 mL) in N,N-dimethylformamide (40 mL) are added tetrabutylammonium chloride (2.9 g) and N-ethyldiisopropylamine (3.5 mL) and the mixture is purged for 10 minutes with argon. Palladium-(II)-acetate (118 mg) is added and the mixture is heated for 2 hours to 90°C. After cooling to room temperature the mixture is diluted with diethylether and washed with 1 N hydrochloric acid. The organic phase is dried (MgSO₄) and concentrated. The residue is chromatographed on silica gel (cyclohexane/ethyl acetate 95:5→90:10) to give the title compound (1.4 g), which is used directly in the next step.

### Step 2: 3-(2-Bromo-4-fluorophenyl)propanoic acid

To a solution of methyl 3-(2-bromo-4-fluorophenyl)propanoate (1.4 g) in tetrahydrofuran (10 mL) and methanol (10 mL) is added 4 M aqueous NaOH solution (10 mL) and the mixture is heated to 50°C for 3 hours. The organic solvents are evaporated in vacuo the residue is diluted with water and washed with tert.-butylmethyl-ether. The aqueous phase is neutralized by addition of 4 M hydrochloric acid (10 mL) and the mixture is stirred for 1 hour. The precipitate formed is filtered off, washed with water and dried to give the title compound. Yield: 1.2 g; LC (method 3): t_{R} = 0.71 min; Mass spectrum (ESI⁻): m/z = 245 [M-H]⁻.

### Step 3: 4-Bromo-6-fluoro-2,3-dihydro-1H-inden-1-one

Under argon 3-(2-bromo-4-fluorophenyl)propanoic acid (1.2 g) is added in small portions to trifluoromethanesulfonic acid (15 mL) at room temperature. The mixture is stirred for 2 hours at 60°C and for 16 hours at 80°C. After cooling to room temperature the mixture is poured slowly in ice-water. The aqueous phase is extracted twice with ethyl acetate. The combined organic phases are washed with saturated aqueous Na₂CO₃ solution and brine. After drying (MgSO₄) the solvent is evaporated. The residue is stirred with dichloromethane (40 mL), filtered and washed with dichloromethane. The combined dichloromethane phases are concentrated to give the title compound. Yield: 970 mg; LC (method 3): t_{R} = 0.60 min; Mass spectrum (ESI⁺): m/z = 229 [M+H]⁺.

### Step 4: (S)-4-Bromo-6-fluoro-2,3-dihydro-1H-inden-1-ol

The title compound is prepared from 4-bromo-6-fluoro-2,3-dihydro-1H-inden-1-one following a procedure analogous to that described in Step 1 of Intermediate 1. LC (method 3): t_{R} = 0.72 min; Mass spectrum (ESI⁻): m/z = 229 [M-H]⁻.

### Step5: Methyl 2-((S)-6-((R)-4-bromo-6-fluoro-2,3-dihydro-1H-inden-1-yloxy)-2,3-dihydrobenzofuran-3-yl)acetate

The title compound is prepared from (S)-4-bromo-6-fluoro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 3): t_{R} = 1.60 min; Mass spectrum (ESI⁺): m/z = 421 [M+H]⁺.

### Step6: Methyl 2-((S)-6-((R)-6-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate

The title compound is prepared from methyl 2-((S)-6-((R)-4-bromo-6-fluoro-2,3-dihydro-1H-inden-1-yloxy)-2,3-dihydrobenzofuran-3-yl)acetate following a procedure analogous to that described in Step 7 of Intermediate 1. LC (method 3): t_{R} = 1.70 min; Mass spectrum (ESI⁺): m/z = 469 [M+H]⁺.

### Step 7: Methyl 2-((S)-6-((R)-6-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate

The title compound is prepared from methyl 2-((S)-6-((R)-6-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenoxy)-2-methylbutan-2-ol following a procedure analogous to that described in Step 8 of Intermediate 1. LC (method 3): t_{R} = 1.68 min; Mass spectrum (ESI⁺): m/z = 549 [M+H]⁺.

### Intermediate 18

### Methyl 2-((S)-6-((R)-4-(2,6-dimethyl-4-((3-methyloxetan-3-yl)methoxy)pheny)-6-fluoro-2,3-dihydro-1H-inden-1-yloxy)-2,3-dihydrobenzofuran-3-yl)acetate

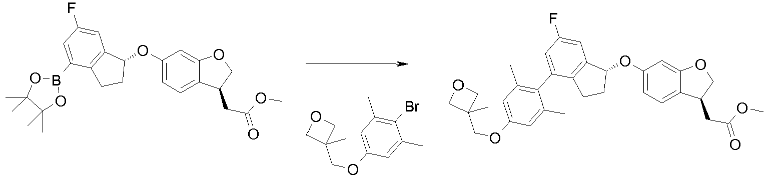

The title compound is prepared from methyl 2-((S)-6-((R)-6-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-((4-bromo-3,5-dimethylphenoxy)methyl)-3-methyloxetane following a procedure analogous to that described in Step 8 of Intermediate 1. LC (method 3): t_{R} = 1.68 min; Mass spectrum (ESI⁺): m/z = 547 [M+H]⁺.

### Intermediate 19

### Methyl 2-((S)-6-((R)-6-fluoro-4-(2-3-hydroxy-3-methylbutoxy)-4,6-dimethylpirimidin-5-yl-2,3ydro-1H-inden-1-yloxy)-2,3-dihydrobenzofuran-3-yl)acetate

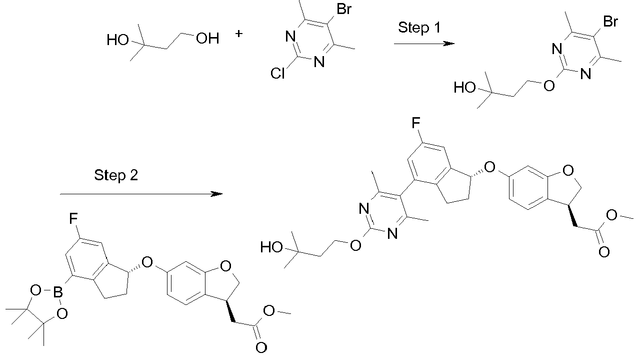

### Step 1: 4-(5-Bromo-4,6-dimethylpyrimidin-2-yloxy)-2-methylbutan-2-ol

Under argon 3-methylbutane-1,3-diol (650 µL) is added at 0°C to a suspension of NaH (ca. 60% in mineral oil; 400 mg). The mixture is stirred for 1 hour and then treated with 5-bromo-2-chloro-4,6-dimethylpyrimidine (1.2 g). After stirring for 2 hours at room temperature the mixture is diluted with ethyl acetate and washed with ice-water and brine. After drying (MgSO₄) the solvents are evaporated and the residue is chromatographed on silica gel (cyclohexane/ethyl acetate 70:39→50:50) to give the title compound. Yield: 1.1 g; LC (method 3): t_{R} = 0.79 min; Mass spectrum (ESI⁺): m/z = 289 [M+H]⁺.

### Step 2: Methyl 2-((S)-6-((R)-6-fluoro-4-(2-(3-hydroxy-3-methylbutoxy)-4,6-dimethylpyrimidin-5-yl)-2,3-dihydro-1H-inden-1-yloxy)-2,3-dihydrobenzofuran-3-yl)acetate

The title compound is prepared from methyl 2-((S)-6-((R)-6-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(5-bromo-4,6-dimethylpyrimidin-2-yloxy)-2-methylbutan-2-ol following a procedure analogous to that described in Step 8 of Intermediate 1. LC (method 3): t_{R} = 1.43 min; Mass spectrum (ESI⁺): m/z = 551 [M+H]⁺.

### Intermediate 20

### Methyl 2-((S)-6-((R)-4-(2-(3-hydroxy-3-methylbutoxy)-4,6-dimethylpyrimidin-5-yl)-2,3-dihydro-1H-inden-1-yloxy)-2,3-dihydrobenzofuran-3-yl)acetate

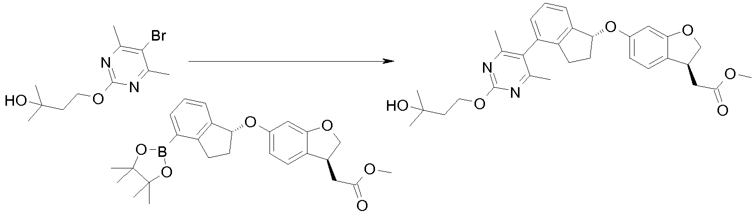

The title compound is prepared from methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(5-bromo-4,6-dimethylpyrimidin-2-yloxy)-2-methylbutan-2-ol following a procedure analogous to that described in Step 4 of Intermediate 10. The mixture is heated for 6 hours at 110°C. LC (method 3): t_{R} = 1.38 min; Mass spectrum (ESI⁺): m/z = 533 [M+H]⁺.

### Intermediate 21

### Methyl 2-((S)-6-((R)-4-(4,6-dimethyl-2-((3-methyloxetan-3-yl)methoxy)pyrimidin-5-yl)-2,3-dihydro-1H-inden-1-yloxy)-2,3-dihydrobenzofuran-3-yl)acetate

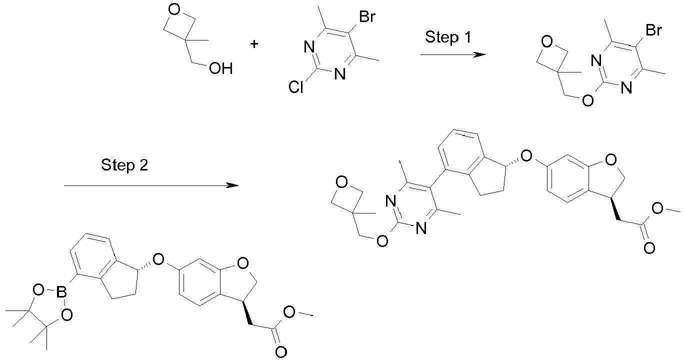

### Step1: 5-Bromo-4,6-dimethyl-2-((3-methyloxetan-3-yl)methoxy)pyrimidine

The title compound is prepared from (3-methyloxetan-3-yl)methanol and 5-bromo-2-chloro-4,6-dimethylpyrimidine following a procedure analogous to that described in Step 1 of Intermediate 19. LC (method 3): t_{R} = 0.74 min; Mass spectrum (ESI⁺): m/z = 287 [M+H]⁺.

### Step 2: Methyl 2-((S)-6-((R)-4-(4,6-dimethyl-2-((3-methyloxetan-3-yl)methoxy)pyrimidin-5-yl)-2,3-dihydro-1H-inden-1-yloxy)-2,3-dihydrobenzofuran-3-yl)acetate

The title compound is prepared from methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate and 5-bromo-4,6-dimethyl-2-((3-methyloxetan-3-yl)methoxy)pyrimidine following a procedure analogous to that described in Step 4 of Intermediate 10. The mixture is heated for 6 hours at 110°C. LC (method 3): t_{R} = 1.41 min; Mass spectrum (ESI⁺): m/z = 531 [M+H]⁺.

### Intermediate 22

### Methyl 2-((S)-6-((R)-4-(4,6-dimethyl-2-((3-methyloxetan-3-yl)methoxy)pyrimidin-5-yl)-6-fluoro-2,3-dihydro-1H-inden-1-yloxy)-2,3-dihydrobenzofuran-3-yl)acetate

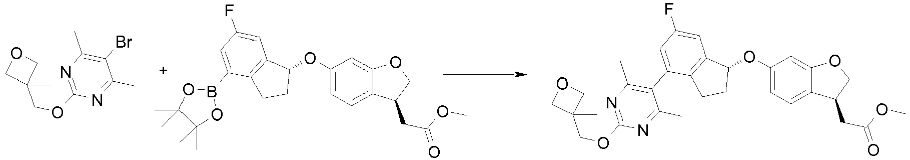

The title compound is prepared from methyl 2-((S)-6-((R)-6-fluoro-4-(4,4,5,5-tetramethyl-1,3,2-dioxaborolan-2-yl)-2,3-dihydro-1H-inden-1-yloxy)-2,3-dihydrobenzo furan-3-yl)acetate and 5-bromo-4,6-dimethyl-2-((3-methyloxetan-3-yl)methoxy)pyrimi dine following a procedure analogous to that described in Step 4 of Intermediate 10. The mixture is heated for 12 hours at 100°C. LC (method 3): t_{R} = 1.44 min; Mass spectrum (ESI⁺): m/z = 549 [M+H]⁺.

### Intermediate 23

### Methyl 2-((3S-6-((1R)-4-(2,6-dimethyl-4-(2-(methylsulfinyl)ethoxv)phenyl)-7-fluoro-2,3-dihydro-1H-inden-1-yloxy)-2,3-dihydrobenzofuran-3-yl)acetate

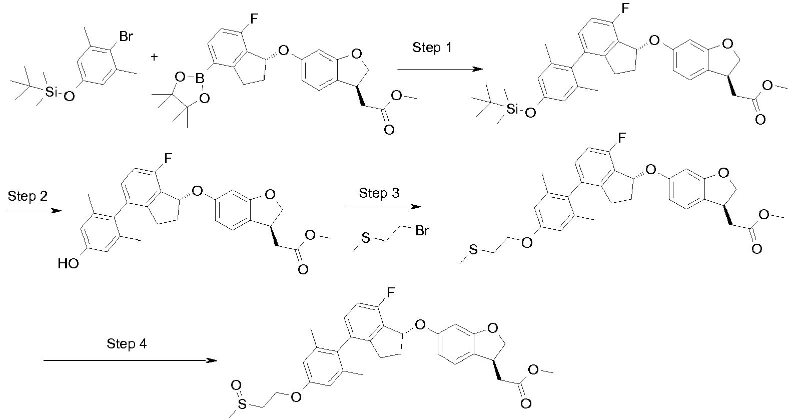

### Step 1: Methyl 2-((S)-6-((R)-4-(4-(tert-butyldimethylsilyloxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo-furan-3-yl)acetate and (4-bromo-3,5-dimethylphenoxy)(tert-butyl)dimethylsilane following a procedure analogous to that described in Step 5 of Intermediate 12. LC (method 4): t_{R} = 2.09 min; Mass spectrum (ESI⁺): m/z = 599 [M+Na]⁺.

### Step 2: Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate

The title compound is prepared from methyl 2-((S)-6-((R)-4-(4-(tert-butyldimethylsilyloxy)-2,6-dimethylphenyl)-7-fluoro-2,3-dihydro-1H-inden-1-yloxy)-2,3-dihydrobenzofuran-3-yl)acetate following a procedure analogous to that described in Step 4 of Intermediate 4. LC (method 4): t_{R} = 1.79 min; Mass spectrum (ESI⁺): m/z = 485 [M+ Na]⁺.

### Step 3: Methyl 2-((S)-6-((R)-4-(2,6-dimethyl-4-(2-(methylthio)ethoxy)phenyl)-7-fluoro-2,3-dihydro-1H-inden-1-yloxy)-2,3-dihydrobenzofuran-3-yl)acetate

To a solution of methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (280 mg) and 2-(methylthio)-ethanol (54 µL) in toluene (8 mL) are successively added 1,1'-(azodicarbonyl)-dipiperidine (240 mg) and tributylphosphine (240 µL). The mixture is stirred for 12 hours at room temperature, partitioned between saturated aqueous NaHCO₃ solution and dichloromethane and stirred for 30 minutes. The phases are separated and the organic phase is dried (MgSO₄). The solvents are evaporated and the residue is chromatographed on silica gel (cyclohexane/ethyl acetate 90:10→70:30) to give the title compound. Yield: 235 mg; LC (method 4): t_{R} = 1.97 min; Mass spectrum (ESI⁺): m/z = 537 [M+H]⁺.

### Step 4: Methyl 2-((3S)-6-((1 R)-4-(2,6-dimethyl-4-(2-(methylsulfinyl)ethoxy)phenyl)-7-fluoro-2,3-dihydro-1H-inden-1-yloxy)-2,3-dihydrobenzofuran-3-yl)acetate

To a solution of methyl 2-((S)-6-((R)-4-(2,6-dimethyl-4-(2-(methylthio)ethoxy)phenyl)-7-fluoro-2,3-dihydro-1H-inden-1-yloxy)-2,3-dihydrobenzofuran-3-yl)acetate (230 mg) in methanol (4 mL) and water (2 mL) is added potassium peroxomonoslufate (oxone^{®}) (490 mg). The mixture is stirred for 1 hour at room temperature and then partitioned between dichloromethane and water. The organic phase is dried (MgSO₄) and concentrated to give the crude product, which is directly in the next step. Yield: 220 mg; LC (method 4): t_{R} = 1.79 min; Mass spectrum (ESI⁺): m/z = 553 [M+H]⁺.

### Intermediate 24

### Methyl 2-((S)-6-((R)-4-(2,6-dimethyl-4-(tetrahydro-2H-pyran-4-yloxy)phenyl)-7-fluoro-2,3-dihydro-1H-inden-1-yloxy)-2,3-dihydrobenzofuran-3-yl)acetate

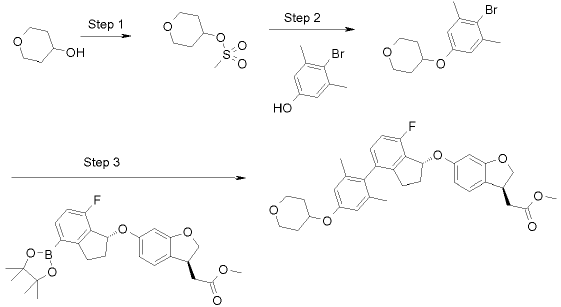

### Step 1: Tetrahydro-2H-pyran-4-yl methanesulfonate

To a solution of tetrahydro-2H-pyran-4-ol (20 g) in tetrahydrofuran (150 mL) and triethylamine (28.5 mL) is slowly added methanesulfonyl chloride (15.5 mL), while keeping the temperature below 30°C. The mixture is stirred for 12 hours at room temperature. The precipitate is filtered off and washed twice with tetrahydrofuran. The combined organic phases are concentrated and partitioned between ethyl acetate and water. The organic phase is dried (Na₂SO₄) and concentrated to give the title compound. Yield: 29.4 g; TLC: r_{f} = 0.36 (silicagel, petrole ether/ethyl acetate 1:1); Mass spectrum (ESI⁺): m/z = 198 [M+NH_{4]}⁺.

### Step 2: 4-(4-Bromo-3,5-dimethylphenoxy)tetrahydro-2H-pyran

To a solution of 4-bromo-3,5-dimethylphenol (3 g) in N-methyl-pyrrolidone (10 mL) is slowly added Cs₂CO₃ (9.7 g) and tetrahydro-2H-pyran-4-yl methanesulfonate (5.4 g). The mixture is heated for 3 hours at 140°C. After cooling to room temperature the mixture is partitioned between saturated aqueous Na₂CO₃ solution and ethyl acetate. The aqueous phase is extracted with ethyl acetate and the combined organic phases are dried (MgSO₄). The solvents are evaporated and the residue is purified by HPLC on reversed phase to give the title compound. Yield: 1.2 g; LC (method 4): t_{R}=1.83 min.

### Step3 Methyl 2-((S)-6-((R)-4-(2,6-dimethyl-4-(tetrahydro-2H-pyran-4-yloxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenoxy)tetrahydro-2H-pyran following a procedure analogous to that described in Step 5 of Intermediate 12. LC (method 4): t_{R} = 1.92 min; Mass spectrum (ESI⁺): m/z = 569 [M+Na]⁺.

### Intermediate 25

### Methyl 2-((S)-6-((R)-6-chloro-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate

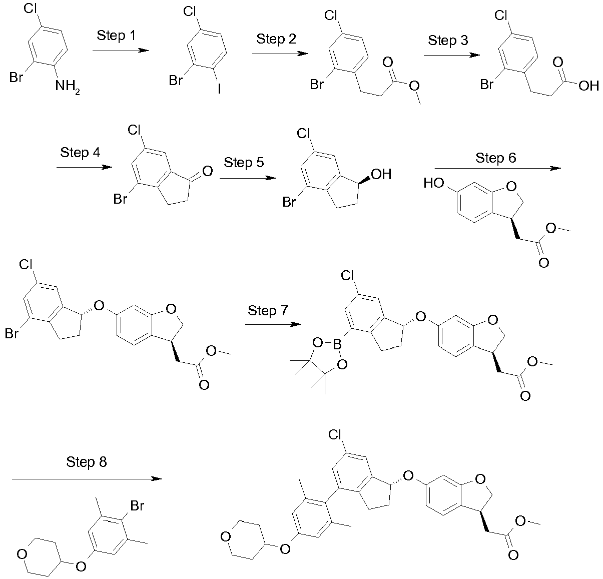

### Step 1: 2-Bromo-4-chloro-1-iodobenzene

To a solution of 2-bromo-4-chloroaniline (15 g) in concentrated hydrochloric acid (85 mL) is added at -15°C a solution of NaNO₂ (5.5 g) in water (10 mL) and the mixture is stirred for 30 minutes. Then the mixture is added at room temperature to a solution of KI (109 g) in water (200 mL). After stirring for 18 hours the mixture is partitioned between dichloromethane and 10% aqueous Na₂S₂O₃ solution. The organic phase is washed with 10% aqueous Na₂S₂O₃ solution and brine and concentrated to give the title compund. Yield: 16.4 g; Mass spectrum (El): m/z = 316 [M]⁺.

### Step 2: Methyl 3-(2-bromo-4-chlorophenyl)propanoate

The title compound is prepared from 2-bromo-4-chloro-1-iodobenzene following a procedure analogous to that described in Step 1 of Intermediate 17. LC (method 1): t_{R} = 1.31 min; Mass spectrum (ESI⁺): m/z = 277 [M+H]⁺.

### Step 3: 3-(2-Bromo-4-chlorophenyl)propanoic acid

To a solution of methyl 3-(2-bromo-4-chlorophenyl)propanoate (5 g) in tetrahydrofuran (20 mL) and methanol (20 mL) is added 4 M aqueous NaOH solution (35 mL) and the mixture is heated to 50°C for 3 hours. The organic solvents are evaporated in vacuo the residue is diluted with water and washed three times with ethyl acetate. The aqueous phase is neutralized by addition of 4 M hydrochloric acid (10 mL) and extracted three times with ethyl acetate. The combined organic phases are washed with brine and dried (Na₂SO₄). Evaporation of the solvents gives the title compound. Yield: 4.7 g; LC (method 1): t_{R} = 1.21 min.

### Step 4: 4-Bromo-6-chloro-2,3-dihydro-1H-inden-1-one

Oxalylchloride (3.6 mL) is added dropwise to a solution of 3-(2-bromo-4-chlorophenyl)propanoic acid (4.4 g) in dichloromethane (40 mL). The mixture is stirred for 2 hours at 40°C and then treated with AlCl₃ (2.7 g). The mixture is heated to reflux for 1 hour, cooled to room temperature and partitioned between ethyl acetate and saturated aqueous NaHCO₃ solution. The aqueous phase is extracted twice with ethyl acetate and the combined organic phases are washed with brine. After drying (Na₂SO₄) the solvents are evaporated. The residue is chromatographed on silica gel (cyclohexane/ethyl acetate 95:5→90:10) to give the title compound. Yield: 3.7 g; LC (method 1): t_{R} = 1.20 min; Mass spectrum (ESI⁺): m/z = 245 [M+H]⁺.

### Step 5: (S)-4-Bromo-6-chloro-2,3-dihydro-1H-inden-1-ol

The title compound is prepared from 4-bromo-6-fluoro-2,3-dihydro-1H-inden-1-one following a procedure analogous to that described in Step 1 of Intermediate 1. LC (method 1): t_{R} = 1.20 min; Mass spectrum (ESI⁻): m/z = 245 [M-H]⁻.

### Step6: Methyl 2-((S)-6-((R)-4-bromo-6-chloro-2,3-dihydro-1H-inden-1-yloxy)-2,3-dihydrobenzofuran-3-yl)acetate

The title compound is prepared from (S)-4-bromo-6-chloro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 1): t_{R} = 1.52 min; Mass spectrum (ESI⁺): m/z = 437 [M+H]⁺.

### Step 7: Methyl 2-((S)-6-((R)-6-chloro-4-(4,4,5,5-tetramethyl-1,3,2-dioxaborolan-2-yl)-2,3-dihydro-1H-inden-1-yloxy)-2,3-dihydrobenzofuran-3-yl)acetate

The title compound is prepared from methyl 2-((S)-6-((R)-4-bromo-6-chloro-2,3-dihydro-1H-inden-1-yloxy)-2,3-dihydrobenzofuran-3-yl)acetate following a procedure analogous to that described in Step 7 of Intermediate 1. LC (method 7): t_{R} = 1.31 min; Mass spectrum (ESI⁺): m/z = 485 [M+H]⁺.

### Step8: Methyl 2-((S)-6-((R)-6-chloro-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate

In a microwave vial methyl 2-((S)-6-((R)-6-chloro-4-(4,4,5,5-tetramethyl-1,3,2-dioxaborolan-2-yl)-2,3-dihydro-1H-inden-1-yloxy)-2,3-dihydrobenzofuran-3-yl)acetate (300 mg), 4-(4-bromo-3,5-dimethylphenoxy)tetrahydro-2H-pyran (194 mg), K₃PO₄ (394 mg) are suspended in toluene (3 mL) and water (0.5 mL) and purged for 10 minutes with argon. Dicyclohexyl(2',6'-dimethoxybiphenyl-2-yl)phosphine (S-Phos) (41 mg) and tris-(dibenzylidenaceton)-dipalladium-(0) (26 mg) are added, the vial is sealed and the mixture is stirred at 100°C for 12 hours. After cooling to room temperature the mixture is partitioned between ethyl acetate and saturated aqueous NH₄Cl solution. The organic phase is dried (MgSO₄) and concentrated. The residue is chromatographed on silica gel (cyclohexane/ethyl acetate 90:10→70:30) to give the title compound. Yield: 236 mg; LC (method 8): t_{R} = 1.00 min; Mass spectrum (ESI⁺): m/z = 563 [M+Na]⁺.

### Intermediate 26

### Methyl 2-((S)-6-((R)-4-(2,6-dimethyl-4-(tetrahydro-2H-pyran-4-yloxy)phenyl)-6,7-difluoro-2,3-dihydro-1H-inden-1-yloxy)-2,3-dihydrobenzofuran-3-yl)acetate

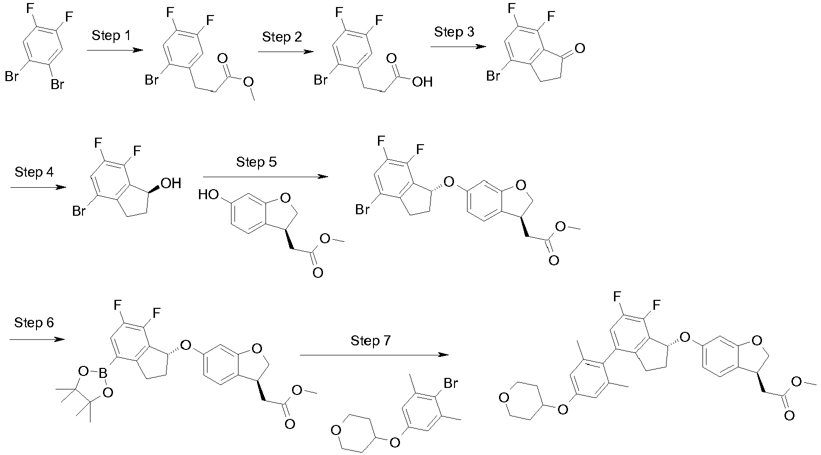

### Step 1: Methyl 3-(2-bromo-4,5-difluorophenyl)propanoate

The title compound is prepared from 1,2-dibromo-4,5-difluorobenzene following a procedure analogous to that described in Step 1 of Intermediate 17. The mixture is heated for 12 hours to 110°C. LC (method 4): t_{R} = 1.69 min.

### Step 2: 3-(2-Bromo-4,5-difluorophenyl)propanoic acid

The title compound is prepared from methyl 3-(2-bromo-4,5-difluorophenyl)propanoate following a procedure analogous to that described in Step 2 of Intermediate 17. The mixture is stirred for 24 hours at room temperature. LC (method 4): t_{R} = 1.56 min; Mass spectrum (ESI⁻): m/z = 263 [M-H]⁻.

### Step 3: 4-Bromo-6,7-difluoro-2,3-dihydro-1H-inden-1-one

The title compound is prepared from 3-(2-bromo-4,5-difluorophenyl)propanoic acid following a procedure analogous to that described in Step 4 of Intermediate 25. The mixture is heated for 1 hour to reflux and stirred for 12 hours at room temperature. LC (method 4): t_{R} = 1.44 min; Mass spectrum (ESI⁺): m/z = 247 [M+H]⁺.

### Step 4: (S)-4-Bromo-6,7-difluoro-2,3-dihydro-1H-inden-1-ol

The title compound is prepared from 4-bromo-6,7-difluoro-2,3-dihydro-1H-inden-1-one following a procedure analogous to that described in Step 1 of Intermediate 1. LC (method 4): t_{R} = 1.51 min.

### Step5: Methyl 2-((S)-6-((R)-4-bromo-6,7-difluoro-2,3-dihydro-1H-inden-1-yloxy)-2,3-dihydrobenzofuran-3-yl)acetate

The title compound is prepared from (S)-4-bromo-6,7-difluoro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 4): t_{R} = 1.98 min; Mass spectrum (ESI⁺): m/z = 439 [M+H]⁺.

### Step 6: Methyl 2-((S)-6-((R)-6,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate

The title compound is prepared from methyl 2-((S)-6-((R)-4-bromo-6,7-difluoro-2,3-dihydro-1H-inden-1-yloxy)-2,3-dihydrobenzofuran-3-yl)acetate following a procedure analogous to that described in Step 7 of Intermediate 1. LC (method 4): t_{R} = 1.96 min; Mass spectrum (ESI⁺): m/z = 487 [M+H]⁺.

### Step 7: Methyl 2-((S)-6-((R)-4-(2,6-dimethyl-4-(tetrahydro-2H-pyran-4-yloxy)phenyl)-6,7-difluoro-2,3-dihydro-1H-inden-1-yloxy)-2,3-dihydrobenzofuran-3-yl)acetate

The title compound is prepared from methyl 2-((S)-6-((R)-6,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenoxy)tetrahydro-2H-pyran following a procedure analogous to that described in Step 5 of Intermediate 12. LC (method 4): t_{R} = 1.94 min; Mass spectrum (ESI⁺): m/z = 587 [M+H]⁺.

### Intermediate 27

### Methyl 2-((S)-6-((R)-4-(2,6-dimethyl-4-((3-methyloxetan-3-yl)methoxy)phenyl)-6,7-difluoro-2,3-dihydro-1H-inden-1-yloxy)-2,3-dihydrobenzofuran-3-yl)acetate

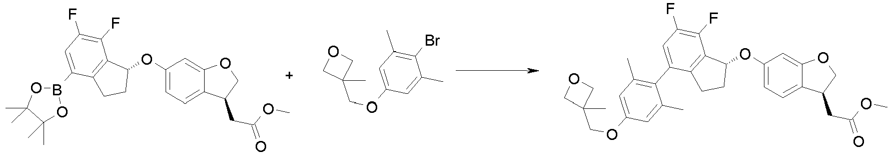

The title compound is prepared from methyl 2-((S)-6-((R)-6,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-((4-bromo-3,5-dimethylphenoxy)methyl)-3-methyloxetane following a procedure analogous to that described in Step 5 of Intermediate 12. LC (method 4): t_{R} = 1.94 min; Mass spectrum (ESI⁺): m/z = 587 [M+H]⁺.

### Intermediate 28

### Methyl 2-((S)-6-((R)-6-chloro-4-(2,6-dimethyl-4-((3-methyloxetan-3-yl)methoxy)phenyl)-2,3-dihydro-1H-inden-1-yloxy)-2,3-dihydrobenzofuran-3-yl)acetate

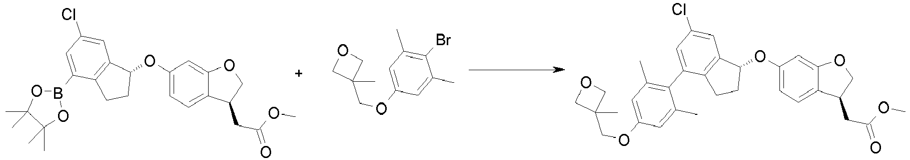

The title compound is prepared from methyl 2-((S)-6-((R)-6-chloro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-((4-bromo-3,5-dimethylphenoxy)methyl)-3-methyloxetane following a procedure analogous to that described in Step 8 of Intermediate 25. The mixture is heated for 4 hours to 100°C. LC (method 8): t_{R} = 1.00 min; Mass spectrum (ESI⁺): m/z = 563 [M+H]⁺.

### Intermediate 29

### Methyl 2-((S)-6-((R)-4-(2,6-dimethyl-4-((S)-tetrahydrofuran-3-yloxy)phenyl)-7-fluoro-2,3-dihydro-1H-inden-1-yloxy)-2,3-dihydrobenzofuran-3-yl)acetate

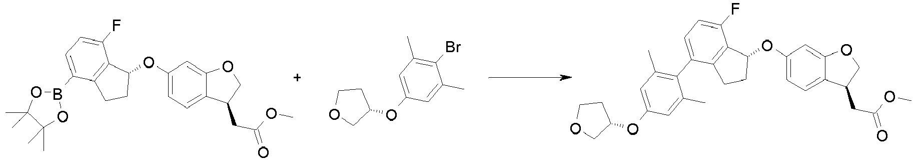

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and (S)-3-(4-bromo-3,5-dimethylphenoxy)tetrahydrofuran following a procedure analogous to that described in Step 5 of Intermediate 12. LC (method 4): t_{R} = 1.89 min; Mass spectrum (ESI⁺): m/z = 555 [M+Na]⁺.

### Intermediate 30

### Methyl 2-((S)-6-((R)-4-(2,6-dimethyl-4-((R)-tetrahydrofuran-3-yloxy)phenyl)-7-fluoro-2,3-dihydro-1H-inden-1-yloxy)-2,3-dihydrobenzofuran-3-yl)acetate

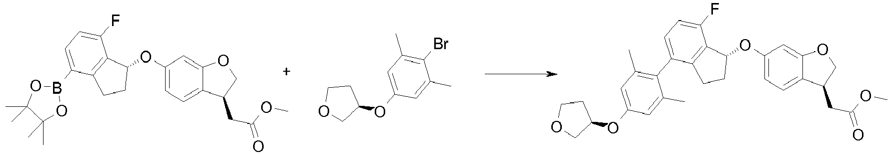

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and (R)-3-(4-bromo-3,5-dimethylphenoxy)tetrahydrofuran following a procedure analogous to that described in Step 5 of Intermediate 12. LC (method 4): t_{R} = 1.89 min; Mass spectrum (ESI⁺): m/z = 555 [M+Na]⁺.

### Intermediate 31

### Methyl 2-((S)-6-((R)-4-(2,6-dimethyl-4-((3-methyloxetan-3-yl)methoxy)phenyl-5-fluoro-2,3-dihydro-1H-inden-1-yloixy-2,3dihydrobenzofuran-3-yl)acetate

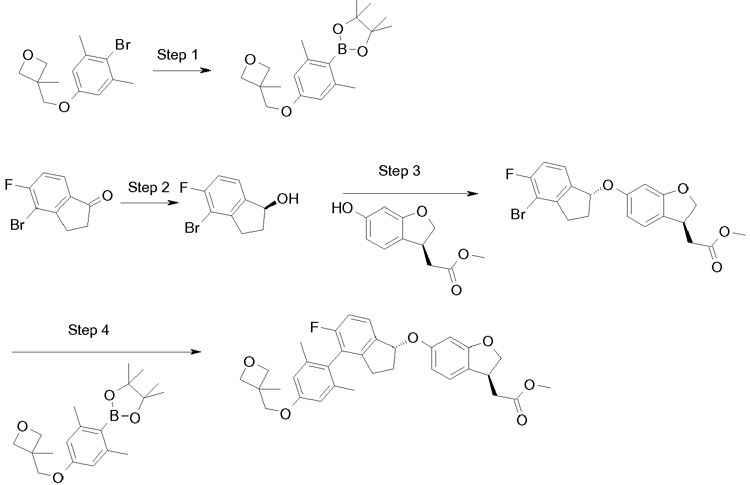

### Step 1: 2-(2,6-Dimethyl-4-((3-methyloxetan-3-yl)methoxy)phenyl)-4,4,5,5-tetramethyl-1,3,2-dioxaborolane

The title compound is prepared from 3-((4-bromo-3,5-dimethylphenoxy)methyl)-3-methyloxetane following a procedure analogous to that described in Step 7 of Intermediate 1. The mixture is heated for 12 hours to 100°C. Mass spectrum (ESI⁺): m/z = 333 [M+H]⁺.

### Step 2: (S)-4-Bromo-5-fluoro-2,3-dihydro-1H-inden-1-ol

The title compound is prepared from 4-bromo-5-fluoro-2,3-dihydro-1H-inden-1-one following a procedure analogous to that described in Step 1 of Intermediate 1. LC (method 4): t_{R} = 1.48 min.

### Step3: Methyl 2-((S)-6-((R)-4-bromo-5-fluoro-2,3-dihydro-1H-inden-1-yloxy)-2,3-dihydrobenzofuran-3-yl)acetate

The title compound is prepared from (S)-4-bromo-6,7-difluoro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 4): t_{R} = 1.89 min; Mass spectrum (ESI⁺): m/z = 421 [M+H]⁺.

### Step 4: Methyl 2-((S)-6-((R)-4-(2,6-dimethyl-4-((3-methyloxetan-3-yl)methoxy)phenyl)-5-fluoro-2,3-dihydro-1H-inden-1-yloxy)-2,3-dihydrobenzofuran-3-yl)acetate

The title compound is prepared from methyl 2-((S)-6-((R)-4-bromo-5-fluoro-2,3-dihydro-1H-inden-1-yloxy)-2,3-dihydrobenzofuran-3-yl)acetate and 2-(2,6-dimethyl-4-((3-methyloxetan-3-yl)methoxy)phenyl)-4,4,5,5-tetramethyl-1,3,2-dioxaborolane following a procedure analogous to that described in Step 5 of Intermediate 12. The reaction is run in the absence of water for 12 hours at 100°C. LC (method 7): t_{R} = 1.22 min; Mass spectrum (ESI⁺): m/z = 547 [M+H]⁺.

### Intermediate 32

### Methyl 2-((S)-6-((R)-6-cyano-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate

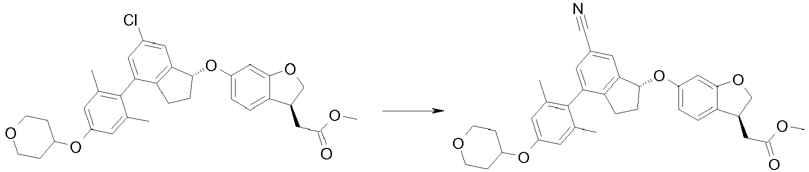

In a microwave vial NaCN (16 mg) is added to a solution of methyl 2-((S)-6-((R)-6-chloro-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate (90 mg) in N-methyl-2-pyrrolidinon (NMP) (2 mL) and the mixture is purged for 5 minutes with argon. NiBr₂ (35 mg) is added, the vial is sealed and the mixture is heated for 30 minutes to 180°C. The mixture is partitioned between water and ethyl acetate. The aqueous phase is extracted twice with ethyl acetate. The combined organic phases are washed with brine, dried (Na₂SO₄) and concentrated. The residue is chromatographed on silica gel (cyclohexane/ethyl acetate 90:10→70:30) to give the title compound. Yield: 34 mg; LC (method 7): t_{R} = 0.79 min.

### Intermediate 33

### Methyl 2-((3S)-6-((1R)-5-cyano-4-(2,6-dimethyl-4-((tetrahydrofuran-3-yl)methoxy)iphenyl)-2,3-dihydro-1H-inden-1-yloxy)-2,3-dihydrobenzofuran-3-yl)acetate

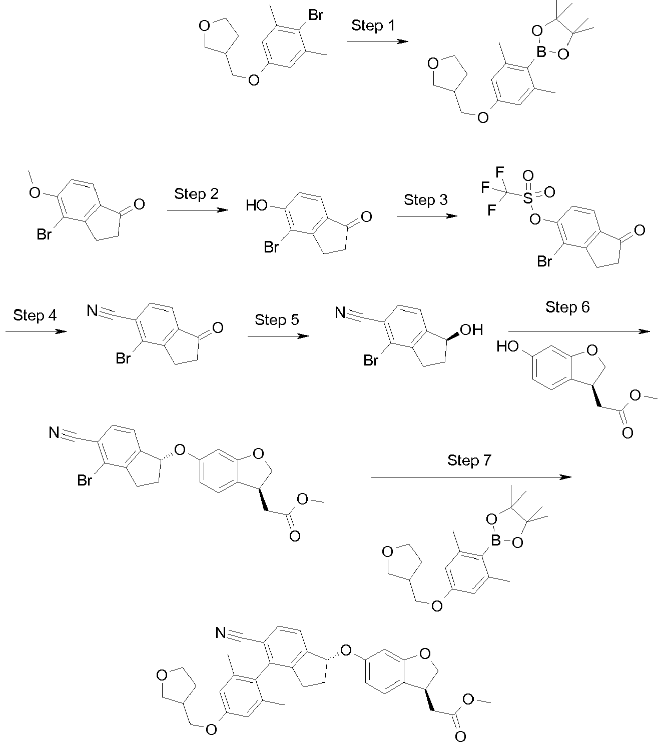

### Step 1: 2-(2,6-Dimethyl-4-((tetrahydrofuran-3-yl)methoxy)phenyl)-4,4,5,5-tetramethyl-1,3,2-dioxaborolane

The title compound is prepared from 3-((4-bromo-3,5-dimethylphenoxy)methyl)tetrahydrofuran following a procedure analogous to that described in Step 7 of Intermediate 1. The mixture is heated for 48 hours to 120°C. LC (method 1): t_{R} = 1.48 min; Mass spectrum (ESI⁺): m/z = 333 [M+H]⁺.

### Step 2: 4-Bromo-5-hydroxy-2,3-dihydro-1H-inden-1-one

In a microwave vial sodium methanthiolate (2 g) is added to a solution of 4-bromo-5-methoxy-2,3-dihydro-1H-inden-1-one in N,N-dimethylformamide (10 mL). The vial is sealed and the mixture is heated for 2 hours to 130°C. The mixture is partitioned between ethyl acetate and 1 N aqueous HCl solution. The aqueous phase is extracted with ethyl acetate and the combined organic phases are washed with brine and dried (MgSO₄). The solvents are evaporated and the residue is triturated with diethylether. The precipitate is filtered off and dried in vacuo to give the title compound. Yield: 1.15 g; LC (method 1): t_{R} = 0.84 min; Mass spectrum (ESI⁺): m/z = 227 [M+H]⁺.

### Step 3: 4-Bromo-1-oxo-2,3-dihydro-1H-inden-5-yl trifluoromethanesulfonate

4-Bromo-5-hydroxy-2,3-dihydro-1H-inden-1-one (5 g) and 2,6-dimethylpyridine (5.1 mL) are dissolved in dichloromethane (50 mL) cooled to 0°C and treated dropwise with trifluoromethanesulfonic anhydride (4 mL). The mixture is stirred for 2 hours at room temperature and then partitioned between saturated aqueous NH₄Cl solution and dichloromethane. The organic phase is washed with brine, dried (MgSO₄) and concentrated. The residue is chromatographed on silica gel (cyclohexane/ethyl acetate 99:1→50:50) to give the title compound. Yield: 5.9 g; LC (method 7): t_{R} = 1.08 min; Mass spectrum (ESI⁺): m/z = 359 [M+H]⁺.

### Step 4: 4-Bromo-1-oxo-2,3-dihydro-1H-indene-5-carbonitrile

Zn(CN)₂ (600 mg) and 1,1'-bis(diphenylphosphino)-ferrocene (920 mg) are added to a solution of 4-bromo-1-oxo-2,3-dihydro-1H-inden-5-yl trifluoromethanesulfonate (5.9 g) in N,N-dimethylformamide (30 mL) and the mixture is purged for 5 minutes with argon. Tris-(dibenzylidenaceton)-dipalladium-(0) (760 mg) is added and the mixture is heated for 1 hour to 70°C. The mixture is diluted with ethyl acetate and washed with water and brine. The organic phase is dried (MgSO₄) and concentrated. The residue is chromatographed on silica gel (cyclohexane/ethyl acetate 99:1→50:50) to give the title compound. Yield: 1.5 g; LC (method 7): t_{R} = 0.88 min; Mass spectrum (ESI⁻): m/z = 234 [M-H]⁻.

### Step5: (S)-4-Bromo-1-hydroxy-2,3-dihydro-1H-indene-5-carbonitrile

The title compound is prepared from 4-bromo-1-oxo-2,3-dihydro-1H-indene-5-carbonitrile following a procedure analogous to that described in Step 1 of Intermediate 1. LC (method 7): t_{R} = 0.84 min; Mass spectrum (ESI⁺): m/z = 260 [M+Na]⁺.

### Step6: Methyl 2-((S)-6-((R)-4-bromo-5-cyano-2,3-dihydro-1H-inden-1-yloxy)-2,3-dihydrobenzofuran-3-yl)acetate

The title compound is prepared from (S)-4-bromo-1-hydroxy-2,3-dihydro-1H-indene-5-carbonitrile and (S)-methyl 2-(6-hydroxy-2,3-dihydrobenzofuran-3-yl)acetate following a procedure analogous to that described in Step 3 of Intermediate 12. LC (method 7): t_{R} = 1.15 min; Mass spectrum (ESI⁺): m/z = 428 [M+H]⁺.

### Step 7: Methyl 2-((3S)-6-((1R)-5-cyano-4-(2,6-dimethyl-4-((tetrahydrofuran-3-yl)methoxy)phenyl)-2,3-dihydro-1H-inden-1-yloxy)-2,3-dihydrobenzofuran-3-yl)acetate

The title compound is prepared from methyl 2-((S)-6-((R)-4-bromo-5-cyano-2,3-dihydro-1H-inden-1-yloxy)-2,3-dihydrobenzofuran-3-yl)acetate and 2-(2,6-dimethyl-4-((tetrahydrofuran-3-yl)methoxy)phenyl)-4,4,5,5-tetramethyl-1,3,2-dioxaborolane following a procedure analogous to that described in Step 8 of Intermediate 25. The reaction is run for 48 hours at 100°C. LC (method 8): t_{R} = 0.73 min; Mass spectrum (ESI⁺): m/z = 554 [M+H]⁺.

### Intermediate 33a

### ((S)-6-1(R)-5-Cyano-4-[4-(3-methanesulfonyl-propoxy)-2,6-dimethyl-lphenyl]-indan-1-yloxyl-2,3-dihydro-benzofuran-3-yl)-acetic acid methyl ester

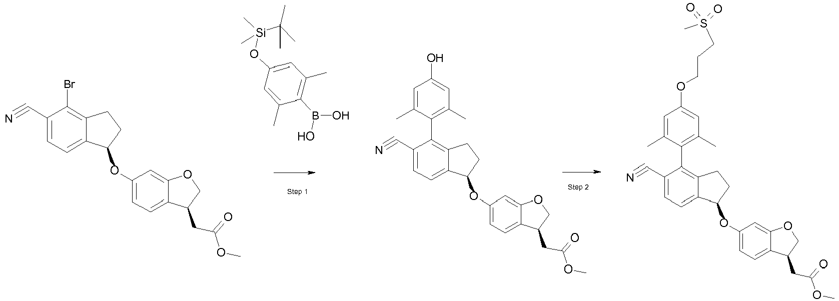

### Stet 1: {(S)-6-[(R)-5-Cyano-4-(4-hydroxy-2,6-dimethyl-phenyl)-indan-1-yloxy]-2,3-dihydro-benzofuran-3-yl}-acetic acid methyl ester

Methyl 2-((S)-6-((R)-4-bromo-5-cyano-2,3-dihydro-1H-inden-1-yloxy)-2,3-dihydroben zofuran-3-yl)acetate (1,7 g), (4-{[tert-butyl(dimethyl)silyl]oxy}-2,6-dimethylphe nyl)boronic acid (EP1726580A1,2006) (2.22 g), 2-dicyclohexylphosphino-2'-(N,N-dimethylamino)biphenyl (85 mg), tris(dibenzylideneacetone)dipalladium(0) (36 mg) and sodium carbonate (120 mg) are stirred in toluene (40 mL) and water (5 mL) at 110 °C for 12 h. The reaction mixture is concentrated under vacuum and the crude product obtained is purified by flash chromatography (cyclohexane/ethyl acetate 100;0→50:50) to give the title compound. Yield: 450 mg.

### Step 2: ((S)-6-{(R)-5-Cyano-4-[4-(3-methanesulfonyl-propoxy)-2,6-dimethyl-phenyl]-indan-1-yloxy}-2,3-dihydro-benzofuran-3-yl)-acetic acid methyl ester

{(S)-6-[(R)-5-Cyano-4-(4-hydroxy-2,6-dimethyl-phenyl)-indan-1-yloxy]-2,3-dihydro-benzofuran-3-yl}-acetic acid methyl ester (112 mg), methanesulfonic acid 3-methanesulfonyl-propyl ester (Moltzen, Ejner K et al. Journal of Medicinal Chemistry, 1995 , vol. 38, # 11 p. 2009 - 2017) (73.5 mg) and cesium carbonate (150 mg) are stirred in 2 mL of N,N-dimethylformamide for 3 h. The reaction mixture is concentrated under vacuum, the residue obtained is dissolved in dichloromethane and the organic phase is washed with water, dried over sodium sulfate and concentrated under vacuum The residue is purified by flash chromatography (cyclohexane/ethyl acetate 100:0→70.30) to give the title compound. Yield: 45 mg.

### Intermediate 33b

### ((S)-6-1(R)-5-Cyano-4-[4-(1,1-dioxo-hexahydro-1-thiopyran-4-ylmethoxy)-2,6-dimethyl-phenyl]-indan-1-yloxy}-2,3-dihydro-benzofuran-3-yl)-acetic acid methyl ester

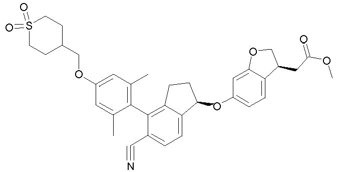

The title compound is prepared in analogy to Step 2 of Intermediate 33a starting from intermediate 49ga.

### Intermediate 33c

### ((S)-6-1(R)-5-Cyano-4-[4-((R)-1-methanesulfonyl-lpyrrolidin-3-yloxy)-2,6-dimethylphenyl]-indan-1-yloxy}-2,3-dihydro-benzofuran-3-yl)-acetic acid methyl ester

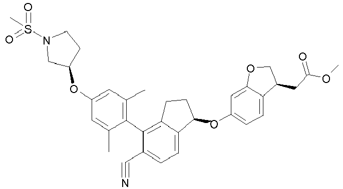

The title compound is prepared in analogy to Step 2 of Intermediate 33a starting from intermediate 49ea.

### Intermediate 34

### Methyl 2-((S)-6-((R)-7-fluoro-4-(4-(2-hydroxy-2-methylpropoxy)-2,6-dimethylphenyl)-2,3-dihydro-1H-inden-1-yloxy)-2,3-dihydrobenzofuran-3-yl)acetate

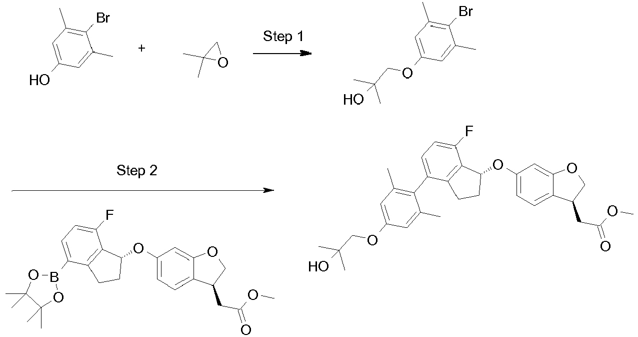

### Step 1: 1-(4-Bromo-3,5-dimethylphenoxy)-2-methylpropan-2-ol

In a microwave vial 2,2-dimethyloxirane (2.2 g) is added to a suspension of 4-bromo-3,5-dimethylphenol and K₂CO₃ (11 g) in N,N-dimethylformamide (6 mL). The vial is sealed and the mixture is heated for 48 hours to 120°C. After cooling to room temperature the mixture is partitioned between saturated aqueous Na₂CO₃ solution and ethyl acetate. The aqueous phase is extracted twice with ethyl acetate and the combined organic phases are dried (MgSO₄) and concentrated. The residue is chromatographed on silica gel (cyclohexane/ethyl acetate 100:0→50:50) to give the title compound. Yield: 2.9 g; LC (method 4): t_{R} = 1.76 min; Mass spectrum (ESI⁺): m/z = 273 [M+H]⁺.

### Step 2: Methyl 2-((S)-6-((R)-7-fluoro-4-(4-(2-hydroxy-2-methylpropoxy)-2,6-dimethylphenyl)-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 1-(4-bromo-3,5-dimethylphenoxy)-2-methylpropan-2-ol following a procedure analogous to that described in Step 5 of Intermediate 12. LC (method 8): t_{R} = 0.77 min; Mass spectrum (ESI⁺): m/z = 535 [M+H]⁺.

### Intermediate 35

### Methyl 2-((3S)-6-((1R)-7-fluoro-4-(4-(3-hydroxycyclopentyloxy)-2,6-dimethyl)phenyl)-2,3-dihydro-1H-inden-1-yloxy)-2,3-dihydrobenzofuran-3-yl)acetate

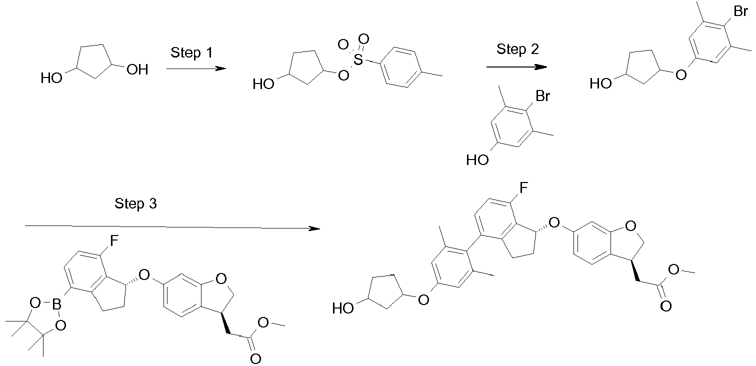

### Step 1: 3-Hydroxycyclopentyl 4-methylbenzenesulfonate

The title compound is prepared from cyclopentane-1,3-diol following a procedure analogous to that described in Step 4 of Intermediate 1. The product is purified by chromatography on silica gel (petrole ether/ethyl acetate 90:10→10:90) to give the title compound. LC (method 7): t_{R} = 0.86 min; Mass spectrum (ESI⁺): m/z = 257 [M+H]⁺.

### Step 2: 3-(4-Bromo-3,5-dimethylphenoxy)cyclopentanol

The title compound is prepared from 3-hydroxycyclopentyl 4-methylbenzenesulfonate and 4-bromo-3,5-dimethylphenol following a procedure analogous to that described in Step 5 of Intermediate 1. LC (method 7): t_{R} = 1.07 min; Mass spectrum (ESI⁺): m/z = 285 [M+H]⁺.

### Step 3: Methyl 2-((3S)-6-((1 R)-7-fluoro-4-(4-(3-hydroxycyclopentyloxy)-2,6-dimethylphenyl)-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-(4-bromo-3,5-dimethylphenoxy)cyclopentanol following a procedure analogous to that described in Step 5 of Intermediate 12. Mass spectrum (ESI⁺): m/z = 547 [M+H]⁺.

### Intermediate 36

### 2-((S)-6-((R)-7-Fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid

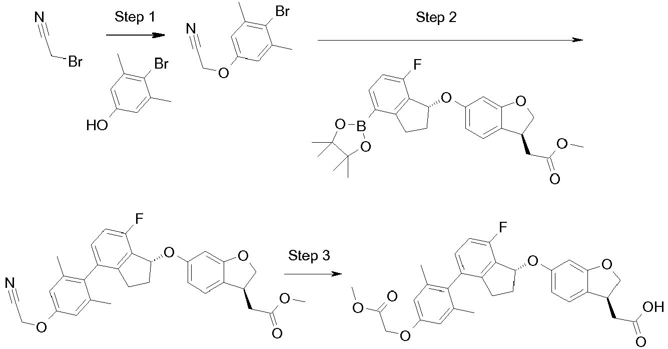

### Step 1: 2-(4-Bromo-3,5-dimethylphenoxy)acetonitrile

To a solution of 4-bromo-3,5-dimethylphenol (500 mg) and Cs₂CO₃ (1.3 g) in N,N-dimethylformamide (5 mL) is added 2-bromoacetonitrile (290 µL) and the mixture is stirred for 2 hours at 35°C. The mixture is poured on waterand stirred for 15 minutes. The precipitate is folitered off and dried to give the title compound. Yield: 535 mg; Mass spectrum (EI): m/z = 239 [M]⁺.

### Step 2: Methyl 2-((S)-6-((R)-4-(4-(cyanomethoxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-(4-bromo-3,5-dimethylphenoxy)acetonitrile following a procedure analogous to that described in Step 5 of Intermediate 12. LC (method 8): t_{R} = 0.71 min; Mass spectrum (ESI⁺): m/z = 502 [M+H]⁺.

### Step3: 2-((S)-6-((R)-7-Fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid

A 1 M aqueous NaOH solution (400 µL) is added to a solution of methyl 2-((S)-6-((R)-4-(4-(cyanomethoxy)-2,6-dimethylphenyl)-7-fluoro-2,3-dihydro-1H-inden-1-yloxy)-2,3-dihydrobenzofuran-3-yl)acetate (120 mg) in methanol (4 mL). The mixture is stirred for 12 hours at room temperature. Methanol is evaporated off in vacuo and the residue is diluted with water. 1 M hydrochloric acid (400 µL) is added and the aqueous phase is extracted twice with dichloromethane The combined organic phases are dried (MgSO₄) and concentrated. The residue is chromatographed on silica gel (petrole ether/ethyl acetate 60:40→0:100). The product thus obtained is crystallized from n-hexane to give the title compound. Yield: 75 mg; LC (method 7): t_{R} = 0.99 min; Mass spectrum (ESI⁺): m/z = 521 [M+H]⁺.

### Intermediate 37

### Methyl 2-((S)-6-((R)-7-fluoro-4-(4-(3-hydroxy-2,2-dimethylpropoxy)-2,6-dimethylphenyl)-2,3-dihydro-1H-inden-1-yloxy)-2,3-dihydrobenzofuran-3-yl)acetate

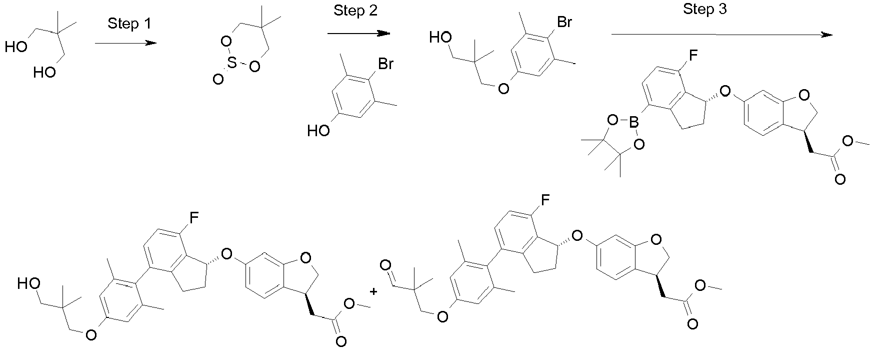

### Step 1: 5,5-Dimethyl-[1,3,2]dioxathiane 2-oxide

Thionylchloride (735 mL) is added to a solution of 2,2-dimethylpropane-1,3-diol (1 g) in dichloromethane (10 mL) chilled in an ice bath. The mixture is heated to 40°C for 3 hours and partitioned between saturated aqueous NaHCO₃ solution and dichloromethane. The organic phase is wahed with brine, dried (MgSO₄) and concentrated to give the title compound. Yield: 535 mg; TLC: r_{f} = 0.65 (silicagel, cyclohexane/ethyl acetate 3:1).

### Step2: 3-(4-Bromo-3,5-dimethylphenoxy)-2,2-dimethylpropan-1-ol

In a microwave vial Cs₂CO₃ (5.4 g) is added to a soltuion of 5,5-dimethyl-[1,3,2]dioxathiane 2-oxide (500 mg) and 4-bromo-3,5-dimethylphenol (670 mg) in N,N-dimethylformamide (5 mL). The vial is sealed and the mixture is heated to 120°C for 12 hours. The mixture is partitioned between ethyl acetate and water. The organic phase is washed three times with 2 M aqueous NaOH and with brine. After drying (MgSO₄) and concentration the residue is chromatographed on silica gel (cyclohexane/ethyl acetate 99:1→50:50) to give the title compound. Yield: 372 mg; LC (method 7): t_{R} = 1.17 min; Mass spectrum (ESI⁺): m/z = 287 [M+H]⁺.

### Step3: Methyl 2-((S)-6-((R)-7-fluoro-4-(4-(3-hydroxy-2,2-dimethylpropoxy)-2,6-dimethylphenyl)-2,3-dihydro-1H-inden-1-yloxy)-2,3-dihydrobenzofuran-3-yl)acetate

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (250 mg), 3-(4-bromo-3,5-dimethylphenoxy)-2,2-dimethylpropan-1-ol (226 mg), K₃PO₄ (230 mg) are suspended in toluene (2 mL) and water (200 µL) and purged for 10 minutes with argon. Palladium-(II)-acetate (12 mg) and dicyclohexyl(2',6'-dimethoxybiphenyl-2-yl)phosphine (S-Phos) (44 mg) are added, the vial is sealed and the mixture is stirred at 100°C for 12 hours. After cooling to room temperature the mixture is partitioned between ethyl acetate and water. The organic phase is washed with brine, dried (MgSO₄) and concentrated. The residue is chromatographed on silica gel (cyclohexane/ethyl acetate 99:1→60:40) to give the title compound. Yield: 99 mg; LC (method 8): t_{R} = 0.87 min; Mass spectrum (ESI⁺): m/z = 549 [M+H]⁺.

Additionally methyl 2-((S)-6-((R)-4-(4-(2,2-dimethyl-3-oxopropoxy)-2,6-dimethylphenyl)-7-fluoro-2,3-dihydro-1H-inden-1-yloxy)-2,3-dihydrobenzofuran-3-yl)acetate is isolated. Yield: 83 mg; LC (method 8): t_{R} = 0.95 min; Mass spectrum (ESI⁺): m/z = 547 [M+H]⁺.

### Intermediate 38

### Methyl 2-((S)-6-((R)-7-fluoro-4-(4-(3-methoxy-2,2-dimethylpropoxy)-2,6-ydimethylphenyl)-2,3-dihydro-1H-inden-1-yloxy)-2,3-dihydrobenzofuran-3-yl)acetate

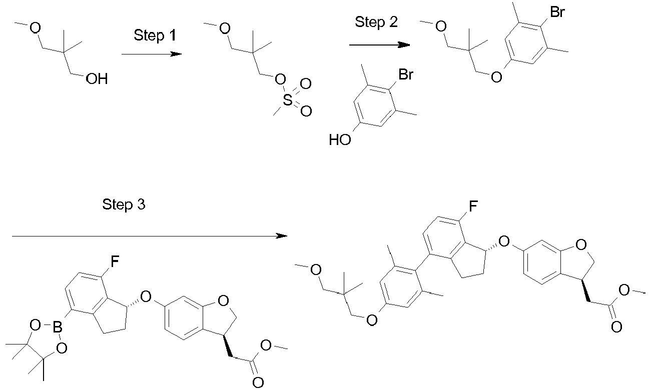

### Step 1: 3-Methoxy-2,2-dimethylpropyl methanesulfonate

Methanesulfonyl chloride (330 mL) is added dropwise to a solution of 3-methoxy-2,2-dimethylpropan-1-ol (250 mg) and N,N-diisopropylethylamine (1.1 mL) in dichloromethane (3 mL) chilled in an ice bath. The mixture is stirred at room temperature for 12 hours and partitioned between saturated aqueous NaHCO₃ solution and dichloromethane. The organic phase is wahed with twice with 1 N hydrochloric acid, saturated aqueous NaHCO₃ solution and brine. Then the organic phase is dried (MgSO₄) and concentrated to give the title compound. Yield: 407 mg; TLC: r_{f} = 0.75 (silicagel, cyclohexane/ethyl acetate 3:1).

### Step 2: 2-Bromo-5-(3-methoxy-2,2-dimethylpropoxy)-1,3-dimethylbenzene

In a microwave vial Cs₂CO₃ (3.3 g) and Kl (50 mg) are added to a soltuion of 3-methoxy-2,2-dimethylpropyl methanesulfonate (407 mg) and 4-bromo-3,5-dimethylphenol (415 mg) in N,N-dimethylformamide (5 mL). The vial is sealed and the mixture is heated to 120°C for 12 hours. The mixture is partitioned between ethyl acetate and water. The organic phase is washed with water and brine and is dried (MgSO₄). The solvents are evaporated and the residue is chromatographed on silica gel (cyclohexane/ethyl acetate 99:1→80:20). The product thus obtained is purified by HPLC on reversed phase to give the title compound. Yield: 171 mg; LC (method 8): t_{R} = 1.02 min; Mass spectrum (ESI⁺): m/z = 301 [M+H]⁺.

### Step3: Methyl 2-((S)-6-((R)-7-fluoro-4-(4-(3-methoxy-2,2-dimethylpropoxy)-2,6-dimethylphenyl)-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-bromo-5-(3-methoxy-2,2-dimethylpropoxy)-1,3-dimethylbenzene following a procedure analogous to that described in Step 5 of Intermediate 12. LC (method 8): t_{R} = 1.12 min; Mass spectrum (ESI⁺): m/z = 563 [M+H]⁺.

### Intermediate 39

### Methyl 2-((S)-6-((R)-5-cyano-4-(2,6-dimethyl-4-((3-methyloxetan-3-yl)methoxy)phe nyl)-2,3-dihydro-1H-inden-1-yloxy)-2,3-dihydrobenzofuran-3-yl)acetate

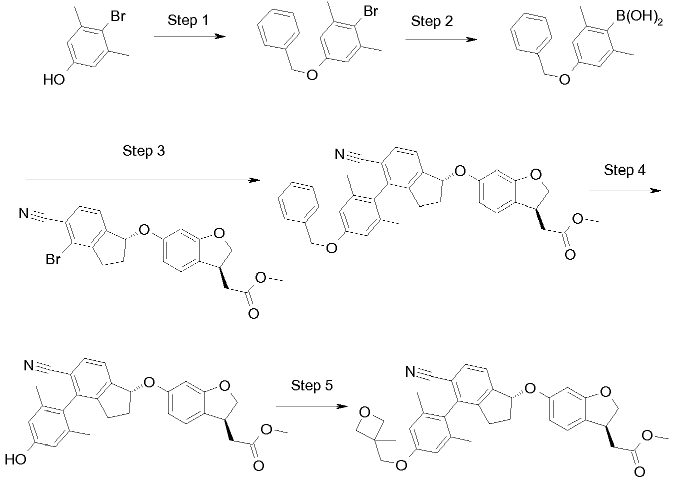

### Step 1: 5-(Benzyloxy)-2-bromo-1,3-dimethylbenzene

K₂CO₃ (1,75 g) is added to a solution of 4-bromo-3,5-dimethylphenol (2 g) and benzylbromide (1.43 mL) in N,N-dimethylformamide (3 mL) and the mixture is heated to 80°C for 12 hours. The mixture is diluted with ethyl acetate, washed with water and dried (MgSO₄). The solvents are evaporated and the residue is chromatograpehd on silica gel (petrole ether/ethyl acetate 98:2→80:20) to give the title compound. Yield: 2.38 g; TLC: r_{f} = 0.50 (silicagel, petrole ether/ethyl acetate 9:1); Mass spectrum (ESI⁺): m/z = 291 [M+H]⁺.

### Step 2: 4-(Benzyloxy)-2,6-dimethylphenylboronic acid

Under argon 5-(benzyloxy)-2-bromo-1,3-dimethylbenzene (2,38 g) is dissolved in tetrahydrofuran (30 mL) and cooled to -78°C. n-Butyllithium (6.2 mL of a 1.6 M solution in tetrahydrofuran) is added dropwise, the mixture is stirred for 4 hours followed by dropwise addition of triisopropylborate (5.7 mL). The mixture is stirred for 12 hours while warming to room temperature. Then the mixture is poured on 2 N hydrochloric acid and stirred vigorously for 30 minutes. The phases are separated and the aqueous phase is extracted with ethyl acetate. The combined organic phases are washed with brine and dried (MgSO₄). The solvents are evaporated and he residue is crystallized from n-hexane. The precipitate is filtered off and dried ti give the title compound. Yield: 1.38 g; LC (method 7): t_{R} = 0.94 min; Mass spectrum (ESI⁻): m/z = 255 [M-H]⁻.

### Step 3: Methyl 2-((S)-6-((R)-4-(4-(benzyloxy)-2,6-dimethylphenyl)-5-cyano-2,3-dihydro-1H-inden-1-yloxy)-2,3-dihydrobenzofuran-3-yl)acetate

In a microwave vial methyl 2-((S)-6-((R)-4-bromo-5-cyano-2,3-dihydro-1H-inden-1-yloxy)-2,3-dihydrobenzofuran-3-yl)acetate (130 mg), 4-(benzyloxy)-2,6-dimethylphenylboronic acid (120 mg), dicyclohexyl(2',6'-dimethoxybiphenyl-2-yl)phosphine (S-Phos) (18 mg) and K₃PO₄ (200 mg) are suspended in toluene (2 mL) and purged for 10 minutes with argon. Palladium-(II)-acetate (5 mg) is added, the vial is sealed and the mixture is stirred at 100°C for 12 hours. After cooling to room temperature the mixture is partitioned between diethylether and saturated aqueous NH₄Cl solution. The organic phase is dried (MgSO₄) and concentrated. The residue is chromatographed on silica gel (cyclohexane/ethyl acetate 90:10→40:60) to give the title compound. Yield: 125 mg; LC (method 10): t_{R} = 1.15 min; Mass spectrum (ESI⁺): m/z = 560 [M+H]⁺.

### Step 4: Methyl 2-((S)-6-((R)-5-cyano-4-(4-hydroxy-2,6-dimethylphenyl)-2,3-dihydro-1 H-inden-1-yloxy)-2,3-dihydrobenzofuran-3-yl)acetate

To a solution of methyl 2-((S)-6-((R)-4-(4-(benzyloxy)-2,6-dimethylphenyl)-5-cyano-2,3-dihydro-1H-inden-1-yloxy)-2,3-dihydrobenzofuran-3-yl)acetate (120 mg) in methanol (10 mL) is added 10% palladium on activated carbon (20 mg) and the mixture is hydrogenated at a pressure of 2 bar for 2 hours. The catalyst is filtered off and washed with methanol. The combined mother liquors are concentrated to give the title compound. Yield: 84 mg; Mass spectrum (ESI⁺): m/z = 470 [M+H]⁺.

### Step5: Methyl 2-((S)-6-((R)-5-cyano-4-(2,6-dimethyl-4-((3-methyloxetan-3-yl)methoxy)phenyl)-2,3-dihydro-1H-inden-1-yloxy)-2,3-dihydrobenzofuran-3-yl)acetate

To a solution of methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (80 mg) and Cs₂CO₃ (120 mg) in N,N-dimethylformamide (2 mL) is added 3-(bromomethyl)-3-methyloxetane (30 µL). The mixture is stirred for 12 hours at room temperature and then partitioned between saturated aqueous NH₄Cl solution and diethylether. The organic phase is dried (MgSO₄) and concentrated. The residue is chromatographed on silica gel (cyclohexane/ethyl acetate 90:10→10:90) to give the title compound. Yield: 50 mg; LC (method 9): t_{R} = 1.21 min; Mass spectrum (ESI⁺): m/z = 554 [M+H]⁺.

### Intermediate 40

### Methyl 2-((S)-6-((R)-4-(2,6-dimethyl-4-(pyrimidin-5-yl)phenyl)-7-fluoro-2,3-dihydro-1H-inden-1-yloxy)-2,3-dihydrobenzofuran-3-yl)acetate

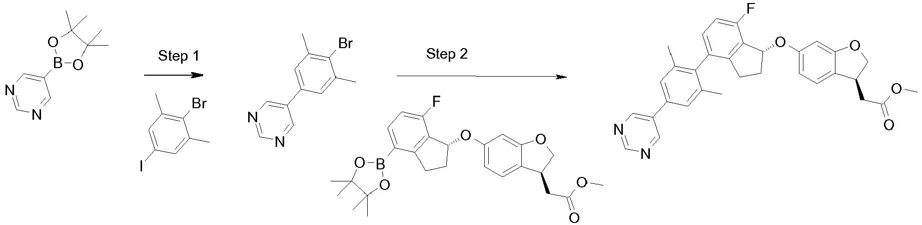

### Step1: 5-(4-Bromo-3,5-dimethylphenyl)pyrimidine

In a microwave vial 2-bromo-5-iodo-1,3-dimethylbenzene (1 g) and 5-(4,4,5,5-tetramethyl-1,3,2-dioxaborolan-2-yl)pyrimidine (665 mg) are suspended in N,N-dimethylformamide (15 mL) and Na₂CO₃ (4 mL of a 2 M aqueous solution). The mixture is purged for 5 minutes with argon. [1,1'-Bis(diphenylphosphino)-ferrocene]-dichloropalladium dichloromethane complex (85 mg) is added, the vial is sealed and the mixture is stirred at 60°C for 3 hours. After cooling to room temperature the mixture is Partitioned between water and ethyl acetate. The aqueous phase is extracted with ethyl acetate and the combined organic phases are washed with brine. Then the organic phase is dried (MgSO₄) and concentrated. The residue is chromatographed on silica gel (cyclohexane/ethyl acetate 80:20→50:50) to give the title compound. Yield: 446 mg; LC (method 9): t_{R} = 1.05 min; Mass spectrum (ESI⁺): m/z = 263 [M+H]⁺.

### Step 2: Methyl 2-((S)-6-((R)-4-(2,6-dimethyl-4-(pyrimidin-5-yl)phenyl)-7-fluoro-2,3-dihydro-1H-inden-1-yloxy)-2,3-dihydrobenzofuran-3-yl)acetate

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (100 mg), 5-(4-bromo-3,5-dimethylphenyl)pyrimidine (85 mg), K₃PO₄ (90 mg) are suspended in toluene (1 mL) and water (100 µL) and purged for 10 minutes with argon. Palladium-(II)-acetate (2.4 mg) and dicyclohexyl(2',6'-dimethoxybiphenyl-2-yl)phosphine (S-Phos) (8.8 mg) are added, the vial is sealed and the mixture is stirred at 100°C for 12 hours. The mixture is diluted with tetrahydrofuran and purified by HPLC on reversed phase to give the title compound. Yield: 40 mg; Mass spectrum (ESI⁺): m/z = 525 [M+H]⁺.

### Intermediate 41

### Methyl 2-((S)-6-((R)-4-(2,6-dimethyl-4-(oxazol-2-yl)phenyl)-7-fluoro-2,3-dihydro-1H-inden-1-yloxy)-2,3-dihydrobenzofuran-3-yl)acetate

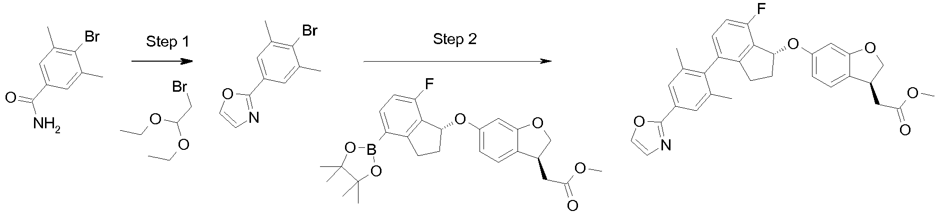

### Step 1: 2-(4-Bromo-3,5-dimethylphenyl)oxazole

In a microwave vial 4-bromo-3,5-dimethylbenzamide (200 mg) is mixed with 2-bromo-1,1-diethoxyethane (1 mL), the vial is sealed and the mixture is heated to 150°C for 30 minutes. The mixture is diluted with acetonitrile (2 mL) and purified by HPLC on reversed phase to give the title compound. Yield: 125 mg; LC (method 7): t_{R} = 1.13 min; Mass spectrum (ESI⁺): m/z = 252 [M+H]⁺.

### Step 2: Methyl 2-((S)-6-((R)-4-(2,6-dimethyl-4-(oxazol-2-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-(4-bromo-3,5-dimethylphenyl)oxazole following a procedure analogous to that described in Step 2 of Intermediate 40. LC (method 11): t_{R} = 1.27 min; Mass spectrum (ESI⁺): m/z = 514 [M+H]⁺.

### Intermediate 42

### Methyl 2-((S)-6-((R)-4-(2,6-dimethyl-4-(1-methyl-1H-pyrazol-4-yl)phenyl)-7-fluoro-2,3-dihydro-1H-inden-1-yloxy)-2,3-dihydrobenzofuran-3-yl)acetate

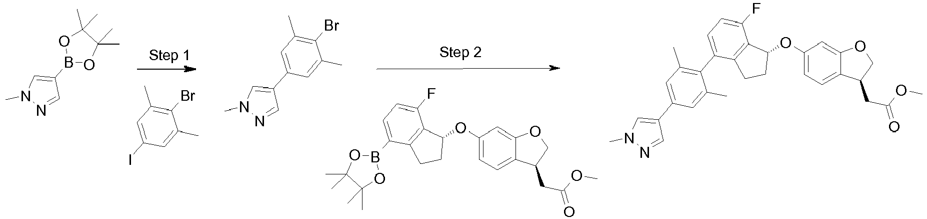

### Step 1: 4-(4-Bromo-3,5-dimethylphenyl)-1-methyl-1H-pyrazole

The title compound is prepared from 2-bromo-5-iodo-1,3-dimethylbenzene and 1-methyl-4-(4,4,5,5-tetramethyl-1,3,2-dioxaborolan-2-yl)-1H-pyrazole following a procedure analogous to that described in Step 1 of Intermediate 40. LC (method 9): t_{R} = 1.09 min; Mass spectrum (ESI⁺): m/z = 265 [M+H]⁺.

### Step 2: Methyl 2-((S)-6-((R)-4-(2,6-dimethyl-4-(1-methyl-1 H-pyrazol-4-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenyl)-1-methyl-1H-pyrazole following a procedure analogous to that described in Step 2 of Intermediate 40. Mass spectrum (ESI⁺): m/z = 527 [M+H]⁺.

### Intermediate 43

### Methyl 2-((S)-6-((R)-4-(2,6-dimethyl-4-(1-methyl-1H-pyrazol-5-yl)phenyl)-7-fluoro-2,3-dihydro-1H-inden-1-yloxy)-2,3-dihydrobenzofuran-3-yl)acetate

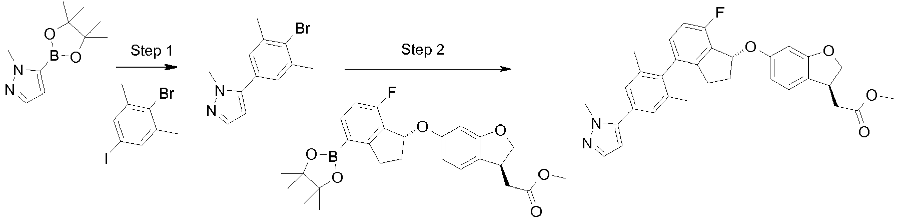

### Step 1: 5-(4-Bromo-3,5-dimethylphenyl)-1-methyl-1H-pyrazole

The title compound is prepared from 2-bromo-5-iodo-1,3-dimethylbenzene and 1-methyl-5-(4,4,5,5-tetramethyl-1,3,2-dioxaborolan-2-yl)-1H-pyrazole following a procedure analogous to that described in Step 1 of Intermediate 40. LC (method 9): t_{R} = 1.11 min; Mass spectrum (ESI⁺): m/z = 265 [M+H]⁺.

### Step 2: Methyl 2-((S)-6-((R)-4-(2,6-dimethyl-4-(1-methyl-1 H-pyrazol-5-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo furan-3-yl)acetate and 5-(4-bromo-3,5-dimethylphenyl)-1-methyl-1H-pyrazole following a procedure analogous to that described in Step 2 of Intermediate 40. Mass spectrum (ESI⁺): m/z = 527 [M+H]⁺.

### Intermediate 44

### Methyl 2-((S)-6-((R)-4-(4-(2-cyanopropan-2-yloxy)-2,6-dimethylphenyl)-7-fluoro-2,3-dihydro-1H-inden-1-yloxy)-2,3-dihydrobenzofuran-3-yl)acetate

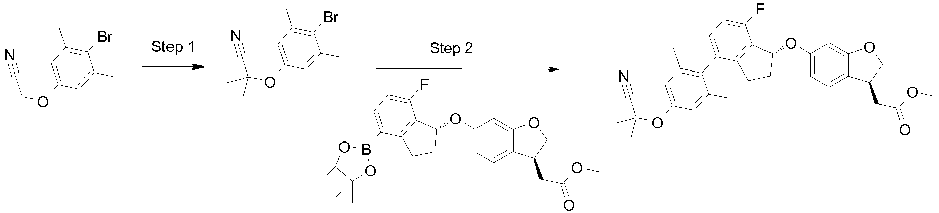

### Step 1: 2-(4-Bromo-3,5-dimethylphenoxy)-2-methylpropanenitrile

Under argon diisopropylamin (650 µL) is dissolved in tetrahydrofuran (6 mL), cooled to -30°C and treated dropwise with n-butyllithium (2.75 mL of a 1.6 M solution in n-hexane). The mixture is stirred for 30 minutes, cooled to -78°C and then treated dropwise with a solution of 2-(4-bromo-3,5-dimethylphenoxy)acetonitrile (350 mg) and methyliodide (549 µL) in tetrahydrofuran (6 mL). The mixture is stitrred for 2 hours warmed to 0°C and stirred for 1 hour. The reaction is quenched by addition of saturated aqueous NH₄Cl solution, the aqueous phase is extracted with diethylether and the organic phase is dried (MgSO₄). The solvents are evaporated and the residue is chromatographed on silica gel (petrole ether/ethyl acetate 95:5→60:40) to give the title compound. Yield: 120 mg; Mass spectrum (El): m/z = 267 [M]⁺.

### Step 2: Methyl 2-((S)-6-((R)-4-(4-(2-cyanopropan-2-yloxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-(4-bromo-3,5-dimethylphenoxy)-2-methylpropanenitrile following a procedure analogous to that described in Step 5 of Intermediate 12. LC (method 7): t_{R} = 1.25 min; Mass spectrum (ESI⁺): m/z = 530 [M+H]⁺.

### Intermediate 45

### Methyl 2-((S)-6-((R)-4-(4-(3-dimethylamino)-2,2-dimethylpropoxy)-2,6-dimethylphe nyl)-7-fluoro-2,3-dihydro-1H-inden-1-yloxy)-2,3-dihydrobenzofuran-3-yl)acetate

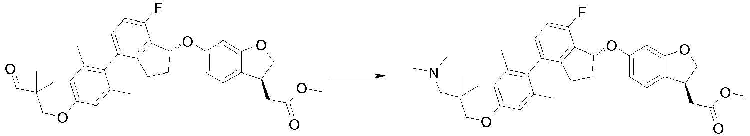

Dimethylamine (95 µL of a 2 M solution in tetrahydrofuran) is added to a solution of methyl 2-((S)-6-((R)-4-(4-(2,2-dimethyl-3-oxopropoxy)-2,6-dimethylphenyl)-7-fluoro-2,3-dihydro-1H-inden-1-yloxy)-2,3-dihydrobenzofuran-3-yl)acetate (83 mg) and acetic acid (13 µL) in 1,2-dichloroethane (2 mL). The mixture is stirred for 20 minutes and then treated with sodium triacetoxyborohydride (130 mg). After stirring for 12 hours the mixture is diluted with dichloromethane, washed with water and brine, dried (MgSO₄) and concentrated. The residue is purified by HPLC on reversed phase to give the title compound. Yield: 20 mg; LC (method 13): t_{R} = 1.18 min; Mass spectrum (ESI⁺): m/z = 576 [M+H]⁺.

### Intermediate 46

### Methyl 2-((S)-6-((R)-4-(2,6-dimethyl-4-(2-(2-oxopyrrolidin-1-yl)ethoxy)phenyl)-7-fluoro-2,3-dihydro-1H-inden-1-yloxy)-2,3-dihydrobenzofuran-3-yl)acetate

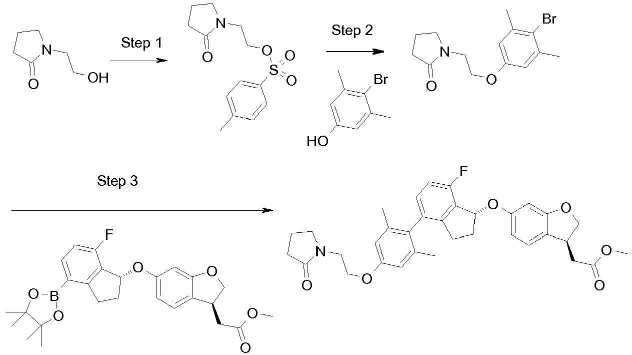

### Step 1: 2-(2-Oxopyrrolidin-1-yl)ethyl 4-methylbenzenesulfonate

p-Toluenesulfonylchloride (1 g) is added portionwise to a solution of 1-(2-hydroxyethyl)pyrrolidin-2-one (500 µL) and pyridine (1.5 mL) in dichloromethane (8 mL) chilled in an ice bath. The mixture is stirred at room temperature for 12 hours and partitioned between 1 M hydrochloric acid and diethylether. The organic phase is dried (MgSO₄) and concentrated. The residue is chromatographed on silica gel (ethyl acetate) to give the title compound. Yield: 390 mg; Mass spectrum (ESI⁺): m/z = 284 [M+H]⁺.

### Step 2: 1-(2-(4-Bromo-3,5-dimethylphenoxy)ethyl)pyrrolidin-2-one

Cs₂CO₃ (850 mg) is added to a soltuion of 2-(2-oxopyrrolidin-1-yl)ethyl 4-methylbenzenesulfonate (390 mg) and 4-bromo-3,5-dimethylphenol (200 mg) in N,N-dimethylformamide (4 mL). The mixture is heated to 50°C for 12 hours and partitioned between diethylether and saturated aqueous NH₄Cl solution. The organic phase is dried (MgSO₄) and concentrated. The residue is chromatographed on silica gel (ethyl acetate) to give the title compound. Yield: 210 mg; Mass spectrum (ESI⁺): m/z = 312 [M+H]⁺.

### Step 3: Methyl 2-((S)-6-((R)-4-(2,6-dimethyl-4-(2-(2-oxopyrrolidin-1-yl)ethoxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo furan-3-yl)acetate and 1-(2-(4-bromo-3,5-dimethylphenoxy)ethyl)pyrrolidin-2-one following a procedure analogous to that described in Step 5 of Intermediate 12. The product is purified by HPLC on reversed phase. LC (method 8): t_{R} = 0.68 min; Mass spectrum (ESI⁺): m/z = 574 [M+H]⁺.

### Intermediate 47

### Methyl 2-((S)-6-((R)-4-(4-3,6-dihydro-2H-pyran-4-yl)-2,6-dimethylphenyl-7-fluoro-2,3-dihydro-1H-inden-1-yloxy)-2,3-dihydrobenzofuran-3-yl)acetate

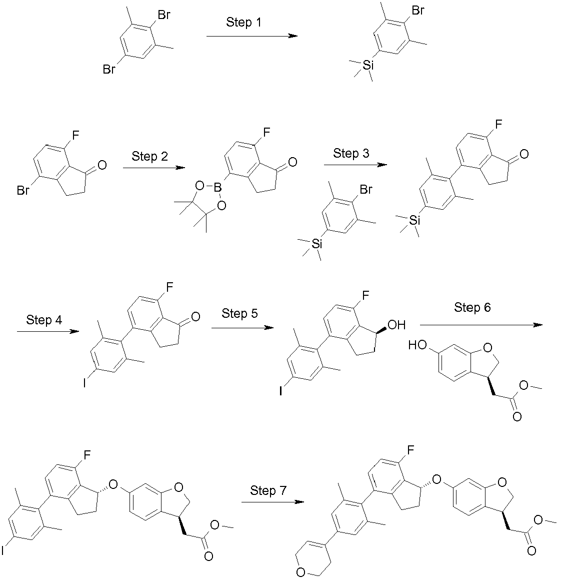

### Step 1: (4-Bromo-3,5-dimethylphenyl)trimethylsilane

A solution of 2,5-dibromo-1,3-dimethylbenzene (500 mg) in diethylether (3 mL) and tetrahydrofuran (2.5 mL) is cooled to -78°C and treated dropwise with n-butyllithium (200 µL of a 10 M solution in n-hexane). The mixture is stirred for 1 hour and treated dropwise with chloro-trimethyl-silane (275 µL). After stirring for 2 hours the reaction is quenched by addition of saturated aqueous NH₄Cl solution. The mixture is extracted with diethylether. The organic phase is dried (MgSO₄) and concentrated. The residue is chromatographed on silica gel (cyclohexane/ethyl acetate 99:1→90:10) to give the title compound. Yield: 260 mg; Mass spectrum (ESI⁺): m/z = 279 [M+Na]⁺.

### Step 2: 7-Fluoro-4-(4,4,5,5-tetramethyl-1,3,2-dioxaborolan-2-yl)-2,3-dihydro-1H-inden-1-one

The title compound is prepared from 4-bromo-7-fluoro-2,3-dihydro-1H-inden-1-one following a procedure analogous to that described in Step 4 of Intermediate 12. LC (method 8): t_{R} = 0.36 min; Mass spectrum (ESI⁺): m/z = 277 [M+H]⁺.

### Step 3: 4-(2,6-Dimethyl-4-(trimethylsilyl)phenyl)-7-fluoro-2,3-dihydro-1H-inden-1-one

The title compound is prepared from 7-fluoro-4-(4,4,5,5-tetramethyl-1,3,2-dioxaborolan-2-yl)-2,3-dihydro-1H-inden-1-one and (4-bromo-3,5-dimethylphenyl)trimethylsilane following a procedure analogous to that described in Step 5 of Intermediate 12. LC (method 8): t_{R} = 0.89 min; Mass spectrum (ESI⁺): m/z = 327 [M+H]⁺.

### Step 4: 7-Fluoro-4-(4-iodo-2,6-dimethylphenyl)-2,3-dihydro-1H-inden-1-one

4-(2,6-Dimethyl-4-(trimethylsilyl)phenyl)-7-fluoro-2,3-dihydro-1H-inden-1-one (110 mg) is dissolved in dichloromethane (2 mL), cooled to -30°C and treated dropwise with ICI (355 µL of a 1 M solution in dichloromethane). The mixture is stirred for 1 hour and then partitioned between saturated aqueous NaHCO₃ solution and dichloromethane. The organic phase is washed with a 10% aqueous solution of Na₂S₂O₃ and brine. After drying (MgSO₄) the solvents are evaporated off to give the title compound. Yield: 160 mg; LC (method 8): t_{R} = 0.66 min; Mass spectrum (ESI⁺): m/z = 381 [M+H]⁺.

### Step 5: (S)-7-Fluoro-4-(4-iodo-2,6-dimethylphenyl)-2,3-dihydro-1H-inden-1-ol

The title compound is prepared from 7-fluoro-4-(4-iodo-2,6-dimethylphenyl)-2,3-dihydro-1H-inden-1-one following a procedure analogous to that described in Step 1 of Intermediate 1. LC (method 8): t_{R} = 0.65 min; Mass spectrum (ESl⁻): m/z = 381 [M-H]⁻.

### Step 6: Methyl 2-((S)-6-((R)-7-fluoro-4-(4-iodo-2,6-dimethylphenyl)-2,3-dihydro-1H-inden-1-yloxy)-2,3-dihydrobenzofuran-3-yl)acetate

The title compound is prepared from (S)-7-fluoro-4-(4-iodo-2,6-dimethylphenyl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 8): t_{R} = 1.08 min; Mass spectrum (ESI⁺): m/z = 595 [M+Na]⁺.

### Step 7: Methyl 2-((S)-6-((R)-4-(4-(3,6-dihydro-2H-pyran-4-yl)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4-iodo-2,6-dimethylphenyl)-2,3-dihydro-1H-inden-1-yloxy)-2,3-dihydrobenzofuran-3-yl)acetate and 2-(3,6-dihydro-2H-pyran-4-yl)-4,4,5,5-tetramethyl-1,3,2-dioxaborolane following a procedure analogous to that described in Step 4 of Intermediate 13. LC (method 8): t_{R} = 0.92 min; Mass spectrum (ESI⁺): m/z = 551 [M+Na]⁺.

### Intermediate 48

### Methyl 2-((S)-6-((R)-4-(2,6-dimethyl-4-(1-(methylsulfonyl)-1,2,3,6-tetrahydropyridin-4-yl)phenyl)-7-fluoro-2,3-dihydro-1H-inden-1-yloxy)-2,3-dihydrobenzofuran-3-yl)acetate

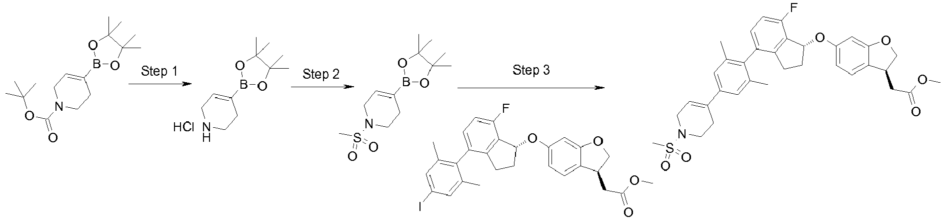

### Step 1: 4-(4,4,5,5-Tetramethyl-1,3,2-dioxaborolan-2-yl)-1,2,3,6-tetrahydropyridinium chloride

Tert-butyl 4-(4,4,5,5-tetramethyl-1,3,2-dioxaborolan-2-yl)-5,6-dihydropyridine-1(2H)-carboxylate (10 g) is dissolved in dichloromethane (100 mL) and 5 M HCl in isopropanol (120 mL) and stirred for 12 hours. The solvents are evaporated, the residue is redissolved in toluene and the sollvent is again evaporated to give the title compound. Yield: 8 g; LC (method 11): t_{R} = 0.68 min; Mass spectrum (ESI⁺): m/z = 210 [M+H]⁺.

### Step 2: 1-(Methylsulfonyl)-4-(4,4,5,5-tetramethyl-1,3,2-dioxaborolan-2-yl)-1,2,3,6-tetrahydropyridine

To a cooled (0°C) solution of 4-(4,4,5,5-tetramethyl-1,3,2-dioxaborolan-2-yl)-1,2,3,6-tetrahydropyridinium chloride (8 g) and N-ethyldiisopropylamine (12 mL) in dichloromethane (100 mL) is added dropwise methanesulfonyl chloride (3 mL). The mixture is stirred for 12 hours at room temperature. The mixture is partitioned between dichloromethane and 0.1 M hydrochloric acid. The organic phase is separated, washed with brine and dried (MgSO₄). The solvent is evaporated and the residue is crystallized from diethylether to give the title compound. Yield: 7.4 g; LC (method 15): t_{R} = 0.98 min; Mass spectrum (ESI⁺): m/z = 288 [M+H]⁺.

### Step 3: Methyl 2-((S)-6-((R)-4-(2,6-dimethyl-4-(1-(methylsulfonyl)-1,2,3,6-tetrahydropyridin-4-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4-iodo-2,6-dimethylphenyl)-2,3-dihydro-1H-inden-1-yloxy)-2,3-dihydrobenzofuran-3-yl)acetate and 1-(methylsulfonyl)-4-(4,4,5,5-tetramethyl-1,3,2-dioxaborolan-2-yl)-1,2,3,6-tetrahydropyridine following a procedure analogous to that described in Step 4 of Intermediate 13. LC (method 8): t_{R} = 0.79 min; Mass spectrum (ESI⁺): m/z = 628 [M+Na]⁺.

### Intermediate 49a

### (1,1-Dioxo-tetrahydrothiophen-3-yl)methanol

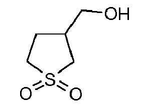

1,1-Dioxo-tetrahydrothiophene-3-carboxylic acid (300 mg) is dissolved in dry tetrahydrofuran (10 mL). The reaction mixture is cooled to 0°C and borane tetrahydrofuran complex (2 mL) is added dropwise. After stirring for 1 hour at 0°C, the mixture is concentrated under vacuum, partitioned between dichloromethane and water. The organic phase is dried (MgSO₄) and concentrated to give the title compound. Yield: 250 mg.

### Intermediate 49b

### (1,1-Dioxo-tetrahydro-2H-thiopyran-4-yl)methanol

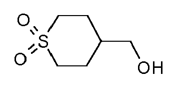

The title compound is prepared in analogy to Intermediate 49a starting from 1,1-dioxo-tetrahydro-2H-thiopyran-4-carboxylic acid.

### Intermediate 49c

### tert-Butyl 3-(hydroxymethyl)azetidine-1-carboxylate

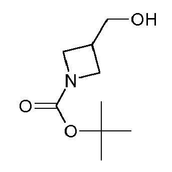

The title compound is prepared in analogy to Intermediate 49a starting from 1-(tert-butoxycarbonyl)azetidine-3-carboxylic acid.

### Intermediate 49d

### 1-(Methylsulfonyl)azetidin-3-yl methanesulfonate

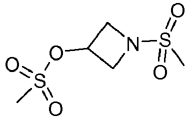

Azetidin-3-ol (3.3 g) and DIPEA (20 mL) are dissolved in dry tetrahydrofuran (40 mL). After 30 min the reaction mixture is cooled to 0°C and methanesulfonyl chloride (10.9 g) is added dropwise. After stirring for 2 hours at ambient temperature, the mixture is concentrated under vacuum, partitioned between ethyl acetate and water. The organic phase is dried (MgSO₄) and concentrated. Cromatography of the residue on silica gel (cyclohexane/ethyl acetate 70:30) gives the title compound. Yield: 5 g.

The intermediates in the following table are prepared following a procedure analogous to that described for Intermediate 49d.

| **starting aminoalcohol** | **Intermediate** | **Molecular structure and name** |
|---|---|---|
| 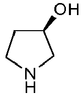 | **49e** | 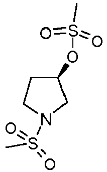 |
| (R)-(+)-3-pyrrolidinol | | (R)-1-(Methylsulfonyl)pyrrolidin-3-yl methanesulfonate |
| 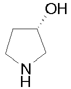 | **49ea** | 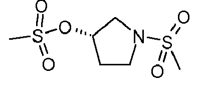 |
| (S)-(+)-3-pyrrolidinol | | (S)-1-(Methylsulfonyl)pyrrolidin-3-yl methanesulfonate |
| 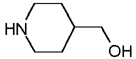 | **49f** | 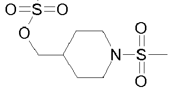 |
| Piperidin-4-yl-methanol | | (1-(Methylsulfonyl)piperidin-4-yl)methyl methanesulfonate |
| 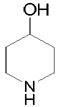 | **49fa** | 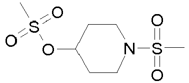 |
| Piperidin-4-ol | | Methanesulfonic acid methanesulfonyl-piperidin-4-yl ester |
| 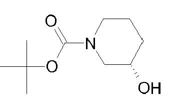 | **49fb** | 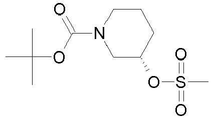 |
| | | (S)-3-Methanesulfonyloxy-piperidine -1-carboxylic acid tert-butyl ester |
| 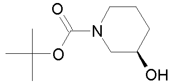 | **49fc** | 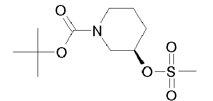 |
| | | (R)-3-Methanesulfonyloxy-piperidine -1-carboxylic acid tert-butyl ester |

### Intermediate 49g

### 1,1-Dioxo-tetrahydro-2H-thiopyran-4-yl methanesulfonate

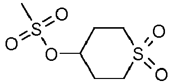

Methanesulfonyl chloride (0.15 mL), (1,1-dioxo-tetrahydro-2H-thiopyran-4-yl)methanol (150 mg) and triethylamine (0.3 mL) are stirred at 0°C in dry dichloromethane (2 mL). After 30 min, the reaction mixture is warmed to ambient temperature. After stirring for 3 hours, the mixture is diluted with dichloromethane, washed with with 10% aqueous solution of KHSO₄, then with 10% aqueous solution of K₂CO₃. The organic phase is collected by passing through a phase separator cartridge and concentrated. The residue is chromatographed on silica gel (dichloromethane/methanol 100:0→95:5) to give the title compound. Yield: 197 mg.

The following intermediates are prepared in analogy to Intermediate 49g starting from the corresponding alcohols.

| **starting alcohol** | **Intermediate** | **Molecular structure and name** |
|---|---|---|
| 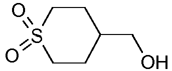 | **49ga** | 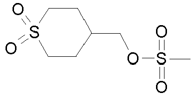 |
| 49b | | Methanesulfonic acid 1,1-dioxo-hexahydro-1-thiopyran-4-ylmethyl ester |
| 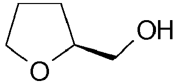 | **49gb** | 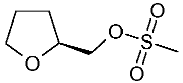 |
| (S)-1-(Tetrahydro-furan-2-yl)-methanol | | Methanesulfonic acid (S)-1-(tetrahy dro-furan-2-yl)methyl ester |
| 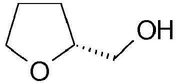 | **49gc** | 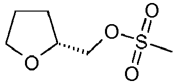 |
| (R)-1-(Tetrahydro-furan-2-yl)-methanol | | Methanesulfonic acid (R)-1-(tetrahy dro-furan-2-yl)methyl ester |
| 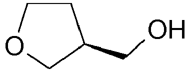 | **49gd** | 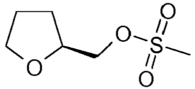 |
| (S)-1-(Tetrahydro-furan-3-yl)-methanol | | Methanesulfonic acid (R)-1-(tetrahy dro-furan-3-yl)methyl ester |

### Intermediate 49h

### Trans-3-(tert-butoxycarbonylamino)cyclobutyl 4-methylbenzenesulfonate

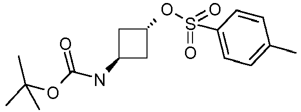

Trans-tert-butyl-3-hydroxycyclobutylcarbamate (440 mg), p-toluenesulfonyl chloride (498 mg) and pyridine (0.74 mL) are dissolved in dichloromethane (7 mL) and a catalytic amount of 4-(dimethylamino)pyridine is added. The reaction mixture is stirred at ambient temperature overnight, concentrated, partitioned between ethyl acetate and water. The organic phase is washed with a 20% aqueous solution of citric acid, dried (MgSO₄) and concentrated to give the title compound. Yield: 806 mg.

The following intermediates are prepared in analogy to Intermediate 49h starting from the corresponding alcohols.

| **starting alcohol** | **Intermediate** | **Molecular structure and name** |
|---|---|---|
| 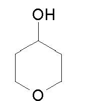 | **49ha** | 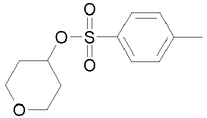 |
| Tetrahydro-pyran-4-ol | | Toluene-4-sulfonic acid tetrahydropyran-4-yl ester |
| 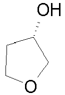 | **49hb** | 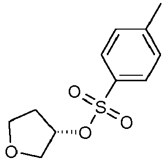 |
| (S)-Tetrahydro-furan-3-ol | | Toluene-4-sulfonic acid (S)-(tetrahydrofuran-3-yl) ester |
| 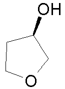 | **49hc** | 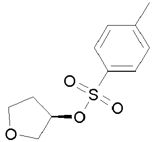 |
| (R)-Tetrahydro-furan-3-ol | | Toluene-4-sulfonic acid (R)-(tetrahydrofuran-3-yl) ester |
| 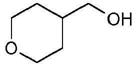 | **49hd** | 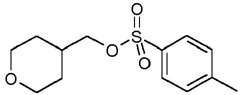 |
| (Tetrahydro-pyran-4-yl)-methanol | | Toluene-4-sulfonic acid tetrahydropyran-4-ylmethyl ester |

### Intermediate 49i

### Methanesulfonic acid 1-methanesulfonyl-3-methyl-azetidin-3-ylmethyl ester

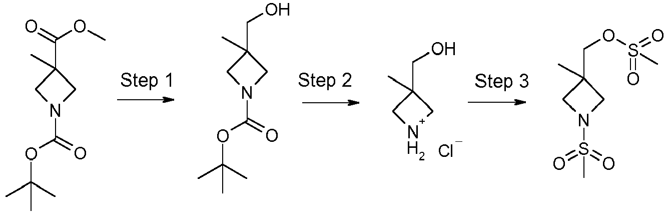

### Step 1: 3-Hydroxymethyl-3-methyl-azetidine-1-carboxylic acid tert-butyl ester

3-Methyl-azetidine-1,3-dicarboxylic acid 1-tert-butyl ester 3-methyl ester (WO2006/73361 A1, 2006) (1.1 g) is dissolved in 20 mL of tetrahydrofurane. Lithiumborohydride (16.8 mL of a 2 M solution in tetrahydrofurane) is added dropwise and the reaction mixture is stirred at room temperature overnight. The reaction mixture is diluted with water, washed with a saturated ammonium chloride water solution and the organic phase is separated, dried over sodium sulfate and concentrated under vacuum. The residue is chromatographed on silica gel (hexane/ethyl acetate 100:0→50:50) to give the title compound. Yield: 970 mg.

### Step 2: 3-Hydroxymethyl-3-methyl-azetidine hydrochloride

3-Hydroxymethyl-3-methyl-azetidine-1-carboxylic acid tert-butyl ester (1 g) is dissolved in 5 mL of ethyl ether. A 2 M aqueous solution of hydrochloric acid (4 mL) is added, the reaction mixture is stirred overnight and then concentrated under vacuum to give the title compound. Yiel: 600 mg.

### Step 3: Methanesulfonic acid 1-methanesulfonyl-3-methyl-azetidin-3-ylmethyl ester

The title compound is prepared following a procedure analogous to that described for Intermediate 49d.

### Intermediate 49j

### Methanesulfonic acid 2-methanesulfonyl-2-methyl-propyl ester

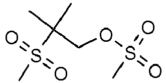

The title compound is prepared following a procedure analogous to that described for Intermediate 49g starting from 2-methanesulfonyl-2-methyl-propan-1-ol (Rouchard, Jean; Moons, Chantal; Meyer, Joseph Bulletin de la Societe Chimique de France, 1980 , vol. 2, # 9-10 p. 441 - 443).

### Intermediate 50a 1,1-Dioxo-3-((4-bromo-3,5-dimethylphenoxy)methyl)tetrahydrothiophene

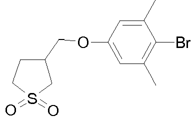

Intermediate 49a (235 mg), 4-bromo-3,5-dimethylphenol (318 mg), di-tert-butyl azodicarboxylate (396 mg) and triphenylphosphine (451 mg) are dissolved in dichloromethane (100 mL). The mixture is stirred at ambient temperature for 2 hours, diluted with dichloromethane and washed with water and brine. The organic phase is dried (MgSO₄) and concentrated. The residue is chromatographed on silica gel (cyclohexane/ethyl acetate 100:0→50:50) to give the title compound. Yield: 260 mg.

### Intermediate 50aa

### (S)-3-(4-Bromo-3,5-dimethyl-phenoxymethyl)-tetrahydro-thiophene 1,1-dioxide

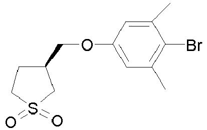

The title compound is obtained by chiral HPLC chromatographic separation of the racemate 1,1-dioxo-3-((4-bromo-3,5-dimethylphenoxy)methyl)tetrahydrothiophene.
Chiral HPLC (method 1 a): t_{R} = 18.85 min.
Absolute configuration unknown.

### Intermediate 50ab

### (R)-3-(4-Bromo-3,5-dimethyl-phenoxymethyl)-tetrahydro-thiophene 1,1-dioxide

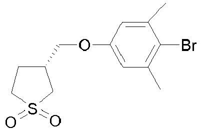

Further elution from the chiral column gives the title compound.
Chiral HPLC (method 1 a): t_{R} = 23,57 min
Absolute configuration unknown.

### Intermediate 50b

### 1,1-Dioxo-4-((4-bromo-3,5-dimethylphenoxy)methyl)tetrahydro-2H-thiopyran

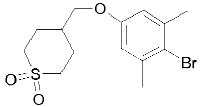

The title compound is prepared in analogy to Intermedate 50a, starting from Intermediate 49b.

### Intermediate 50ba

### 3-(4-Bromo-3,5-dimethyl-phenoxymethyl)-tetrahydro-thiopyran 1,1-dioxide

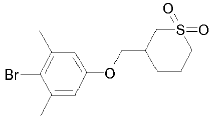

3-(Bromomethyl)tetrahydro-2H-thiopyrane 1,1-dioxide (1 g) is dissolved in 40 mL of N,N-dimethylformamide. 4-Bromo-3,5-dimethylphenol (885 mg) and cesium carbonate (2.85 g) are added and the reaction mixture is stirred at 130°C overnight. The reaction mixture is concentrated under vacuum. Water is added and the mixture is extracted with ethyl acetate, The organic phase is separated, dried over sodium sulfate and concentrated under vacuum. The residue is chromatographed on silica gel (cyclohexane/ethyl acetate 100:0→80:20) to give the title compound. Yield: 800 mg

### Intermediate 50bb

### 4-(4-Bromo-3,5-dimethyl-phenoxymethyl)-tetrahydro-pyran-4-ol

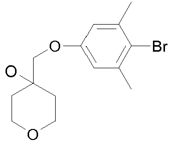

4-Bromo-3,5-dimethylphenol (1 g) and cesium carbonate (5.7 g) are suspended in 30 mL of N,N-dimethylacetamide, 1,6-dioxa-spiro[2.5]octane (US2012/46304 A1, 2012) (1.78 g) is added and the reaction mixture is stirred at 110 °C overnight. Water is added and the mixture is extracted with ethyl acetate, the organic phase is separated, dried over sodium sulfate and concentrated under vacuum. The residue is chromatographed on silica gel (cyclohexane/ethyl acetate 95:5) to give the title compound. Yield: 400 mg

### Intermediate 50bc

### 4-(4-Bromo-3,5-dimethyl-phenoxymethyl)-1,1-dioxo-hexahydro-1-thiopyran-4-ol

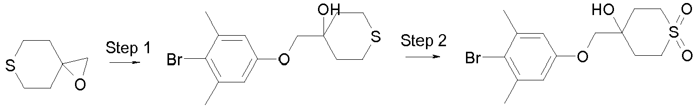

### Step 1: 4-(4-Bromo-3,5-dimethyl-phenoxymethyl)-tetrahydro-thiopyran-4-ol

The title compound is prepared in analogy to intermediate 50bb starting from 1-oxa-6-thia-spiro[2.5]octane (EP1726580 A1, 2006).

### Step 2: 4-(4-Bromo-3,5-dimethyl-phenoxymethyl)-1,1-dioxo-hexahydro-1-thiopyran-4-ol.

4-(4-Bromo-3,5-dimethyl-phenoxymethyl)-tetrahydro-thiopyran-4-ol (820 mg) is stirred in 20 mL of tetrahydrofurane. A solution of potassium peroxomonosulfate (4.33 g) in 5 mL of water is added dropwise and the reaction mixture is stirred at room temperature for 2 h. Water is added and the reaction mixture is extracted with dichloromethane, the organic phase is separated, dried over sodium sulfate and concentrated under vacuum. The residue is chromatographed on silica gel (cyclohexane/ethyl acetate 80:20→0:100) to give the title compound. Yield: 850 mg.

### Intermediate 50c

### 3-((4-Bromo-3,5-dimethylphenoxy)methyl)azetidine

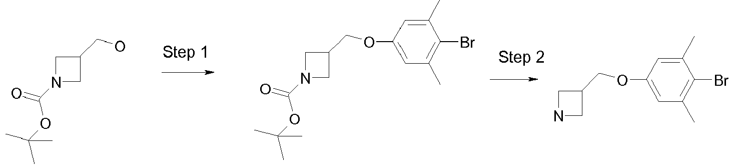

Step 1: tert-Butyl 3-((4-bromo-3,5-dimethylphenoxy)methyl)azetidine-1-carboxylate the title compound is prepared starting from Intermediate 49c and 4-bromo-3,5-dimethylphenol as described in the preparation of Intermediate 50a.

Step 2: 3-((4-Bromo-3,5-dimethylphenoxy)methyl)azetidine tert-Butyl 3-((4-bromo-3,5-dimethylphenoxy)methyl)azetidine-1-carboxylate (900 mg) and trifluoroacetic acid (0.3 mL) are dissolved in dichloromethane (40 mL). The reaction mixture is stirred at 0°C for 6 hours, diluted with a saturated aqueous solution of NaHCO₃, dried (MgSO₄) and concentrated to give the title compound.
Yield: 400 mg

### Intermediate 50d

### 3-((4-Bromo-3,5-dimethyphenoxy)methyl)-1-(methylsulfonyl)azetidine

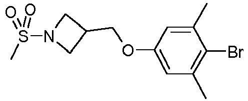

Intermediate 50c (400 mg) and N-ethyldiisopropylamine (0.48 mL) are dissolved in dry dichloromethane (26 mL). The reaction mixture is cooled to 0°C and methanesulfonyl chloride (0.1 mL) is added dropwise. After stirring for 0.5 hours at ambient temperature, the mixture is partitioned between dichloromethane and water. The organic phase is dried (MgSO₄) and concentrated. The residue is chromatographed on silica gel (cyclohexane/ethyl acetate 100:0→50:50) to give the title compound. Yield: 330 mg.

The bromo-aryl intermediates in the following table are prepared starting from Intermediate 50c and the corresponding sulfonyl chlorides following a procedure analogous to that described for Intermediate 50d.

| **Sulfonyl chloride** | **Intermediate** | **Molecular structure and name** |
|---|---|---|
| Ethanesulfonyl chloride | **50e** | 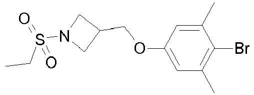 |
| | | 3-((4-bromo-3,5-dimethylphenoxy)methyl)-1-(ethylsulfonyl)azetidine |
| Isopropylsulfonyl chloride | **50f** | 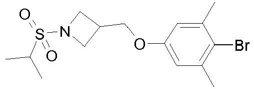 |
| | | 3-((4-bromo-3,5-dimethylphenoxy)methyl)-1-(isopropylsulfonyl)azetidine |

### Intermediate 50g

### 3-(4-Bromo-3,5-dimethylphenoxy)-1-(methylsulfonyl)azetidine

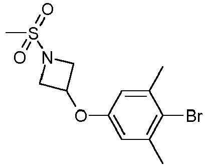

In a microwave vial 4-bromo-3,5-dimethylphenol (1 g) and sodium hydride (100 mg) are stirred in dry N,N-dimethylacetamide (25 mL) for 30 minutes. Intermediate 49d (1 g) is added, the vial is sealed and the mixture is stirred at 140°C for 1.5 hours (100 W). After cooling to ambient temperature, the mixture is concentrated, partitioned between ethyl acetate and water. The organic phase is dried (MgSO₄) and concentrated. The residue is chromatographed on silica gel (cyclohexane/ethyl acetate 100:0→70:30) to give the title compound. Yield: 500 mg.

### Intermediate 50ga

### 4-(4-Bromo-3,5-dimethyl-phenoxy)-1-methanesulfonyl-piperidine

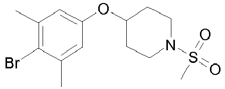

The title compound is prepared in analogy to Intermedate 50g, starting from Intermediate 49fa.

### Intermediate 50gb

### 3-(4-Bromo-3,5-dimethyl-phenoxyl)-1-methanesulfonyl-3-methyl-azetidine

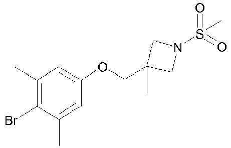

The title compound is prepared in analogy to Intermedate 50g, starting from Intermediate 49i.

### Intermediate 50gc

### (R)-3-(4-Bromo-3,5-dimethyl-phenoxy)-1-methanesulfonyl-pyrrolidine

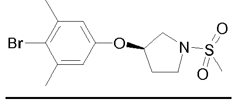

The title compound is prepared in analogy to Intermediate 50g, starting from intermediate 49ea.

### Intermediate 50gd

### (R)-(4-Bromo-3,5-dimethyl-phenoxy)-1-methanesulfonyl-piperidine

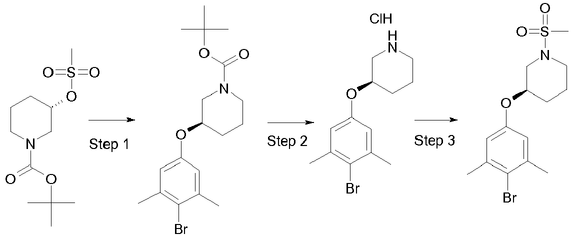

### Step 1: (R)-3-(4-Bromo-3,5-dimethyl-phenoxy)-piperidine-1-carboxylic acid tert-butyl ester.

The title compound is prepared in analogy to Intermediate 50g, starting from intermediate 49fb.

### Step 2: (R)-3-(4-Bromo-3,5-dimethyl-phenoxy)-piperidine hydrochloride

The title compound is prepared in analogy to Step 2 in the preparation of Intermediate 49i, starting from (R)-3-(4-Bromo-3,5-dimethyl-phenoxy)-piperidine-1-carboxylic acid tert-butyl ester

### Step 3: (R)-3-(4-Bromo-3,5-dimethyl-phenoxy)-1-methanesulfonyl-piperidine

The title compound is prepared in analogy to Intermediate 50d starting from (R)-3-(4-bromo-3,5-dimethyl-phenoxy)-piperidine hydrochloride.

### Intermediate 50ge

### (S)-3-(4-Bromo-3,5-dimethyl-phenoxy)-1-methanesulfonyl-piperidine

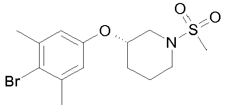

The title compound is prepared in analogy to Intermediate 50gd, starting from intermediate 49fc.

### Intermediate 50h

### 1,1-Dioxo-4-(4-bromo-3,5-dimethylphenoxy)tetrahydro-2H-thiopyran

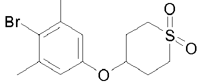

The title compound is prepared in analogy to Intermedate 50g, starting from Intermediate 49g.

### Intermediate 50ha

### 2-Bromo-5-(2-methanesulfonyl-2-methyl-propoxy)-1,3-dimethyl-benzene

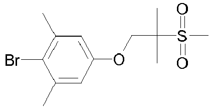

The title compound is prepared in analogy to Intermedate 50g, starting from Intermediate 49j.

### Intermediate 50i

### N-((1s,3s)-3-(4-Bromo-3,5-dimethylphenoxy)cyclobutyl)-N-methylmethanesulfonamide

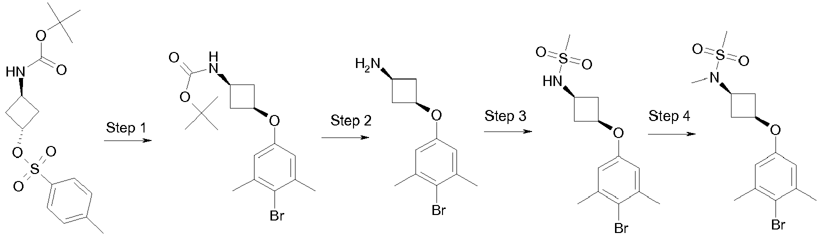

### Step 1: tert-Butyl cis-3-(4-bromo-3,5-dimethylphenoxy)cyclobutylcarbamate

4-bromo-3,5-dimethylphenol (450 mg) and sodium hydride (60 mg) are stirred in N,N-dimethylacetamide (15 mL) for 30 minutes. Intermediate 49h (300 mg) is added and the mixture is stirred at 80°C for 3 hours. After cooling to ambient temperature, the mixture is concentrated, partitioned between ethyl acetate and water. The organic phase is dried (MgSO₄) and concentrated. The residue is chromatographed on silica gel (cyclohexane/ethyl acetate 90:10→40:60) to give the title compound. Yield: 200 mg.

### Step 2: cis-3-(4-Bromo-3,5-dimethylphenoxy)cyclobutanamine

tert-Butyl cis-3-(4-bromo-3,5-dimethylphenoxy)cyclobutylcarbamate (200 mg) and trifluoroacetic acid (0.13 mL) are dissolved in dichloromethane (6 mL). The reaction mixture is stirred at 0°C for 5 hours, diluted with a saturated aqueous solution of NaHCO₃, dried (MgSO₄) and concentrated to give the title compound. Yield: 400 mg. Step 3: N-(cis-3-(4-bromo-3,5-dimethylphenoxy)cyclobutyl)methanesulfonamide Methanesulfonyl chloride (0.16 mL), cis-3-(4-Bromo-3,5-dimethylphenoxy)cyclobutanamine (300 mg) and triethylamine (0.3 mL) are stirred at 0°C in dry dichlorometane (25 mL). After 10 min, the reaction mixture is warmed to ambient temperature. After stirring for 3 hours, the mixture is diluted with dichloromethane, washed with with 10% aqueous solution of KHSO₄, then with 10% aqueous solution of K₂CO₃. The organic phase was passed through a phase separator and concentrated. The residue is chromatographed on silica gel (cyclohexane/ethyl acetate 90:10→50:50) to give the title compound. Yield: 200 mg.

### Step 4: N-(cis-3-(4-bromo-3,5-dimethylphenoxy)cyclobutyl)-N-methylmethanesulfonamide

N-(cis-3-(4-bromo-3,5-dimethylphenoxy)cyclobutyl)methanesulfonamide (130 mg) and sodium hydride (16 mg) are stirred in N,N-dimethylacetamide (2 mL) for 10 minutes. Iodomethane (0.1 mL) is added and the mixture is stirred at ambient temperature overnight. After cooling to ambient temperature, the mixture is concentrated, partitioned between ethyl acetate and water. The organic phase is dried (MgSO₄) and concentrated. The residue is chromatographed on silica gel (cyclohexane/ethyl acetate gradient of increasing polarity) to give the title compound. Yield: 110 mg.

### Intermediate 50j

### (4-Bromo-3,5-dimethylphenoxy)(tert-butyl)dimethylsilane

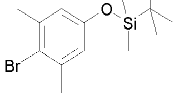

4-Bromo-3,5-dimethylphenol (3.5 g), 4-(dimethylamino)pyridine (212 mg) and triethylamine (2.7 mL) are stirred in dichloromethane (25 mL) at 0°C, then tert-butyldimethylchlorosilane (19 mL) is added dropwise. The reaction mixture is warmed to ambient temperature and stirred overnight. The reaction mixture is diluted with dicloromethane, washed with aqueous HCl (1 M, 25 mL) and then with saturated aqueous NaHCO₃ (25 mL). The organic phase is dried (MgSO₄) and concentrated to give the title compound. Yield: 5.4 g

### Intermediate 50k

### 1-Bromo-2-methyl-3-(3-(methylsulfonyl)propoxy)benzene

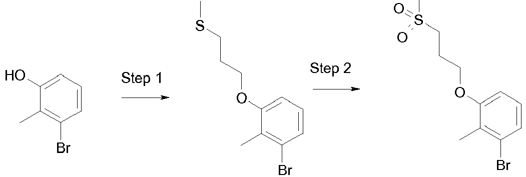

### Step 1: (3-(3-Bromo-2-methylphenoxy)propyl)(methyl)sulfane

3-Bromo-2-methylphenol (0.10 g), 3-(methylthio)propanol (0.055 mL), tri-n-butyl phosphine (0.12 g) and 1,1'-(azodicarbonyl)dipiperidine (0.15 g) are stirred in tetrahydofurane for 16 hours. Water is added and the mixture extracted with diethyl ether. The organic layer is washed with brine, dried (Na₂SO₄) and volatiles removed under reduced pressure. The product (80 mg) is obtained after column chromatography (silica gel, n-hexane/ethyl acetate gradient 100:0 to 50:50).

### Step 2: 1-Bromo-2-methyl-3-(3-(methylsulfonyl)propoxy)benzene

(3-(3-Bromo-2-methylphenoxy)propyl)(methyl)sulfane (0.90 g) and 3-chloroperbenzoic acid (1.7 g) are allowed to react in dichloromethane (15 mL) for 16 h. A saturated solution of K₂CO₃ is added with vigorous stirring, the organic layer collected, washed (brine), dried (Na₂SO₄) and volatiles removed under reduced pressure. The product (0.91 g) is obtained after column chromatography (silica gel, n-hexane/ethyl acetate gradient 100:0 to 70:30).

### Intermediate 50ka

### 2-Bromo-5-(4-methanesulfonyl-butoxy)-1,3-dimethyl-benzene

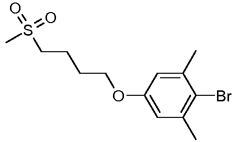

The title compound is prepared in analogy to Intermedate 50k, starting from 4-methylsulfanyl-butan-1-ol and from 4-bromo-3,5-dimethyl-phenol.

### Intermediate 501

### 4-(3-Bromo-2-methyl-phenoxy)-tetrahydro-thiopyran 1,1-dioxide

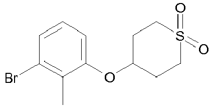

The title compound is prepared in analogy to Intermedate 50g, starting from Intermediate 49g and from 3-bromo-2-methyl-phenol.

### Intermediate 50m

### 2-Bromo-5-((R)-3-methanesulfonyl-2-methyl-propoxy)-1,3-dimethyl-benzene

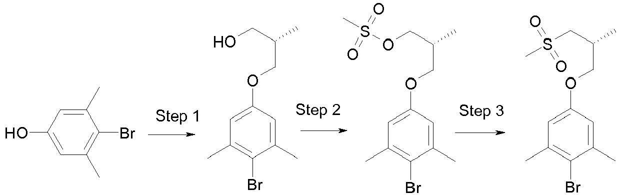

### Step 1: (S)-3-(4-Bromo-3,5-dimethyl-phenoxy)-2-methyl-propan-1-ol

(S)-3-Bromo-2-methyl-1-propanol (150 mg), 4-bromo-3,5-dimethylphenol (98.5 mg) and potassium carbonate are stirred in 5 mL of acetonitrile at 80 °C for 4 h. The reaction mixture is cooled to room temperature, water is added and the reaction mixture is extracted with diethyl ether. The organic phase is separated, dried over sodium sulfate and concentrated under vacuum to give a solid, used in the next step without further purification. Yield: 130 mg.

### Step 2: Methanesulfonic acid (R)-3-(4-bromo-3,5-dimethyl-phenoxy)-2-methyl-propyl ester

(S)-3-(4-Bromo-3,5-dimethyl-phenoxy)-2-methyl-propan-1-ol (130 mg), methanesulfonyl chloride (0.073 mL) and triethylamine (0.264 mL) are stirred in 5 mL of dichloromethane at room temperature overnight. The reaction mixture is washed with citric acid saturated aqueous solution and with brine. The organic phase is separated, dried over sodium sulfate and concentrated under vacuum to give the title compound, used in the next step without further purification. Yield: 148 mg.

### Step 3: 2-Bromo-5-((R)-3-methanesulfonyl-2-methyl-propoxy)-1,3-dimethyl-benzene

Methanesulfonic acid (R)-3-(4-bromo-3,5-dimethyl-phenoxy)-2-methyl-propyl ester (148 mg) and sodium methanesulfinate (215 mg) are stirred in 3 mL of N,N-dimethylformamide at 80 °C for 4 h. The reaction mixture is cooled to room temperature, water is added and the reaction mixture is extracted with diethyl ether. The organic phase is separated, dried over sodium sulfate and concentrated under vacuum to give the title compound. Yield: 118 mg.

### Intermediate 50n

### 2-Bromo-5-((S)-3-methanesulfonyl-2-methyl-propoxy)-1,3-dimethyl-benzene

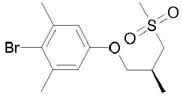

The title compound is prepared in analogy to Intermedate 50m, starting from (R)-3-bromo-2-methyl-1-propanol.

### Intermediate 50o

### 2-[2-(4-Bromo-3,5-dimethyl-phenoxy)-ethyl]-isothiazolidine 1,1-dioxide

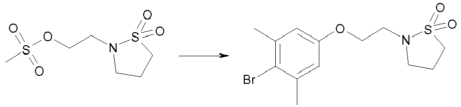

The title compound is prepared in analogy to Intermedate 50g, starting from methanesulfonic acid 2-(1,1-dioxo-1-isothiazolidin-2-yl)-ethyl ester (EP1479684 A1, 2004).

### Intermediate 50p

### 4-(4-Bromo-3,5-dimethyl-phenoxy)-[1,2]thiazinane 1,1-dioxide

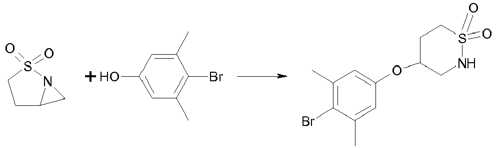

2-Thia-1-aza-bicyclo[3.1.0]hexane 2,2-dioxide (Liang, Jiang-Lin et al., Organic Letters, 2002 , vol. 4, # 25 p. 4507 - 4510) (300 mg) is added to a suspension of 4-bromo-3,5-dimethylphenol (1.14 g) and sodium hydride (140 mg of a 60% mineral oil suspension) in 25 mL of N,N-dimethylacetamide. The reaction mixture is stirred at 130 °C for 5 h. Water is added and the reaction mixture is extracted with ethyl acetate. The organic phase is separated, dried over sodium sulfate and concentrated under vacuum. The residue is chromatographed on silica gel (cyclohexane/ethyl acetate 100:0→30:70) to give the title compound. Yield: 220 mg.

### Intermediate 50q

### 4-(4-Bromo-3,5-dimethyl-phenoxy)-2-methyl-[1,2]thiazinane 1,1-dioxide

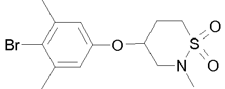

4-(4-Bromo-3,5-dimethyl-phenoxy)-[1,2]thiazinane 1,1-dioxide (110 mg) and sodium hydride (14,5 mg of a 60% mineral oil suspension) are suspended in 2 mL of N,N-dimethylformamide. Iodomethane (0.02 mL) is added and the reaction mixture is stirred at room temperature for 3 h. The solvent is removed, water is added and the reaction mixture is extracted with ethyl acetate. The organic phase is separated, dried over sodium sulfate and concentrated under vacuum. The residue is chromatographed on silica gel (cyclohexane/ethyl acetate 90:10→60:40) to give the title compound. Yield: 110 mg.

### Intermediate 50r

### 2-(4-Bromo-3,5-dimethyl-phenoxy)-2-methyl-propan-1-ol

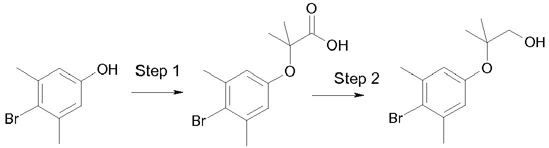

### Step 1: 2-(4-Bromo-3,5-dimethyl-phenoxy)-2-methyl-propionic acid.

To a solution of 4-3,5-dimethylphenol (5 g) in 100 mL of acetone stirred at -30 °C, sodium hydroxide (31 g) is added and the reaction mixture is allowed to reach room temperature in 30 min, then warmed to reflux for 3 h. The reaction mixture is concentrated under vacuum, iced-water is added and a 6 M solution of HCl is added up to acidic pH. The reaction mixture is extracted with ethyl acetate. The organic phase is separated, dried over sodium sulfate and concentrated under vacuum. The residue is chromatographed on silica gel (hexane/ethyl acetate:50/50) to give the title compound. Yield: 8 g.

Step 2: 2-(4-Bromo-3,5-dimethyl-phenoxy)-2-methyl-propan-1-ol2-(4-Bromo-3,5-dimethyl-phenoxy)-2-methyl-propionic acid (4.45 g) is dissolved in 50 mL of tetrahydrofurane, borane methylsulfide complex (2.6 mL) is added and the reaction mixture is stirred at room temperature for 5 h. The reaction mixture is cooled to 0 °C, 10% HCl water solution is added dropwise and the reaction mixture is extracted with dichloromethane. The organic phase is separated, dried over sodium sulfate and concentrated under vacuum. The residue is chromatographed on silica gel (hexane/ethyl acetate 100:0→50:50) to give the title compound. Yield: 2.6 g.

### Intermediate 50s

### 2-(4-Bromo-3,5-dimethyl-phenoxy)-2-methyl-propionamide

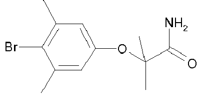

2-(4-Bromo-3,5-dimethyl-phenoxy)-2-methyl-propionic acid (500 mg) is dissolved in 10 mL of tetrahydrofurane, 1,1'-carbonyldimidazole (217 mg) and 30% ammonium hydroxide water solution are added and the reaction mixture is stirred at room temperature for 3h. The reaction mixture is concentrated under vacuum, ethyl acetate is added and the organic solution is washed with a 10% HCl water solution. The organic phase is separated, dried over sodium sulfate and concentrated under vacuum to give the title compound. Yield: 300 mg.

### Intermediate 50ta

### 4-(4-Bromo-3-methyl-phenoxymethyl)-tetrahydro-pyran-4-ol

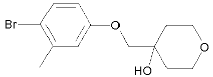

The title compound is prepared in analogy to Intermediate 50bb, starting from 4-bromo-3-methyl phenol.

### Intermediate 50tb

### 4-(4-Bromo-3-methyl-phenoxymethyl)-tetrahydro-thiopyran 1,1-dioxide

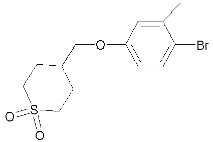

Intermediate 49ga (400 mg) is dissolved in 10 mL of N,N-dimethylformamide, 4-bromo-3-methylphenol (309 mg) and cesium carbonate (1.08 g) are added and the reaction mixture is stirred at 130 °C for 12 h. The reaction mixture is concentrated under vacuum, water is added and the reaction mixture is extracted with dichloromethane, The organic phase is separated, dried over sodium sulfate and concentrated under vacuum. The residue is triturated with ethyl acetate to give the title compound. Yield: 250 g.

### Intermediate 50tc

### 4-(4-Bromo-3-methyl-phenoxy)-2-methyl-butan-2-ol

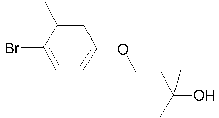

Intermediate 7 Step 1 (440 mg) is dissolved in 5 mL of N,N-dimethylformamide, 4-Bromo-3-methylphenol (300 mg) and cesium carbonate (950 g) are added and the reaction mixture is stirred at 80 °C for 5 h. The reaction mixture is concentrated under vacuum, water is added and the reaction mixture is extracted with ethyl acetate, The organic phase is separated, dried over sodium sulfate and concentrated under vacuum. The residue is chromatographed on silica gel (cyclohexane/ethyl acetate 100:0→50:50) to give the title compound. Yield: 250 mg.

### Intermediate 50td

### 4-(4-Bromo-3-methyl-phenoxymethyl)-1,1-dioxo-hexahydro-1-thiopyran-4-ol

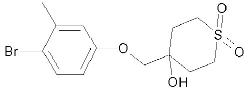

The title compound is prepared in analogy to Intermediate 50bc starting from 4-bromo-3-methyl phenol.

### Intermediate 50ua

### 4-(4-Bromo-2,6-difluoro-phenoxymethyl)-tetrahydro-pyran-4-ol

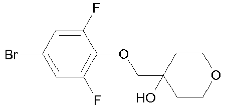

The title compound is prepared in analogy to Intermediate 50bb, starting from 4-bromo-2,6-difluorophenol.

### Intermediate 50ub

### 1-(4-Bromo-2,6-difluoro-phenoxy)-2-methyl-propan-2-ol

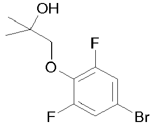

The title compound is prepared in analogy to Intermediate 50ua starting from 2,2-dimethyl-oxirane.

### Intermediate 50uc

### 4-(4-Bromo-2,6-difluoro-phenoxy)-2-methyl-butan-2-ol

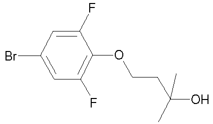

Intermediate 7 Step 1 (1 g) is dissolved in 10 mL of N,N'-dimethylacetamide, 4-bromo-2,6-difluorophenol (1 g) and cesium carbonate (2.5 g) are added and the reaction mixture is stirred at 80 °C for 5 h. The reaction mixture is concentrated under vacuum, water is added and the reaction mixture is extracted with ethyl acetate, The organic phase is separated, dried over sodium sulfate and concentrated under vacuum. The residue is chromatographed on silica gel (cyclohexane/ethyl acetate 100:0→80:20) to give the title compound. Yield: 640 mg.

The following intermediates are prepared in analogy to Intermediate 50uc starting from the corresponding tosilate intermediate.

| **Starting tosilate Intermediate** | **Intermediate** | **Molecular structure and name** |
|---|---|---|
| 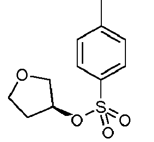 | **50ud** | 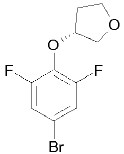 |
| 49hb | | (R)-3-(4-Bromo-2,6-difluoro-phenoxy)-tetrahydro-furan |
| 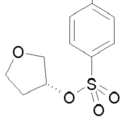 | **50ue** | 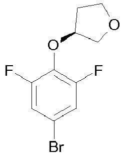 |
| 49hc | | (S)-3-(4-Bromo-2,6-difluoro-phenoxy)-tetrahydro-furan |

### Intermediate 50uf

### 5-Bromo-1,3-difluoro-2-(3-methanesulfonyl-propoxy)-benzene

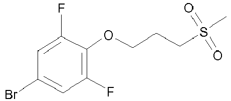

The title compound is prepared in analogy to Intermediate 50uc starting from methanesulfonic acid 3-methanesulfonyl-propyl ester

### Intermediate 50ug

### 2-(4-Bromo-2,6-difluoro-phenyl)-5-methyl-[1,3,4]oxadiazole

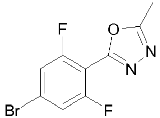

Acetic acid hydrazide (340 mg) N,N,N',N'-tetramethyl-O-(benzotriazol-1-yl)uronium tetrafluoroborate (1.63 g) and di-isopropyl-ethyl amine (1.64 g) are stirred in 20 mL of acetonitrile at room temperature for 5 minutes. 4-Bromo-2,6-difluoro-benzoic acid (WO2005/12283 A1, 2005) (1 g) is added and the reaction mixture is stirred at room temperature for 24 h, then at 70 °C for 3 h. The reaction mixture is concentrated under vacuum, The residue is chromatographed on silica gel (cyclohexane/ethyl acetate 100:0→60:40) to give the title compound. Yield: 300 mg.

### Intermediate 50uh

### 3-(4-Bromo-2,6-difluoro-phenyl)-5-methyl-[1,2,4]oxadiazole

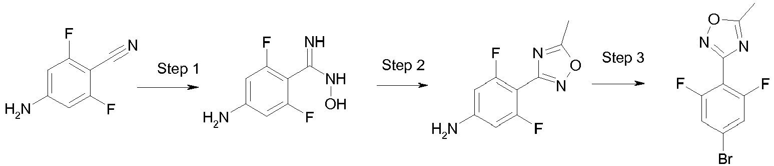

### Step 1: 4-Amino-2,6-difluoro-N-hydroxy-benzamidine

In a microwave vial, 4-amino-2,5-difluoro-benzonitrile (2 g) is dissolved in 10 mL of ethanol. Hydroxylamine (1,71 g) is added, the vial is sealed and the mixture is stirred at 100 °C for 1 h (100 W). The solvent is removed, the residue is washed with water. The precipitated solid is dried under vacuum and used in the next step without further purification. Yield: 2.4 g.

### Step 2: 3,5-Difluoro-4-(5-methyl-[1,2,4]oxadiazol-3-yl)-phenylamine

4-Amino-2,6-difluoro-N-hydroxy-benzamidine (2.4 g) acetyl chloride (25,64 mL) and triethylamine (5.35 mL) are stirred in 10 mL of 1,4-dioxane at room temperature for 30 minutes, then at 120 °C for 2 h. the solvent is removed, the residue is partitioned between a bicarbonate satured water solution and ethyl acetate. The organic phase is separated, dried over sodium sulfate and concentrated under vacuum. The residue is chromatographed on silica gel (cyclohexane/ethyl acetate 100:0→50.50) to give the title compound. Yield: 620 mg.

### Step 3: 4-Amino-2,6-difluoro-N-hydroxy-benzamidine

Copper bromide (790 mg) and tert-butylnitrite (870 mL) are stirred in 15 mL of acetonitrile at 65 °C for 20 minutes, 3,5-Difluoro-4-(5-methyl-[1,2,4]oxadiazol-3-yl)-phenylamine (620 mg) is added and the reaction mixture is stirred at 65 °C for 4 h. The reaction mixture is concentrated under vacuum, The residue is chromatographed on silica gel (cyclohexane/ethyl acetate 100:0→80:20) to give the title compound. Yield: 700 mg.

### Intermediate 50w

### 4-(4-Bromo-2,6-dimethyl-phenoxy)-2-methyl-butan-2-ol

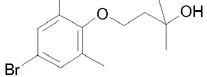

The title compound is prepared in analogy to Intermediate 7 starting from 4-bromo-2,6-dimethyl-phenol.

### Intermediate 50x

### 3-(4-Bromo-3,5-dimethyl-benzenesulfonyl)-propan-1-ol

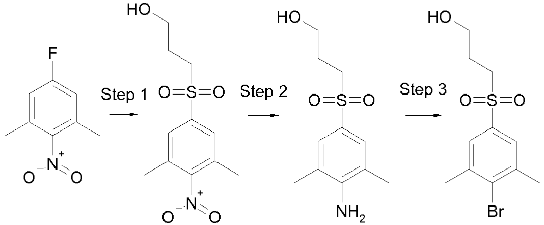

### Step 1: 3-(3,5-Dimethyl-4-nitro-benzenesulfonyl)-propan-1-ol

5-Fluoro-1,3-dimethyl-2-nitrobenzene (2 g), 3-mercapto-1-propanol (1.5 mL) and potassium carbonate (2.4 g) are stirred in 20 mL of N,N-dimethylformamide at 60 °C for 4 h. The reaction mixture is cooled to 0 °C and 3-chloroperbenzoic acid (10 g) is added. The reaction mixture is stirred at room temperature for 1 h, water is added and the reaction mixture is extracted with ethyl acetate. The organic phase is washed with a bicarbonate saturated aqueous solution, with a 5% sodium sulfite water solution and with brine. The solvent is remove to give the title cmpound, used in the next step without further purification. Yield: 2.8 g.

### Step 2: 3-(4-Amino-3,5-dimethyl-benzenesulfonyl)-propan-1-ol

3-(3,5-Dimethyl-4-nitro-benzenesulfonyl)-propan-1-ol is dissolved in 15 mL of ethanol, Pd/C(5%) (55 mg) is added and the reaction mixture is stirred under hydrogen atmosphere (3 bar) for 3 h. The reaction mixture is filtered on a celite pad, the solvent is concentrated under vacuum to give the title compound. Yield: 1 g.

### Step 3: 3-(4-Bromo-3,5-dimethyl-benzenesulfonyl)-propan-1-ol

The title compound is prepared following a procedure analogous to Step 3 in the preparation of Intermediate 50uh, starting from 2: 3-(4-Amino-3,5-dimethyl-benzenesulfonyl)-propan-1-ol. Yield: 740 mg.

### Intermediate 50y

### 2-Bromo-5-(3-methoxy-propane-1-sulfonyl)-1,3-dimethyl-benzene

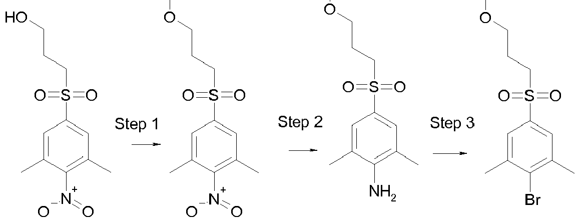

### Step 1: 5-(3-Methoxy-propane-1-sulfonyl)-1,3-dimethyl-2-nitro-benzene

3-(3,5-Dimethyl-4-nitro-benzenesulfonyl)-propan-1-ol (1.4 g) is dissolved in 20 mL of acetonitrile, cesium carbonate (3.34 g) and iodometane (638 mL) are added and the reaction mixture is stirred at room temperature overnight. Water is added and the reaction mixture is extracted with ethyl ether. The organic phase is separated, dried over sodium sulfate and concentrated under vacuum. The residue is chromatographed on silica gel (hexane/ethyl acetate 100:0→50.50) to give the title compound. Yield: 1 g.

### Step 2: 4-(3-Methoxy-propane-1-sulfonyl)-2,6-dimethyl-phenylamine

The title compound is prepared following a procedure analogous to Step 2 in the preparation of Intermediate 50x, starting from 5-(3-Methoxy-propane-1-sulfonyl)-1,3-dimethyl-2-nitro-benzene

### Step 3: 2-Bromo-5-(3-methoxy-propane-1-sulfonyl)-1,3-dimethyl-benzene

The title compound is prepared following a procedure analogous to Step 3 in the preparation of Intermediate 50uh, starting from 4-(3-Methoxy-propane-1-sulfonyl)-2,6-dimethyl-phenylamine.

### Intermediate 50z

### 4-(2-Bromo-pyrimidin-5-yloxy)-2-methyl-butan-2-ol

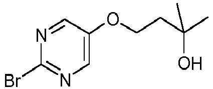

3-Methyl-1,3-butanediol (180 mg is stirred in 5 mL of N,N-dimethylformamide at 0 °C, sodium hydride (6 mg of a 60% mineral oil suspension) is added and the reaction mixture is stirred for 2 h, then it is added dropwise to a solution of 2-bromo-4-fluoro-pyrimidine (300 mg) in 5 mL of N,N- dimethylformamide and the reaction mixture stirred at 0 °C for 2 h again. The solvent is removed and the residue is purified by flash chromatography (hexane/ethyl acetate 90:10→70:30) to give the title compound. Yield: 20 mg.

### Intermediate 51a

### Methyl 2-((S)-6-((R)-4-(2,6-dimethyl-4-(1-(methylsulfonyl)azetidin-3-yloxy)phenyl)-7-fluoro-2,3-dihydro-1H-inden-1-yloxy)-2,3-dihydrobenzofuran-3-yl)acetate

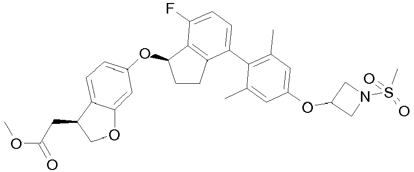

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (130 mg), intermediate 50g (104 mg), K₃PO₄ (133 mg) are suspended in toluene (2 mL) and water (200 µL) and purged for 10 minutes with nitrogen. Palladium-(II)-acetate (5 mg) and dicyclohexyl(2',6'-dimethoxybiphenyl-2-yl)phosphine (S-Phos) (8.5 mg) are added, the vial is sealed and the mixture is stirred at 120°C for 1 hours (100 W). After cooling to ambient temperature the mixture is partitioned between diethylether and saturated aqueous NH₄Cl solution. The organic phase is dried (MgSO₄) and concentrated. The residue is chromatographed on silica gel (cyclohexane/ethyl acetate 100:0→50:50) to give the title compound. Yield: 55 mg.

The intermediates in the following table are prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and the corresponding starting bromo-aryl intermediates following a procedure analogous to that described for Intermediate 51a.

| **Starting bromoaryl Intermediate** | **Intermediate** | **Molecular structure and name** |
|---|---|---|
| 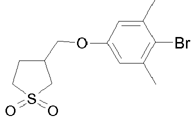 | **51b** | 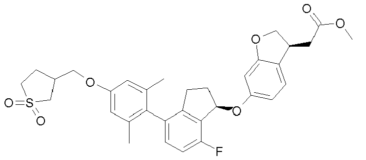 |
| **50a** | | Methyl 2-((3S)-6-((1R)-4-(2,6-dimethyl-4-((1,1-dioxo-tetrahydrothiophen-3-yl)methoxy)phenyl)-7-fluoro-2,3-dihydro-1H-inden-1-yloxy)-2,3-dihydrobenzofuran-3-yl)acetate |
| 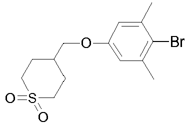 | **51c** | 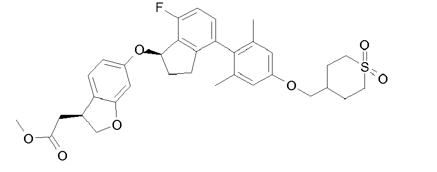 |
| **50b** | | Methyl 2-((S)-6-((R)-4-(2,6-dimethyl-4-((1,1-dioxo-tetrahydro-2H-thiopyran-4-yl)methoxy)phenyl)-7-fluoro-2,3-dihydro-1H-inden-1-yloxy)-2,3-dihydrobenzofuran-3-yl)acetate |
| 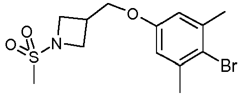 | **51d** | 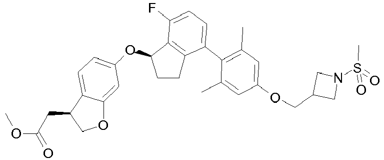 |
| **50d** | | Methyl 2-((S)-6-((R)-4-(2,6-dimethyl-4-((1-(methylsulfonyl)azetidin-3-yl)methoxy)phenyl)-7-fluoro-2,3-dihydro-1H-inden-1-yloxy)-2,3-dihydrobenzofuran-3-yl)acetate |
| 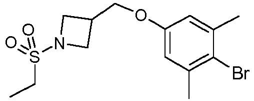 | **51e** | 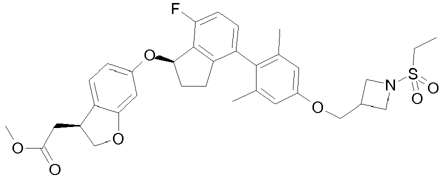 |
| **50e** | | Methyl 2-((S)-6-((R)-4-(4-((1-(ethylsulfonyl)azetidin-3-yl)methoxy)-2,6-dimethylphenyl)-7-fluoro-2,3-dihydro-1H-inden-1-yloxy)-2,3-dihydrobenzofuran-3-yl)acetate |
| 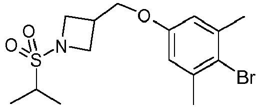 | **51f** | 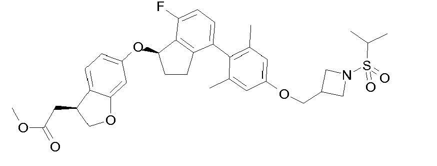 |
| **50f** | | Methyl 2-((S)-6-((R)-7-fluoro-4-(4-((1-(isopropylsulfonyl)azetidin-3-yl)methoxy)-2,6-dimethylphenyl)-2,3-dihydro-1H-inden-1-yloxy)-2,3-dihydrobenzofuran-3-yl)acetate |
| 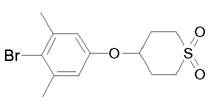 | **51g** | 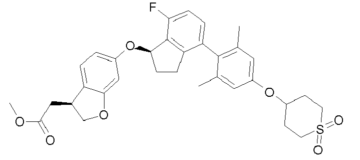 |
| **50h** | | Methyl 2-((S)-6-((R)-4-(2,6-dimethyl-4-(1,1-dioxo-tetrahydro-2H-thiopyran-4-yloxy)phenyl)-7-fluoro-2,3-dihydro-1H-inden-1-yloxy)-2,3-dihydrobenzofuran-3-yl)acetate |
| 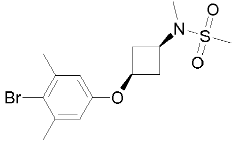 | **51h** | 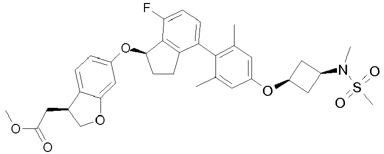 |
| **50i** | | Methyl 2-((S)-6-((R)-4-(2,6-dimethyl-4-((1s,3S)-3-(N-methylmethylsulfonamido)cyclobutoxy)phenyl)-7-fluoro-2,3-dihydro-1H-inden-1-yloxy)-2,3-dihydrobenzofuran-3-yl)acetate |
| 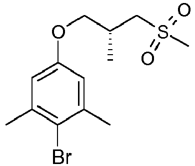 | **51n** | 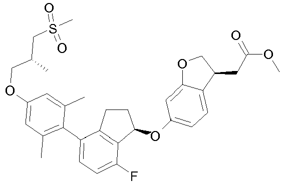 |
| **50m** | | ((S)-6-{(R)-7-Fluoro-4-[4-((R)-3-me thanesulfonyl-2-methyl-propoxy)-2,6 -dimethyl-phenyl]-indan-1-yloxy}-2, 3-dihydro-benzofuran-3-yl)-acetic a cid methyl ester |
| 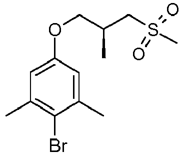 | **51o** | 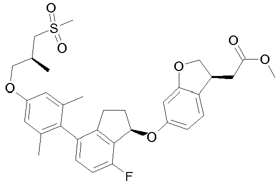 |
| **50n** | | ((S)-6-{(R)-7-Fluoro-4-[4-((S)-3-me thanesulfonyl-2-methyl-propoxy)-2,6-dimethyl-phenyl]-indan-1-yloxy}-2, 3-dihydro-benzofuran-3-yl)-acetic a cid methyl ester |
| 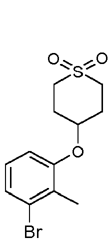 | **51p** | 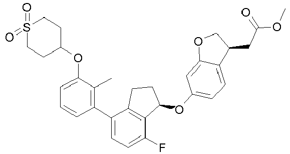 |
| **50l** | | ((S)-6-{(R)-4-[3-(1,1-Dioxo-hexahyd ro-1--thiopyran-4-yloxy)-2-methyl-phenyl]-7-fluoro-indan-1-ylo xy}-2,3-dihydro-benzofuran-3-yl)-ac etic acid methyl ester |
| 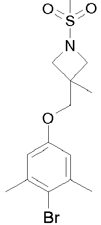 | **51q** | 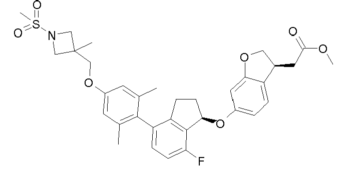 |
| **50gb** | | ((S)-6-{(R)-7-Fluoro-4-[4-(1-methan esulfonyl-3-methyl-azetidin-3-ylmet hoxy)-2,6-dimethyl-phenyl]-indan-1-yloxy}-2,3-dihydro-benzofuran-3-yl) -acetic acid methyl ester |
| 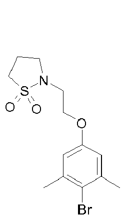 | **51r** | 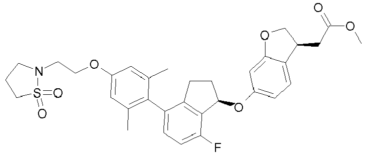 |
| **50o** | | [(S)-6-((R)-4-{4-[2-(1,1-Dioxo-1-isothiazolidin-2-yl)-ethoxy] -2,6-dimethyl-phenyl}-7-fluoro-inda n-1-yloxy)-2,3-dihydro-benzofuran-3 -yl]-acetic acid methyl ester |
| 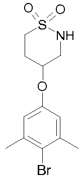 | **51s** | 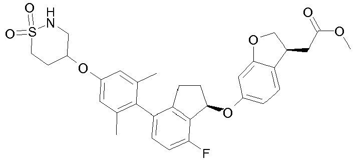 |
| **50p** | | ((S)-6-{(R)-4-[4-(1,1-Dioxo-1--[1,2]thiazinan-4-yloxy)-2,6-dim ethyl-phenyl]-7-fluoro-indan-1-ylox y}-2,3-dihydro-benzofuran-3-yl)-ace tic acid methyl ester |
| 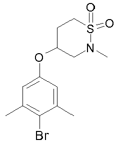 | **51t** | 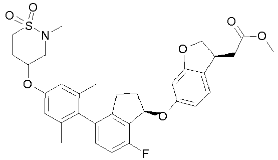 |
| **50q** | | ((S)-6-{(R)-4-[2,6-Dimethyl-4-(2-me thyl-1,1-dioxo-1--1,2]thia zinan-4-yloxy)-phenyl]-7-fluoro-ind an-1-yloxy}-2,3-dihydro-benzofuran-3-yl)-acetic acid methyl ester |
| 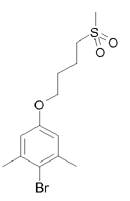 | **51u** | 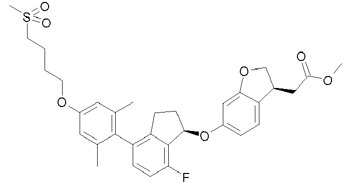 |
| **50ka** | | ((S)-6-{(R)-7-Fluoro-4-[4-(4-methan esulfonyl-butoxy)-2,6-dimethyl-phen yl]-indan-1-yloxy}-2,3-dihydro-benz ofuran-3-yl)-acetic acid methyl ester |
| 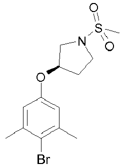 | **51v** | 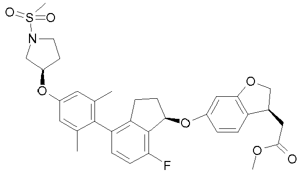 |
| **50gc** | | ((S)-6-{(R)-7-Fluoro-4-[4-((R)-1-me thanesulfonyl-pyrrolidin-3-yloxy)-2 ,6-dimethyl-phenyl]-indan-1-yloxy}-2,3-dihydro-benzofuran-3-yl)-acetic acid methyl ester |
| 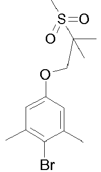 | **51w** | 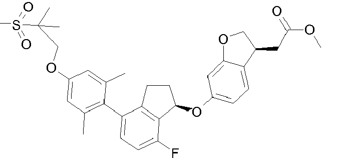 |
| **50ha** | | ((S)-6-{(R)-7-Fluoro-4-[4-(2-methan esulfonyl-2-methyl-propoxy)-2,6-dim ethyl-phenyl]-indan-1-yloxy}-2,3-di hydro-benzofuran-3-yl)-acetic acid methyl ester |
| 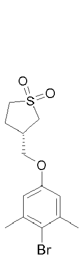 | **51x** | 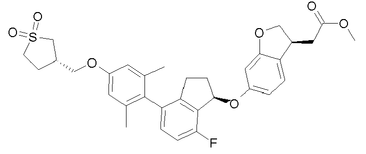 |
| **50aa** | | ((S)-6-{(R)-4-[4-((S)-1,1-Dioxo-tet rahydro-1-thiophen-3-ylmet hoxy)-2,6-dimethyl-phenyl]-7-fluoro -indan-1-yloxy}-2,3-dihydro-benzofu ran-3-yl)-acetic acid methyl ester Absolute configuration unknown. |
| 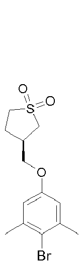 | **51y** | 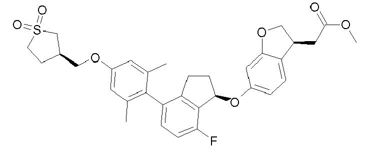 |
| **50aa** | | ((S)-6-{(R)-4-[4-((R)-1,1-Dioxo-tet rahydro-1-thiophen-3-ylmet hoxy)-2,6-dimethyl-phenyl]-7-fluoro -indan-1-yloxy}-2,3-dihydro-benzofu ran-3-yl)-acetic acid methyl ester. Absolute configuration unknown. |
| 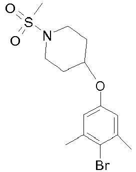 | **51z** | 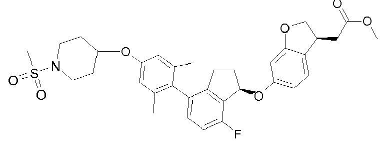 |
| **50ga** | | ((S)-6-{(R)-7-Fluoro-4-[4-(1-methan esulfonyl-piperidin-4-yloxy)-2,6-dimethyl-phenyl]-indan-1-yloxy}-2,3-d ihydro-benzofuran-3-yl)-acetic acid methyl ester |
| 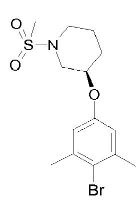 | **51za** | 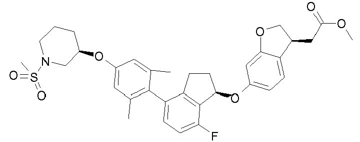 |
| **50gd** | | ((S)-6-{(R)-7-Fluoro-4-[4-((R)-1-me thanesulfonyl-piperidin-3-yloxy)-2, 6-dimethyl-phenyl]-indan-1-yloxy}-2 ,3-dihydro-benzofuran-3-yl)-acetic acid methyl ester |
| 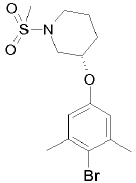 | **51zb** | 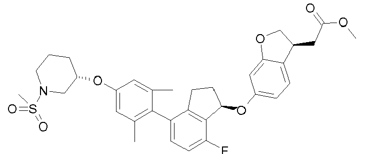 |
| **50ge** | | ((S)-6-{(R)-7-Fluoro-4-[4-((S)-1-me thanesulfonyl-piperidin-3-yloxy)-2, 6-dimethyl-phenyl]-indan-1-yloxy}-2 ,3-dihydro-benzofuran-3-yl)-acetic acid methyl ester |
| 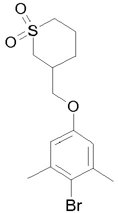 | **51zc** | 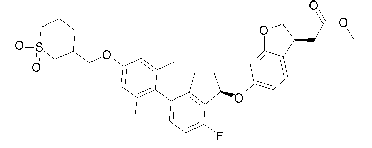 |
| **50ba** | | ((S)-6-{(R)-4-[4-(1,1-Dioxo-hexahyd ro-1-thiopyran-3-ylmethoxy )-2,6-dimethyl-phenyl]-7-fluoro-ind an-1-yloxy}-2,3-dihydro-benzofuran-3-yl)-acetic acid methyl ester |
| 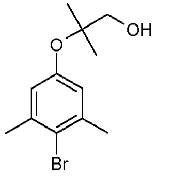 | **51zd** | 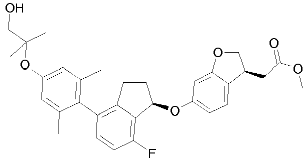 |
| **50r** | | ((S)-6-{(R)-7-Fluoro-4-[4-(2-hydrox y-1,1-dimethyl-ethoxy)-2,6-dimethyl-phenyl]-indan-1-yloxy}-2,3-dihydro-benzofuran-3-yl)-acetic acid methyl ester |
| 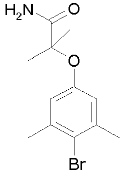 | **51zeb** | 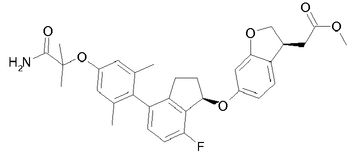 |
| **50s** | | ((S)-6-{(R)-4-[4-(1-Carbamoyl-1-met hyl-ethoxy)-2,6-dimethyl-phenyl]-7-fluoro-indan-1-yloxy}-2,3-dihydro-b enzofuran-3-yl)-acetic acid methyl ester |
| 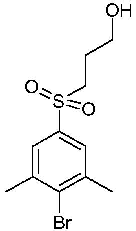 | **51zf** | 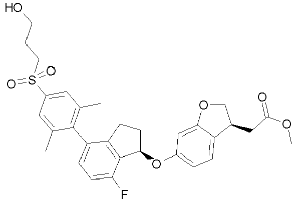 |
| 50x | | ((S)-6-{(R)-7-Fluoro-4-[4-(3-hydrox y-propane-1-sulfonyl)-2,6-dimethyl-phenyl]-indan-1-yloxy}-2,3-dihydro-benzofuran-3-yl)-acetic acid methyl ester |
| 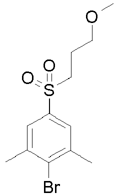 | **51zg** | 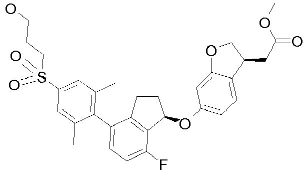 |
| 50y | | (6-{7-Fluoro-4-[4-(3-methoxy-propan e-1-sulfonyl)-2,6-dimethyl-phenyl]-indan-1-yloxy}-2,3-dihydro-benzofur an-3-yl)-acetic acid methyl ester |

### Intermediate 51i

### Methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate

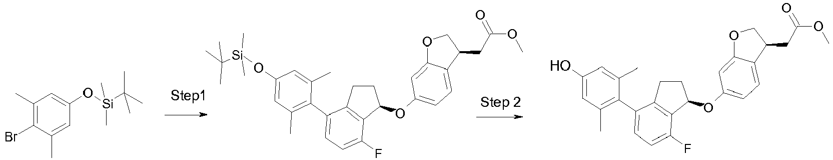

### Step 1: Methyl 2-((S)-6-((R)-4-(4-(tert-butyldimethylsilyloxy)-2,6-dimethylphenyl)-7-fluoro-2,3-dihydro-1H-inden-1-yloxy)-2,3-dihydrobenzofuran-3-yl)acetate

Methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (500 mg), Intermediate 50j (500 mg), K₃PO₄ (450 mg) are suspended in toluene (2.5 mL) and water (0.2 mL) and purged for 10 minutes with argon. Palladium-(II)-acetate (12 mg) and dicyclohexyl(2',6'-dimethoxybiphenyl-2-yl)phosphine (S-Phos) (44 mg) are added and the mixture is stirred at 100°C overnight. After cooling to ambient temperature the mixture is partitioned between diethylether and saturated aqueous NH₄Cl solution. The organic phase is dried (MgSO₄) and concentrated. The residue is chromatographed on silica gel (cyclohexane/ethyl acetate 99:1→70:30) to give the title compound. Yield: 440 mg.

### Step 2: Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate

Methyl 2-((S)-6-((R)-4-(4-(tert-butyldimethylsilyloxy)-2,6-dimethylphenyl)-7-fluoro-2,3-dihydro-1H-inden-1-yloxy)-2,3-dihydrobenzofuran-3-yl)acetate (440 mg) and tetrabutylammonium fluoride in tetrahydrofuran (1 M; 3.1 mL) are stirred in tetrahydrofuran (5 mL) at ambient temperature overnight. The reaction mixture is partitioned between diethylether and saturated aqueous NH₄Cl solution. The organic phase is dried (MgSO₄) and concentrated. The residue is chromatographed on silica gel (cyclohexane/ethyl acetate 90:10→60:40) to give the title compound. Yield: 280 mg.

### Intermediate 51j

### Methyl 2-((S)-6-((R)-4-(2,6-dimethyl-4-((S)-1-(methylsulfonyl)pyrrolidin-3-yloxy)phenyl)-7-fluoro-2,3-dihydro-1H-inden-1-yloxy)-2,3-dihydrobenzofuran-3-yl)acetate

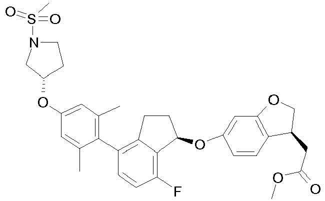

Methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (60 mg), Intermdiate 49e and K₂CO₃ (34 mg) are suspended in N,N-dimethylformamide (5 mL) and stirred at 95°C for 72 hours. The reaction mixture is concentrated under vacuum, partitioned between ethyl acetate and water. The organic phase is dried (MgSO₄) and concentrated. The residue is chromatographed on silica gel (cyclohexane/ethyl acetate 100:0→75:25) to give the title compound. Yield: 29 mg.

### Intermediate 51k

### Methyl 2-((S)-6-((R)-4-(2,6-dimethyl-4-(1-(methylsulfonyl)piperidin-4-yl)methoxy)phenyl)-7-fluoro-2,3-dihydro-1H-inden-1-yloxy)-2,3-dihydrobenzofuran-3-yl)acetate

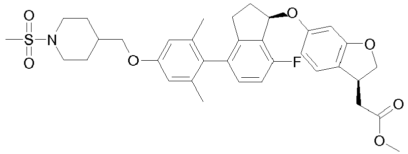

The title compound is prepared in analogy to Intermediate 51j starting from Intermediate 49f and Intermediate 51 i.

### Intermediate 51l

### Methyl 2-((S)-6-((R)-4-(2,6-dimethyl-4-(1,1-dioxo-tetrahydro-2H-thiopyran-4-yloxy)phenyl)-5,7-difluoro-2,3-dihydro-1H-inden-1-yloxy)-2,3-dihydrobenzofuran-3-yl)acetate

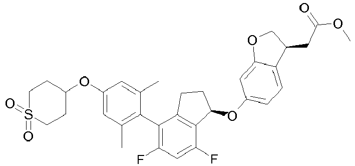

The title compound is prepared in analogy to Intermediate 51a, starting from Intermediate 50h and Intermediate 52.

### Intermediate 51la

### ((S)-6-{(R)-5,7-Difluoro-4-[4-(1-methanesulfonyl-azetidin-3-ylmethoxy)-2,6-dimethylphenyl]-indan-1-yloxy}-2,3-dihydro-benzofuran-3-yl)-acetic acid methyl ester

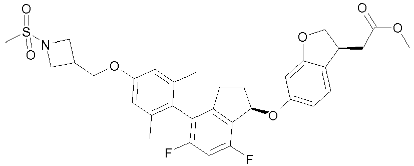

The title compound is prepared in analogy to Intermediate 51 a starting from Intermediate 50d and Intermediate 52.

### Intermediate 51m

### Methyl 2-((3S)-6-((1R)-7-fluoro-4-(2-methyl-3-(3-(methylsulfonyl)propoxy)phenyl)-2,3-dihydro-1H-inden-1-yloxy)-2,3-dihydrobenzofuran-3-yl)acetate

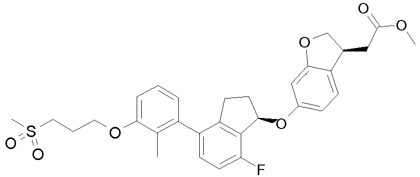

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and Intermediate 50k following a procedure analogous to that described for Intermediate 51 a.
LC (method 17): t_{R} = 1.38 min.

### Intermediate 52

### Methyl 2-((S)-6-((R)-5,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate

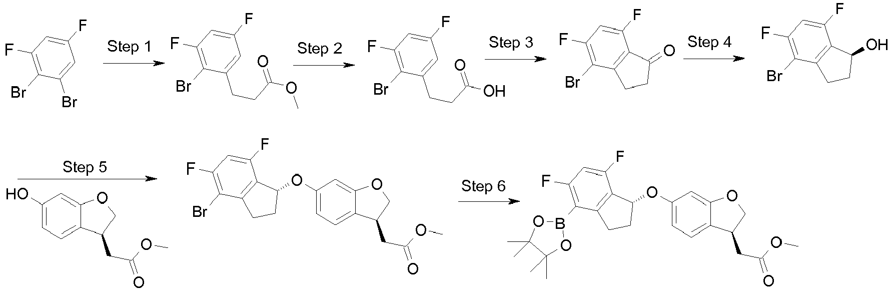

### Step 1: Methyl 3-(2-bromo-3,5-difluorophenyl)propanoate

A mixture of 1,2-dibromo-3,5-difluorobenzene (4.5 g), 3,3-dimethoxyprop-1-ene (5.1 mL), tetrabutylammonium chloride (4.6 g) and triethylamine (3.5 mL) in N,N-dimethylformamide (50 mL) is purged with nitrogen for 20 minutes. Palladium-(II)-acetate (111 mg) is added and the mixture heated for 16 hours at 90°C. After cooling the mixture is partitioned between aqueous HCl (1 M) and diethyl ether, the organic layer dried (Na₂SO₄), volatiles evaporated under reduced pressure and the residue purified by column chromatography (silica gel; n-hexane/ethyl acetate 98:2→80:20) to give the title compound (3.6 g).
GC (method 1): t_{R} = 9.36 min.

### Step 2: 3-(2-Bromo-3,5-difluorophenyl)propanoic acid

Methyl 3-(2-bromo-3,5-difluorophenyl)propanoate (4.3 g) in aqueous NaOH (4 M, 13.5 mL) and 2-propanol (30 mL) is heated at 50°C for 2 hours. Water is added, the mixture washed with diethyl ether, acidified to ca. pH 5 by adding concentrated aqueous HCl, and extracted twice with diethyl ether. The combined extracts of the acidified mixture are washed with brine and volatiles evaporated under reduced pressure. The resulting material (3.6 g) is used in the next step without further purification.
LC (method 16): t_{R} = 0.99 min.

### Step 3: 4-Bromo-5,7-difluoro-2,3-dihydro-1H-inden-1-one

3-(2-Bromo-3,5-difluorophenyl)propanoic acid (3.0 g) is stirred with chlorosulfonic acid (10 mL) for 2 hours at ambient temperature. The mixture is then added dropwise to a vigorously stirred mixture of water and ice (200 mL) and further stirred for 15 minutes. Extraction with diethyl ether, subsequent washing of the organic layer with water, NaOH (0.2 M), and brine, drying (Na₂SO₄) and removal of the solvent under reduced pressure gives the crude product (2.7 g) that is used in the next step without further purification.
LC (method 16): t_{R} = 1.16 min.

### Step 4: (S)-4-Bromo-5,7-difluoro-2,3-dihydro-1H-inden-1-ol

To triethylamine (3.7 mL) in dichloromethane is added dropwise with cooling in an ice bath formic acid (1.3 mL). 4-Bromo-5,7-difluoro-2,3-dihydro-1H-inden-1-one (2.9 g) is added and the mixture degassed with a stream of argon. Chloro([(1S,2S)-(-)-2-amino-1,2-diphenylethyl](4-toluenesulfonyl)amido)(mesitylene)ruthenium(II) is added and the mixture stirred at ambient temperature for 16 hours. The mixture is washed with water, dried (Na₂SO₄) and the solvent removed under reduced pressure. Purification by column chromatography (silica gel; n-hexane/ethyl acetate 98:2→80:20) gives the title compound (2.5 g).
GC (method 1): t_{R} = 9.33 min. Chiral HPLC t_{R} = 9.33 min (Column Daicel Chiralcel OJ-H; 250 x 4.6 mm 5 µm; n-hexane/EtOH 95:5; 1 mL/min; 25°C).

### Step 5: Methyl 2-((S)-6-((R)-4-bromo-5,7-difluoro-2,3-dihydro-1H-inden-1-yloxy)-2,3-dihydrobenzofuran-3-yl)acetate

(S)-4-Bromo-5,7-difluoro-2,3-dihydro-1H-inden-1-ol (1.5 g), (S)-methyl 2-(6-hydroxy-2,3-dihydrobenzofuran-3-yl)acetate (1.4 g), triphenylphosphine (1.7 g) and di-tert-butyl azodicarboxylate (1.5 g) are stirred in tetrahydrofuran (50 mL) for 1 hour. Volatiles are evaporated under reduced pressure and the residue purified by column chromatography (silica gel; n-hexane/ethyl acetate 98:2→80:20) to give the title compound (1.5 g).
LC (method 16): t_{R} = 2.82 min.

### Step 6: Methyl 2-((S)-6-((R)-5,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate

Methyl 2-((S)-6-((R)-4-bromo-5,7-difluoro-2,3-dihydro-1H-inden-1-yloxy)-2,3-dihydrobenzofuran-3-yl)acetate (210 mg), bis(pinacolato)diboron (182 mg), [1,1'-bis(diphenylphosphino)-ferrocene]-dichloropalladium-(II) (35 mg) and potassium acetate (117 mg) are suspended in dry 1,4-dioxane (3 mL) in a microwave vial and degassed for 5 minutes with a flow of argon. The mixture is heated under microwave irradiation for 150 minutes at 120°C, then diluted with ethyl acetate, washed with water, the organic phase dried and the solvent removed under vacuum. The residue is purified by chromatography on silica gel (cyclohexane/ethyl acetate 100:0→80:20) to give the title compound (220 mg).
LC (method 18): t_{R} = 0.75 min; Mass spectrum: m/z = 487 [M+H]⁺.

### Intermediate 52a

### [(S)-6-((R)-4-Bromo-5-trifluoromethyl-indan-1-yloxy)-2,3-dihydro-benzofuran-3-yl]-acetic acid methyl ester

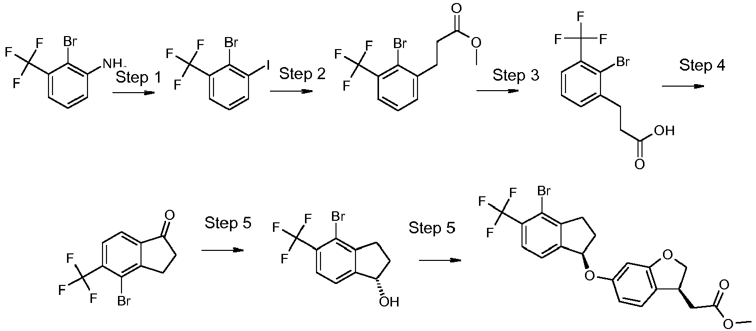

### Step 1: 2-Bromo-1-iodo-3-trifluoromethyl-benzene

2-Bromo-3-trifluoromethyl-phenylamine (8 g) is stirred in 90 mL of water at 0°C, sulfuric acid (96%) (27 mL) is added and the reaction mixture is stirred for 30 minutes. A solution of sodium nitrite (2.40 g) in 5 mL of water is added dropwise and after 30 minutes at 0 °C, potassium iodide (9.24 g) and iodine (9.31 g) are added. After completion of nitrogen evolution, the reaction mixture is warmed to 40 °C for 30 minutes. A saturated sodium sulfite water solution is added and the reaction mixtue is extracted with ethyl acetate. The organic phase dried over sodium sulfate and concentrated under vacuum. The residue is purified by chromatography on silica gel (cyclohexane 100%) to give the title compound. Yield: 10 g.

### Step 2: 3-(2-Bromo-3-trifluoromethyl-phenyl)-propionic acid methyl ester

The title compound is prepared following a procedure analogous to Step 1 in the preparation of Intermediate 17, starting from 2-bromo-1-iodo-3-trifluoromethylbenzene.

### Step 3: 3-(2-Bromo-3-trifluoromethyl-phenyl)-propionic acid

The title compound is prepared following a procedure analogous to Step 2 in the preparation of Intermediate 17, starting from 3-(2-bromo-3-trifluoromethyl-phenyl)-propionic acid methyl ester.

### Step 4: 4-Bromo-5-trifluoromethyl-indan-1-one

3-(2-Bromo-3-trifluoromethyl-phenyl)-propionic acid (3 g), oxalyl chloride (1.8 mL) and few drops of N,N-dimethylformamide are stirred in 30 mL of dichloromethane under reflux for 1 h. The solvent is removed, trifluoromethane sulfonic acid (20 mL) is added and the reaction mixture is stirred at 60 °C for 3 h. The reaction mixture is slowly added dropwise to 300 mL of iced-water, then partitioned between bicarbonate saturated aqueous solution and ethyl acetate. The organic phase dried over sodium sulfate and concentrated under vacuum to give the title compound.
Yield: 2.5 g.

### Step 5: (S)-4-Bromo-5-trifluoromethyl-indan-1-ol

The title compound is obtained following a procedure analogousous to Step 1 in the preparation of Intermediate 1, starting from 4-bromo-5-trifluoromethyl-indan-1-one.

### Step 6: [(S)-6-((R)-4-Bromo-5-trifluoromethyl-indan-1-yloxy)-2,3-dihydro-benzofuran-3-yl]-acetic acid methyl ester

The title compound is obtained following a procedure analogousous to Step 6 in the preparation of Intermediate 1, starting from (S)-4-bromo-5-trifluoromethyl-indan-1-ol.

### Intermediate 52b

### (S)-6-((R)-4-Bromo-7-fluoro-5-trifluoromethyl-indan-1-yloxy)-2,3-dihydro-benzofuran-3-yl]-acetic acid methyl ester

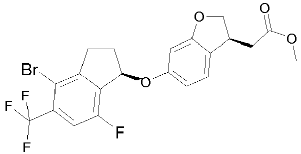

The title compound is prepared following a analogousprocedure analogousous to that described in the preparation of Intermediate 52a, starting in Step 2 with 2,3-dibromo-5-fluoro-benzotrifluoride as starting material.

### Intermediate 52c

### [(S)-6-((R)-4-Bromo-6-trifluoromethyl-indan-1-yloxy)-2,3-dihydro-benzofuran-3-yl]-acetic acid methyl ester

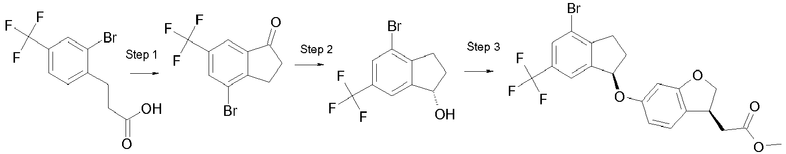

### Step 1: 4-Bromo-6-trifluoromethyl-indan-1-one

3-(2-Bromo-4-trifluoromethyl-phenyl)-propionic acid (US2005/261310A1,2005) (1.45 g) is suspended in 15 mL of trifluoromethanesulfonic acid. The reaction mixture is stirred at 85 °C for 4 h. Iced water and then a bicarbonate saturated aqueous solution are added. The reaction mixture is extracted with ethyl acetate. The organic phase is dried over sodium sulfate and concentrated under vacuum to give the title compound.
Yield: 600 mg.

### Step 2: (S)-4-Bromo-6-trifluoromethyl-indan-1-ol

The title compound is obtained following a procedure analogousous to Step 1 in the preparation of Intermediate 1, starting from 4-bromo-6-trifluoromethyl-indan-1-one.

### Step 3: [(S)-6-((R)-4-Bromo-6-trifluoromethyl-indan-1-yloxy)-2,3-dihydro-benzofuran-3-yl]-acetic acid methyl ester

The title compound is obtained following a procedure analogous to Step 6 in the preparation of Intermediate 1, starting from (S)-4-bromo-6-trifluoromethyl-indan-1-ol.

### Intermediate 52d

### [(S)-6-((R)-4-Bromo-7-fluoro-6-trifluoromethyl-indan-1-yloxy)-2,3-dihydro-benzofuran-3-yl]-acetic acid methyl ester

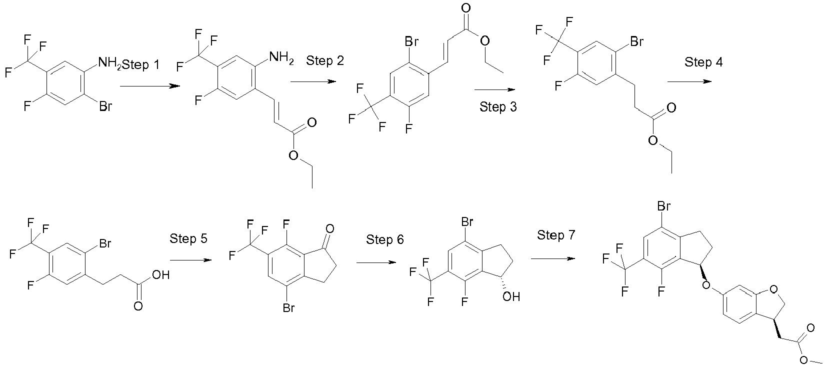

### Step 1: (E)-3-(2-Amino-5-fluoro-4-trifluoromethyl-phenyl)-acrylic acid ethyl ester

2-Bromo-4-fluoro-5-(trifluoromethyl)aniline (15 g), ethylacrylate (17.45 g) tri-o-tolyl-phosphyne (1.77 g), palladium(II) acetate (650 mg), and N,N'-diisopropylethylamine (15 g) are stirred in 100 mL of propionitrile at 100 °C for 12 h. The reaction mixture is concentrated under vacuum. The residue is purified by chromatography on silica gel (cyclohexane/ethyl acetate 100:0→80:20) to give the title compound. Yield: 10 g.

### Step 2: (E)-3-(2-Bromo-5-fluoro-4-trifluoromethyl-phenyl)-acrylic acid ethyl ester

The title compound is prepared following a procedure analogous to Step 3 in the preparation of Intermediate 50uh, starting from (E)-3-(2-amino-5-fluoro-4-trifluoromethyl-phenyl)-acrylic acid ethyl ester.

### Step 3: 3-(2-Bromo-5-fluoro-4-trifluoromethyl-phenyl)-propionic acid ethyl ester

(E)-3-(2-Bromo-5-fluoro-4-trifluoromethyl-phenyl)-acrylic acid ethyl ester (10 g) and hydrazine hydrate (5 mL) are dissolved in 180 mL of ethanol, the reaction mixture is cooled to 0 °C and hydrogen peroxide (12 mL) is added dropwise. After 10 minutes, the reaction mixture is warmed to 70 °C for 2h. The reaction mixture is concentrated under vacuum, the residue is partitioned between water and ethyl acetate. The organic phase dried over sodium sulfate and concentrated under vacuum to give the title compound. Yield: 10 g.

### Step 4: 3-(2-Bromo-5-fluoro-4-trifluoromethyl-phenyl)-propionic acid

The title compound is prepared following a procedure analogous to Step 2 in the preparation of Intermediate 17, starting from 3-(2-bromo-5-fluoro-4-trifluoromethylphenyl)-propionic acid ethyl ester.

### Step 5: 4-Bromo-7-fluoro-6-trifluoromethyl-indan-1-one

The title compound is prepared following a procedure analogous to Step 1 in the preparation of Intermediate 52c, starting from 3-(2-bromo-5-fluoro-4-trifluoromethylphenyl)-propionic acid.

### Step 6: (S)-4-Bromo-7-fluoro-6-trifluoromethyl-indan-1-ol

The title compound is obtained following a procedure analogous to Step 1 in the preparation of Intermediate 1, starting from 4-bromo-7-fluoro-6-trifluoromethyl-indan-1-one.

### Step 7: [(S)-6-((R)-4-Bromo-7-fluoro-6-trifluoromethyl-indan-1-yloxy)-2,3-dihydro-benzofuran-3-yl]-acetic acid methyl ester

The title compound is obtained following a procedure analogous to Step 6 in the preparation of Intermediate 1, starting from (S)-4-bromo-7-fluoro-6-trifluoromethyl-indan-1-ol.

### Intermediate 52aa

### {(S)-6-[(R)-4-(4-Hydroxy-phenyl)-5-trifluoromethyl-indan-1-yloxy]-2,3-dihydro-benzofuran-3-yl}-acetic acid methyl ester

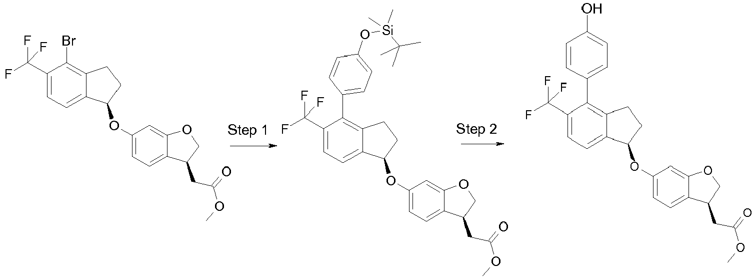

### Step 1: ((S)-6-{(R)-4-[4-(tert-Butyl-dimethyl-silanyloxy)-phenyl]-5-trifluoromethylindan-1-yloxy}-2,3-dihydro-benzofuran-3-yl)-acetic acid methyl ester

Intermediate 52a (1 g), 4-(tert-butyl-dimethylsilyloxy)phenylboronic acid (500 mg), potassium carbonate (822 mg) 2,6-di-tert-butyl-4-methylphenol (218 mg) and tricyclohexyl-phosphine (44 mg) are stirred in 15 mL of 1,4-dioxane and 3 mL of water, tris(dibenzylideneacetone)dipalladium(0) (73 mg) is added and the reaction mixture is stirred at 110 °C for 2 h. Ammonium chloride saturated aqueous solution is added and the reaction mixture is extracted with ethyl acetate. The organic phase is separated, dried over sodium sulfate and concentrated under vacuum. The residue is purified by chromatography on silica gel (cyclohexane/ethyl acetate 100:0→80:20) to give the title compound. Yield: 1 g.

### Step 2: {(S)-6-[(R)-4-(4-Hydroxy-phenyl)-5-trifluoromethyl-indan-1-yloxy]-2,3-dihydro-benzofuran-3-yl}-acetic acid methyl ester

((S)-6-{(R)-4-[4-(tert-Butyl-dimethyl-silanyloxy)-phenyl]-5-trifluoromethylindan-1-yloxy}-2,3-dihydro-benzofuran-3-yl)-acetic acid methyl ester (570 mg) is dissolved in 50 mL of tetrahydrofurane. Tetrabutylammonium fluoride (19 mL) is added and the reaction mixture is stirred at room temperature overnight. The reaction mixture is partitioned between ammonium chloride saturated aqueous solution and ethyl acetate. The organic phase is separated, dried over sodium sulfate and concentrated under vacuum. The residue is purified by chromatography on silica gel (cyclohexane/ethyl acetate 90:10→30:70) to give the title compound. Yield: 80 mg.

### Intermediate 52aa'

### {(S)-6-[(R)-4-(4-Hydroxy-phenyl)-5-trifluoromethyl-indan-1-yloxy]1-2,3-dihydrobenzofuran-3-yl}-acetic acid

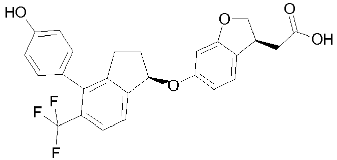

The title compound is obtained by further elution from the column with dichloromethane/methanol 80:20 in the preparation of intermediate 52aa.

### Intermediate 52aa"

### {(S)-6-[(R)-4-(2-Fluoro-4-hydroxy-phenyl)-5-trifluoromethyl-indan-1-yloxy]-2,3-dihydro-benzofuran-3-yl}-acetic acid methyl ester

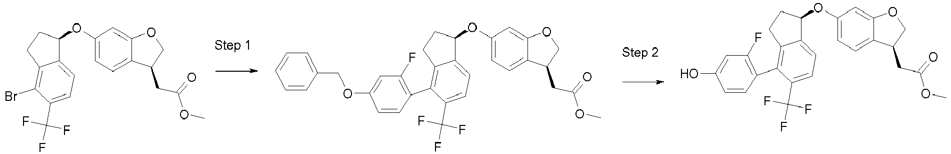

### Step 1: {(S)-6-[(R)-4-(4-Benzyloxy-2-fluoro-phenyl)-5-trifluoromethyl-indan-1-yloxy]-2,3-dihydro-benzofuran-3-yl}-acetic acid methyl ester

The title compound is prepared following a procedure analogous to Step 1 in the preparation of Intermediate 52aa, starting from 2-fluoro-4-(benzyloxy)phenylboronic acid.

### Step 2: {(S)-6-[(R)-4-(2-Fluoro-4-hydroxy-phenyl)-5-trifluoromethyl-indan-1-yloxy]-2,3-dihydro-benzofuran-3-yl}-acetic acid methyl ester

{(S)-6-[(R)-4-(4-Benzyloxy-2-fluoro-phenyl)-5-trifluoromethyl-indan-1-yloxy]-2,3-dihydro-benzofuran-3-yl}-acetic acid methyl ester (500 mg) is dissolved in 10 mL of tetrahydrofurane and 20 mL of methanol, Pd/C(5%) (10 mg) is added and the reaction mixture is stirred under hydrogen atmophere (50 psi) for 2 h.

The reaction mixture is filtered on a celite pad and the solvent is concentrated under vacuum. The residue is purified by chromatography on silica gel (cyclohexane/ethyl acetate 100:0→75:25) to give the title compound. Yield: 300 mg.

### Intermediate 52aaa

### ((S)-6-{(R)-4-[4-(1,1-Dioxo-hexahydro-1-thiopyran-4-ylmethoxy)-phenyl]-5-trifluoromethyl-indan-1-yloxy}-2,3-dihydro-benzofuran-3-yl)-acetic acid methyl ester

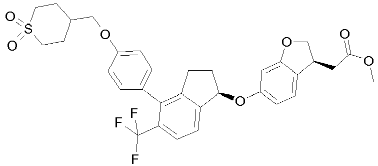

The title compound is prepared following a procedure analogous to the preparation of intermediate 50tb, starting from Intermediate 52aa and mesylate intemediate 49ga.

The following intermediates are prepared in analogy to Intermediate 52aaa starting from Intermediate 52aa and the corresponding mesylate intermediates.

| **Starting mesylate Intermediate** | **Intermediate** | **Molecular structure and name** |
|---|---|---|
| 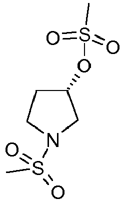 | **52aab** | 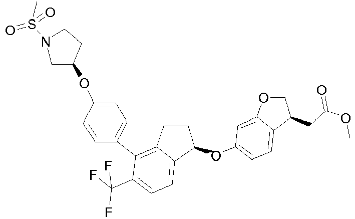 |
| 49ea | | ((S)-6-{(R)-4-[4-((R)-1-Methanesulfonyl-pyrrolidin-3-yloxy)-phenyl]-5-trifluoromethyl-indan-1-yloxy}-2,3-dihydro-benzofuran-3-yl)-acetic acid methyl ester |
| 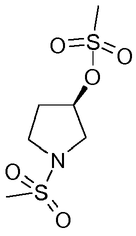 | **52aac** | 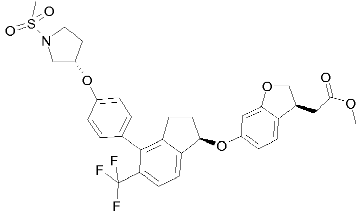 |
| 49ea | | ((S)-6-{(R)-4-[4-((R)-1-Methanesulfonyl-pyrrolidin-3-yloxy)-phenyl]-5-trifluoromethyl-indan-1-yloxy}-2,3-dihydro-benzofuran-3-yl)-acetic acid methyl ester |
| 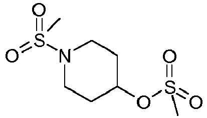 | **52aad** | 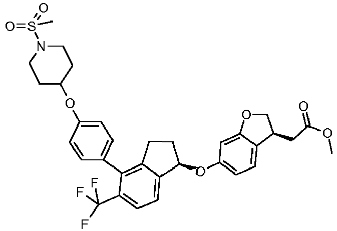 |
| 49fa | | ((S)-6-{(R)-4-[4-(1-Methanesulfonyl-piperidin-4-yloxy)-phenyl]-5-trifluoromethyl-indan-1-yloxy}-2,3-dihydro-benzofuran-3-yl)-acetic acid methyl ester |
| 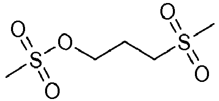 | **52aae** | 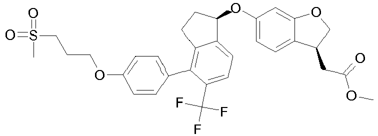 |
| US2011/130398 A1, 2011 | | ((S)-6-{(R)-4-[4-(3-Methanesulfonyl-propoxy)-phenyl]-5-trifluoromethyl-indan-1-yloxy}-2,3-dihydro-benzofuran-3-yl)-acetic acid methyl ester |
| 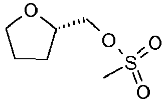 | **52aaf** | 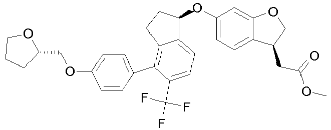 |
| 49gb | | [(S)-6-((R)-4-{4-[(S)-1-(Tetrahydro-furan-2-yl)methoxy]-phenyl}-5-trifluoromethyl-indan-1-yloxy)-2,3-dihydro-benzofuran-3-yl]-acetic acid methyl ester |
| 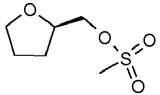 | **52aag** | 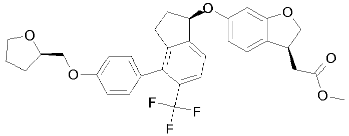 |
| 49gb | | [(S)-6-((R)-4-{4-[(R)-1-(Tetrahydro-furan-2-yl)methoxy]-phenyl}-5-trifluoromethyl-indan-1-yloxy)-2,3-dihydro-benzofuran-3-yl]-acetic acid methyl ester |
| 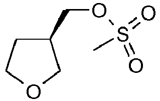 | **52aah** | 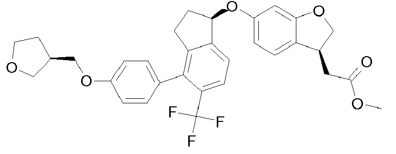 |
| 49gd | | [(S)-6-((R)-4-{4-[(R)-1-(Tetrahydro-furan-3-yl)methoxy]-phenyl}-5-trifluoromethyl-indan-1-yloxy)-2,3-dihydro-benzofuran-3-yl]-acetic acid methyl ester |

### Intermediate 52aai

### ((S)-6-{(R)-4-[4-(Tetrahydro-pyran-4-yloxy)-phenyl]-5-trifluoromethyl-indanyloxy}-2,3-dihydro-benzofuran-3-yl)-acetic acid methyl ester

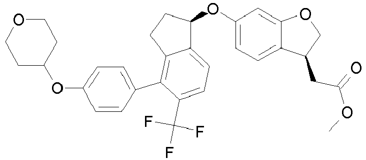

The title compound is prepared following a procedure analogous to the preparation of intermediate 50tc, starting from Intermediate 52aa and tosylate Intermediate 49ha.

The following intermediates are prepared in analogy to Intermediate 52aai starting from Intermediate 52aa and the corresponding tosylate intermediates.

| **Starting tosylate Intermediate** | **Intermediate** | **Molecular structure and name** |
|---|---|---|
| 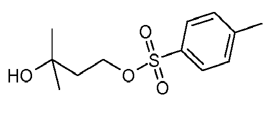 | **52aaj** | 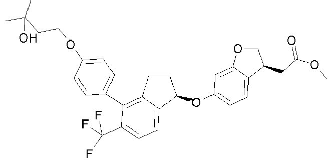 |
| Toluene-4-sulfonic acid 3-hydroxy-3 -methyl-butyl ester (Intermediate 7; step 1) | | ((S)-6-{(R)-4-[4-(3-Hydroxy-3-methyl-butoxy)-phenyl]-5-trifluoromethyl-indan-1-yloxy}-2,3-dihydro-benzofuran-3-yl)-acetic acid methyl ester |
| 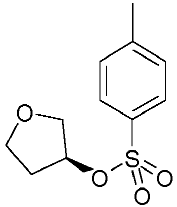 | **52aak** | 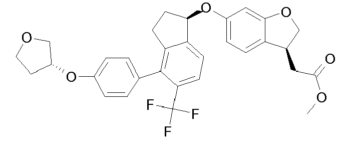 |
| 49hb | | [(S)-6-((R)-4-{4-[(R)-(Tetrahydro-furan-3-yl)oxy]-phenyl}-5-trifluoromethyl-indan-1-yloxy)-2,3-dihydro-benzofuran-3-yl]-acetic acid methyl ester |
| 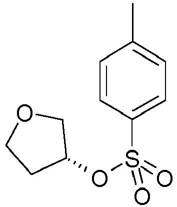 | **52aal** | 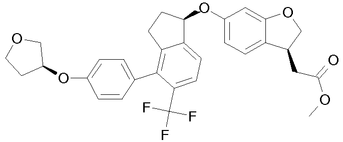 |
| 49hc | | [(S)-6-((R)-4-{4-[(S)-(Tetrahydro-furan-3-yl)oxy]-phenyl}-5-trifluoromethyl-indan-1-yloxy)-2,3-dihydro-benzofuran-3-yl]-acetic acid methyl ester |
| 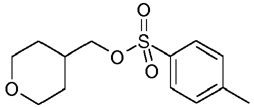 | **52aam** | 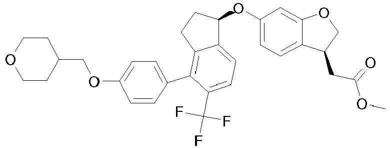 |
| 49hd | | ((S)-6-{(R)-4-[4-(Tetrahydro-pyran-4-ylmethoxy)-phenyl]-5-trifluoromethyl-indan-1-yloxy}-2,3-dihydro-benzofuran-3-yl)-acetic acid methyl ester |

### Intermediate 52aan

### ((S)-6-{(R)-4-[4-(4-Hydroxy-tetrahydro-pyran-4-ylmethoxy)-phenyl]-5-trifluoromethyl-indan-1-yloxy}-2,3-dihydro-benzofuran-3-yl)-acetic acid methyl ester

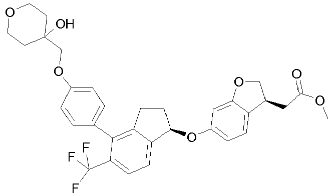

The title compound is prepared following a procedure analogous to the preparation of intermediate 50bb, starting from Intermediate 52aa and the epoxide intermediate 1,6-dioxa-spiro[2.5]octane (US2012/46304 A1, 2012)

The following intermediate is prepared in analogy to Intermediate 52aan starting from Intermediate 52aa and the corresponding epoxide intermediates.

| **Starting epoxide** | **Intermediate** | **Molecular structure and name** |
|---|---|---|
| 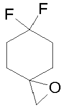 | **52aao** | 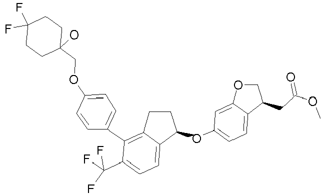 |
| 6,6-Difluoro-1-oxa-spiro[2.5]octane | | |
| Prepared in analogy to 1,6-Dioxa-spiro[2.5]octane starting from 4,4-difluoro-cyclohexanone | | ((S)-6-{(R)-4-[4-(4,4-Difluoro-1-hydroxy-cyclohexylmethoxy)-phenyl]-5-trifluoromethyl-indan-1-yloxy}-2,3-dihydro-benzofuran-3-yl)-acetic acid methyl ester |

### Intermediate 52aap

### ((S)-6-{(R)-4-[4-(1,1-Dioxo-hexahydro-1-thiopyran-4-ylmethoxy)-2-fluoro-phenyl]-5-trifluoromethyl-indan-1-yloxy}-2,3-dihydro-benzofuran-3-yl)-acetic acid methyl ester

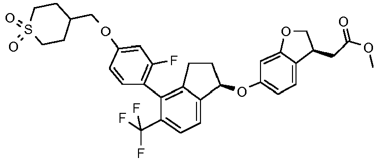

The title compound is prepared in analogy to intermediate 52aaa starting from Intermediate 52aa".

### Intermediate 52ba

### {(S)-6-[(R)-7-Fluoro-4-(4-hydroxy-phenyl)-5-trifluoromethyl-indan-1-yloxy]-2,3-dihydro-benzofuran-3-yl}-acetic acid methyl ester

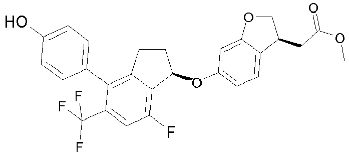

The title compound is prepared in analogy to Intermediate 52aa starting from Intermediate52b.

The following intermediates are prepared in analogy to Intermediate 52aaa or 52aai starting from Intermediate 52ba and the corresponding mesylate or tosylate intermediates.

| **Starting mesylate/tosylate Intermediate** | **Intermediate** | **Molecular structure and name** |
|---|---|---|
| 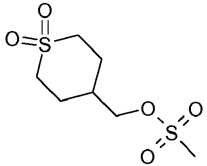 | **52baa** | 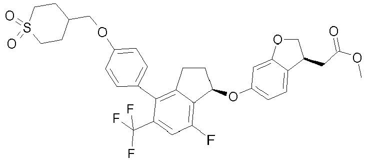 |
| 49ga | | ((S)-6-{(R)-4-[4-(1,1-Dioxo-hexahydro-1-thiopyran-4-ylmethoxy)-phenyl]-7-fluoro-5-trifluoromethyl-indan-1-yloxy}-2,3-dihydro-benzofuran-3-yl)-acetic acid methyl ester |
| 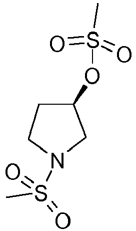 | **52bab** | 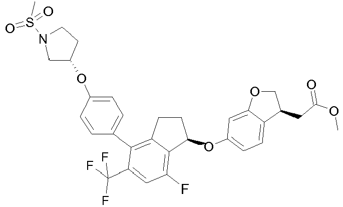 |
| 49e | | ((S)-6-{(R)-7-Fluoro-4-[4-((S)-1-methanesulfonyl-pyrrolidin-3-yloxy)-phenyl]-5-trifluoromethyl-indan-1-yloxy}-2,3-dihydro-benzofuran-3-yl)-acetic acid methyl ester |
| 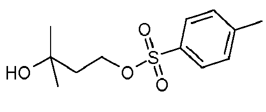 | **52bac** | 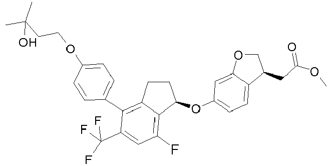 |
| Toluene-4-sulfonic acid 3-hydroxy-3 -methyl-butyl ester (Intermediate 7; step 1) | | ((S)-6-{(R)-7-Fluoro-4-[4-(3-hydroxy-3-methyl-butoxy)-phenyl]-5-trifluoromethyl-indan-1-yloxy}-2,3-dihydro-benzofuran-3-yl)-acetic acid methyl ester |

The following intermediates are prepared in analogy to Intermediate 52aan starting from Intermediate 52ba and the corresponding epoxide intermediates.

| **Starting epoxide** | **Intermediate** | **Molecular structure and name** |
|---|---|---|
| 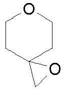 | **52bad** | 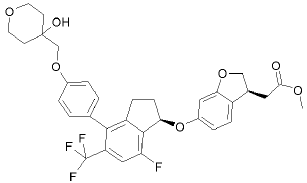 |
| 1,6-Dioxaspiro[2.5]octane | | ((S)-6-{(R)-7-Fluoro-4-[4-(4-hydroxy-tetrahydro-pyran-4-ylmethoxy)-phenyl]-5-trifluoromethyl-indan-1-yloxy}-2,3-dihydro-benzofuran-3-yl)-acetic acid methyl ester |
| 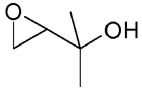 | **52bae** | 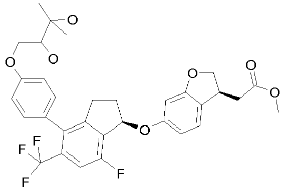 |
| Yamada, Sachiko et al. Chemical & Pharmaceutical Bulletin, 1981, vol. 29, # 4 p. 1187 - 1188 | | ((S)-6-{(R)-4-[4-(2,3-Dihydroxy-3-methyl-butoxy)-phenyl]-7-fluoro-5-trifluoromethyl-indan-1-yloxy}-2,3-dihydro-benzofuran-3-yl)-acetic acid methyl ester |
| | **52baf*** | 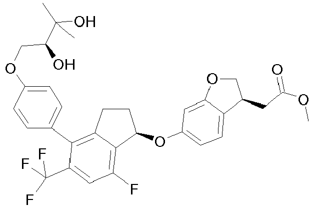 |
| | | ((S)-6-{(R)-4-[4-((S)-2,3-Dihydroxy-3-methyl-butoxy)-phenyl]-7-fluoro-5-trifluoromethyl-indan-1-yloxy}-2,3-dihydro-benzofuran-3-yl)-acetic acid methyl ester Absolute configuration unknown. |
| | **52bag*** | 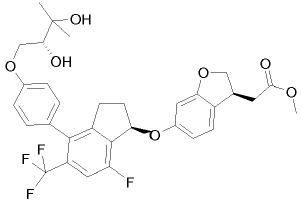 |
| | | ((S)-6-{(R)-4-[4-((R)-2,3-Dihydroxy-3-methyl-butoxy)-phenyl]-7-fluoro-5-trifluoromethyl-indan-1-yloxy}-2,3-dihydro-benzofuran-3-yl)-acetic acid methyl ester Absolute configuration unknown. |

| | | |
|---|---|---|
| *Intermediate 52baf and intermediate 52bag are obtained from chiral HPLC separation of racemate Intermediate 52bae. Intermediate 52baf: first eluted: Chiral HPLC (method 1 b): t_{R} = 12.35 min. Absolute configuration unknown. Intermediate 52bag: second eluted: Chiral HPLC (method 1 b): t_{R} = 12.40 min. Absolute configuration unknown. | | |

### Intermediate 52ca

### {(S)-6-[(R)-4-(4,4,5,5-Tetramethyl-[1,3,2]dioxaborolan-2-yl)-6-trifluoromethyl-indan-1-yloxy]-2,3-dihydro-benzofuran-3-yl}-acetic acid methyl ester

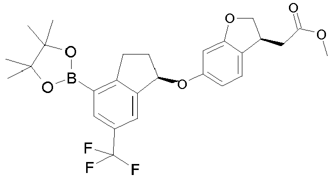

The title compound is prepared in analogy to Step 6 in the preparation of Intermediate 52 starting from Intermediate 52c.

### Intermediate 52caa

### ((S)-6-{(R)-4-[4-(3-Hydroxy-3-methyl-butoxy)-2,6-dimethyl-phenyl]-6-trifluoromethyl-indan-1-yloxy}-2,3-dihydro-benzofuran-3-yl)-acetic acid methyl ester

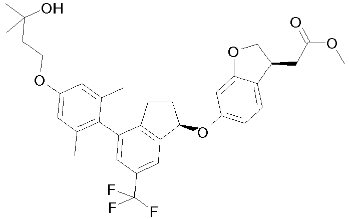

The title compound is prepared starting from Intermediate 52ca and intermediate 7 following a procedure analogous to that described for Intermediate 51 a.

The intermediates in the following table are prepared from Intermediate 52ca and the corresponding starting bromo-aryl intermediates following a procedure analogous to that described for Intermediate 52caa.

| **Starting bromo-aryl Intermediate** | **Intermediate** | **Molecular structure and name** |
|---|---|---|
| 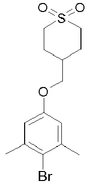 | **52cab** | 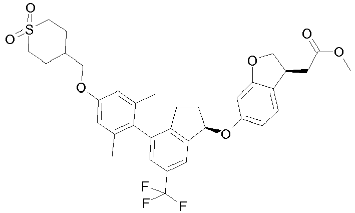 |
| 50b | | ((S)-6-{(R)-4-[4-(1,1-Dioxo-hexahydro-1-thiopyran-4-ylmethoxy)-2,6-dimethyl-phenyl]-6-trifluoromethyl-indan-1-yloxy}-2,3-dihydro-benzofuran-3-yl)-acetic acid methyl ester |
| 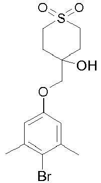 | **52cac** | 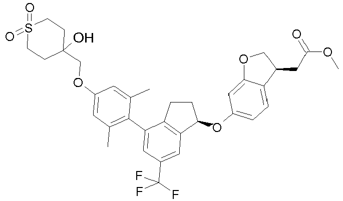 |
| 50bc | | ((S)-6-{(R)-4-[4-(4-Hydroxy-1,1-dioxo-hexahydro-1-thiopyran-4-ylmethoxy)-2,6-dimethyl-phenyl]-6-trifluoromethyl-indan-1-yloxy}-2,3-dihydro-benzofuran-3-yl)-acetic acid methyl ester |
| 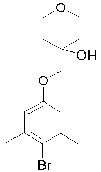 | **52cad** | 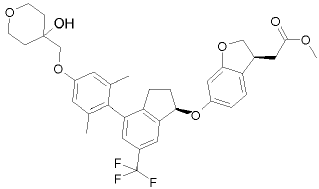 |
| 50bb | | ((S)-6-{(R)-4-[4-(4-Hydroxy-tetrahydro-pyran-4-ylmethoxy)-2,6-dimethyl-phenyl]-6-trifluoromethyl-indan-1-yloxy}-2,3-dihydro-benzofuran-3-yl)-acetic acid methyl ester |
| 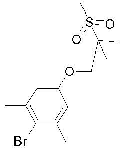 | **52cae** | 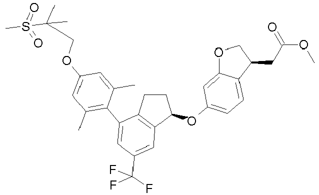 |
| 50ha | | ((S)-6-{(R)-4-[4-(2-Methanesulfonyl-2-methyl-propoxy)-2,6-dimethyl-phenyl]-6-trifluoromethyl-indan-1-yloxy}-2,3-dihydro-benzofuran-3-yl)-acetic acid methyl ester |
| 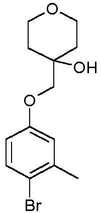 | **52caf** | 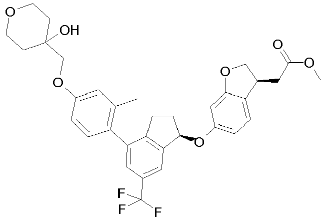 |
| 50ta | | ((S)-6-{(R)-4-[4-(4-Hydroxy-tetrahydro-pyran-4-ylmethoxy)-2-methyl-phenyl]-6-trifluoromethyl-indan-1-yloxy}-2,3-dihydro-benzofuran-3-yl)-acetic acid methyl ester |
| 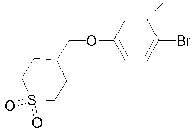 | **52cag** | 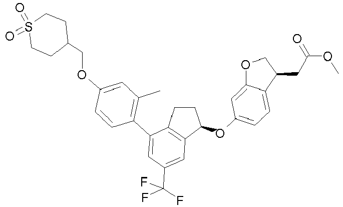 |
| 50tb | | ((S)-6-{(R)-4-[4-(1,1-Dioxo-hexahydro-1-thiopyran-4-ylmethoxy)-2-methyl-phenyl]-6-trifluoromethyl-indan-1-yloxy}-2,3-dihydro-benzofuran-3-yl)-acetic acid methyl ester |
| 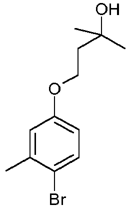 | **52cah** | 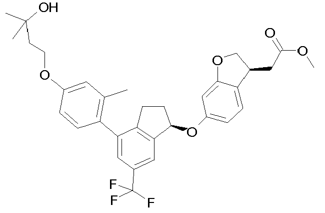 |
| 50tc | | ((S)-6-{(R)-4-[4-(3-Hydroxy-3-methyl-butoxy)-2-methyl-phenyl]-6-trifluoromethyl-indan-1-yloxy}-2,3-dihydro-benzofuran-3-yl)-acetic acid methyl ester |
| 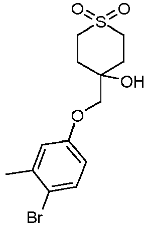 | **52cai** | 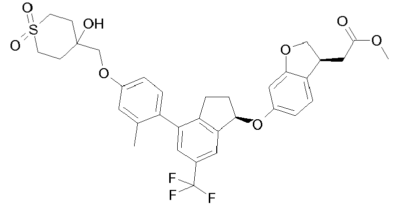 |
| 50td | | ((S)-6-{(R)-4-[4-(4-Hydroxy-1,1-dioxo-hexahydro-1-thiopyran-4-ylmethoxy)-2-methyl-phenyl]-6-trifluoromethyl-indan-1-yloxy}-2,3-dihydro-benzofuran-3-yl)-acetic acid methyl ester |

### Intermediate 52da

### {(S)-6-[(R)-7-Fluoro-4-(4,4,5,5-tetramethyl-[1,3,2]dioxaborolan-2-yl)-6-trifluoromethyl-indan-1-yloxy]-2,3-dihydro-benzofuran-3-yl}-acetic acid methyl ester

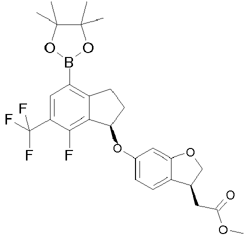

The title compound is prepared in analogy to Step 6 in the preparation of Intermediate 52 starting from Intermediate52d.

The intermediates in the following table are prepared from Intermediate 52da and the corresponding starting bromo-aryl intermediates following a procedure analogous to that described for Intermediate 52caa.

| **Starting bromo-aryl Intermediate** | **Intermediate** | **Molecular structure and name** |
|---|---|---|
| 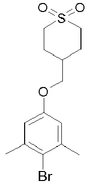 | **52daa** | 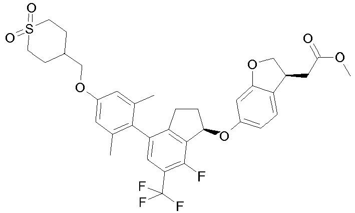 |
| 50b | | ((S)-6-{(R)-4-[4-(1,1-Dioxo-hexahydro-1-thiopyran-4-ylmethoxy)-2,6-dimethyl-phenyl]-7-fluoro-6-trifluoromethyl-indan-1-yloxy}-2,3-dihydro-benzofuran-3-yl)-acetic acid methyl ester |
| 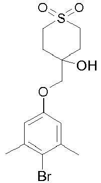 | **52dab** | 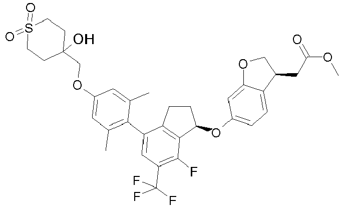 |
| 50bc | | ((S)-6-{(R)-7-Fluoro-4-[4-(4-hydroxy-1,1-dioxo-hexahydro-1-thiopyran-4-ylmethoxy)-2,6-dimethyl-phenyl]-6-trifluoromethyl-indan-1-yloxy}-2,3-dihydro-benzofuran-3-yl)-acetic acid methyl ester |
| 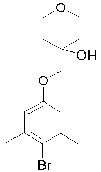 | **52dac** | 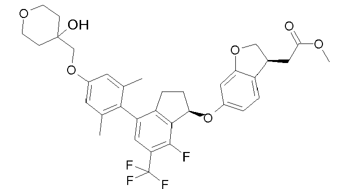 |
| 50bb | | ((S)-6-{(R)-7-Fluoro-4-[4-(4-hydroxy-tetrahydropyran-4-ylmethoxy)-2,6-dimethyl-phenyl]-6-trifluoromethyl-indan-1-yloxy}-2,3-dihydro-benzofuran-3-yl)-acetic acid methyl ester |
| 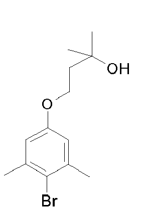 | **52dad** | 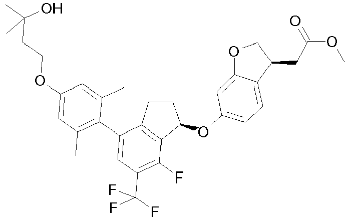 |
| 7 | | ((S)-6-{(R)-7-Fluoro-4-[4-(3-hydroxy-3-methyl-butoxy)-2,6-dimethyl-phenyl]-6-trifluoromethyl-indan-1-yloxy}-2,3-dihydro-benzofuran-3-yl)-acetic acid methyl ester |

### Intermediate 52ea

### {(S)-6-[(R)-4-(4-Hydroxy-2,6-dimethyl-phenyl)-5-trifluoromethyl-indan-1-yloxy]-2,3-dihydro-benzofuran-3-yl}-acetic acid methyl ester

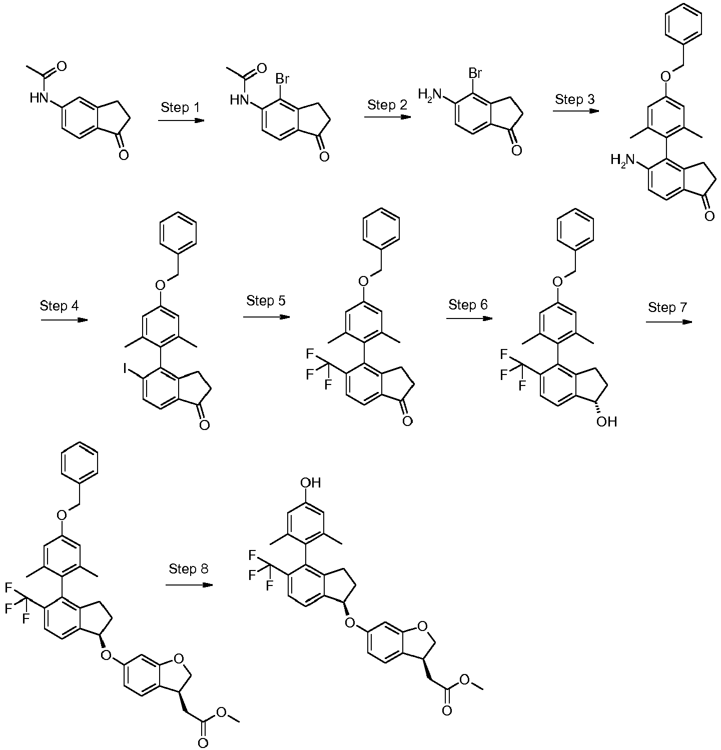

### Step 1: N-(4-Bromo-1-oxo-indan-5-yl)-acetamide

N-(1-Oxo-indan-5-yl)-acetamide (5.0 g) is suspended in a mixture of acetonitrile (40 mL), water (1 mL) and acetic acid (1 mL) and N-bromo succinimide (5.64 g) is added. The mixture is heated at reflux for 6 hours then allowed to cool to room temperature. The title compound is collected by filtration and dried under vacuum. Yield 3.14 g.

### Step 2: 5-Amino-4-bromo-indan-1-one

N-(4-Bromo-1-oxo-indan-5-yl)-acetamide (1.81 g) is suspended in 2 M aqueous hydrochloric acid solution and heated at 100 °C for 2 hours then allowed to cool to room temperature. The title compound is collected by filtration and dried under vacuum.Yield 1.37 g.

### Step 3: 5-Amino-4-(4-benzyloxy-2,6-dimethyl-phenyl)-indan-1-one

Toluene (80 mL) and water (40 mL) are degassed with a flow of nitrogen for 30 minutes and then added to 5-amino-4-bromo-indan-1-one (700 mg), 4-benzyloxy-2,6-dimethyl-phenyl-boronic acid (Example 39, step 2, 3.3 g), and sodium carbonate (1.15 g) in a nitrogen flushed flask. The mixture is heated at reflux overnight under a nitrogen atmosphere then allowed to cool to room temperature, diluted with ethyl acetate (100 mL) and the phases seperated. The organic phase is washed with water and brine, dried over sodium sulfate and the solvent removed under vacuum. The residue is purified by flash chromatography (cyclohexane/ethyl acetate 100:0→70:30) to give the title compound.Yield 985 mg.

### Step 4: 4-(4-Benzyloxy-2,6-dimethyl-phenyl)-5-iodo-indan-1-one

5-Amino-4-(4-benzyloxy-2,6-dimethyl-phenyl)-indan-1-one (985 mg) is suspended in diiodomethane and heated to 100 °C. Isoamyl nitrate (1.47 mL) is added and the mixture stirred at 100 °C for 30 minutes then allowed to cool to room temperature. The excess reagents are removed by distilation under vacuum (90 °C, 8 mbar) and the residue is purified by flash chromatography (cyclohexane/ethyl acetate 100:0→80:20) to give the title compound.Yield 776 mg.

### Step 5: 4-(4-Benzyloxy-2,6-dimethyl-phenyl)-5-trifluoromethyl-indan-1-one

4-(4-Benzyloxy-2,6-dimethyl-phenyl)-5-iodo-indan-1-one (770 mg) is suspended in N-methylpyrrolidone (20 mL) in a flask equipped with an overhead mechanical stirrer and copper(I)iodide (9.39 g) is added followed by methyl 2,2-difluoro-2-(fluorosulfonyl)acetate (4 mL). The mixture is stirred for 30 minutes at 110 °C then further 2,2-difluoro-2-(fluorosulfonyl)acetate (4 mL).is added and the mixture stirred for 90 minutes at 100 °C. A further portion of 2,2-difluoro-2-(fluorosulfonyl)acetate (4 mL) is added and the mixture stirred for 30 minutes at 110 °C then allowed to cooled to room temperature. The mixture is diluted with water (150 mL) and ethyl acetate (150 mL), shaken well and the phases seperated. The organic phase is washed with brine and the solvent removed under vacuum. The residue is suspended in diethyl ether, washed with water and brine, dried over sodium sulfate and the solvent removed under vacuum. The residue is purified by flash chromatography (cyclohexane/ethyl acetate 100:0→80:20) to give the title compound (Yield 452 mg).

### Step 6: (S)-4-(4-Benzyloxy-2,6-dimethyl-phenyl)-5-trifluoromethyl-indan-1-ol

Triethylamine 0.46 mL) is suspended in dichloromethane (2 mL) and cooled to 0 °C. Formic acid (0.15 mL) is added and the mixture stirred for 5 minutes. 4-(4-Benzyloxy-2,6-dimethyl-phenyl)-5-trifluoromethyl-indan-1-one (452 mg) is added followed by chloro([(1S,2S)-(-)-2-amino-1,2-diphenylethyl](4 toluenesulfonyl)amido)(mesitylene)ruthenium(II) (14 mg) and the mixture stirred overnight at room temperature. The solution is diluted with dichloromethane, washed with water, dried over sodium sulfate and the solvent removed under vacuum. The residue is purified by flash chromatography (cyclohexane/ethyl acetate 100:0→80:20) to give the title compound-Yield 494 mg.

### Step 7: {(S)-6-[(R)-4-(4-Benzyloxy-2,6-dimethyl-phenyl)-5-trifluoromethyl-indan-1-yloxy]-2,3-dihydro-benzofuran-3-yl}-acetic acid methyl ester

(S)-4-(4-Benzyloxy-2,6-dimethyl-phenyl)-5-trifluoromethyl-indan-1-ol (494 mg), (S)-(6-hydroxy-2,3-dihydro-benzofuran-3-yl)-acetic acid methyl ester (275 mg), triphenylphosphine (345 mg) and di-tert-butylazodicarboxylate (303 mg) are combined in dry tetrahydrofuran (10 mL) at 0 °C. The mixture is allowed to warm to room temperature and stirred for 2 hours. The solvent is removed under vacuum and the residue is purified by flash chromatography (cyclohexane/ethyl acetate 100:0→80:20) to give the title compound.Yield 573 mg.

### Step 8: {(S)-6-[(R)-4-(4-Hydroxy-2,6-dimethyl-phenyl)-5-trifluoromethyl-indan-1-yloxy]-2,3-dihydro-benzofuran-3-yl}-acetic acid methyl ester

{(S)-6-[(R)-4-(4-Benzyloxy-2,6-dimethyl-phenyl)-5-trifluoromethyl-indan-1-yloxy]-2,3-dihydro-benzofuran-3-yl}-acetic acid methyl ester (350 mg) is suspended in methanol (20 mL) and 10% palladium on carbon (35 mg) is added. The mixture is shaken under a hydrogen atmosphere at 2 Bar for one hour then filtered through celite and the solvent removed. The residue is purified by flash chromatography (cyclohexane/ethyl acetate 100:0→90:10) to give the title compound. Yield 213 mg.

### Intermediate 52eaa

### ((S)-6-{(R)-4-[4-(4-Hydroxy-tetrahydro-pyran-4-ylmethoxy)-2,6-dimethyl-phenyl]-5-trifluoromethyl-indan-1-yloxy}-2,3-dihydro-benzofuran-3-yl)-acetic acid methyl ester

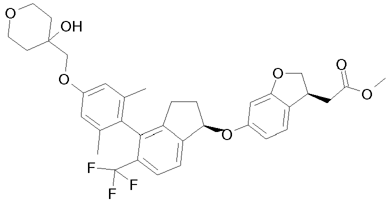

{(S)-6-[(R)-4-(4-Hydroxy-2,6-dimethyl-phenyl)-5-trifluoromethyl-indan-1-yloxy]-2,3-dihydro-benzofuran-3-yl}-acetic acid methyl ester (Intermediate 52ea, 79 mg), 1,6-dioxa-spiro[2.5]octane (30 mg) and cesium carbonate (134 mg) are suspended in dry N,N-dimethylformamide and stirred at 100 °C for 3 hours. An additional portion of 1,6-dioxa-spiro[2.5]octane (30 mg) are added and the mixture is stirred a further 2 hours at 100 °C. The mixture is diluted with ethyl acetate, washed with water, dried over sodium sulfate and the solvent removed under vacuum. The residue is purified by flash chromatography (cyclohexane/ethyl acetate 100:0→60:40) to give the title compound (Yield 35 mg).

### Intermediate 52eab

### ((S)-6-{(R)-4-[4-(4-Hydroxy-1,1-dioxo-hexahydro-1-thiopyran-4-ylmethoxy)-2,6-dimethyl-phenyl]-5-trifluoromethyl-indan-1-yloxy}-2,3-dihydro-benzofuran-3-yl)-acetic acid methyl ester

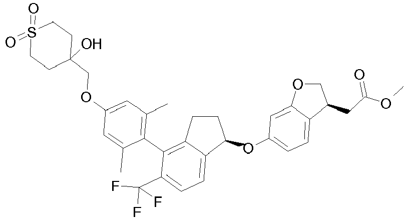

The title compound is prepared following a procedure analogous to that described in the preparation of Intermediate 50bc, starting from Intermediate 52ea.

### Intermediate 52eac

### ((S)-6-{(R)-4-[4-(3-Hydroxy-3-methyl-butoxy)-2,6-dimethyl-phenyl]-5-trifluoromethyl-indan-1-yloxy}-2,3-dihydro-benzofuran-3-yl)-acetic acid methyl ester

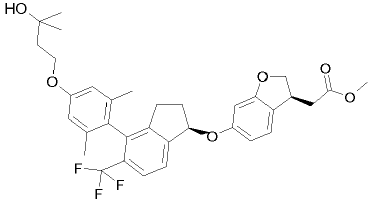

Intermediate 52ea (78 mg), Intermediate 7 (67 mg) and cesium carbonate (133 mg) are suspended in dry N,N-dimethylformamide and stirred at 100 °C for 1 hour. The mixture is diluted with ethyl acetate, washed with water, dried over sodium sulfate and the solvent removed under vacuum. The residue is purified by flash chromatography (cyclohexane/ethyl acetate 100:0→60:40) to give the title compound.Yield 64 mg.

### Intermediate 52ead

### ((S)-6-{(R)-4-[4-(2-Hydroxy-2-methyl-propoxy)-2,6-dimethyl-phenyl]-5-trifluoromethyl-indan-1-yloxy}-2,3-dihydro-benzofuran-3-yl)-acetic acid methyl ester

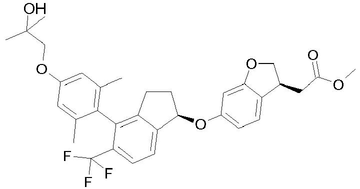

Intermediate 52ea (78 mg), isobutylene oxide (19 mg) and cesium carbonate (133 mg) are suspended in dry N,N-dimethylformamide and stirred at 100 °C for 1 hour. A further portion of isobutylene oxide (48 mg) are added and the mixture is stirred for 2 hours at 100 °C. The mixture is diluted with ethyl acetate, washed with water, dried over sodium sulfate and the solvent removed under vacuum. The residue is purified by flash chromatography (cyclohexane/ethyl acetate 100:0→60:40) to give the title compound.Yield 28 mg.

### Intermediate 52eae

### ((S)-6-{(R)-4-[2,6-Dimethyl-4-(tetrahydro-pyran-4-yloxy)-phenyl]-5-trifluoromethyl-indan-1-yloxy}-2,3-dihydro-benzofuran-3-yl)-acetic acid methyl ester

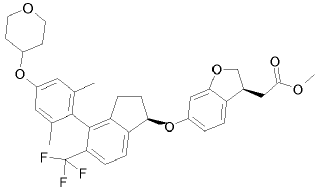

Intermediate 52ea (78 mg), Intermediate 49ha (66 mg) and cesium carbonate (133 mg) are suspended in dry N,N-dimethylformamide and stirred at 100 °C for 1 hour. toluene-4-sulfonic acid tetrahydro-pyran-4-yl ester (66 mg) are added and the mixture is stirred for 2 hours at 100 °C. The mixture is diluted with ethyl acetate, washed with water, dried over sodium sulfate and the solvent removed under vacuum. The residue is purified by flash chromatography (cyclohexane/ethyl acetate 100:0→70:30) to give the title compound.Yield 50 mg.

### Intermediate 52eaf

### ((S)-6-{(R)-4-[2,6-Dimethyl-4-(5-methyl-[1,2,4]oxadiazol-3-yl)-phenyl]-5-trifluoromethyl-indan-1-yloxy}-2,3-dihydro-benzofuran-3-yl)-acetic acid methyl ester

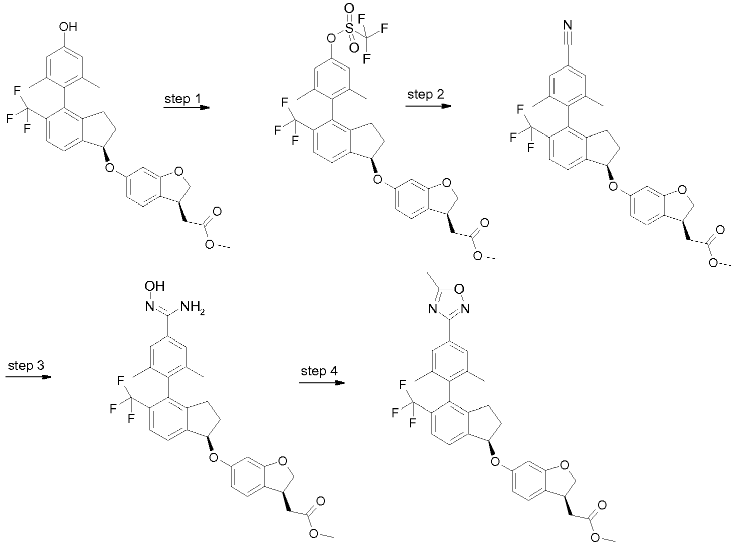

### Step 1: {(S)-6-[(R)-4-(2,6-Dimethyl-4-trifluoromethanesulfonyloxy-phenyl)-5-trifluoromethyl-indan-1-yloxy]-2,3-dihydro-benzofuran-3-yl}-acetic acid methyl ester

Intermediate 52ea (213 mg) and pyridine (74 µL) are suspended in dichloromethane (10 mL) and cooled to 0 °C. Trifluoromethane sulfonyl chloride (0.127 mL) is added, the mixture allowed to warm to room temperature and stirred for 30 minutes. The mixture is washed with saturated aqueous sodium bicarbonate solution, 1 M aqueous hydrochloric acid, dried over sodium sulfate and the solvent removed under vacuum to give the crude title compound which is used without further purification.Yield 291 mg.

### Step 2: {(S)-6-[(R)-4-(4-Cyano-2,6-dimethyl-phenyl)-5-trifluoromethyl-indan-1-yloxy]-2,3-dihydro-benzofuran-3-yl}-acetic acid methyl ester

{(S)-6-[(R)-4-(2,6-Dimethyl-4-trifluoromethanesulfonyloxy-phenyl)-5-trifluoromethyl-indan-1-yloxy]-2,3-dihydro-benzofuran-3-yl}-acetic acid methyl ester (291 mg), zinc cyanide (80 mg), tris(dibenzylideneacetone)dipalladium(0) (21 mg) and 1,1'-bis(diphenylphosphino)ferrocene (25 mg) are suspended in dry N,N-dimethylformamide (5 mL), degassed with a flow of argon and stirred for 1 hour at 70 °C. The mixture is diluted with ethylacetate, washed with water and brine, dried over sodium sulfate and the solvent removed under vacuum. The residue is purified by flash chromatography (cyclohexane/ethyl acetate 100:0→90:10) to give the title compound.Yield 66 mg.

### Step 3: ((S)-6-{(R)-4-[4-(N-Hydroxycarbamimidoyl)-2,6-dimethyl-phenyl]-5-trifluorome thylindan-1-yloxy}-2,3-dihydro-benzofuran-3-yl)-acetic acid methyl ester

{(S)-6-[(R)-4-(4-Cyano-2,6-dimethyl-phenyl)-5-trifluoromethyl-indan-1-yloxy]-2,3-dihydro-benzofuran-3-yl}-acetic acid methyl ester (61 mg) is suspended in absolute ethanol (7 mL) and hydroxylamine hydrochloride (12 mg) and triethylamine (24 µL,) are added. The mixture is refluxed for 2 hours then cooled, diluted with water and extracted with ethyl acetate. The extracts are dried over sodium sulfate and the solvent removed under vacuum to give the crude title compound which is used without further purification.Yield 64 mg.

### Step 4: ((S)-6-{(R)-4-[2,6-Dimethyl-4-(5-methyl-[1,2,4]oxadiazol-3-yl)-phenyl]-5-triflu oromethyl-indan-1-yloxy}-2,3-dihydro-benzofuran-3-yl)-acetic acid methyl ester

((S)-6-{(R)-4-[4-(N-Hydroxycarbamimidoyl)-2,6-dimethyl-phenyl]-5-trifluoromethyl-indan-1-yloxy}-2,3-dihydro-benzofuran-3-yl)-acetic acid methyl ester (64 mg) is suspended in absolute ethanol (7 mL) and acetic anhydride (20 mg) and triethylamine (46 µL) are added. The mixture is refluxed for 3 hours then cooled, and the solvent removed under vacuum. The residue is partitioned between dichloromethane and water, the organic phase is collected, dried over sodium sulfate and the solvent removed under vacuum. The residue is purified by flash chromatography (cyclohexane/ethyl acetate 100:0→80:20) to give the title compound. Yield 41 mg.

### Intermediate 52fa

### 1-[4-(4-Bromo-phenyl)-pyrazol-1-yl]-2-methyl-propan-2-ol

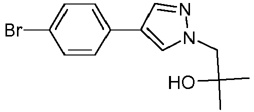

1-Bromo-4-iodo-bromobenzene (780 mg) and 2-methyl-1-[4-(4,4,5,5-tetramethyl-[1,3,2]dioxaborolan-2-yl)-pyrazol-1-yl]-propan-2-ol (US2011/76291 A1, 2011) (832 mg) are stirred in 6 mL of 1,4-dioxane and 2 mL of water, 1,1'-bis(diphenylphosphino)ferrocenedichloropalladium(II) (100 mg) and cesium carbonate (1.8 g) are added and the reaction mixture is stirred under nitrogen atmosphere at 100 °C overnight. Water is added and the reaction mixture is extracted with ethyl acetate. The organic phase is collected, dried over sodium sulfate and the solvent removed under vacuum. The residue is purified by flash chromatography (cyclohexane/ethyl acetate 100:0→50:50) to give the title compound. Yield: 670 mg.

### Intermediate 52fb

### 3-(4-Bromo-phenyl)-5-trifluoromethyl-1H-[1,2,4]triazole

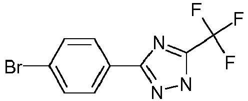

4-Bromobenzimidoethylether hydrochloride (WO2010/62571 A1, 2010) (3 g), trifluoroacetic acid hydrazide (2.53 g) and triethylamine (2.55 g) are stirred in 1,4-dioxane at room temperature overnight. Solvent is concentrated under vacuum. The residue is purified by flash chromatography (cyclohexane/ethyl acetate 100:0→50:50) to give the title compound. Yield: 2.8 g.

### Intermediate 52fc

### 3-4-Bromo-phenyl)-5-cyclopropyl-[1,2,4]oxadiazole

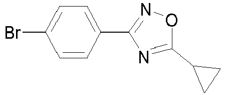

The title compound is prepared in analogy to 3-(4-bromo-phenyl)-5-methyl-[1,2,4]-oxadiazole (WO2006/40192 A1, 2006) starting from cyclopropane carbonyl chloride.

### Intermediate 52fca

### 3-4-Bromo-phenyl)-5-ethyl-[1,2,4]oxadiazole

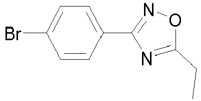

The title compound is prepared in analogy to 3-(4-bromo-phenyl)-5-methyl-[1,2,4]-oxadiazole (WO2006/40192 A1, 2006) starting from propionyl chloride.

### Intermediate 52fcb

### 3-(4-Bromo-phenyl)-5-isopropyl-[1,2,4]oxadiazole

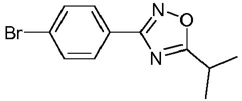

The title compound is prepared in analogy to 3-(4-bromo-phenyl)-5-methyl-[1,2,4]-oxadiazole (WO2006/40192 A1, 2006) starting from isobutyryl chloride.

### Intermediate 52fda

### 5-(4-Bromo-phenyl)-1-methyl-3-trifluoromethyl-1H-[1,2,4]triazole

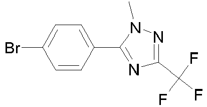

Intermediate 52fb (2.8 g) and potassium hydroxide (132 mg) are stirred in 30 mL of acetone, iodomethane (0.211 mL) is added and the reaction mixture is stirred at room temperature overnight. Solvent is concentrated under vacuum. The residue is purified by flash chromatography (cyclohexane/ethyl acetate 100:0→50:50) to give the title compound. Yield: 220 mg.

### Intermediate 52fdb

### 3-(4-Bromo-phenyl)-1-methyl-5-trifluoromethyl-1H-[1,2,4]triazole

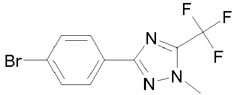

The title compound is obtained by further elution from the flash chromatography performed in the preparation of Intermediate 52fda.

### Intermediate 52fe

### 2-[3-(4-Bromo-phenyl)-[1,2,4]oxadiazol-5-yl]-propan-2-ol

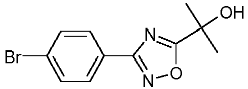

2-Hydroxyisobutyric acid (970 mg), N,N,N',N'-tetramethyl-O-(benzotriazol-1-yl)uronium tetrafluoroborate (1.79 g) and triethylamine (1.8 g) are stirred in 20 mL of acetonitrile at room temperature for 5 minutes, 4-bromo-N-hydroxybenzenecarboximidamide (WO2004/101546 A1, 2004) (1 g) is added and the reaction mixture is stirred at room temperature for 24 h. The mixture is concentrated under vacuum. The residue is purified by flash chromatography (cyclohexane/ethyl acetate 100:0→50:50) to give the title compound. Yield: 530 mg.

### Intermediate 52fea

### 1-[3-(4-Bromo-phenyl)-[1,2,4]oxadiazol-5-yl]-2-methyl-propan-2-ol

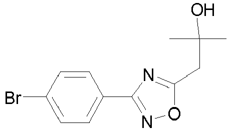

The title compound is prepared in analogy to Intermediate 52fe, starting from beta-hydroxy-isovaleric acid.

### Intermediate 52ff

### 4-(4-Bromo-phenyl)-1-methyl-1H-pyrazole

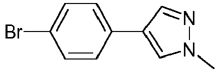

The title compound is prepared in analogy to Intermediate 52fa, starting from 1-methyl-4-(4,4,5,5-tetranethyl-1,3,2-dioxaborolan-2-yl)-1H-pyrazole.

### Intermediate 52fg

### 5-(4-Bromo-phenyl)-1,3-dimethyl-1H-pyrazole

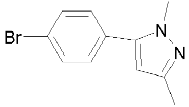

The title compound is prepared in analogy to Intermediate 52fa, starting from 1,3-dimethyl-5-(4,4,5,5-tetranethyl-1,3,2-dioxaborolan-2-yl)-1H-pyrazole

### Intermediate 52fha

### 1-(4-Bromo-phenyl)-3-methyl-1H-pyrazole

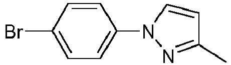

1-Bromo-4-iodo-bromobenzene (4 g), 3-methyl-pyrazole (2.85 g), N,N'-dimethylethylenediamine (0.38 mL), copper(I) iodide (673 mg) and cesium carbonate (3.45 g) are stirred in 20 mL of N,N-dimethylformamide at 100 °C for 24 h. Water is added and the reaction mixture is extracted with ethyl acetate. The organic phase is collected, dried over sodium sulfate and the solvent removed under vacuum. The residue is purified by flash chromatography (cyclohexane/ethyl acetate 100:0→80:20) to give the title compound. Yield: 650 mg.

### Intermediate 52fhb

### 1-(4-Bromo-phenyl)-5-methyl-1H-pyrazole

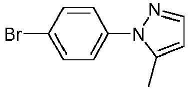

The title compounds is obtained by further elution from the column in the peparation of Intermediate 52fha.

### Intermediate 52fi

### 1-(4-Bromo-phenyl)-3-methyl-1H-[1,2,4]triazole

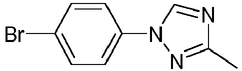

The title compound is prepared in analogy to Intermediate 52fha starting from 3-methyl-1H-1,2,4-triazole.

### Intermediate 52fj

### 4-(4-Bromo-phenyl)-pyridazine

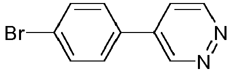

4-(Tributylstannyl)pyridazyne (1.1 g), tris(dibenzylideneacetone)dipalladium(0) (136 mg) tri-o-tolylphosphine (280 mg), triethylamine (1.24 g) and 1-bromo-4-iodo-benzene (927 mg) are stirred in 4 mL of N,N-dimethylformamide at 80 °C for 3 h. The solvent is removed and the residue is partitioned between ammonium chloride saturated water soluion and dichloromethane. The organic phase is collected, dried over sodium sulfate and the solvent removed under vacuum. The residue is purified by flash chromatography (cyclohexane/ethyl acetate 100:0→70:30) to give the title compound. Yield: 350 mg.

### Intermediate 52fk

### 5-(4-Bromo-phenyl)-1-methyl-1H-pyridin-2-one

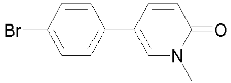

The title compound is prepared in analogy to Intermediate 52fa starting from 1-methyl-5-(4-(4,4,5,5-tetramethyl-1,3,2-dioxaborolan-2-yl)phenyl)pyridin-2(1H)-one (WO2010/47982 A1, 2010).

### Intermediate 52fl

### 2-[1-(4-Bromo-phenyl)-1H-[1,2,4]triazol-3-yl]-propan-2-ol

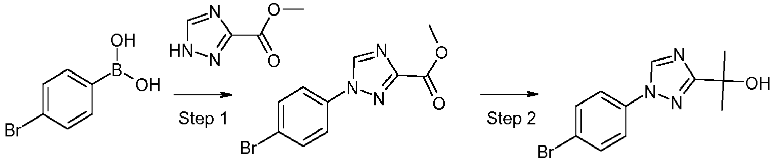

### Step 1: 1-(4-Bromo-phenyl)-1H-[1,2,4]triazole-3-carboxylic acid methyl ester

4-Bromophenylboronic acid (1.58 g), methyl-1,2,4-triazole-3-carboxylate (1 g), copper (I) acetate and pyridine (1.94 mL) are stirred in 20 mL of dichloromethane in oxygen atmosphere for 72 h. Ammonium hydroxide saturated aqueous solution is added and the reaction mixture is extracted with dichloromethane.The organic phase is collected, dried over sodium sulfate and the solvent removed under vacuum. The residue is purified by flash chromatography (cyclohexane/ethyl acetate 100:0→60:40) to give the title compound. Yield: 1.6 mg.

### Step 2: 2-[1-(4-Bromo-phenyl)-1H-[1,2,4]triazol-3-yl]-propan-2-ol

1-(4-Bromo-phenyl)-1H-[1,2,4]triazole-3-carboxylic acid methyl ester (400 mg) is dissolved in 10 mL of dry tetrahydrofurane, methylmagnesium bromide (1 mL of a 3 M solution in tetrahydrofurane) is added and the reaction mixture is stirred at room temperature for 3 h. Water is added and the reaction mixture is extracted with ethyl acetate.The organic phase is collected, dried over sodium sulfate and the solvent removed under vacuum. The residue is purified by flash chromatography (cyclohexane/ethyl acetate 100:0→50.50) to give the title compound. Yield: 390 mg.

### Intermediate 52fm

### 1-[4-(4-Bromo-phenyl)-3,5-dimethyl-pyrazol-1-yl]-2-methyl-propan-2-ol

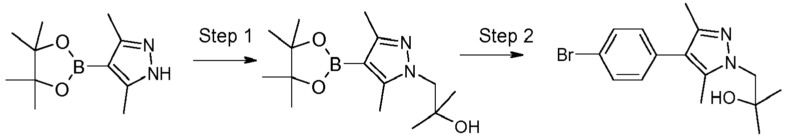

### Step 1: 1-[3,5-Dimethyl-4-(4,4,5,5-tetramethyl-[1,3,2]dioxaborolan-2-yl)-pyrazol-1-yl]-2-methyl-propan-2-ol

In a microwave vial, 3,5-dimethylpyrazole-4-boronic acid pinacol ester (500 mg) is dissolved in isobutylene oxide (1 mL), cesium carbonate (147 mg) is added and the reaction mixture is stirred at 120 °C for 30 min under microwave irradiation (100 W). Ethyl acetate is added and the reaction mixture filtered. The organic phase is concentrated under vacuum to give the title compound, used in the next step without further purification. Yield: 630 mg.

### Step 2: 1-[4-(4-Bromo-phenyl)-3,5-dimethyl-pyrazol-1-yl]-2-methyl-propan-2-ol

The title compound is prepared in analogy to Intermediate 52fa, starting from 1-[3,5-dimethyl-4-(4,4,5,5-tetramethyl-[1,3,2]dioxaborolan-2-yl)-pyrazol-1-yl]-2-methyl-propan-2-ol

### Intermediate 52fn

### 1-(4-Bromo-phenyl)-3-isopropyl-1H-pyrazole

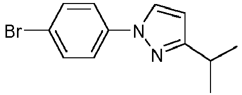

The title compound is prepared in analogy to intermediate 52fha starting from 3-isopropylpyrazole

### Intermediate 52fo

### 2-[5-(4-Bromo-phenyl)-2-methyl-2H-[1,2,4]triazol-3-yl]-propan-2-ol

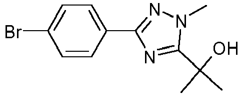

2-Hydroxyisobutyric acid (1.1 g), N,N,N',N'-tetramethyl-O-(benzotriazol-1-yl)uronium tetrafluoroborate (2.04g) and triethylamine (2 g) are stirred in 20 mL of acetonitrile at room temperature for 5 minutes, 4-bromo-N-(methylamino)benzenecarboximidamide (WO2009/105500 A1, 2009) (2 g) is added and the reaction mixture is stirred at room temperature for 24 h, then at 80 °C for 2 h. Solvent is concentrated under vacuum. The residue is purified by flash chromatography (cyclohexane/ethyl acetate 100:0→50:50) to give the title compound. Yield: 280 mg.

### Intermediate 52ga

### 2-Methyl-1-{4-[4-(4,4,5,5-tetramethyl-[1,3,2]dioxaborolan-2-yl)-phenl]-pyrazol-1-yl}-propan-2-ol

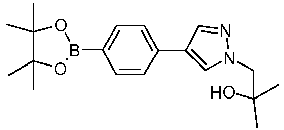

Intermediate 52fa (452 mg) is stirred in 25 mL of 1,4-dioxane, Bis(pinacolate)diboron (405 mg), 1,1'-bis(diphenylphosphino)ferrocenedichloropalladium(II) (100 mg) and potassium acetate (324 mg) are added and the reaction mixture is stirred under nitrogen atmosphere at 100 °C for 6 h. Solvent is removed. Water is added and the reaction mixture is extracted with ethyl acetate. The organic phase is collected, dried over sodium sulfate and the solvent removed under vacuum. The residue is purified by flash chromatography (cyclohexane/ethyl acetate 100:0→50:50) to give the title compound. Yield: 310 mg.

The intermediates in the following table are prepared from the corresponding starting bromo-aryl intermediates following a procedure analogous to that described for Intermediate 52ga.

| **Starting bromoaryl Intermediate** | **Intermediate** | **Molecular structure and name** |
|---|---|---|
| 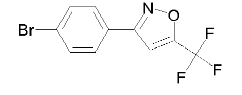 | **52gb** | 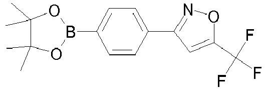 |
| Martins, Marcos A. P et al.Journal of Heterocyclic Chemistry, 1996 , vol. 33, # 6 p. 1619 - 1622 | | 3-[4-(4,4,5,5-Tetramethyl-[1,3,2]dioxaborolan-2-yl)-phenyl]-5-trifluoromethyl-isoxazole |
| 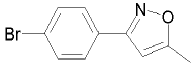 | **52gc** | 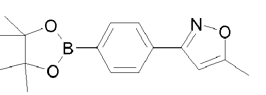 |
| Tang, Shibing et al. Organic Letters, 2009 , vol. 11, # 17 p. 3982-3985 | | 5-Methyl-3-[4-(4,4,5,5-tetramethyl-[1,3,2]dioxaborolan-2-yl)-phenyl]-isoxazole |
| 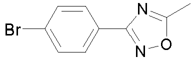 | **52gd** | 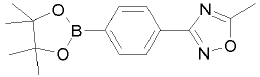 |
| WO2006/40192 A1, 2006 ; | | 5-Methyl-3-[4-(4,4,5,5-tetramethyl-[1,3,2]dioxaborolan-2-yl)-phenyl]-[ 1,2,4]oxadiazole |
| 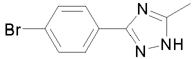 | **52ge** | 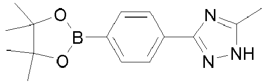 |
| Ueda, Satoshi et al. Journal of the American Chemical Society, 2009 , vol. 131, # 42 p. 15080 - 15081 | | 5-Methyl-3-[4-(4,4,5,5-tetramethyl-[1,3,2]dioxaborolan-2-yl)-phenyl]-1 H-[1,2,4]triazole |
| 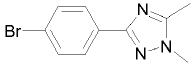 | **52gf** | 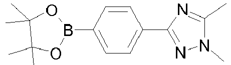 |
| WO2009/105500 A1, 2009 ; | | 1,5-Dimethyl-3-[4-(4,4,5,5-tetramethyl-[1,3,2]dioxaborolan-2-yl)-phenyl]-1H-[1,2,4]triazole |
| 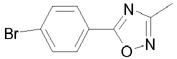 | **52gg** | 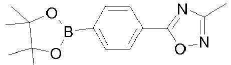 |
| WO2004/101546 A1, 2004 | | 3-Methyl-5-[4-(4,4,5,5-tetramethyl-[1,3,2]dioxaborolan-2-yl)-phenyl]-[1,2,4]oxadiazole |
| 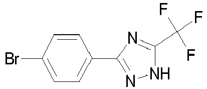 | **52gh** | 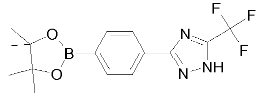 |
| 52fb | | 3-[4-(4,4,5,5-Tetramethyl-[1,3,2]dioxaborolan-2-yl)-phenyl]-5-trifluoromethyl-1H-[1,2,4]triazole |
| 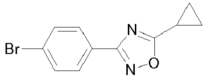 | **52gi** | 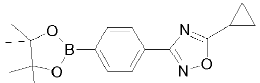 |
| 52fc | | 5-Cyclopropyl-3-[4-(4,4,5,5-tetramethyl-[1,3,2]dioxaborolan-2-yl)-phenyl]-[1,2,4]oxadiazole |
| 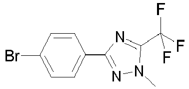 | **52gj** | 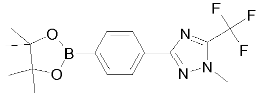 |
| 52fd b | | 1-Methyl-3-[4-(4,4,5,5-tetramethyl-[1,3,2]dioxaborolan-2-yl)-phenyl]-5 -trifluoromethyl-1H-[1,2,4]triazole |
| 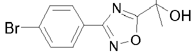 | **52gk** | 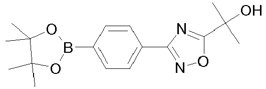 |
| 52fe | | 2-{3-[4-(4,4,5,5-Tetramethyl-[1,3,2]dioxaborolan-2-yl)-phenyl]-[1,2,4] oxadiazol-5-yl}-propan-2-ol |
| 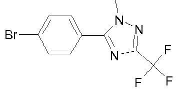 | **52gl** | 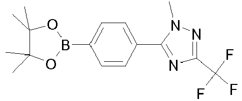 |
| 52fda | | 1-Methyl-5-[4-(4,4,5,5-tetramethyl-[1,3,2]dioxaborolan-2-yl)-phenyl]-3 -trifluoromethyl-1H-[1,2,4]triazole |
| 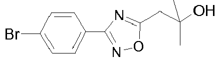 | **52gka** | 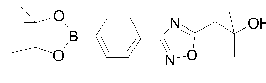 |
| 52fea | | 2-Methyl-1-{3-[4-(4,4,5,5-tetramethyl-[1,3,2]dioxaborolan-2-yl)-phenyl]-[1,2,4]oxadiazol-5-yl}-propan-2-ol |
| 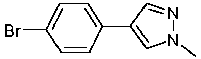 | **52gm** | 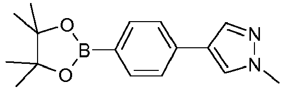 |
| 52ff | | 1-Methyl-4-[4-(4,4,5,5-tetramethyl-[1,3,2]dioxaborolan-2-yl)-phenyl]-1H-pyrazole |
| 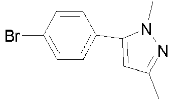 | **52gn** | 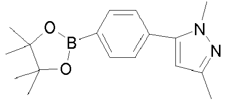 |
| 52fg | | 1,3-Dimethyl-5-[4-(4,4,5,5-tetramethyl-[1,3,2]dioxaborolan-2-yl)-phenyl]-1H-pyrazole |
| 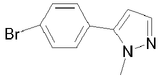 | **52go** | 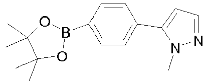 |
| WO2009/69044 A1, 2009 | | 1-Methyl-5-[4-(4,4,5,5-tetramethyl-[1,3,2]dioxaborolan-2-yl)-phenyl]-1H-pyrazole |
| 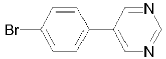 | **52gp** | 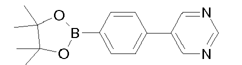 |
| EP2166015 A1, 2010 | | 5-[4-(4,4,5,5-Tetramethyl-[1,3,2]dioxaborolan-2-yl)-phenyl]-pyrimidine |
| 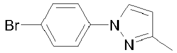 | **52gra** | 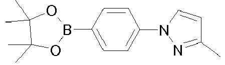 |
| 52fha | | 3-Methyl-1-[4-(4,4,5,5-tetramethyl-[1,3,2]dioxaborolan-2-yl)-phenyl]-1H-pyrazole |
| 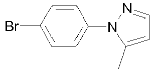 | **52grb** | 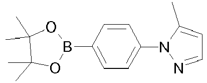 |
| 52fhb | | 5-Methyl-1-[4-(4,4,5,5-tetramethyl-[1,3,2]dioxaborolan-2-yl)-phenyl]-1H-pyrazole |
| 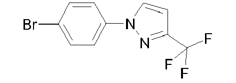 | **52gs** | 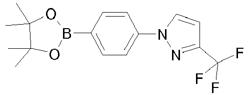 |
| Suh, Jeehee et al. Bulletin of the Korean Chemical Society, 2012 , vol. 33, # 6 p. 2067 - 2070 | | 1-[4-(4,4,5,5-Tetramethyl-[1,3,2]dioxaborolan-2-yl)-phenyl]-3-trifluoromethyl-1H-pyrazole |
| 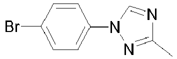 | **52gt** | 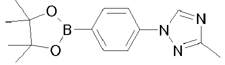 |
| 52fi | | 3-Methyl-1-[4-(4,4,5,5-tetramethyl-[1,3,2]dioxaborolan-2-yl)-phenyl]-1H-[1,2,4]triazole |
| 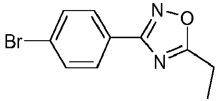 | **52gu** | 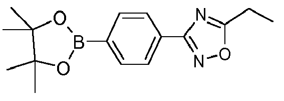 |
| 52fca | | 5-Ethyl-3-[4-(4,4,5,5-tetramethyl-[1,3,2]dioxaborolan-2-yl)-phenyl]-[1,2,4]oxadiazole |
| 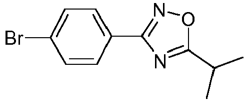 | **52gw** | 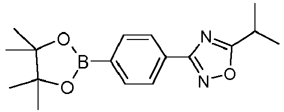 |
| 52fcb | | 5-Isopropyl-3-[4-(4,4,5,5-tetramethyl-[1,3,2]dioxaborolan-2-yl)-phenyl]-[1,2,4]oxadiazole |
| 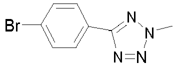 | **52gx** | 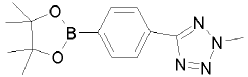 |
| WO2008/108602 A1, 2008 | | 2-Methyl-5-[4-(4,4,5,5-tetramethyl-[1,3,2]dioxaborolan-2-yl)-phenyl]-2 H-tetrazole |
| 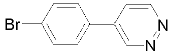 | **52gy** | 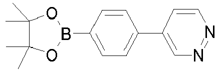 |
| 52fj | | 4-[4-(4,4,5,5-Tetramethyl-[1,3,2]dioxaborolan-2-yl)-phenyl]-pyridazine |
| 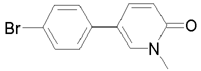 | **52gz** | 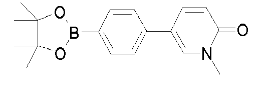 |
| 52fk | | |
| 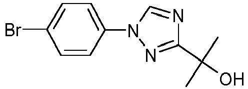 | **52gza** | 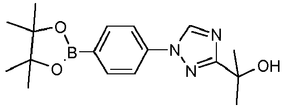 |
| 52fl | | 2-{1-[4-(4,4,5,5-Tetramethyl-[1,3,2]dioxaborolan-2-yl)-phenyl]-1H-[1,2,4]triazol-3-yl}-propan-2-ol |
| 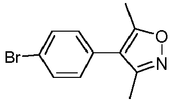 | **52gzb** | 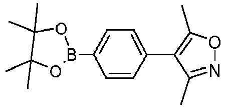 |
| WO2004/74270 A2, 2004 | | 3,5-Dimethyl-4-[4-(4,4,5,5-tetramethyl-[1,3,2]dioxaborolan-2-yl)-phenyl]-isoxazole |
| 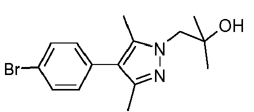 | **52gzc** | 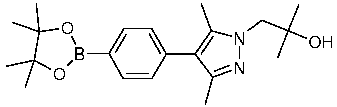 |
| 52fm | | 1-{3,5-Dimethyl-4-[4-(4,4,5,5-tetramethyl-[1,3,2]dioxaborolan-2-yl)-phenyl]-pyrazol-1-yl}-2-methyl-propan-2-ol |
| 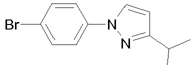 | **52gzd** | 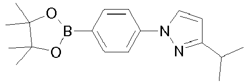 |
| 52fn | | 3-Isopropyl-1-[4-(4,4,5,5-tetramethyl-[1,3,2]dioxaborolan-2-yl)-phenyl]-1 H-pyrazole |
| 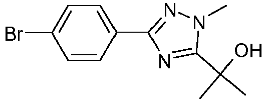 | **52gze** | 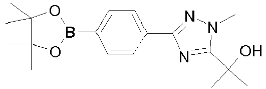 |
| 52fo | | 2-{2-Methyl-5-[4-(4,4,5,5-tetramethyl-[1,3,2]dioxaborolan-2-yl)-phenyl]-2H-[1,2,4]triazol-3-yl}-propan-2-ol |
| 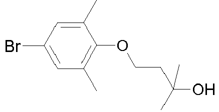 | **52gzf** | 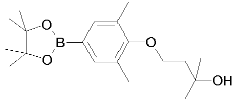 |
| 50w | | 4-[2,6-Dimethyl-4-(4,4,5,5-tetramethyl-[1,3,2]dioxaborolan-2-yl)-phenoxy]-2-methyl-butan-2-ol |

### Intermediate 52gzda

### 4-(3-Isopropyl-pyrazol-1-yl)-boronic acid

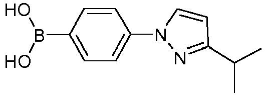

Intermediate 52gzd (3.6 g), sodium metaperiodate (24.7 g) and ammonium acetate (8.9 g) are stirred in 40 mL of acetone and 40 mL of water at room temperature overnight. The reaction mixture is filtered and the solvent concentrated. The residue is cooled to 0 °C and a 1 M sodium hydroxide water solution is added up to basic pH. The organic phase is extracted with dichloromethane, separated, dried over sodium sulfate and concetrated under vacuum to give the title compound. Yield: 390 mg.

### Intermediate 52gzg

### 4-(Tetrahydro-pyran-4-ylcarbamoyl)-benzeneboronic acid

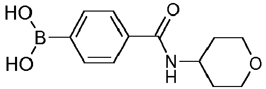

To a mixture of 4-carboxyphenylboronic acid (500 mg) and 4-aminoetrahydrofurane hydrochloride (413 mg) in 20 mL of tetrahydrofurane, N,N'-diisopropylethylamine (1,55 g) and *N,N,N,'N'*-tetramethyl-*O*-(benzotriazol-1-yl)uronium tetrafluoroborate (953 mg) are added and the reaction mixture is stirred at room temperature overnight, The solvent is removed and the residue is partitioned between a 1 M hydrochloric acid acqueous solution and ethyl acetate. The solid precipatated is filtered and dried under vacuum to give the title compound. Yield: 537 mg.

The intermediates in the following table are prepared from the corresponding starting bromo-aryl intermediates following a procedure analogous to that described for Intermediate 52ga.

| **Starting bromo-aryl-intermediate** | **Intermediate** | **Molecular structure and name** |
|---|---|---|
| 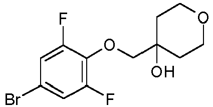 | **52gzua** | 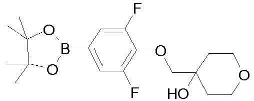 |
| 50ua | | 4-[2,6-Difluoro-4-(4,4,5,5-tetramethyl-[1,3,2]dioxaborolan-2-yl)-phenoxymethyl]-tetrahydro-pyran-4-ol |
| 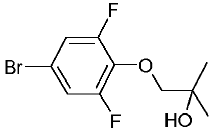 | **52gzub** | 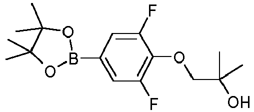 |
| 50ub | | 1-[2,6-Difluoro-4-(4,4,5,5-tetramethyl-[1,3,2]dioxaborolan-2-yl)-phenoxy]-2-methyl-propan-2-ol |
| 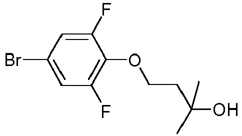 | **52gzuc** | 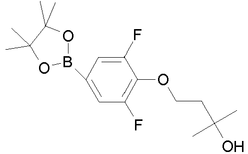 |
| 50uc | | 4-[2,6-Difluoro-4-(4,4,5,5-tetramethyl-[1,3,2]dioxaborolan-2-yl)-phenoxy]-2-methyl-butan-2-ol |
| 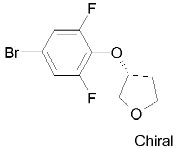 | **52gzud** | 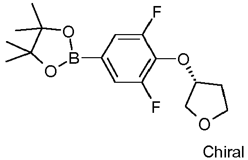 |
| 50ud | | 2-{3,5-Difluoro-4-[(R)-(tetrahydro-furan-3-yl)oxy]-phenyl}-4,4,5,5-tetramethyl-[1,3,2]dioxaborolane |
| 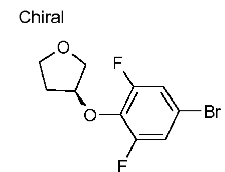 | **52gzue** | 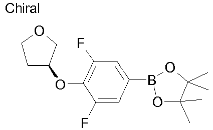 |
| 50ue | | 2-{3,5-Difluoro-4-[(S)-(tetrahydro-furan-3-yl)oxy]-phenyl}-4,4,5,5-tetramethyl-[1,3,2]dioxaborolane |
| 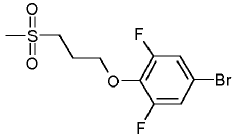 | **52gzuf** | 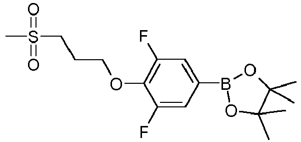 |
| 52uf | | 2-[3,5-Difluoro-4-(3-methanesulfonyl-propoxy)-phenyl]-4,4,5,5-tetramethyl-[1,3,2]dioxaborolane |
| 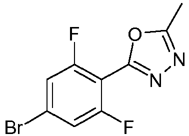 | **52gzug** | 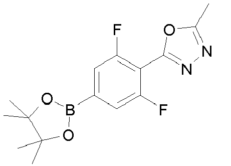 |
| 50ug | | 2-[2,6-Difluoro-4-(4,4,5,5-tetramethyl-[1,3,2]dioxaborolan-2-yl)-phenyl]-5-methyl-[1,3,4]oxadiazole |
| 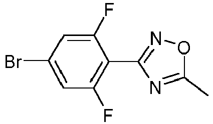 | **52gzuh** | 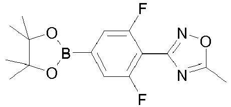 |
| 50uh | | 3-[2,6-Difluoro-4-(4,4,5,5-tetramethyl-[1,3,2]dioxaborolan-2-yl)-phenyl]-5-methyl-[1,2,4]oxadiazole |

### Intermediate 52gzui

### 1-{4-[2,6-Difluoro-4-(4,4,5,5-tetramethyl-[1,3,2]dioxaborolan-2-yl)-phenyl]-pyrazol-1-yl}-2-methyl-propan-2-ol

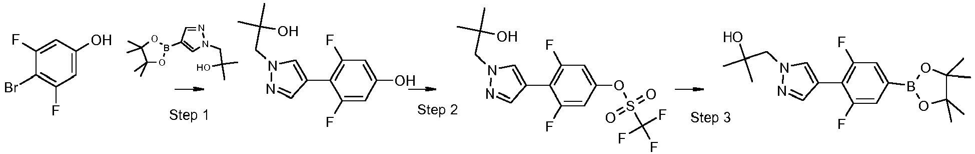

### Step1: 3,5-Difluoro-4-[1-(2-hydroxy-2-methyl-propyl)-1H-pyrazol-4-yl]-phenol

The title compound is prepared following what described in Step 1 in the preparation of Intermediate 52aa, starting from 1-[3,5-dimethyl-4-(4,4,5,5-tetramethyl-[1,3,2]dioxaborolan-2-yl)-pyrazol-1-yl]-2-methyl-propan-2-ol and 4-bromo-3,5-difluorophenol.

### Step2: Trifluoro-methanesulfonic acid 3,5-difluoro-4-[1-(2-hydroxy-2-methyl-propyl)-1 H-pyrazol-4-yl]-phenyl ester

3,5-Difluoro-4-[1-(2-hydroxy-2-methyl-propyl)-1 H-pyrazol-4-yl]-phenol (900 mg) is stirred at 0 °C in 10 mL of dichloromethane. Trifluoromethanesulfonic anhydride (0.41 mL) is added dropwise and the reaxtion micture is stirred for 30 minutes. The reaction mixture is partitioned between a bicarbonate water saturated solution and dichloromethane, the organic phase is separated, dried over sodium sulfate and concentrated under vacuum to give the tile compound. Yield: 900 mg.

### Step 3: 1-{4-[2,6-Difluoro-4-(4,4,5,5-tetramethyl-[1,3,2]dioxaborolan-2-yl)-phenyl]-pyrazol-1-yl}-2-methyl-propan-2-ol

The title compound is prepared in analogy to Intermediate 52ga, starting from trifluoro-methanesulfonic acid 3,5-difluoro-4-[1-(2-hydroxy-2-methyl-propyl)-1H-pyrazol-4-yl]-phenyl ester

### Intermediate 52ha

### [(S)-6-((R)-4-{4-[1-(2-Hydroxy-2-methyl-propyl)-1H-pyrazol-4-yl]-phenyl}-5-trifluoromethyl-indan-1-yloxy)-2,3-dihydro-benzofuran-3-yl]-acetic acid methyl ester

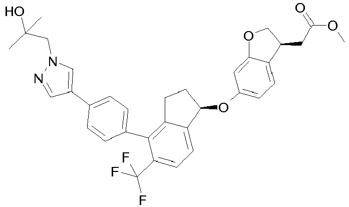

Intermediate 52a (89 mg), Intermediate 52ga (86 mg), potassium carbonate (78 mg) 2,6-di-tert-butyl-4-methylphenol (21 mg) and tricyclohexyl-phosphine (4 mg) are stirred in 4 mL of 1,4-dioxane and 1 mL of water, tris(dibenzylideneacetone) dipalladium(0) (7 mg) is added and the reaction mixture is stirred at 110 °C for 1 h. Water is added and the reaction mixture is extracted with ethyl acetate. The organic phase is separated, dried over sodium sulfate and concentrated under vacuum. The residue is purified by chromatography on silica gel (cyclohexane/ethyl acetate 100:0→50:50) to give the title compound. Yield: 34 mg.

The intermediates in the following table are prepared from Intermediate 52a and the corresponding starting boronic ester intermediates following a analogousprocedure analogous to that described for Intermediate 52ha.

| **Starting boronic ester/acid Intermediate** | **Intermediate** | **Molecular structure and name** |
|---|---|---|
| 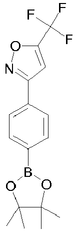 | **52hb** | 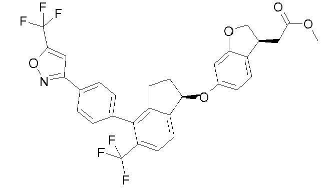 |
| 52gb | | ((S)-6-{(R)-5-Trifluoromethyl-4-[4-(5-trifluoromethyl-isoxazol-3-yl)-phenyl]-indan-1-yloxy}-2,3-dihydro-benzofuran-3-yl)-acetic acid methyl ester |
| 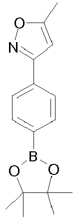 | **52hc** | 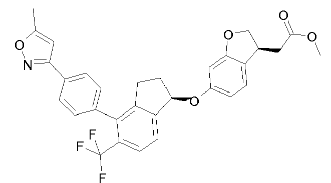 |
| 52gc | | ((S)-6-{(R)-4-[4-(5-Methyl-isoxazol-3-yl)-phenyl]-5-trifluoromethyl-indan-1-yloxy}-2,3-dihydro-benzofuran-3-yl)-acetic acid methyl ester |
| 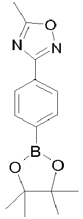 | **52hd** | 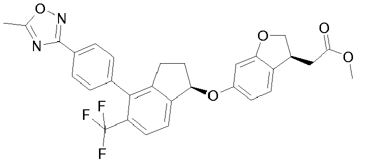 |
| 52gd | | ((S)-6-{(R)-4-[4-(5-Methyl-[1,2,4]oxadiazol-3-yl)-phenyl]-5-trifluoromethyl-indan-1-yloxy}-2,3-dihydro-benzofuran-3-yl)-acetic acid methyl ester |
| 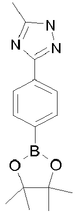 | **52he** | 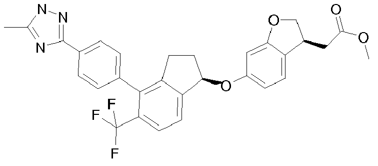 |
| 52ge | | ((S)-6-{(R)-4-[4-(5-Methyl-1H-[1,2,4]triazol-3-yl)-phenyl]-5-trifluoromethyl-indan-1-yloxy}-2,3-dihydro-benzofuran-3-yl)-acetic acid methyl ester |
| 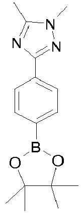 | **52hf** | 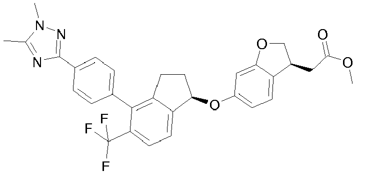 |
| 52gf | | ((S)-6-{(R)-4-[4-(1,5-Dimethyl-1H-[1,2,4]triazol-3-yl)-phenyl]-5-trifluoromethyl-indan-1-yloxy}-2,3-dihydro-benzofuran-3-yl)-acetic acid methyl ester |
| 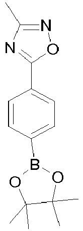 | **52hg** | 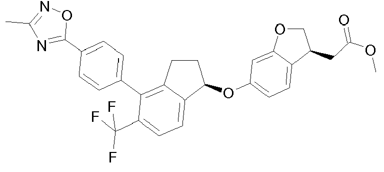 |
| 52gg | | ((S)-6-{(R)-4-[4-(3-Methyl-[1,2,4]oxadiazol-5-yl)-phenyl]-5-trifluoromethyl-indan-1-yloxy}-2,3-dihydro-benzofuran-3-yl)-acetic acid methyl ester |
| 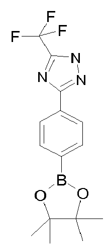 | **52hh** | 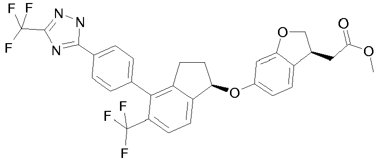 |
| 52gh | | ((S)-6-{(R)-5-Trifluoromethyl-4-[4-(5-trifluoromethyl-2H-[1,2,4]triazol-3-yl)-phenyl]-indan-1-yloxy}-2,3-dihydro-benzofuran-3-yl)-acetic acid methyl ester |
| 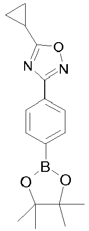 | **52hi** | 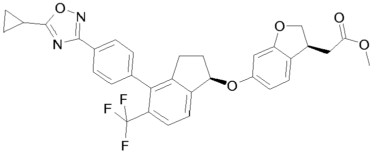 |
| 52gi | | ((S)-6-{(R)-4-[4-(5-Cyclopropyl-[1,2,4]oxadiazol-3-yl)-phenyl]-5-trifluoromethyl-indan-1-yloxy}-2,3-dihydro-benzofuran-3-yl)-acetic acid methyl ester |
| 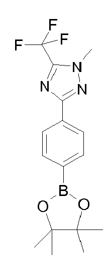 | **52hj** | 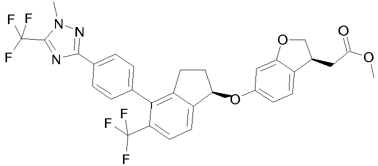 |
| 52gj | | ((S)-6-{(R)-4-[4-(1-Methyl-5-trifluoromethyl-1H-[1,2,4]triazol-3-yl)-phenyl]-5-trifluoromethyl-indan-1-yloxy}-2,3-dihydro-benzofuran-3-yl)-acetic acid methyl ester |
| 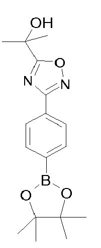 | **52hk** | 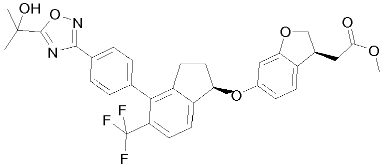 |
| 52gk | | [(S)-6-((R)-4-{4-[5-(1-Hydroxy-1-methyl-ethyl)-[1,2,4]oxadiazol-3-yl]-phenyl}-5-trifluoromethyl-indan-1-yloxy)-2,3-dihydro-benzofuran-3-yl]-acetic acid methyl ester |
| 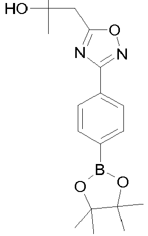 | **52hka** | 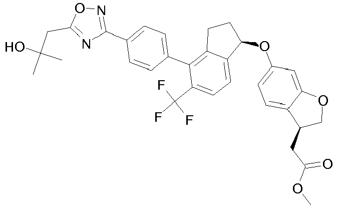 |
| 52gka | | [(S)-6-((R)-4-{4-[5-(2-Hydroxy-2-methyl-propyl)-[1,2,4]oxadiazol-3-yl]-phenyl}-5-trifluoromethyl-indan-1-yloxy)-2,3-dihydro-benzofuran-3-yl]-acetic acid methyl ester |
| 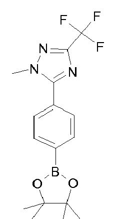 | **52hl** | 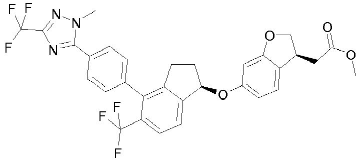 |
| 52gl | | ((S)-6-{(R)-4-[4-(2-Methyl-5-trifluoromethyl-2H-[1,2,4]triazol-3-yl)-phenyl]-5-trifluoromethyl-indan-1-yloxy}-2,3-dihydro-benzofuran-3-yl)-acetic acid methyl ester |
| 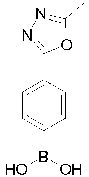 | **52hla** | 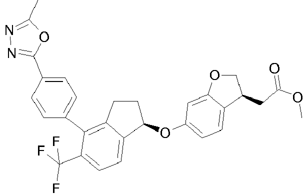 |
| 4-(5-methyl-1,3,4-oxadiazol-2-yl)-benzene boronic acid | | ((S)-6-{(R)-4-[4-(5-Methyl-[1,3,4]oxadiazol-2-yl)-phenyl]-5-trifluoromethyl-indan-1-yloxy}-2,3-dihydro-benzofuran-3-yl)-acetic acid methyl |
| Commercially available | | ester |
| 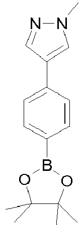 | **52hm** | 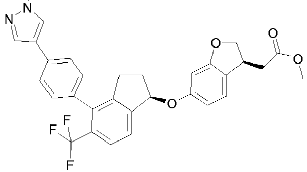 |
| 52gm | | ((S)-6-{(R)-4-[4-(1-Methyl-1H-pyrazol-4-yl)-phenyl]-5-trifluoromethyl-indan-1-yloxy}-2,3-dihydro-benzofuran-3-yl)-acetic acid methyl ester |
| 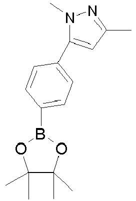 | **52hn** | 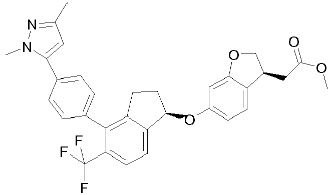 |
| 52gn | | ((S)-6-{(R)-4-[4-(2,5-Dimethyl-2H-pyrazol-3-yl)-phenyl]-5-trifluoromethyl-indan-1-yloxy}-2,3-dihydro-benzofuran-3-yl)-acetic acid methyl ester |
| 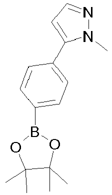 | **52ho** | 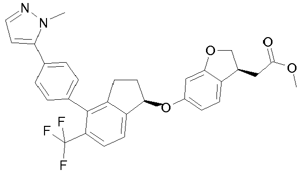 |
| 52go | | ((S)-6-{(R)-4-[4-(2-Methyl-2H-pyrazol-3-yl)-phenyl]-5-trifluoromethyl-indan-1-yloxy}-2,3-dihydro-benzofuran-3-yl)-acetic acid methyl ester |
| 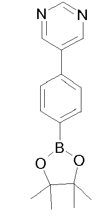 | **52hp** | 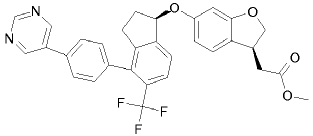 |
| 52gp | | {(S)-6-[(R)-4-(4-Pyrimidin-5-yl-phenyl)-5-trifluoromethyl-indan-1-yloxy]-2,3-dihydro-benzofuran-3-yl}-acetic acid methyl ester |
| 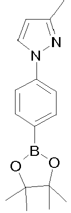 | **52hra** | 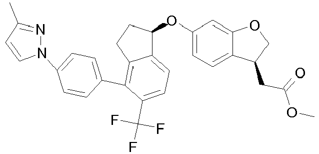 |
| 52gra | | ((S)-6-{(R)-4-[4-(3-Methyl-pyrazol-1-yl)-phenyl]-5-trifluoromethyl-indan-1-yloxy}-2,3-dihydro-benzofuran-3-yl)-acetic acid methyl ester |
| 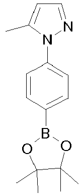 | **52hrb** | 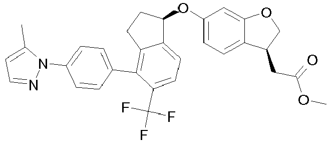 |
| 52grb | | ((S)-6-{(R)-4-[4-(5-Methyl-pyrazol-1-yl)-phenyl]-5-trifluoromethyl-indan-1-yloxy}-2,3-dihydro-benzofuran-3-yl)-acetic acid methyl ester |
| 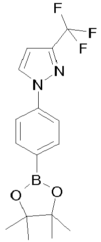 | **52hs** | 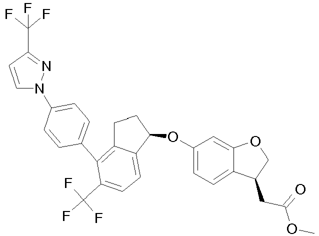 |
| 52gs | | ((S)-6-{(R)-5-Trifluoromethyl-4-[4-(3-trifluoromethyl-pyrazol-1-yl)-phenyl]-indan-1-yloxy}-2,3-dihydro-benzofuran-3-yl)-acetic acid methyl ester |
| 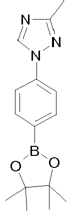 | **52ht** | 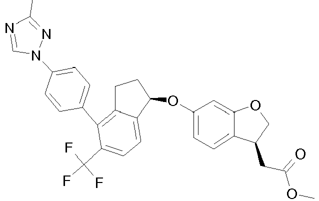 |
| 52gt | | ((S)-6-{(R)-4-[4-(3-Methyl-[1,2,4]triazol-1-yl)-phenyl]-5-trifluoromethyl-indan-1-yloxy}-2,3-dihydro-benzofuran-3-yl)-acetic acid methyl ester |
| 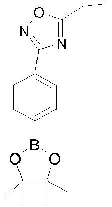 | **52hu** | 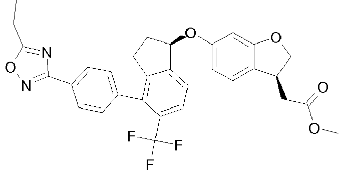 |
| 52gu | | ((S)-6-{(R)-4-[4-(5-Ethyl-[1,2,4]oxadiazol-3-yl)-phenyl]-5-trifluoromethyl-indan-1-yloxy}-2,3-dihydro-benzofuran-3-yl)-acetic acid methyl ester |
| 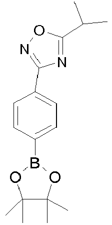 | **52hw** | 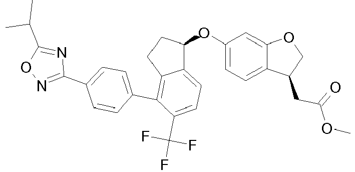 |
| 52gw | | ((S)-6-{(R)-4-[4-(5-Isopropyl-[1,2,4]oxadiazol-3-yl)-phenyl]-5-trifluoromethyl-indan-1-yloxy}-2,3-dihydro-benzofuran-3-yl)-acetic acid methyl ester |
| 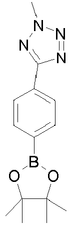 | **52hx** | 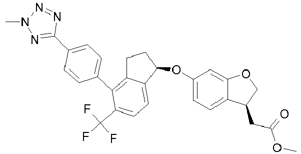 |
| 52gx | | ((S)-6-{(R)-4-[4-(2-Methyl-2H-tetrazol-5-yl)-phenyl]-5-trifluoromethyl-indan-1-yloxy}-2,3-dihydro-benzofuran-3-yl)-acetic acid methyl ester |
| 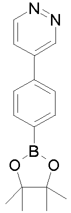 | **52hy** | 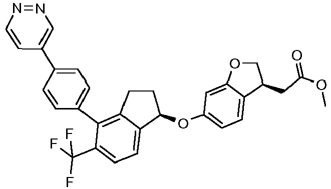 |
| 52gy | | {(S)-6-[(R)-4-(4-Pyridazin-4-yl-phenyl)-5-trifluoromethyl-indan-1-yloxy]-2,3-dihydro-benzofuran-3-yl}-acetic acid methyl ester |
| 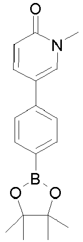 | **52hz** | 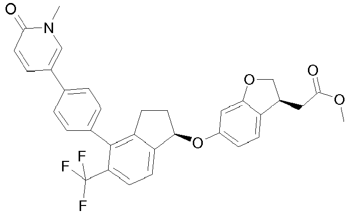 |
| 52gz | | ((S)-6-{(R)-4-[4-(1-Methyl-6-oxo-1,6-dihydro-pyridin-3-yl)-phenyl]-5-trifluoromethyl-indan-1-yloxy}-2,3-dihydro-benzofuran-3-yl)-acetic acid methyl ester |
| 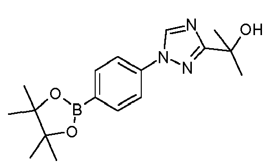 | **52hza** | 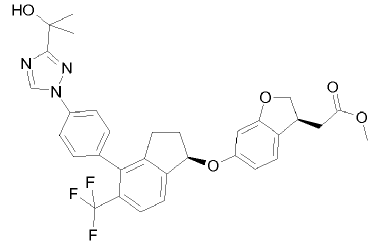 |
| 52gza | | [(S)-6-((R)-4-{4-[3-(1-Hydroxy-1-methyl-ethyl)-[1,2,4]triazol-1-yl]-phenyl}-5-trifluoromethyl-indan-1-yloxy)-2,3-dihydro-benzofuran-3-yl]-acetic acid methyl ester |
| 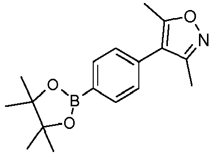 | **52hzb** | 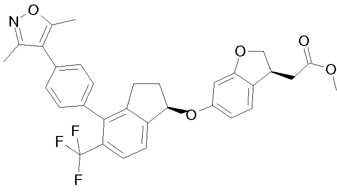 |
| 52gzb | | ((S)-6-{(R)-4-[4-(3,5-Dimethyl-isoxazol-4-yl)-phenyl]-5-trifluoromethyl-indan-1-yloxy}-2,3-dihydro-benzofuran-3-yl)-acetic acid methyl ester |
| 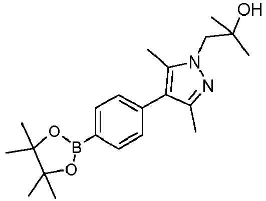 | **52hzc** | 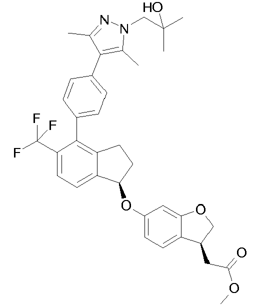 |
| 52gzc | | [(S)-6-((R)-4-{4-[1-(2-Hydroxy-2-methyl-propyl)-3,5-dimethyl-1H-pyrazol-4-yl]-phenyl}-5-trifluoromethyl-indan-1-yloxy)-2,3-dihydro-benzofuran-3-yl]-acetic acid methyl ester |
| 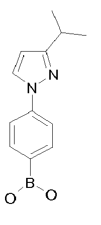 | **52hzd** | 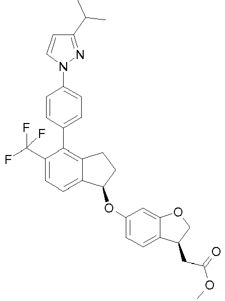 |
| 52gzda | | ((S)-6-{(R)-4-[4-(3-Isopropyl-pyrazol-1-yl)-phenyl]-5-trifluoromethyl-indan-1-yloxy}-2,3-dihydro-benzofuran-3-yl)-acetic acid methyl ester |
| 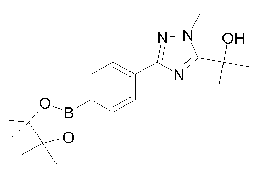 | **52hze** | 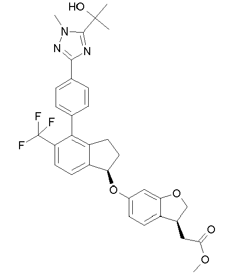 |
| 52gze | | [(S)-6-((R)-4-{4-[5-(1-Hydroxy-1-me thyl-ethyl)-1-methyl-1H-[1,2,4]triazol-3-yl]-phenyl}-5-trifluoromethyl -indan-1-yloxy)-2,3-dihydro-benzofuran-3-yl]-acetic acid methyl ester |
| 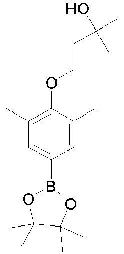 | **52hzf** | 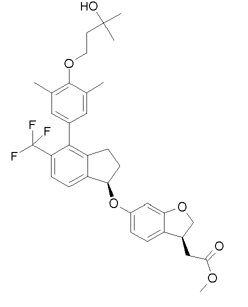 |
| 52gzf | | ((S)-6-{(R)-4-[4-(3-Hydroxy-3-methy l-butoxy)-3,5-dimethyl-phenyl]-5-trifluoromethyl-indan-1-yloxy}-2,3-dihydro-benzofuran-3-yl)-acetic acid methyl ester |
| 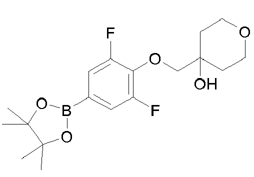 | **52hzg** | 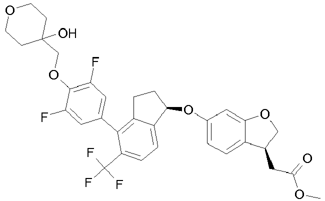 |
| 52gzua | | ((S)-6-{(R)-4-[3,5-Difluoro-4-(4-hy droxy-tetrahydro-pyran-4-ylmethoxy) -phenyl]-5-trifluoromethyl-indan-1-yloxy}-2,3-dihydro-benzofuran-3-yl) -acetic acid methyl ester |
| 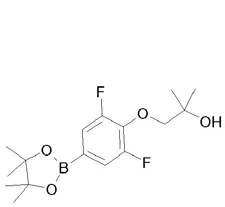 | **52hzh** | 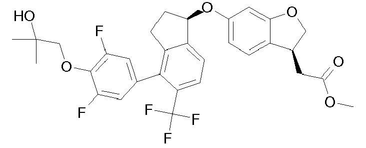 |
| 52gzub | | ((S)-6-{(R)-4-[3,5-Difluoro-4-(2-hy droxy-2-methyl-propoxy)-phenyl]-5-t rifluoromethyl-indan-1-yloxy}-2,3-d ihydro-benzofuran-3-yl)-acetic acid methyl ester |
| 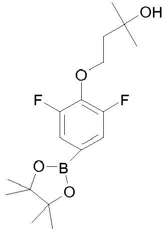 | **52hzi** | 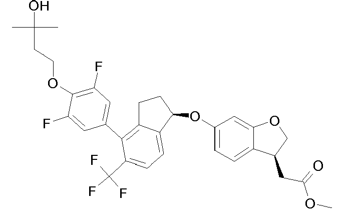 |
| 52gzuc | | ((S)-6-{(R)-4-[3,5-Difluoro-4-(3-hy d roxy-3-methyl-butoxy)-phenyl]-5-tr ifluoromethyl-indan-1-yloxy}-2,3-di hydro-benzofuran-3-yl)-acetic acid methyl ester |
| 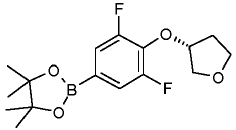 | **52hzj** | 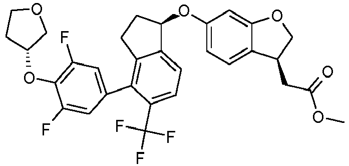 |
| 52gzud | | [(S)-6-((R)-4-{3,5-Difluoro-4-[(R)-(tetrahydro-furan-3-yl)oxy]-phenyl} -5-trifluoromethyl-indan-1-yloxy)-2 ,3-dihydro-benzofuran-3-yl]-acetic acid methyl ester |
| 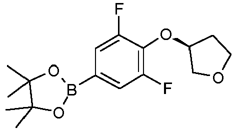 | **52hzk** | 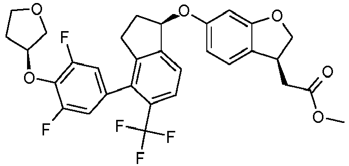 |
| 52gzue | | [(S)-6-((R)-4-{3,5-Difluoro-4-[(S)-(tetrahydro-furan-3-yl)oxy]-phenyl} -5-trifluoromethyl-indan-1-yloxy)-2 ,3-dihydro-benzofuran-3-yl]-acetic acid methyl ester |
| 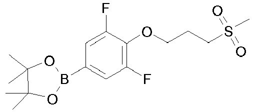 | **52hzl** | 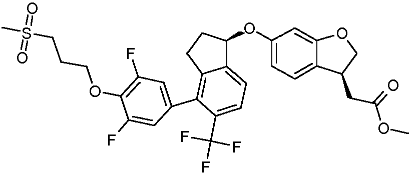 |
| 52gzuf | | ((S)-6-{(R)-4-[3,5-Difluoro-4-(3-me thanesulfonyl-propoxy)-phenyl]-5-trifluoromethyl-indan-1-yloxy}-2,3-di hydro-benzofuran-3-yl)-acetic acid methyl ester |
| 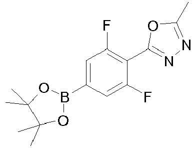 | **52hzm** | 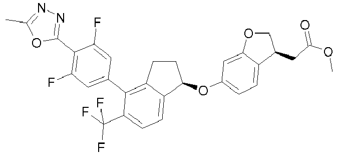 |
| 52gzug | | ((S)-6-{(R)-4-[3,5-Difluoro-4-(5-me thyl-[1,3,4]oxadiazol-2-yl)-phenyl] -5-trifluoromethyl-indan-1-yloxy}-2 ,3-dihydro-benzofuran-3-yl)-acetic acid methyl ester |
| 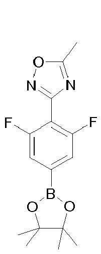 | **52hzn** | 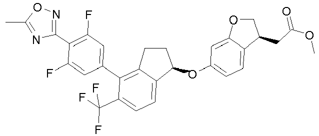 |
| 52gzuh | | ((S)-6-{(R)-4-[3,5-Difluoro-4-(5-me thyl-[1,2,4]oxadiazol-3-yl)-phenyl] -5-trifluoromethyl-indan-1-yloxy}-2 ,3-dihydro-benzofuran-3-yl)-acetic acid methyl ester |
| 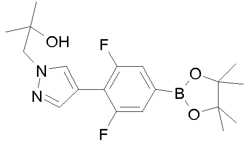 | **52hzo** | 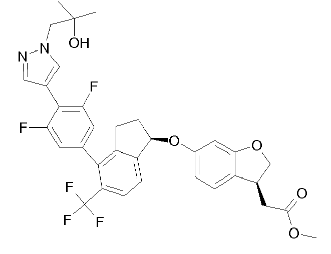 |
| 52gzui | | [(S)-6-((R)-4-{3,5-Difluoro-4-[1-(2 -hydroxy-2-methyl-propyl)-1 H-pyrazo l-4-yl]-phenyl}-5-trifluoromethyl-i ndan-1-yloxy)-2,3-dihydro-benzofura n-3-yl]-acetic acid methyl ester |

### Intermediate 52hzp

### ((S)-6-{(R)-4-[5-(3-Hydroxy-3-methyl-butoxy)-pyrimidin-2-yl]-5-trifluoromethyl-indan-1-yloxy}-2,3-dihydro-benzofuran-3-yl)-acetic acid methyl ester

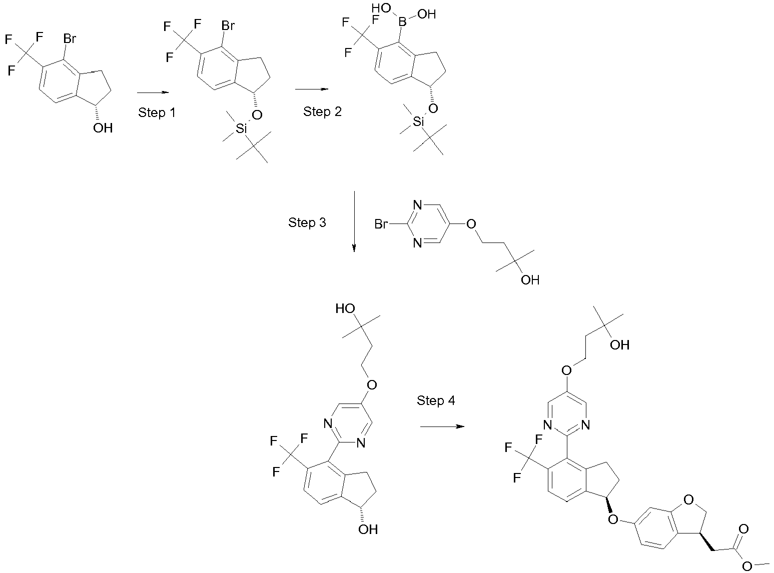

### Step 1: ((S)-4-Bromo-5-trifluoromethyl-indan-1-yloxy)-tert-butyl-dimethyl-silane

(S)-4-Bromo-5-trifluoromethyl-indan-1-ol (1 g), tert-butyl-dimethylchlorosilane (895 mg), imidazole (810 mg) and 4-dimethylamino pyridine (45 mg) are stirred in 20 mL of dichloromethane at room temperature for 1 h. The reaction mixture is washed with water, the organic phase is separated, dried over sodium sulfate and concentrated under vacuum. The residue is purified by flash chromatography (cyclohexane/ethyl acetate 100:0→90:10) to give the title compound. Yield: 1.32 g.

### Step 2: (S)-1-(tert-Butyl-dimethyl-silanyloxy)-5-trifluoromethyl-indan-4-boronic acid

((S)-4-Bromo-5-trifluoromethyl-indan-1-yloxy)-tert-butyl-dimethyl-silane (1.2 g) is stirred in 20 mL of tetrahydrofurane at -78 °C, a 2 M solution of N-butyllithium in cyclohexane (1.82 mL) is added and the reaction mixture is stirred for 2 h. Triisopropyl borate (800 mg) is added and the reaction mixture is stirred at -78 °C for 30 minutes, then allowed to reach room temperature and stirred 1 h. Saturated ammonium chloride water solution is added and the reaction mixture is extracted with ethyl acetate. The organic phase is washed with brine, separated, dried over sodium sulfate and concentrated under vacuum to give the tile compound. Yiel: 1.14 g.

### Step 3: (S)-4-[5-(3-Hydroxy-3-methyl-butoxy)-pyrimidin-2-yl]-5-trifluoromethyl-indan-1-ol

In a microwave vial, Intermediate 50z (99 mg), (S)-1-(tert-butyl-dimethyl-silanyloxy)-5-trifluoromethyl-indan-4-boronic acid (10 mg) tetrakis(triphenylphosphine) palladium(0) (46 mg) and bicarbonate saturated aqueous solution (450 mL) are suspended in 2 mL of 1,2-dimethoxyethane. The reaction mixture is irradiated in a MW oven at 120 °C for 1 h (50 W). Solvent is removed, saturated ammonium chloride water solution is added and the reaction mixture is extracted with dichloromethane. The organic phase is washed with brine, layers separated, the organic layer dried over sodium sulfate and concentrated under vacuum. The residue is purified by flash chromatography (cyclohexane/ethyl acetate 90:10→40:60) to give the title compound. Yield: 55 mg.

### Step 4: ((S)-6-{(R)-4-[5-(3-Hydroxy-3-methyl-butoxy)-pyrimidin-2-yl]-5-trifluoromethyl-indan-1-yloxy}-2,3-dihydro-benzofuran-3-yl)-acetic acid methyl ester

The title compound is prepared in analogy to methyl 2-((S)-6-((R)-4-bromo-2,3-dihydro-1H-inden-1-yloxy)-2,3-dihydrobenzofuran-3-yl)acetate as in Step 6 for the preparation of Intermediate 1

### Intermediate 52hzq

### ((S)-6-{(R)-4-[4-(Tetrahydro-pyran-4-ylcarbamoyl)-phenyl]-5-trifluoromethyl-indan-1-yloxy}-2,3-dihydro-benzofuran-3-yl)-acetic acid methyl ester

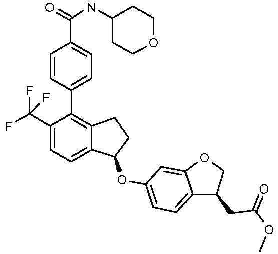

Intermediate 52a (70 mg), intermediate 52gzg (186 mg), 2-dicyclohexylphosphino-2'-(N,N'-dimethylamino)biphenyl (6 mg), tris(dibenzylideneacetone)dipalladium(0) (14 mg) and sodium carbonate (56 mg) are stirred in 10 mL of toluene and 5 mL of water at 90 °C for 4 h. then at room temperature overnight. The reaction mixture is washed with water, the organic phase is separated, dried over sodium sulfate and concentrated under vacuum. The residue is purified by flash chromatography (cyclohexane/ethyl acetate 70:30→50:50) to give the title compound. Yield: 31 mg.

The intermediates in the following table are prepared from Intermediate 52b and the corresponding starting boronic ester intermediates following a procedure analogous to that described for Intermediate 52ha

| **Starting boronic ester/acid Intermediate** | **Intermediate** | **Molecular structure and name** |
|---|---|---|
| 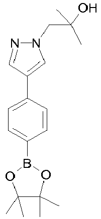 | **52ia** | 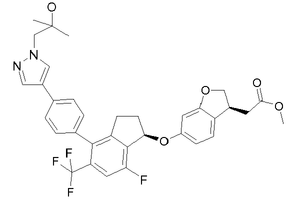 |
| 52ga | | [(S)-6-((R)-7-Fluoro-4-{4-[1-(2-hyd roxy-2-methyl-propyl)-1H-pyrazol-4-yl]-phenyl}-5-trifluoromethyl-indan -1-yloxy)-2,3-dihydro-benzofuran-3-yl]-acetic acid methyl ester |
| 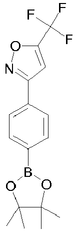 | **52ib** | 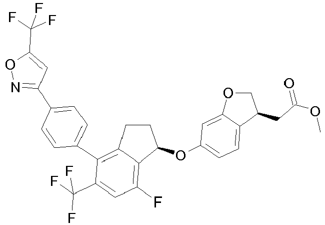 |
| 52gb | | ((S)-6-{(R)-7-Fluoro-5-trifluoromet hyl-4-[4-(5-trifluoromethyl-isoxazo l-3-yl)-phenyl]-indan-1-yloxy}-2,3-dihydro-benzofuran-3-yl)-acetic acid methyl ester |
| 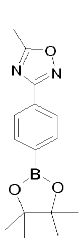 | **52ic** | 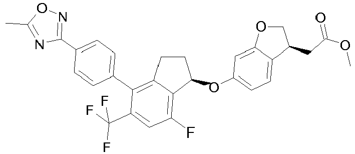 |
| 52gd | | ((S)-6-{(R)-7-Fluoro-4-[4-(5-methyl -[1,2,4]oxadiazol-3-yl)-phenyl]-5-t rifluoromethyl-indan-1-yloxy}-2,3-d ihydro-benzofuran-3-yl)-acetic acid methyl ester |
| 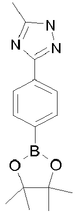 | **52id** | 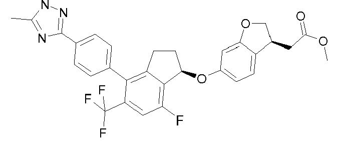 |
| 52ge | | ((S)-6-{(R)-7-Fluoro-4-[4-(5-methyl -1H-[1,2,4]triazol-3-yl)-phenyl]-5-trifluoromethyl-indan-1-yloxy}-2,3-dihydro-benzofuran-3-yl)-acetic acid methyl ester |
| 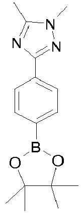 | **52ie** | 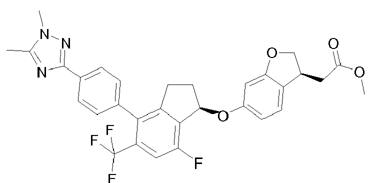 |
| 52gf | | ((S)-6-{(R)-4-[4-(1,5-Dimethyl-1H-[ 1,2,4]triazol-3-yl)-phenyl]-7-fluor o-5-trifluoromethyl-indan-1-yloxy}-2,3-dihydro-benzofuran-3-yl)-acetic acid methyl ester |
| 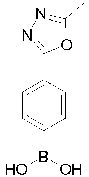 | **52if** | 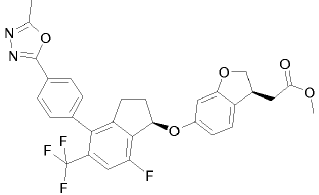 |
| 4-(5-methyl-1,3,4-oxadiazol-2-yl)-benzene boronic acid Commercially available | | ((S)-6-{(R)-7-Fluoro-4-[4-(5-methyl -[1,3,4]oxadiazol-2-yl)-phenyl]-5-t rifluoromethyl-indan-1-yloxy}-2,3-d ihydro-benzofuran-3-yl)-acetic acid methyl ester |
| 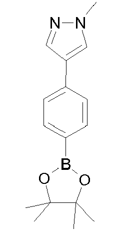 | **52ig** | 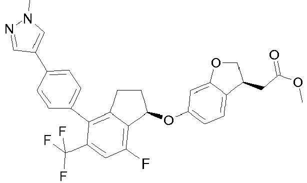 |
| 52gm | | ((S)-6-{(R)-7-Fluoro-4-[4-(1-methyl -1H-pyrazol-4-yl)-phenyl]-5-trifluo romethyl-indan-1-yloxy}-2,3-dihydro -benzofuran-3-yl)-acetic acid methyl ester |
| 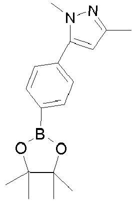 | **52ih** | 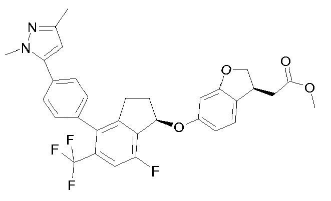 |
| 52gn | | ((S)-6-{(R)-4-[4-(2,5-Dimethyl-2H-p yrazol-3-yl)-phenyl]-7-fluoro-5-tri fluoromethyl-indan-1-yloxy}-2,3-dih ydro-benzofuran-3-yl)-acetic acid methyl ester |
| 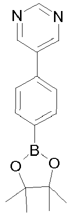 | **52ii** | 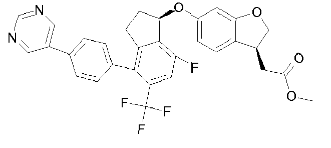 |
| 52gp | | {(S)-6-[(R)-7 -Fluoro-4-(4-pyrimidin -5-yl-phenyl)-5-trifluoromethyl-ind an-1-yloxy]-2,3-dihydro-benzofuran-3-yl}-acetic acid methyl ester |
| 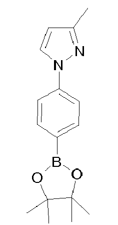 | **52ij** | 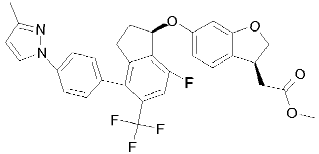 |
| 52gra | | ((S)-6-{(R)-7-Fluoro-4-[4-(3-methyl -pyrazol-1-yl)-phenyl]-5-trifluorom ethyl-indan-1-yloxy}-2,3-dihydro-be nzofuran-3-yl)-acetic acid methyl ester |
| 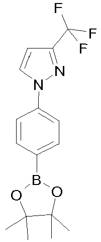 | **52ik** | 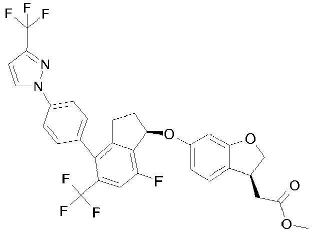 |
| 52gs | | ((S)-6-{(R)-7-Fluoro-5-trifluoromet hyl-4-[4-(3-trifluoromethyl-pyrazol -1-yl)-phenyl]-indan-1-yloxy}-2,3-d ihydro-benzofuran-3-yl)-acetic acid methyl ester |
| 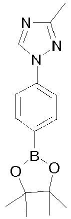 | **52il** | 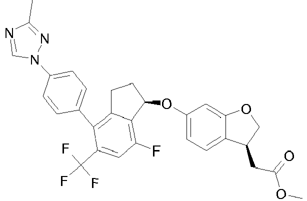 |
| 52gt | | ((S)-6-{(R)-7-Fluoro-4-[4-(3-methyl -[1,2,4]triazol-1-yl)-phenyl]-5-tri fluoromethyl-indan-1-yloxy}-2,3-dih ydro-benzofuran-3-yl)-acetic acid m ethyl ester |
| 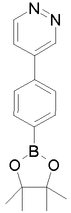 | **52im** | 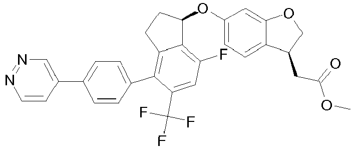 |
| 52gy | | {(S)-6-[(R)-7-Fluoro-4-(4-pyridazin -4-yl-phenyl)-5-trifluoromethyl-ind an-1-yloxy]-2,3-dihydro-benzofuran-3-yl}-acetic acid methyl ester |
| 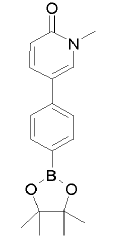 | **52in** | 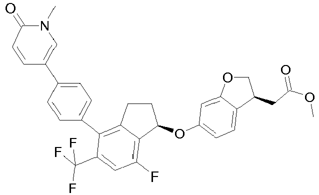 |
| 52gz | | ((S)-6-{(R)-7-Fluoro-4-[4-(1-methyl-6-oxo-1,6-dihydro-pyridin-3-yl)-ph enyl]-5-trifluoromethyl-indan-1-ylo xy}-2,3-dihydro-benzofuran-3-yl)-ac etic acid methyl ester |
| 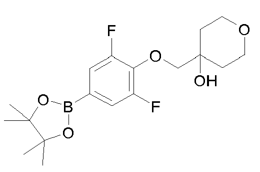 | **52io** | 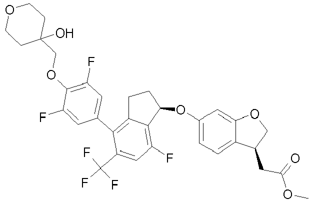 |
| 52gzua | | ((S)-6-{(R)-4-[3,5-Difluoro-4-(4-hy d roxy-tetra hyd ro-pyra n-4-yl m ethoxy) -phenyl]-7-fluoro-5-trifluoromethyl -indan-1-yloxy}-2,3-dihydro-benzofu ran-3-yl)-acetic acid methyl ester |

### Intermediate 53

### Methyl 2-((S)-6-((R)-4-(2,6-dimethyl-4-(3-(methylsulfonyl)propoxy)phenyl)-7-fluoro-2,3-dihydro-1H-inden-1-yloxy)-2,3-dydrobenzofuran-3-yl)acetate

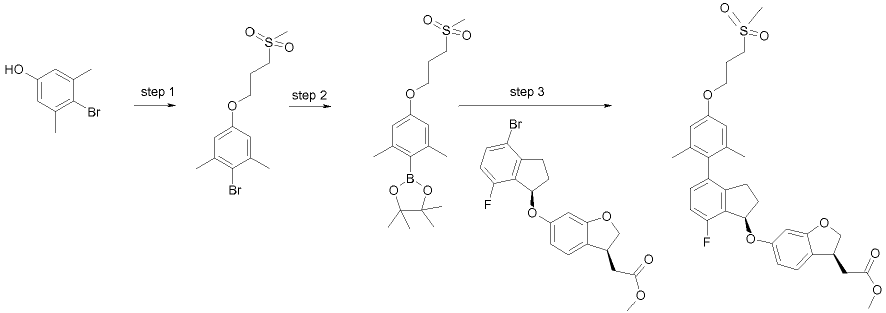

### Step 1: 2-Bromo-1,3-dimethyl-5-(3-(methylsulfonyl)propoxy)benzene

4-Bromo-3,5-dimethylphenol (0.5 g), 3-methylthiopropanol (0.25 mL), triphenyl phosphine (710 mg) and di-tert-butyl azodicarboxylate (624 mg) are dissolved in dichloromethane (10 mL) and stirred for 2 hours. 3-Chloroperbenzoic acid (1.3 g) is added and the mixture is stirred overnight. The mixture is washed with saturated sodium carbonate solution, the organic phase collected by passing through a phase seperator cartridge and the solvent removed under vacuum. The residue is purified by chromatography on silica gel (cyclohexane/ethyl acetate 100:0→0:100) to give the title compound (480 mg).

### Step 2: 2-(2,6-Dimethyl-4-(3-(methylsulfonyl)propoxy)phenyl)-4,4,5,5-tetramethyl-1,3,2-dioxaborolane

2-Bromo-1,3-dimethyl-5-(3-(methylsulfonyl)propoxy)benzene (100 mg), bis(pinaco lato)diboron (158 mg), [1,1'-bis(diphenylphosphino)-ferrocene]-dichloropalladium-(II) (23 mg) and potassium acetate (92 mg) are suspended in dry 1,4-dioxane (3 mL) in a microwave vial and degassed for 5 minutes with a flow of nitrogen. The mixture is heated under microwave irradiation for 60 minutes at 110°C. The mixture is diluted with ethyl acetate, washed with water, the organic phase dried and the solvent removed under vacuum. The residue is purified by chromatography on silica gel (cyclohexane/ethyl acetate 100:0→0:100) to give the title compound (110 mg).
LC (method 19): t_{R} = 1.24 min; Mass spectrum: m/z = 386 [M+NH₄]⁺.

### Step 3: Methyl 2-((S)-6-((R)-4-(2,6-dimethyl-4-(3-(methylsulfonyl)propoxy)phenyl)-7-fluoro-2,3-dihydro-1H-inden-1-yloxy)-2,3-dihydrobenzofuran-3-yl)acetate

Methyl 2-((S)-6-((R)-4-bromo-7-fluoro-2,3-dihydro-1H-inden-1-yloxy)-2,3-dihydroben zofuran-3-yl)acetate [intermediate 13, step 3] (32 mg), 2-(2,6-dimethyl-4-(3-(methylsulfonyl)propoxy)phenyl)-4,4,5,5-tetramethyl-1,3,2-dioxaborolane (42 mg), [1,1'-bis(diphenylphosphino)-ferrocene]-dichloropalladium-(II) (6 mg), 1,1'-bis(diphenylphosphino)-ferrocene (4 mg) and caesium carbonate (50 mg) are suspended in toluene (2 mL) and degassed for 5 minutes with a flow of nitrogen. The mixture is heated under microwave irradiation for 90 minutes at 120°C. The mixture is diluted with ethyl acetate, washed with water, the organic phase dried and the solvent removed under vacuum. The residue is purified by chromatography on silica gel (cyclohexane/ethyl acetate 100:0→50:50) to give the title compound (9 mg). LC (method 19): t_{R} = 1.38 min; Mass spectrum: m/z = 583 [M+H]⁺.

### Intermediate 54

### Methyl 2-((S)-6-((R)-4-(2,6-dimethyl-4-(3-(methylsulfonyl)propoxy)phenyl)-5,7-difluoro-2,3-dihydro-1H-inden-1-yloxy)-2,3-dihydrobenzofuran-3-yl)acetate

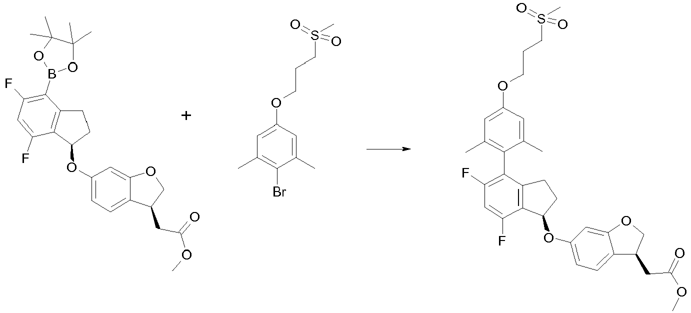

Methyl 2-((S)-6-((R)-5,7-difluoro-4-(4,4,5,5-tetramethyl-1,3,2-dioxaborolan-2-yl)-2,3-dihydro-1H-inden-1-yloxy)-2,3-dihydrobenzofuran-3-yl)acetate (Intermediate 52, 220 mg), 2-bromo-1,3-dimethyl-5-(3-(methylsulfonyl)propoxy)benzene (Intermediate 53, Step 1; 153 mg), palladium-(II)-acetate (7 mg), dicyclohexyl(2',6'-dimethoxybiphenyl-2-yl)phosphine (S-Phos) (13 mg) and K₃PO₄ (202 mg) are suspended in toluene (5 mL) and water (0.5 mL) and degassed for 5 minutes with a flow of argon. The mixture is heated under microwave irradiation for 2.5 hours at 120°C. The mixture is diluted with ethyl acetate, washed with water, the organic phase dried and the solvent removed under vacuum. The residue is purified by chromatography on silica gel (cyclohexane/ethyl acetate 100:0→0:100) to give the title compound (29 mg).
LC (method 19): t_{R} = 1.38 min; Mass spectrum: m/z = 601 [M+H]⁺.

### Intermediate 55

### Methyl 2-((3S)-6-((1R)-4-(2-(oxazol-4-yl)phenyl)-2,3-dihydro-1H-inden-1-yloxy)-2,3-dihydrobenzofuran-3-yl))acetate

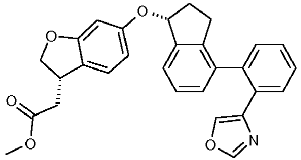

Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (45 mg) and 5-(2-Bromophenyl)-1,3-oxazole (33.6 mg) are dissolved in dimethylformamide (1 mL) and purged with argon. [1,1'-Bis(diphenylphosphino)ferrocene]dichloropalladium(II), complex with dichloromethane (1:1)] (8.2 mg) and aqueous Cs₂CO₃-solution (0.1 mL, 2 Mol/L) are added and the reaction mixture is shaken overnight in a sealed vial at 80°C. The reaction mixture is acidified with aqueous trifluoroacetic acid, filtrated and chromatographed by HPLC on reversed phase. The product fractions are collected and lyophilized to give the title compound. Yield: 15.7 mg; LC (method 22): t_{R} = 1.67 min; Mass spectrum (ESI⁺): m/z = 490 [M+Na]⁺.

### Intermediate 56

### Methyl 2-((3S)-6-((1R)-4-(2-(oxazol-5-yl)phenyl)-2,3-dihydro-1H-inden-1-yloxy)-2,3-dihydrobenzofuran-3-yl)acetate

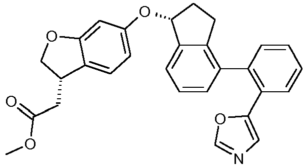

Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (45 mg) and 5-(2-Bromophenyl)-1,3-oxazole (33.6 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken overnight in a sealed vial at 80°C. The reaction mixture is acidified with aqueous trifluoroacetic acid, filtrated and chromatographed by HPLC on reversed phase. The product fractions are collected and lyophilized to give the title compound. Yield: 25 mg; LC (method 22): t_{R} = 1.67 min; Mass spectrum (ESI⁺): m/z = 468 [M+H]⁺.

### Intermediate 57

### Methyl 2-((3S)-6-((1R)-4-(2-(2-oxopyrrolidin-1-yl)phenyl)-2,3-dihydro-1H-inden-1-yloxy)-2,3-dihydrobenzofuran-3-yl)acetate

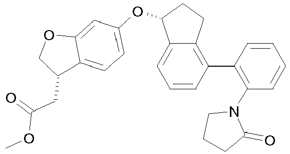

Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (45 mg) and 1-(2-bromophenyl)pyrrolidin-2-one (36.0 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken overnight in a sealed vial at 80°C. The reaction mixture is acidified with aqueous trifluoroacetic acid, filtrated and chromatographed by HPLC on reversed phase. The product fractions are collected and lyophilized to give the title compound. Yield: 16.4 mg; LC (method 11): t_{R} = 1.04 min; Mass spectrum (ESI⁺): m/z = 484 [M+H]⁺.

### Intermediate 58

### Methyl 2-((3S)-6-((1R)-4-(2-(isoxazol-5-yl)phenyl)-2,3-dihydro-1H-inden-1-yloxy)-2,3-dihydrobenzofuran-3-yl)acetate

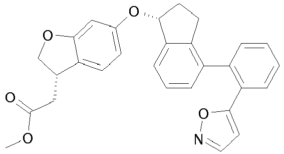

Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (45 mg) and 5-(2-Bromophenyl)isoxazole (33.6 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken 5 hours in a sealed vial at 80°C. The reaction mixture is acidified with aqueous trifluoroacetic acid, filtrated and chromatographed by HPLC on reversed phase. The product fractions are collected and lyophilized to give the title compound. Yield: 3 mg; LC (method 11): t_{R} = 1.14 min; Mass spectrum (ESI⁺): m/z = 490 [M+Na]⁺.

### Intermediate 59

### Methyl 2-((S)-6-((R)-4-(4-carbamoyl-2,6-dimethylphenyl)-2,3-dihydro-1H-inden-1-yloxy)-2,3-dihydrobenzofuran-3-yl)acetate

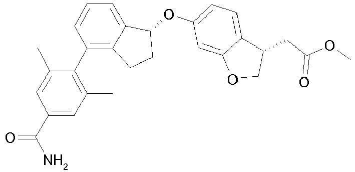

Methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (45 mg) and 4-Bromo-3,5-dimethyl-benzamide (34.2 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken 5 hours in a sealed vial at 80°C. The reaction mixture is acidified with aqueous trifluoroacetic acid, filtrated and chromatographed by HPLC on reversed phase. The product fractions are collected and lyophilized to give the title compound. Yield: 21.9 mg; LC (method 23): t_{R} = 1.88 min; Mass spectrum (ESI⁺): m/z = 472 [M+H]⁺.

### Intermediate 60

### Methyl 2-((S)-6-((R)-4-(4-sulfamoylphenyl)-2,3-dihydro-1H-inden-1-yloxy-2,3-dihydrobenzofuran-3-yl)acetate

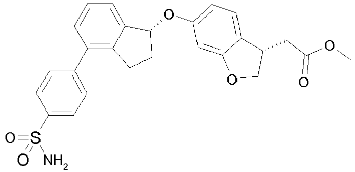

Methyl 2-((S)-6-((R)-4-bromo-2,3-dihydro-1H-inden-1-yloxy)-2,3-dihydrobenzofuran-3-yl)acetate (40.3 mg) and 4-sulfamoylphenylboronic acid (40.2 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken 12 hours in a sealed vial at 80°C. The reaction mixture is acidified with aqueous trifluoroacetic acid, filtrated and chromatographed by HPLC on reversed phase. The product fractions are collected and lyophilized to give the title compound. Yield: 4.8 mg.

### Intermediate 61

### Methyl 2-((3S)-6-((1R)-4-(2-acetylphenyl)-2,3-dihydro-1H-inden-1-yloxy)-2,3-dihydrobenzofuran-3-yl)acetate

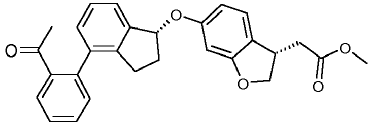

Methyl 2-((S)-6-((R)-4-bromo-2,3-dihydro-1H-inden-1-yloxy)-2,3-dihydrobenzofuran-3-yl)acetate (40.3 mg) and 2-acetylphenylboronic acid (32.8 mg) are dissolved in dimethylformamide (1 mL) and purged with argon. [1,1'-Bis(di-tert butylphosphino)-ferrocene]-dichloropalladium-(II) (6.5 mg) and aqueous Cs₂CO₃-solution (0.1 mL, 2 Mol/L) are added and the reaction mixture is shaken 12 hours in a sealed vial at 80°C. The reaction mixture is acidified with aqueous trifluoroacetic acid, filtrated and chromatographed by HPLC on reversed phase. The product fractions are collected and lyophilized to give the title compound. Yield: 5.9 mg; LC (method 24): t_{R} = 1.38 min.

### Intermediate 62

### 2-((3S)-6-((1R)-4-(3-Cyano-2-methylphenyl)-7-fluoro-2,3-dihydro-1H-inden-1-yloxy)-2,3-dihydrobenzofuran-3-yl)acetic acid

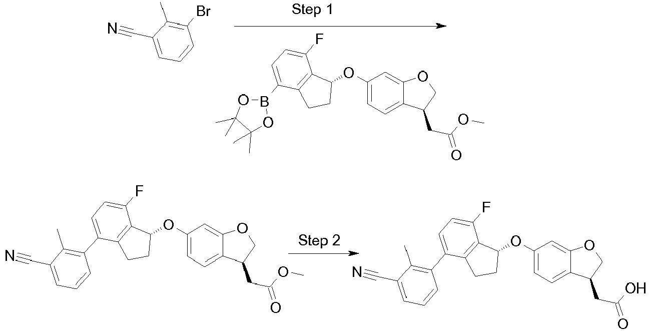

### Step 1: Methyl 2-((3S)-6-((1 R)-4-(3-cyano-2-methylphenyl)-7-fluoro-2,3-dihydro-1 H-inden-1-yloxy)-2,3-dihydrobenzofuran-3-yl)acetate

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (200 mg), 3-bromo-2-methylbenzonitrile (169 mg), K₃PO₄ (274 mg), Palladium-(II)-acetate (10 mg) and dicyclohexyl(2',6'-dimethoxybiphenyl-2-yl)phosphine (S-Phos) (18 mg) are suspended in toluene (10 mL) and water (2 mL) and purged for 10 minutes with argon. The vial is sealed and the mixture is stirred at 100°C for 4 hours. After cooling to ambient temperature the mixture is partitioned between ethyl acetate and water. The organic phase is dried (MgSO₄) and concentrated. The residue is chromatographed on silica gel (cyclohexane/ethyl acetate 90:10) to give the title compound. Yield: 124 mg; LC (method 20): t_{R} = 8.09 min; Mass spectrum (ESI⁺): m/z = 558 [M+-H]⁺.

### Step 2: 2-((3S)-6-((1R)-4-(3-Cyano-2-methylphenyl)-7-fluoro-2,3-dihydro-1H-inden-1-yloxy)-2,3-dihydrobenzofuran-3-yl)acetic acid

LiOHxH₂O (131 mg) is added to a solution of methyl 2-((3S)-6-((1 R)-4-(3-cyano-2-methylphenyl)-7-fluoro-2,3-dihydro-1H-inden-1-yloxy)-2,3-dihydrobenzofuran-3-yl)acetate (119 mg) in tetrahydrofuran (1 mL), water (1 mL) and methanol (1 mL) at room temperature. The mixture is stirred at room temperature for 12 hours. The mixture is concentrated, cooled to 0°C, diluted with water and acidified with 4 M aqueous HCl solution. The resulting mixture is extracted with dichloromethane. The organic phase is dried (MgSO₄). The solvent is evaporated to give the title compound. Yield: 103 mg; LC (method 20): t_{R} = 6.30 min; Mass spectrum (ESI⁻): m/z = 442 [M-H]⁻.

### Intermediate 63

### 2-((S)-6-((R)-4-(4-Cyano-2,6-dimethyliphenyl)-7-fluoro-2,3-dihydro-1H-inden-1-yloxy)-2,3-dihydrobenzofuran-3-yl)acetic acid

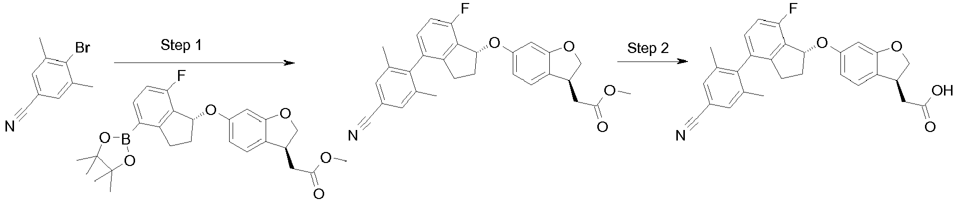

### Step 1: Methyl 2-((S)-6-((R)-4-(4-cyano-2,6-dimethylphenyl)-7-fluoro-2,3-dihydro-1H-inden-1-yloxy)-2,3-dihydrobenzofuran-3-yl)acetate

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (200 mg), 4-bromo-3,5-dimethylbenzonitrile (90 mg), K₃PO₄ (273 mg), Palladium-(II)-acetate (10 mg) and dicyclohexyl(2',6'-dimethoxybiphenyl-2-yl)phosphine (S-Phos) (17 mg) are suspended in toluene (10 mL) and water (2 mL) and purged for 10 minutes with argon. The vial is sealed and the mixture is stirred at 100°C for 4 hours. After cooling to ambient temperature the mixture is partitioned between ethyl acetate and water. The organic phase is dried (MgSO₄) and concentrated. The residue is chromatographed on silica gel (cyclohexane/ethyl acetate 90:10) to give the title compound. Yield: 85 mg; LC (method 19): t_{R} = 1.52 min; Mass spectrum (ESI⁺): m/z = 471 [M+-H]⁺.

### Step 2: 2-((S)-6-((R)-4-(4-Cyano-2,6-dimethylphenyl)-7-fluoro-2,3-dihydro-1H-inden-1-yloxy)-2,3-dihydrobenzofuran-3-yl)acetic acid

LiOHxH₂O (38 mg) is added to a solution of methyl 2-((S)-6-((R)-4-(4-cyano-2,6-dimethylphenyl)-7-fluoro-2,3-dihydro-1H-inden-1-yloxy)-2,3-dihydrobenzofuran-3-yl)acetate (85 mg) in tetrahydrofuran (1 mL), water (1 mL) and methanol (1 mL) at room temperature. The mixture is stirred at room temperature for 12 hours. The mixture is concentrated, cooled to 0°C, diluted with water and acidified with 4 M aqueous HCl solution. The resulting mixture is extracted with dichloromethane. The organic phase is dried (MgSO₄). The solvent is evaporated and the residue is chromatographed on silica gel (dichloroemthane/methanol/acetic acid 9:0.05:0.05) to give the title compound. Yield: 41 mg; LC (method 20): t_{R} = 6.91 min; Mass spectrum (ESI⁻): m/z = 456 [M-H]⁻.

### Intermediate 64

### Methyl 2-((S)-6-((R)-4-(4-(dimethylcarbamoyl)-2,6-dimethylphenyl)-7-fluoro-2,3-dihydro-1H-inden-1-yloxy)-2,3-dihydrobenzofuran-3-yl)acetate

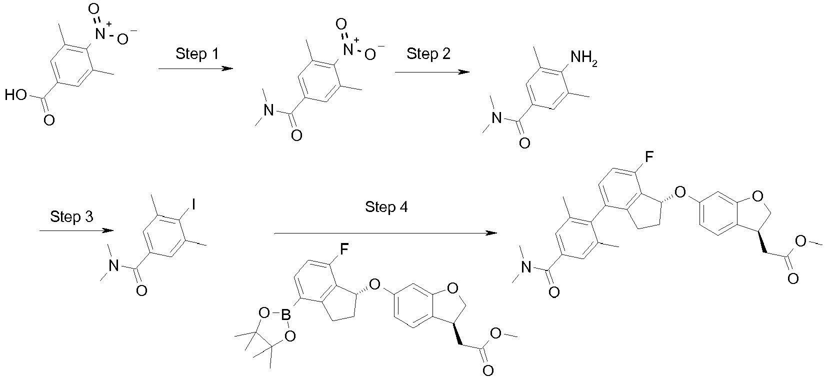

### Step 1: N,N,3,5-Tetramethyl-4-nitrobenzamide

1,1'-Carbonyldiimidazole (914 mg) is added to a solution of 3,5-dimethyl-4-nitrobenzoic acid (1 g) in tetrahydrofuran (10 mL). The mixture is stirred for 3 hours at room temperature, dimethylamine (7.7 mL, 2 M in tetrahydrofuran) is added and the mixture is stirred for further 30 minutes. After concentration the mixture is diluted with ethyl acetate and washed with hydrochloric acid (0.2 M), saturated aqueous NaHCO₃ solution and brine. The organic phase is dried (Na₂SO₄) and concentrated to give the title compound. Yield: 1 g; LC (method 20): t_{R} = 5.11 min; Mass spectrum (ESI⁺): m/z = 223 [M+H]⁺.

### Step 2: 4-Amino-N,N,3,5-tetramethylbenzamide

10% Palladium on carbon (100 mg) is adde to a solution of N,N,3,5-tetramethyl-4-nitrobenzamide (1 g) in methanol (10 mL) and the mixture is hydrogenated for 3 hours under 2 bar hydrogen pressure. Then the catalyst is filtered off and washed with methanol. The combined mother liquours are concentrated to give the title compound. Yield: 1 g; LC (method 20): t_{R} = 2.75 min; Mass spectrum (ESI⁺): m/z = 193 [M+H]⁺.

### Step 3: 4-lodo-N,N,3,5-tetramethylbenzamide

4-Amino-N,N,3,5-tetramethylbenzamide (500 mg) is dissolved in concentrated hydrochloric acid (2 mL), cooled to 0°C and treated dropwise with a solution of NaNO₂ (269 mg) in water (0.5 mL). The mixture is stirred for 1 hour and a solution of Kl (1.3 g) in water (1.5 mL) is added dropwise. The mixture is stirred for 15 minutes, allowed to warm to room temperature and then partitioned between dichloromethane and water. The organic phase is washed with 10% aqueous solution of Na₂S₂O₃, dried (MgSO₄) and concentrated. The residue is chromatographed on silica gel (cyclohexane/ethyl acetate 100:0→50:50) to give the title compound. Yield: 300 mg; LC (method 20): t_{R} = 6.32 min; Mass spectrum (ESI⁺): m/z = 304 [M+H]⁺.

### Step 4: Methyl 2-((S)-6-((R)-4-(4-(dimethylcarbamoyl)-2,6-dimethylphenyl)-7-fluoro-2,3-dihydro-1H-inden-1-yloxy)-2,3-dihydrobenzofuran-3-yl)acetate

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (75 mg), 4-iodo-N,N,3,5-tetramethylbenzamide (97 mg), K₃PO₄ (102 mg) and dicyclohexyl(2',6'-dimethoxybiphenyl-2-yl)phosphine (S-Phos) (7 mg) are suspended in toluene (2 mL) and water (0.2 mL) and purged for 10 minutes with argon. Palladium-(II)-acetate (4 mg) are added, the vial is sealed and the mixture is stirred at 120°C for 5 hours. After cooling to ambient temperature the mixture is partitioned between ethyl acetate and water. The organic phase is dried (MgSO₄) and concentrated. The residue is chromatographed on silica gel (cyclohexane/ethyl acetate 100:0→80:20) to give the title compound. Yield: 70 mg; LC (method 25): t_{R} = 1.41 min; Mass spectrum (ESI⁺): m/z = 518 [M+H]⁺.

### Intermediate 65

### Methyl 2-((S)-6-((R)-4-(2,6-dimethyl-4-(methylcarbamoyl)lphenyl)-7-fluoro-2,3-dihydro-1H-inden-1-yloxy)-2,3-dihydrobenzofuran-3-yl)acetate

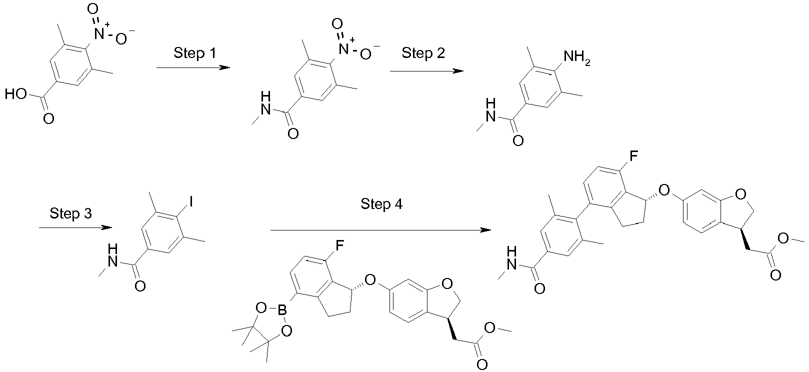

### Step 1: N,3,5-Trimethyl-4-nitrobenzamide

1,1'-Carbonyldiimidazole (914 mg) is added to a suspension of 3,5-dimethyl-4-nitrobenzoic acid (1 g) in tetrahydrofuran (10 mL). The mixture is stirred for 3 hours at room temperature, methylamine (7.7 mL, 2 M in tetrahydrofuran) is added and the mixture is stirred for further 30 minutes. After concentration the mixture is diluted with ethyl acetate and washed with hydrochloric acid (0.2 M), saturated aqueous NaHCO₃ solution and brine. The organic phase is dried (Na₂SO₄) and concentrated to give the title compound. Yield: 1 g; LC (method 20): t_{R} = 4.82 min; Mass spectrum (ESI⁺): m/z = 209 [M+H]⁺.

### Step 2: 4-Amino-N,3,5-trimethylbenzamide

10% Palladium on carbon (100 mg) is added to a solution of N,3,5-trimethyl-4-nitrobenzamide (1 g) in methanol (10 mL) and the mixture is hydrogenated for 3 hours under 3 bar hydrogen pressure. Then the catalyst is filtered off and washed with methanol. The combined mother liquours are concentrated to give the title compound. Yield: 850 mg; LC (method 21): t_{R} = 6.02; Mass spectrum (ESI⁺): m/z = 179 [M+H]⁺.

### Step 3: 4-lodo-N,3,5-trimethylbenzamide

4-Amino-N,3,5-trimethylbenzamide (850 mg) is dissolved in concentrated hydrochloric acid (2 mL), cooled to 0°C and treated dropwise with a solution of NaNO₂ (580 mg) in water (0.5 mL). The mixture is stirred for 1 hour and a solution of Kl (2.8 g) in water (1.5 mL) is added dropwise. The mixture is stirred for 15 minutes, allowed to warm to room temperature and then partitioned between dichloromethane and water. The organic phase is washed with 10% aqueous solution of Na₂S₂O₃, dried (MgSO₄) and concentrated. The residue is chromatographed on silica gel (cyclohexane/ethyl acetate 100:0→50:50) to give the title compound. Yield: 850 mg; LC (method 20): t_{R} = 5.88 min; Mass spectrum (ESI⁺): m/z = 290 [M+H]⁺.

### Step 4: Methyl 2-((S)-6-((R)-4-(2,6-dimethyl-4-(methylcarbamoyl)phenyl)-7-fluoro-2,3-dihydro-1H-inden-1-yloxy)-2,3-dihydrobenzofuran-3-yl)acetate

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (75 mg), 4-iodo-N,3,5-trimethylbenzamide (93 mg), K₃PO₄ (102 mg) and dicyclohexyl(2',6'-dimethoxybiphenyl-2-yl)phosphine (S-Phos) (7 mg) are suspended in toluene (1.8 mL) and water (0.2 mL) and purged for 10 minutes with argon. Palladium-(II)-acetate (4 mg) is added, the vial is sealed and the mixture is stirred at 120°C for 5 hours. After cooling to ambient temperature the mixture is partitioned between ethyl acetate and water. The organic phase is dried (MgSO₄) and concentrated. The residue is chromatographed on silica gel (cyclohexane/ethyl acetate 100:0→80:20) to give the title compound. Yield: 16 mg; LC (method 25): t_{R} = 1.32 min; Mass spectrum (ESI⁺): m/z = 504 [M+H]⁺.

### Intermediate 66

### Methyl 2-((S)-6-((R)-4-(2,6-dimethyl-4-(morpholine-4-carbonyl)phenyl)-7-fluoro-2,3-dihydro-1H-inden-1-yloxy)-2,3-dihydrobenzofuran-3-yl)acetate

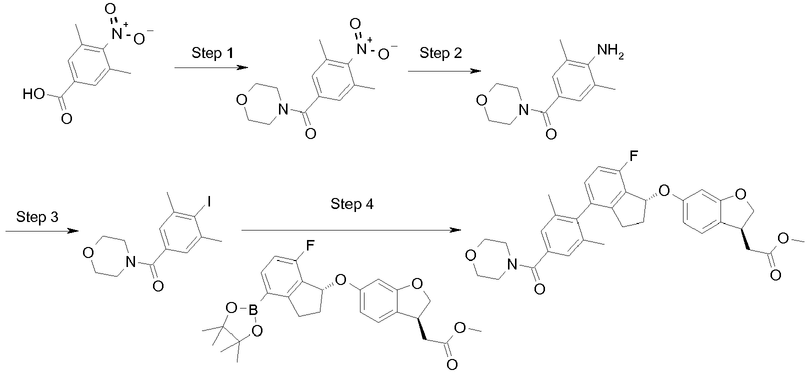

### Step 1: (3,5-Dimethyl-4-nitrophenyl)(morpholino)methanone

1,1'-Carbonyldiimidazole (1.66 g) is added to a solution of 3,5-dimethyl-4-nitrobenzoic acid (1 g) in tetrahydrofuran (10 mL). The mixture is stirred for 3 hours at room temperature, morpholine (0.9 mL) is added and the mixture is stirred for further 30 minutes. After concentration the mixture is diluted with ethyl acetate and washed with hydrochloric acid (0.2 M), saturated aqueous NaHCO₃ solution and brine. The organic phase is dried (Na₂SO₄) and concentrated to give the title compound. Yield: 1.3 g; LC (method 20): t_{R} = 5.10 min; Mass spectrum (ESI⁺): m/z = 265 [M+H]⁺.

### Step 2: (4-Amino-3,5-dimethylphenyl)(morpholino)methanone

10% Palladium on carbon (100 mg) is adde to a solution of (3,5-dimethyl-4-nitrophenyl)(morpholino)methanone (1.3 g) in methanol (10 mL) and the mixture is hydrogenated for 3 hours under 2 bar hydrogen pressure. Then the catalyst is filtered off and washed with methanol. The combined mother liquours are concentrated to give the title compound. Yield: 1.1 g; LC (method 20): t_{R} = 1.94; Mass spectrum (ESI⁺): m/z = 235 [M+H]⁺.

### Step 3: (4-lodo-3,5-dimethylphenyl)(morpholino)methanone

(4-Amino-3,5-dimethylphenyl)(morpholino)methanone (1.2 g) is dissolved in concentrated hydrochloric acid (2 mL), cooled to 0°C and treated dropwise with a solution of NaNO₂ (530 mg) in water (0.5 mL). The mixture is stirred for 1 hour and a solution of Kl (2.6 g) in water (1.5 mL) is added dropwise. The mixture is stirred for 15 minutes at 0°C and for 12 hours at room temperature and is then partitioned between dichloromethane and water. The organic phase is washed with 10% aqueous solution of Na₂S₂O₃, dried (MgSO₄) and concentrated. The residue is chromatographed on silica gel (cyclohexane/ethyl acetate 100:0→50:50) to give the title compound. Yield: 1.1 g; LC (method 20): t_{R} = 6.13 min; Mass spectrum (ESI⁺): m/z = 346 [M+H]⁺.

### Step 4: Methyl 2-((S)-6-((R)-4-(2,6-dimethyl-4-(morpholine-4-carbonyl)phenyl)-7-fluoro-2,3-dihydro-1H-inden-1-yloxy)-2,3-dihydrobenzofuran-3-yl)acetate

In a microwave vial, methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (75 mg), (4-iodo-3,5-dimethylphenyl)(morpholino)methanone (110 mg), K₃PO₄ (102 mg) and dicyclohexyl(2',6'-dimethoxybiphenyl-2-yl)phosphine (S-Phos) (7 mg) are suspended in toluene (2 mL) and water (0.2 mL) and purged for 10 minutes with argon. Palladium-(II)-acetate (4 mg) are added, the vial is sealed and the mixture is stirred at 120°C for 5 hours. After cooling to ambient temperature the mixture is partitioned between ethyl acetate and water. The organic phase is dried (MgSO₄) and concentrated. The residue is chromatographed on silica gel (cyclohexane/ethyl acetate 100:0→80:20) to give the title compound. Yield: 55 mg; LC (method 25): t_{R} = 1.38 min; Mass spectrum (ESI⁺): m/z = 560 [M+H]⁺.

### Intermediate 66a

### ((S)-6-{(R)-7-Fluoro-4-[4-(2-hydroxy-2-methyl-propylcarbamoyl)-2,6-dimethyl-phenyl]-indan-1-yloxy}-2,3-dihydro-benzofuran-3-yl)-acetic acid methyl ester

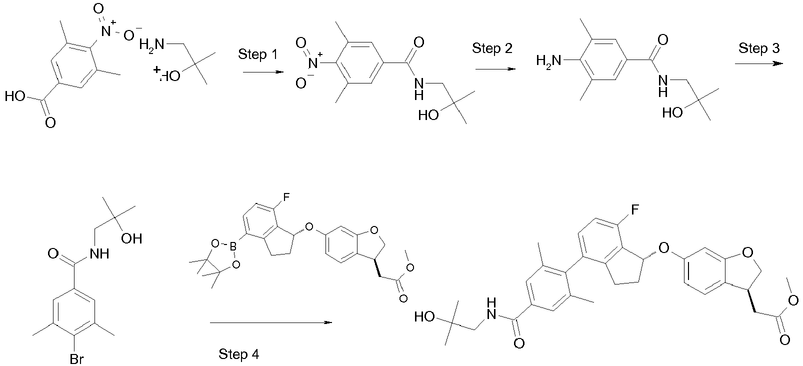

### Step 1: N-(2-Hydroxy-2-methyl-propyl)-3,5-dimethyl-4-nitro-benzamide.

The title compound is prepared following a procedure analogous to that described in Step 1 in the preparation of Intermediate 66, starting from 1-amino-2-methyl-propan-2-ol (Kasai, Shizuo et al. Journal of Medicinal Chemistry, 2012 , vol. 55, # 9 p. 4336-4351).

### Step 2: 4-Amino-N-(2-hydroxy-2-methyl-propyl)-3,5-dimethyl-benzamide.

The title compound is prepared following a procedure analogous to that described in Step 2 in the preparation of Intermediate 50x, starting from N-(2-hydroxy-2-methylpropyl)-3,5-dimethyl-4-nitro-benzamide.

### Step 3: 4-Bromo-N-(2-hydroxy-2-methyl-propyl)-3,5-dimethyl-benzamide.

The title compound is prepared following a procedure analogous to that described in Step 3 in the preparation of Intermediate 50x, starting from 4-amino-N-(2-hydroxy-2-methyl-propyl)-3,5-dimethyl-benzamide.

### Step 4: ((S)-6-{(R)-7-Fluoro-4-[4-(2-hydroxy-2-methyl-propylcarbamoyl)-2,6-dimethyl-phenyl]-indan-1-yloxy}-2,3-dihydro-benzofuran-3-yl)-acetic acid methyl ester.

The title compound is prepared following a procedure analogous to that described in Step 6 in the preparation of Intermediate 1, starting from 4-bromo-N-(2-hydroxy-2-methyl-propyl)-3,5-dimethyl-benzamide.

### Intermediate 67

### Methyl 2-((S)-6-((R)-4-(2-cyano-2-methylpropoxy)-2,6-dimethylphenyl-7-fluoro-2,3-dihydro-1H-inden-1-yloxy)-2,3-dihydrobenzofuran-3-yl)acetate

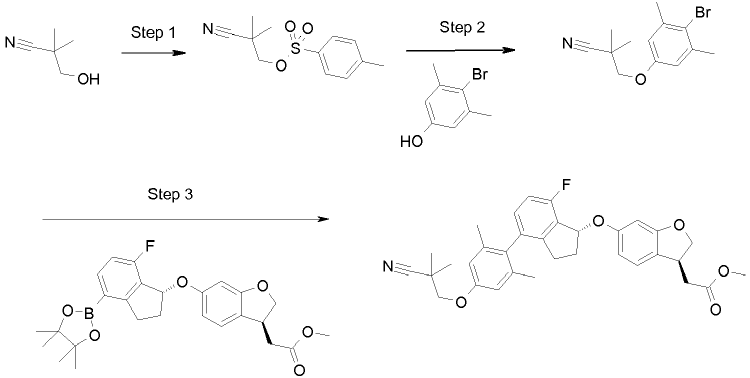

### Step 1: 2-Cyano-2-methylpropyl 4-methylbenzenesulfonate

To a solution of 3-hydroxy-2,2-dimethylpropanenitrile (0.5 g) in dichloromethane (8 mL) and pyridine (1.5 mL) is added at 0°C p-toluene-sulfonylchloride (1.0 g) in portions. The mixture is stirred for 12 hours at room temperature, diluted with diethylether and washed with 1 M aqueous HCl solution and brine. After drying (MgSO₄) the solvent is evaporated and the residue is chromatographed on silica gel (petrole ether/ethyl acetate 90:10→50:50) to give the title compound. Yield: 770 mg; Mass spectrum (ESI⁺): m/z = 254 [M+H]⁺.

### Step 2: 3-(4-Bromo-3,5-dimethylphenoxy)-2,2-dimethylpropanenitrile

To a solution of 2-cyano-2-methylpropyl 4-methylbenzenesulfonate (760 mg) and 4-bromo-3,5-dimethylphenol (500 mg) in N,N-dimethylformamide (8 mL) is added Cs₂CO₃ (2.0 g). The mixture is stirred for 12 hours at 80°C, diluted with diethylether and washed with water and brine. After drying (MgSO₄) the solvent is evaporated and the residue is chromatographed on silica gel (petrole ether/ethyl acetate 90:10→60:40) to give the title compound. Yield: 572 mg; Mass spectrum (ESI⁺): m/z = 282 [M+H]⁺.

### Step 3: Methyl 2-((S)-6-((R)-4-(4-(2-cyano-2-methylpropoxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-(4-bromo-3,5-dimethylphenoxy)-2,2-dimethylpropanenitrile following a procedure analogous to that described in Step 5 of Intermediate 12. LC (method 8): t_{R} = 0.89 min; Mass spectrum (ESI⁺): m/z = 544 [M+H]⁺.

### Intermediate 68

### Methyl 2-((S)-6-((R)-5-cyano-4-(4-(3-hydroxy-3-methylbutoxy)-2,6-dimethylphenyl)-2,3-dihydro-1H-inden-1-yloxy)-2,3-dihydrobenzofuran-3-yl)acetate

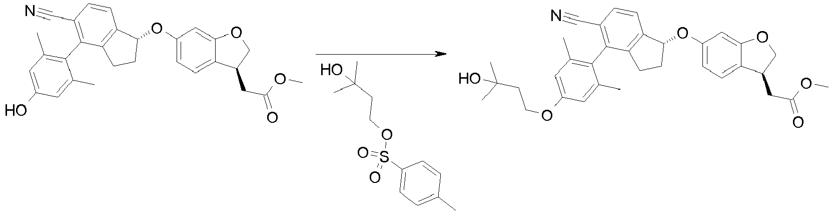

The title compound is prepared from methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate and 3-hydroxy-3-methylbutyl 4-methylbenzenesulfonate following a procedure analogous to that described in Step 5 of Intermediate 39. LC (method 7): t_{R} = 1.17 min; Mass spectrum (ESI⁺): m/z = 578 [M+Na]⁺.

### Intermediate 69

### Methyl 2-((S)-6-((R)-5-cyano-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate

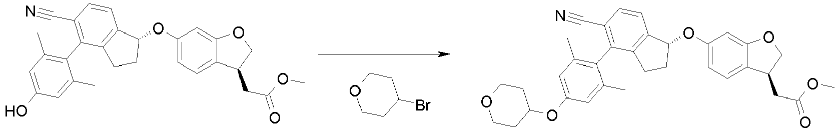

To a solution of methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (136 mg) in N,N-dimethylformamide (2 mL) is added 4-bromotetrahydro-2H-pyran (72 mg) and Cs₂CO₃ (200 mg). The mixture is stirred for 12 hours at room temperature and 1 hour at 100°C. 4-Bromotetrahydro-2H-pyran (72 mg) is added and the mixture is heated to 100°C for further 3 hours. Then the mixture is diluted with ethyl acetate and washed successively with water and brine. The organic phase is dried (MgSO₄) and concentrated. The residue is chromatographed on silica gel (cyclohexane/ethyl acetate 99:1→40:60) to give the title compound. Yield: 37 mg; LC (method 7): t_{R} = 1.19 min; Mass spectrum (ESI⁺): m/z = 554 [M+H]⁺.

### Intermediate 70

### Methyl 2-(S)-6-((R)-4-(4-(2-(tert-butoxycarbonylamino)ethoxy)-2,6-dimethylphenyl)-7-fluoro-2,3-dihydro-1H-inden-1-yloxy)-2,3-dihydrobenzofuran-3-yl)acetate

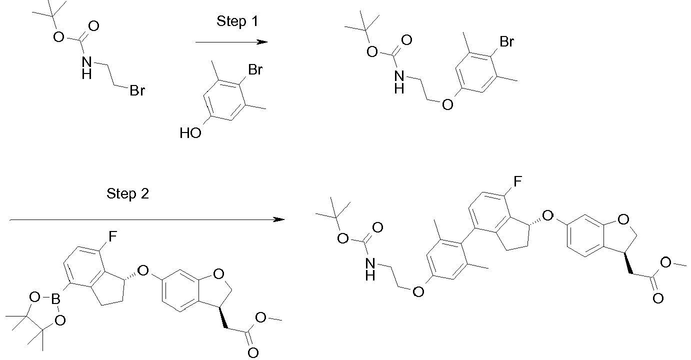

### Step 1: tert-Butyl 2-(4-bromo-3,5-dimethylphenoxy)ethylcarbamate

To a solution of 4-bromo-3,5-dimethylphenol (200 mg) in N,N-dimethylformamide (5 mL) is added tert-butyl 2-bromoethylcarbamate (270 mg) and Cs₂CO₃ (810 mg). The mixture is stirred for 20 hours at room temperature. tert-Butyl 2-bromoethylcarbamate (110 mg) and Cs₂CO₃ (325 mg) are added and the mixture is stirred for 4 hours. Again tert-butyl 2-bromoethylcarbamate (110 mg) and Cs₂CO₃ (325 mg) are added and the mixture is stirred for 12 hours. The mixture is partitioned between water and diethylether. The organic phase is washed with brine and dried (MgSO₄). The solvent is evaporated and the residue is chromatographed on silica gel (cyclohexane/ethyl acetate 99:1→80:20) to give the title compound. Yield: 320 mg; LC (method 8): t_{R} = 0.66 min; Mass spectrum (ESI⁺): m/z = 344 [M+H]⁺.

### Step 2: Methyl 2-((S)-6-((R)-4-(4-(2-(tert-butoxycarbonylamino)ethoxy)-2,6-dimethyl phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and tert-butyl 2-(4-bromo-3,5-dimethylphenoxy)ethylcarbamate following a procedure analogous to that described in Step 5 of Intermediate 12. LC (method 8): t_{R} = 0.92 min; Mass spectrum (ESI⁺): m/z = 606 [M+H]⁺.

### Intermediate 71

### Methyl 2-((S)-6-((R)-5-cyano-4-(4-(2-hydroxy-2-methylpropoxy)-2,6-dimethylphenyl)-2,3-dihydro-1H-inden-1-yloxy)-2,3-dihydrobenzofuran-3-yl)acetate

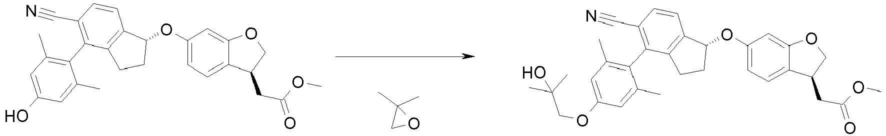

To a solution of methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (120 mg) in N,N-dimethylformamide (2 mL) is added 2,2-dimethyloxirane (30 µL) and Cs₂CO₃ (167 mg). The mixture is stirred for 12 hours at 100°C. 2,2-Dimethyloxirane (30 µL) and Cs₂CO₃ (167 mg) are added and the mixture is stirred for 5 hours at 100°C. The mixture is then partitioned between saturated water and ethyl acetate. The organic phase is washed with brine, dried (MgSO₄) and concentrated. The residue is chromatographed on silica gel (cyclohexane/ethyl acetate 99:1→50:50) to give the title compound. Yield: 48 mg; LC (method 7): t_{R} = 1.16 min; Mass spectrum (ESI⁺): m/z = 542 [M+H]⁺.

### Intermediate 72

### Methyl 2-((S)-6-((R)-7-fluoro-4-(4-((4-hydroxytetrahydro-2H-pyran-4-yl)methoxy)-2,6-dimethyliphenyl)-2,3-dihydro-1H-inden-1-yloxy)-2,3-dihydrobenzofuran-3-yl)acetate

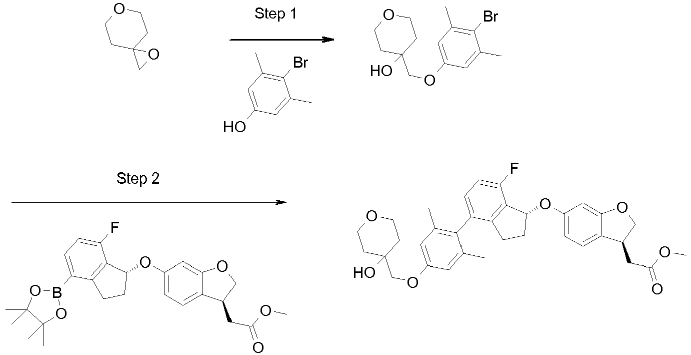

### Step 1: 4-((4-Bromo-3,5-dimethylphenoxy)methyl)tetrahydro-2H-pyran-4-ol

The title compound is prepared from 4-bromo-3,5-dimethylphenol and 1,6-dioxaspiro[2.5]octane following a procedure analogous to that described in Step 5 of Intermediate 1. LC (method 7): t_{R} = 1.04 min; Mass spectrum (ESI⁻): m/z = 313 [M-H]⁻

### Step 2: Methyl 2-((S)-6-((R)-7-fluoro-4-(4-((4-hydroxytetrahydro-2H-pyran-4-yl)methoxy)-2,6-dimethylphenyl)-2,3-dihydro-1H-inden-1-yloxy)-2,3-dihydrobenzofuran-3-yl)acetate

The title compound is prepared from methyl methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-((4-bromo-3,5-dimethylphenoxy)methyl)tetrahydro-2H-pyran-4-ol following a procedure analogous to that described in Step 5 of Intermediate 12. The product is purified by HPLC on reversed phase. LC (method 7): t_{R} = 1.17 min; Mass spectrum (ESI⁺): m/z = 577 [M+H]⁺.

### Intermediate 73

### Methyl 2-((S)-6-((R)-7-fluoro-4-(4-(3-hydroxy-3-methylbutyl)-2,6-dimethylphenyl)-2,3-dihydro-1H-inden-1-yloxy)-2,3-dihydrobenzofuran-3-yl)acetate

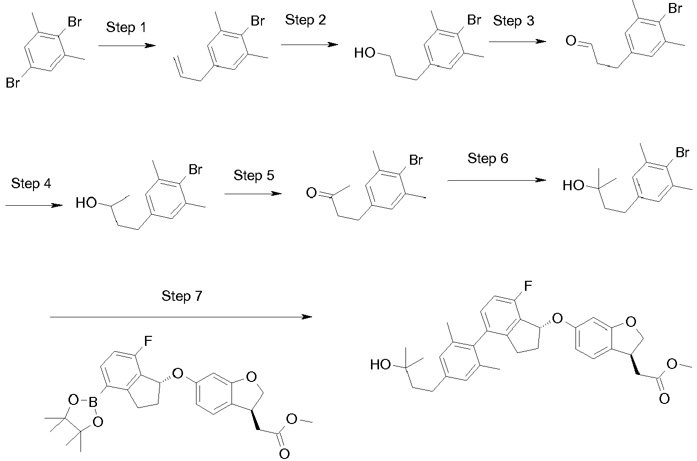

### Step 1: 5-Allyl-2-bromo-1,3-dimethylbenzene

To a solution of isopropylmagnesium chloride (6.15 mL of a 2 M solution in tetrahydrofurane) in tetrahydrofurane (75 mL) is added dropwise at 0°C n-butyllithium (15.4 mL of a 1.6 M solution in n-hexane). The mixture is stirred for 10 minutes, a solution of 2,5-dibromo-1,3-dimethylbenzene (5 g) in tetrahydrofurane (75 mL) is added within 10 minutes. The mixture is stirred for 2 hours, cooled to -40°C and then CuCNxLiCl (5.5 mL of a 1 M solution in tetrahydrofurane) is added dropwise. After stirring for 5 minutes 3-bromopropene (6.6 mL) is added dropwise, the mixture is stirred for 1 hour, warmed to room temperature and stirred for further 12 hours. Then the reaction is quenched by addition of saturated aqueous NH₄Cl solution. The mixture is extracted with diethylether, the organic phase is dried (MgSO₄) and concentrated. The residue is chromatographed on silica gel (dichloromethane) to give the title compound. Yield: 3.76 g; LC (method 8): t_{R} = 0.83 min; Mass spectrum (ESI⁺): m/z = 225 [M+H]⁺.

### Step 2: 3-(4-Bromo-3,5-dimethylphenyl)propan-1-ol

A solution of 5-allyl-2-bromo-1,3-dimethylbenzene (1.0 g) is cooled to 0°C, 9-borabicyclo[3.3.1]nonane (9-BBN, 26 mL of a 0.5 M solution in tetrahydrofurane) is added dropwise and the mixture is stirred for 2 hours at room temperature. After cooling to 0°C NaOH (6.7 mL, 4 M) and H₂O₂ (6.7 mL, 35%) are added dropwise. The mixture is stirred for 12 hours while warming to room temperature. Then the mixture is partitioned between brine and diethylether. The organic phase is dried (MgSO₄) and concentrated. The residue is chromatographed on silica gel (cyclohexane/ethyl acetate 95:5→70:30) to give the title compound. Yield: 750 mg; LC (method 8): t_{R} = 0.28 min; Mass spectrum (ESI⁺): m/z = 260 [M+NH₄]⁺.

### Step 3: 3-(4-Bromo-3,5-dimethylphenyl)propanal

To a solution of 3-(4-bromo-3,5-dimethylphenyl)propan-1-ol (250 mg) in dichloromethane (5 mL) is added at 0°C 1,1-dihydro-1,1,1-triacetoxy-1,2-benziodoxol-3(1H)-on (Dess-Martin periodinan, 2.7 mL of a 15% solution in dichloromethane). The mixture is stirred for 12 hours at room temperature, cooled to 0°C and 1,1-dihydro-1,1,1-triacetoxy-1,2-benziodoxol-3(1H)-on (Dess-Martin periodinan, 1.6 mL of a 15% solution in dichloromethane) is added. After stirring for 4 hours isopropanol (5 mL) is added, the mixture is stirred for 15 minutes and the solvents are evaporated in vacuo. The residue is chromatographed on silica gel (cyclohexane/ethyl acetate 99:1→70:30) to give the title compound. Yield: 120 mg; LC (method 8): t_{R} = 0.38 min; Mass spectrum (ESI⁻): m/z = 239 [M-H]⁻.

### Step 4: 4-(4-Bromo-3,5-dimethylphenyl)butan-2-ol

To a solution of 3-(4-bromo-3,5-dimethylphenyl)propanal (120 mg) in tetrahydrofurane (2 mL) is added at 0°C methylmagnesium bromide (800 µL of a 1.4 M solution in tetrahydrofurane/toluene 1:3). After stirring for 30 minutes the mixture is partitioned between saturated aqueous NH₄Cl solution and diethylether. The organic phase is dried (MgSO₄) and concentrated. The residue is chromatographed on silica gel (cyclohexane/ethyl acetate 99:1 →70:30) to give the title compound. Yield: 130 mg; LC (method 8): t_{R} = 0.37 min; Mass spectrum (ESI⁺): m/z = 274 [M+NH_{4]}⁺.

### Step 5: 4-(4-Bromo-3,5-dimethylphenyl)butan-2-one

To a solution of 4-(4-bromo-3,5-dimethylphenyl)butan-2-ol (130 mg) in dichloromethane (8 mL) is added at 0°C 1,1-dihydro-1,1,1-triacetoxy-1,2-benziodoxol-3(1H)-on (Dess-Martin periodinan, 1.4 mL of a 15% solution in dichloromethane). The mixture is stirred for 12 hours at room temperature. The solvents are evaporated in vacuo. The residue is chromatographed on silica gel (cyclohexane/ethyl acetate 99:1 →70:30) to give the title compound. Yield: 100 mg; LC (method 8): t_{R} = 0.44 min; Mass spectrum (ESI⁺): m/z = 255 [M+H]⁺.

### Step 6: 4-(4-Bromo-3,5-dimethylphenyl)-2-methylbutan-2-ol

To a solution of 4-(4-bromo-3,5-dimethylphenyl)butan-2-one (100 mg) in tetrahydrofurane (2 mL) is added at 0°C methylmagnesium bromide (840 µL of a 1.4 M solution in tetrahydrofurane/toluene 1:3). The mixture is stirred for 2 hours while warming to room temperature and then partitioned between saturated aqueous NH₄Cl solution and diethylether. The organic phase is dried (MgSO₄) and concentrated to give the title compound. Yield: 105 mg; LC (method 8): t_{R} = 0.47 min; Mass spectrum (ESI⁺): m/z = 271 [M+H]⁺.

### Step 7: Methyl 2-((S)-6-((R)-7-fluoro-4-(4-(3-hydroxy-3-methylbutyl)-2,6-dimethylphenyl)-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenyl)-2-methylbutan-2-ol following a procedure analogous to that described in Step 5 of Intermediate 12. LC (method 8): t_{R} = 0.83 min; Mass spectrum (ESI⁺): m/z = 533 [M+H]⁺.

### Intermediate 74

### 1-Oxa-6-thiaspiro[2.5]octane

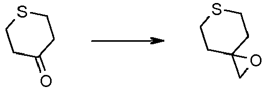

To a suspension of trimethylsulfoxonium iodide (2 g) in dimethylsulfoxide (15 mL) is added portionwise NaH (820 mg of a 60% dispersion in mineral oil). The mixture is stirred for 1 hour and a solution of dihydro-2H-thiopyran-4(3H)-one (2 g) in dimethylsulfoxide (2 mL) is added dropwise. The mixture is stirred for 12 hours, diluted with ethylacetate and washed twice with water and brine. The organic phase is dried (MgSO₄) and concentrated. The residue is chromatographed on silica gel (cyclohexane/ethyl acetate 99:1→70:30) to give the title compound. Yield: 742 mg; LC (method 7): t_{R} = 0.64 min; Mass spectrum (ESI⁺): m/z = 131 [M+H]⁺.

### Intermediate 75

### Methyl 2-((S)-6-((R)-7-Fluoro-4-(4-(1,1-dioxo-4-hydroxytetrahydro-2H-thiopyran-4-yl)methoxy)-2,6-dimethylphenyl)-2,3-dihydro-1H-inden-1-yloxy)-2,3-dihydrobenzofuran-3-yl)acetate

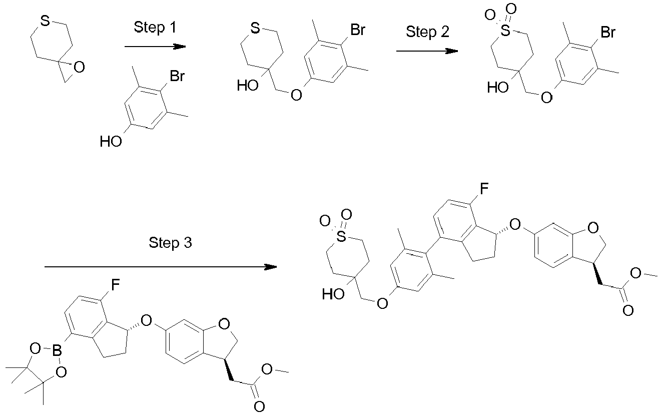

### Step 1: 4-((4-Bromo-3,5-dimethylphenoxy)methyl)tetrahydro-2H-thiopyran-4-ol

To a solution of 4-bromo-3,5-dimethylphenol (700 mg) in N,N-dimethylformamide (7 mL) is added Cs₂CO₃ (1.7 g) and 1-oxa-6-thiaspiro[2.5]octane (586 mg). The mixture is stirred for 12 hour at 100°C. The mixture is diluted with water and extracted twice with ethyl acetate. The combined organic phases are washed with water and brine, dried (MgSO₄) and concentrated. The residue is chromatographed on silica gel (cyclohexane/ethyl acetate 99:1→60:40) to give the title compound. Yield: 986 mg; LC (method 7): t_{R} = 1.14 min; Mass spectrum (ESI⁺): m/z = 348 [M+NH_{4]}⁺.

### Step 2: 1,1-Dioxo-4-((4-bromo-3,5-dimethylphenoxy)methyl)tetrahydro-2H-thiopyran-4-ol

To a solution of 4-((4-bromo-3,5-dimethylphenoxy)methyl)tetrahydro-2H-thiopyran-4-ol (175 mg) in dichloromethane (3 mL) is added at -10°C meta-chloro-perbenzoic acid (MCPBA, 270 mg, 70%). The mixture is stirred for 12 hour while warming to room temperature. The mixture is diluted with dichloromethane and washed with 1 M aqueous NaOH solution. The organic phase is dried (MgSO₄) and concentrated. The residue is chromatographed on silica gel (petrole ether/ethyl acetate 70:30→0:100) to give the title compound. Yield: 152 mg; Mass spectrum (ESI⁺): m/z = 380 [M+NH₄]⁺.

### Step 3: Methyl 2-((S)-6-((R)-7-fluoro-4-(4-((1,1-dioxo-4-hydroxytetrahydro-2H-thiopyran-4-yl)methoxy)-2,6-dimethylphenyl)-2,3-dihydro-1H-inden-1-yloxy)-2,3-dihydrobenzofuran-3-yl)acetate

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (190 mg), 1,1-dioxo-4-((4-bromo-3,5-dimethylphenoxy)methyl)tetrahydro-2H-thiopyran-4-ol (150 mg), K₃PO₄ (250 mg) and dicyclohexyl(2',6'-dimethoxybiphenyl-2-yl)phosphine (S-Phos) (21 mg) are suspended in N,N-dimethylformamide (2 mL) and water (100 µL). The mixture is purged for 10 minutes with argon. Palladium-(II)-acetate (6 mg) is added, the vial is sealed and the mixture is stirred at 110°C for 3 hours. After cooling to room temperature the mixture is partitioned between diethylether and saturated aqueous NH₄Cl solution. The organic phase is dried (MgSO₄) and concentrated. The residue is chromatographed on silica gel (petrole ether/ethyl acetate 50:50→0:100) to give the title compound. Yield: 90 mg; LC (method 8): t_{R} = 0.53 min; Mass spectrum (ESI⁺): m/z = 625 [M+H]⁺.

### Intermediate 76

### Methyl 2-((S)-6-((R)-4-(2,6-dimethyl-4-(3-(methylsulfonyl)lprolpyl)lphenyl)-7-fluoro-2,3-dihydro-1H-inden-1-yloxy)-2,3-dihydrobenzofuran-3-yl)acetate

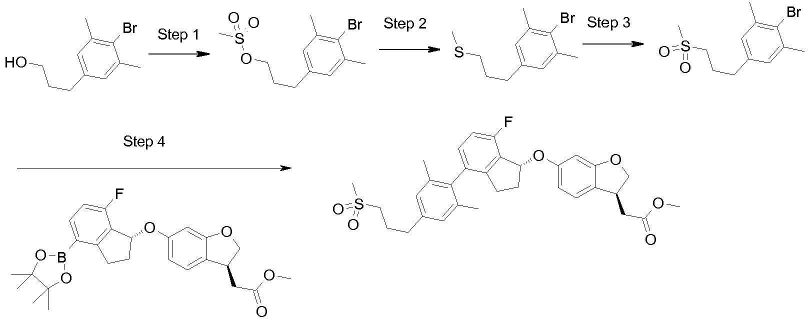

### Step 1: 3-(4-Bromo-3,5-dimethylphenyl)propyl methanesulfonate

To a cooled (0°C) solution of 3-(4-bromo-3,5-dimethylphenyl)propan-1-ol (250 mg) and triethylamine (160 µL) in dichloromethane (2 mL) is added methanesulfonyl chloride (850 µL). The mixture is stirred for 12 hours at room temperature. The mixture is partitioned between dichloromethane and saturated aqueous NaHCO₃ solution and stirred vigorously for 30 minutes. The organic phase is separated, washed with brine and dried (MgSO₄). The solvent is evaporated and the residue is chromatographed on silica gel (cyclohexane/ethyl acetate 80:20→50:50) to give the title compound. Yield: 235 mg; LC (method 8): t_{R} = 0.44 min; Mass spectrum (ESI⁺): m/z = 343 [M+Na]⁺.

### Step 2: (3-(4-Bromo-3,5-dimethylphenyl)propyl)(methyl)sulfane

NaSMe (65 mg) is added to a solution of 3-(4-bromo-3,5-dimethylphenyl)propyl methanesulfonate (230 mg) in N,N-dimethylformamide (5 mL). The mixture is stirred for 12 hours. NaSMe (25 mg) is added and the mixture is stirred for 1 hour. Then the mixture is partitioned between water and diethylether. The organic phase is dried (MgSO₄) and concentrated. The residue is chromatographed on silica gel (cyclohexane/ethyl acetate 99:1→80:20) to give the title compound. Yield: 145 mg; LC (method 8): t_{R} = 0.88 min.

### Step 3: 2-bromo-1,3-dimethyl-5-(3-(methylsulfonyl)propyl)benzene

The title compound is prepared from (3-(4-bromo-3,5-dimethylphenyl)propyl) (methyl)sulfane following a procedure analogous to that described in Step 2 of Intermediate 75. LC (method 7): t_{R} = 1.04 min; Mass spectrum (ESI⁺): m/z = 305 [M+H]⁺.

### Step 4: Methyl 2-((S)-6-((R)-4-(2,6-dimethyl-4-(3-(methylsulfonyl)propyl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetrame thyl-1,3,2-dioxaborolan-2-yl)-2,3-dihydro-1H-inden-1-yloxy)-2,3-dihydrobenzofuran-3-yl)acetate and 2-bromo-1,3-dimethyl-5-(3-(methylsulfonyl)propyl)benzene following a procedure analogous to that described in Step 5 of Intermediate 12. LC (method 8): t_{R} = 0.66 min; Mass spectrum (ESI⁺): m/z = 567 [M+H]⁺.

### Intermediate 77

### Methyl 2-((S)-6-((R)-4-(2,6-dimethyl-4-(2-(methylsulfonamido)ethoxy)phenyl)-7-fluoro-2,3-dihydro-1H-inden-1-yloxy)-2,3-dihydrobenzofuran-3-yl)acetate

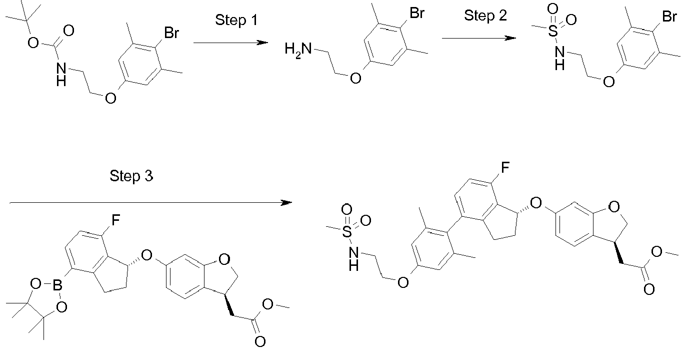

### Step 1: 2-(4-Bromo-3,5-dimethylphenoxy)ethanamine

To a solution of tert-butyl 2-(4-bromo-3,5-dimethylphenoxy)ethylcarbamate (310 mg) in dichloromethane (8 mL) is added trifluoroacetic acid (700 µL). The mixture is stirred for 12 hours at room temperature. Then saturated aqueous K₂CO₃ solution is added, the mixture is stirred for 30 minutes and the phases are separated. The organic phase is washed with saturated aqueous K₂CO₃ solution and dried (MgSO₄). The solvent is evaporated to give the title compound. Yield: 200 mg; LC (method 7): t_{R} = 0.83 min; Mass spectrum (ESI⁺): m/z = 244 [M+H]⁺.

### Step 2: N-(2-(4-bromo-3,5-dimethylphenoxy)ethyl)methanesulfonamide

To a cooled (0°C) solution of 2-(4-bromo-3,5-dimethylphenoxy)ethanamine (100 mg) and triethylamine (63 µL) in dichloromethane (2 mL) is added methanesulfonyl chloride (32 µL). The mixture is stirred for 2 hours at 0°C. Then the mixture is partitioned between dichloromethane and water. The organic phase is separated, washed with brine and dried (MgSO₄). The solvent is evaporated and the residue is purified by HPLC on reversed phase to give the title compound. Yield: 90 mg; LC (method 7): t_{R} = 1.01 min; Mass spectrum (ESI⁺): m/z = 322 [M+H]⁺.

### Step 3: Methyl 2-((S)-6-((R)-4-(2,6-dimethyl-4-(2-(methylsulfonamido)ethoxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo furan-3-yl)acetate and N-(2-(4-bromo-3,5-dimethylphenoxy)ethyl)methanesulfonami de following a procedure analogous to that described in Step 5 of Intermediate 12. LC (method 8): t_{R} = 0.59 min; Mass spectrum (ESI⁺): m/z = 584 [M+H]⁺.

### Intermediate 78

### Methyl 2-((S)-6-((R)-4-(4-(2-acetamidoethyl)-2,6-dimethylphenyl)-7-fluoro-2,3-dihydro-1H-inden-1-yloxy)-2,3-dihydrobenzofuran-3-yl)acetate

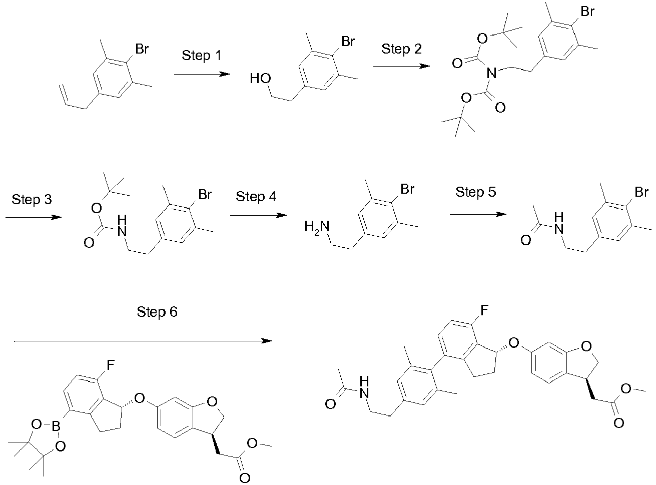

### Step 1: 2-(4-Bromo-3,5-dimethylphenyl)ethanol

O₃ is bubbled through a cooled (-78°C) solution of 5-allyl-2-bromo-1,3-dimethylbenzene (2 g) in dichloromethane (70 mL) until a light blue color is observed. Then O₂ is bubbled through the solution until the color disappears. Afterwards methanol (70 mL) and NaBH₄ (1.45 g) are added and the mixture is stirred for 12 hours while warming to room temperature. The mixture is then partitioned between 1 M aqueous HCl solution and diethylether. The organic phase is dried (MgSO₄) and concentrated. The residue is chromatographed on silica gel (cyclohexane/ethyl acetate 95:5→70:30) to give the title compound. Yield: 390 mg; LC (method 8): t_{R} = 0.22 min; Mass spectrum (ESI⁺): m/z = 211 [M+H-H₂O]⁺.

### Step 2: N,N-Di-tert.-butoxycarbonyl-2-(4-bromo-3,5-dimethylphenyl)ethanamine

A solution of di-tert.-butyl azodicarboxylate (2.85 g) in dichloromethane (10 mL) is added dropwise over 15 minutes to a solution of 2-(4-bromo-3,5-dimethylphenyl)ethanol (950 mg), di-tert.butyl-iminodicarboxylate (3.75 g) and tributylphosphine (4.4 mL) in dichloromethane (40 mL) at -10°C. The resulting solution is stirred for 2 hours while warming to rom temperature and then partitioned between saturated aqueous NaHCO₃ solution and dichloromethane. The aqueous phase is extracted with dichloromethane. The combined organic phases are dried (MgSO₄) and concentrated. The residue is chromatographed on silica gel (cyclohexane/ethyl acetate 99:1→70:30) to give the title compound. Yield: 1.19 g; LC (method 8): t_{R} = 1.06 min; Mass spectrum (ESI⁺): m/z = 451 [M+Na]⁺.

### Step 3: tert-Butyl 4-bromo-3,5-dimethylphenethylcarbamate

To a solution of N,N-di-tert.-butoxycarbonyl-2-(4-bromo-3,5-dimethylphenyl) ethanamine (1.19 g) in dichloromethane (25 mL) is added trifluoroacetic acid (320 µL). The mixture is stirred for 12 hours at room temperature. Then the mixture is partitioned between saturated aqueous NaHCO₃ solution and dichloromethane. The organic phase is dried (MgSO₄), the solvent is evaporated and the residue is chromatographed on silica gel (cyclohexane/ethyl acetate 99:1→70:30) to give the title compound. Yield: 550 mg; LC (method 8): t_{R} = 0.68 min; Mass spectrum (ESI⁺): m/z = 328 [M+H]⁺.

### Step 4: 2-(4-Bromo-3,5-dimethylphenyl)ethanamine

The title compound is prepared from tert-butyl 4-bromo-3,5-dimethylphenethylcarbamate following a procedure analogous to that described in Step 1 of Intermediate 77. LC (method 7): t_{R} = 0.83 min; Mass spectrum (ESI⁺): m/z = 228 [M+H]⁺.

### Step 5: N-(4-Bromo-3,5-dimethylphenethyl)acetamide

To a cooled (0°C) solution of 2-(4-bromo-3,5-dimethylphenyl)ethanamine (100 mg) and triethylamine (68 µL) in dichloromethane (3 mL) is added acetyl chloride (31 µL). The mixture is stirred for 2 hours at 0°C. Then the mixture is partitioned between dichloromethane and water. The organic phase is separated and dried (MgSO₄). The solvent is evaporated and the residue is purified by HPLC on reversed phase to give the title compound. Yield: 25 mg; LC (method 7): t_{R} = 0.98 min; Mass spectrum (ESI⁺): m/z = 270 [M+H]⁺.

### Step 6: Methyl 2-((S)-6-((R)-4-(4-(2-acetamidoethyl)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and N-(4-bromo-3,5-dimethylphenethyl)acetamide following a procedure analogous to that described in Step 5 of Intermediate 12. LC (method 8): t_{R} = 0.53 min; Mass spectrum (ESI⁺): m/z = 532 [M+H]⁺.

### Intermediate 79

### Methyl 2-((S)-6-((R)-4-(4-(2-acetamidoethoxy)-2,6-dimethylphenyl)-7-fluoro-2,3-dihydro-1H-inden-1-yloxy)-2,3-dihydrobenzofuran-3-yl)acetate

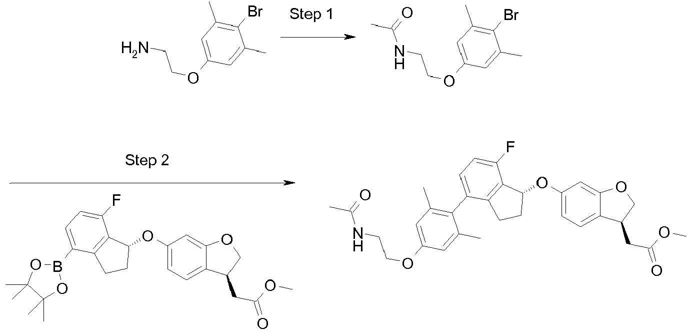

### Step 1: N-(2-(4-Bromo-3,5-dimethylphenoxy)ethyl)acetamide

The title compound is prepared from 2-(4-bromo-3,5-dimethylphenoxy)ethanamine following a procedure analogous to that described in Step 5 of Intermediate 78. LC (method 7): t_{R} = 0.97 min; Mass spectrum (ESI⁺): m/z = 286 [M+H]⁺.

### Step 2: Methyl 2-((S)-6-((R)-4-(4-(2-acetamidoethoxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo-furan-3-yl)acetate and N-(2-(4-bromo-3,5-dimethylphenoxy)ethyl)acetamide following a procedure analogous to that described in Step 5 of Intermediate 12. LC (method 8): t_{R} = 0.50 min; Mass spectrum (ESI⁺): m/z = 548 [M+H]⁺.

### Intermediate 80

### Methyl 2-((S)-6-((R)-4-(2,6-dimethyl-4-(2-(methylsulfonamido)ethyl)lphenyl)-7-fluoro-2,3-dihydro-1H-inden-1-yloxy)-2,3-dihydrobenzofuran-3-yl)acetate

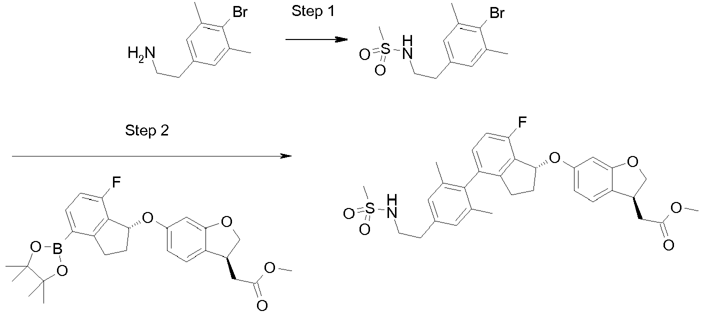

### Step 1: N-(4-Bromo-3,5-dimethylphenethyl)methanesulfonamide

The title compound is prepared from 2-(4-bromo-3,5-dimethylphenyl)ethanamine following a procedure analogous to that described in Step 2 of Intermediate 77. LC (method 7): t_{R} = 1.02 min; Mass spectrum (ESI⁺): m/z = 306 [M+H]⁺.

### Step 2: Methyl 2-((S)-6-((R)-4-(2,6-dimethyl-4-(2-(methylsulfonamido)ethyl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and N-(4-bromo-3,5-dimethylphenethyl)methanesulfonamide following a procedure analogous to that described in Step 5 of Intermediate 12. LC (method 8): t_{R} = 0.58 min; Mass spectrum (ESI⁺): m/z = 568 [M+H]⁺.

### Intermediate 81

### Methyl 2-((3S)-6-((1R)-4-(2,6-dimethyl-4-(1-methyl-2-oxopyrrolidin-3-yloxy)phenyl)-7-fluoro-2,3-dihydro-1H-inden-1-yloxy)-2,3-dihydrobenzofuran-3-yl)acetate

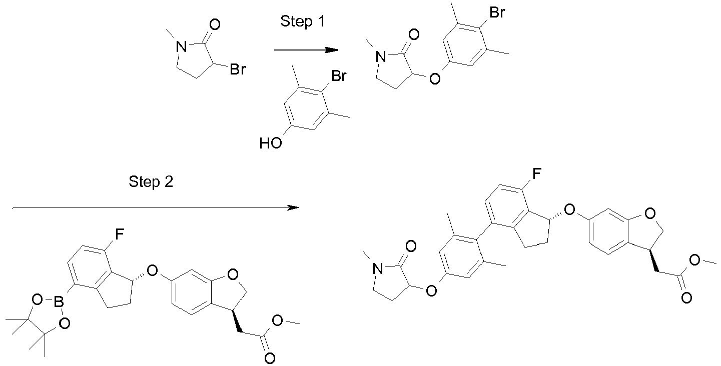

### Step 1: 3-(4-Bromo-3,5-dimethylphenoxy)-1-methylpyrrolidin-2-one

To a solution of 4-bromo-3,5-dimethylphenol (100 mg) in N,N-dimethylformamide (2 mL) is added 3-bromo-1-methylpyrrolidin-2-one (100 mg) and K₂CO₃ (105 mg). The mixture is stirred for 12 hours at room temperature. Then the mixture is partitioned between water and diethylether. The organic phase is dried (MgSO₄) and concentrated. The residue is chromatographed on silica gel (cyclohexane/ethyl acetate 80:20→50:50) to give the title compound. Yield: 65 mg; LC (method 7): t_{R} = 1.0 min; Mass spectrum (ESI⁺): m/z = 298 [M+H]⁺.

### Step 2: Methyl 2-((3S)-6-((1 R)-4-(2,6-dimethyl-4-(1-methyl-2-oxopyrrolidin-3-yloxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzo furan-3-yl)acetate and 3-(4-bromo-3,5-dimethylphenoxy)-1-methylpyrrolidin-2-one following a procedure analogous to that described in Step 5 of Intermediate 12. LC (method 8): t_{R} = 0.60 min; Mass spectrum (ESI⁺): m/z = 560 [M+H]⁺.

### Intermediate 82

### Methyl 2-((S)-6-((R)-4-(4-(2,2-dimethyl-3-(methylsulfonyl)propoxy)-2,6-dimethyliphenyl)-7-fluoro-2,3-dihydro-1H-inden-1-yloxy)-2,3-dihydrobenzofuran-3-yl)acetate

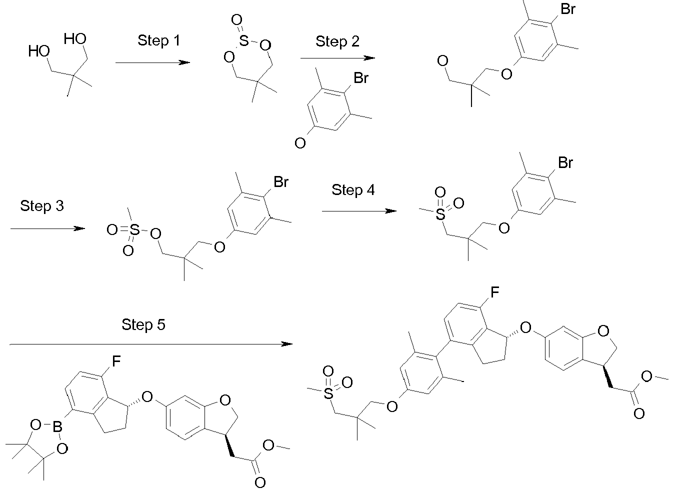

### Step 1: 5,5-Dimethyl-[1,3,2]dioxathiane-2-oxide

To a solution of 2,2-dimethyl-propane-1,3-diol (1 g) in dichloromethane (5 mL) is added dropwise at 0°C thionylchloride (735 µL). The mixture is stirred for 3 hours at 40°C and then partitioned between saturated aqueous NaHCO₃ solution and dichloromethane. The organic phase is washed with brine and dried (MgSO₄). The solvent is evaporated to give the title compound. Yield: 1 g; TLC: r_{f} = 0.65 (silicagel, cyclohexane/ethyl acetate 3:1).

### Step 2: 3-(4-Bromo-3,5-dimethylphenoxy)-2,2-dimethylpropan-1-ol

To a mixture of 4-bromo-3,5-dimethylphenol (625 mg) and Cs₂CO₃ (5 g) in N,N-dimethylformamide (10 mL) is added 5,5-dimethyl-[1,3,2]dioxathiane-2-oxide (467 mg). The mixture is stirred for 12 hours at 100°C and then partitioned between water and ethyl acetate. The organic phase is washed with brine and dried (MgSO₄). The solvent is evaporated and the residue is purified by HPLC on reversed phase to give the title compound. Yield: 251 mg; LC (method 7): t_{R} = 1.16 min; Mass spectrum (ESI⁺): m/z = 287 [M+H]⁺.

### Step 3: 3-(4-Bromo-3,5-dimethylphenoxy)-2,2-dimethylpropyl methanesulfonate

To a cooled (0°C) solution of 3-(4-bromo-3,5-dimethylphenoxy)-2,2-dimethylpropan-1-ol (251 mg) and triethylamine (160 µL) in dichloromethane (3 mL) is added methanesulfonyl chloride (75 µL). The mixture is stirred for 2 days while warming to room temperature. Then the mixture is diluted with dichloromethane, washed with saturated aqueous NaHCO₃ solution and dried (MgSO₄). The solvent is evaporated and the residue is chromatographed on silica gel (cyclohexane/ethyl acetate 99:1→50:50) to give the title compound. Yield: 271 mg; LC (method 7): t_{R} = 1.18 min; Mass spectrum (ESI⁺): m/z = 365 [M+H]⁺.

### Step 4: 2-Bromo-5-(2,2-dimethyl-3-(methylsulfonyl)propoxy)-1,3-dimethyl benzene

In a microwave vial sodium methanesulfinate (720 mg) is added to a solution of 3-(4-bromo-3,5-dimethylphenoxy)-2,2-dimethylpropyl methanesulfonate (271 mg) in N-methyl-2-pyrrolidinon (NMP) (8 mL). The mixture is heated to 180°C for 30 minutes. Then the mixture is diluted with diethylether, washed with water and brine and dried (MgSO₄). The solvent is evaporated and the residue is purified by HPLC on reversed phase to give the title compound. Yield: 32 mg; LC (method 8): t_{R} = 0.47 min; Mass spectrum (ESI⁺): m/z = 349 [M+H]⁺.

### Step 5: Methyl 2-((S)-6-((R)-4-(4-(2,2-dimethyl-3-(methylsulfonyl)propoxy)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 2-bromo-5-(2,2-dimethyl-3-(methylsulfonyl)propoxy)-1,3-dimethylbenzene following a procedure analogous to that described in Step 5 of Intermediate 12. LC (method 8): t_{R} = 0.81 min; Mass spectrum (ESI⁺): m/z = 611 [M+H]⁺.

### Intermediate 83

### 3-(4-Bromo-3,5-dimethylphenoxy)-1-methylcyclolpentanol(enantiomer 1 and enantiomer 2)

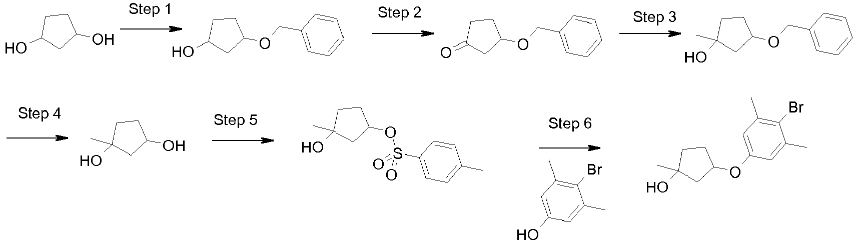

### Step 1: 3-(Benzyloxy)cyclopentanol

Under argon NaH (60% dispersion in mineral oil; 2.85 g) is added to a cold (0°C) solution of cyclopentane-1,3-diol (3.6 g) in N,N-dimethylformamide (40 mL). The mixture is stirred for 12 hours, followed by dropwise addition of (4.2 mL) and stirring for 12 hours at room temperature. Afterwards the mixture is partitioned between saturated aqueous NH₄Cl solution and diethylether. The organic phase is washed with water, dried (MgSO₄) and concentrated. The residue is chromatographed on silica gel (petrole ether/ethyl acetate 50:50→20:80) to give the title compound. Yield: 540 mg; LC (method 7): t_{R} = 0.85 min; Mass spectrum (ESI⁺): m/z = 193 [M+H]⁺.

### Step 2: 3-(Benzyloxy)cyclopentanone

To a solution of 3-(benzyloxy)cyclopentanol (530 mg) in dichloromethane (8 mL) is added at 0°C 1,1-dihydro-1,1,1-triacetoxy-1,2-benziodoxol-3(1H)-on (Dess-Martin periodinan, 8 mL of a 15% solution in dichloromethane). The mixture is stirred for 3 hours, diluted with dichloromethane, washed with 1 M aqueous NaOH solution and dried (MgSO₄). The solvents are evaporated in vacuo and the residue is chromatographed on silica gel (petrole ether/ethyl acetate 90:10→40:60) to give the title compound. Yield: 430 mg; LC (method 7): t_{R} = 0.90 min; Mass spectrum (ESI⁺): m/z = 191 [M+H]⁺.

### Step 3: 3-(Benzyloxy)-1-methylcyclopentanol

Methylmagnesium bromide (4.8 mL, 1.4 M solution in toluene/tetrahydrofurane 3:1) is added dropwise under argon to a solution of 3-(benzyloxy)cyclopentanone (420 mg) in tetrahydrofurane (10 mL) at -78°C. The mixture is stirred for 12 hours while warming to room temperature and partitioned between 1 M aqueous HCl solution and ethyl acetate. The aqueous phase is extracted with ethyl acetate. The combined organic phases are washed with brine and dried (MgSO₄). The solvents are evaporated in vacuo to give the title compound. Yield: 400 mg; LC (method 7): t_{R} = 0.93 min; Mass spectrum (ESI⁺): m/z = 229 [M+Na]⁺.

### Step 4: 1-Methylcyclopentane-1,3-diol

The title compound is prepared from 3-(benzyloxy)-1-methylcyclopentanol following a procedure analogous to that described in Step 4 of Intermediate 39. The mixture is hydrogenated for 12 hours. TLC: r_{f} = 0.18 (silicagel, petrole ether/ethyl acetate 1:1).

### Step 5: 3-Hydroxy-3-methylcyclopentyl 4-methylbenzenesulfonate

The title compound is prepared from 1-methylcyclopentane-1,3-diol following a procedure analogous to that described in Step 1 of Intermediate 46. TLC: r_{f} = 0.48 (silicagel, petrole ether/ethyl acetate 1:1).

### Step 6: 3-(4-Bromo-3,5-dimethylphenoxy)-1-methylcyclopentanol (enantiomer 1 and enantiomer 2)

To a mixture of 4-bromo-3,5-dimethylphenol (300 mg) and Cs₂CO₃ (600 mg) in N,N-dimethylformamide (3 mL) is added 3-hydroxy-3-methylcyclopentyl 4-methylbenzenesulfonate (275 mg). The mixture is stirred for 12 hours at 50°C and then partitioned between water and diethylether. The organic phase is dried (MgSO₄) and concentrated. The residue is chromatographed on silica gel (petrole ether/ethyl acetate 90:10→30:70). The enantiomers are separated by SFC on chiral phase (column: Daicel IA, 5 µm, 250 mm x 10 mm; eluent: scCO₂/(methanol+0.2% diethylamine) 85:15, 10 mL/min):
Enantiomer 1: t_{R} = 4.7 min; Yield: 31 mg; LC (method 8): t_{R} = 0.47 min; Mass spectrum (ESI⁺): m/z = 299 [M+H]⁺.
Enantiomer 2: t_{R} = 5.7 min; Yield: 42 mg; LC (method 8): t_{R} = 0.47 min; Mass spectrum (ESI⁺): m/z = 299 [M+H]⁺.

### Intermediate 84

### Methyl 2-((3S)-6-((1R)-7-fluoro-4-(4-(3-hydroxy-3-methylcyclopentyloxy)-2,6-dimethylphenyl)-2,3-dihydro-1H-inden-1-yloxy)-2,3-dihydrobenzofuran-3-yl)acetate (diastereomer 1)

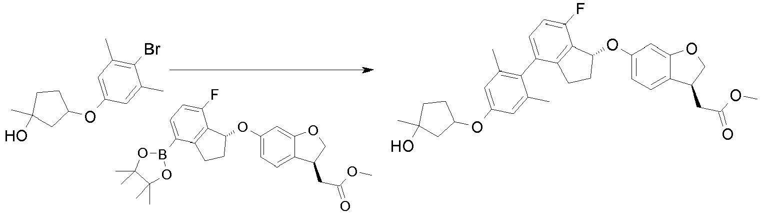

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-(4-bromo-3,5-dimethylphenoxy)-1-methylcyclopentanol (enantiomer 1) following a procedure analogous to that described in Step 5 of Intermediate 12. LC (method 8): t_{R} = 0.81 min.

### Intermediate 85

### Methyl 2-((3S)-6-((1R)-7-fluoro-4-(4-(3-hydroxy-3-methylcyclopentyloxy)-2,6-dimethyliphenyl)-2,3-dihydro-1H-inden-1-yloxy)-2,3-dihydrobenzofuran-3-yl)acetate (diastereomer 2)

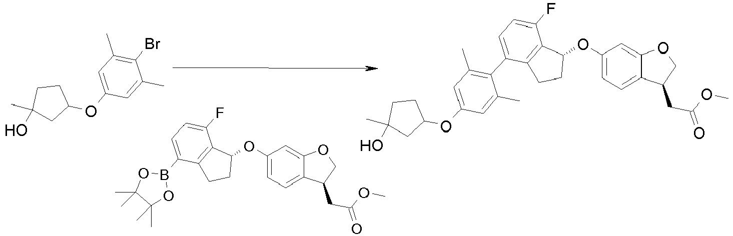

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 3-(4-bromo-3,5-dimethylphenoxy)-1-methylcyclopentanol (enantiomer 2) following a procedure analogous to that described in Step 5 of Intermediate 12. LC (method 8): t_{R} = 0.81 min.

### Intermediate 86

### Methyl 2-((3S)-6-(7-fluoro-4-(4-(3-hydroxy-3-methylbutoxy)-2,6-dimethyphenyl)-2,3-dihydro-1H-inden-1-yloxy)-2,3-dihydrobenzofuran-3-yl)acetate

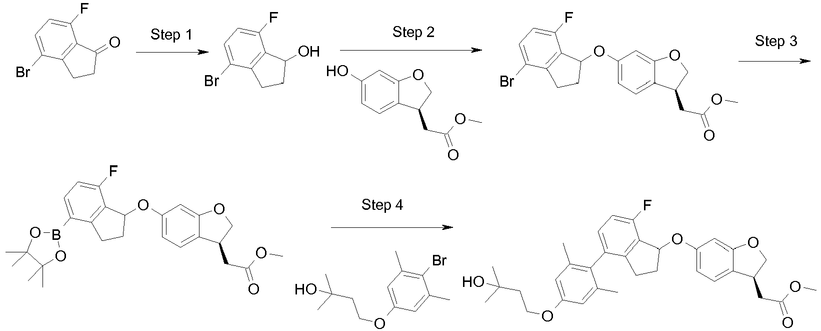

### Step 1: 4-Bromo-7-fluoro-2,3-dihydro-1H-inden-1-ol

NaBH₄ (60 mg) is added portionwise under argon to a solution of 4-bromo-7-fluoro-2,3-dihydro-1H-inden-1-one (500 mg) in methanol (5 mL) at 0°C. The mixture is stirred for 12 hours while warming to room temperature and partitioned between ice-water and ethyl acetate. 1 M aqueous HCl solution is added slowly under vigorous stirring. The phases are separated and the organic phase is washed with brine. After drying (MgSO₄) the solvents are evaporated in vacuo and the residue is chromatographed on silica gel (petrole ether/ethyl acetate 95:5→70:30) to give the title compound. Yield: 425 mg.

### Step 2: Methyl 2-((3S)-6-(4-bromo-7-fluoro-2,3-dihydro-1H-inden-1-yloxy)-2,3-dihydrobenzofuran-3-yl)acetate

The title compound is prepared from 4-bromo-7-fluoro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. Mass spectrum (ESI⁺): m/z = 421 [M+H]⁺.

### Step 3: Methyl 2-((3S)-6-(7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate

The title compound is prepared from methyl 2-((3S)-6-(4-bromo-7-fluoro-2,3-dihydro-1 H-inden-1-yloxy)-2,3-dihydrobenzofuran-3-yl)acetate following a procedure analogous to that described in Step 4 of Intermediate 12. Mass spectrum (ESI⁺): m/z = 469 [M+H]⁺.

### Step 4: Methyl 2-((3S)-6-(7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate

The title compound is prepared from methyl 2-((3S)-6-(7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate and 4-(4-bromo-3,5-dimethylphenoxy)-2-methylbutan-2-ol following a procedure analogous to that described in Step 5 of Intermediate 12. LC (method 8): t_{R} = 0.74 min / 0.78 min (mixture of diastereomers); Mass spectrum (ESI⁺): m/z = 571 [M+Na]⁺.

### Intermediate 87

### Methyl 2-((S)-6-((R)-5-cyano-4-(2,6-dimethyl-4-((1-methyl-2-oxo-1,2-dihydropyridin-4-yl)methoxy)phenyl)-2,3-dihydro-1H-inden-1-yloxy)-2,3-dihydrobenzofuran-3-yl)acetate

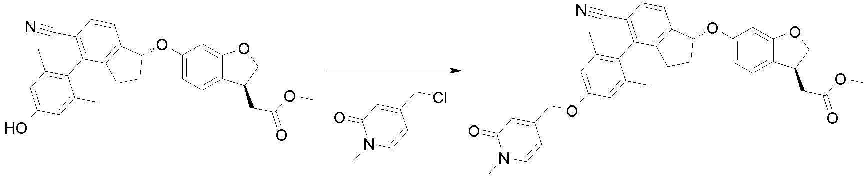

To a mixture of methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (40 mg) and K₂CO₃ (15 mg) in N,N-dimethylformamide (2 mL) is added 4-(chloromethyl)-1-methylpyridin-2(1 H)-one (15 mg). The mixture is stirred for 12 hours at 80°C and then partitioned between water and diethylether. The organic phase is dried (MgSO₄) and concentrated. The residue is purified by HPLC on reversed phase to give the title compound. Yield: 44 mg; LC (method 9): t_{R} = 1.11 min; Mass spectrum (ESI⁺): m/z = 591 [M+H]⁺.

### Intermediate 88

### Methyl 2-((S)-6-((R)-5-cyano-4-(2,6-dimethyl-4-((tetrahydro-2H-pyran-4-yl)methoxy)phenyl)-2,3-dihydro-1H-inden-1-yloxy)-2,3-dihydrobenzofuran-3-yl)acetate

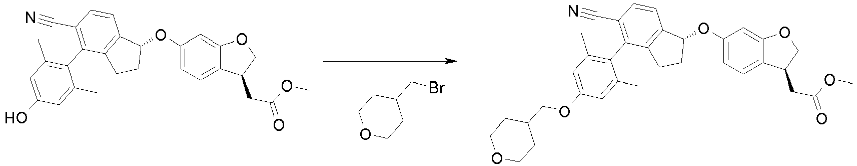

To a mixture of methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (40 mg) and K₂CO₃ (15 mg) in N,N-dimethylformamide (2 mL) is added 4-(bromomethyl)tetrahydro-2H-pyran (17 mg). The mixture is stirred for 12 hours at 80°C. K₂CO₃ (15 mg) and 4-(bromomethyl)tetrahydro-2H-pyran (17 mg) are added and the mixture is stirred for 6 hours at 80°C. Again K₂CO₃ (15 mg) and 4-(bromomethyl)tetrahydro-2H-pyran (17 mg) are added and the mixture is stirred for 12 hours at 80°C. Then the mixture is partitioned between water and diethylether. The organic phase is dried (MgSO₄) and concentrated. The residue is purified by HPLC on reversed phase to give the title compound. Yield: 35 mg; LC (method 9): t_{R} = 1.23 min; Mass spectrum (ESI⁺): m/z = 568 [M+H]⁺.

### Intermediate 89

### 4-(4-(3,6-dihydro-2H-pyran-4-yl)-2,6-dimethyliphenyl)-7-fluoro-2,3-dihydro-1H-inden-1-one and 4-(4-(7-fluoro-1-oxo-2,3-dihydro-1H-inden-4-yl)-3,5-dimethyphenyl)-5,6-dihydro-2H-pyran-2-one

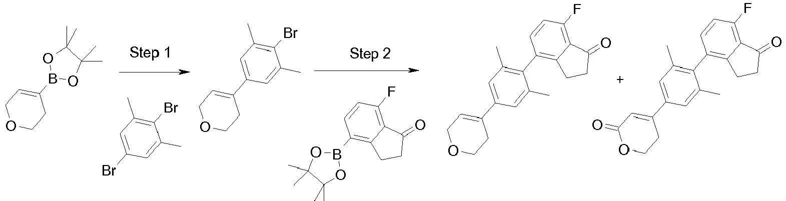

### Step 1: 4-(4-Bromo-3,5-dimethylphenyl)-3,6-dihydro-2H-pyran

In a microwave vial 2,5-dibromo-1,3-dimethylbenzene (800 mg), 2-(3,6-dihydro-2H-pyran-4-yl)-4,4,5,5-tetramethyl-1,3,2-dioxaborolane (640 mg) and K₃PO₄ (1.3 g) are suspended in toluene (12 mL) and water (1.2 mL) and purged for 10 minutes with argon. [1,1'-Bis(diphenylphosphino)-ferrocene]-dichloropalladium-(II) (100 mg) is added, the vial is sealed and the mixture is stirred at 60°C for 12 hours. After cooling to room temperature the mixture is diluted with diethylether and washed with saturated aqueous NH₄Cl solution. The organic phase is dried (MgSO₄) and concentrated. The residue is chromatographed on silica gel (cyclohexane/ethyl acetate 99:1→60:40) to give the title compound. Yield: 404 mg.

### Step 2: 4-(4-(3,6-dihydro-2H-pyran-4-yl)-2,6-dimethylphenyl)-7-fluoro-2,3-dihydro-1H-inden-1-one and 4-(4-(7-fluoro-1-oxo-2,3-dihydro-1H-inden-4-yl)-3,5-dimethylphenyl)-5,6-dihydro-2H-pyran-2-one

In a microwave vial 7-fluoro-4-(4,4,5,5-tetramethyl-1,3,2-dioxaborolan-2-yl)-2,3-dihydro-1H-inden-1-one (200 mg), 4-(4-bromo-3,5-dimethylphenyl)-3,6-dihydro-2H-pyran (200 mg), K₃PO₄ (310 mg) and dicyclohexyl(2',6'-dimethoxybiphenyl-2-yl)phosphine (S-Phos) (25 mg) are suspended in toluene (2 mL), 1,4-dioxane (2 mL) and water (250 µL) and purged for 10 minutes with argon. Palladium-(II)-acetate (7 mg) is added, the vial is sealed and the mixture is stirred at 110°C for 3 hours. After cooling to room temperature the mixture is partitioned between diethylether and saturated aqueous NH₄Cl solution. The organic phase is dried (MgSO₄) and concentrated. The residue is chromatographed on silica gel (petrole ether/ethyl acetate 90:10→50:50) to give the title compounds.
4-(4-(3,6-dihydro-2H-pyran-4-yl)-2,6-dimethylphenyl)-7-fluoro-2,3-dihydro-1H-inden-1-one: Yield: 100 mg; LC (method 7): t_{R} = 1.10 min; Mass spectrum (ESI⁺): m/z = 337 [M+H]⁺.
4-(4-(7-fluoro-1-oxo-2,3-dihydro-1H-inden-4-yl)-3,5-dimethylphenyl)-5,6-dihydro-2H-pyran-2-one: Yield: 48 mg; LC (method 7): t_{R} = 0.99 min; Mass spectrum (ESI⁺): m/z = 351 [M+H]⁺.

### Intermediate 90

### Methyl 2-((S)-6-((R)-4-(2,6-dimethyl-4-(tetrahydro-2H-pyran-4-yl)lphenyl)-7-fluoro-2,3-dihydro-1H-inden-1-yloxy)-2,3-dihydrobenzofuran-3-yl)acetate

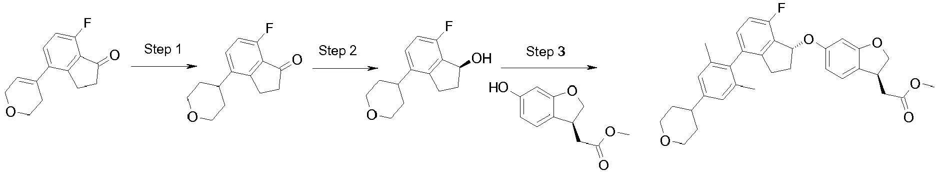

### Step 1: 7-Fluoro-4-(tetrahydro-2H-pyran-4-yl)-2,3-dihydro-1H-inden-1-one

To a solution of 4-(3,6-dihydro-2H-pyran-4-yl)-7-fluoro-2,3-dihydro-1H-inden-1-one (100 mg) in tetrahydrofurane (10 mL) is added 10% palladium on activated carbon (30 mg) and the mixture is hydrogenated at a pressure of 2 bar for 12 hours. The catalyst is filtered off and washed with methanol. The combined mother liquors are concentrated and the residue is chromatographed on silica gel (petrole ether/ethyl acetate 80:20→50:50) to give the title compound. Yield: 55 mg; LC (method 8): t_{R} = 0.36 min; Mass spectrum (ESI⁺): m/z = 339 [M+H]⁺.

### Step 2: (S)-7-Fluoro-4-(tetrahydro-2H-pyran-4-yl)-2,3-dihydro-1H-inden-1-ol

The title compound is prepared from 7-fluoro-4-(tetrahydro-2H-pyran-4-yl)-2,3-dihydro-1H-inden-1-one following a procedure analogous to that described in Step 1 of Intermediate 1. Mass spectrum (ESI⁺): m/z = 363 [M+Na]⁺.

### Step 3: Methyl 2-((S)-6-((R)-4-(2,6-dimethyl-4-(tetrahydro-2H-pyran-4-yl)phenyl)-7-fluoro-2,3-dihydro-1 H-inden-1-yloxy)-2,3-dihydrobenzofuran-3-yl)acetate

The title compound is prepared from (S)-7-fluoro-4-(tetrahydro-2H-pyran-4-yl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 26): t_{R} = 0.95 min; Mass spectrum (ESI⁺): m/z = 531 [M+H]⁺.

### Intermediate 91

### Methyl 2-((S)-6-((R)-4-(2,6-dimethyl-4-(6-oxo-3,6-dihydro-2H-pyran-4-yl)phenyl)-7-fluoro-2,3-dihydro-1H-inden-1-yloxy)-2,3-dihydrobenzofuran-3-yl)acetate

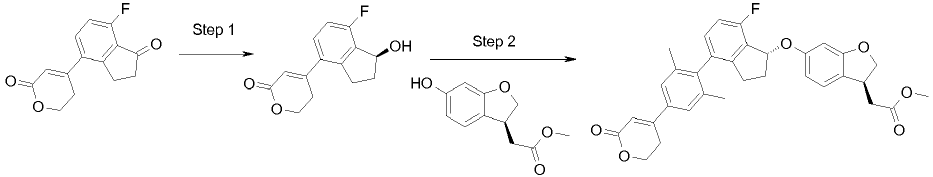

### Step 1: (S)-4-(7-Fluoro-1-hydroxy-2,3-dihydro-1H-inden-4-yl)-5,6-dihydro-2H-pyran-2-one

The title compound is prepared from 4-(7-fluoro-1-oxo-2,3-dihydro-1H-inden-4-yl)-5,6-dihydro-2H-pyran-2-one following a procedure analogous to that described in Step 1 of Intermediate 1. LC (method 7): t_{R} = 0.97 min; Mass spectrum (ESI⁺): m/z = 353 [M+H]⁺.

### Step 2: Methyl 2-((S)-6-((R)-4-(2,6-dimethyl-4-(6-oxo-3,6-dihydro-2H-pyran-4-yl)phenyl)-7-fluoro-2,3-dihydro-1H-inden-1-yloxy)-2,3-dihydrobenzofuran-3-yl)acetate

The title compound is prepared from (S)-4-(7-fluoro-1-hydroxy-2,3-dihydro-1H-inden-4-yl)-5,6-dihydro-2H-pyran-2-one and (S)-methyl 2-(6-hydroxy-2,3-dihydrobenzo furan-3-yl)acetate following a procedure analogous to that described in Step 3 of Intermediate 12. LC (method 7): t_{R} = 1.18 min; Mass spectrum (ESI⁺): m/z = 543 [M+H]⁺.

### Intermediate 92

### Methyl 2-((S)-6-((R)-5-cyano-4-(2,6-dimethyl-4-(pyrimidin-5-yl)phenyl)-2,3-dihydro-1H-inden-1-yloxy)-2,3-dihydrobenzofuran-3-yl)acetate

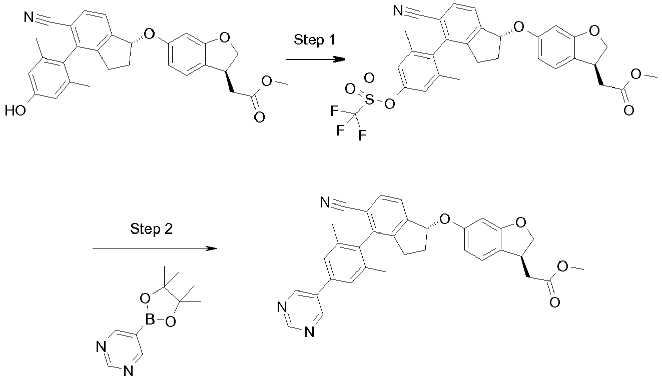

### Step 1: Methyl 2-((S)-6-((R)-5-cyano-4-(2,6-dimethyl-4-(trifluoromethylsulfonyloxy)phenyl)-2,3-dihydro-1H-inden-1-yloxy)-2,3-dihydrobenzofuran-3-yl)acetate

Methyl 2-((S)-6-((R)-5-cyano-4-(4-hydroxy-2,6-dimethylphenyl)-2,3-dihydro-1H-inden-1-yloxy)-2,3-dihydrobenzofuran-3-yl)acetate (170 mg) and 2,6-dimethylpyridine (lutidine) (85 µL) are dissolved in dichloromethane (2 mL) cooled to 0°C and treated dropwise with trifluoromethanesulfonic anhydride (80 µL). The mixture is stirred for 4 hours at room temperature, cooled to 0°C, 2,6-dimethylpyridine (lutidine) (85 µL) and trifluoromethanesulfonic anhydride (80 µL) are added and the mixture is stirred for 12 hours while warming to room temperature. After cooling to 0°C 2,6-dimethylpyridine (lutidine) (85 µL) and trifluoromethanesulfonic anhydride (80 µL) are added and the mixture is stirred for 4 hours while warming to room temperature. The mixture is diluted with diethylether and washed with saturated aqueous NH₄Cl solution and brine. After drying (MgSO₄) the solvents are evaporated in vacuo and the residue is chromatograped on silica gel (cyclohexane/ethyl acetate 90:10→60:40) to give the title compound. Yield: 225 mg; LC (method 9): t_{R} = 1.25 min, Mass spectrum (ESI⁺): m/z = 602 [M+H]⁺.

### Step 2: Methyl 2-((S)-6-((R)-5-cyano-4-(2,6-dimethyl-4-(pyrimidin-5-yl)phenyl)-2,3-dihydro-1H-inden-1-yloxy)-2,3-dihydrobenzofuran-3-yl)acetate

In a microwave vial methyl 2-((S)-6-((R)-5-cyano-4-(2,6-dimethyl-4-(trifluoromethylsulfonyloxy)phenyl)-2,3-dihydro-1H-inden-1-yloxy)-2,3-dihydrobenzofuran-3-yl)acetate (50 mg), 5-(4,4,5,5-tetramethyl-1,3,2-dioxaborolan-2-yl)pyrimidine (23 mg) and K₃PO₄ (40 mg) are suspended in tetrahydrofurane (2 mL). The mixture is purged for 10 minutes with argon. [1,1'-Bis(diphenylphosphino)-ferrocene]-dichloropalladium dichloromethane complex (3 mg) is added, the vial is sealed and the mixture is stirred at 70°C for 12 hours. After cooling to room temperature the mixture is diluted with diethylether and washed with saturated aqueous NH₄Cl solution. The organic phase is dried (MgSO₄) and concentrated. The residue is purified by HPLC on reversed phase to give the title compound. Yield: 27 mg; LC (method 9): t_{R} = 1.13 min; Mass spectrum (ESI⁺): m/z = 532 [M+H]⁺.

### Intermediate 93

### Methyl 2-((S)-6-((R)-5-cyano-4-(2,6-dimethyl-4-(1-methyl-2-oxo-1,2-dihydropyridin-4-yl)phenyl)-2,3-dihydro-1H-inden-1-yloxy)-2,3-dihydrobenzofuran-3-yl)acetate

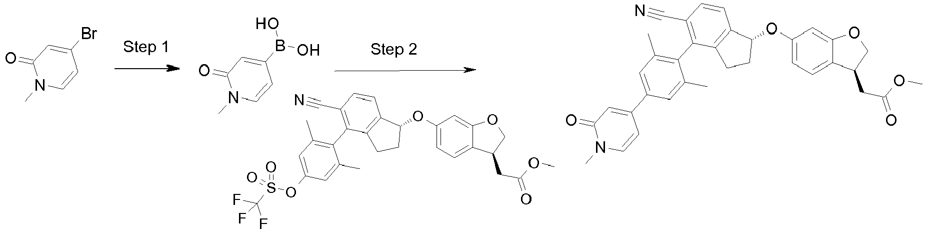

### Step 1: 1-Methyl-2-oxo-1,2-dihydropyridin-4-ylboronic acid

A solution of 4-bromo-1-methylpyridin-2(1 H)-one (8 g) in tetrahydrofurane (100 mL) is added dropwise to a cold (-18°C) solution of isopropylmagnesium chloride in tetrahydrofurane (32 mL of a 2 M solution). The mixture is stirred for 1 hour at -10°C and then trimethylborate (8.1 mL) is added dropwise. Afterwards the mixture is stirred for 12 hours while warming to room temperature. The mixture is cooled to 0°C, hydrochloric acid (19 mL, 4 N) is added and the mixture is diluted with with ethyl acetate. After stirring for 12 hours the organic phase is decanted. Ethyl acetate (50 mL) and water (50 mL) is added to the residue followed by dichloromethane (250 mL). The mixture is stirred for 5 minutes and the precipitate formed is isolated by filtration. The solid material is triturated with diisopropylether, filtered and dried in vacuo. Yield: 4.6 g; Mass spectrum (ESI⁺): m/z = 154 [M+H]⁺.

### Step 2: Methyl 2-((S)-6-((R)-5-cyano-4-(2,6-dimethyl-4-(1-methyl-2-oxo-1,2-dihydroyridin-4-yl)phenyl)-2,3-dihydro-1H-inden-1-yloxy)-2,3-dihydrobenzofuran-3-yl)acetate

In a microwave vial methyl 2-((S)-6-((R)-5-cyano-4-(2,6-dimethyl-4-(trifluoromethylsulfonyloxy)phenyl)-2,3-dihydro-1H-inden-1-yloxy)-2,3-dihydrobenzofuran-3-yl)acetate (50 mg), 1-methyl-2-oxo-1,2-dihydropyridin-4-ylboronic acid (17 mg) are mixed with aqueous Na₂CO₃ solution (125 µL, 2 M tetrahydrofurane (2 mL) and N,N-dimethylformamide (2mL). The mixture is purged for 5 minutes with argon. [1,1'-Bis(diphenylphosphino)-ferrocene]-dichloropalladium dichloromethane complex (3 mg) is added, the vial is sealed and the mixture is stirred at 70°C for 2 hours. After cooling to room temperature the mixture is diluted with diethylether and washed with saturated aqueous NH₄Cl solution. The organic phase is dried (MgSO₄) and concentrated. The residue is purified by HPLC on reversed phase to give the title compound. Yield: 32 mg; LC (method 9): t_{R} = 1.09 min; Mass spectrum (ESI⁺): m/z = 561 [M+H]⁺.

### Intermediate 94

### Methyl 2-((S)-6-((R)-5-chloro-4-(2,6-dimethyl-4-((3-methyloxetan-3-yl)methoxy)phenyl)-2,3-dihydro-1H-inden-1-yloxy)-2,3-dihydrobenzofuran-3yl)acetate

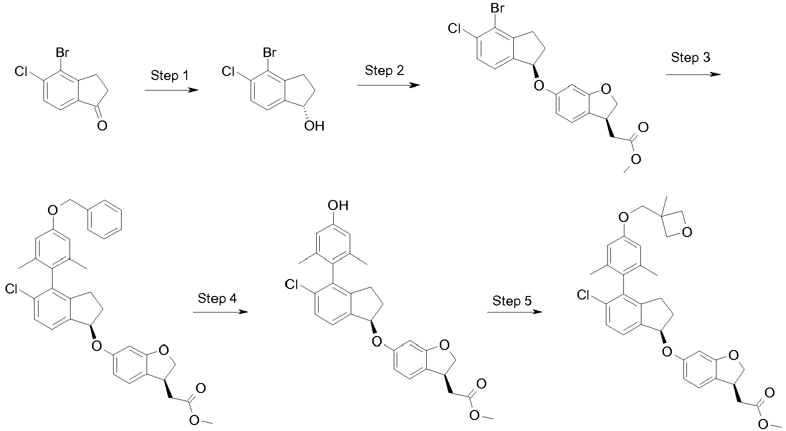

### Step 1: (S)-4-Bromo-5-chloro-2,3-dihydro-1 H-inden-1-ol

The title compound is prepared from 4-bromo-5-chloro-2,3-dihydro-1H-inden-1-one following a procedure analogous to that described in Step 1 of Intermediate 1. LC (method 7): t_{R} = 0.96 min; Mass spectrum (ESI⁻): m/z = 244, 246, 248 [M-H]⁻.

### Step 2: Methyl 2-((S)-6-((R)-4-bromo-5-chloro-2,3-dihydro-1 H-inden-1-yloxy)-2,3-dihydrobenzofuran-3-yl)acetate

The title compound is prepared from (S)-4-bromo-5-chloro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 8): t_{R} = 0.84 min; Mass spectrum (ESI⁺): m/z = 437, 439, 441 [M+H]⁺.

Step 3: Methyl 2-((S)-6-((R)-4-(4-(benzyloxy)-2,6-dimethylphenyl)-5-chloro-2,3-dihydro-1H-inden-1-yloxy)-2,3-dihydrobenzofuran-3-yl)acetateThe title compound is prepared from methyl 2-((S)-6-((R)-4-bromo-5-chloro-2,3-dihydro-1H-inden-1-yloxy)-2,3-dihydrobenzofuran-3-yl)acetate and 4-(benzyloxy)-2,6-dimethyl phenylboronic acid following a procedure analogous to that described in Step 2 of Intermediate 39. LC (method 8): t_{R} = 1.08 min; Mass spectrum (ESI⁺): m/z = 569 [M+H]⁺.

### Step 4: Methyl 2-((S)-6-((R)-5-chloro-4-(4-hydroxy-2,6-dimethylphenyl)-2,3-dihydro-1 H-inden-1-yloxy)-2,3-dihydrobenzofuran-3-yl)acetate

The title compound is prepared from methyl 2-((S)-6-((R)-4-(4-(benzyloxy)-2,6-dimethylphenyl)-5-chloro-2,3-dihydro-1H-inden-1-yloxy)-2,3-dihydrobenzofuran-3-yl)acetate by hydrogenation in the presence of Raney nickel in tetrahydrofuran at room temperature. LC (method 8): t_{R} = 0.63 min; Mass spectrum (ESI⁻): m/z = 477, 479 [M-H]⁻.

### Step 5: Methyl 2-((S)-6-((R)-5-chloro-4-(2,6-dimethyl-4-((3-methyloxetan-3-yl)methoxy)phenyl)-2,3-dihydro-1H-inden-1-yloxy)-2,3-dihydrobenzofuran-3-yl)acetate

The title compound is prepared from methyl 2-((S)-6-((R)-5-chloro-4-(4-hydroxy-2,6-dimethylphenyl)-2,3-dihydro-1H-inden-1-yloxy)-2,3-dihydrobenzofuran-3-yl)acetate and 3-(bromomethyl)-3-methyloxetane following a procedure analogous to that described in Step 5 of Intermediate 39. LC (method 8): t_{R} = 0.93 min; Mass spectrum (ESI⁺): m/z = 563, 565 [M+H]⁺.

### Intermediate 95

### Methyl 2-((S)-6-((R)-4-(2,6-bis(methoxymethyl)-4-((3-methyloxetan-3-yl)methoxy)phenyl)-7-fluoro-2,3-dihydro-1H-inden-1-yloxy)-2,3-dihydrobenzofuran-3-yl)acetate

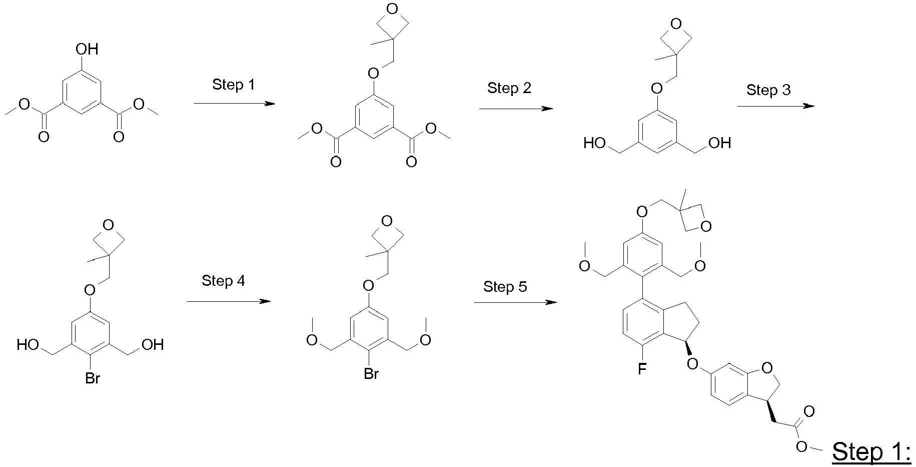

### Dimethyl 5-((3-methyloxetan-3-yl)methoxy)isophthalate

A mixture of dimethyl 5-hydroxyisophthalate (500 mg), (3-methyloxetan-3-yl)methyl 4-methylbenzenesulfonate (915 mg), and potassium carbonte (850 mg) in N,N-dimethylformamide (3 mL) is stirred at 50 °C for 5 h. The reaction mixture is diluted with water and the precipitate is filtered off, washed with water, and dried to give the title compound. LC (method 7): t_{R} = 0.97 min; Mass spectrum (ESI⁺): m/z = 295 [M+H]⁺.

### Step 2: (5-((3-Methyloxetan-3-yl)methoxy)-1,3-phenylene)dimethanol

The title compound is prepared from dimethyl 5-((3-methyloxetan-3-yl)methoxy)isophthalate by reduction with lithium aluminum hydride in tetrahydrofuran. LC (method 7): t_{R} = 0.69 min; Mass spectrum (ESI⁺): m/z = 239 [M+H]⁺.

### Step 3: (2-Bromo-5-((3-methyloxetan-3-yl)methoxy)-1,3-phenylene)dimethanol

NBS (212 mg) is added to ((5-((3-Methyloxetan-3-yl)methoxy)-1,3-phenylene)dimethanol (270 mg) in acetonitrile (5 mL) and the resulting mixture is stirred at room temperature for 3 h. The solvent is evaporated *in vacuo* and the residue is chromatographed on silica gel (cyclohexane/ethyl acetate 50:50→0:100) to give the title compound. LC (method 7): t_{R} = 0.76 min; Mass spectrum (ESI⁻): m/z = 315 [M-H]⁻.

### Step 4: 3-((4-Bromo-3,5-bis(methoxymethyl)phenoxy)methyl)-3-methyloxetane

Sodium hydride (50 % in mineral oil; 150 mg) is to (2-bromo-5-((3-methyloxetan-3-yl)methoxy)-1,3-phenylene)dimethanol (370 mg) in tetrahydrofuran (10 mL ander an argon atmosphere. The resulting mixture is stirred for 20 min at room temperature prior to the addition of methyl iodide (546 mg). The reaction mixture is stirred for 3 h at room temperature. More sodium hydride (50 % in mineral oil; 110 mg) and methyl iodide (200 µL) are added and the mixture is stirred over night at room temperature. The reaction mixture is quenched with ice water and extracted with ethyl acetate. The combined extracts are washed with water, dried over MgSO₄ and concentrated *in vacuo.* The residue is chromatographed on silica gel (cyclohexane/ethyl acetate 90:10→60:40) to give the title compound. LC (method 7): t_{R} = 1.05 min; Mass spectrum (ESI⁺): m/z = 362 [M+NH₄]⁺.

### Step 5: Methyl 2-((S)-6-((R)-4-(2,6-bis(methoxymethyl)-4-((3-methyloxetan-3-yl)methoxy)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 3-((4-bromo-3,5-bis(methoxymethyl)phenoxy)methyl)-3-methyloxetane following a procedure analogous to that described in Step 5 of Intermediate 12. LC (method 8): t_{R} = 0.72 min; Mass spectrum (ESI⁺): m/z = 607 [M+H]⁺.

### Intermediate 96

### Methyl 2-((S)-6-((R)-7-fluoro-4-(4-(1-(2-hydroxy-2-methylpropyl)-1H-pyrazol-4-yl)-2,6-dimethylphenyl)-2,3-dihydro-1H-inden-1-yloxy)-2,3-dihydrobenzofuran-3-yl)acetate

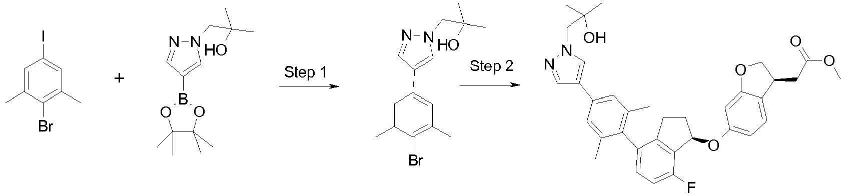

### Step 1: 1-(4-(4-Bromo-3,5-dimethylphenyl)-1H-pyrazol-1-yl)-2-methylpropan-2-ol

A mixture of 2-bromo-5-iodo-1,3-dimethylbenzene (1.00 g), 2-methyl-1-(4-(4,4,5,5-tetramethyl-1,3,2-dioxaborolan-2-yl)-1H-pyrazol-1-yl)propan-2-ol (1.11 g), and 2 M aqueous Na₂CO₃ solution (4.0 mL) in N,N-dimethylformamide is purged with argon for 3 min. [1,1'-Bis(diphenylphosphino)-ferrocene]-dichloropalladium dichloromethane complex (85 mg) is added and the mixture is stirred at 60 °C for 3 h. After cooling to room temperature the mixture is diluted with water and ethyl acetate. The organic phase is washed with brine, dried over MgSO₄ and concentrated *in vacuo.* The residue is purified by HPLC on reversed phase to give the title compound. LC (method 11): t_{R} = 1.12 min; Mass spectrum (ESI⁺): m/z = 323, 325 [M+H]⁺.

### Step 2: Methyl 2-((S)-6-((R)-7-fluoro-4-(4-(1-(2-hydroxy-2-methylpropyl)-1H-pyrazol-4-yl)-2,6-dimethylphenyl)-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 1-(4-(4-bromo-3,5-dimethylphenyl)-1H-pyrazol-1-yl)-2-methylpropan-2-ol following a procedure analogous to that described in Step 5 of Intermediate 12. LC (method 11): t_{R} = 1.23 min; Mass spectrum (ESI⁺): m/z = 585 [M+H]⁺.

### Intermediate 97

### Methyl 2-((S)-6-((R)-4-(2,6-dimethyl-4-(1-methyl-2-oxo-1,2-dihydropyridin-4-yl)phenyl)-7-fluoro-2,3-dihydro-1H-inden-1-yloxy)-2,3-dihydrobenzofuran-3-yl)acetate

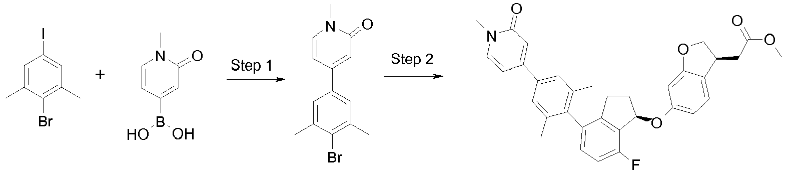

### Step 1: 4-(4-Bromo-3,5-dimethylphenyl)-1-methylpyridin-2(1H)-one

The title compound is prepared from 2-bromo-5-iodo-1,3-dimethylbenzene and 1-methyl-2-oxo-1,2-dihydropyridin-4-ylboronic acid following a procedure analogous to that described in Step 1 of Intermediate 96. LC (method 11): t_{R} = 1.04 min; Mass spectrum (ESI⁺): m/z = 292, 294 [M+H]⁺.

### Step 2: Methyl 2-((S)-6-((R)-4-(2,6-dimethyl-4-(1-methyl-2-oxo-1,2-dihydropyridin-4-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 4-(4-bromo-3,5-dimethylphenyl)-1-methylpyridin-2(1H)-one following a procedure analogous to that described in Step 5 of Intermediate 12. LC (method 11): t_{R} = 1.19 min; Mass spectrum (ESI⁺): m/z = 554 [M+H]⁺.

### Intermediate 98

### Methyl 2-((S)-6-((R)-4-(2,6-dimethyl-4-(1-methyl-6-oxo-1,6-dihydropyrin-3-yl)phenyl)-7-fluoro-2,3-dihydro-1H-inden-1-yloxy)-2,3-dihydrobenzofuran-3-yl)acetate

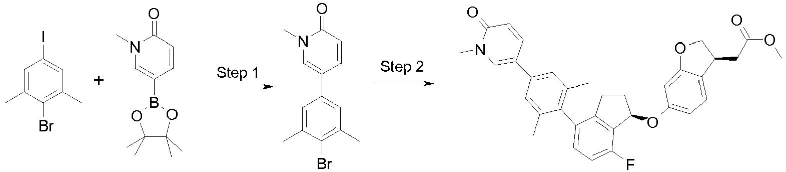

### Step 1: 5-(4-Bromo-3,5-dimethylphenyl)-1-methylpyridin-2(1H)-one

The title compound is prepared from 2-bromo-5-iodo-1,3-dimethylbenzene and 1-methyl-5-(4,4,5,5-tetramethyl-1,3,2-dioxaborolan-2-yl)pyridin-2(1H)-one following a procedure analogous to that described in Step 1 of Intermediate 96. LC (method 11): t_{R} = 1.05 min; Mass spectrum (ESI⁺): m/z = 292, 294 [M+H]⁺.

### Step 2: Methyl 2-((S)-6-((R)-4-(2,6-dimethyl-4-(1-methyl-6-oxo-1,6-dihydropyridin-3-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 5-(4-bromo-3,5-dimethylphenyl)-1-methylpyridin-2(1H)-one following a procedure analogous to that described in Step 5 of Intermediate 12. LC (method 11): t_{R} = 1.19 min; Mass spectrum (ESI⁺): m/z = 554 [M+H]⁺.

### Intermediate 99

### Methyl 2-((S)-6-((R)-4-(2,6-dimethyl-4-(1H-1,2,4-triazol-1-yl)phenyl)-7-fluoro-2,3-dihydro-1H-inden-1-yloxy)-2,3-dihydrobenzofuran-3-yl)acetate

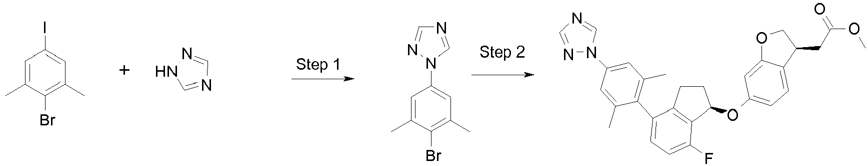

### Step 1: 1-(4-Bromo-3,5-dimethylphenyl)-1H-1,2,4-triazole

A mixture of 2-bromo-5-iodo-1,3-dimethylbenzene (500 mg), 1,2,4-triazole (340 mg), potassium carbonate (770 mg), and copper(I) iodide (232 mg) in N-methyl-2-pyrrolidinone is stirred at 130 °C over night. More potassium carbonate (770 mg) and copper(I) iodide (232 mg) are added and the mixture is heated to 150 °C for 4 h. After cooling to room temperature the mixture is diluted with tetrahydrofuran and filtered. The filtrate is concentrated *in vacuo* and purified by HPLC on reversed phase to give the title compound. LC (method 7): t_{R} = 0.99 min; Mass spectrum (ESI⁺): m/z = 252, 254 [M+H]⁺.

### Step 2: Methyl 2-((S)-6-((R)-4-(2,6-dimethyl-4-(1H-1,2,4-triazol-1-yl)phenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 1-(4-bromo-3,5-dimethylphenyl)-1H-1,2,4-triazole following a procedure analogous to that described in Step 5 of Intermediate 12. LC (method 7): t_{R} = 1.16 min; Mass spectrum (ESI⁺): m/z = 514 [M+H]⁺.

### Intermediate 100

### 2-((S)-6-((R)-4-(4-(2,5-Dihydrofuran-3-yl)-2,6-dimethylphenyl)-7-fluoro-2,3-dihydro-1H-inden-1-yloxy)-2,3-dihydrobenzofuran-3-yl)acetic acid

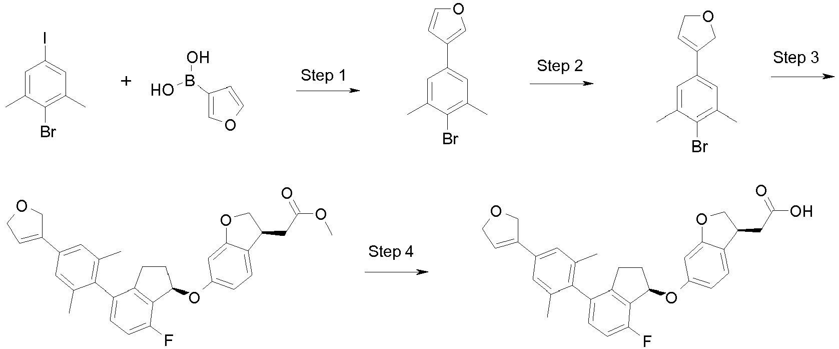

### Step 1: 3-(4-Bromo-3,5-dimethylphenyl)furan

A mixture of 2,5-dibromo-1,3-dimethylbenzene (2.00 g), 1-furan-3-ylboronic acid (856 mg), and 2 M aqueous Na₂CO₃ solution (11 mL) in 1,4-dioxane (40 mL) is purged with argon for 5 min. Tetrakis-triphenylphosphine-palladium-(0) (270 mg) is added and the mixture is stirred at 100 °C over night. More tetrakis-triphenylphosphine-palladium-(0) (50 mg) is added and the mixture is stirred for another 5 h at 100 °C. After cooling to room temperature the mixture is diluted with ethyl acetate and aqueous NH₄Cl solution. The aqueous phase is extracted with ethyl acetate and the combined extracts are washed with brine, dried over MgSO₄ and concentrated *in vacuo.* The residue is chromatographed on silica gel (cyclohexane/ethyl acetate 100:0→90:10) to give the title compound. LC (method 7): t_{R} = 1.22 min; Mass spectrum (EI⁺): m/z = 250 [M]⁺.

### Step 2: 3-(4-Bromo-3,5-dimethylphenyl)-2,5-dihydrofuran

Triethylsilane (3.67 mL) is added dropwise to a solution of 3-(4-bromo-3,5-dimethylphenyl)furan (580 mg) in trifluoroacetic acid and the resulting mixture is stirred at room temperature for 3 h. Saturated aqueous NaHCO₃ solution is added and the aqueous phase is separated and extracted with ethyl acetate. The combined extracts are washed with brine, dried over MgSO₄ and concentrated *in vacuo.* The residue is chromatographed on silica gel (cyclohexane/ethyl acetate 95:5) to give the title compound. LC (method 9): t_{R} = 1.16 min; Mass spectrum (ESI⁺): m/z = 251, 253 [M+H]⁺.

### Step 3: Methyl 2-((S)-6-((R)-4-(4-(2,5-dihydrofuran-3-yl)-2,6-dimethylphenyl)-7-fluoro-2,3-dihydro-1H-inden-1-yloxy)-2,3-dihydrobenzofuran-3-yl)acetate

The title compound is prepared from methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydro-benzofuran-3-yl)acetate and 3-(4-bromo-3,5-dimethylphenyl)-2,5-dihydrofuran following a procedure analogous to that described in Step 5 of Intermediate 12. LC (method 9): t_{R} = 1.26 min; Mass spectrum (ESI⁺): m/z = 515 [M+H]⁺.

### Step 4: 2-((S)-6-((R)-4-(4-(2,5-Dihydrofuran-3-yl)-2,6-dimethylphenyl)-7-fluoro-2,3-dihydro-1H-inden-1-yloxy)-2,3-dihydrobenzofuran-3-yl)acetic acid

The title compound is prepared from methyl 2-((S)-6-((R)-4-(4-(2,5-dihydrofuran-3-yl)-2,6-dimethylphenyl)-7-fluoro-2,3-dihydro-1H-inden-1-yloxy)-2,3-dihydrobenzofuran-3-yl)acetate following a procedure analogous to that described in Step 2 of Intermediate 62 using NaOH instead of LiOHxH₂O. LC (method 9): t_{R} = 0.16 min; Mass spectrum (ESI⁺): m/z = 501 [M+H]⁺.

### Intermediate 101

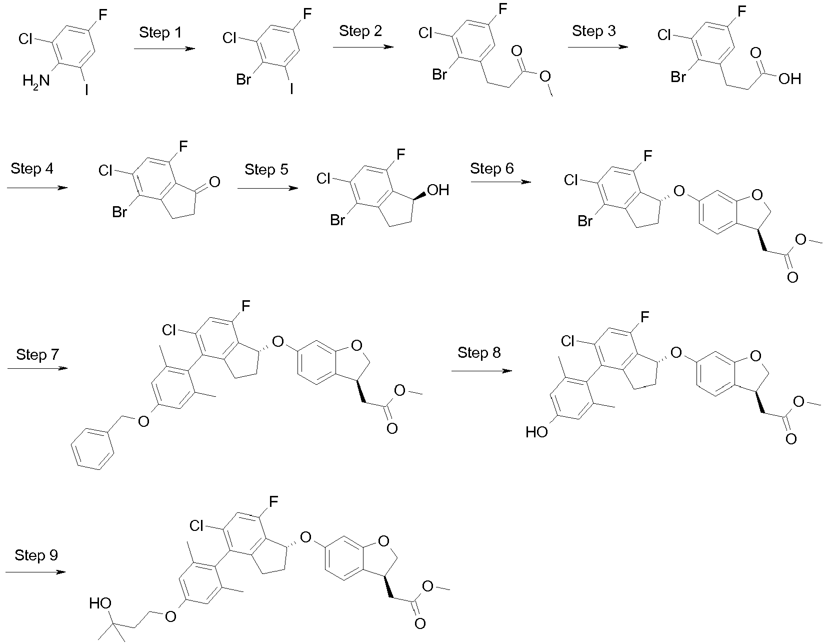

### Step 1: 2-Bromo-1-chloro-5-fluoro-3-iodobenzene

Tert-butyl nitrite (4.00 ml) is added dropwise to a suspension of copper(II) bromide in acetonitrile (50 mL) at 0 °C. The mixture is heated to 65 °C and stirred for 30 min. A solution of 2-chloro-4-fluoro-6-iodo-phenylamine (6.30 g) in acetonitrile (15 mL) is added dropwise and the resulting mixture is stirred at 65 °C for 1 h. After cooling to room temperature the mixture is diluted with ethyl acetate and saturated NaHCO₃ solution. The aqueous phase is extracted with ethyl acetate and the combined extracts are washed with brine, dried over MgSO₄ and concentrated *in vacuo.* The residue is chromatographed on silica gel (cyclohexane/ethyl acetate 100:0→98:2) to give the title compound. LC (method 7): t_{R} = 1.18 min; Mass spectrum (EI⁺): m/z = 334 [M]⁺.

### Step 2: Methyl 3-(2-bromo-3-chloro-5-fluorophenyl)propanoate

The title compound is prepared from 2-bromo-1-chloro-5-fluoro-3-iodobenzene following a procedure analogous to that described in Step 1 of Intermediate 17. LC (method 7): t_{R} = 1.11 min.

### Step 3: 3-(2-Bromo-3-chloro-5-fluorophenyl)propanoic acid

The title compound is prepared from methyl 3-(2-bromo-3-chloro-5-fluorophenyl)propanoate following a procedure analogous to that described in Step 2 of Intermediate 17. LC (method 9): t_{R} = 0.99 min; Mass spectrum (ESI⁻): m/z = 279 [M-H]⁻.

### Step 4: 4-Bromo-5-chloro-7-fluoro-2,3-dihydro-1H-inden-1-one

The title compound is prepared from 3-(2-bromo-3-chloro-5-fluorophenyl)propanoic acid following a procedure analogous to that described in Step 4 of Intermediate 25. LC (method 9): t_{R} = 1.02 min; Mass spectrum (ESI⁺): m/z = 263 [M+H]⁺.

### Step 5: (S)-4-Bromo-5-chloro-7-fluoro-2,3-dihydro-1H-inden-1-ol

The title compound is prepared from 4-bromo-5-chloro-7-fluoro-2,3-dihydro-1H-inden-1-one following a procedure analogous to that described in Step 1 of Intermediate 1. LC (method 9): t_{R} = 0.99 min; Mass spectrum (ESI⁻): m/z = 263 [M-H]⁻.

### Step 6: Methyl 2-((S)-6-((R)-4-bromo-5-chloro-7-fluoro-2,3-dihydro-1H-inden-1-yloxy)-2,3-dihydrobenzofuran-3-yl)acetate

The title compound is prepared from (S)-4-bromo-5-chloro-7-fluoro-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 9): t_{R} = 1.23 min; Mass spectrum (ESI⁺): m/z = 455 [M+H]⁺.

### Step 7: Methyl 2-((S)-6-((R)-4-(4-(benzyloxy)-2,6-dimethylphenyl)-5-chloro-7-fluoro-2,3-dihydro-1H-inden-1-yloxy)-2,3-dihydrobenzofuran-3-yl)acetate

The title compound is prepared from methyl 2-((S)-6-((R)-4-bromo-5-chloro-7-fluoro-2,3-dihydro-1H-inden-1-yloxy)-2,3-dihydrobenzofuran-3-yl)acetate and 4-(benzyloxy)-2,6-dimethylphenylboronic acid following a procedure analogous to that described in Step 3 of Intermediate 39. LC (method 9): t_{R} = 1.32 min.

### Step 8: Methyl 2-((S)-6-((R)-5-chloro-7-fluoro-4-(4-hydroxy-2,6-dimethylphenyl)-2,3-dihydro-1H-inden-1-yloxy)-2,3-dihydrobenzofuran-3-yl)acetate

The title compound is prepared from methyl 2-((S)-6-((R)-4-(4-(benzyloxy)-2,6-dimethylphenyl)-5-chloro-7-fluoro-2,3-dihydro-1H-inden-1-yloxy)-2,3-dihydrobenzofuran-3-yl)acetate following a procedure analogous to that described in Step 4 of Intermediate 39. LC (method 9): t_{R} = 1.17 min; Mass spectrum (ESI⁺): m/z = 497 [M+H]⁺.

### Step 9: Methyl 2-((S)-6-((R)-5-chloro-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate

The title compound is prepared from methyl 2-((S)-6-((R)-5-chloro-7-fluoro-4-(4-hydroxy-2,6-dimethylphenyl)-2,3-dihydro-1H-inden-1-yloxy)-2,3-dihydrobenzofuran-3-yl)acetate and 3-hydroxy-3-methylbutyl 4-methylbenzenesulfonate following a procedure analogous to that described in Step 5 of Intermediate 39. LC (method 9): t_{R} = 1.25 min.

### Intermediate 102

### Methyl 2-((S)-6-((R)-5-chloro-4-(2,6-dimethyl-4-((R)-tetrahydrofuran-3-yloxy)phenyl)-7-fluoro-2,3-dihydro-1H-inden-1-yloxy)-2,3-dihydrobenzofuran-3-yl)acetate

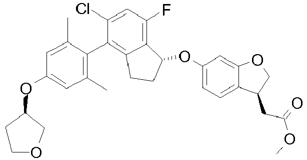

The title compound is prepared from methyl 2-((S)-6-((R)-5-chloro-7-fluoro-4-(4-hydroxy-2,6-dimethylphenyl)-2,3-dihydro-1H-inden-1-yloxy)-2,3-dihydrobenzofuran-3-yl)acetate and (S)-tetrahydrofuran-3-yl 4-methylbenzenesulfonate following a procedure analogous to that described in Step 5 of Intermediate 39. LC (method 9): t_{R} = 1.26 min.

### Intermediate 103

### Methyl 2-((3S)-6-((1R)-4-(2-(pyrrolidin-1-yl)pyridin-3-yl)-2,3-dihydro-1H-inden-1-yl oxy)-2,3-dihydrobenzofuran-3-yl)acetate

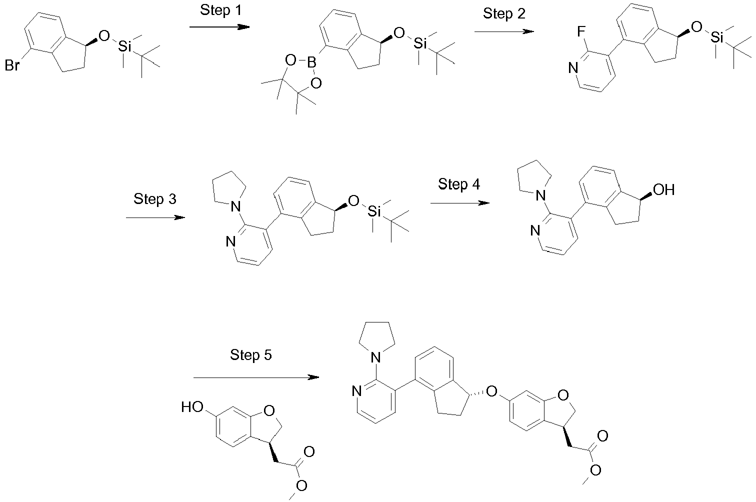

### Step 1: (S)-tert-Butyldimethyl(4-(4,4,5,5-tetramethyl-1,3,2-dioxaborolan-2-yl)-2,3-dihy dro-1H-inden-1-yloxy)silane

The title compound is prepared from (S)-(4-bromo-2,3-dihydro-1H-inden-1-yloxy)(tert-butyl)dimethylsilane following a procedure analogous to that described in Step 4 of Intermediate 12. LC (method 8): t_{R} = 1.26 min.

### Step 2: 3-((1S)-1-(tert-Butyldimethylsilyloxy)-2,3-dihydro-1H-inden-4-yl)-2-fluoropyridine

The title compound is prepared from (S)-tert-butyldimethyl(4-(4,4,5,5-tetramethyl-1,3,2-dioxaborolan-2-yl)-2,3-dihydro-1H-inden-1-yloxy)silane and 3-bromo-2-fluoropyridine following a procedure analogous to that described in Step 5 of Intermediate 12. LC (method 8): t_{R} = 1.32 min; Mass spectrum (ESI⁺): m/z = 344 [M+H]⁺.

### Step 3: 3-((1S)-1-(tert-Butyldimethylsilyloxy)-2,3-dihydro-1H-inden-4-yl)-2-(pyrrolidin-1-yl)pyridine

To a solution of 3-((1S)-1-(tert-butyldimethylsilyloxy)-2,3-dihydro-1H-inden-4-yl)-2-fluoropyridine (230 mg) in N-methyl-pyrrolidon (NMP, 2 mL) is added pyrrolidine (166 µL). The mixture is stirred for 12 hours at 100°C, partitioned between water and ethyl acetate and the aqueous phase is twice extracted with ethyl acetate. The combined organic phases are washed with brine, dried (MgSO₄) and concentrated to give the title compound. Yield: 260 mg; LC (method 7): t_{R} = 1.15 min.

### Step 4: (1S)-4-(2-(Pyrrolidin-1-yl)pyridin-3-yl)-2,3-dihydro-1H-inden-1-ol

The title compound is prepared from 3-((1S)-1-(tert-butyldimethylsilyloxy)-2,3-dihydro-1H-inden-4-yl)-2-(pyrrolidin-1-yl)pyridine following a procedure analogous to that described in Step 4 of Intermediate 4. LC (method 7): t_{R} = 0.70 min.

### Step 5: Methyl 2-((3S)-6-((1R)-4-(2-(pyrrolidin-1-yl)pyridin-3-yl)-2,3-dihydro-1H-inden-1-yloxy)-2,3-dihydrobenzofuran-3-yl)acetate

The title compound is prepared from (1S)-4-(2-(pyrrolidin-1-yl)pyridin-3-yl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 7): t_{R} = 0.96 min.

### Intermediate 104

### 2,6-Dimethyl-4-(4,4,5,5-tetramethyl-1,3,2-dioxaborolan-2-yl)aniline

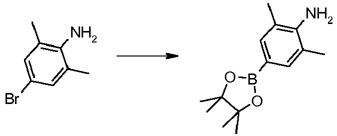

The title compound is prepared from 4-bromo-2,6-dimethylaniline following a procedure analogous to that described in Step 4 of Intermediate 12. LC (method 7): t_{R} = 0.99 min; Mass spectrum (ESI⁺): m/z = 248 [M+H]⁺.

### Intermediate 105

### Methyl 2-((S)-6-((R)-4-(2,6-dimethyl-4-(1-methyl-6-oxo-1,6-dihydropyrimidin-4-yl)phenyl)-7-fluoro-2,3-dihydro-1H-inden-1-yloxy)-2,3-dihydrobenzofuran-3-yl)acetate

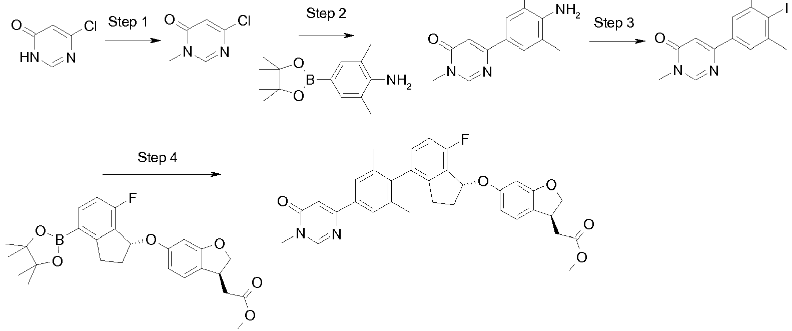

### Step 1: 6-Chloro-3-methylpyrimidin-4(3H)-one

A mixture of 6-chloropyrimidin-4(3H)-one (5 g), methyliodide (2.6 mL) and K₂CO₃ (10.6 g) in acetone (100 mL) is stirred at room temperature over night. The mixture is partitioned between water and ethyl acetate. The organic phase is washed with brine, dried (MgSO₄) and concentrated. The residue is triturated with diisopropylether to give the title compound. Yield: 5.1 g; LC (method 11): t_{R} = 0.25 min; Mass spectrum (ESI⁺): m/z = 145 [M+H]⁺.

### Step 2: 6-(4-Amino-3,5-dimethylphenyl)-3-methylpyrimidin-4(3H)-one

The title compound is prepared from 6-chloro-3-methylpyrimidin-4(3H)-one and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 65°C. LC (method 11): t_{R} = 0.71 min; Mass spectrum (ESI⁺): m/z = 230 [M+H]⁺.

### Step 3: 6-(4-Iodo-3,5-dimethylphenyl)-3-methylpyrimidin-4(3H)-one

To a solution of 6-(4-amino-3,5-dimethylphenyl)-3-methylpyrimidin-4(3H)-one (2.6 g) and p-toluenesulfonic acid monohydrate (6.5 g) in tert.-butanol (30 mL) is added dropwise at 10-15°C a solution of NaNO₃ (1.6 g) and Kl (4.7 g) in water (10 mL). The mixture is stirred for 20 minutes at 15°C and 3 hours at room temperature. Thereafter the mixture is treated with water (50 mL), saturated aqueous NaHCO₃ solution (20 mL) and 10% aqueous solution of Na₂S₂O₃ (20 mL). The mixture is extracted 4 times with ethyl acetate. The combined organic phases are washed with 10% aqueous solution of Na₂S₂O₃ and brine. After drying (MgSO₄) the solvents are evaporated. The crude product is used directly in the next step.

### Step 4: Methyl 2-((S)-6-((R)-4-(2,6-dimethyl-4-(1-methyl-6-oxo-1,6-dihydropyrimidin-4-yl)phenyl)-7-fluoro-2,3-dihydro-1H-inden-1-yloxy)-2,3-dihydrobenzofuran-3-yl)acetate

The title compound is prepared from 6-(4-iodo-3,5-dimethylphenyl)-3-methylpyrimidin-4(3H)-one and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 11): t_{R} = 1.19 min; Mass spectrum (ESI⁺): m/z = 555 [M+H]⁺.

### Intermediate 106

### Methyl 2-((S)-6-((R)-4-(2,6-dimethyl-4-(1-methyl-2-oxo-1,2-dihydropyrimidin-5-yl)phenyl)-7-fluoro-2,3-dihydro-1H-inden-1-yloxy)-2,3-dihydrobenzofuran-3-yl)acetate

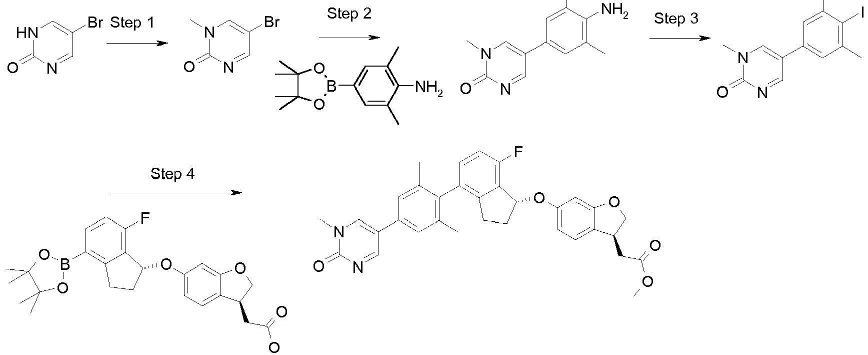

### Step 1: 5-Bromo-1-methylpyrimidin-2(1H)-one

A mixture of 5-bromopyrimidin-2(1H)-one (3 g), methyliodide (1.1 mL) and K₂CO₃ (2.4 g) in N,N-dimethylformamide (60 mL) is stirred for 5 hours at room temperature. The mixture is filtered and the mother liquor is concentrated. The residue is partitioned between water and dichloromethane. The organic phase is washed with brine, dried (MgSO₄) and concentrated to give the title compound. Yield: 385 mg; LC (method 9): t_{R} = 0.25 min; Mass spectrum (ESI⁺): m/z = 189 [M+H]⁺.

### Step 2: 5-(4-Amino-3,5-dimethylphenyl)-1-methylpyrimidin-2(1H)-one

The title compound is prepared from 5-bromo-1-methylpyrimidin-2(1 H)-one and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 70°C. LC (method 9): t_{R} = 0.63 min; Mass spectrum (ESI⁺): m/z = 230 [M+H]⁺.

### Step 3: 5-(4-Iodo-3,5-dimethylphenyl)-1-methylpyrimidin-2(1H)-one

The title compound is prepared from 5-(4-amino-3,5-dimethylphenyl)-1-methylpyrimidin-2(1 H)-onefollowing a procedure analogous to that described in Step 3 of Intermediate 105. LC (method 9): t_{R} = 0.96 min; Mass spectrum (ESI⁺): m/z = 341 [M+H]⁺.

### Step 4: Methyl 2-((S)-6-((R)-4-(2,6-dimethyl-4-(1-methyl-2-oxo-1,2-dihydropyrimidin-5-yl)phenyl)-7-fluoro-2,3-dihydro-1H-inden-1-yloxy)-2,3-dihydrobenzofuran-3-yl)acetate

The title compound is prepared from 5-(4-iodo-3,5-dimethylphenyl)-1-methylpyrimidin-2(1H)-one and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 14): t_{R} = 1.08 min; Mass spectrum (ESI⁺): m/z = 555 [M+H]⁺.

### Intermediate 107

### Methyl 2-((S)-6-((R)-4-(2,6-dimethyl-4-(pyridazin-4-yl)phenyl)-7-fluoro-2,3-dihydro-1H-inden-1-yloxy)-2,3-dihydrobenzofuran-3-yl)acetate

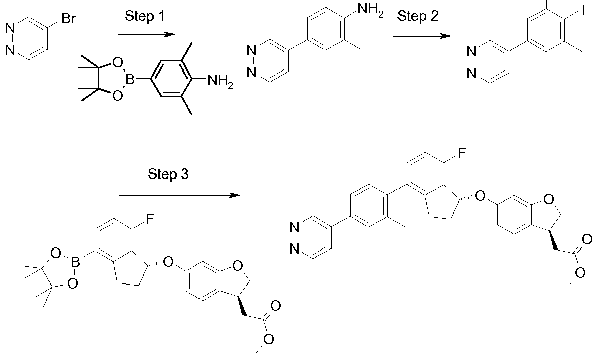

### Step 1: 2,6-Dimethyl-4-(pyridazin-4-yl)aniline

The title compound is prepared from 4-bromopyridazine hydrobromide and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 70°C. LC (method 9): t_{R} = 0.66 min; Mass spectrum (ESI⁺): m/z = 200 [M+H]⁺.

### Step 2: 4-(4-lodo-3,5-dimethylphenyl)pyridazine

The title compound is prepared from 2,6-dimethyl-4-(pyridazin-4-yl)aniline following a procedure analogous to that described in Step 3 of Intermediate 105. LC (method 9): t_{R} = 1.02 min; Mass spectrum (ESI⁺): m/z = 311 [M+H]⁺.

### Step 3: Methyl 2-((S)-6-((R)-4-(2,6-dimethyl-4-(pyridazin-4-yl)phenyl)-7-fluoro-2,3-dihydro-1H-inden-1-yloxy)-2,3-dihydrobenzofuran-3-yl)acetate

The title compound is prepared from 4-(4-iodo-3,5-dimethylphenyl)pyridazine and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 15): t_{R} = 1.20 min; Mass spectrum (ESI⁺): m/z = 525 [M+H]⁺.

### Intermediate 108

### Methyl 2-((S)-6-((R)-4-(2,6-dimethyl-4-(pyrimidin-4-yl)phenyl)-7-fluoro-2,3-dihydro-1H-inden-1-yloxy)-2,3-dihydrobenzofuran-3-yl)acetate

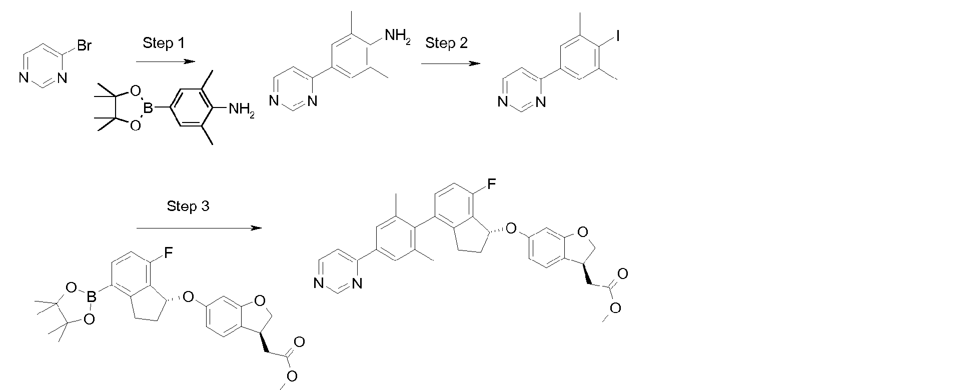

### Step 1: 2,6-Dimethyl-4-(pyrimidin-4-yl)aniline

The title compound is prepared from 4-bromopyrimidine hydrochloride and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 70°C. LC (method 9): t_{R} = 0.74 min; Mass spectrum (ESI⁺): m/z = 200 [M+H]⁺.

### Step 2: 4-(4-lodo-3,5-dimethylphenyl)pyrimidine

The title compound is prepared from 2,6-dimethyl-4-(pyrimidin-4-yl)aniline following a procedure analogous to that described in Step 3 of Intermediate 105. LC (method 9): t_{R} = 1.10 min; Mass spectrum (ESI⁺): m/z = 311 [M+H]⁺.

### Step 3: Methyl 2-((S)-6-((R)-4-(2,6-dimethyl-4-(pyrimidin-4-yl)phenyl)-7-fluoro-2,3-dihydro-1H-inden-1-yloxy)-2,3-dihydrobenzofuran-3-yl)acetate

The title compound is prepared from 4-(4-iodo-3,5-dimethylphenyl)pyrimidine and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 15): t_{R} = 1.26 min.

### Intermediate 109

### Methyl 2-((S)-6-((R)-4-(2,6-dimethyl-4-(1-methyl-6-oxo-1,6-dihydropyridazin-4-yl)phenyl)-7-fluoro-2,3-dihydro-1H-inden-1-yloxy)-2,3-dihydrobenzofuran-3-yl)acetate

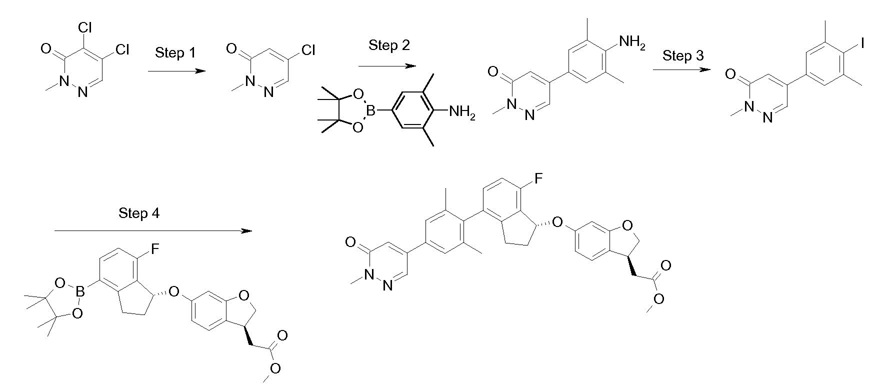

### Step 1: 5-Chloro-2-methylpyridazin-3(2H)-one

4,5-Dichloro-2-methylpyridazin-3(2H)-one (1 g) is dissolved in aqueous HI solution (57 %, 8.5 mL) and the mixture is heated to reflux for 12 hours. After cooling to room temperature the mixture is treated with aqueous Na₂S₂O₃ solution (30 %, 100 mL) and stirrd for 1 hour. The mixture is then extracted three times with dichloromethane. The combined organic phases are washed with brine, dried (MgSO₄) and concentrated. The resudue is triturated with diisopropylether. Yield: 414 mg; Mass spectrum (ESI⁺): m/z = 145 [M+H]⁺.

### Step 2: 5-(4-Amino-3,5-dimethylphenyl)-2-methylpyridazin-3(2H)-one

The title compound is prepared from 5-chloro-2-methylpyridazin-3(2H)-one and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 60°C. LC (method 9): t_{R} = 0.79 min; Mass spectrum (ESI⁺): m/z = 230 [M+H]⁺.

### Step 3: 5-(4-lodo-3,5-dimethylphenyl)-2-methylpyridazin-3(2H)-one

The title compound is prepared from 5-(4-amino-3,5-dimethylphenyl)-2-methylpyridazin-3(2H)-one following a procedure analogous to that described in Step 3 of Intermediate 105. LC (method 9): t_{R} = 1.06 min; Mass spectrum (ESI⁺): m/z = 341 [M+H]⁺.

### Step 4: Methyl 2-((S)-6-((R)-4-(2,6-dimethyl-4-(1-methyl-6-oxo-1,6-dihydropyridazin-4-yl)phenyl)-7-fluoro-2,3-dihydro-1H-inden-1-yloxy)-2,3-dihydrobenzofuran-3-yl)acetate

The title compound is prepared from 5-(4-iodo-3,5-dimethylphenyl)-2-methylpyridazin-3(2H)-one and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 14): t_{R} = 1.18 min; Mass spectrum (ESI⁺): m/z = 555 [M+H]⁺.

### Intermediate 110

### Methyl 2-((S)-6-((R)-7-fluoro-4-(4-(2-methoxypyrimidin-4-yl)-2,6-dimethylphenyl)-2,3-dihydro-1H-inden-1-yloxy)-2,3-dihydrobenzofuran-3-yl)acetate

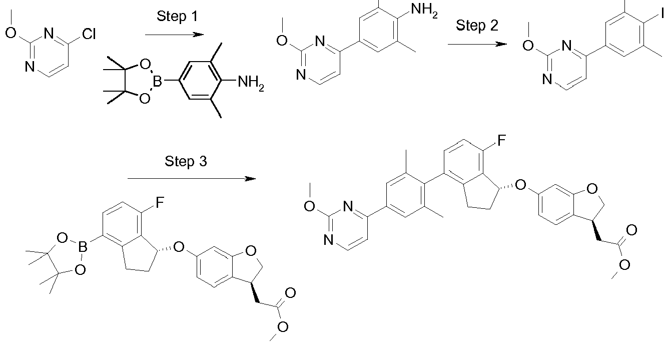

### Step 1: 4-(2-Methoxypyrimidin-4-yl)-2,6-dimethylaniline

The title compound is prepared from 4-chloro-2-methoxypyrimidine and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 60°C. LC (method 14): t_{R} = 0.89 min; Mass spectrum (ESI⁺): m/z = 230 [M+H]⁺.

### Step 2: 4-(4-lodo-3,5-dimethylphenyl)-2-methoxypyrimidine

The title compound is prepared from 5-(4-amino-3,5-dimethylphenyl)-2-methylpyridazin-3(2H)-one following a procedure analogous to that described in Step 3 of Intermediate 105. LC (method 14): t_{R} = 1.18 min; Mass spectrum (ESI⁺): m/z = 341 [M+H]⁺.

### Step 3: Methyl 2-((S)-6-((R)-7-fluoro-4-(4-(2-methoxypyrimidin-4-yl)-2,6-dimethylphe nyl)-2,3-dihydro-1H-inden-1-yloxy)-2,3-dihydrobenzofuran-3-yl)acetate

The title compound is prepared from 4-(4-iodo-3,5-dimethylphenyl)-2-methoxypyrimidine and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 8): t_{R} = 0.82 min; Mass spectrum (ESI⁺): m/z = 555 [M+H]⁺.

### Intermediate 111

### Methyl 2-((S)-6-((R)-4-(2,6-dimethyl-4-(1H-tetrazol-5-yl)phenyl)-7-fluoro-2,3-dihydro-1H-inden-1-yloxy)-2,3-dihydrobenzofuran-3-yl)acetate

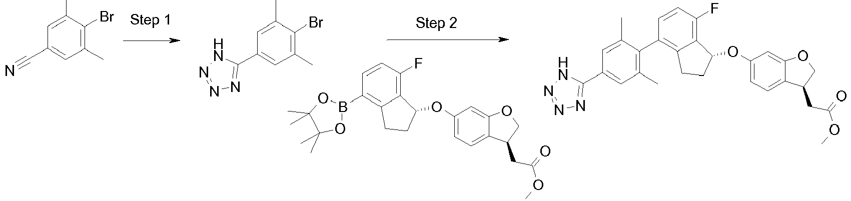

### Step 1: 5-(4-Bromo-3,5-dimethylphenyl)-1H-tetrazole

To a solution of 4-bromo-3,5-dimethylbenzonitrile (1 g) in N,N-dimethylformamide (10 mL) is added NH₄Cl (770 mg) and NaN₃ (800 mg) and the mixture is heated to 100°C for 12 hours. After cooling to room temperature the mixture is diluted with water. The formed precipitate is filtered off, washed with water and dried to give the title compound. Yield: 650 mg; Mass spectrum (ESI⁺): m/z = 253 [M+H]⁺.

### Step 2: Methyl 2-((S)-6-((R)-4-(2,6-dimethyl-4-(1H-tetrazol-5-yl)phenyl)-7-fluoro-2,3-dihydro-1H-inden-1-yloxy)-2,3-dihydrobenzofuran-3-yl)acetate

The title compound is prepared from 5-(4-bromo-3,5-dimethylphenyl)-1H-tetrazole and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 11): t_{R} = 1.05 min; Mass spectrum (ESI⁺): m/z = 515 [M+H]⁺.

### Intermediate 112

### 5-(4-Bromo-3,5-dimethylphenyl)-2-methyl-2H-tetrazole and 5-(4-bromo-3,5-dimethylphenyl)-1-methyl-1H-tetrazole

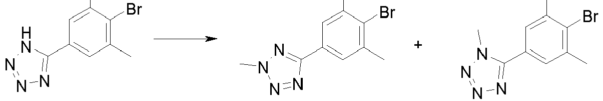

To a solution of 5-(4-bromo-3,5-dimethylphenyl)-1H-tetrazole (780 mg) in N,N-dimethylformamide (12 mL) is added KOH (432 mg) and Mel (210 µL) and the mixture is stirred for 4 hours at room temperature. Then the mixture is diluted with water and extracted 3 times with dichloromethane. The combined organic phases are washed with water, dried (MgSO₄) and concentrated. The residue is chromatographed on silica gel (cyclohexane/ethyl acetate 95:5→70:30) to give the title compounds.
5-(4-bromo-3,5-dimethylphenyl)-1-methyl-1H-tetrazole: Yield: 135 mg; LC (method 11): t_{R} = 1.03 min; Mass spectrum (ESI⁺): m/z = 267 [M+H]⁺.
5-(4-bromo-3,5-dimethylphenyl)-2-methyl-2H-tetrazole: Yield: 660 mg; LC (method 11): t_{R} = 1.16 min; Mass spectrum (ESI⁺): m/z = 267 [M+H]⁺.

### Intermediate 113

### Methyl 2-((S)-6-((R)-4-(2,6-dimethyl-4-(2-methyl-2H-tetrazol-5-yl)phenyl)-7-fluoro-2,3-dihydro-1H-inden-1-yloxy)-2,3-dihydrobenzofuran-3-yl)acetate

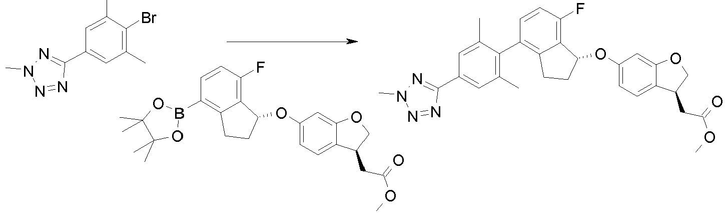

The title compound is prepared from 5-(4-bromo-3,5-dimethylphenyl)-2-methyl-2H-tetrazole and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 11): t_{R} = 1.26 min; Mass spectrum (ESI⁺): m/z = 529 [M+H]⁺.

### Intermediate 114

### Methyl 2-((S)-6-((R)-4-(2,6-dimethyl-4-(1-methyl-1H-tetrazol-5-yl)phenyl)-7-fluoro-2,3-dihydro-1H-inden-1-yloxy)-2,3-dihydrobenzofuran-3-yl)acetate

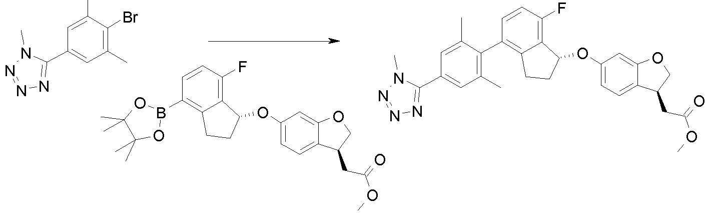

The title compound is prepared from 5-(4-bromo-3,5-dimethylphenyl)-1-methyl-1H-tetrazole and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 11): t_{R} = 1.20 min; Mass spectrum (ESI⁺): m/z = 529 [M+H]⁺.

### Intermediate 115

### Methyl 2-((S)-6-((R)-4-(2,6-dimethyl-4-(1-methyl-2-oxo-1,2-dihydropyrimidin-4-yl)phenyl)-7-fluoro-2,3-dihydro-1H-inden-1-yloxy)-2,3-dihydrobenzofuran-3-yl)acetate

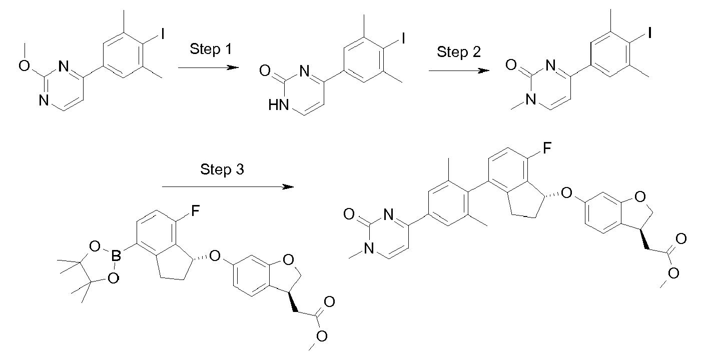

### Step 1: 4-(4-Iodo-3,5-dimethylphenyl)pyrimidin-2(1H)-one

4-(4-Iodo-3,5-dimethylphenyl)-2-methoxypyrimidine is dissolved in 33 % solution of HBr in acetic acid (7.7 mL) and the mixture is stirred for 12 hours at room temperature. Then the mixture is diluted with water. The formed precipitate is filtered off, washed with acetone and dired. Yield: 1 g; LC (method 11): t_{R} = 0.92 min; Mass spectrum (ESI⁺): m/z = 327 [M+H]⁺.

### Step 2: 4-(4-Iodo-3,5-dimethylphenyl)-1-methylpyrimidin-2(1H)-one

A mixture of 4-(4-iodo-3,5-dimethylphenyl)pyrimidin-2(1H)-one (500 mg), methyliodide (165 µL) and K₂CO₃ (254 mg) in N,N-dimethylformamide (10 mL) is stirred for 12 hours at room temperature. Then the mixture is partitioned between water and dichloromethane. The aqueous phase is extracted twice with dichloromethane and the combined organic phases are washed with brine. After drying (MgSO₄) the solvents are evaporated. The residue is triturated with diethylether. The solid is filtered off and dried to give the title compound. Yield: 380 mg; LC (method 11): t_{R} = 0.96 min; Mass spectrum (ESI⁺): m/z = 341 [M+H]⁺.

### Step 3: Methyl 2-((S)-6-((R)-4-(2,6-dimethyl-4-(1-methyl-2-oxo-1,2-dihydropyrimidin-4-yl)phenyl)-7-fluoro-2,3-dihydro-1H-inden-1-yloxy)-2,3-dihydrobenzofuran-3-yl)acetate

The title compound is prepared from 4-(4-iodo-3,5-dimethylphenyl)-1-methylpyrimidin-2(1H)-one and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 15): t_{R} = 1.14 min; Mass spectrum (ESI⁺): m/z = 555 [M+H]⁺.

### Intermediate 116

### 1-(5-(4-Bromo-3,5-dimethylphenyl)-2H-tetrazol-2-yl)-2-methylpropan-2-ol and 2-(5-(4-bromo-3,5-dimethylphenyl)-2H-tetrazol-2-yl)-2-methylpropan-1-ol

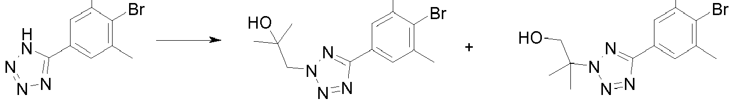

To a solution of 5-(4-bromo-3,5-dimethylphenyl)-1H-tetrazole (500 mg) in methanol (5 mL) is added Cs₂CO₃ (645 mg) and 1,1-dimethyloxirane (450 µL). The vial is sealed and the mixture is heated to 60°C for 3 hours. Cs₂CO₃ (400 mg) and 1,1-dimethyloxirane (300 µL) are added and the mixture is heated to 60°C for 12 hours. After addition of 1,1-dimethyloxirane (450 µL) the mixture is heated to 80°C for 6 hours. Then the solvents are evaporated and the residue is partitioned between water and ethyl acetate. The aqueous phase is extracted twice with ethyl acetate and the combined organic phases are dried (MgSO₄). After concentration the residue is purified by HPLC on reversed phase to give the title compounds.
1-(5-(4-bromo-3,5-dimethylphenyl)-2H-tetrazol-2-yl)-2-methylpropan-2-ol: Yield: 310 mg; LC (method 11): t_{R} = 1.14 min; Mass spectrum (ESI⁺): m/z = 325 [M+H]⁺.
2-(5-(4-bromo-3,5-dimethylphenyl)-2H-tetrazol-2-yl)-2-methylpropan-1-ol: Yield: 40 mg; LC (method 11): t_{R} = 1.16 min; Mass spectrum (ESI⁺): m/z = 325 [M+H]⁺.

### Intermediate 117

### Methyl 2-((S)-6-((R)-7-fluoro-4-(4-(2-(2-hydroxy-2-methylpropyl)-2H-tetrazol-5-yl)-2,6-dimethylphenyl)-2,3-dihydro-1H-inden-1-yloxy)-2,3-dihydrobenzofuran-3-yl)acetate

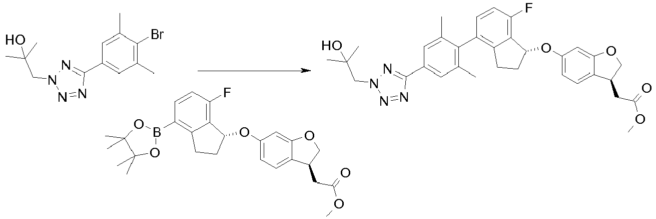

The title compound is prepared from 1-(5-(4-bromo-3,5-dimethylphenyl)-2H-tetrazol-2-yl)-2-methylpropan-2-ol and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 15): t_{R} = 1.24 min; Mass spectrum (ESI⁺): m/z = 587 [M+H]⁺.

### Intermediate 118

### Methyl 2-((S)-6-((R)-7-fluoro-4-(4-(2-(1-hydroxy-2-methylpropan-2-yl)-2H-tetrazol-5-yl)-2,6-dimethylphenyl)-2,3-dihydro-1H-inden-1-yloxy)-2,3-dihydrobenzofuran-3-yl)acetate

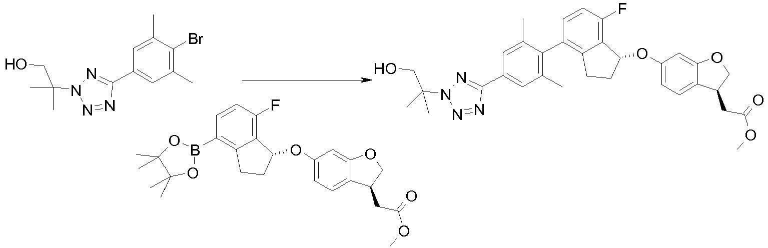

The title compound is prepared from 2-(5-(4-bromo-3,5-dimethylphenyl)-2H-tetrazol-2-yl)-2-methylpropan-1-ol and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 15): t_{R} = 1.25 min; Mass spectrum (ESI⁺): m/z = 587 [M+H]⁺.

### Intermediate 119

### Methyl 2-((3S)-6-((1R)-4-(2-(piperidin-1-yl)pyridin-3-yl)-2,3-dihydro-1H-inden-1-yloxy)-2,3-dihydrobenzofuran-3-yl)acetate

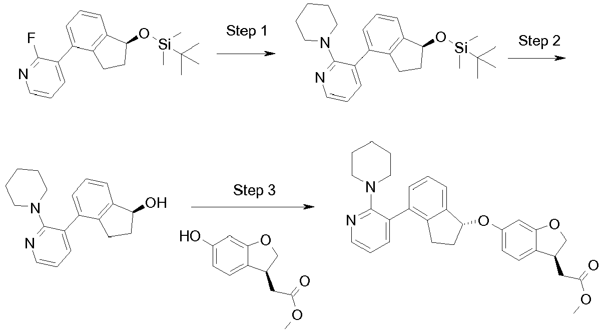

### Step 1: 3-((1S)-1-(tert-Butyldimethylsilyloxy)-2,3-dihydro-1H-inden-4-yl)-2-(piperidin-1-yl)pyridine

The title compound is prepared from piperidine and 3-((1S)-1-(tert-butyldimethylsilyloxy)-2,3-dihydro-1H-inden-4-yl)-2-fluoropyridine following a procedure analogous to that described in Step 3 of Intermediate 103. LC (method 7): t_{R} = 1.20 min.

### Step 2: (1S)-4-(2-(Piperidin-1-yl)pyridin-3-yl)-2,3-dihydro-1H-inden-1-ol

The title compound is prepared from 3-((1S)-1-(tert-butyldimethylsilyloxy)-2,3-dihydro-1H-inden-4-yl)-2-(piperidin-1-yl)pyridine following a procedure analogous to that described in Step 4 of Intermediate 4. LC (method 7): t_{R} = 0.73 min.

### Step 3: Methyl 2-((3S)-6-((1R)-4-(2-(piperidin-1-yl)pyridin-3-yl)-2,3-dihydro-1H-inden-1-yloxy)-2,3-dihydrobenzofuran-3-yl)acetate

The title compound is prepared from (1S)-4-(2-(piperidin-1-yl)pyridin-3-yl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 7): t_{R} = 0.92 min.

### Intermediate 120

### Methyl 2-((3S)-6-((1R)-4(2-phenoxypyridin-3-yl)-2,3-dihydro-1H-inden-1-yloxy)-2,3-dihydrobenzofuran-3-yl)acetate

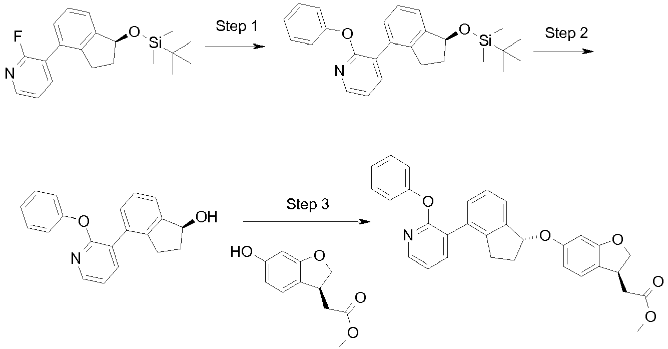

### Step 1: 3-((1S)-1-(tert-Butyldimethylsilyloxy)-2,3-dihydro-1H-inden-4-yl)-2-phenoxypyridine

3-((1S)-1-(tert-Butyldimethylsilyloxy)-2,3-dihydro-1H-inden-4-yl)-2-fluoropyridine (220 mg) is dissolved in N-methylpyrrolidone (NMP, 2.5 mL), treated with a solution of LiOtBu in tetrahydrofurane (1 mL, 1 M) and phenole (180 mg) and heated to 200°C for 2 hours. The mixture thus obtained is directly used in the next step.

### Step 2: (1S)-4-(2-Phenoxypyridin-3-yl)-2,3-dihydro-1H-inden-1-ol

The title compound is prepared from 3-((1S)-1-(tert-butyldimethylsilyloxy)-2,3-dihydro-1H-inden-4-yl)-2-phenoxypyridine following a procedure analogous to that described in Step 4 of Intermediate 4. LC (method 7): t_{R} = 0.98 min.

### Step 3: Methyl 2-((3S)-6-((1R)-4-(2-phenoxypyridin-3-yl)-2,3-dihydro-1H-inden-1-yloxy)-2,3-dihydrobenzofuran-3-yl)acetate

The title compound is prepared from ((1S)-4-(2-phenoxypyridin-3-yl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 9): t_{R} = 1.22 min.

### Intermediate 121

### Methyl 2-((3S)-6-((1R)-4-(2-(neopentyloxy)pyridin-3-yl)-2,3-dihydro-1H-inden-1-yloxy)-2,3-dihydrobenzofuran-3-yl)acetate

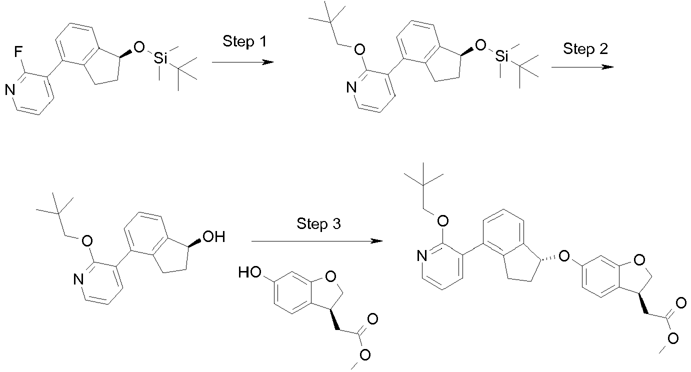

### Step 1: 3-((1S)-1-(tert-Butyldimethylsilyloxy)-2,3-dihydro-1H-inden-4-yl)-2-(neopentyloxy)pyrid ine

2,2-Dimethylpropan-1-ol (59 mg) is dissolved at 0°C in N-methylpyrrolidone (NMP, 2 mL), treated with NaH (27 mg, 55% dispersion in mineral oil) and stirred for 15 minutes. The mixture is warmed to room temperature, treated with a solution of 3-((1S)-1-(tert-Butyldimethylsilyloxy)-2,3-dihydro-1H-inden-4-yl)-2-fluoropyridine (200 mg) in N-methylpyrrolidone (NMP, 1 mL) and stirred for 12 hours at 75°C. 2,2-Dimethylpropan-1-ol (59 mg) and NaH (27 mg, 55% dispersion in mineral oil) are added and the mixture is stirred for 4 hours at 80°C. The mixture thus obtained is directly used in the next step.

### Step 2: (1+S)-4-(2-(Neopentyloxy)pyridin-3-yl)-2,3-dihydro-1H-inden-1-ol

The title compound is prepared from 3-((1S)-1-(tert-butyldimethylsilyloxy)-2,3-dihydro-1H-inden-4-yl)-2-(neopentyloxy)pyridine following a procedure analogous to that described in Step 4 of Intermediate 4. LC (method 7): t_{R} = 1.11 min.

### Step 3: Methyl 2-((3S)-6-((1R)-4-(2-(neopentyloxy)pyridin-3-yl)-2,3-dihydro-1H-inden-1-yloxy)-2,3-dihydrobenzofuran-3-yl)acetate

The title compound is prepared from (1S)-4-(2-(neopentyloxy)pyridin-3-yl)-2,3-dihydro-1H-inden-1-ol and (S)-methyl 2-(6-hydroxy-2,3-dihydrobenzofuran-3-yl)acetate following a procedure analogous to that described in Step 3 of Intermediate 12. LC (method 15): t_{R} = 1.32 min.

### Intermediate 122

### Methyl 2-((S)-6-((R)-4-(2,6-dimethyl-4-(1-methyl-6-oxo-1,6-dihydropyridazin-3-yl)phenyl)-7-fluoro-2,3-dihydro-1H-inden-1-yloxy)-2,3-dihydrobenzofuran-3-yl)acetate

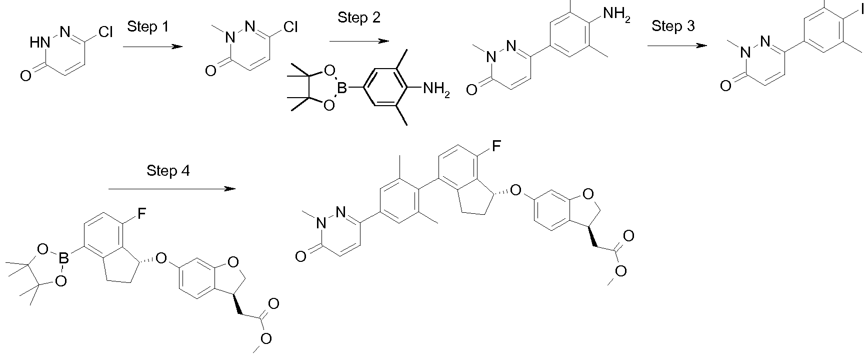

### Step 1: 6-Chloro-2-methylpyridazin-3(2H)-one

The title compound is prepared from 6-chloropyridazin-3(2H)-one following a procedure analogous to that described in Step 1 of Intermediate 106.

### Step 2: 6-(4-Amino-3,5-dimethylphenyl)-2-methylpyridazin-3(2H)-one

The title compound is prepared from 6-chloro-2-methylpyridazin-3(2H)-one and 2,6-dimethyl-4-(4,4,5,5-tetramethyl-1,3,2-dioxaborolan-2-yl)aniline following a procedure analogous to that described in Step 3 of Intermediate 5. The mixture is stirred for 12 hours at 60°C. LC (method 9): t_{R} = 0.78 min; Mass spectrum (ESI⁺): m/z = 230 [M+H]⁺.

### Step 3: 6-(4-Iodo-3,5-dimethylphenyl)-2-methylpyridazin-3(2H)-one

The title compound is prepared from 6-(4-amino-3,5-dimethylphenyl)-2-methylpyridazin-3(2H)-one following a procedure analogous to that described in Step 3 of Intermediate 105. LC (method 7): t_{R} = 1.08 min; Mass spectrum (ESI⁺): m/z = 341 [M+H]⁺.

### Step 4: Methyl 2-((S)-6-((R)-4-(2,6-dimethyl-4-(1-methyl-6-oxo-1,6-dihydropyridazin-3-yl)phenyl)-7-fluoro-2,3-dihydro-1H-inden-1-yloxy)-2,3-dihydrobenzofuran-3-yl)acetate

The title compound is prepared from 6-(4-iodo-3,5-dimethylphenyl)-2-methylpyri dazin-3(2H)-one and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 9): t_{R} = 1.18 min; Mass spectrum (ESI⁺): m/z = 555 [M+H]⁺.

### Intermediate 123

### Methyl 2-((3S)-6-((1R)-4-(2-(dimethylcarbamoyl)phenyl)-7-fluoro-2,3-dihydro-1H-inden-1-yloxy)-2,3-dihydrobenzofuran-3-yl)acetate

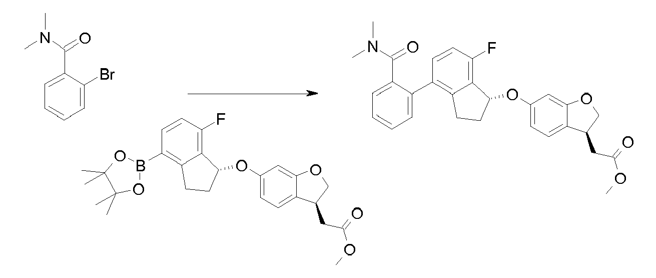

The title compound is prepared from 2-bromo-N,N-dimethylbenzamide and methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate following a procedure analogous to that described in Step 5 of Intermediate 12. LC (method 9): t_{R} = 1.14 min; Mass spectrum (ESI⁺): m/z = 490 [M+H]⁺.

### Intermediate 124

### 5-(2-Fluoro-6-iodophenyl)-1-methyl-1H-tetrazole and 5-(2-fluoro-6-iodophenyl)-2-methyl-2H-tetrazole

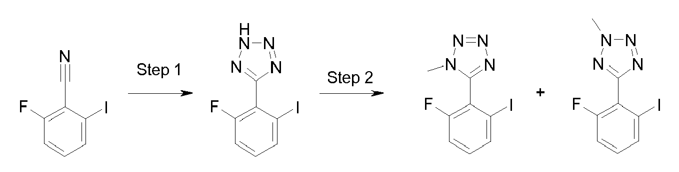

### Step 1: 5-(2-Fluoro-6-iodophenyl)-2H-tetrazole

In a microwave vial 2-fluoro-6-iodobenzonitrile (1 g) and azidotributyltin (1.12 mL) are dissolved in toluene (8.4 mL). The vial is sealed and the mixture is heated to 125°C for 72 hours. After cooling to room temperature the sovent is evaporated and the residue is purified by HPLC on reversed phase. Yield: 494 mg; Mass spectrum (ESI⁺): m/z = 291 [M+H]⁺.

### Step 2: 5-(2-Fluoro-6-iodophenyl)-1-methyl-1H-tetrazole and 5-(2-fluoro-6-iodophenyl)-2-methyl-2H-tetrazole

To a solution of 5-(2-fluoro-6-iodophenyl)-2H-tetrazole (986 mg) in N,N-dimethylformamide (3.4 mL) is added K₂CO₃ (530 mg) and Mel (295 µL) and the mixture is stirred for 12 hours at room temperature. Then the solvent is evaporated and the residue is partitioned between water and ethyl acetate. The aqueous phase is twice extracted with ethyl acetate and the combined organic phases are washed with brine, dried (Na₂SO₄). After concentration the residue is purified by HPLC on reversed phase to give the title compounds.
5-(2-fluoro-6-iodophenyl)-1-methyl-1H-tetrazole: Yield: 547 mg; Mass spectrum (ESI⁺): m/z = 305 [M+H]⁺.
5-(2-fluoro-6-iodophenyl)-2-methyl-2H-tetrazole: Yield: 457 mg; Mass spectrum (ESI⁺): m/z = 305 [M+H]⁺.

### Intermediate 125

### Methyl 2-((3S)-6-((1R)-7-fluoro-4-(3-fluoro-2-(1-methyl-1H-tetrazol-5-yl)phenyl)-2,3-dihydro-1H-inden-1-yloxy)-2,3-dihydrobenzofuran-3-yl)acetate

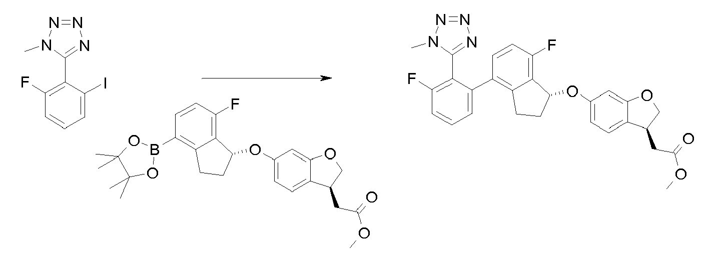

In a microwave vial methyl 2-((S)-6-((R)-7-fluoro-4-(4,4,5,5-tetramethyl-1,3,2-dioxaborolan-2-yl)-2,3-dihydro-1H-inden-1-yloxy)-2,3-dihydrobenzofuran-3-yl)acetate (100 mg), 5-(2-fluoro-6-iodophenyl)-1-methyl-1H-tetrazole (78 mg), K₃PO₄ (136 mg) are suspended in 1,4-dioxane (3 mL) and purged for 10 minutes with argon. [1,3-Bis(2,6-di-3-pentylphenyl)inidazol-2-ylidene](3-chloropyridyl)palladium(II) dichloride (8.5 mg) is added, the vial is sealed and the mixture is stirred at 100°C for 12 hours. After cooling to room temperature the solvents are evaporated and the product thus obtained is used directly in the next step.

### Intermediate 126

### 4-(4-Bromo-3,5-dimethyl-phenyl)-pyridine

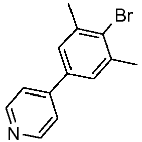

The title compound is prepared from 4-(4,4,5,5-tetramethyl-[1,3,2]dioxaborolan-2-yl)-pyridine and 2-bromo-5-iodo-1,3-dimethyl-benzene following a procedure analogous to that described in Step 1 of Intermediate 96. LC (method 9): t_{R} = 0.90 min; Mass spectrum (ESI⁺): m/z = 262/264 (Br) [M+H]⁺.

### Intermediate 127

### {(S)-6-[(R)-4-(2,6-Dimethyl-4-pyridin-4-yl-phenyl)-7-fluoro-indan-1-yloxy]-2,3-dihydrobenzofuran-3-yl}-acetic acid methyl ester

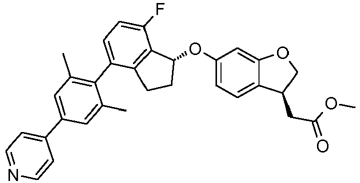

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-bromo-3,5-dimethyl-phenyl)-pyridine following a procedure analogous to that described in Step 5 of Intermediate 12. LC (method 9): t_{R} = 1.08 min; Mass spectrum (ESI⁺): m/z = 524 [M+H]⁺.

### Intermediate 128

### {(S)-6-[(R)-4-(2-Bromo-pyridin-3-yl)-7-fluoro-indan-1-yloxy]-2,3-dihydro-benzofuran-3-yl}-acetic acid methyl ester

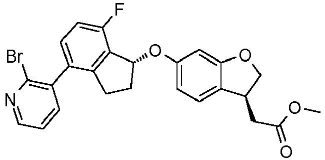

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-bromo-3-iodo-pyridine following a procedure analogous to that described in Step 1 of Intermediate 96. LC (method 9): t_{R} = 1.15 min; Mass spectrum (ESI⁺): m/z = 498/500 (Br) [M+H]⁺.

### Intermediate 129

### {(S)-6-[(R)-7-Fluoro-4-(2-furan-3-yl-pyridin-3-yl)-indan-1-yloxy]-2,3-dihydro-benzofuran-3-yl}-acetic acid methyl ester

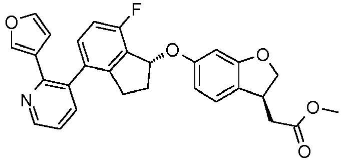

The title compound is prepared from {(*S*)-6-[(*R*)-4-(2-bromo-pyridin-3-yl)-7-fluoro-indan-1-yloxy]-2,3-dihydro-benzofuran-3-yl}-acetic acid methyl ester and furan-3-boronic acid following a procedure analogous to that described in Step 1 of Intermediate 96. LC (method 9): t_{R} = 1.04 min; Mass spectrum (ESI⁺): m/z = 486 [M+H]⁺.

### Intermediate 130

### {(S)-6-[(R)-7-Fluoro-4-(2-phenyl-pyridin-3-yl)-indan-1-yloxy]-2,3-dihydro-benzofuran-3-yl}-acetic acid methyl ester

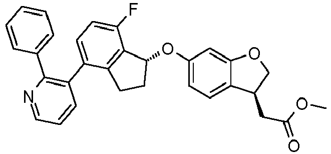

The title compound is prepared from {(*S*)-6-[(*R*)-4-(2-bromo-pyridin-3-yl)-7-fluoro-indan-1-yloxy]-2,3-dihydro-benzofuran-3-yl}-acetic acid methyl ester and phenylboronic acid following a procedure analogous to that described in Step 1 of Intermediate 96. LC (method 9): t_{R} = 1.15 min; Mass spectrum (ESI⁺): m/z = 496 [M+H]⁺.

### Intermediate 131

### 4-(4-Bromo-3,5-dimethyl-phenyl)-2-methyl-pyridine

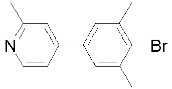

The title compound is prepared from 2-methyl-4-(4,4,5,5-tetramethyl-[1,3,2]dioxaborolan-2-yl)-pyridine and 2-bromo-5-iodo-1,3-dimethyl-benzene following a procedure analogous to that described in Step 1 of Intermediate 96. LC (method 9): t_{R} = 0.87 min; Mass spectrum (ESI⁺): m/z = 276/278 (Br) [M+H]⁺.

### Intermediate 132

### 4-(4-Bromo-3,5-dimethyl-phenyl)-1,3,5-trimethyl-pyrazole

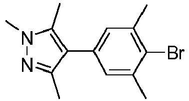

The title compound is prepared from 4-(4,4,5,5-tetramethyl-[1,3,2]dioxaborolan-2-yl)-1,3,5-trimethyl-pyrazole and 2-bromo-5-iodo-1,3-dimethyl-benzene following a procedure analogous to that described in Step 1 of Intermediate 96. LC (method 7): t_{R} = 1.09 min; Mass spectrum (ESI⁺): m/z = 293/295 (Br) [M+H]⁺.

### Intermediate 133

### {(S)-6-[(R)-4-(2,6-Dimethyl-4-(1,3,5-trimethyl-pyrazol-4-yl)-phenyl)-7-fluoro-indan-1-yloxy]-2,3-dihydro-benzofuran-3-yl}-acetic acid methyl ester

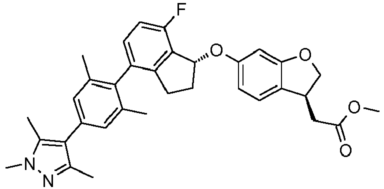

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-bromo-3,5-dimethyl-phenyl)-1,3,5-trimethyl-pyrazole following a procedure analogous to that described in Step 5 of Intermediate 12. LC (method 9): t_{R} = 1.24 min; Mass spectrum (ESI⁺): m/z = 555 [M+H]⁺.

### Intermediate 134

### {(S)-6-[(R)-7-Fluoro-4-(2-(3,6-dihydropyran-4-yl)-pyridin-3-yl)-indan-1-yloxy]-2,3-dihydro-benzofuran-3-yl}-acetic acid

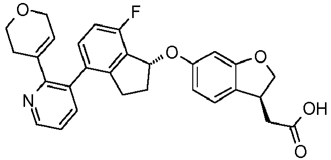

The methyl ester of the title compound is prepared from {(*S*)-6-[(*R*)-4-(2-bromo-pyridin-3-yl)-7-fluoro-indan-1-yloxy]-2,3-dihydro-benzofuran-3-yl}-acetic acid methyl ester and 4-(4,4,5,5-tetramethyl-[1,3,2]dioxaborolan-2-yl)-3,6-dihydropyran following a procedure analogous to that described in Step1 of Intermediate 96. Saponification of the methyl ester, {(*S*)-6-[(*R*)-7-fluoro-4-(3,6-dihydropyran-4-yl-pyridin-3-yl)-indan-1-yloxy]-2,3-dihydro-benzofuran-3-yl}-acetic acid methyl ester, gives the title compound following a procedure analogous to that described for Example 10. LC (method 9): t_{R} = 0.93 min; Mass spectrum (ESI⁺): m/z = 488 [M+H]⁺.

### Intermediate 135

### 3-(4-Bromo-3,5-dimethyl-phenyl)-5-methyl-pyridine

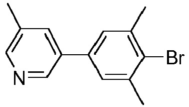

The title compound is prepared from 5-methyl-pyridine-3-boronic acid and 2-bromo-5-iodo-1,3-dimethyl-benzene following a procedure analogous to that described in Step1 of Intermediate 96. LC (method 9): t_{R} = 0.93 min; Mass spectrum (ESI⁺): m/z = 276/278 (Br) [M+H]⁺.

### Intermediate 136

### {(S)-6-[(R)-4-(2,6-Dimethyl-4-(5-methyl-pyridin-3-yl)-phenyl)-7-fluoro-indan-1-yloxy]-2,3-dihydro-benzofuran-3-yl}-acetic acid methyl ester

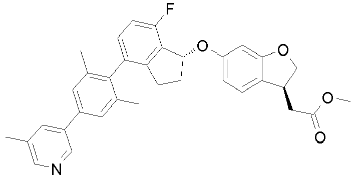

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-bromo-3,5-dimethyl-phenyl)-5-methyl-pyridine following a procedure analogous to that described in Step 5 of Intermediate 12.

### Intermediate 137

### 3-(4-Bromo-3,5-dimethyl-phenyl)-pyridine

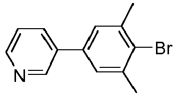

The title compound is prepared from pyridine-3-boronic acid and 2-bromo-5-iodo-1,3-dimethyl-benzene following a procedure analogous to that described in Step1 of Intermediate 96. LC (method 9): t_{R} = 0.99 min; Mass spectrum (ESI⁺): m/z = 262/264 (Br) [M+H]⁺.

### Intermediate 138

### {(S)-6-[(R)-4-(2,6-Dimethyl-4-pyridin-3-yl-phenyl)-7-fluoro-indan-1-yloxy]-2,3-dihydrobenzofuran-3-yl}-acetic acid methyl ester

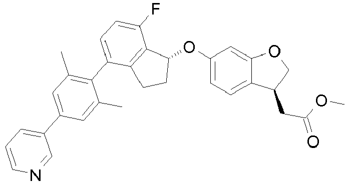

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-bromo-3,5-dimethyl-phenyl)-pyridine following a procedure analogous to that described in Step 5 of Intermediate 12. Mass spectrum (ESI⁺): m/z = 524 [M+H]⁺.

### Intermediate 139

### 2-(4-Bromo-3,5-dimethyl-phenyl)-5-methyl-pyrimidine

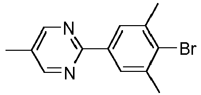

The title compound is prepared from 2-bromo-1,3-dimethyl-5-(4,4,5,5-tetramethyl-[1,3,2]dioxaborolan-2-yl)-benzene and 2-bromo-5-methyl-pyrimidine following a procedure analogous to that described in Step 1 of Intermediate 96. LC (method 7): t_{R} = 1.20 min; Mass spectrum (ESI⁺): m/z = 277/279 (Br) [M+H]⁺.

### Intermediate 140

### {(S)-6-[(R)-4-(2,6-Dimethyl-4-(5-methyl-pyrimidin-2-yl)-phenyl)-7-fluoro-indan-1-yloxy]-2,3-dihydro-benzofuran-3-yl}-acetic acid methyl ester

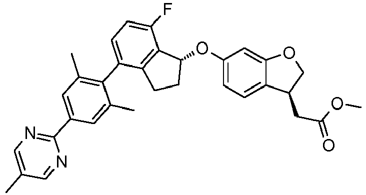

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-5-methyl-pyrimidine following a procedure analogous to that described in Step 5 of Intermediate 12. LC (method 9): t_{R} = 1.27 min; Mass spectrum (ESI⁺): m/z = 539 [M+H]⁺.

### Intermediate 141

### 2-(4-Bromo-3,5-dimethyl-phenyl)-5-methyl-pyrazine

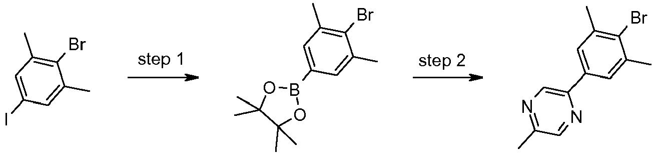

### Step 1: 2-bromo-1,3-dimethyl-5-(4,4,5,5-tetramethyl-[1,3,2]dioxaborolan-2-yl)-benzene

A flask charged with a stir bar, 2-bromo-5-iodo-1,3-dimethyl-benzene (1.0 g), bis-(pinacolato)-diboron (1.0 g), potassium acetate (1.1 g) and dimethyl sulfoxide (10 mL) is purged with argon for 5 min. [1,1'-Bis(diphenylphosphino)-ferrocene]-dichloropalladium(II) (0.26 g) is added at room temperature, and the mixture is stirred at 90 °C for 3 h. After cooling to room temperature, water is added and the resulting mixture is extracted with ethyl acetate. The combined extracts are dried (MgSO₄) and concentrated. The residue is chromatographed on reversed phase (HPLC; acetonitrile/water) to give the title compound. LC (method 9): t_{R} = 1.30 min; Mass spectrum (ESI⁺): m/z = 311/313 (Br) [M+H]⁺.

### Step 2: 2-(4-bromo-3,5-dimethyl-phenyl)-5-methyl-pyrazine

The title compound is prepared from 2-bromo-1,3-dimethyl-5-(4,4,5,5-tetramethyl-[1,3,2]dioxaborolan-2-yl)-benzene and 2-bromo-5-methyl-pyrazine following a procedure analogous to that described in Step 1 of Intermediate 96. Mass spectrum (ESI⁺): m/z = 277/279 (Br) [M+H]⁺.

### Intermediate 142

### {(S)-6-[(R)-4-(2,6-Dimethyl-4-(5-methyl-pyrazin-2-yl)-phenyl)-7-fluoro-indan-1-yloxy]-2,3-dihydro-benzofuran-3-yl}-acetic acid methyl ester

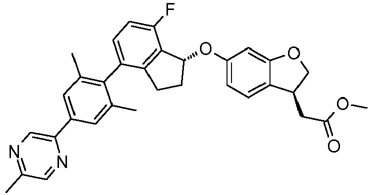

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-5-methyl-pyrazine following a procedure analogous to that described in Step 5 of Intermediate 12.

### Intermediate 143

### 4-(4-Bromo-3,5-dimethyl-phenyl)-2,6-dimethyl-pyrimidine

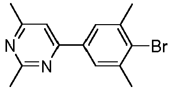

The title compound is prepared from 2-bromo-1,3-dimethyl-5-(4,4,5,5-tetramethyl-[1,3,2]dioxaborolan-2-yl)-benzene and 4-bromo-2,6-dimethyl-pyrimidine following a procedure analogous to that described in Step 1 of Intermediate 96. LC (method 7): t_{R} = 1.01 min; Mass spectrum (ESI⁺): m/z = 291/293 (Br) [M+H]⁺.

### Intermediate 144

### {(S)-6-[(R)-4-(2,6-Dimethyl-4-(2,6-dimethyl-pyrimidin-4-yl)-phenyl)-7-fluoro-indan-1-yloxy]-2,3-dihydro-benzofuran-3-yl}-acetic acid methyl ester

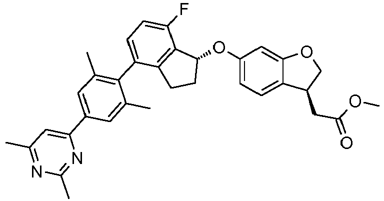

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-bromo-3,5-dimethyl-phenyl)-2,6-dimethyl-pyrimidine following a procedure analogous to that described in Step 5 of Intermediate 12. LC (method 9): t_{R} = 1.24 min; Mass spectrum (ESI⁺): m/z = 553 [M+H]⁺.

### Intermediate 145

### 4-(4-Bromo-3,5-dimethyl-phenyl)-2-methoxy-pyridine

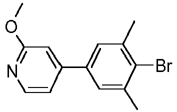

The title compound is prepared from 2-methoxy-pyridine-4-boronic acid and 2-bromo-5-iodo-1,3-dimethyl-benzene following a procedure analogous to that described in Step 1 of Intermediate 96. LC (method 9): t_{R} = 1.24 min; Mass spectrum (ESI⁺): m/z = 293/295 (Br) [M+H]⁺.

### Intermediate 146

### {(S)-6-[(R)-4-(2,6-Dimethyl-4-(2-methoxy-pyridin-4-yl)-phenyl)-7-fluoro-indan-1-yloxy]-2,3-dihydro-benzofuran-3-yl}-acetic acid methyl ester

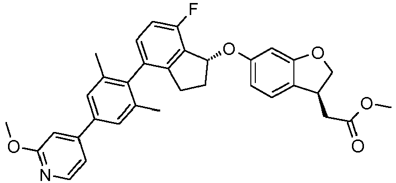

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-bromo-3,5-dimethyl-phenyl)-2-methoxy-pyridine following a procedure analogous to that described in Step 5 of Intermediate 12. LC (method 9): t_{R} = 1.30 min; Mass spectrum (ESI⁺): m/z = 554 [M+H]⁺.

### Intermediate 147

### 5-(4-Bromo-3,5-dimethyl-phenyl)-2-methyl-pyridine

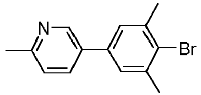

The title compound is prepared from 2-methyl-pyridine-5-boronic acid and 2-bromo-5-iodo-1,3-dimethyl-benzene following a procedure analogous to that described in Step 1 of Intermediate 96. LC (method 9): t_{R} = 0.93 min; Mass spectrum (ESI⁺): m/z = 276/278 (Br) [M+H]⁺.

### Intermediate 148

### {(S)-6-[(R)-4-(2,6-Dimethyl-4-(6-methyl-pyridin-3-yl)-phenyl)-7-fluoro-indan-1-yloxy]-2,3-dihydro-benzofuran-3-yl}-acetic acid methyl ester

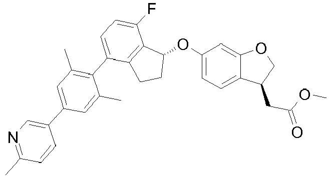

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 5-(4-bromo-3,5-dimethyl-phenyl)-2-methyl-pyridine following a procedure analogous to that described in Step 5 of Intermediate 12. LC (method 9): t_{R} = 1.12 min; Mass spectrum (ESI⁺): m/z = 538 [M+H]⁺.

### Intermediate 149

### 2-(4-Bromo-3,5-dimethyl-phenyl)-6-methyl-pyrazine

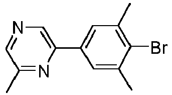

The title compound is prepared from 2-bromo-1,3-dimethyl-5-(4,4,5,5-tetramethyl-[1,3,2]dioxaborolan-2-yl)-benzene and 2-bromo-6-methyl-pyrazine following a procedure analogous to that described in Step 1 of Intermediate 96. LC (method 9): t_{R} = 1.04 min; Mass spectrum (ESI⁺): m/z = 277/279 (Br) [M+H]⁺.

### Intermediate 150

### {(S)-6-[(R)-4-(2,6-Dimethyl-4-(6-methyl-pyrazin-2-yl)-phenyl)-7-fluoro-indan-1-yloxy]-2,3-dihydro-benzofuran-3-yl}-acetic acid methyl ester

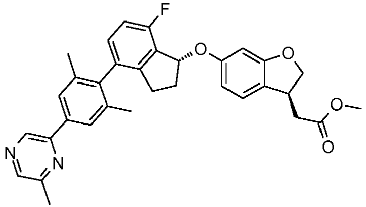

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-6-methyl-pyrazine following a procedure analogous to that described in Step 5 of Intermediate 12. LC (method 26): t_{R} = 0.93 min; Mass spectrum (ESI⁺): m/z = 539 [M+H]⁺.

### Intermediate 151

### 2-(4-Bromo-3,5-dimethyl-phenyl)-4-methyl-pyrimidine

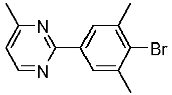

The title compound is prepared from 2-bromo-1,3-dimethyl-5-(4,4,5,5-tetramethyl-[1,3,2]dioxaborolan-2-yl)-benzene and 2-bromo-4-methyl-pyrimidine following a procedure analogous to that described in Step 1 of Intermediate 96. LC (method 9): t_{R} = 1.08 min; Mass spectrum (ESI⁺): m/z = 277/279 (Br) [M+H]⁺.

### Intermediate 152

### {(S)-6-[(R)-4-(2,6-Dimethyl-4-(4-methyl-pyrimidin-2-yl)-phenyl)-7-fluoro-indan-1-yloxy]-2,3-dihydro-benzofuran-3-yl}-acetic acid methyl ester

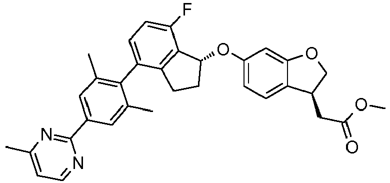

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-4-methyl-pyrimidine following a procedure analogous to that described in Step 5 of Intermediate 12. LC (method 26): t_{R} = 0.96 min; Mass spectrum (ESI⁺): m/z = 539 [M+H]⁺.

### Intermediate 153

### 3-(4-Bromo-3,5-dimethyl-phenyl)-6-methyl-pyridazine

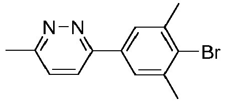

The title compound is prepared from 2-bromo-1,3-dimethyl-5-(4,4,5,5-tetramethyl-[1,3,2]dioxaborolan-2-yl)-benzene and 3-bromo-6-methyl-pyridazine following a procedure analogous to that described in Step1 of Intermediate 96. LC (method 9): t_{R} = 1.11 min; Mass spectrum (ESI⁺): m/z = 277/279 (Br) [M+H]⁺.

### Intermediate 154

### {(S)-6-[(R)-4-(2,6-Dimethyl-4-(6-methyl-pyridazin-3-yl)-phenyl)-7-fluoro-indan-1-yloxy]-2,3-dihydro-benzofuran-3-yl}-acetic acid methyl ester

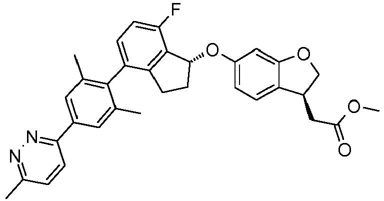

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-bromo-3,5-dimethyl-phenyl)-6-methyl-pyridazine following a procedure analogous to that described in Step 5 of Intermediate 12. LC (method 26): t_{R} = 0.75 min; Mass spectrum (ESI⁺): m/z = 539 [M+H]⁺.

### Intermediate 155

### 4-(4-Chloro-3,5-dimethyl-phenyl)-2,6-dimethyl-pyridine

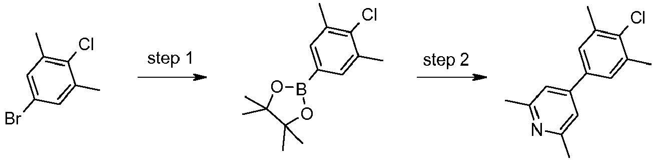

### Step 1: 2-chloro-1,3-dimethyl-5-(4,4,5,5-tetramethyl-[1,3,2]dioxaborolan-2-yl)-benzene

The title compound is prepared from 5-bromo-2-chloro-1,3-dimethyl-benzene and bis-(pinacolato)-diboron following a procedure analogous to that described in Step 1 of Intermediate **141.** LC (method 7): t_{R} = 1.10 min; Mass spectrum (ESI⁺): m/z = 552 [M+H]⁺.

### Step 2: 4-(4-chloro-3,5-dimethyl-phenyl)-2,6-dimethyl-pyridine

The title compound is prepared from 2-chloro-1,3-dimethyl-5-(4,4,5,5-tetramethyl-[1,3,2]dioxaborolan-2-yl)-benzene and 4-bromo-2,6-dimethyl-pyridine following a procedure analogous to that described in Step 1 of Intermediate 96. Mass spectrum (ESI⁺): m/z = 246/248 (CI) [M+H]⁺.

### Intermediate 156

### {(S)-6-[(R)-4-(2,6-Dimethyl-4-(2,6-dimethyl-pyridin-4-yl)-phenyl)-7-fluoro-indan-1-yloxy]-2,3-dihydro-benzofuran-3-yl}-acetic acid methyl ester

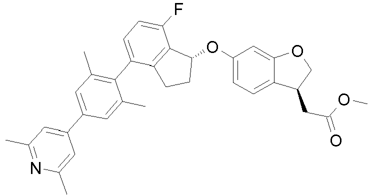

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-chloro-3,5-dimethyl-phenyl)-2,6-dimethyl-pyridine following a procedure analogous to that described in Step 5 of Intermediate 12. LC (method 7): t_{R} = 1.10 min; Mass spectrum (ESI⁺): m/z = 552 [M+H]⁺.

### Intermediate 157

### 2-(4-Bromo-3,5-dimethyl-phenyl)-pyrazine

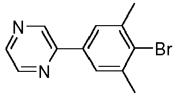

The title compound is prepared from 2-bromo-1,3-dimethyl-5-(4,4,5,5-tetramethyl-[1,3,2]dioxaborolan-2-yl)-benzene and 2-iodo-pyrazine following a procedure analogous to that described in Step 1 of Intermediate 96. LC (method 9): t_{R} = 1.12 min; Mass spectrum (ESI⁺): m/z = 263/265 (Br) [M+H]⁺.

### Intermediate 158

### {(S)-6-[(R)-4-(2,6-Dimethyl-4-pyrazin-2-yl)-phenyl)-7-fluoro-indan-1-yoloxy]-2,3-dihydro-benzofuran-3-yl}-acetic acid methyl ester

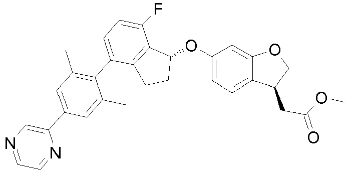

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-pyrazine following a procedure analogous to that described in Step 5 of Intermediate 12. LC (method 9): t_{R} = 1.31 min; Mass spectrum (ESI⁺): m/z = 525 [M+H]⁺.

### Intermediate 159

### 2-(4-Chloro-3,5-dimethyl-phenyl)-5-cyclopropyl-pyrazine

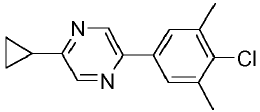

The title compound is prepared from 2-chloro-1,3-dimethyl-5-(4,4,5,5-tetramethyl-[1,3,2]dioxaborolan-2-yl)-benzene and 2-bromo-5-cyclopropyl-pyrazine following a procedure analogous to that described in Step1 of Intermediate 96. LC (method 11): t_{R} = 1.28 min; Mass spectrum (ESI⁺): m/z = 259/261 (CI) [M+H]⁺.

### Intermediate 160

### {(S)-6-[(R)-4-(2,6-Dimethyl-4-(5-cyclopropyl-pyrazin-2-yl)-phenyl)-7-fluoro-indan-1-yloxy]-2,3-dihydro-benzofuran-3-yl}-acetic acid methyl ester

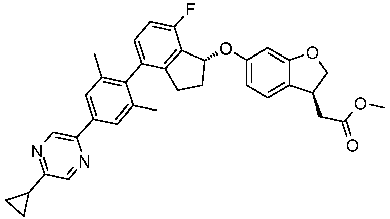

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-chloro-3,5-dimethyl-phenyl)-5-cyclopropyl-pyrazine following a procedure analogous to that described in Step 5 of Intermediate 12. LC (method 7): t_{R} = 1.31 min; Mass spectrum (ESI⁺): m/z = 565 [M+H]⁺.

### Intermediate 161

### {(S)-6-[(R)-7-Fluoro-4-(2,6-dimethyl-phenyl)-pyridin-3-yl)-indan-1-yloxy]-2,3-dihydro-benzofuran-3-yl}-acetic acid methyl ester

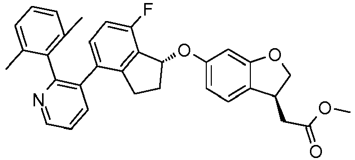

The title compound is prepared from {(*S*)-6-[(*R*)-4-(2-bromo-pyridin-3-yl)-7-fluoro-indan-1-yloxy]-2,3-dihydro-benzofuran-3-yl}-acetic acid methyl ester and 2,6-dimethyl-phenylboronic acid following a procedure analogous to that described in Step 5 of Intermediate 12.

### Intermediate 162

### 3-(4-Chloro-3,5-dimethyl-phenyl)-6-ethyl-pyridazine

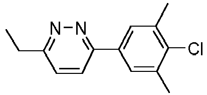

The title compound is prepared from 2-cloro-1,3-dimethyl-5-(4,4,5,5-tetramethyl-[1,3,2]dioxaborolan-2-yl)-benzene and 3-bromo-6-ethyl-pyridazine following a procedure analogous to that described in Step 1 of Intermediate 96. LC (method 11): t_{R} = 1.00 min; Mass spectrum (ESI⁺): m/z = 247/249 (CI) [M+H]⁺.

### Intermediate 163

### {(S)-6-[(R)-4-(2,6-Dimethyl-4-(6-ethyl-pyridazin-3-yl)-phenyl)-7-fluoro-indan-1-yloxy]-2,3-dihydro-benzofuran-3-yl}-acetic acid methyl ester

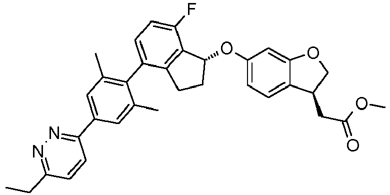

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-chloro-3,5-dimethyl-phenyl)-6-ethyl-pyridazine following a procedure analogous to that described in Step 5 of Intermediate 12. LC (method 7): t_{R} = 1.14 min; Mass spectrum (ESI⁺): m/z = 553 [M+H]⁺.

### Intermediate 164

### 5-(4-Chloro-3,5-dimethyl-phenyl)-2-methoxy-pyridine

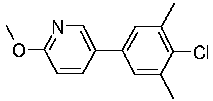

The title compound is prepared from 2-methoxy-pyridine-5-boronic acid and 5-bromo-2-chloro-1,3-dimethyl-benzene following a procedure analogous to that described in Step 1 of Intermediate 96. LC (method 7): t_{R} = 1.21 min; Mass spectrum (ESI⁺): m/z = 248/250 (CI) [M+H]⁺.

### Intermediate 165

### {(S)-6-[(R)-4-(2,6-Dimethyl-4-(6-methoxy-pyridin-3-yl)-phenyl)-7-fluoro-indan-1-yloxy]-2,3-dihydro-benzofuran-3-yl}-acetic acid methyl ester

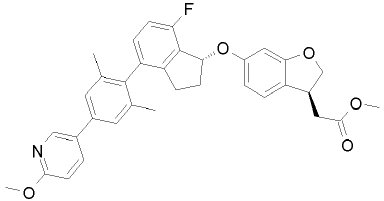

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 5-(4-chloro-3,5-dimethyl-phenyl)-2-methoxy-pyridine following a procedure analogous to that described in Step 5 of Intermediate 12. LC (method 7): t_{R} = 1.29 min; Mass spectrum (ESI⁺): m/z = 554 [M+H]⁺.

### Intermediate 166

### 3-(4-Chloro-3,5-dimethyl-phenyl)-5-methoxy-pyridazine

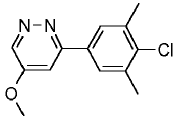

The title compound is prepared from 2-chloro-1,3-dimethyl-5-(4,4,5,5-tetramethyl-[1,3,2]dioxaborolan-2-yl)-benzene and 3-chloro-5-methoxy-pyridazine following a procedure analogous to that described in Step 1 of Intermediate 96. LC (method 11): t_{R} = 0.93 min; Mass spectrum (ESI⁺): m/z = 249/251 (CI) [M+H]⁺.

### Intermediate 167

### {(S)-6-[(R)-4-(2,6-Dimethyl-4-(5-methoxy-pyridazin-3-yl)-phenyl)-7-fluoro-indan-1-yloxy]-2,3-dihydro-benzofuran-3-yl}-acetic acid methyl ester

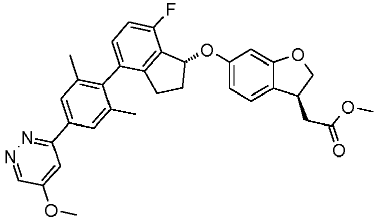

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-chloro-3,5-dimethyl-phenyl)-5-methoxy-pyridazine following a procedure analogous to that described in Step 5 of Intermediate 12. LC (method 11): t_{R} = 1.10 min; Mass spectrum (ESI⁺): m/z = 555 [M+H]⁺.

### Intermediate 168

### 5-(4-Chloro-3,5-dimethyl-phenyl)-3-methyl-pyridazine

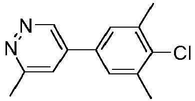

The title compound is prepared from 3-methyl-5-(4,4,5,5-tetramethyl-[1,3,2]dioxaborolan-2-yl)-pyridazine and 5-bromo-2-chloro-1,3-dimethyl-benzene following a procedure analogous to that described in Step 1 of Intermediate 96. LC (method 11): t_{R} = 0.91 min; Mass spectrum (ESI⁺): m/z = 233/235 (CI) [M+H]⁺.

### Intermediate 169

### {(S)-6-[(R)-4-(2,6-Dimethyl-4-(6-methyl-pyridazin-4-yl)-phenyl)-7-fluoro-indan-1-yloxy]-2,3-dihydro-benzofuran-3-yl}-acetic acid methyl ester

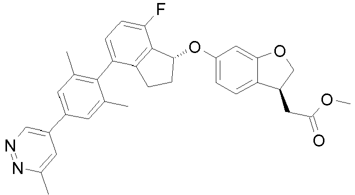

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 5-(4-chloro-3,5-dimethyl-phenyl)-3-methyl-pyridazine following a procedure analogous to that described in Step 5 of Intermediate 12. LC (method 11): t_{R} = 1.09 min; Mass spectrum (ESI⁺): m/z = 539 [M+H]⁺.

### Intermediate 170

### 4-(4-Chloro-3,5-dimethyl-phenyl)-1,2-dimethyl-imidazole

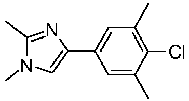

The title compound is prepared from 2-chloro-1,3-dimethyl-5-(4,4,5,5-tetramethyl-[1,3,2]dioxaborolan-2-yl)-benzene and 4-bromo-1,2-dimethyl-imidazole following a procedure analogous to that described in Step 1 of Intermediate 96.

### Intermediate 171

### {(S)-6-[(R)-4-(2,6-Dimethyl-4-(1,2-dimethyl-imidazol-4-yl)-phenyl)-7-fluoro-indan-1-yloxy]-2,3-dihydro-benzofuran-3-yl}-acetic acid methyl ester

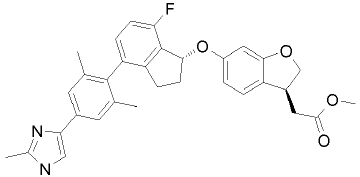

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-chloro-3,5-dimethyl-phenyl)-1,2-dimethyl-imidazole following a procedure analogous to that described in Step 5 of Intermediate 12. LC (method 7): t_{R} = 1.06 min; Mass spectrum (ESI⁺): m/z = 541 [M+H]⁺.

### Intermediate 172

### 2-(4-Bromo-3,5-dimethyl-phenyl)-thiazole

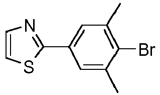

A mixture of 2-bromo-5-iodo-1,3-dimethyl-benzene (0.28 g), 2-thiazolylzinc bromide (0.5 mol/L in tetrahydrofuran; 1.9 mL), tetrakis(triphenylphosphine)palladium(0) (52 mg), and tetrahydrofuran (5 mL) under argon atmosphere is stirred at 100 °C for 3 h. After cooling to room temperature, water is added and the resulting mixture is extracted with ethyl acetate. The solvent is evaporated and the residue is chromatographed on silica gel (cyclohexane/ethyl acetate) to give the title compound. LC (method 9): t_{R} = 1.19 min; Mass spectrum (ESI⁺): m/z = 268/270 (Br) [M+H]⁺.

### Intermediate 173

### {(S)-6-[(R)-4-(2,6-Dimethyl-4-thiazol-2-yl-phenyl)-7-fluoro-indan-1-yloxy]-2,3-dihydrobenzofuran-3-yl}-acetic acid methyl ester

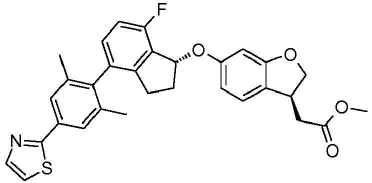

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-thiazole following a procedure analogous to that described in Step 5 of Intermediate 12. LC (method 7): t_{R} = 1.29 min; Mass spectrum (ESI⁺): m/z = 530 [M+H]⁺.

### Intermediate 174

### 4-(4-Chloro-3,5-dimethyl-phenyl)-1-methyl-imidazole

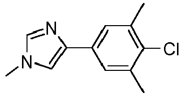

The title compound is prepared from 5-bromo-2-chloro-1,3-dimethyl-benzene and 1-methyl-4-(4,4,5,5-tetramethyl-[1,3,2]dioxaborolan-2-yl)-imidazole following a procedure analogous to that described in Step 1 of Intermediate 96.

### Intermediate 175

### {(S)-6-[(R)-4-(2,6-Dimethyl-4-(1-methyl-imidazol-4-yl)-phenyl)-7-fluoro-indan-1-yloxy]-2,3-dihydro-benzofuran-3-yl}-acetic acid methyl ester

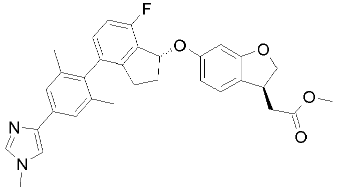

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-chloro-3,5-dimethyl-phenyl)-1-methyl-imidazole following a procedure analogous to that described in Step 5 of Intermediate 12.

### Intermediate 176

### 4-Bromo-N-hydroxy-3,5-dimethyl-benzamidine

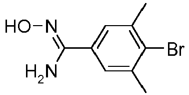

A mixture of 4-bromo-3,5-dimethyl-benzonitrile (1.50 g), hydroxylamine hydrochloride (0.90 g), triethylamine (1.8 mL) and ethanol (30 mL) is stirred at reflux temperature for 4 h. After cooling to room temperature, the mixture is concentrated, the residue is taken up in water, and the resulting mixture is extracted with ethyl acetate. The combined extract is washed with brine, dried (Na₂SO₄), and concentrated. The residue is chromatographed on silica gel (cyclohexane/ethyl acetate 7:3→3:7) to give the title compound. LC (method 11): t_{R} = 0.73 min; Mass spectrum (ESI⁺): m/z = 243/245 (Br) [M+H]⁺.

### Intermediate 177

### 3-(4-Bromo-3,5-dimethyl-phenyl)-5-methyl-[1,2,4]oxadiazole

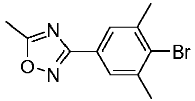

Acetic anhydride (0.35 mL) is added to a solution of 4-bromo-N-hydroxy-3,5-dimethyl-benzamidine (0.30 g) in collidine (3 mL) at room temperature. The solution is stirred at room temperature for 1 h and then at 120 °C for 3 h. After cooling to room temperature, the mixture is concentrated, the residue is taken up in water and acetonitrile, and the resulting mixture is filtered. The filtrate is chromatographed on reversed phase (HPLC; acetonitrile/water/trifluoroacetic acid) to give the title compound. LC (method 11): t_{R} = 1.20 min; Mass spectrum (ESI⁺): m/z = 267/269 (Br) [M+H]⁺.

### Intermediate 178

### {(S)-6-[(R)-4-(2,6-Dimethyl-4-(5-methyl-[1,2,4]oxadiazol-3-yl)-phenyl)-7-fluoro-indan-1-yloxy]-2,3-dihydro-benzofuran-3-yl}-acetic acid methyl ester

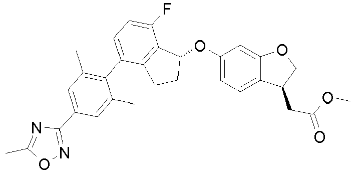

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-bromo-3,5-dimethyl-phenyl)-5-methyl-[1,2,4]oxadiazole following a procedure analogous to that described in Step 5 of Intermediate 12. LC (method 11): t_{R} = 1.28 min; Mass spectrum (ESI⁺): m/z = 551 [M+Na]⁺.

### Intermediate 179

### 2-(4-Bromo-3,5-dimethyl-phenyl)-5-methoxy-pyridine

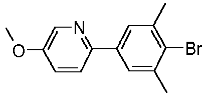

The title compound is prepared from potassium 5-methoxy-pyridine-2-trifluoroborate and 2-bromo-5-iodo-1,3-dimethyl-benzene following a procedure analogous to that described in Step 1 of Intermediate 96. LC (method 8): t_{R} = 0.72 min; Mass spectrum (ESI⁺): m/z = 292/294 (Br) [M+H]⁺.

### Intermediate 180

### {(S)-6-[(R)-4-(2,6-Dimethyl-4-(5-methoxy-pyridin-2-yl)-phenyl)-7-fluoro-indan-1-yloxy]-2,3-dihydro-benzofuran-3-yl}-acetic acid methyl ester

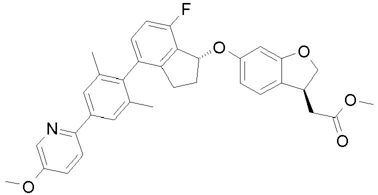

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-5-methoxy-pyridine following a procedure analogous to that described in Step 5 of Intermediate 12. LC (method 8): t_{R} = 0.98 min; Mass spectrum (ESI⁺): m/z = 554 [M+H]⁺.

### Intermediate 181

### 3-(4-Bromo-3,5-dimethyl-phenyl)-5-(2-hydroxy-prop-2-yl)-[1,2,4]oxadiazole

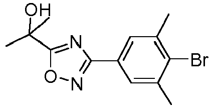

2-(Benzotriazol-1-yl)-1,1,3,3-tetramethyluronium tetrafluoroborate (TBTU; 0.55 g) is added to a solution of 2-hydroxy-isobutyric acid (0.18 g) and N,N-diisopropylethylamine (1.4 mL) in N,N-dimethylformamide (5 mL) at room temperature. The solution is stirred at room temperature for 10 min prior to the addition of 4-bromo-N-hydroxy-3,5-dimethyl-benzamidine (0.40 g). The solution is stirred at room temperature for another 10 min and then at 110 °C overnight. After cooling to room temperature, water is added and the resulting mixture is extracted with ethyl acetate. The combined extract is washed with brine, dried (MgSO₄), and concentrated. The residue is chromatographed on reversed phase (HPLC; acetonitrile/water/trifluoroacetic acid) to give the title compound. LC (method 11): t_{R} = 1.16 min; Mass spectrum (ESI⁺): m/z = 313/315 (Br) [M+H]⁺.

### Intermediate 182

### {(S)-6-[(R)-4-(2,6-Dimethyl-4-(5-[2-hydroxy-prop-2-yl]-[1,2,4]oxadiazol-3-yl)-phenyl)-7-fluoro-indan-1-yloxy]-2,3-dihydro-benzofuran-3-yl}-acetic acid methyl ester

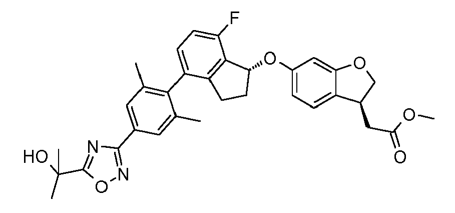

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 3-(4-bromo-3,5-dimethyl-phenyl)-5-(2-hydroxy-prop-2-yl)-[1,2,4]oxadiazole following a procedure analogous to that described in Step 5 of Intermediate 12. LC (method 11): t_{R} = 1.25 min; Mass spectrum (ESI⁺): m/z = 573 [M+H]⁺.

### Intermediate 183

### 2-(4-Bromo-3,5-dimethyl-phenyl)-pyridine

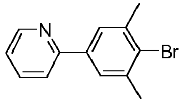

The title compound is prepared from 2-bromo-1,3-dimethyl-5-(4,4,5,5-tetramethyl-[1,3,2]dioxaborolan-2-yl)-benzene and 2-bromo-pyridine following a procedure analogous to that described in Step 1 of Intermediate 96. LC (method 7): t_{R} = 0.91 min; Mass spectrum (ESI⁺): m/z = 262/264 (Br) [M+H]⁺.

### Intermediate 184

### {(S)-6-[(R)-4-(2,6-Dimethyl-4-pyridin-2-yl-phenyl)-7-fluoro-indan-1-yloxy]-2,3-dihydrobenzofuran-3-yl}-acetic acid methyl ester

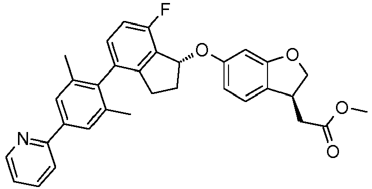

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-pyridine following a procedure analogous to that described in Step 5 of Intermediate 12. LC (method 7): t_{R} = 1.09 min; Mass spectrum (ESI⁺): m/z = 524 [M+H]⁺.

### Intermediate 185

### 2-(4-Bromo-3,5-dimethyl-phenyl)-3-methyl-pyridine

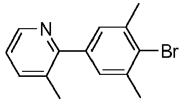

The title compound is prepared from 2-bromo-1,3-dimethyl-5-(4,4,5,5-tetramethyl-[1,3,2]dioxaborolan-2-yl)-benzene and 2-bromo-3-methyl-pyridine following a procedure analogous to that described in Step 1 of Intermediate 96. LC (method 7): t_{R} = 0.88 min; Mass spectrum (ESI⁺): m/z = 276/278 (Br) [M+H]⁺.

### Intermediate 186

### {(S)-6-[(R)-4-(2,6-Dimethyl-4-(3-methyl-pyridin-2-yl)-phenyl)-7-fluoro-indan-1-yloxy]-2,3-dihydro-benzofuran-3-yl}-acetic acid methyl ester

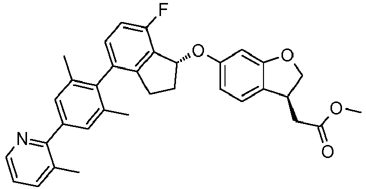

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-3-methyl-pyridine following a procedure analogous to that described in Step 5 of Intermediate 12. LC (method 7): t_{R} = 1.07 min; Mass spectrum (ESI⁺): m/z = 538 [M+H]⁺.

### Intermediate 187

### 2-(4-Bromo-3,5-dimethyl-phenyl)-6-methyl-pyridine

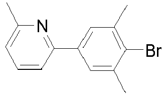

The title compound is prepared from 2-bromo-1,3-dimethyl-5-(4,4,5,5-tetramethyl-[1,3,2]dioxaborolan-2-yl)-benzene and 2-bromo-6-methyl-pyridine following a procedure analogous to that described in Step 1 of Intermediate 96. LC (method 7): t_{R} = 0.88 min; Mass spectrum (ESI⁺): m/z = 276/278 (Br) [M+H]⁺.

### Intermediate 188

### {(S)-6-[(R)-4-(2,6-Dimethyl-4-(6-methyl-pyridin-2-yl)-phenyl)-7-fluoro-indan-1-yloxy]-2,3-dihydro-benzofuran-3-yl}-acetic acid methyl ester

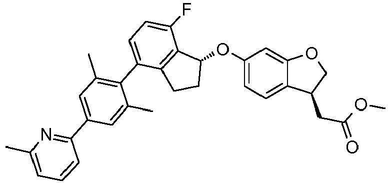

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-6-methyl-pyridine following a procedure analogous to that described in Step 5 of Intermediate 12. LC (method 7): t_{R} = 1.07 min; Mass spectrum (ESI⁺): m/z = 538 [M+H]⁺.

### Intermediate 189

### 2-(4-Bromo-3,5-dimethyl-phenyl)-4-methyl-pyridine

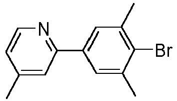

The title compound is prepared from 2-bromo-1,3-dimethyl-5-(4,4,5,5-tetramethyl-[1,3,2]dioxaborolan-2-yl)-benzene and 2-bromo-4-methyl-pyridine following a procedure analogous to that described in Step 1 of Intermediate 96. LC (method 7): t_{R} = 0.90 min; Mass spectrum (ESI⁺): m/z = 276/278 (Br) [M+H]⁺.

### Intermediate 190

### {(S)-6-[(R)-4-(2,6-Dimethyl-4-(4-methyl-pyridin-2-yl)-phenyl)-7-fluoro-indan-1-yloxy]-2,3-dihydro-benzofuran-3-yl}-acetic acid methyl ester

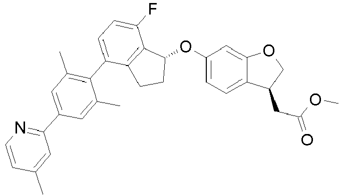

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-bromo-3,5-dimethyl-phenyl)-4-methyl-pyridine following a procedure analogous to that described in Step 5 of Intermediate 12. LC (method 7): t_{R} = 1.07 min; Mass spectrum (ESI⁺): m/z = 538 [M+H]⁺.

### Intermediate 191

### 2-(4-Chloro-3,5-dimethyl-phenyl)-4,6-dimethyl-pyridine

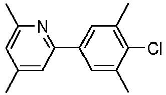

The title compound is prepared from 2-chloro-1,3-dimethyl-5-(4,4,5,5-tetramethyl-[1,3,2]dioxaborolan-2-yl)-benzene and 2-bromo-4,6-dimethyl-pyridine following a procedure analogous to that described in Step 1 of Intermediate 96. LC (method 7): t_{R} = 0.89 min; Mass spectrum (ESI⁺): m/z = 246/248 (CI) [M+H]⁺.

### Intermediate 192

### {(S)-6-[(R)-4-(2,6-Dimethyl-4-(4,6-dimethyl-pyridin-2-yl)-phenyl)-7-fluoro-indan-1-yloxy]-2,3-dihydro-benzofuran-3-yl}-acetic acid methyl ester

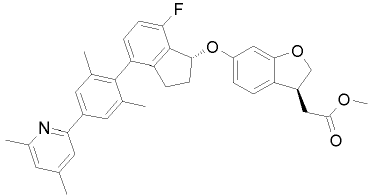

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-chloro-3,5-dimethyl-phenyl)-4,6-methyl-pyridine following a procedure analogous to that described in Step 5 of Intermediate 12. LC (method 7): t_{R} = 1.02 min; Mass spectrum (ESI⁺): m/z = 552 [M+H]⁺.

### Intermediate 193

### 2-(4-Chloro-3,5-dimethyl-phenyl)-1,4-dimethyl-imidazole

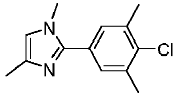

The title compound is prepared from 2-chloro-1,3-dimethyl-5-(4,4,5,5-tetramethyl-[1,3,2]dioxaborolan-2-yl)-benzene and 2-bromo-1,4-dimethyl-imidazole following a procedure analogous to that described in Step 1 of Intermediate 96. LC (method 7): t_{R} = 0.82 min; Mass spectrum (ESI⁺): m/z = 235/237 (CI) [M+H]⁺.

### Intermediate 194

### {(S)-6-[(R)-4-(2,6-Dimethyl-4-(1,4-dimethyl-imidazol-2-yl)-phenyl)-7-fluoro-indan-1-yloxy]-2,3-dihydro-benzofuran-3-yl}-acetic acid methyl ester

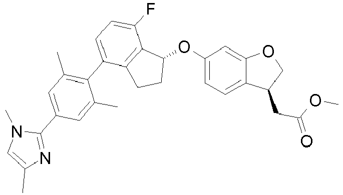

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-chloro-3,5-dimethyl-phenyl)-1,4-dimethyl-imidazole following a procedure analogous to that described in Step 5 of Intermediate 12. LC (method 7): t_{R} = 0.98 min; Mass spectrum (ESI⁺): m/z = 541 [M+H]⁺.

### Intermediate 195

### 2-(4-Chloro-3,5-dimethyl-phenyl)-5-methyl-pyridine

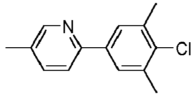

The title compound is prepared from 2-chloro-1,3-dimethyl-5-(4,4,5,5-tetramethyl-[1,3,2]dioxaborolan-2-yl)-benzene and 2-bromo-5-methyl-pyridine following a procedure analogous to that described in Step 1 of Intermediate 96. LC (method 7): t_{R} = 0.90 min; Mass spectrum (ESI⁺): m/z = 232/234 (CI) [M+H]⁺.

### Intermediate 196

### {(S)-6-[(R)-4-(2,6-Dimethyl-4-(5-methyl-pyridin-2-yl)-phenyl)-7-fluoro-indan-1-yloxy]-2,3-dihydro-benzofuran-3-yl}-acetic acid methyl ester

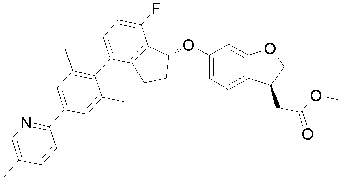

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-chloro-3,5-dimethyl-phenyl)-5-methyl-pyridine following a procedure analogous to that described in Step 5 of Intermediate 12. LC (method 7): t_{R} = 1.08 min; Mass spectrum (ESI⁺): m/z = 538 [M+H]⁺.

### Intermediate 197

### 2-(4-Chloro-3,5-dimethyl-phenyl)-1-methyl-imidazole

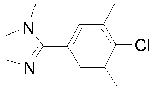

The title compound is prepared from 2-chloro-1,3-dimethyl-5-(4,4,5,5-tetramethyl-[1,3,2]dioxaborolan-2-yl)-benzene and 2-iodo-1-methyl-imidazole following a procedure analogous to that described in Step 1 of Intermediate 96. LC (method 7): t_{R} = 0.80 min; Mass spectrum (ESI⁺): m/z = 221/223 (CI) [M+H]⁺.

### Intermediate 198

### {(S)-6-[(R)-4-(2,6-Dimethyl-4-(1-methyl-imidazol-2-yl)-phenyl)-7-fluoro-indan-1-yloxy]-2,3-dihydro-benzofuran-3-yl}-acetic acid methyl ester

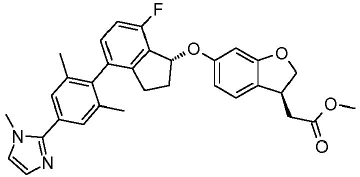

The title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 2-(4-chloro-3,5-dimethyl-phenyl)-1-methyl-imidazole following a procedure analogous to that described in Step 5 of Intermediate 12. LC (method 7): t_{R} = 1.02 min; Mass spectrum (ESI⁺): m/z = 527 [M+H]⁺.

### Intermediate 199

### Methyl 2-((S)-6-((R)-4-(4-(cyclopropylmethoxy)-2,6-dimethylphenyl)-2,3-dihydro-1H-inden-1-yloxy)-2,3-dihydrobenzofuran-3-yl)acetate

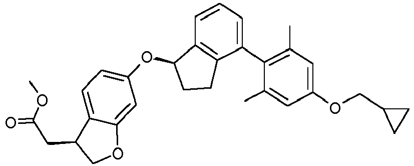

Methyl 2-((S)-6-((R)-4-(4-hydroxy-2,6-dimethylphenyl)-2,3-dihydro-1 H-inden-1-yloxy)-2,3-dihydrobenzofuran-3-yl)acetate (45 mg) and bromomethylcyclopropane (29.4 µl) were suspended in dimethylformamide (2 mL) and potassium carbonate (63 mg) was added. The reaction mixture was shaken for 24 h at 60 °C and than directly chromatographed by HPLC on reversed phase. The product fractions are collected and lyophilized to give the title compound. Yield: 17 mg; LC (method 27): t_{R} = 1.32 min.

### Intermediate 200

### Methyl 2-((S)-6-((R)-4-(4-(2,6-dimethyl-4-((1-methyl-1H-pyrazol-3yl)methoxy)phenyl)-7-fluoro-2,3-dihydro-1H-inden-1-yloxy)-2,3-dihydrobenzofuran-3-yl)acetate

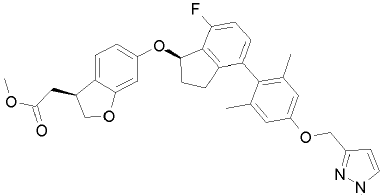

Methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (Intermediate 51 i) (46.3 mg) and 3-(chloromethyl)-1-methyl-1 H-pyrazole (39.2 mg) were suspended in dimethylformamide (1.8 mL) and potassium carbonate (62 mg) was added. The reaction mixture was shaken for 24 h at 60 °C and than directly chromatographed by HPLC on reversed phase. The product fractions are collected and lyophilized to give the title compound. Yield: 44.5 mg; LC (method 23): t_{R} = 2.09 min; Mass spectrum (ESI⁺): m/z = 557 [M+H]⁺.

### Intermediate 201

### Methyl 2-((S)-6-((R)-4-(2,6-dimethyl-4-((1-methyl-2-oxo-1,2-dihydropyridin-4-yl)methoxy)phenyl)-7-fluoro-2,3-dihydro-1H-inden-1-yloxy)-2,3-dihydrobenzofuran-3-yl)acetate

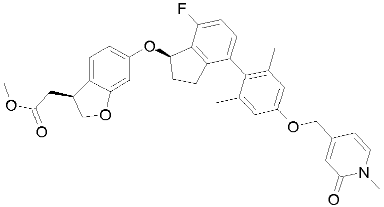

Methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (Intermediate 51 i) (46.3 mg) and 4-(chloromethyl)-1-methylpyridin-2(1 H)-one (47.3 mg) were suspended in dimethylformamide (1.8 mL) and potassium carbonate (62 mg) was added. The reaction mixture was shaken for 24 h at 60 °C and than directly chromatographed by HPLC on reversed phase. The product fractions are collected and lyophilized to give the title compound. Yield: 49.6 mg; LC (method 23): t_{R} = 1.96 min; Mass spectrum (ESI⁺): m/z = 584 [M+H]⁺.

### Intermediate 202

### Methyl 2-((S)-6-((R)-7-fluoro-4-(4-((2-methoxypyridin-4-yl)methoxy)-2,6-dimethylphenyl)-2,3-dihydro-1H-inden-1-yloxy)-2,3-dihydrobenzofuran-3-yl)acetate

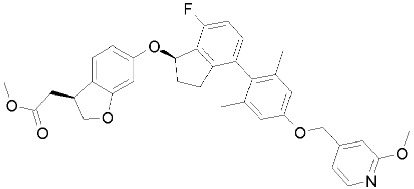

Methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (Intermediate 51 i) (46.3 mg) and 4-(chloromethyl)-2-methoxypyridine hydrochloride (58.2 mg) were suspended in dimethylformamide (1.8 mL) and potassium carbonate (62 mg) was added. The reaction mixture was shaken for 24 h at 60 °C and than directly chromatographed by HPLC on reversed phase. The product fractions are collected and lyophilized to give the title compound. Yield: 42 mg; LC (method 23): t_{R} = 2.21 min; Mass spectrum (ESI⁺): m/z = 584 [M+H]⁺.

### Intermediate 203

### Methyl 2-((S)-6-((R)-4-(2,6-dimethyl-4-(oxazol-2-ylmethoxy)phenyl)-7-fluoro-2,3-dihydro-1H-inden-1-yloxy)-2,3-dihydrobenzofuran-3-yl)acetate

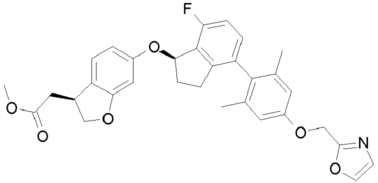

Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate (Intermediate 51 i) (46.3 mg) and 2-(chloromethyl)oxazole (25.3 mg) were suspended in dimethylformamide (2.0 mL) and potassium carbonate (62 mg) was added. The reaction mixture was shaken for 24 h at 60 °C. Another portion of 2-(chloromethyl)oxazole (25.3 mg) and potassium carbonate (62 mg) was added and the mixture was shaken again for 24 h at 60 °C and than directly chromatographed by HPLC on reversed phase. The product fractions are collected and lyophilized to give the title compound. Yield: 30.9 mg; LC (method 23): t_{R} = 2.09 min; Mass spectrum (ESI⁺): m/z = 544 [M+H]⁺.

### Intermediate 204

### Methyl 2-((S)-6-((R)-4-(2,6-dimethyl-4-((1-methyl-1H-imidazol-2-yl)methoxy)phenyl)-7-fluoro-2,3-dihydro-1H-inden-1-yloxy)-2,3-dihvdrobenzofuran-3-yl)acetate

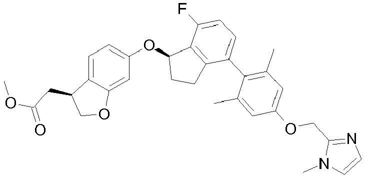

Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate (Intermediate 51 i) (46.3 mg) and 2-(chloromethyl)-1-methyl-1 H-imidazole (39.2 mg) were suspended in dimethylformamide (1.8 mL) and potassium carbonate (62 mg) was added. The reaction mixture was shaken for 24 h at 60 °C. Another portion of 2-(chloromethyl)-1-methyl-1 H-imidazole (39.2 mg) and potassium carbonate (62 mg) was added and the mixture was shaken again for 24 h at 60 °C and than directly chromatographed by HPLC on reversed phase. The product fractions are collected and lyophilized to give the title compound. Yield: 31.5 mg; LC (method 23): t_{R} = 1.54 min; Mass spectrum (ESI⁺): m/z = 557 [M+H]⁺.

### Intermediate 205

### Methyl 2-((S)-6-((R)-4-(2,6-dimethyl-4-((tetrahydro-2H-pyran-4-yl)methoxy)phenyl)-7-fluoro-2,3-dihydro-1H-inden-1-yloxy)-2,3-dihydrobenzofuran-3-yl)acetate

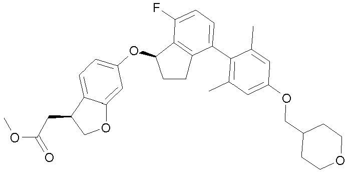

Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate (Intermediate 51i) (138 mg) and 4-(bromomethyl)tetrahydro-2H-pyran (160.3 mg) were suspended in dimethylformamide (5 mL) and potassium carbonate (185.7 mg) was added. The reaction mixture was shaken for 48 h at 60 °C and than directly chromatographed by HPLC on reversed phase. The product fractions are collected and lyophilized to give the title compound. Yield: 117 mg; LC (method 23): t_{R} = 2.26 min; Mass spectrum (ESI⁺): m/z = 561 [M+H]⁺.

### Intermediate 206

### Methyl 2-((S)-6-((R)-4-(2,6-dimethyl-4-((1-methyl-1H-pyrazol-5-yl)methoxy)phenyl)-7-fluoro-2,3-dihydro-1H-inden-1-yloxy)-2,3-dihydrobenzofuran-3-yl)acetate

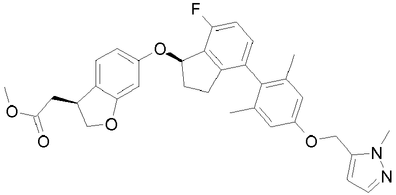

Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate (Intermediate 51 i) (46.3 mg) and 5-(chloromethyl)-1-methyl-1 H-pyrazole hydrochloride (50.1 mg) were suspended in dimethylformamide (1.8 mL) and potassium carbonate (62 mg) was added. The reaction mixture was shaken for 24 h at 60 °C. Another portion of 5-(chloromethyl)-1-methyl-1 H-pyrazole hydrochloride (50.1 mg) and potassium carbonate (62 mg) was added and the mixture was shaken again for 24 h at 60 °C and than directly chromatographed by HPLC on reversed phase. The product fractions are collected and lyophilized to give the title compound. Yield: 27.8 mg; LC (method 11): t_{R} = 1.13 min; Mass spectrum (ESI⁺): m/z = 557 [M+H]⁺.

### Intermediate 207

### Methyl 2-((S)-6-((R)-4-(2,6-dimethyl-4-((1-methyl-1H-pyrazol-4-yl)methoxy)phenyl)-7-fluoro-2,3-dihydro-1H-inden-1-yloxy)-2,3-dihydrobenzofuran-3-yl)acetate

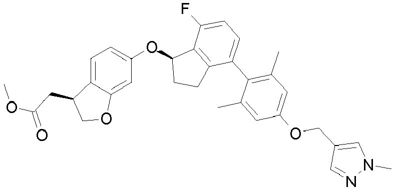

Methyl 2-((S)-6-((R)-7-fluoro-4-(4-hydroxy-2,6-dimethylphenyl)-2,3-dihydro-1 H-inden-1-yloxy)-2,3-dihydrobenzofuran-3-yl)acetate (Intermediate 51 i) (46.3 mg) and 4-(chloromethyl)-1-methyl-1 H-pyrazole dihydrochloride (61.1 mg) were suspended in dimethylformamide (1.8 mL) and potassium carbonate (124.4 mg) was added. The reaction mixture was shaken for 24 h at 60 °C and 72 h at 70 °C. Another portion of 4-(chloromethyl)-1-methyl-1 H-pyrazole dihydrochloride (124.4 mg) and potassium carbonate (124.4 mg) was added and the mixture was shaken again for 24 h at 120 °C and then directly chromatographed by HPLC on reversed phase. The product fractions are collected and lyophilized to give the title compound. Yield: 10,1 mg; LC (method 23): t_{R} = 2.06 min; Mass spectrum (ESI⁺): m/z = 557 [M+H]⁺.

### Example 1*

### 2-((3S)-6-((1R)-4-(2-methyl-5-(3-(methylsulfonyl)propoxy)phenyl)-2,3-dihydro-1H-inden-1-yloxy)-2,3-dihydrobenzofuran-3-yl)acetic acid

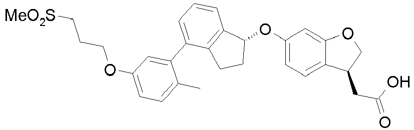

4 M aqueous NaOH solution (39 µL) is added to a solution of methyl 2-((3S)-6-((1R)-4-(2-methyl-5-(3-(methylsulfonyl)propoxy)phenyl)-2,3-dihydro-1 H-inden-1-yloxy)-2,3-dihydrobenzofuran-3-yl)acetate (34 mg) in tetrahydrofuran (0.5 mL) and methanol (0.5 mL) at room temperature. The mixture is stirred at room temperature for 12 hours. The mixture is diluted with water and neutralized with 4 M aqueous HCl solution (41 µL). The resulting mixture is extracted with ethyl acatete, and the combined extract is washed with brine and dried (MgSO₄). The solvent is evaporated and the residue is chromatographed on silica gel (cyclohexane/ethyl acetate 70:30→10:90). The product thus obtained is dissolved in acetonitrile/water (1:1) and lyophilized to give the title compound. Yield: 25 mg; LC (method 1): t_{R} = 1.04 min; Mass spectrum (ESI⁺): m/z = 537 [M+H]⁺.

### Example 2*

### 2-((S)-6-((R)-4-(2,6-dimethyl-4-(3-(methylsulfonyl)propoxy)phenyl)-2,3-dihydro-1H-inden-1-yloxy)-2,3-dihydrobenzofuran-3-yl)acetic acid

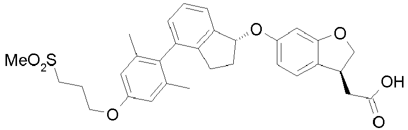

The title compound is prepared from methyl 2-((S)-6-((R)-4-(2,6-dimethyl-4-(3-(methylsulfonyl)propoxy)phenyl)-2,3-dihydro-1H-inden-1-yloxy)-2,3-dihydrobenzofuran-3-yl)acetate following a procedure analogous to that described in example 1. LC (method 1): t_{R} = 1.31 min; Mass spectrum (ESI⁺): m/z = 551 [M+H]⁺.

### Example 3*

### 2-((3S)-6-((1R)-4-(1-phenyl-1H-tetrazol-5-yl)-2,3-dihydro-1H-inden-1-yloxy)-2,3-dihydrobenzofuran-3-yl)acetic acid

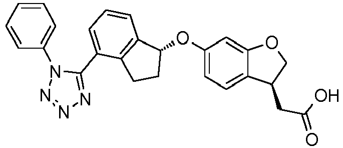

The title compound is prepared from methyl 2-((3S)-6-((1R)-4-(1-methyl-1H-tetrazol-5-yl)-2,3-dihydro-1H-inden-1-yloxy)-2,3-dihydrobenzofuran-3-yl)acetate following a procedure analogous to that described in example 1. The mixture is stirred for 1 hour at 35°C. The product is purified by HPLC on reversed phase. LC (method 1): t_{R} = 1.62 min; Mass spectrum (ESI⁺): m/z = 455 [M+H]⁺.

### Example 4*

### 2-((3S)-6-((1R)-4-(2,6-dimethyl-3-(3-(methylsulfonyl)propoxy)phenyl)-2,3-dihydro-1H-inden-1-yloxy)-2,3-dihydrobenzofuran-3-yl)acetic acid

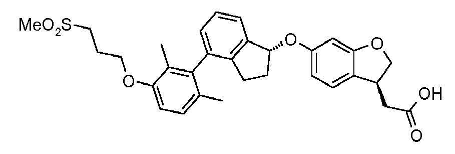

The title compound is prepared from methyl 2-((3S)-6-((1 R)-4-(2,6-dimethyl-3-(3-(methylsulfonyl)propoxy)phenyl)-2,3-dihydro-1 H-inden-1-yloxy)-2,3-dihydrobenzofuran-3-yl)acetate following a procedure analogous to that described in example 1. LC (method 1): t_{R} = 1.62 min; Mass spectrum (ESI⁺): m/z = 551 [M+H]⁺.

### Example 5*

### 2-((S)-6-((R)-4-(2,6-Dimethyl-4-((R)-tetrahydrofuran-3-yloxy)phenyl)-2,3-dihydro-1H-inden-1-yloxy)-2,3-dihydrobenzofuran-3-yl)acetic acid

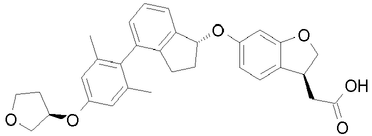

The title compound is prepared from methyl 2-((S)-6-((R)-4-(2,6-dimethyl-4-((R)-tetrahydrofuran-3-yloxy)phenyl)-2,3-dihydro-1H-inden-1-yloxy)-2,3-dihydrobenzofuran-3-yl)acetate following a procedure analogous to that described in example 1. LC (method 4): t_{R} = 1.85 min; Mass spectrum (ESI⁻): m/z = 499 [M-H]⁻.

### Example 6*

### 2-((S)-6-((R)-4-(2,6-Dimethyl-4-((S)-tetrahydrofuran-3-yloxy)phenyl)-2,3-dihydro-1H-inden-1-yloxy)-2,3-dihydrobenzofuran-3-yl)acetic acid

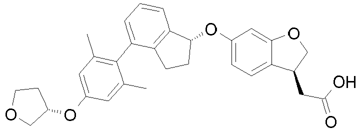

The title compound is prepared from methyl 2-((S)-6-((R)-4-(2,6-dimethyl-4-((S)-tetrahydrofuran-3-yloxy)phenyl)-2,3-dihydro-1H-inden-1-yloxy)-2,3-dihydrobenzofuran-3-yl)acetate following a procedure analogous to that described in example 1. The product is purified by HPLC on reversed phase. LC (method 4): t_{R} = 1.86 min; Mass spectrum (ESI⁻): m/z = 499 [M-H]⁻.

### Example 7*

### 2-((S)-6-((R)-4-(4-(3-Hydroxy-3-methylbutoxy)-2,6-dimethylphenyl)-2,3-dihydro-1H-inden-1-yloxy)-2,3-dihydrobenzofuran-3-yl)acetic acid

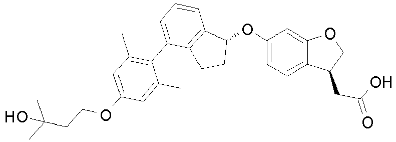

In a microwave vial a mixture of methyl 2-((S)-6-((R)-4-(4,4,5,5-tetramethyl-1,3,2-dioxaborolan-2-yl)-2,3-dihydro-1H-inden-1-yloxy)-2,3-dihydrobenzofuran-3-yl)acetate (100 mg), 4-(4-bromo-3,5-dimethylphenoxy)-2-methylbutan-2-ol (70 mg), Na₂CO₃ (1.2 mL of a 2 M aqueous solution), 1,4-dioxane (3 mL) and ethanol (1 mL) is purged for 5 minutes with argon. Tetrakis-triphenylphosphine-palladium-(0) (13 mg) is added, the vial is sealed and the mixture is stirred at 90°C for 12 hours. The mixture is purified by HPLC on reversed phase to give the title compound. Yield: 23 mg; LC (method 5): t_{R} = 1.48 min; Mass spectrum (ESI⁻): m/z = 515 [M-H]⁻.

### Example 8*

### 2-((S)-6-((R)-4-(4-(2-methoxyethoxy)-2,6-dimethylphenyl)-2,3-dihydro-1H-inden-1-yloxy)-2,3-dihydrobenzofuran-3-yl)acetic acid

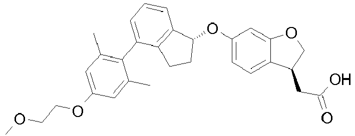

1 M aqueous NaOH solution (80 µL) is added to a solution of methyl 2-((S)-6-((R)-4-(4-(2-methoxyethoxy)-2,6-dimethylphenyl)-2,3-dihydro-1 H-inden-1-yloxy)-2,3-dihydrobenzofuran-3-yl)acetate (28 mg) in isopropanol (2 mL). The mixture is stirred at 40°C for 12 hours. After addition of 1 N hydrochloric acid (80 µL) the mixture is diluted with diethylether and washed with water and brine. The organic phase is dried (MgSO₄). The solvent is evaporated and the residue is chromatographed on silica gel (cyclohexane/ethyl acetate 80:20→0:100). The product thus obtained is dissolved in acetonitrile/water (1:1) and lyophilized to give the title compound. Yield: 18 mg; LC (method 1): t_{R} = 1.40 min; Mass spectrum (ESI⁺): m/z = 489 [M+H]⁺.

### Example 9*

### 2-((S)-6-((R)-4-(2,6-dimethyl-4-((3-methyloxetan-3-yl)methoxy)phenyl)-2,3-dihydro-1H-inden-1 -yloxy)-2,3-dihydrobenzofuran-3-yl)acetic acid

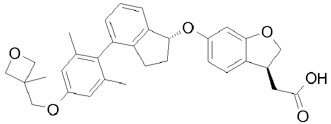

The title compound is prepared from methyl 2-((S)-6-((R)-4-(2,6-dimethyl-4-((3-methyloxetan-3-yl)methoxy)phenyl)-2,3-dihydro-1 H-inden-1-yloxy)-2,3-dihydrobenzofuran-3-yl)acetate following a procedure analogous to that described in example 8. LC (method 1): t_{R} = 1.44 min; Mass spectrum (ESI⁺): m/z = 515 [M+H]⁺.

### Example 10*

### 2-((3S)-6-((1R)-4-(3-(2-Methoxyethoxy)-2-methylphenyl)-2,3-dihydro-1H-inden-1-yloxy)-2,3-dihydrobenzofuran-3-yl)acetic acid

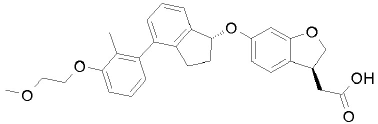

1 M aqueous NaOH solution (340 µL) is added to a solution of methyl 2-((3S)-6-((1R)-4-(3-(2-methoxyethoxy)-2-methylphenyl)-2,3-dihydro-1H-inden-1-yloxy)-2,3-dihydrobenzofuran-3-yl)acetate (33 mg) in tetrahydrofuran (2 mL) and methanol (2 mL) at room temperature. The mixture is stirred at room temperature for 12 hours. The organic solvents are evaporated in vacuo. The residue is diluted with water and neutralized with 1 M aqueous HCl solution (340 µL). The mixture is stirred for 1 hour. The precipitate formed is filtered off, washed with water and dried in vacuo to give the title compound. Yield: 32 mg; LC (method 3): t_{R} = 1.43 min; Mass spectrum (ESI⁺): m/z = 475 [M+H]⁺.

### Example 11*

### 2-((3S)-6-((1R)-4-(3-(2-Hydroxyethoxy)-2-methylphenyl)-2,3-dihydro-1H-inden-1-yloxy)-2,3-dihydrobenzofuran-3-yl)acetic acid

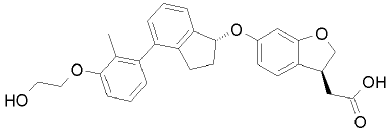

The title compound is prepared from methyl 2-((3S)-6-((1R)-4-(3-(2-hydroxyethoxy)-2-methylphenyl)-2,3-dihydro-1H-inden-1-yloxy)-2,3-dihydrobenzofuran-3-yl)acetate following a procedure analogous to that described in example 10. LC (method 3): t_{R} = 1.26 min; Mass spectrum (ESI⁺): m/z = 461 [M+H]⁺.

### Example 12

### 2-((S)-6-((R)-4-(2,6-dimethyl-4-((3-methyloxetan-3-yl)methoxy)phenyl)-7-fluoro-2,3-dihydro-1 H-inden-1-yloxy)-2,3-dihydrobenzofuran-3-yl)acetic acid

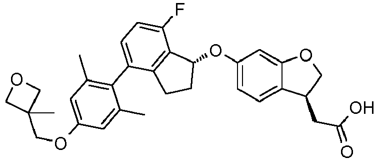

The title compound is prepared from methyl 2-((S)-6-((R)-4-(2,6-dimethyl-4-((3-methyloxetan-3-yl)methoxy)phenyl)-7-fluoro-2,3-dihydro-1H-inden-1-yloxy)-2,3-dihydrobenzofuran-3-yl)acetate following a procedure analogous to that described in example 1. LC (method 4): t_{R} = 1.87 min; Mass spectrum (ESI⁺): m/z = 533 [M+H]⁺.

### Example 13

### 2-((3S)-6-((1R)-4-(2,6-dimethyl-4-((tetrahydrofuran-3-yl)methoxy)phenyl)-7-fluoro-2,3-dihydro-1H-inden-1-yloxy)-2,3-dihydrobenzofuran-3-yl)acetic acid

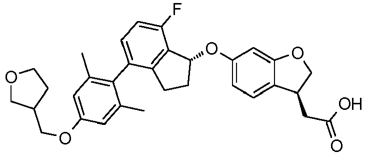

The title compound is prepared from methyl 2-((3S)-6-((1 R)-4-(2,6-dimethyl-4-((tetrahydrofuran-3-yl)methoxy)phenyl)-7-fluoro-2,3-dihydro-1 H-inden-1-yloxy)-2,3-dihydrobenzofuran-3-yl)acetate following a procedure analogous to that described in example 8. LC (method 1): t_{R} = 1.42 min; Mass spectrum (ESI⁺): m/z = 533 [M+H]⁺.

### Example 14

### 2-((3S)-6-((1R)-4-(2-(1-Cyanocyclopropyl)phenyl)-2,3-dihydro-1H-inden-1-yloxy)-2,3-dihydrobenzofuran-3-yl)acetic acid

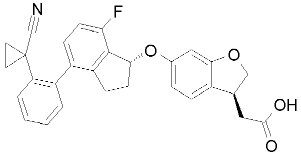

The title compound is prepared from methyl 2-((3S)-6-((1 R)-4-(2-(1-cyanocyclopropyl)phenyl)-2,3-dihydro-1H-inden-1-yloxy)-2,3-dihydrobenzofuran-3-yl)acetate following a procedure analogous to that described in example 10. LC (method 4): t_{R} = 1.84 min; Mass spectrum (ESI⁺): m/z = 452 [M+H]⁺.

### Example 15

### 2-((3S)-6-((1R)-4-(2,6-dimethyl-4-((tetrahydrofuran-2-yl)methoxy)phenyl)-7-fluoro-2,3-dihydro-1H-inden-1-yloxy)-2,3-dihydrobenzofuran-3-yl)acetic acid

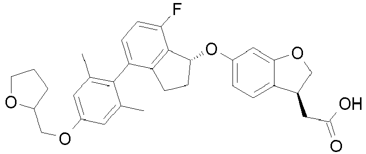

The title compound is prepared from methyl 2-((3S)-6-((1R)-4-(2,6-dimethyl-4-((tetrahydrofuran-2-yl)methoxy)phenyl)-7-fluoro-2,3-dihydro-1 H-inden-1-yloxy)-2,3-dihydrobenzofuran-3-yl)acetate following a procedure analogous to that described in example 8. LC (method 1): t_{R} = 1.42 min; Mass spectrum (ESI⁺): m/z = 533 [M+H]⁺.

### Example 16

### 2-((S)-6-((R)-7-Fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid

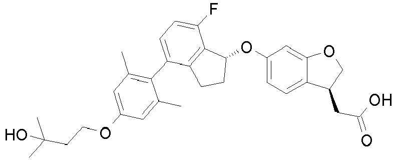

The title compound is prepared from methyl 2-((S)-6-((R)-7-fluoro-4-(4-(3-hydroxy-3-methylbutoxy)-2,6-dimethylphenyl)-2,3-dihydro-1 H-inden-1-yloxy)-2,3-dihydrobenzofuran-3-yl)acetate following a procedure analogous to that described in example 10. LC (method 4): t_{R} = 1.85 min; Mass spectrum (ESI⁺): m/z = 535 [M+H]⁺.

### Example 17

### 2-((S)-6-((R)-6-Fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid

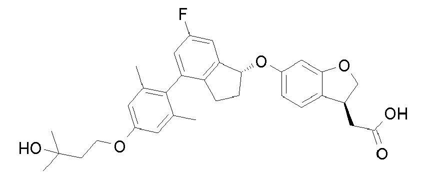

The title compound is prepared from methyl 2-((S)-6-((R)-6-fluoro-4-(4-(3-hydroxy-3-methylbutoxy)-2,6-dimethylphenyl)-2,3-dihydro-1 H-inden-1-yloxy)-2,3-dihydrobenzofuran-3-yl)acetate following a procedure analogous to that described in example 10. LC (method 3): t_{R} = 1.54 min; Mass spectrum (ESI⁺): m/z = 535 [M+H]⁺.

### Example 18

### 2-((S)-6-((R)-4-(2,6-Dimethyl-4-((3-methyloxetan-3-yl)methoxy)phenyl)-6-fluoro-2,3-dihydro-1H-inden-1-yloxy)-2,3-dihydrobenzofuran-3-yl)acetic acid

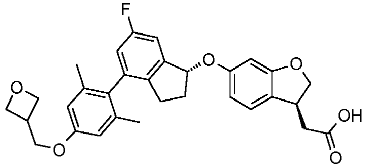

The title compound is prepared from methyl 2-((S)-6-((R)-4-(2,6-dimethyl-4-((3-methyloxetan-3-yl)methoxy)phenyl)-6-fluoro-2,3-dihydro-1 H-inden-1-yloxy)-2,3-dihydrobenzofuran-3-yl)acetate following a procedure analogous to that described in example 10. LC (method 3): t_{R} = 1.56 min; Mass spectrum (ESI⁺): m/z = 533 [M+H]⁺.

### Example 19

### 2-((S)-6-((R)-6-Fluoro-4-(2-(3-hydroxy-3-methylbutoxy)-4,6-dimethylpyrimidin-5-yl)-2,3-dihydro-1H-inden-1-yloxy)-2,3-dihydrobenzofuran-3-yl)acetic acid

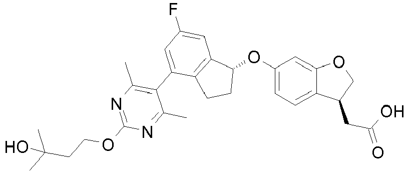

The title compound is prepared from methyl 2-((S)-6-((R)-6-fluoro-4-(2-(3-hydroxy-3-methylbutoxy)-4,6-dimethylpyrimidin-5-yl)-2,3-dihydro-1 H-inden-1-yloxy)-2,3-dihydrobenzofuran-3-yl)acetate following a procedure analogous to that described in example 10. LC (method 3): t_{R} = 1.30 min; Mass spectrum (ESI⁺): m/z = 537 [M+H]⁺.

### Example 20*

### 2-((S)-6-((R)-4-(2-(3-Hydroxy-3-methylbutoxy)-4,6-dimethylpyrimidin-5-yl)-2,3-dihydro-1H-inden-1-yloxy)-2,3-dihydrobenzofuran-3-yl)acetic acid

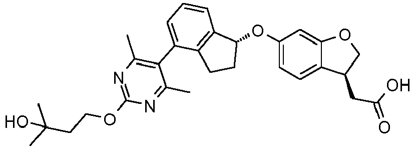

The title compound is prepared from methyl 2-((S)-6-((R)-4-(2-(3-hydroxy-3-methylbutoxy)-4,6-dimethylpyrimidin-5-yl)-2,3-dihydro-1 H-inden-1-yloxy)-2,3-dihydrobenzofuran-3-yl)acetate following a procedure analogous to that described in example 10. LC (method 3): t_{R} = 1.24 min; Mass spectrum (ESI⁺): m/z = 519 [M+H]⁺.

### Example 21*

### 2-((S)-6-((R)-4-(4,6-Dimethyl-2-((3-methyloxetan-3-yl)methoxy)pyrimidin-5-yl)-2,3-dihydro-1H-inden-1-yloxy)-2,3-dihydrobenzofuran-3-yl)acetic acid

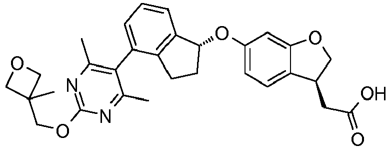

The title compound is prepared from methyl 2-((S)-6-((R)-4-(4,6-dimethyl-2-((3-methyloxetan-3-yl)methoxy)pyrimidin-5-yl)-2,3-dihydro-1H-inden-1-yloxy)-2,3-dihydrobenzofuran-3-yl)acetate following a procedure analogous to that described in example 10. LC (method 3): t_{R} = 1.25 min; Mass spectrum (ESI⁺): m/z = 517 [M+H]⁺.

### Example 22

### 2-((S)-6-((R)-4-(4,6-Dimethyl-2-((3-methyloxetan-3-yl)methoxy)pyrimidin-5-yl)-6-fluoro-2,3-dihydro-1H-inden-1-yloxy)-2,3-dihydrobenzofuran-3-yl)acetic acid

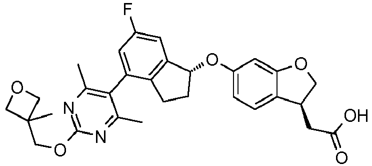

The title compound is prepared from methyl 2-((S)-6-((R)-4-(4,6-dimethyl-2-((3-methyloxetan-3-yl)methoxy)pyrimidin-5-yl)-6-fluoro-2,3-dihydro-1H-inden-1-yloxy)-2,3-dihydrobenzofuran-3-yl)acetate following a procedure analogous to that described in example 10. LC (method 3): t_{R} = 1.29 min; Mass spectrum (ESI⁺): m/z = 535 [M+H]⁺.

### Example 23

### 2-((3S)-6-((1R)-4-(2,6-Dimethyl-4-(2-(methylsulfinyl)ethoxy)phenyl)-7-fluoro-2,3-dihydro-1H-inden-1-yloxy)-2,3-dihydrobenzofuran-3-yl)acetic acid

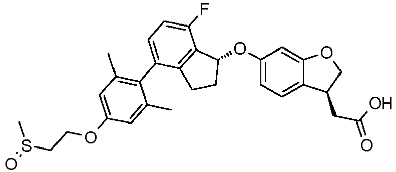

1 M aqueous NaOH solution (480 µL) is added to a solution of methyl 2-((3S)-6-((1 R)-4-(2,6-dimethyl-4-(2-(methylsulfinyl)ethoxy)phenyl)-7-fluoro-2,3-dihydro-1 H-inden-1-yloxy)-2,3-dihydrobenzofuran-3-yl)acetate (180 mg) in methanol (5 mL) at room temperature. The mixture is stirred at room temperature for 12 hours. The mixture is neutralized with 1 M aqueous HCl solution and partitioned between diethylether and brine. The organic phase is dried (MgSO₄) and concentrated. The residue is chromatographed on silica gel (cyclohexane/ethyl acetate 60:40→10:90) to give the title compound. Yield: 80 mg; LC (method 4): t_{R} = 1.71 min; Mass spectrum (ESI⁺): m/z = 539 [M+NH_{4]}⁺.

### Example 24

### 2-((S)-6-((R)-4-(2,6-Dimethyl-4-(2-(methylsulfonyl)ethoxy)phenyl)-7-fluoro-2,3-dihydro-1H-inden-1-yloxy)-2,3-dihydrobenzofuran-3-yl)acetic acid

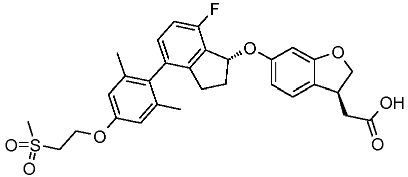

To a solution of 2-((3S)-6-((1R)-4-(2,6-dimethyl-4-(2-(methylsulfinyl)ethoxy)phenyl)-7-fluoro-2,3-dihydro-1H-inden-1-yloxy)-2,3-dihydrobenzofuran-3-yl)acetic acid (70 mg) in methanol (1.5 mL) and water (0.75 mL) is added potassium peroxomonoslufate (oxone^{®}) (150 mg). The mixture is stirred for 3 hour at room temperature and then potassium peroxomonoslufate (oxone^{®}) (80 mg) is added. After stirring for 12 hours at room temperature the mixture is partitioned between ethyl acetate and water. The organic phase is dried (MgSO₄) and concentrated. The residue is chromatographed on silica gel (cyclohexane/ethyl acetate 50:50→0:100). The product thus obtained is dissolved in acetonitrile/water (1:1) and lyophilized to give the title compound. Yield: 7 mg; LC (method 4): t_{R} = 1.71 min; Mass spectrum (ESI⁺): m/z = 555 [M+H]⁺.

### Example 25

### 2-((S)-6-((R)-4-(2,6-Dimethyl-4-(tetrahydro-2H-pyran-4-yloxy)phenyl)-7-fluoro-2,3-dihydro-1H-inden-1-yloxy)-2,3-dihydrobenzofuran-3-yl)acetic acid

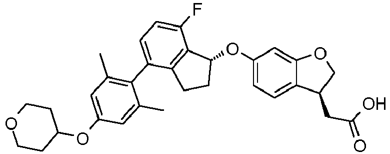

The title compound is prepared from methyl 2-((S)-6-((R)-4-(2,6-dimethyl-4-(tetrahydro-2H-pyran-4-yloxy)phenyl)-7-fluoro-2,3-dihydro-1 H-inden-1-yloxy)-2,3-dihydrobenzofuran-3-yl)acetate following a procedure analogous to that described in example 1. LC (method 4): t_{R} = 1.90 min; Mass spectrum (ESI⁺): m/z = 533 [M+H]⁺.

### Example 26

### 2-((S)-6-((R)-6-Chloro-4-(2,6-dimethyl-4-(tetrahydro-2H-pyran-4-yloxy)phenyl)-2,3-dihydro-1H-inden-1-yloxy)-2,3-dihydrobenzofuran-3-yl)acetic acid

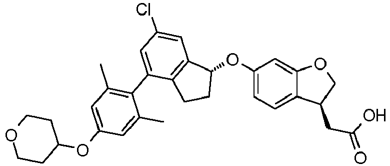

The title compound is prepared from methyl 2-((S)-6-((R)-6-chloro-4-(2,6-dimethyl-4-(tetrahydro-2H-pyran-4-yloxy)phenyl)-2,3-dihydro-1 H-inden-1-yloxy)-2,3-dihydrobenzofuran-3-yl)acetate following a procedure analogous to that described in example 10. LC (method 8): t_{R} = 0.78 min; Mass spectrum (ESI⁺): m/z = 549 [M+H]⁺.

### Example 27

### 2-((S)-6-((R)-4-(2,6-Dimethyl-4-(tetrahydro-2H-pyran-4-yloxy)phenyl)-6,7-difluoro-2,3-dihydro-1H-inden-1-yloxy)-2,3-dihydrobenzofuran-3-yl)acetic acid

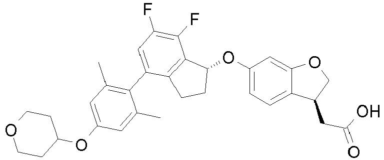

The title compound is prepared from methyl 2-((S)-6-((R)-4-(2,6-dimethyl-4-(tetrahydro-2H-pyran-4-yloxy)phenyl)-6,7-difluoro-2,3-dihydro-1 H-inden-1-yloxy)-2,3-dihydrobenzofuran-3-yl)acetate following a procedure analogous to that described in example 10. LC (method 4): t_{R} = 1.89 min; Mass spectrum (ESI⁺): m/z = 551 [M+H]⁺.

### Example 28

### 2-((S)-6-((R)-4-(2,6-Dimethyl-4-((3-methyloxetan-3-yl)methoxy)phenyl)-6,7-difluoro-2,3-dihydro-1H-inden-1-yloxy)-2,3-dihydrobenzofuran-3-yl)acetic acid

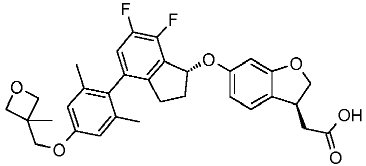

The title compound is prepared from methyl 2-((S)-6-((R)-4-(2,6-dimethyl-4-((3-methyloxetan-3-yl)methoxy)phenyl)-6,7-difluoro-2,3-dihydro-1H-inden-1-yloxy)-2,3-dihydrobenzofuran-3-yl)acetate following a procedure analogous to that described in example 10. LC (method 4): t_{R} = 1.89 min; Mass spectrum (ESI⁺): m/z = 551 [M+H]⁺.

### Example 29

### 2-((S)-6-((R)-6-Chloro-4-(2,6-dimethyl-4-((3-methyloxetan-3-yl)methoxy)phenyl)-2,3-dihydro-1H-inden-1-yloxy)-2,3-dihydrobenzofuran-3-yl)acetic acid

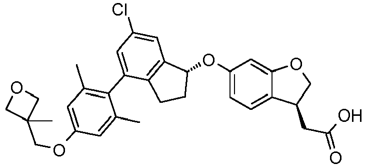

The title compound is prepared from methyl 2-((S)-6-((R)-6-chloro-4-(2,6-dimethyl-4-((3-methyloxetan-3-yl)methoxy)phenyl)-2,3-dihydro-1 H-inden-1-yloxy)-2,3-dihydrobenzofuran-3-yl)acetate following a procedure analogous to that described in example 10. LC (method 8): t_{R} = 0.79 min; Mass spectrum (ESI⁺): m/z = 549 [M+H]⁺.

### Example 30

### 2-((S)-6-((R)-4-(2,6-Dimethyl-4-((S)-tetrahydrofuran-3-yloxy)phenyl)-7-fluoro-2,3-dihydro-1H-inden-1-yloxy)-2,3-dihydrobenzofuran-3-yl)acetic acid

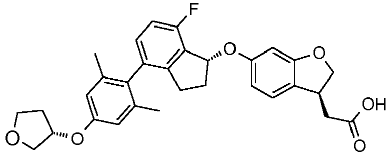

1 M aqueous NaOH solution (585 µL) is added to a solution of methyl 2-((S)-6-((R)-4-(2,6-dimethyl-4-((S)-tetrahydrofuran-3-yloxy)phenyl)-7-fluoro-2,3-dihydro-1H-inden-1-yloxy)-2,3-dihydrobenzofuran-3-yl)acetate (125 mg) in methanol (3 mL). The mixture is stirred at 40°C for 12 hours. After addition of 1 N hydrochloric acid (585 µL) the mixture is diluted with diethylether and washed with water and brine. The organic phase is dried (MgSO₄). The solvent is evaporated and the residue is chromatographed on silica gel (cyclohexane/ethyl acetate 70:30→20:80). The product thus obtained is dissolved in acetonitrile/water (1:1) and lyophilized to give the title compound. Yield: 55 mg; LC (method 4): t_{R} = 1.82 min; Mass spectrum (ESI⁺): m/z = 519 [M+H]⁺.

### Example 31

### 2-((S)-6-((R)-4-(2,6-Dimethyl-4-((R)-tetrahydrofuran-3-yloxy)phenyl)-7-fluoro-2,3-dihydro-1H-inden-1-yloxy)-2,3-dihydrobenzofuran-3-yl)acetic acid

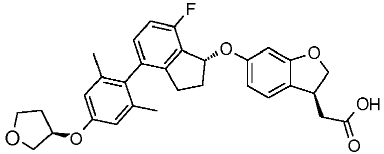

The title compound is prepared from methyl 2-((S)-6-((R)-4-(2,6-dimethyl-4-((R)-tetrahydrofuran-3-yloxy)phenyl)-7-fluoro-2,3-dihydro-1 H-inden-1-yloxy)-2,3-dihydrobenzofuran-3-yl)acetate following a procedure analogous to that described in example 30. LC (method 4): t_{R} = 1.82 min; Mass spectrum (ESI⁺): m/z = 519 [M+H]⁺.

### Example 32

### 2-((S)-6-((R)-4-(2,6-Dimethyl-4-((3-methyloxetan-3-yl)methoxy)phenyl)-5-fluoro-2,3-dihydro-1H-inden-1-yloxy)-2,3-dihydrobenzofuran-3-yl)acetic acid

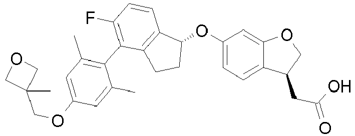

The title compound is prepared from methyl 2-((S)-6-((R)-4-(2,6-dimethyl-4-((3-methyloxetan-3-yl)methoxy)phenyl)-5-fluoro-2,3-dihydro-1 H-inden-1-yloxy)-2,3-dihydrobenzofuran-3-yl)acetate following a procedure analogous to that described in example 10. LC (method 7): t_{R} = 1.15 min; Mass spectrum (ESI⁻): m/z = 531 [M-H]⁻.

### Example 33

### 2-((S)-6-((R)-6-Cyano-4-(2,6-dimethyl-4-(tetrahydro-2H-pyran-4-yloxy)phenyl)-2,3-dihydro-1H-inden-1-yloxy)-2,3-dihydrobenzofuran-3-yl)acetic acid

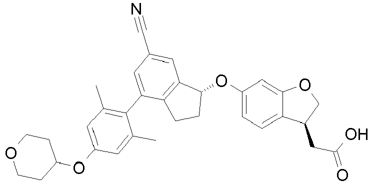

4 M aqueous NaOH solution (200 µL) is added to a solution of methyl 2-((S)-6-((R)-6-cyano-4-(2,6-dimethyl-4-(tetrahydro-2H-pyran-4-yloxy)phenyl)-2,3-dihydro-1 H-inden-1-yloxy)-2,3-dihydrobenzofuran-3-yl)acetate (34 mg) in tetrahydrofuran (1 mL) and methanol (1 mL) at room temperature. The mixture is stirred at room temperature for 3 hours. The organic solvents are evaporated and the residue is partitioned between water and ethyl acetate. 4 M Aqueous HCl solution is added to adjust the pH to 3. The phases are separated and the aqueous phase is extracted twice with ethyl acetate. The combined organic phases are washed with brine, dried (MgSO₄) and concentrated. The residue is purified by HPLC on reversed phase. Product containing fractions are pooled, the acetonitrile is evaporated and the residue acidified to pH 3 by addition of 1 M aqueous HCl solution. The precipitate is filtered off and dried to give the title compound. Yield: 9 mg; LC (method 7): t_{R} = 1.13 min; Mass spectrum (ESI⁻): m/z = 538 [M-H]⁻.

### Example 34

### 2-((3S)-6-((1R)-5-Cyano-4-(2,6-dimethyl-4-((tetrahydrofuran-3-yl)methoxy)phenyl)-2,3-dihydro-1H-inden-1-yloxy)-2,3-dihydrobenzofuran-3-yl)acetic acid

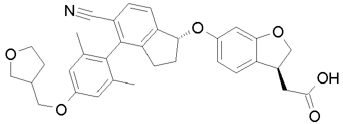

1 M Aqueous NaOH solution (37 µL) is added to a solution of methyl 2-((3S)-6-((1 R)-5-cyano-4-(2,6-dimethyl-4-((tetrahydrofuran-3-yl)methoxy)phenyl)-2,3-dihydro-1H-inden-1-yloxy)-2,3-dihydrobenzofuran-3-yl)acetate (10 mg) in methanol (2 mL) at room temperature. The mixture is stirred at room temperature for 12 hours, treated with 1 M aqueous NaOH solution (37 µL) and heated for 12 hours at 50°C. The mixture is neutralized by addition of 1 N aqueous HCl solution and purified by HPLC on reversed phase to give the title compound. Yield: 2 mg; LC (method 8): t_{R} = 0.44 min; Mass spectrum (ESI⁺): m/z = 540 [M+H]⁺.

### Example 35

### 2-((S)-6-((R)-7-Fluoro-4-(4-(2-hydroxy-2-methylpropoxy)-2,6-dimethylphenyl)-2,3-dihydro-1H-inden-1-yloxy)-2,3-dihydrobenzofuran-3-yl)acetic acid

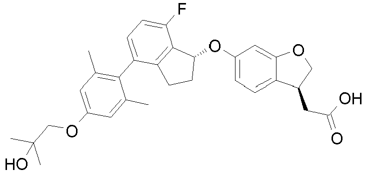

The title compound is prepared from methyl 2-((S)-6-((R)-7-fluoro-4-(4-(2-hydroxy-2-methylpropoxy)-2,6-dimethylphenyl)-2,3-dihydro-1 H-inden-1-yloxy)-2,3-dihydrobenzofuran-3-yl)acetate following a procedure analogous to that described in example 10. LC (method 8): t_{R} = 0.50 min; Mass spectrum (ESI⁺): m/z = 521 [M+H]⁺.

### Example 36

### 2-((3S)-6-((1R)-7-Fluoro-4-(4-(3-hydroxycyclopentyloxy)-2,6-dimethylphenyl)-2,3-dihydro-1H-inden-1-yloxy)-2,3-dihydrobenzofuran-3-yl)acetic acid

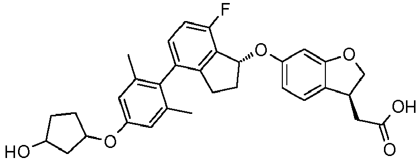

The title compound is prepared from methyl 2-((3S)-6-((1 R)-7-fluoro-4-(4-(3-hydroxycyclopentyloxy)-2,6-dimethylphenyl)-2,3-dihydro-1 H-inden-1-yloxy)-2,3-dihydrobenzofuran-3-yl)acetate following a procedure analogous to that described in example 10. The product is purified by chromatography on silica gel (petrole ether/ethyl acetate 50:50→0:10) to give the title compound. LC (method 8): t_{R} = 0.41 min; Mass spectrum (ESI⁺): m/z = 533 [M+H]⁺.

### Example 37

### 2-((S)-6-((R)-4-(2,6-Dimethyl-4-(2-(methylamino)-2-oxoethoxy)phenyl)-7-fluoro-2,3-dihydro-1H-inden-1-yloxy)-2,3-dihydrobenzofuran-3-yl)acetic acid

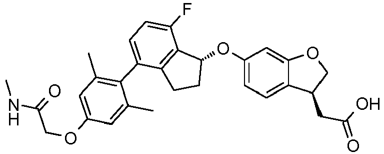

In a microwave vial 2-((S)-6-((R)-7-fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid (15 mg) is dissolved in a 2 M solution of methylamin in tetrahydrofuran (1 mL). The vial is sealed and the mixture is heated to 80°C for 12 hours. The mixture is concentrated and the residue is purified by HPLC on reversed phase. Yield: 8 mg; LC (method 9): t_{R} = 1.03 min; Mass spectrum (ESI⁺): m/z = 520 [M+H]⁺.

### Example 38

### 2-((S)-6-((R)-4-(4-(2-(Dimethylamino)-2-oxoethoxy)-2,6-dimethylphenyl)-7-fluoro-2,3-dihydro-1H-inden-1-yloxy)-2,3-dihydrobenzofuran-3-yl)acetic acid

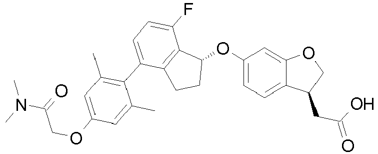

The title compound is prepared from 2-((S)-6-((R)-7-fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid and 2 M solution of dimethylamin in tetrahydrofuran following a procedure analogous to that described in example 37. LC (method 9): t_{R} = 1.06 min; Mass spectrum (ESI⁺): m/z = 534 [M+H]⁺.

### Example 39

### 2-((S)-6-((R)-4-(4-(2-Amino-2-oxoethoxy)-2,6-dimethylphenyl)-7-fluoro-2,3-dihydro-1H-inden-1-yloxy)-2,3-dihydrobenzofuran-3-yl)acetic acid

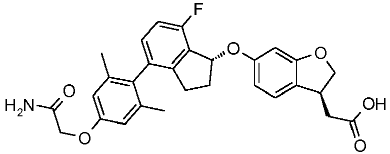

The title compound is prepared from 2-((S)-6-((R)-7-fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid and 0.5 M solution of ammonia in 1,4-dioxane following a procedure analogous to that described in example 37. LC (method 9): t_{R} = 1.01 min; Mass spectrum (ESI⁺): m/z = 506 [M+H]⁺.

### Example 40

### 2-((S)-6-((R)-7-Fluoro-4-(4-(3-hydroxy-2,2-dimethylpropoxy)-2,6-dimethylphenyl)-2,3-dihydro-1H-inden-1-yloxy)-2,3-dihydrobenzofuran-3-yl)acetic acid

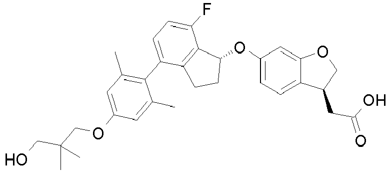

The title compound is prepared from methyl 2-((S)-6-((R)-7-fluoro-4-(4-(3-hydroxy-2,2-dimethylpropoxy)-2,6-dimethylphenyl)-2,3-dihydro-1 H-inden-1-yloxy)-2,3-dihydrobenzofuran-3-yl)acetate following a procedure analogous to that described in example 30. LC (method 8): t_{R} = 0.67 min; Mass spectrum (ESI⁺): m/z = 535 [M+H]⁺.

### Example 41

### 2-((S)-6-((R)-7-Fluoro-4-(4-(3-methoxy-2,2-dimethylpropoxy)-2,6-dimethylphenyl)-2,3-dihydro-1H-inden-1-yloxy)-2,3-dihydrobenzofuran-3-yl)acetic acid

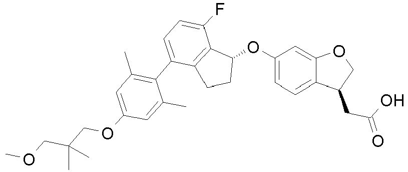

1 M aqueous NaOH solution (450 µL) is added to a solution of methyl 2-((S)-6-((R)-7-fluoro-4-(4-(3-methoxy-2,2-dimethylpropoxy)-2,6-dimethylphenyl)-2,3-dihydro-1 H-inden-1-yloxy)-2,3-dihydrobenzofuran-3-yl)acetate (82 mg) in methanol (2 mL). The mixture is stirred at room temperature for 12 hours. 1 N Hydrochloric acid (450 µL) is added and the residue is purified by HPLC on reversed phase to give the title compound. Yield: 39 mg; LC (method 8): t_{R} = 0.97 min; Mass spectrum (ESI⁺): m/z = 549 [M+H]⁺.

### Example 42

### 2-((S)-6-((R)-5-Cyano-4-(2,6-dimethyl-4-((3-methyloxetan-3-yl)methoxy)phenyl)-2,3-dihydro-1H-inden-1-yloxy)-2,3-dihydrobenzofuran-3-yl)acetic acid

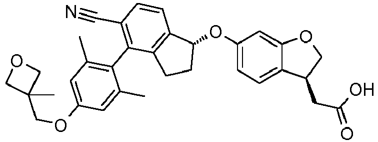

The title compound is prepared from methyl 2-((S)-6-((R)-5-cyano-4-(2,6-dimethyl-4-((3-methyloxetan-3-yl)methoxy)phenyl)-2,3-dihydro-1 H-inden-1-yloxy)-2,3-dihydrobenzofuran-3-yl)acetate following a procedure analogous to that described in example 10. LC (method 8): t_{R} = 0.43 min; Mass spectrum (ESI⁺): m/z = 540 [M+H]⁺.

### Example 43

### 2-((S)-6-((R)-4-(2,6-Dimethyl-4-(pyrimidin-5-yl)phenyl)-7-fluoro-2,3-dihydro-1H-inden-1-yloxy)-2,3-dihydrobenzofuran-3-yl)acetic acid

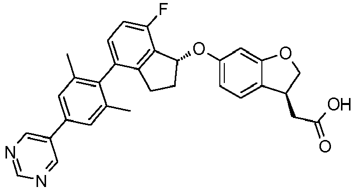

1 M aqueous NaOH solution (167 µL) is added to a solution of methyl 2-((S)-6-((R)-4-(2,6-dimethyl-4-(pyrimidin-5-yl)phenyl)-7-fluoro-2,3-dihydro-1 H-inden-1-yloxy)-2,3-dihydrobenzofuran-3-yl)acetate (35 mg) in methanol (3 mL). The mixture is stirred at 40°C for 1 hour. After addition of 1 N hydrochloric acid (167 µL) the mixture is diluted with ethyl acetate and washed with brine. The organic phase is dried (MgSO₄) and concentrated. The product thus obtained is dissolved in 1,4-dioxane and lyophilized to give the title compound. Yield: 29 mg; LC (method 11): t_{R} = 1.14 min; Mass spectrum (ESI⁺): m/z = 511 [M+H]⁺.

### Example 44

### 2-((S)-6-((R)-4-(2,6-Dimethyl-4-(oxazol-2-yl)phenyl)-7-fluoro-2,3-dihydro-1H-inden-1-yloxy)-2,3-dihydrobenzofuran-3-yl)acetic acid

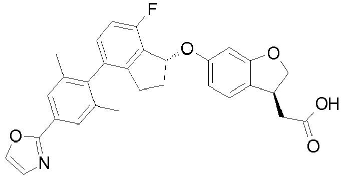

The title compound is prepared from methyl 2-((S)-6-((R)-4-(2,6-dimethyl-4-(oxazol-2-yl)phenyl)-7-fluoro-2,3-dihydro-1 H-inden-1-yloxy)-2,3-dihydrobenzofuran-3-yl)acetate following a procedure analogous to that described in example 43. LC (method 11): t_{R} = 1.19 min; Mass spectrum (ESI⁺): m/z = 500 [M+H]⁺.

### Example 45

### 2-((S)-6-((R)-4-(2,6-Dimethyl-4-(1-methyl-1H-pyrazol-4-yl)phenyl)-7-fluoro-2,3-dihydro-1H-inden-1-yloxy)-2,3-dihydrobenzofuran-3-yl)acetic acid

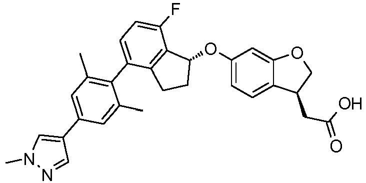

The title compound is prepared from methyl 2-((S)-6-((R)-4-(2,6-dimethyl-4-(1-methyl-1H-pyrazol-4-yl)phenyl)-7-fluoro-2,3-dihydro-1H-inden-1-yloxy)-2,3-dihydrobenzofuran-3-yl)acetate following a procedure analogous to that described in example 43. LC (method 11): t_{R} = 1.15 min; Mass spectrum (ESI⁺): m/z = 513 [M+H]⁺.

### Example 46

### 2-((S)-6-((R)-4-(2,6-Dimethyl-4-(1-methyl-1H-pyrazol-5-yl)phenyl)-7-fluoro-2,3-dihydro-1H-inden-1-yloxy)-2,3-dihydrobenzofuran-3-yl)acetic acid

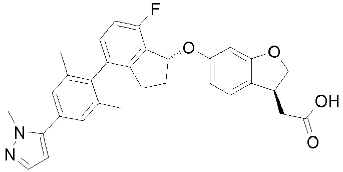

The title compound is prepared from methyl 2-((S)-6-((R)-4-(2,6-dimethyl-4-(1-methyl-1H-pyrazol-5-yl)phenyl)-7-fluoro-2,3-dihydro-1H-inden-1-yloxy)-2,3-dihydrobenzofuran-3-yl)acetate following a procedure analogous to that described in example 43. LC (method 11): t_{R} = 1.16 min; Mass spectrum (ESI⁺): m/z = 513 [M+H]⁺.

### Example 47

### 2-((S)-6-((R)-4-(4-(2-Cyanolprolpan-2-yloxy)-2,6-dimethyliphenyl)-7-fluoro-2,3-dihydro-1H-inden-1-yloxy)-2,3-dydrobenzofuran-3-yl)acetic acid

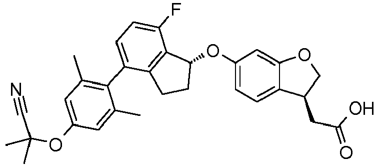

1 M aqueous LiOH solution (500 µL) is added to a solution of methyl 2-((S)-6-((R)-4-(4-(2-cyanopropan-2-yloxy)-2,6-dimethylphenyl)-7-fluoro-2,3-dihydro-1H-inden-1-yloxy)-2,3-dihydrobenzofuran-3-yl)acetate (130 mg) in tetrahydrofuran (2 mL). The mixture is stirred at room temperature for 48 hours. 1 N Hydrochloric acid (500 µL) is added and the residue is purified by HPLC on reversed phase. The product thus obtained is chromatographed on silica gel (petrole ether/ethyl acetate 80:20→20:80) to give the title compound. Yield: 23 mg; LC (method 12): t_{R} = 0.67 min; Mass spectrum (ESI⁺): m/z = 516 [M+H]⁺.

### Example 48

### 2-((S)-6-((R)-4-(4-(3-(Dimethylamino)-2,2-dimethylpropoxy)-2,6-dimethylphenyl)-7-fluoro-2,3-dihydro-1H-inden-1-yloxy)-2,3-dihydrobenzofuran-3-yl)acetic acid

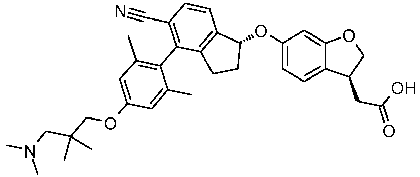

1 M Aqueous NaOH solution (70 µL) is added to a solution of methyl 2-((S)-6-((R)-4-(4-(3-(dimethylamino)-2,2-dimethylpropoxy)-2,6-dimethylphenyl)-7-fluoro-2,3-dihydro-1 H-inden-1-yloxy)-2,3-dihydrobenzofuran-3-yl)acetate (20 mg) in methanol (2 mL) at room temperature. The mixture is stirred at room temperature for 48 hours, treated with 1 M aqueous NaOH solution (70 µL) and heated for 4 hours at 50°C. The mixture is neutralized by addition of 1 N aqueous HCl solution and purified by HPLC on reversed phase to give the title compound. Yield: 11 mg; LC (method 14): t_{R} = 0.95 min; Mass spectrum (ESI⁺): m/z = 562 [M+H]⁺.

### Example 48a

### ((S)-6-{(R)-5-Cyano-4-[4-(3-methanesulfonyl-propoxy)-2,6-dimethyl-phenyl-indan-1-yloxy}-2,3-dihydro-benzofuran-3-yl)-acetic acid

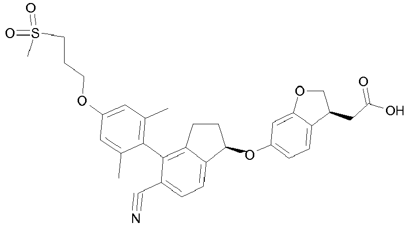

Intermediate 33a (45 mg) and 1 M water solution of NaOH (0.11 mL) are stirred in 2.5 mL of 2-propanol at room temperature for 12 h. The solvent is removed and a 10% water solution of citric acid (10 mL) is added. The reaction mixture is extracted with dichloromethane and the organic phase is separated, dried over sodium sulfate and concentrated under vacuum to give the title compound. Yield: 21 mg. LC (method 20): t_{R} = 6.29 min; Mass spectrum (ESI⁺): m/z = 576 [M+H]⁺.

The following examples are prepared in analogy to Example 48a starting from the correspondent ester intermediates.

| **Example No** | **Starting intermedi ate** | **Structure** | **Name** |
|---|---|---|---|
| **48b** | **33b** | 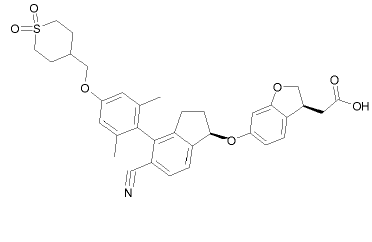 | ((S)-6-{(R)-5-Cyano-4-[4-(1,1-dioxo-hexahydro-1-thiopyran-4-ylmethoxy)-2,6-dimethyl-phenyl]-indan-1-yloxy}-2,3-dihydro-benzofuran-3-yl)-acetic acid |
| **48c** | **33c** | 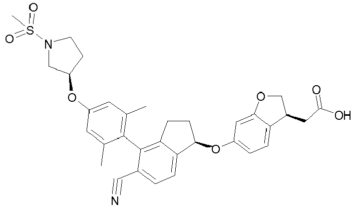 | ((S)-6-{(R)-5-Cyano-4-[4-((R)-1-methanesulfonyl-pyrrolidin-3-yloxy)-2,6-dimethyl-phenyl]-indan-1-yloxy}-2,3-dihydro-benzofuran-3-yl)-acetic acid |

| **Example No** | **t_{R} [Min]** | **LC method No** | **(ESI⁺) or (ESI⁻): m/z** |
|---|---|---|---|
| **48b** | 6,55 | 20 | 602 [M+H]+ |
| **48c** | 2,63 | 20B | 603 [M+H]+ |

### Example 49

### 2-((S)-6-((R)-4-(2,6-Dimethyl-4-(2-(2-oxopyrrolidin-1-yl)ethoxy)phenyl)-7-fluoro-2,3-dihydro-1H-inden-1-yloxy)-2,3-dihydrobenzofuran-3-yl)acetic acid

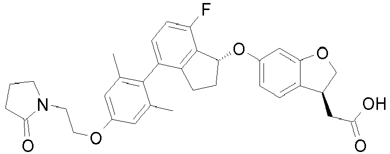

The title compound is prepared from methyl 2-((S)-6-((R)-4-(2,6-dimethyl-4-(2-(2-oxopyrrolidin-1-yl)ethoxy)phenyl)-7-fluoro-2,3-dihydro-1H-inden-1-yloxy)-2,3-dihydrobenzofuran-3-yl)acetate following a procedure analogous to that described in example 10. LC (method 8): t_{R} = 0.35 min; Mass spectrum (ESI⁺): m/z = 560 [M+H]⁺.

### Example 50

### 2-((S)-6-((R)-4-(4-(3,6-dihydro-2H-pyran-4-yl)-2,6-dimethylphenyl)-7-fluoro-2,3-dihydro-1H-inden-1-yloxy)-2,3-dihydrobenzofuran-3-yl)acetic acid

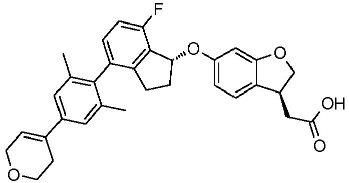

The title compound is prepared from methyl 2-((S)-6-((R)-4-(4-(3,6-dihydro-2H-pyran-4-yl)-2,6-dimethylphenyl)-7-fluoro-2,3-dihydro-1H-inden-1-yloxy)-2,3-dihydrobenzofuran-3-yl)acetate following a procedure analogous to that described in example 10. The reaction is run for 12 hours at room temperature and for 3 hours at 50°C. LC (method 8): t_{R} = 0.71 min; Mass spectrum (ESI⁺): m/z = 515 [M+H]⁺.

### Example 51

### 2-((S)-6-((R)-4-(2,6-dimethyl-4-(1-(methylsulfonyl)-1,2,3,6-tetrahydropyridin-4-yl)phenyl)-7-fluoro-2,3-dihydro-1H-inden-1-yloxy)-2,3-dihydrobenzofuran-3-yl)acetic acid

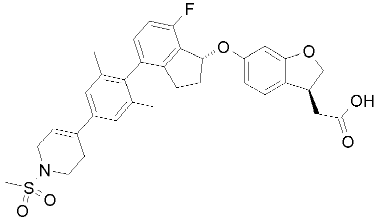

The title compound is prepared from methyl 2-((S)-6-((R)-4-(2,6-dimethyl-4-(1-(methylsulfonyl)-1,2,3,6-tetrahydropyridin-4-yl)phenyl)-7-fluoro-2,3-dihydro-1H-inden-1-yloxy)-2,3-dihydrobenzofuran-3-yl)acetate following a procedure analogous to that described in example 10. The reaction is run for 12 hours at room temperature and for 3 hours at 50°C. LC (method 8): t_{R} = 0.51 min; Mass spectrum (ESI⁺): m/z = 592 [M+H]⁺.

### Example 52

### 2-((S)-6-((R)-4-(2,6-Dimethyl-4-(1-(methylsulfonyl)azetidin-3-yloxy)phenyl)-7-fluoro-2,3-dihydro-1H-inden-1-yloxy)-2,3-dihydrobenzofuran-3-yl)acetic acid

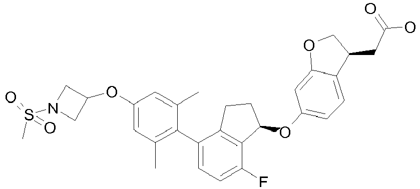

Aqueous NaOH solution (1 M; 39 µL) is added to a solution of methyl 2-((S)-6-((R)-4-(2,6-dimethyl-4-(1-(methylsulfonyl)azetidin-3-yloxy)phenyl)-7-fluoro-2,3-dihydro-1H-inden-1-yloxy)-2,3-dihydrobenzofuran-3-yl)acetate (Intermediate 51 a, 50 mg) in water (0.5 mL) and isopropanol (0.5 mL) at ambient temperature. The mixture is stirred at ambient temperature for 12 hours. The mixture is concentrated and acidified with 10% aqueous citric acid solution. The resulting mixture is extracted with dochloromethane, and the extract dried (MgSO₄). The solvent is evaporated and the residue is chromatographed on silica gel (cyclohexane/ethyl acetate 80:30→0:100) to give the title compound. Yield: 35 mg.
LC (method 20): t_{R} = 6.19 min; Mass spectrum (ESI⁻): m/z = 580 [M-H]⁻.

The examples in the following table are prepared from the corresponding starting ester intermediates following a procedure analogous to that described for Example 52.

| **Example No** | **Starting intermedi ate** | **Structure** | **Name** |
|---|---|---|---|
| **53** | **51b** | 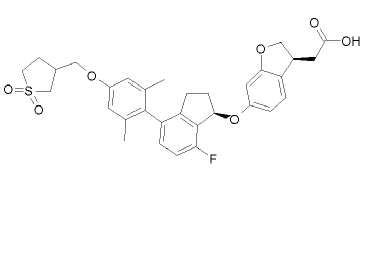 | 2-((3S)-6-((1R)-4-(2,6-Dimethyl-4-((1,1-dioxo-tetrahydrothiophen-3-yl)methoxy)phenyl)-7-fluoro-2,3-dihydro-1H-inden-1-yloxy)-2,3-dihydrobenzofuran-3-yl)acetic acid |
| **54** | **51c** | 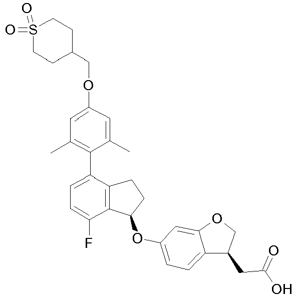 | 2-((S)-6-((R)-4-(2,6-Dimethyl-4-((1,1-dioxo-tetrahydro-2H-thiopyran-4-yl)methoxy)phenyl)-7-fluoro-2,3-dihydro-1H-inden-1-yloxy)-2,3-dihydrobenzofuran-3-yl)acetic acid |
| **55** | **51d** | 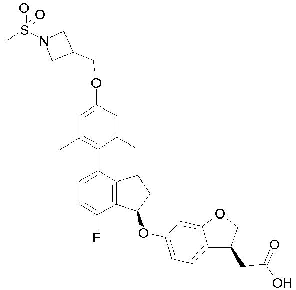 | 2-((S)-6-((R)-4-(2,6-Dimethyl-4-((1-(methylsulfonyl)azetidin-3-yl)methoxy)phenyl)-7-fluoro-2,3-dihydro-1H-inden-1-yloxy)-2,3-dihydrobenzofuran-3-yl)acetic acid |
| **56** | **51e** | 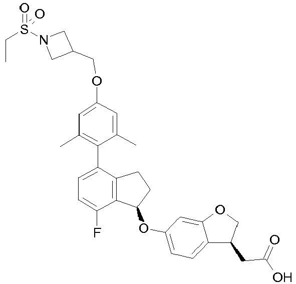 | 2-((S)-6-((R)-4-(4-((1-(Ethylsulfonyl)azetidin-3-yl)methoxy)-2,6-dimethylphenyl)-7-fluoro-2,3-dihydro-1H-inden-1-yloxy)-2,3-dihydrobenzofuran-3-yl)acetic acid |
| **57** | **51f** | 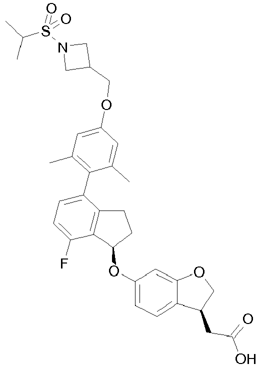 | 2-((S)-6-((R)-7-Fluoro-4-(4-((1-(isopropylsulfonyl)azetidin-3-yl)methoxy)-2,6-dimethylphenyl)-2,3-dihydro-1H-inden-1-yloxy)-2,3-dihydrobenzofuran-3-yl)acetic acid |
| **58** | **51g** | 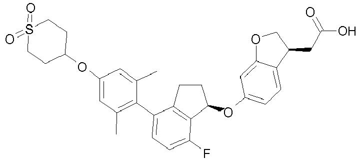 | 2-((S)-6-((R)-4-(2,6-Dimethyl-4-(1,1-dioxo-tetrahydro-2H-thiopyran-4-yloxy)phenyl)-7-fluoro-2,3-dihydro-1H-inden-1-yloxy)-2,3-dihydrobenzofuran-3-yl)acetic acid |
| **59** | **51h** | 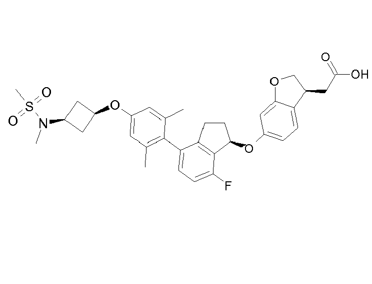 | 2-((S)-6-((R)-4-(2,6-Dimethyl-4-((1s,3S)-3-(N-methylmethylsulfonamido)cycl obutoxy)phenyl)-7-fluoro-2,3-dihydro-1H-inden-1-yloxy)-2,3-dihydrobenzofuran-3-yl)acetic acid |
| **60** | **51i** | 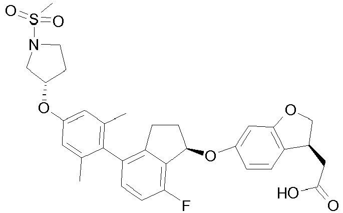 | 2-((S)-6-((R)-4-(2,6-Dimethyl-4-((S)-1-(methylsulfonyl)pyrrolidin-3-yloxy)phenyl)-7-fluoro-2,3-dihydro-1H-inden-1-yloxy)-2,3-dihydrobenzofuran-3-yl)acetic acid |
| **61** | **51k** | 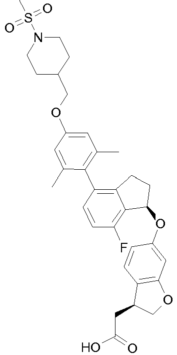 | 2-((S)-6-((R)-4-(2,6-Dimethyl-4-((1-(methylsulfonyl)piperidin-4-yl)methoxy)phenyl)-7-fluoro-2,3-dihydro-1H-inden-1-yloxy)-2,3-dihydrobenzofuran-3-yl)acetic acid |
| **62** | **511** | 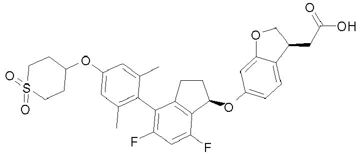 | 2-((S)-6-((R)-4-(2,6-Dimethyl-4-(1,1-dioxo-tetrahydro-2H-thiopyran-4-yloxy)phenyl)-5,7-difluoro-2,3-dihydro-1H-inden-1-yloxy)-2,3-dihydrobenzofuran-3-yl)acetic acid |
| **62a** | **51n** | 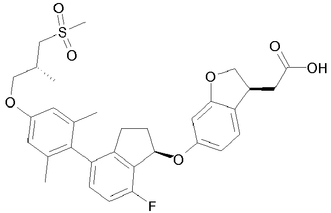 | ((S)-6-{(R)-7-Fluoro-4-[4-((R)-3-methanesulfonyl-2-methyl-propoxy)-2,6 -dimethyl-phenyl]-indan-1-yloxy}-2,3-dihydro-benzofuran-3-yl)-acetic acid |
| **62b** | **51o** | 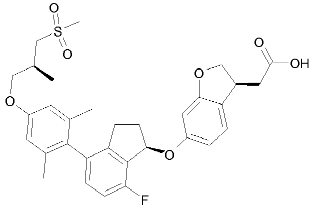 | ((S)-6-{(R)-7-Fluoro-4-[4-((S)-3-methanesulfonyl-2-methyl-propoxy)-2,6-dimethyl-phenyl]-indan-1-yloxy}-2,3-dihydro-benzofuran-3-yl)-acetic acid |
| **62c** | **51p** | 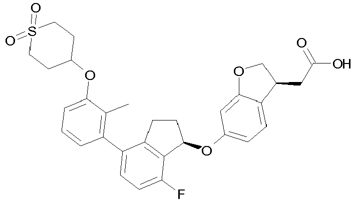 | ((S)-6-{(R)-4-[3-(1,1-Dioxo-hexahydro-1-thiopyran-4-yloxy)-2-methyl-phenyl]-7-fluoro-indan-1-yloxy}-2,3-dihydro-benzofuran-3-yl)-acetic acid |
| **62d** | **51q** | 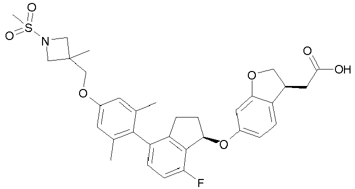 | ((S)-6-{(R)-7-Fluoro-4-[4-(1-methanesulfonyl-3-methyl-azetidin-3-ylmethoxy)-2,6-dimethyl-phenyl]-indan-1-yloxy}-2,3-dihydro-benzofuran-3-yl)-acetic acid |
| **62e** | **51r** | 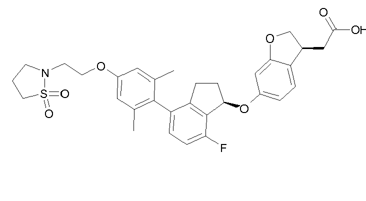 | [(S)-6-((R)-4-{4-[2-(1,1-Dioxo-1-isothiazolidin-2-yl)-ethoxy]-2,6-dimethyl-phenyl}-7-fluoro-indan-1-yloxy)-2,3-dihydro-benzofuran-3-yl]-acetic acid |
| **62f** | **51s** | 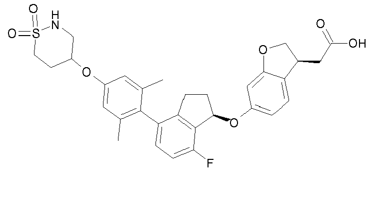 | ((S)-6-{(R)-4-[4-(1,1-Dioxo-1-[1,2]thiazinan-4-yloxy)-2,6-dimethyl-phenyl]-7-fluoro-indan-1-yloxy}-2,3-dihydro-benzofuran-3-yl)-acetic acid |
| **62g** | **51t** | 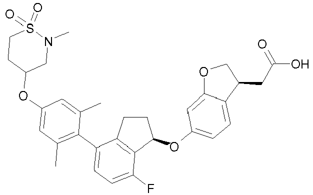 | ((S)-6-{(R)-4-[2,6-Dimethyl-4-(2-methyl-1,1-dioxo-1-[1,2]thiazinan-4-yloxy)-phenyl]-7-fluoro-indan-1-yloxy}-2,3-dihydro-benzofuran-3-yl)-acetic acid |
| **62h** | **51u** | 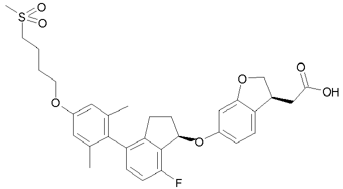 | ((S)-6-{(R)-7-Fluoro-4-[4-(4-methanesulfonyl-butoxy)-2,6-dimethyl-phenyl]-indan-1-yloxy}-2,3-dihydro-benzofuran-3-yl)-acetic acid |
| **62i** | **511a** | 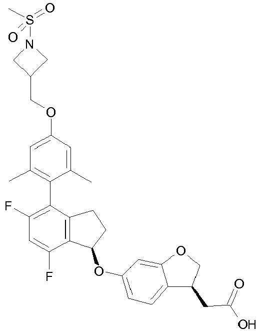 | ((S)-6-{(R)-5,7-Difluoro-4-[4-(1-methanesulfonyl-azetidin-3-ylmethoxy)-2,6-dimethyl-phenyl]-indan-1-yloxy}-2,3-dihydro-benzofuran-3-yl)-acetic acid3-yl)-acetic acid |
| **62j** | **51v** | 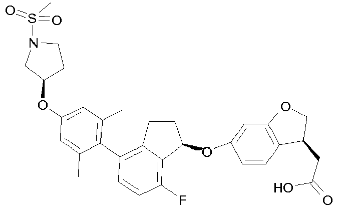 | ((S)-6-{(R)-7-Fluoro-4-[4-((R)-1-methanesulfonyl-pyrrolidin-3-yloxy)-2 ,6-dimethyl-phenyl]-indan-1-yloxy}-2,3-dihydro-benzofuran-3-yl)-aceticacid |
| **62k** | **51w** | 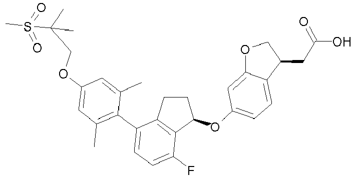 | ((S)-6-{(R)-7-Fluoro-4-[4-(2-methanesulfonyl-2-methyl-propoxy)-2,6-dimethyl-phenyl]-indan-1-yloxy}-2,3-di hydro-benzofuran-3-yl)-acetic acid |
| **62l** | **51x** | 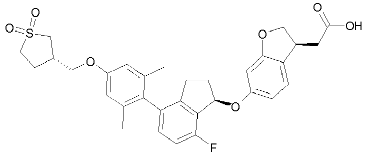 | ((S)-6-{(R)-4-[4-((S)-1,1-Dioxo-tetrahydro-1-thiophen-3-ylmethoxy)-2,6-dimethylphenyl]-7-fluoro -indan-1-yloxy}-2,3-dihydro-benzofuran-3-yl)-acetic acid. Absolute configuration unknown |
| **62m** | **51y** | 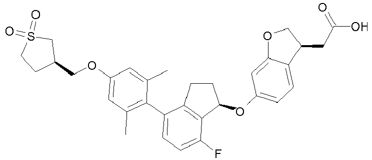 | ((S)-6-{(R)-4-[4-((R)-1,1-Dioxo-tetahydro-1-thiophen-3-ylmethoxy)-2,6-dimethylphenyl]-7-fluoro-indan-1-yloxy}-2,3-dihydro-benzofuran-3-yl)-acetic acid Absolute configuration unknown |
| **62n** | **51z** | 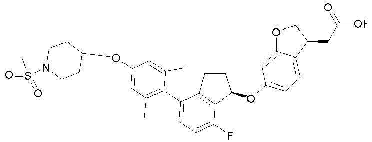 | ((S)-6-{(R)-7-Fluoro-4-[4-(1-methanesulfonyl-piperidin-4-yloxy)-2,6-dimethyl-phenyl]-indan-1-yloxy}-2,3-dihydro-benzofuran-3-yl)-acetic acid |
| **62o** | **51za** | 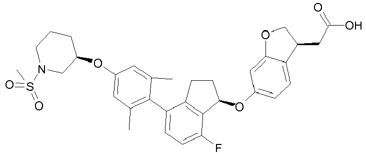 | ((S)-6-{(R)-7-Fluoro-4-[4-((R)-1-methanesulfonyl-piperidin-3-yloxy)-2,6-dimethyl-phenyl]-indan-1-yloxy}-2,3-dihydro-benzofuran-3-yl)-acetic acid |
| **62p** | **51zb** | 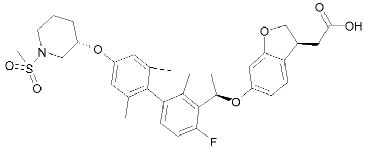 | ((S)-6-{(R)-7-Fluoro-4-[4-((S)-1-methanesulfonyl-piperidin-3-yloxy)-2,6-dimethyl-phenyl]-indan-1-yloxy}-2,3-dihydro-benzofuran-3-yl)-acetic acid |
| **62q** | **51zc** | 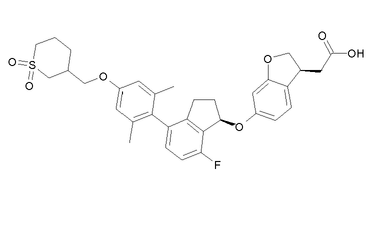 | ((S)-6-{(R)-4-[4-(1,1-Dioxo-hexahydro-1-thiopyran-3-ylmethoxy)-2,6-dimethylphenyl]-7-fluoro-indan-1-yloxy}-2,3-dihydro-benzofuran-3-yl)-acetic acid |
| **62r** | **51zf** | 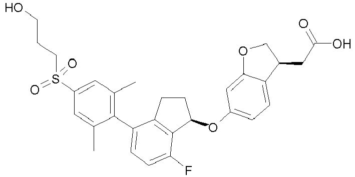 | ((S)-6-{(R)-7-Fluoro-4-[4-(3-hydroxy-propane-1-sulfonyl)-2,6-dimethyl-phenyl]-indan-1-yloxy}-2,3-dihydro-benzofuran-3-yl)-acetic acid |
| **62s** | **51zg** | 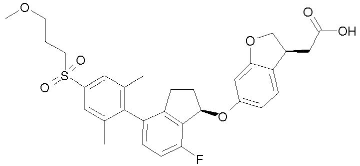 | ((S)-6-{(R)-7-Fluoro-4-[4-(3-methoxy-propane-1-sulfonyl)-2,6-dimethyl-phenyl]-indan-1-yloxy}-2,3-dihydro-benzofuran-3-yl)-acetic acid |
| **62t** | **66a** | 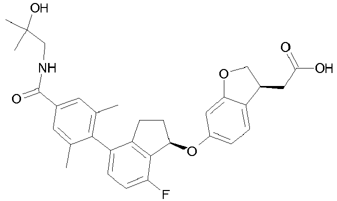 | ((S)-6-{(R)-7-Fluoro-4-[4-(2-hydroxy-2-methylpropylcarbamoyl)-2,6-dimethyl-phenyl]-indan-1-yloxy}-2,3-dihydro-benzofuran-3-yl)-acetic acid |
| **62u** | **51zd** | 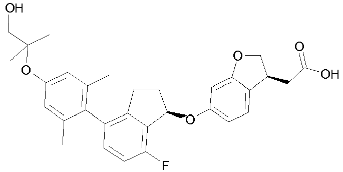 | ((S)-6-{(R)-7-Fluoro-4-[4-(2-hydroxy-1,1-dimethyl-ethoxy)-2,6-dimethyl-phenyl]-indan-1-yloxy}-2,3-dihydro-benzofuran-3-yl)-acetic acid |
| **62w** | **51ze** | 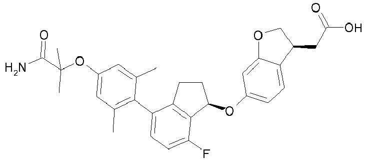 | ((S)-6-{(R)-4-[4-(1-Carbamoyl-1-methyl-ethoxy)-2,6-dimethylphenyl]-7-fluoro-indan-1-yloxy}-2,3-dihydro-benzofuran-3-yl)-acetic acid |

| **Example No** | **t_{R} [Min]** | **LC method No** | **(ESI⁺) or (ESI⁻): m/z** |
|---|---|---|---|
| **53** | 6.44 | 20 | 579 [M-H]⁻ |
| **54** | 6.52 | 20 | 593 [M-H]⁻ |
| **55** | 6.45 | 20 | 596 [M+H]⁺ |
| **56** | 6.67 | 20 | 608 [M-H]⁻ |
| **57** | 13.48 | 21 | 624 [M+H]⁺ |
| **58** | 6.33 | 20 | 579 [M-H]⁻ |
| **59** | 6.93 | 20 | 610 [M+H]⁺ |
| **60** | 12.8 | 21 | 596 [M+H]⁺ |
| **61** | 6.93 | 20 | 624 [M+H]⁺ |
| **62** | 12.61 | 21 | 599 [M+H]⁺ |
| **62a** | 6,88 | 20 | 583 [M+H]+ |
| **62b** | 6,92 | 20 | 583 [M+H]+ |
| **62c** | 8,73 | 20A | 567 [M+H]+ |
| **62d** | 7,07 | 20 | 608 [M-H]- |
| **62e** | 6,33 | 20 | 596 [M+H]+ |
| **62f** | 6,35 | 20 | 580 [M-H]- |
| **62g** | 6,81 | 20 | 594 [M-H]- |
| **62h** | 6,84 | 20 | 583 [M+H]+ |
| **62i** | 6,38 | 20 | 614 [M+H]+ |
| **62j** | 6,47 | 20 | 596 [M+H]+ |
| **62k** | 6,53 | 20 | 583 [M+H]+ |
| **62l** | 6,73 | 20 | 579 [M-H]- |
| **62m** | 6,83 | 20 | 579 [M+H]+ |
| **62n** | 7,20 | 20 | 608 [M-H]- |
| **62o** | 7,10 | 20 | 610 [M+H]+ |
| **62p** | 7,18 | 20 | 610 [M+H]+ |
| **62q** | 7,04 | 20 | 593 [M-H]- |
| **62r** | 5,66 | 20 | 533 [M-H]- |
| **62s** | 6,50 | 20 | 569 [M-H]- |
| **62t** | 7,78 | 20A | 548 [M-H]- |
| **62u** | 6,88 | 20 | 519 [M-H]- |
| **62w** | 6,57 | 20 | 532 [M-H]- |

### Example 62aa

### 2-((S)-6-((R)-4-(4-(3- Hydroxy-3-methylbutoxy)-2,6-dimethylphenyl)-6-(trifluoro methyl)-2,3-dihydro-1H-inden-1-yloxy)-2,3-dihydrobenzofuran-3-yl)acetic acid

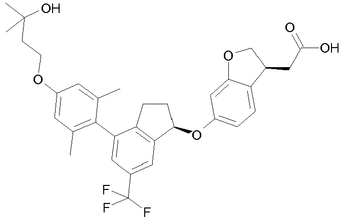

The title compound is prepared in analogy to Example 52, starting from Intermediate 52caa.

The following examples are prepared in analogy to Example 52, starting from the correspondent ester intermediates.

| **Example No** | **Starting intermedi ate** | **Structure** | **Name** |
|---|---|---|---|
| **62ab** | **52cab** | 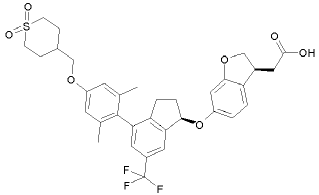 | ((S)-6-{(R)-4-[4-(1,1-Dioxo-hexahydro-1-thiopyran-4-ylmethoxy)-2,6-dimethyl-phenyl]-6-trifluoromethyl-indan-1-yloxy}-2,3dihydro-benzofuran-3-yl)-acetic acid |
| **62ac** | **52ha** | 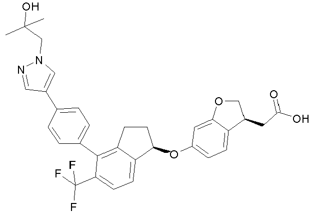 | [(S)-6-((R)-4-{4-[11-(2-Hydroxy-2-methyl-propyl)-1H-pyrazol-4-yl]-pheny 1}-5-trifluoromethyl-indan-1-yloxy)-2,3-dihydro-benzofuran-3-yl]-acetic acid |
| **62ad** | **52hb** | 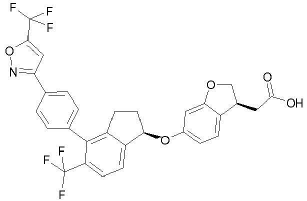 | ((S)-6-{(R)-5-Trifluoromethyl-4-[4-(5-trifluoromethyl-isoxazol-3yl)-phenyl]-indan-1-yloxy}-2,3-dihydro-benzofuran-3-yl)-acetic acid |
| **62ae** | **52ib** | 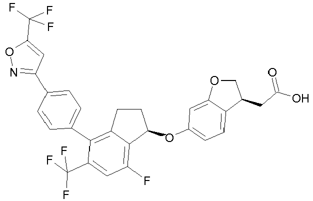 | ((S)-6-{(R)-7-Fluoro-5-trifluoromethyl-4-[4-(5-trifluoromethyl-isoxazol-3-yl)-phenyl]-indan-1-yloxy}-2,3-dihydro-benzofuran-3-yl)-acetic acid |
| **62af** | **52hc** | 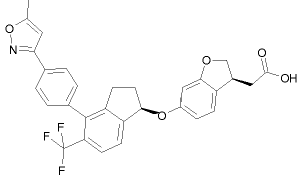 | ((S)-6-{(R)-4-[4-(5-Methyl-isoxazol-3-yl)-phenyl]-5-trifluoromethyl-indan-1-yloxy}-2,3-dihydro-benzofuran-3-yl)-acetic acid |
| **62ag** | **52cac** | 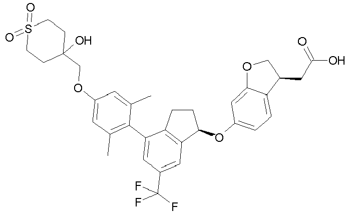 | ((S)-6-{(R)-4-[4-(4-Hydroxy-1,1-dioxo-hexahydro-1-thiopyran-4-ylmethoxy)-2,6-dimethyl-phenyl]-6-trifluoromethyl-indan-1-yloxy}-2,3-dihydro-benzofuran-3-yl)-acetic acid |
| **62ah** | **52dad** | 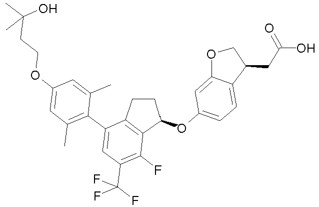 | ((S)-6-{(R)-7-Fluoro-4-[4-(3-hydroxy-3-methyl-butoxy)-2,6-dimethyl-phenyl]-6-trifluoromethyl-indan-1-ylox y}-2,3-dihydro-benzofuran-3-yl)-acetic acid |
| **62ai** | **52daa** | 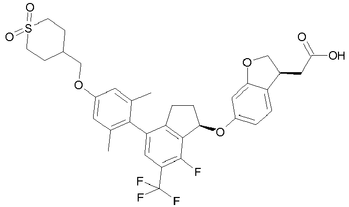 | ((S)-6-{(R)-4-[4-(1,1-Dioxohexahydro-1-thiopyran-4-ylmethoxy)-2,6-dimethylphenyl]-7-fluoro-6-trifluorom.ethyl-indan-1-yloxy}-2,3-dihydro-benzofuran-3-yl)-acetic acid |
| **62aj** | **52dab** | 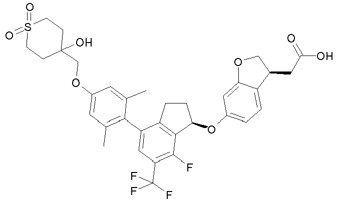 | ((S)-6-{(R)-7-Fluoro-4-[4-(4-hydroxy-1,1-dioxo-hexahydro-1-thiopyran-4-ylmethoxy)-2,6-dimethyl-phenyl]-6-trifluoromethyl-indan-1-yloxy}-2,3-dihydro-benzofuran-3-yl)-acetic acid |
| **62ak** | **52dac** | 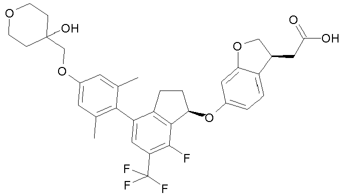 | 2-((S)-6-((R)-7-fluoro-4-(4-((4-hydroxytetrahydro-2H-pyran-4-yl)methoxy)-2,6-dimethyl-phenyl)-6-(trifluoromethyl)-2,3-dihydro-1H-inden-1-yloxy)-2,3-dihydrobenzofuran-3-yl)acetic acid |
| **62al** | **52hzp** | 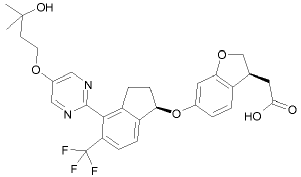 | ((S)-6-{(R)-4-[5-(3-Hydroxy-3-methyl-butoxy)-pyrimidin-2-yl]-5-trifluoromethyl-indan-1-yloxy}-2,3-dihydro-benzofuran-3-yl)-acetic acid |
| **62am** | **52hd** | 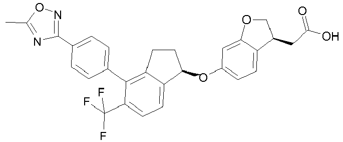 | ((S)-6-{(R)-4-[4-(5-Methyl-[1,2,4]oxadiazol-3-yl)-phenyl]-5-trifluoromethyl-indan-1-yloxy}-2,3-dihydro-benzofuran-3-yl)-acetic acid |
| **62an** | **52ic** | 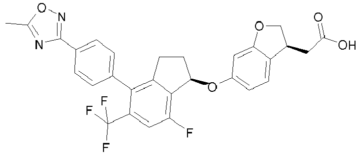 | ((S)-6-{(R)-7-Fluoro-4-[4-(5-methyl-[1,2,4]oxadiazol-3-yl)-phenyl]-5-trifluoromethyl-indan-1-yloxy}-2,3-dihydrobenzofuran-3-yl)-acetic acid |
| **62ao** | **52he** | 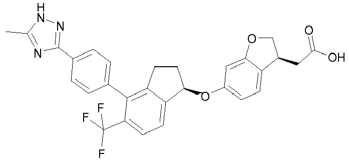 | ((S)-6-{(R)-4-[4-(5-Methyl-1H-[1,2,4]triazol-3-yl)-phenyl]-5-trifluoromethyl-indan-1-yloxy}-2,3-dihydro-benzofuran-3-yl)-acetic acid |
| **62ap** | **52hf** | 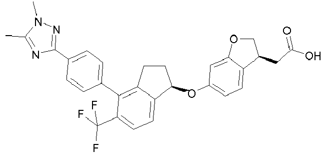 | ((S)-6-{(R)-4-[4-(1,5-Dimethyl-1H-[1,2,4]triazol-3-yl)-phenyl]-5-trifluoromethyl-indan-1-yloxy}-2,3-dihydro-benzofuran-3-yl)-acetic acid |
| **62aq** | **52ie** | 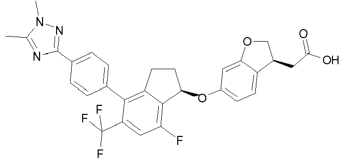 | ((S)-6-{(R)-4-[4-(1,5-Dimethyl-1H-[1,2,4]triazol-3-yl)-phenyl]-7-fluoro-5-trifluoromethyl-indan-1-yloxy}-2,3-dihydro-benzofuran-3-yl)-acetic acid |
| **62ar** | **52hg** | 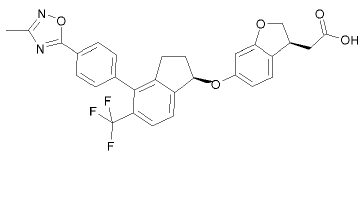 | ((S)-6-{(R)-4-[4-(3-Methyl-[1,2,4]oxadiazol-5-yl)-phenyl]-5-trifluoromethyl-indan-1-yloxy}-2,3-dihydro-benzofuran-3-yl)-acetic acid |
| **62as** | **52hh** | 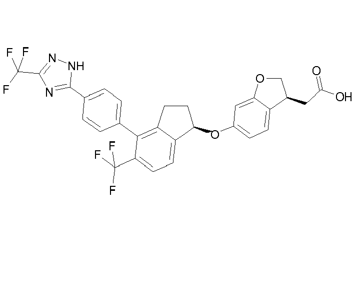 | ((S)-6-{(R)-5-Trifluoromethyl-4-[4-(5-trifluoromethyl-2H-[1,2,4]triazol-3-yl)-phenyl]-indan-1-yloxy}-2,3-dihydro-benzofuran-3-yl)-acetic acid |
| **62at** | **52id** | 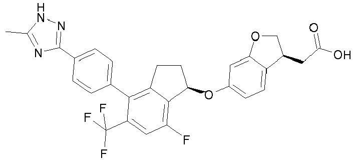 | ((S)-6-{(R)-7-Fluoro-4-[4-(5-methyl-1H-[1,2,4]triazol-3-yl)-phenyl]-5-trifluoromethyl-indan-1-yloxy}-2,3-dihydro-benzofuran-3-yl)-acetic acid |
| **62au** | **52hi** | 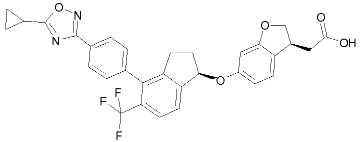 | ((S)-6-{(R)-4-[4-(5-Cyclopropyl-[1,2,4]oxadiazol-3-yl)-phenyl]-5-trifluoromethyl-indan-1-yloxy}-2,3-dihydro-benzofuran-3-yl)-acetic acid |
| **62aw** | **52hj** | 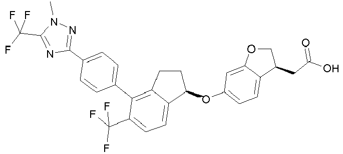 | ((S)-6-{(R)-4-[4-(1-Methyl-5-trifluoromethyl-1H-[1,2,4]triazol-3-yl)-phenyl]-5-trifluoromethyl-indan-1-yloxy}-2,3-dihydro-benzofuran-3-yl)-acetic acid |
| **62ax** | **52hk** | 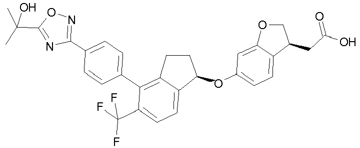 | [(S)-6-((R)-4-{4-[5-(1-Hydroxy-1-methyl-ethyl)-[1,2,4]oxadiazol-3-yl]-phenyl}-5-trifluoromethyl-indan-1-yloxy)-2,3-dihydro-benzofuran-3-yl]-acetic acid |
| **62ay** | **52hzf** | 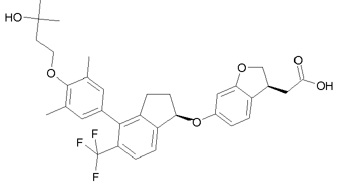 | ((S)-6-{(R)-4-[4-(3-Hydroxy-3-methyl-butoxy)-3,5-dimethyl-phenyl]-5-trifluoromethyl-indan-1-yloxy}-2,3-dihydro-benzofuran-3-yl)-acetic acid |
| **62az** | **52hl** | 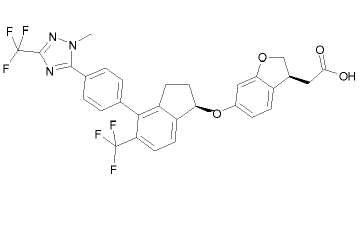 | ((S)-6-{(R)-4-[4-(2-Methyl-5-trifluoromethyl-2H-[1,2,4]triazol-3-yl)-phenyl]-5-trifluoromethyl-indan-1-yloxy}-2,3-dihydro-benzofuran-3-yl)-acetic acid |
| **62ba** | **52hka** | 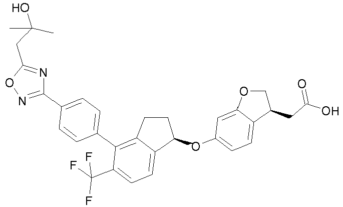 | [(S)-6-((R)-4-{4-[5-(2-Hydroxy-2-methyl-propyl)-[1,2,4]oxadiazol-3-yl]-phenyl}-5-trifluoromethyl-indan-1-yloxy)-2,3-dihydro-benzofuran-3-yl]-acetic acid |
| **62bb** | **52hzm** | 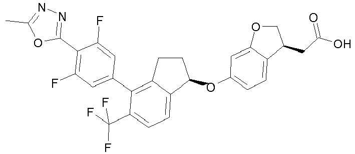 | ((S)-6-{(R)-4-[3,5-Difluoro-4-(5-methyl-[1,3,4]oxadiazol-2-yl)-phenyl]-5-trifluoromethyl-indan-1-yloxy}-2,3-dihydrobenzofuran-3-yl)-acetic acid |
| **62bc** | **52hzn** | 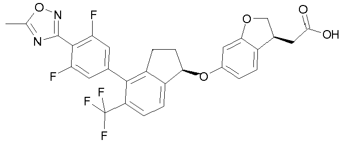 | ((S)-6-{(R)-4-[3,5-Difluoro-4-(5-methyl-[1,2,4]oxadiazol-3-yl)-phenyl]-5-trifluoromethyl-indan-1-yloxy}-2,3-dihydrobenzofuran-3-yl)-acetic acid |
| **62be** | **52hze** | 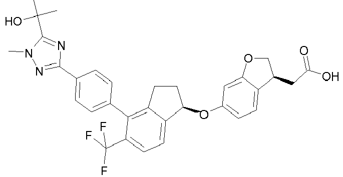 | [(S)-6-((R)-4-{4-[5-(1-Hydroxy-1-methyl-ethyl)-1-methyl-1H-[1,2,4]triazol-3-yl]-phenyl}-5-trifluoromethyl -indan-1-yloxy)-2,3-dihydro-benzofuran-3-yl]-acetic acid |
| **62bf** | **52eaa** | 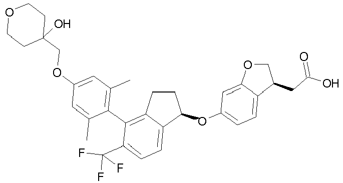 | ((S)-6-{(R)-4-[4-(4-Hydroxy-tetrahydro-pyran-4-ylmethoxy)-2,6-dimethyl -phenyl]-5-trifluoromethyl-indan-1-yloxy}-2,3-dihydro-benzofuran-3-yl)-acetic acid |
| **62bg** | **52eac** | 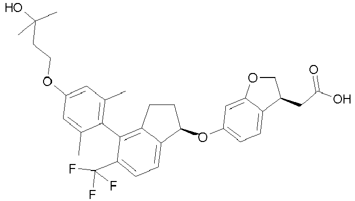 | ((S)-6-{(R)-4-[4-(3-Hydroxy-3-methyl-butoxy)-2,6-dimethylphenyl]-5-tr ifluoromethyl-indan-1-yloxy}-2,3-dihydro-benzofuran-3-yl)-acetic acid |
| **62bh** | **52ead** | 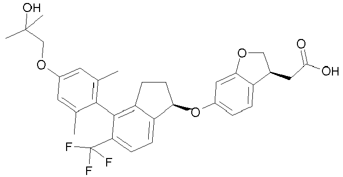 | ((S)-6-{(R)-4-[4-(2-Hydroxy-2-methyl-propoxy)-2,6-dimethyl-phenyl]-5-trifluoromethyl-indan-1-yloxy}-2,3-dihydro-benzofuran-3-yl)-acetic acid |
| **62bi** | **52aaa** | 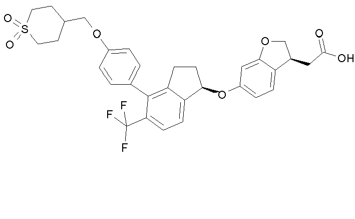 | ((S)-6-{(R)-4-[4-(1,1-Dioxohexahydro-1-thiopyran-4-ylmethoxy)-phenyl]-5-trifluoromethyl-indan-1 -yloxy}-2,3-dihydrobenzofuran-3-yl)-acetic acid |
| **62bj** | **52aaj** | 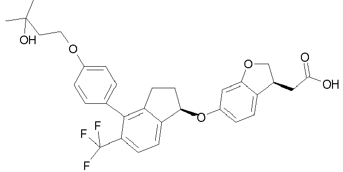 | ((S)-6-{(R)-4-[4-(3-Hydroxy-3-methyl-butoxy)-phenyl]-5-trifluoromethyl-indan-1-yloxy}-2,3-dihydro-benzofuran-3-yl)-acetic acid |
| **62bk** | **52aan** | 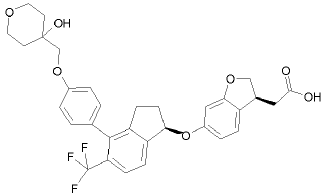 | ((S)-6-{(R)-4-[4-(4-Hydroxytetrahydro-pyran-4-ylmethoxy)-phenyl]-5-trifluoromethyl-indan-1-yloxy}-2,3-dihydrobenzofuran-3-yl)-acetic acid |
| **62bl** | **52cad** | 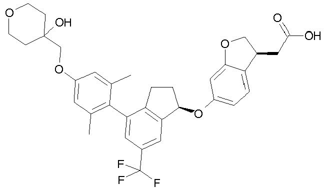 | ((S)-6-{(R)-4-[4-(4-Hydroxytetrahydro-pyran-4-ylmethoxy)-2,6-dimethyl -phenyl]-6-trifluoromethylindan-1-yloxy}-2,3-dihydrobenzofuran-3-yl)-acetic acid |
| **62bm** | **52cae** | 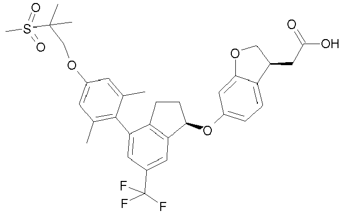 | ((S)-6-{(R)-4-[4-(2-Methanesulfonyl-2-methylpropoxy)-2,6-dimethyl-phenyl]-6-trifluoromethyl-indan-1-yloxy}-2,3-dihydro-benzofuran-3-yl)-acetic acid |
| **62bn** | **52aab** | 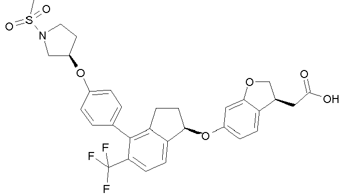 | ((S)-6-{(R)-4-[4-((R)-1-Methanesulfonyl-pyrrolidin-3-yloxy)-phenyl]-5-trifluoromethyl-indan-1-yloxy}-2,3-dihydro-benzofuran-3-yl)-acetic acid |
| **62bo** | **52aac** | 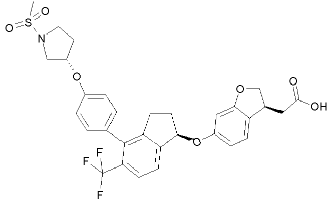 | ((S)-6-{(R)-4-[4-((S)-1-Methanesulfonyl-pyrrolidin-3-yloxy)-phenyl]-5-trifluoromethyl-indan-1-yloxy}-2,3-dihydro-benzofuran-3-yl)-acetic acid |
| **62bp** | **52caf** | 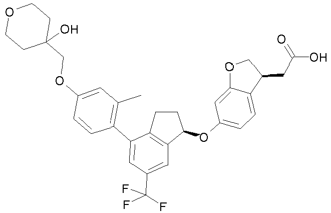 | ((S)-6-{(R)-4-[4-(4-Hydroxy-tetrahydro-pyran-4-ylmethoxy)-2-methyl-phenyl]-6-trifluoromethyl-indan-1-yloxy}-2,3-dihydro-benzofuran-3-yl)-acetic acid |
| **62bq** | **52cag** | 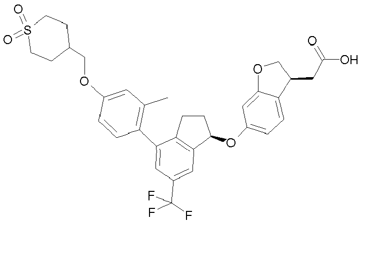 | ((S)-6-{(R)-4-[4-(1,1-Dioxo-hexahydro-1-thiopyran-4-ylmethoxy)-2-methyl-phenyl]-6-trifluoromethyl-indan-1-yloxy}-2,3-dihydro-benzofuran-3-yl)-acetic acid |
| **62br** | **52cah** | 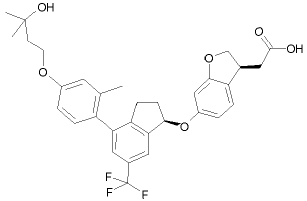 | ((S)-6-{(R)-4-[4-(3-Hydroxy-3-methyl-butoxy)-2-methyl-phenyl]-6-trifluoromethyl-indan-1-yloxy}-2,3-dihydro-benzofuran-3-yl)-acetiacid |
| **62bs** | **52cai** | 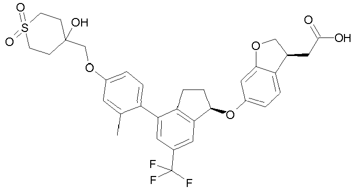 | ((S)-6-{(R)-4-[4-(4-Hydroxy-1,1-dioxo-hexahydro-1-thiopyran-4-ylmethoxy)-2-methyl-phenyl]-6-trifluoromethyl-indan-1-yloxy}-2,3-dihydro-benzofuran-3-yl)-acetic acid |
| **62bt** | **52hla** | 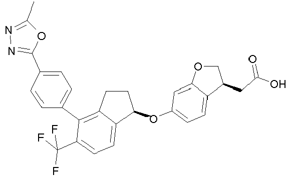 | ((S)-6-{(R)-4-[4-(5-Methyl-[1,3,4]oxadiazol-2-yl)-phenyl]-5-trifluoromethyl-indan-1-yloxy}-2,3-dihydro-benzofuran-3-yl)-acetic acid |
| **62bu** | **52aap** | 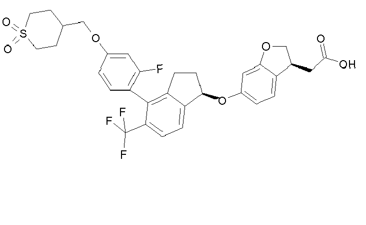 | ((S)-6-{(R)-4-[4-(1,1-Dioxo-hexahydro-1-thiopyran-4-ylmethoxy)-2-fluoro-phenyl]-5-trifluoromethyl-indan-1-yloxy}-2,3-dihydro-benzofuran-3-yl)-acetic acid |
| **62bw** | **52aad** | 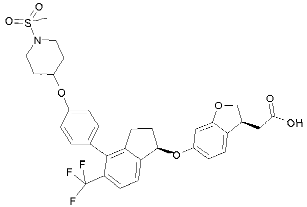 | ((S)-6-{(R)-4-[4-(1-Methanesulfonyl-piperidin-4-yloxy)-phenyl]-5-trifluoromethyl-indan-1-yloxy}-2,3-dihydro-benzofuran-3-yl)-acetic acid |
| **62bx** | **52aae** | 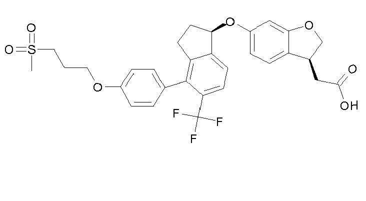 | ((S)-6-{(R)-4-[4-(3-Methanesulfonyl-propoxy)-phenyl]-5-trifluoromethyl-indan-1-yloxy}-2,3-dihydro-benzofuran-3-yl)-acetic acid |
| **62by** | **52hm** | 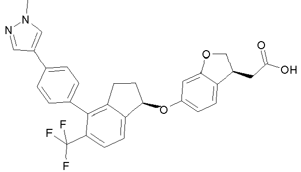 | ((S)-6-{(R)-4-[4-(1-Methyl-1H-pyrazol-4-yl)-phenyl]-5-trifluoromethyl-indan-1-yloxy}-2,3-dihydro-benzofuran-3-yl)-acetic acid |
| **62bz** | **52hn** | 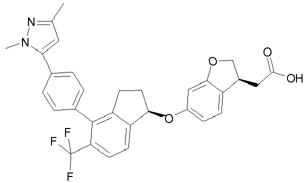 | ((S)-6-{(R)-4-[4-(2,5-Dimethyl-2H-pyrazol-3-yl)-phenyl]-5-trifluoromethyl-indan-1-yloxy}-2,3-dihydro-benzofuran-3-yl)-acetic acid |
| **62ca** | **52ho** | 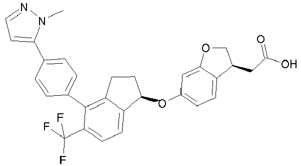 | ((S)-6-{(R)-4-[4-(2-Methyl-2H-pyrazol-3-yl)-phenyl]-5-trifluoromethyl-indan-1-yloxy}-2,3-dihydro-benzofuran-3-yl)-acetic acid |
| **62cb** | **52aai** | 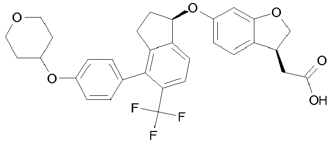 | ((S)-6-{(R)-4-[4-(Tetrahydropyran-4-yloxy)-phenyl]-5-trifluoromethyl-indan-1-yloxy}-2,3-dihydro-benzofuran-3-yl)-acetic acid |
| **62cc** | **52hp** | 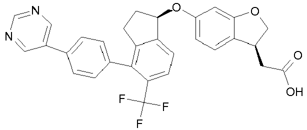 | (S)-6-[(R)-4-(4-Pyrimidin-5-yl-phenyl)-5-trifluoromethyl-indan-1-yloxy]-2,3-dihydro-benzofuran-3-yl}-acetic acid |
| **62cd** | **52aak** | 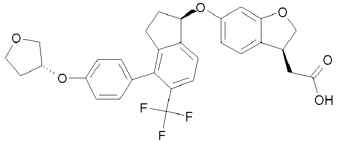 | [(S)-6-((R)-4-{4-[(R)-(Tetrahydro-furan-3-yl)oxy]-phenyl}-5-trifluoromethyl-indan-1-yloxy)-2,3-dihydro-benzofuran-3-yl]-acetic acid |
| **62ce** | **52aal** | 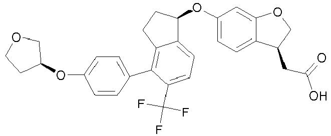 | [(S)-6-((R)-4-{4-[(S)-(Tetrahydro-furan-3-yl)oxy]-phenyl}-5-trifluoromethyl-indan-1-yloxy)-2,3-dihydro-benzofuran-3-yl]-acetic acid |
| **62cf** | **52hra;** | 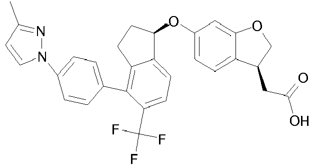 | ((S)-6-{(R)-4-[4-(3-Methyl-pyrazol-1-yl)-phenyl]-5-trifluoromethyl-indan-1-yloxy}-2,3-dihydro-benzofuran-3-yl)-acetic acid |
| **62cg** | **52hrb** | 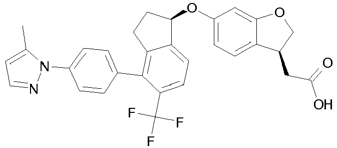 | ((S)-6-{(R)-4-[4-(5-Methyl-pyrazol-1-yl)-phenyl]-5-trifluoromethyl-indan-1-yloxy}-2,3-dihydro-benzofuran-3-yl)-acetic acid |
| **62ch** | **52aaf** | 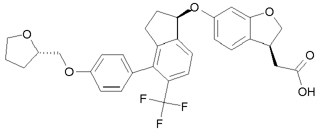 | [(S)-6-((R)-4-{4-[(S)-1-(Tetrahydro-furan-2-yl)methoxy]-phenyl}-5-trifluoromethyl-indan-1-yloxy)-2,3-dihydro-benzofuran-3-yl]-acetic acid |
| **62ci** | **52aag** | 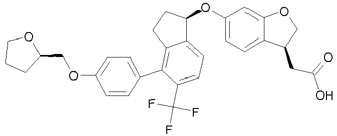 | [(S)-6-((R)-4-{4-[(R)-1-(Tetrahydro-furan-2-yl)methoxy]-phenyl}-5-trifluoromethyl-indan-1-yloxy)-2,3-dihydro-benzofuran-3-yl]-acetic acid |
| **62cj** | **52aam** | 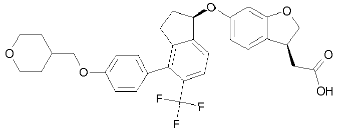 | ((S)-6-{(R)-4-[4-(Tetrahydropyran-4-ylmethoxy)-phenyl]-5-trifluoromethyl-indan-1-yloxy}-2,3-dihydro-benzofuran-3-yl)-acetic acid |
| **62ck** | **52ii** | 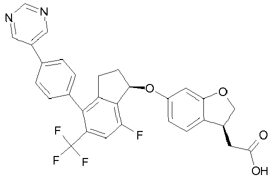 | {(S)-6-[(R)-7-Fluoro-4-(4-pyrimidin-5-yl-phenyl)-5-trifluoromethyl-indan-1-yloxy]-2,3-dihydro-benzofuran-3-yl}-acetic acid |
| **62cl** | **52ij** | 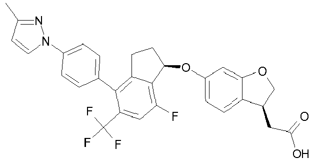 | ((S)-6-{(R)-7-Fluoro-4-[4-(3-methyl-pyrazol-1-yl)-phenyl]-5-trifluoromethyl-indan-1-yloxy}-2,3-dihydro-benzofuran-3-yl)-acetic acid |
| **62cm** | **52aah** | 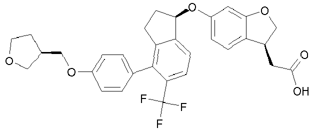 | [(S)-6-((R)-4-{4-[(R)-1-(Tetrahydro-furan-3-yl)methoxy]-phenyl}-5-trifluoromethyl-indan-1-yloxy)-2,3-dihydro-benzofuran-3-yl]-acetic acid |
| **62cn** | **52ik** | 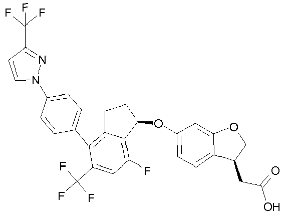 | ((S)-6-{(R)-7-Fluoro-5-trifluoromethyl-4-[4-(3-trifluoromethyl-pyrazol-1-yl)-phenyl]-indan-1-yloxy}-2,3-dihydro-benzofuran-3-yl)-acetic acid |
| **62co** | **52hs** | 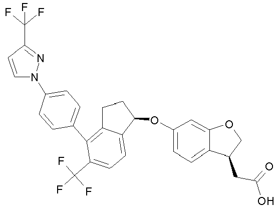 | ((S)-6-{(R)-5-Trifluoromethyl-4-[4-(3-trifluoromethyl-pyrazol-1-yl)-phenyl]-indan-1-yloxy}-2,3-dihydro-benzofuran-3-yl)-acetic acid |
| **62cp** | **52ht** | 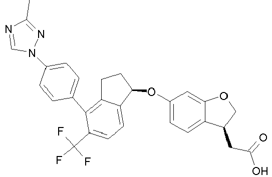 | ((S)-6-{(R)-4-[4-(3-Methyl-[1,2,4]triazol-1-yl)-phenyl]-5-trifluoromethyl-indan-1-yloxy}-2,3-dihydro-benzofuran-3-yl)-acetic acid |
| **62cq** | **52il** | 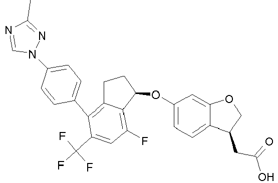 | ((S)-6-{(R)-7-Fluoro-4-[4-(3-methyl-[1,2,4]triazol-1-yl)-phenyl]-5-trifluoromethyl-indan-1-yloxy}-2,3-dihydro-benzofuran-3-yl)-acetic acid |
| **62cr** | **52hzg** | 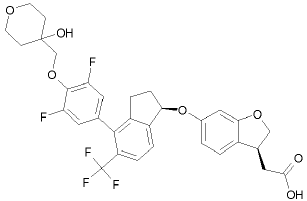 | ((S)-6-{(R)-4-[3,5-Difluoro-4-(4-hydroxy-tetrahydro-pyran-4-ylmethoxy) -phenyl]-5-trifluoromethyl-indan-1-yloxy}-2,3-dihydro-benzofuran-3-yl)-acetic acid |
| **62cs** | **52io** | 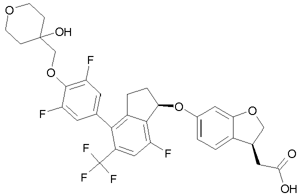 | ((S)-6-{(R)-4-[3,5-Difluoro-4-(4-hydroxy-tetrahydro-pyran-4-ylmethoxy)-phenyl]-7-fluoro-5-trifluoromethyl -indan-1-yloxy}-2,3-dihydro-benzofuran-3-yl)-acetic acid |
| **62ct** | **52hzi** | 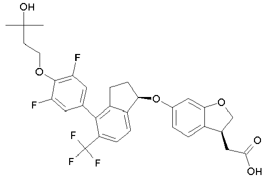 | ((S)-6-{(R)-4-[3,5-Difluoro-4-(3-hydroxy-3-methyl-butoxy)-phenyl]-5-trifluoromethyl-indan-1-yloxy}-2,3-dihydro-benzofuran-3-yl)-acetic acid |
| **62cu** | **52aao** | 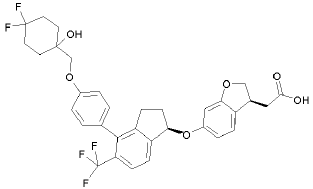 | ((S)-6-{(R)-4-[4-(4,4-Difluoro-1-hydroxy-cyclohexylmethoxy)-phenyl]-5-trifluoromethyl-indan-1-yloxy}-2,3-dihydro-benzofuran-3-yl)-acetic acid |
| **62cw** | **52hu** | 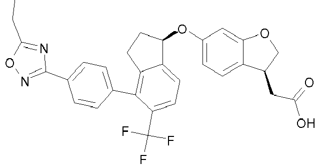 | ((S)-6-{(R)-4-[4-(5-Ethyl-[1,2,4]oxadiazol-3-yl)-phenyl]-5-trifluoromethyl-indan-1-yloxy}-2,3-dihydro-benzofuran-3-yl)-acetic acid |
| **62cx** | **52hw** | 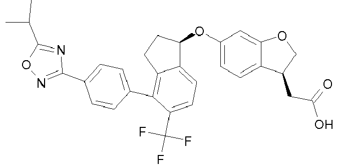 | ((S)-6-{(R)-4-[4-(5-lsopropyl-[1,2,4]oxadiazol-3-yl)-phenyl]-5-trifluoromethyl-indan-1-yloxy}-2,3-dihydro -benzofuran-3-yl)-acetic acid |
| **62cy** | **52hzl** | 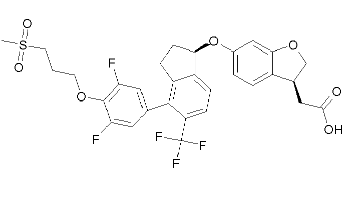 | ((S)-6-{(R)-4-[3,5-Difluoro-4-(3-methanesulfonyl-propoxy)-phenyl]-5-trifluoromethyl-indan-1-yloxy}-2,3-dihydro-benzofuran-3-yl)-acetic acid |
| **62cz** | **52hzh** | 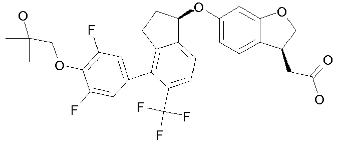 | ((S)-6-{(R)-4-[3,5-Difluoro-4-(2-hydroxy-2-methyl-propoxy)-phenyl]-5-trifluoromethyl-indan-yloxy}-2,3-dihydro-benzofuran-3-yl)-acetic acid |
| **62da** | **52hzj** | 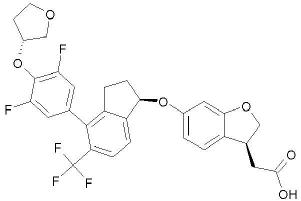 | [(S)-6-((R)-4-{3,5-Difluoro-4-[(R)-(tetrahydro-furan-3-yl)oxy]-phenyl}-5-trifluoromethyl-indan-1-yloxy)-2,3-dihydro-benzofuran-3-yl]-acetic acid |
| **62db** | **52hzk** | 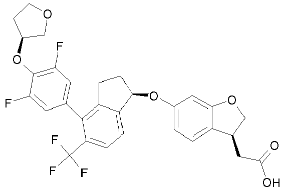 | [(S)-6-((R)-4-{3,5-Difluoro-4-[(S)-(tetrahydro-furan-3-yl)oxy]-phenyl}-5-trifluoromethyl-indan-1-yloxy)-2,3-dihydro-benzofuran-3-yl]-acetic acid |
| **62dc** | **52hx** | 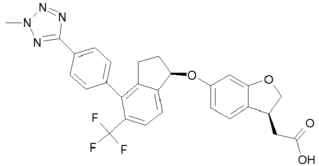 | ((S)-6-{(R)-4-[4-(2-Methyl-2H-tetrazol-5-yl)-phenyl]-5-trifluoromethyl-indan-1-yloxy}-2,3-dihydro-benzofuran-3-yl)-acetic acid |
| **62dd** | **52hzo** | 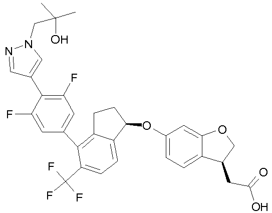 | [(S)-6-((R)-4-{3,5-Difluoro-4-[1-(2-hydroxy-2-methyl-propyl)-1H-pyrazol-4-yl]-phenyl}-5-trifluoromethyl-i ndan-1-yloxy)-2,3-dihydro-benzofuran-3-yl]-acetic acid |
| **62de** | **52eab** | 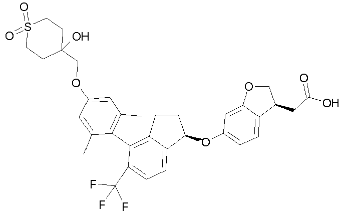 | ((S)-6-{(R)-4-[4-(4-Hydroxy-1,1-dioxo-hexahydro-1-thiopyran-4-ylmethoxy)-2,6-dimethyl-phenyl]-5-trifluoromethyl-indan-1-yloxy}-2,3-dihydro-benzofuran-3-yl)-acetic acid |
| **62df** | **52eae** | 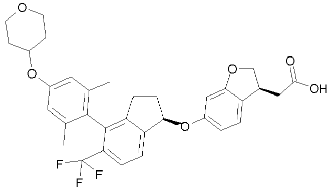 | ((S)-6-{(R)-4-[2,6-Dimethyl-4-(tetrahydro-pyran-4-yloxy)-phenyl]-5-trifluoromethyl-indan-1-yloxy}-2,3-dihydro-benzofuran-3-yl)-acetic acid |
| **62dg** | **52bac** | 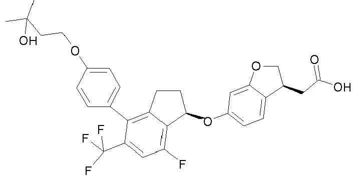 | ((S)-6-{(R)-7-Fluoro-4-[4-(3-hydroxy-3-methyl-butoxy)-phenyl]-5-trifluoromethyl-indan-1-yloxy}-2,3-dihydro-benzofuran-3-yl)-acetic acid |
| **62dh** | **52baa** | 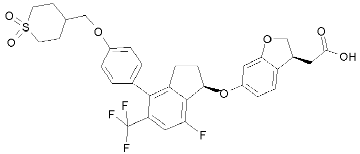 | ((S)-6-{(R)-4-[4-(1,1-Dioxo-hexahydro-1-thiopyran-4-ylmethoxy)-phenyl]-7-fluoro-5-trifluoromethyl-indan-1-yloxy}-2,3-dihydro-benzofuran-3-yl)-acetic acid |
| **62di** | **52bab** | 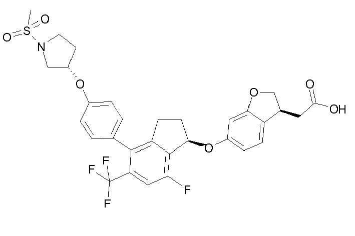 | ((S)-6-{(R)-7-Fluoro-4-[4-((S)-1-methanesulfonyl-pyrrolidin-3-yloxy)-phenyl]-5-trifluoromethyl-indan-1-yl oxy}-2,3-dihydro-benzofuran-3-yl)-acetic acid |
| **62dj** | **52baf** | 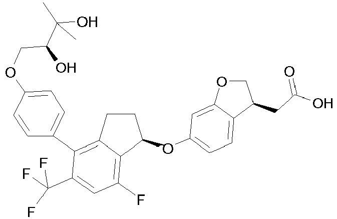 | ((S)-6-{(R)-4-[4-((S)-2,3-Dihydroxy-3-methyl-butoxy)-phenyl]-7-fluoro-5-trifluoromethyl-indan-1-yloxy}-2,3-dihydro-benzofuran-3-yl)-acetic acid Absolute configuration unknown |
| **62dk** | **52bag** | 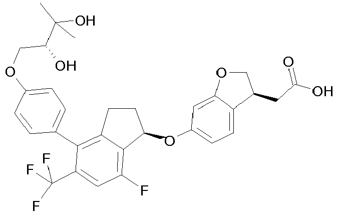 | ((S)-6-{(R)-4-[4-((R)-2,3-Dihydroxy-3-methyl-butoxy)-phenyl]-7-fluoro-5-trifluoromethyl-indan-1-yloxy}-2,3-dihydro-benzofuran-3-yl)-acetic acid Absolute configuration unknown |
| **62dl** | **52ig** | 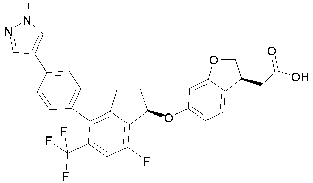 | ((S)-6-{(R)-7-Fluoro-4-[4-(1-methyl-1H-pyrazol-4-yl)-phenyl]-5-trifluoromethyl-indan-1-yloxy}-2,3-dihydro -benzofuran-3-yl)-acetic acid |
| **62dm** | **52ih** | 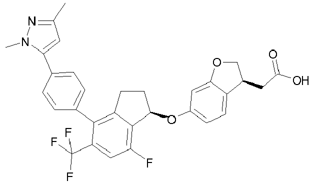 | ((S)-6-{(R)-4-[4-(2,5-Dimethyl-2H-pyrazol-3-yl)-phenyl]-7-fluoro-5-trifluoromethyl-indan-1-yloxy}-2,3-dihydro-benzofuran-3-yl)-acetic acid |
| **62dn** | **52if** | 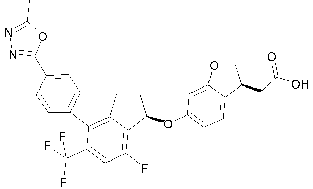 | ((S)-6-{(R)-7-Fluoro-4-[4-(5-methyl-[1,3,4]oxadiazol-2-yl)-phenyl]-5-trifluoromethyl-indan-1-yloxy}-2,3-dihydro-benzofuran-3-yl)-acetic acid |
| **62do** | **52ia** | 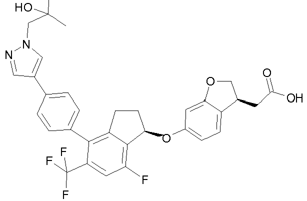 | [(S)-6-((R)-7-Fluoro-4-{4-[1-(2-hydroxy-2-methyl-propyl)-1H-pyrazol-4-yl]-phenyl}-5-trifluoromethyl-indan-1-yloxy)-2,3-dihydro-benzofuran-3-yl]-acetic acid |
| **62dp** | **52hy** | 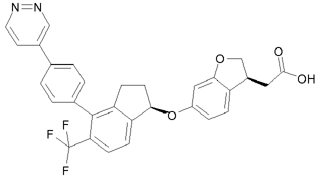 | {(S)-6-[(R)-4-(4-Pyridazin-4-yl-phenyl)-5-trifluoromethyl-indan-1-yloxy]-2,3-dihydro-benzofuran-3-yl}-acetic acid |
| **62dq** | **52im** | 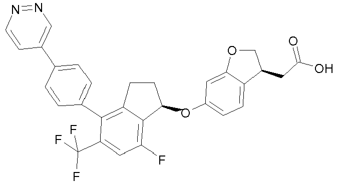 | {(S)-6-[(R)-7-Fluoro-4-(4-pyridazin-4-yl-phenyl)-5-trifluoromethyl-indan-1-yloxy]-2,3-dihydro-benzofuran-3-yl}-acetic acid |
| **62dr** | **52hz** | 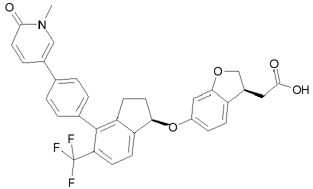 | ((S)-6-{(R)-4-[4-(1-Methyl-6-oxo-1,6-dihydro-pyridin-3-yl)-phenyl]-5-trifluoromethyl-indan-1-yloxy}-2,3-dihydro-benzofuran-3-yl)-acetic acid |
| **62ds** | **52in** | 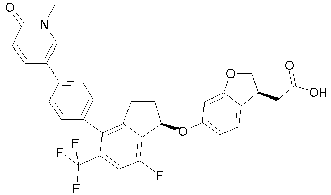 | ((S)-6-{(R)-7-Flouoro-4-[4-(1-methyl-6-oxo-1,6-dihydro-pyridin-3-yl)-phenyl]-5-trifluoromethyl-indan-1-yloxy}-2,3-dihydro-benzofuran-3-yl)-acetic acid |
| **62dt** | **52hza** | 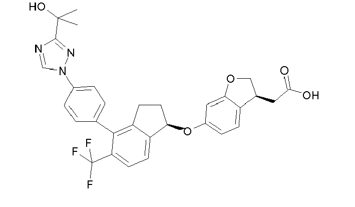 | [(S)-6-((R)-4-{4-[3-(1-Hydroxy-1-methyl-ethyl)-[1,2,4]triazol-1-yl]-phenyl}-5-trifluoromethyl-indan-1-yloxy)-2,3-dihydro-benzofuran-3-yl]-acetic acid |
| **62du** | **52hzb** | 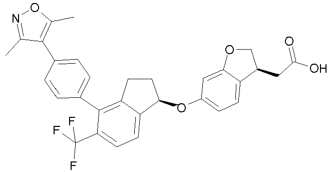 | ((S)-6-{(R)-4-[4-(3,5-Dimethyl-isoxazol-4-yl)-phenyl]-5-trifluoromethyl-indan-1-yloxy}-2,3-dihydro-benzofuran-3-yl)-acetic acid |
| **62dw** | **52hzc** | 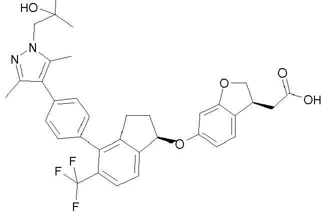 | [(S)-6-((R)-4-{4-[1-(2-Hydroxy-2-methyl-propyl)-3,5-dimethyl-1 H-pyrazo 1-4-yl]-phenyl}-5-trifluoromethyl-indan-1-yloxy)-2,3-dihydro-benzofuran-3-yl]-acetic acid |
| **62dx** | **Step 7 in the preparation of 52ea** | 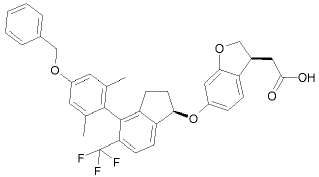 | {(S)-6-[(R)-4-(4-Benzyloxy-2,6-dimethyl-phenyl)-5-trifluoromethyl-indan-1-yloxy]-2,3-dihydro-benzofuran-3-yl}-acetic acid |
| **62dy** | **52hzq** | 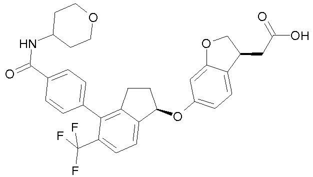 | ((S)-6-{(R)-4-[4-(Tetrahydropyran-4-ylcarbamoyl)-phenyl]-5-trifluoromethyl-indan-1-yloxy}-2,3-dihydro-benzofuran-3-yl)-acetic acid |
| **62dz** | **52eaf** | 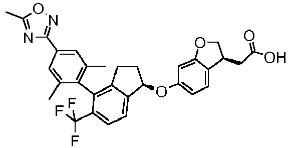 | ((S)-6-{(R)-4-[2,6-Dimethyl-4-(5-methyl-[1,2,4]oxadiazol-3-yl)-phenyl]-5-trifluoromethyl-indan-1-yloxy}-2,3-dihydro-benzofuran-3-yl)-acetic acid |
| **62ea** | **52hzd** | 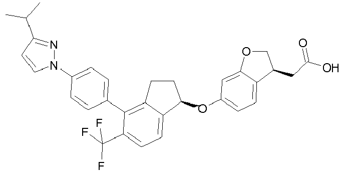 | ((S)-6-{(R)-4-[4-(3-lsopropyl-pyrazol-1-yl)-phenyl]-5-trifluoromethyl-indan-1-yloxy}-2,3-dihydro-benzofuran-3-yl)-acetic acid |

| **Example No** | **t_{R} [Min]** | **LC method No** | **(ESI⁺) or (ESI⁻): m/z** |
|---|---|---|---|
| **62ab** | 3,27 | 20B | 643 [M-H]- |
| **62ac** | 2,85 | 20B | 593 [M+H]+ |
| **62ad** | 8,09 | 20B | 588 [M-H]- |
| **62ae** | 3,93 | 20B | 606 [M-H]- |
| **62af** | 3,56 | 20B | 536 [M+H]+ |
| **62ag** | 2,83 | 20B | 659 [M-H]- |
| **62ah** | 3,60 | 20B | 603 [M+H]+ |
| **62ai** | 3,28 | 20B | 663 [M+H]+ |
| **62aj** | 2,89 | 20B | 679 [M+H]+ |
| **62ak** | 3,22 | 20B | 631 [M+H]+ |
| **62al** | 2,58 | 20B | 559 [M+H]+ |
| **62am** | 3,37 | 20B | 537 [M+H]+ |
| **62an** | 3,16 | 20B | 553 [M-H]- |
| **62ao** | 2,52 | 20B | 536 [M+H]+ |
| **62ap** | 2,74 | 20B | 550 [M+H]+ |
| **62aq** | 2,83 | 20B | 568 [M+H]+ |
| **62ar** | 3,35 | 20B | 537 [M+H]+ |
| **62as** | 3,13 | 20B | 590 [M+H]+ |
| **62at** | 2,57 | 20B | 554 [M+H]+ |
| **62au** | 4,00 | 20B | 563 [M+H]+ |
| **62aw** | 3,76 | 20B | 604 [M+H]+ |
| **62ax** | 3,25 | 20B | 581 [M+H]+ |
| **62ay** | 3,28 | 20B | 585 [M+H]+ |
| **62az** | 3,32 | 20B | 604 [M+H]+ |
| **62ba** | 3,00 | 20B | 595 [M+H]+ |
| **62bb** | 2,82 | 20B | 573 [M+H]+ |
| **62bc** | 3,15 | 20B | 573 [M+H]+ |
| **62be** | 3,06 | 20B | 594 [M+H]+ |
| **62bf** | 2,90 | 20B | 611 [M-H]- |
| **62bg** | 3,23 | 20B | 583 [M-H]- |
| **62bh** | 3,10 | 20B | 569 [M-H]- |
| **62bi** | 6,52 | 20C | 615 [M+H]+ |
| **62bj** | 3,15 | 20B | 555 [M-H]- |
| **62bk** | 2,77 | 20B | 583 [M-H]- |
| **62bl** | 3,00 | 20B | 613 [M+H]+ |
| **62bm** | 3,34 | 20B | 633 [M+H]+ |
| **62bn** | 3,04 | 20B | 618 [M+H]+ |
| **62bo** | 3,02 | 20B | 618 [M+H]+ |
| **62bp** | 2,99 | 20B | 597 [M+H]+ |
| **62bq** | 3,20 | 20B | 629 [M+H]+ |
| **62br** | 4,33 | 20D | 571 [M+H]+ |
| **62bs** | 4,01 | 20E | 647 [M+H]+ |
| **62bt** | 2,83 | 20B | 537 [M+H]+ |
| **62bu** | 3,00 | 20B | 635 [M+H]+ |
| **62bw** | 3,23 | 20B | 630 [M+H]+ |
| **62bx** | 3,13 | 20B | 589 [M-H]- |
| **62by** | 3,13 | 20B | 535 [M+H]+ |
| **62bz** | 3,39 | 20B | 549 [M+H]+ |
| **62ca** | 3,13 | 20B | 535 [M+H]+ |
| **62cb** | 3,31 | 20B | 553 [M-H]- |
| **62cc** | 2,86 | 20B | 531 [M-H]- |
| **62cd** | 3,12 | 20B | 539 [M-H]- |
| **62ce** | 3,12 | 20B | 539 [M-H]- |
| **62cf** | 3,40 | 20B | 535 [M+H]+ |
| **62cg** | 3,23 | 20B | 535 [M+H]+ |
| **62ch** | 3,40 | 20B | 555 [M+H]+ |
| **62ci** | 3,42 | 20B | 555 [M+H]+ |
| **62cj** | 3,65 | 20B | 569 [M+H]+ |
| **62ck** | 2,97 | 20B | 551 [M+H]+ |
| **62cl** | 3,63 | 20B | 553 [M+H]+ |
| **62cm** | 3,28 | 20B | 555 [M+H]+ |
| **62cn** | 3,68 | 20B | 605 [M-H]- |
| **62co** | 3,72 | 20B | 587 [M-H]- |
| **62cp** | 2,80 | 20B | 536 [M+H]+ |
| **62cq** | 2,80 | 20B | 554 [M+H]+ |
| **62cr** | 2,94 | 20B | 619 [M-H]- |
| **62cs** | 2,99 | 20B | 637[M-H]- |
| **62ct** | 3,23 | 20B | 591 [M-H]- |
| **62cu** | 4,34 | 20F | 617 [M-H]- |
| **62cw** | 4,05 | 20B | 551 [M+H]+ |
| **62cx** | 3,74 | 20B | 565 [M+H]+ |
| **62cy** | 2,87 | 20B | 625 [M-H]- |
| **62cz** | 3,05 | 20B | 577 [M-H]- |
| **62da** | 3,26 | 20B | 577 [M+H]+ |
| **62db** | 3,22 | 20B | 577 [M+H]+ |
| **62dc** | 3,10 | 20B | 537 [M+H]+ |
| **62dd** | 3,08 | 20B | 629 [M+H]+ |
| **62de** | 2,81 | 20B | 659 [M-H]- |
| **62df** | 3,44 | 20B | 581 [M-H]- |
| **62dg** | 3,17 | 20B | 575 [M+H]+ |
| **62dh** | 4,28 | 20D | 635 [M+H]+ |
| **62di** | 4,28 | 20E | 636 [M+H]+ |
| **62dj** | 2,76 | 20B | 591 [M+H]+ |
| **62dk** | 2,76 | 20B | 591 [M+H]+ |
| **62dl** | 4,15 | 20D | 553 [M+H]+ |
| **62dm** | 4,26 | 20D | 567 [M+H]+ |
| **62dn** | 2,82 | 20B | 555 [M+H]+ |
| **62do** | 4,08 | 20B | 611 [M+H]+ |
| **62dp** | 2,71 | 20B | 533 [M+H]+ |
| **62dq** | 2,69 | 20B | 551 [M+H]+ |
| **62dr** | 3,15 | 20B | 562 [M+H]+ |
| **62ds** | 3,13 | 20B | 580 [M+H]+ |
| **62dt** | 2,65 | 20B | 580 [M+H]+ |
| **62du** | 3,43 | 20B | 550 [M+H]+ |
| **62dw** | 6,68 | 20C | 621 [M+H]+ |
| **62dx** | 4,17 | 20B | 589 [M+H]+ |
| **62dy** | 2,65 | 20B | 582 [M+H]+ |
| **62dz** | 3,38 | 20B | 565 [M+H]+ |
| **62ea** | 3,95 | 20B | 563 [M+H]+ |

### Example 62eb

### ((S)-6-1(R)-4-[4-(4-Hydroxy-tetrahydro-thiolpyran-4-ylmethoxy)-phenyl]-5-trifluoromethyl-indan-1-yloxy}-2,3-dihydro-benzofuran-3-yl)-acetic acid

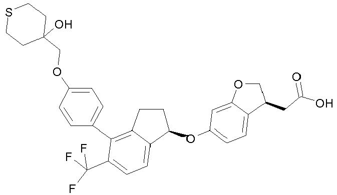

The title compound is prepared following a procedure analogous to that described for Step 1 in the preparation of Intermediate 50bc, starting from Intermediate 52aa'.
LC (method 20C): t_{R} = 7.15 min; Mass spectrum (ESI⁺): m/z = 601 [M+H]⁺.

### Example 62ec

### ((S)-6-{(R)-4-[4-(4-Hydroxy-1,1-dioxo-hexahydro-1-thiopyan-4-ylmethoxy)-phenyl]-5-trifluoromethyl-indan-1-yloxy)-2,3-dihydro-benzofuran-3-yl)-acetic acid

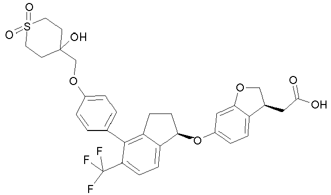

The title compound is prepared following a analogousprocedure analogous to that described for Step 2 in the preparation of Intermediate 50bc, starting from Example 62eb.
LC (method 20B): t_{R} = 2.33 min; Mass spectrum (ESI⁺): m/z = 633 [M+H]⁺.

### Example 62ed

### ((S)-6-1(R)-4-[2-Fluoro-4-(4-hydroxy-1,1-dioxo-hexahydro-1-thiopyran-4-ylmethoxy)-phenyl]-5-trifluoromethyl-indan-1-yloxy}-2,3-dihydro-benzofuran-3-yl)-acetic acid

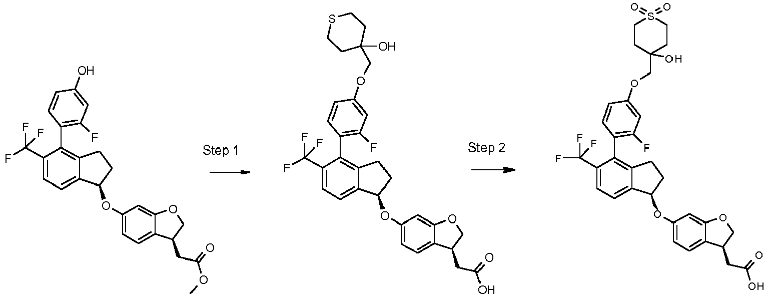

The title compound is prepared following a procedure analogous to that described in the preparation of Intermediate 50bc, starting from Intermediate 52aa".
LC (method 20B): t_{R} = 2.71 min; Mass spectrum (ESI⁺): m/z = 651 [M+H]⁺.

### Example 62ee

### ((S)-6-{(R)-7-Fluoro-4-[4-(4-hydroxy-1,1-dioxo-hexahydro-1-thiopyran-4-ylmethoxy)-phenyl]-5-trifluoromethyl-indan-1-yloxy}-2,3-dihydro-benzofuran-3-yl)-acetic acid

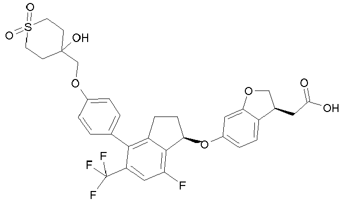

The title compound is prepared in analogy to Example 62ed, starting from Intermediate 52ba. LC (method 20B): t_{R} = 2.71 min; Mass spectrum (ESI⁺): m/z = 651 [M+H]⁺.

### Example 62ef

### ((S)-6-{(R)-4-[4-(2-Hydroxy-2-methyl-propoxy)-phenyl]-5-trifluoromethyl-indan-1-yloxy}-2,3-dihydro-benzofuran-3-yl)-acetic acid

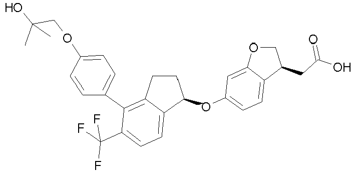

Intermediate 52aa (90 mg) and cesium carbonate (120 g) are suspended in 2 mL of N,N-dimethylformamide, Isobutylene oxide (20 g) is added and the reaction mixture is stirred at 100 °C 3 h. Water is added and the mixture is extracted with ethyl acetate, the organic phase is separated, dried over sodium sulfate and concentrated under vacuum. The residue is chromatographed on silica gel (cyclohexane/ethyl acetate 100:0→60:40) to give the title compound. Yield: 22 mg
LC (method 20B): t_{R} = 3.09 min; Mass spectrum (ESI⁺): m/z = 541 [M+H]⁺.

### Example 62eg

### ((S)-6-{(R)-7-Fluoro-4-[4-(2-hydroxy-2-methyl-propoxy)-phenyl1-5-trifluoromethyl-indan-1-yloxy}-2,3-dihydro-benzofuran-3-yl)-acetic acid

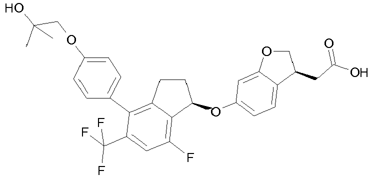

The title compound is prepared in analogy to Intermediate 62ef starting from Intermediate 52ba.
LC (method 20D): t_{R} = 4.00 min; Mass spectrum (ESI⁺): m/z = 561 [M+H]⁺.

### Example 62eh

### ((S)-6-{(R)-7-Fluoro-4-[4-(4-hydroxy-tetrahydro-pyran-4-ylmethoxy)-phenyl]-5-trifluoromethyl-indan-1-yloxy}-2,3-dihydro-benzofuran-3-yl)-acetic acid

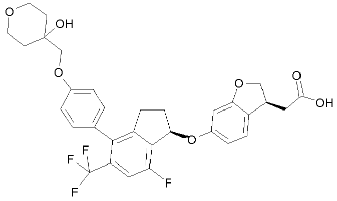

The title compound is prepared in analogy to example 62ef, starting from Intermediate 52ba and 1,6-dioxa-spiro[2.5]octane (US2012/46304 A1, 2012).
LC (method 20B): t_{R} = 2.76 min; Mass spectrum (ESI⁺): m/z = 603[M+H]⁺.

### Example 62ei

### ((S)-6-{(R)-4-r4-(2,3-Dihydroxy-3-methyl-butoxy)-2,6-dimethyl-phenyl]-5-trifluoromethyl-indan-1-yloxy}-2,3-dihydro-benzofuran-3-yl)-acetic acid

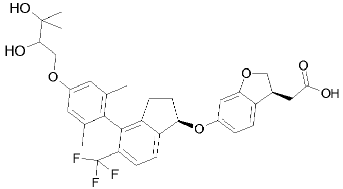

Intermediate 52ea (128 mg I), 2-oxiranyl-propan-2-ol (64 mg) and cesium carbonate (179 mg) are suspended in dry N,N-dimethylformamide and stirred at 100 °C for 5 hours. The mixture is diluted with water, acidified with 1 M aqueous hydrochloric acid and extracted with ethyl acetate. The organice phase is magensium sulfate and the solvent removed under vacuum. The residue is purified by flash chromatography (dichloromethane/methanol/acetic acid 90:1:1) to give the title compound (Yield 45 mg).
LC (method 20B): t_{R} = 2.97 min; Mass spectrum (ESI⁻): m/z = 599 [M-H]⁻.

### Example 62ej

### ((S)-6-{(R)-4-[4-((R)-2,3-Dihydroxy-3-methyl-butoxy)-2,6-dimethyl-phenyl]-5-trifluoromethyl-indan-1-yloxy-2,3-dihydro-benzofuran-3-yl)-acetic acid methyl ester

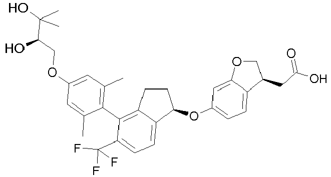

Example 62ei is separated by preparative chiral HPLC to give the title compound. Chiral HPLC (method 1c)::t_{R} = 7.75 min
LC (method 20B): t_{R} = 2.82. Mass spectrum (ESI⁻): m/z = 599 [M-H]⁻.
Absolute configuration unknown.

### Example 62ek

### ((S)-6-1(R)-4-[4-((S)-2,3-Dihydroxy-3-methyl-butoxy)-2,6-dimethyl-phenyl]-5-trifluoromethyl-indan-1-yloxy}-2,3-dihydro-benzofuran-3-yl)-acetic acid

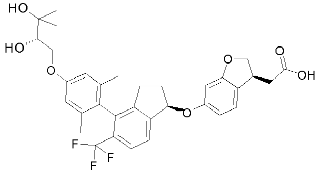

The title compound is obtained from further elution in the chiral HPLC chromatography for the preparation of Example 62ej,
Chiral HPLC (method 1c): t_{R} = 7.84 min
LC (method 20B): t_{R} = 2.83. Mass spectrum (ESI⁻): m/z = 599 [M-H]⁻.
Absolute configuration unknown

### Example 63

### 2-((S)-6-((R)-4-(2,6-Dimethyl-4-(3-(methylsulfonyl)propoxy)phenyl)-7-fluoro-2,3-dihydro-1H-inden-1-yloxy)-2,3-dihydrobenzofuran-3-yl)acetic acid

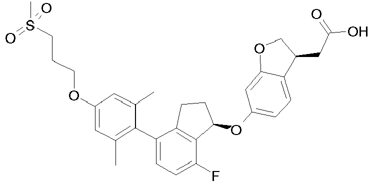

Methyl 2-((S)-6-((R)-4-(2,6-dimethyl-4-(3-(methylsulfonyl)propoxy)phenyl)-7-fluoro-2,3-dihydro-1H-inden-1-yloxy)-2,3-dihydrobenzofuran-3-yl)acetate (Intermediate 53; 9 mg) is suspended in ethanol (1 mL) and aqueous NaOH (1 M; 0.2 mL) is added. The mixture is stirred for 1 hour then concentracted under vacuum, acidified with aqueous HCl (1 M) and extracted twice with dichloromethane. The combined organic extracts are dried and the solvent is removed to give the title compound (8.5 mg). LC (method 20): t_{R} = 6.37 min; Mass spectrum (ESI⁻): m/z = 567 [M-H]⁻.

### Example 64

### 2-((S)-6-((R)-4-(2,6-Dimethyl-4-(3-(methylsulfonyl)propoxy)phenyl)-5,7-difluoro-2,3-dihydro-1H-inden-1-yloxy)-2,3-dihydrobenzofuran-3-yl)acetic acid

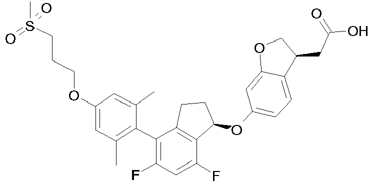

The title compound is prepared from methyl 2-((S)-6-((R)-4-(2,6-dimethyl-4-(3-(methylsulfonyl)propoxy)phenyl)-5,7-difluoro-2,3-dihydro-1H-inden-1-yloxy)-2,3-dihydrobenzofuran-3-yl)acetate (Intermediate 54; 29 mg) following a procedure analogous to that described in Example 63. Yield 25 mg.
LC (method 20): t_{R} = 6.20 min; Mass spectrum: m/z = 587 [M+H]⁺.

### Example 65

### 2-((3S)-6-((1R)-7-Fluoro-4-(2-methyl-3-(3-(methylsulfonyl)propoxy)phenyl)-2,3-dihydro-1H-inden-1-yloxy)-2,3-dihydrobenzofuran-3-yl)acetic acid

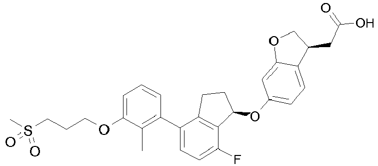

Methyl 2-((3S)-6-((1R)-7-fluoro-4-(2-methyl-3-(3-(methylsulfonyl)propoxy)phenyl)-2,3-dihydro-1H-inden-1-yloxy)-2,3-dihydrobenzofuran-3-yl)acetate (Intermediate 51 m; 64 mg) in methanol (3 mL) and aqueous NaOH (1 M; 0.5 mL) is stirred for 16 hours, concentrated under vacuum, acidified with aqueous HCl (1 M) and extracted with diethyl ether. A precipitate that forms is collected by filtration, washed with some n-hexane, dried and subsequently triturated with a mixture of n-hexane and diethyl ether to give the title compound (30 mg).
LC (method 20): t_{R} = 6.15 min; Mass spectrum: m/z = 555 [M+H]⁺.

### Example 66*

### 2-((3S)-6-((1R)-4-(2-(oxazol-4-yl)lphenyl)-2,3-dihydro-1H-inden-1-yloxy)-2,3-dihydrobenzofuran-3-yl)acetic acid

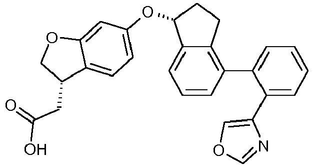

Intermediate 55 (15.7 mg) is dissolved in methanol (1 mL) and tetrahydrofuran (1 mL) and aqueous sodium hydroxide solution (0.084 mL, 1 Mol/L) is added. The reaction mixture is shaken over night at room temperature and half of the solvent is removed under reduced pressure. Aqueous hydrochloric acid (0.084 mL, 1 Mol/L) and water are added. The precipitated product is collected and washed with water. The organic phase is extracted with ethyl acetate, evaporated and the residue is chromatographed by HPLC on reversed phase to yield additional product. Yield 7 mg, LC (method 22): t_{R} = 1,6 min; Mass spectrum (ESI⁺): m/z = 454 [M+H]⁺.

### Example 71*

### 2-((S)-6-((R)-4-(2,6-dimethyl-4-((1-methyl-1H-imidazol-2-yl)methoxy)phenyl)-2,3-dihydro-1H-inden-1-yloxy)-2,3-dihydrobenzofuran-3-yl)acetic acid

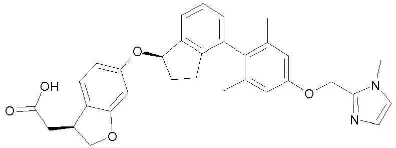

2-Chloromethyl-1-methyl-1H-imidazole (39 mg) is suspended with a solution of methyl 2-((S)-6-((R)-4-(4-hydroxy-2,6-dimethylphenyl)-2,3-dihydro-1H-inden-1-yloxy)-2,3-dihydrobenzofuran-3-yl)acetate (45 mg) in dimethylformamide (2 mL) and solid K₂CO₃ (63 mg) is added. The reaction mixture is heated at 60°C and shaken for 24 hours. The reaction mixture is acidified with aqueous trifluoroacetic acid, filtrated and chromatographed by HPLC on reversed phase. The product fractions are collected and lyophilized to give the methylester of title compound.

The ester is dissolved in methanol (1mL) and tetrahydrofuran (1 mL) and aqueous NaOH solution (0.2 mL, 1 Mol/L) was added. The reaction mixture is shaken for 72 hours at room temperature. Aqueous hydrochloric acid (0.2 mL, 1 Mol/L) and dimethylformamide (1 mL) are added and the product is purified by HPLC on reversed phase. The product fractions are collected and lyophilized to give the title compound. LC (method 24): t_{R} = 0.75 min; Mass spectrum (ESI⁺): m/z = 525 [M+H]⁺.

### Example 88*

### 2-((S)-6-((R)-4-(4-Sulfamoyliphenyl)-2,3-dihydro-1H-inden-1-yloxy)-2,3-dihydrobenzofuran-3-yl)acetic acid

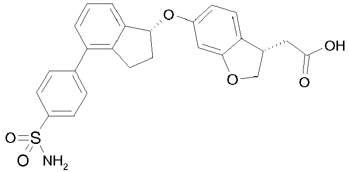

Methyl 2-((S)-6-((R)-4-(4-sulfamoylphenyl)-2,3-dihydro-1H-inden-1-yloxy)-2,3-dihydro benzofuran-3-yl)acetate (4.8 mg) is dissolved in methanol (1 mL) and tetrahydrofuran (1 mL) and aqueous sodium hydroxide solution (0.2 mL, 1 Mol/L) is added. The reaction mixture is shaken for 36 hours at room temperature and half of the solvent is removed under reduced pressure. Aqueous hydrochloric acid (0.2 mL, 1 Mol/L) and N,N-dimethylformamide are added and the product is purified by HPLC on reversed phase. The product fractions are collected and lyophilized to give the title compound. Yield 2.6 mg, LC (method 14): t_{R} = 0.54 min; Mass spectrum (ESI⁻): m/z = 464 [M-H]⁻.

### Example 89*

### 2-((3S)-6-((1R)-4-(2-Acetylphenyl)-2,3-dihydro-1H-inden-1-yloxy)-2,3-dihydrobenzofuran-3-yl)acetic acid

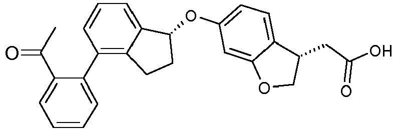

Methyl 2-((3S)-6-((1R)-4-(2-acetylphenyl)-2,3-dihydro-1H-inden-1-yloxy)-2,3-dihydro benzofuran-3-yl)acetate (5.9 mg) is dissolved in methanol (1 mL) and tetrahydrofuran (1 mL) and aqueous sodium hydroxide solution (0.2 mL, 1 Mol/L) is added. The reaction mixture is shaken for 36 hours at room temperature and half of the solvent is removed under reduced pressure. Aqueous hydrochloric acid (0.2 mL, 1 Mol/L) and N,N-dimethylformamide are added and the product is purified by HPLC on reversed phase. The product fractions are collected and lyophilized to give the title compound. Yield 5.4 mg, LC (method 14): t_{R} = 0.65 min; Mass spectrum (ESI⁻): m/z = 427 [M-H]⁻.

### Example 90

### 2-((3S)-6-((1R)-4-(3-Carbamoyl-2-methylphenyl)-7-fluoro-2,3-dihydro-1H-inden-1-yloxy)-2,3-dihydrobenzofuran-3-yl)acetic acid

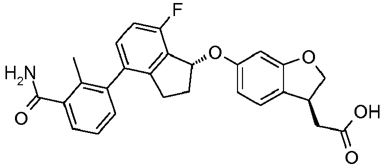

30% Aqueous NH₃ solution (4.2 mL) and 35% aqueous H₂O₂ solution (1.4 ml) are added at 0°C to a solution of 2-((3S)-6-((1R)-4-(3-cyano-2-methylphenyl)-7-fluoro-2,3-dihydro-1H-inden-1-yloxy)-2,3-dihydrobenzofuran-3-yl)acetic acid (30 mg) in ethanol (5 mL). The mixture is stirred at room temperature for 12 hours, while warming to room temperature. The mixture is concentrated, diluted with water and neutralized with 4 M aqueous HCl solution. The resulting mixture is extracted with dichloromethane. The organic phase is dried (MgSO₄). The solvent is evaporated to give the title compound. Yield: 29 mg; LC (method 20): t_{R} = 5.02 min; Mass spectrum (ESI⁺): m/z = 462 [M+H]⁺.

### Example 91

### 2-((S)-6-((R)-4-(4-Carbamoyl-2,6-dimethylphenyl)-7-fluoro-2,3-dihydro-H-inden-1-yloxy)-2,3-dihydrobenzofuran-3-yl)acetic acid

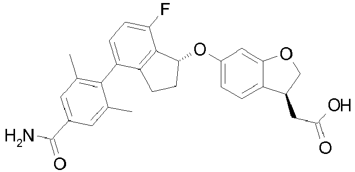

30% Aqueous NH₃ solution (250 µL) and 35% aqueous H₂O₂ solution (83 µl) are added at 0°C to a solution of 2-((S)-6-((R)-4-(4-cyano-2,6-dimethylphenyl)-7-fluoro-2,3-dihydro-1H-inden-1-yloxy)-2,3-dihydrobenzofuran-3-yl)acetic acid (19 mg) in ethanol (2 mL). The mixture is stirred at room temperature for 12 hours, while warming to room temperature. The mixture is concentrated, diluted with water and neutralized with 4 M aqueous HCl solution. The resulting mixture is extracted with dichloromethane. The organic phase is dried (MgSO₄). The solvent is evaporated to give the title compound. Yield: 19 mg; LC (method 20): t_{R} = 5.33 min; Mass spectrum (ESI⁺): m/z = 476 [M+H]⁺.

### Example 92

### 2-((S)-6-((R)-4-(4-(Dimethylcarbamoyl)-2,6-dimethyliphenyl)-7-fluoro-2,3-dihydro-1H-inden-1-yloxy)-2,3-dihydrobenzofuran-3-yl)acetic acid

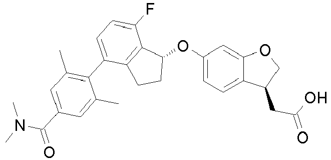

LiOHxH₂O (11 mg) is added to a solution of methyl 2-((S)-6-((R)-4-(4-(dimethylcarbamoyl)-2,6-dimethylphenyl)-7-fluoro-2,3-dihydro-1H-inden-1-yloxy)-2,3-dihydrobenzofuran-3-yl)acetate (70 mg) in ethanol (2.6 mL) and water (0.5 mL) and the mixture is stirred for 24 hours at room temperature. After concentration the mixture is diluted with water, acidified with aqueous citric acid solution and extracted with dichloromethane. After concentration the mixture is partitioned between dichloromethane and citric acid. The organic phase is dried (MgSO₄) and concentrated to give the title compound. Yield 21 mg, LC (method 20): t_{R} = 5.87 min; Mass spectrum (ESI⁺): m/z = 504 [M+H]⁺.

### Example 93

### 2-((S)-6-((R)-4-(2,6-Dimethyl-4-(methylcarbamoyl)phenyl)-7-fluoro-2,3-dihydro-1H-inden-1-yloxy)-2,3-dihydrobenzofuran-3-yl)acetic acid

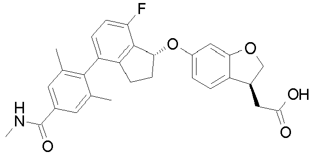

LiOHxH₂O (3 mg) is added to a solution of methyl 2-((S)-6-((R)-4-(2,6-dimethyl-4-(methylcarbamoyl)phenyl)-7-fluoro-2,3-dihydro-1H-inden-1-yloxy)-2,3-dihydrobenzofuran-3-yl)acetate (20 mg) in ethanol (0.77 mL) and water (0.5 mL) and the mixture is stirred for 24 hours at room temperature. After concentration the mixture is diluted with water, acidified with aqueous citric acid solution and extracted with dichloromethane. The organic phase is dried (MgSO₄) and the residue chromatographed on silica gel (cyclohexane/ethyl acetate 100:0→60:40) to give the title compound. Yield 11 mg, LC (method 20): t_{R} = 5.54 min; Mass spectrum (ESI⁺): m/z = 490 [M+H]⁺.

### Example 94

### 2-((S)-6-((R)-4-(2,6-Dimethyl-4-(morpholine-4-carbonyl)lphenyl)-7-fluoro-2,3-dihydro-1H-inden-1-yloxy)-2,3-dihydrobenzofuran-3-yl)acetic acid

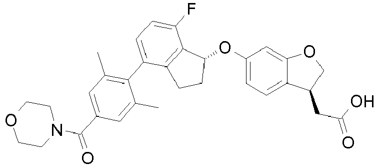

LiOHxH₂O (4.1 mg) is added to a solution of methyl 2-((S)-6-((R)-4-(2,6-dimethyl-4-(morpholine-4-carbonyl)phenyl)-7-fluoro-2,3-dihydro-1H-inden-1-yloxy)-2,3-dihydrobenzofuran-3-yl)acetate (55 mg) in ethanol (1.9 mL) and water (0.5 mL) and the mixture is stirred for 24 hours at room temperature. After concentration the mixture is diluted with water, acidified with aqueous citric acid solution and extracted with dichloromethane. The organic phase is dried (MgSO₄) and concentrated. The residue is chromatographed on silica gel (cyclohexane/ethyl acetate 50:50) to give the title compound. Yield 10 mg, LC (method 20): t_{R} = 5.73 min; Mass spectrum (ESI⁺): m/z = 546 [M+H]⁺.

### Example 95

### 2-((S)-6-((R)-4-(4-(2-Cyano-2-methylpropoxy)-2,6-dimethylphenyl)-7-fluoro-2.3-dihydro-1H-inden-1-yloxy)-2,3-dihydrobenzofuran-3-yl)acetic acid

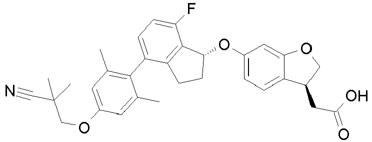

The title compound is prepared from methyl 2-((S)-6-((R)-4-(4-(2-cyano-2-methylpropoxy)-2,6-dimethylphenyl)-7-fluoro-2,3-dihydro-1H-inden-1-yloxy)-2,3-dihydrobenzofuran-3-yl)acetate following a procedure analogous to that described in example 10. LC (method 8): t_{R} = 0.70 min; Mass spectrum (ESI⁺): m/z = 530 [M+H]⁺.

### Example 96

### 2-((S)-6-((R)-5-Cyano-4-(4-(3-hydroxy-3-methylbutoxy)-2,6-dimethylphenyl)-2,3-dihydro-1H-inden-1-yloxy)-2,3-dihydrobenzofuran-3-yl)acetic acid

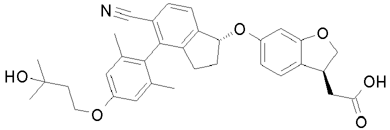

The title compound is prepared from methyl 2-((S)-6-((R)-5-cyano-4-(4-(3-hydroxy-3-methylbutoxy)-2,6-dimethylphenyl)-2,3-dihydro-1H-inden-1-yloxy)-2,3-dihydrobenzofuran-3-yl)acetate following a procedure analogous to that described in example 1. The mixture is stirred for 2 days. The product is purified by HPLC on reversed phase. LC (method 7): t_{R} = 1.08 min; Mass spectrum (ESI⁺): m/z = 559 [M+NH₄]⁺.

### Example 97

### 2-((S)-6-((R)-5-Cyano-4-(2,6-dimethyl-4-(tetrahydro-2H-pyran-4-yloxy)phenyl)-2,3-dihydro-1H-inden-1-yloxy)-2,3-dihydrobenzofuran-3-yl)acetic acid

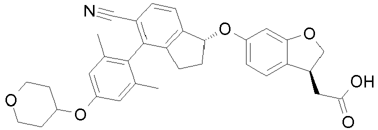

The title compound is prepared from methyl 2-((S)-6-((R)-5-cyano-4-(2,6-dimethyl-4-(tetrahydro-2H-pyran-4-yloxy)phenyl)-2,3-dihydro-1H-inden-1-yloxy)-2,3-dihydrobenzofuran-3-yl)acetate following a procedure analogous to that described in example 1. The mixture is stirred for 2 days at room temperature and for 2 hours at 50°C. The product is purified by HPLC on reversed phase. LC (method 7): t_{R} = 1.10 min; Mass spectrum (ESI⁺): m/z = 540 [M+H]⁺.

### Example 98

### 2-((S)-6-((R)-4-(4-(2-(tert-Butoxycarbonylamino)ethoxy)-2,6-dimethylphenyl)-7-fluoro-2,3-dihydro-1H-inden-1-yloxy)-2,3-dihydrobenzofuran-3-yl)acetic acid

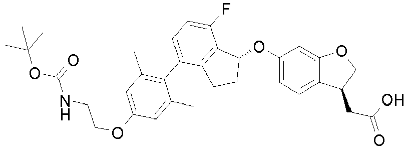

The title compound is prepared from methyl 2-((S)-6-((R)-4-(4-(2-(tert-butoxycarbonylamino)ethoxy)-2,6-dimethylphenyl)-7-fluoro-2,3-dihydro-1H-inden-1-yloxy)-2,3-dihydrobenzofuran-3-yl)acetate following a procedure analogous to that described in example 10. The mixture is stirred for 2 days at 50°C. The product is purified by HPLC on reversed phase. LC (method 8): t_{R} = 0.73 min; Mass spectrum (ESI⁺): m/z = 592 [M+H]⁺.

### Example 99

### 2-((S)-6-((R)-5-Cyano-4-(4-(2-hydroxy-2-methylpropoxy)-2,6-dimethylphenyl)-2,3-dihydro-1H-inden-1-yloxy)-2,3-dihydrobenzofuran-3-yl)acetic acid

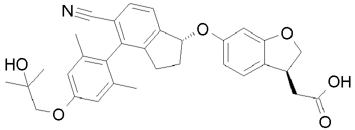

The title compound is prepared from methyl 2-((S)-6-((R)-5-cyano-4-(4-(3-hydroxy-3-methylbutoxy)-2,6-dimethylphenyl)-2,3-dihydro-1H-inden-1-yloxy)-2,3-dihydrobenzo furan-3-yl)acetate following a procedure analogous to that described in example 1. The mixture is stirred for 2 days. The product is purified by HPLC on reversed phase. LC (method 7): t_{R} = 1.07 min; Mass spectrum (ESI⁺): m/z = 545 [M+NH₄]⁺.

### Example 100

### 2-((S)-6-((R)-7-Fluoro-4-(4-((4-hydroxytetrahydro-2H-pyran-4-yl)methoxy)-2,6-dimethyliphenyl)-2,3-dihydro-1H-inden-1-yloxy)-2,3-dihydrobenzofuran-3-yl)acetic acid

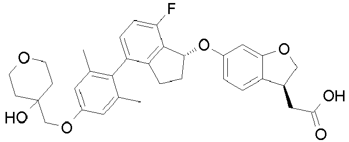

The title compound is prepared from methyl 2-((S)-6-((R)-7-fluoro-4-(4-((4-hydroxytetrahydro-2H-pyran-4-yl)methoxy)-2,6-dimethylphenyl)-2,3-dihydro-1H-inden-1-yloxy)-2,3-dihydrobenzofuran-3-yl)acetate following a procedure analogous to that described in example 10. TLC: r_{f} = 0.2 (silicagel, ethyl acetate); Mass spectrum (ESI⁺): m/z = 563 [M+H]⁺.

### Example 101

### 2-((S)-6-((R)-7-Fluoro-4-(4-(3-hydroxy-3-methylbutyl)-2,6-dimethylphenyl)-2,3-dihydro-1H-inden-1-yloxy)-2,3-dihydrobenzofuran-3-yl)acetic acid

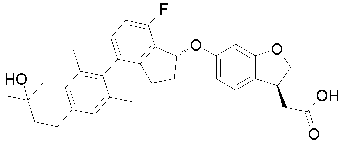

1 M aqueous NaOH solution (310 µL) is added to a solution of methyl 2-((S)-6-((R)-7-fluoro-4-(4-(3-hydroxy-3-methylbutyl)-2,6-dimethylphenyl)-2,3-dihydro-1H-inden-1-yloxy)-2,3-dihydrobenzofuran-3-yl)acetate (110 mg) in methanol (4 mL). The mixture is stirred at room temperature for 12 hours and at 50°C for 2 days. 1 M aqueous NaOH solution (100 µL) is added and the mixture is stirred at 50°C for 1 hour. Methanol is evaporated in vacuo, the residue is diluted with water and neutralized with 1 M aqueous HCl. The resulting mixture is extracted with ethyl acatete, and the combined organic phases are washed with brine and dried (MgSO₄). The solvent is evaporated to give the title compound. Yield: 80 mg; LC (method 8): t_{R} = 0.59 min; Mass spectrum (ESI⁺): m/z = 519 [M+H]⁺.

### Example 102

### 2-((S)-6-((R)-7-Fluoro-4-(4-((4-hydroxytetrahydro-2H-thiolpyran-4-yl)methoxy)-2,6-dimethyliphenyl)-2,3-dihydro-1H-inden-1-yloxy)-2,3-dihydrobenzofuran-3-yl)acetic acid

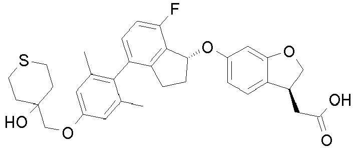

To a solution of methyl 2-((S)-6-((R)-7-fluoro-4-(4-hydroxy-2,6-dimethylphenyl)-2,3-dihydro-1H-inden-1-yloxy)-2,3-dihydrobenzofuran-3-yl)acetate (100 mg) in N,N-dimethylformamide (2 mL) is added Cs₂CO₃ (106 mg) and 1-oxa-6-thiaspiro[2.5]octane (37 mg). The mixture is stirred for 12 hour at 100°C. The mixture is diluted with water, acidified with 1 M aqueous HCl solution and extracted twice with ethyl acetate. The combined organic phases are washed with water and brine, dried (MgSO₄) and concentrated. The residue is chromatographed on silica gel (cyclohexane/ethyl acetate/methanol 80:20:0→0:80:20) to give the title compound. Yield: 31 mg; LC (method 7): t_{R} = 1.16 min; Mass spectrum (ESI⁺): m/z = 579 [M+H]⁺.

### Example 103

### 2-((S)-6-((R)-7-Fluoro-4-(4-((1,1-dioxo-4-hydroxytetrahydro-2H-thiolpyran-4-yl)metho xy)-2,6-dimethylphenyl-2,3-dihydro-11H-inden-1-yloxy)-2,3-dihydrobenzofuran-3-yl)acetic acid

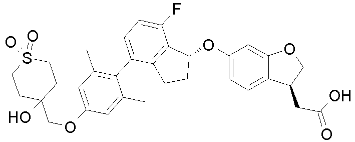

1 M aqueous NaOH solution (250 µL) is added to a solution of methyl 2-((S)-6-((R)-7-fluoro-4-(4-((1,1-dioxo-4-hydroxytetrahydro-2H-thiopyran-4-yl)methoxy)-2,6-dimethylphenyl)-2,3-dihydro-1H-inden-1-yloxy)-2,3-dihydrobenzofuran-3-yl)acetate (85 mg) in tetrahydrofurane (4 mL). The mixture is stirred at room temperature for 12 hours. Then the mixture is diluted with ethyl acetate and neutralized with 1 M aqueous HCl solution. The resulting mixture is diluted with saturated aqueous NaCl solution and the phases are separated. The organic phase is dried (MgSO₄) and concentrated to give the title compound. Yield: 80 mg; LC (method 7): t_{R} = 1.04 min; Mass spectrum (ESI⁺): m/z = 611 [M+H]⁺.

### Example 104

### 2-((S)-6-((R)-4-(2,6-Dimethyl-4-(3-(methylsulfonyl)propyl)phenyl)-7-fluoro-2,3-dihydro-1H-inden-1-yloxy)-2,3-dihydrobenzofuran-3-yl)acetic acid

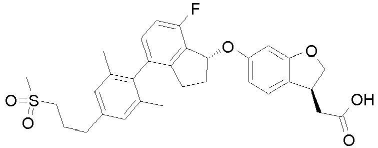

1 M aqueous NaOH solution (50 µL) is added to a solution of methyl 2-((S)-6-((R)-4-(2,6-dimethyl-4-(3-(methylsulfonyl)propyl)phenyl)-7-fluoro-2,3-dihydro-1H-inden-1-yloxy)-2,3-dihydrobenzofuran-3-yl)acetate (20 mg) in methanol (2 mL). The mixture is stirred at room temperature for 12 hours and at 50°C for 2 days. 1 M aqueous NaOH solution (18 µL) is added and the mixture is stirred at 50°C for 2 hours. Methanol is evaporated in vacuo, the residue is diluted with water and neutralized with 1 M aqueous HCl. The resulting mixture is extracted with ethyl acatete, and the organic phase is dried (MgSO₄). The solvent is evaporated and the residue is purified by HPLC on reversed phase to give the title compound. Yield: 7 mg; LC (method 8): t_{R} = 0.34 min; Mass spectrum (ESI⁺): m/z = 553 [M+H]⁺.

### Example 105

### 2-((S)-6-((R)-4-(2,6-Dimethyl-4-(2-(methylsulfonamido)ethoxy)phenyl)-7-fluoro-2,3-dihydro-1H-inden-1-yloxy)-2,3-dihydrobenzofuran-3-yl)acetic acid

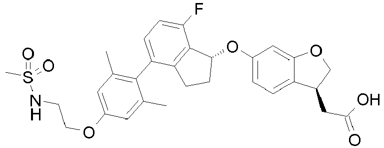

The title compound is prepared from methyl 2-((S)-6-((R)-4-(2,6-dimethyl-4-(2-(methylsulfonamido)ethoxy)phenyl)-7-fluoro-2,3-dihydro-1H-inden-1-yloxy)-2,3-dihydrobenzofuran-3-yl)acetate following a procedure analogous to that described in example 10. The mixture is stirred for 2 days at 50°C. The product is purified by HPLC on reversed phase. LC (method 7): t_{R} = 1.05 min; Mass spectrum (ESI⁺): m/z = 570 [M+H]⁺.

### Example 106

### 2-((S)-6-((R)-4-(4-(2-Acetamidoethyl)-2,6-dimethylphenyl)-7-fluoro-2,3-dihydro-1H-inden-1-yloxy)-2,3-dihydrobenzofuran-3-yl)acetic acid

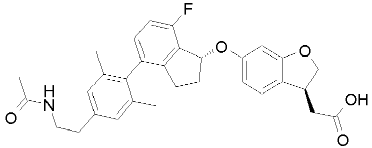

The title compound is prepared from methyl 2-((S)-6-((R)-4-(4-(2-acetamidoethyl)-2,6-dimethylphenyl)-7-fluoro-2,3-dihydro-1H-inden-1-yloxy)-2,3-dihydrobenzofuran-3-yl)acetate following a procedure analogous to that described in example 10. The mixture is stirred for 12 hours at 50°C. The product is purified by HPLC on reversed phase. LC (method 7): t_{R} = 1.04 min; Mass spectrum (ESI⁺): m/z = 518 [M+H]⁺.

### Example 107

### 2-((S)-6-((R)-4-(4-(2-Acetamidoethoxy)-2,6-dimethyliphenyl)-7-fluoro-2,3-dihydro-1H-inden-1-yloxy)-2,3-dihydrobenzofuran-3-yl)acetic acid

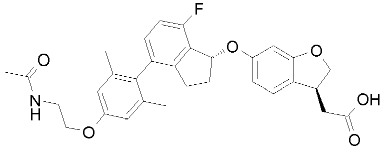

The title compound is prepared from methyl 2-((S)-6-((R)-4-(4-(2-acetamidoethoxy)-2,6-dimethylphenyl)-7-fluoro-2,3-dihydro-1H-inden-1-yloxy)-2,3-dihydrobenzofuran-3-yl)acetate following a procedure analogous to that described in example 10. The mixture is stirred for 5 hours at 50°C. The product is purified by HPLC on reversed phase. LC (method 7): t_{R} = 1.03 min; Mass spectrum (ESI⁺): m/z = 534 [M+H]⁺.

### Example 108

### 2-((S)-6-((R)-4-(2,6-dimethyl-4-(2-(methylsulfonamido)ethyl)phenyl)-7-fluoro-2,3-dihydro-1H-inden-1-yloxy)-2,3-dihydrobenzofuran-3-yl)acetic acid

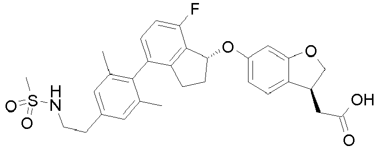

The title compound is prepared from methyl 2-((S)-6-((R)-4-(2,6-dimethyl-4-(2-(methylsulfonamido)ethyl)phenyl)-7-fluoro-2,3-dihydro-1H-inden-1-yloxy)-2,3-dihydrobenzofuran-3-yl)acetate following a procedure analogous to that described in example 10. The mixture is stirred for 20 hours at 50°C. The product is purified by HPLC on reversed phase. LC (method 7): t_{R} = 1.06 min; Mass spectrum (ESI⁺): m/z = 554 [M+H]⁺.

### Example 109

### 2-((S)-6-((R)-7-Fluoro-4-(4-((1-hydroxycyclopropyl)methoxy)-2,6-dimethylphenyl)-2,3-dihydro-1H-inden-1-yloxy)-2,3-dihydrobenzofuran-3-yl)acetic acid

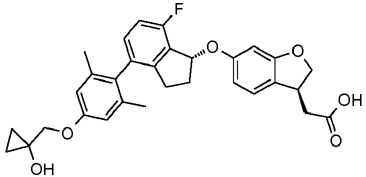

Ethylmagnesium chloride (380 µL of a 1 M solution in tetrahydrofurane) is added dropwise under argon to a solution of 2-((S)-6-((R)-7-fluoro-4-(4-(2-methoxy-2-oxoethoxy)-2,6-dimethylphenyl)-2,3-dihydro-1H-inden-1-yloxy)-2,3-dihydrobenzofuran-3-yl)acetic acid in tetrahydrofurane (4 mL). The mixture is stirred at room temperature for 30 minutes. Ti(OiPr)₄ (120 µL) is added dropwise and after stirring for 30 minutes ethylmagnesium chloride (1.15 mL of a 1 M solution in tetrahydrofurane) is added dropwise. The mixture is stirred for 2 hours, partitioned between ethylacetate and saturated aqueous NH₄Cl solution and stirred vigorously for 30 minutes. After filtering over celite the phases are separated, the aqueous phase is extracted with diethylether and the combined organic phases are washed with brine and dried (MgSO₄). The solvents are evaporated and the residue is purified by HPLC on reversed phase. Yield: 38 mg; LC (method 8): t_{R} = 0.38 min; Mass spectrum (ESI⁺): m/z = 519 [M+H]⁺.

### Example 110

### 2-((3S)-6-((1R)-4-(2,6-Dimethyl-4-(1-methyl-2-oxopyrrolidin-3-yloxy)phenyl)-7-fluoro-2,3-dihydro-1H-inden-1-yloxy)-2,3-dihydrobenzofuran-3-yl)acetic acid

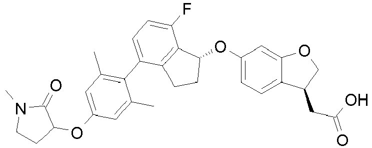

The title compound is prepared from methyl 2-((3S)-6-((1R)-4-(2,6-dimethyl-4-(1-methyl-2-oxopyrrolidin-3-yloxy)phenyl)-7-fluoro-2,3-dihydro-1H-inden-1-yloxy)-2,3-dihydrobenzofuran-3-yl)acetate following a procedure analogous to that described in example 10. The mixture is stirred for 12 hours at 50°C. The product is purified by HPLC on reversed phase. LC (method 7): t_{R} = 1.06 min; Mass spectrum (ESI⁺): m/z = 546 [M+H]⁺.

### Example 111

### 2-((S)-6-((R)-4-(4-(2,2-Dimethyl-3-(methylsulfonyl)propoxy)-2,6-dimethylphenyl)-7-fluoro-2,3-dihydro-1H-inden-1-yloxy)-2,3-dihydrobenzofuran-3-yl)acetic acid

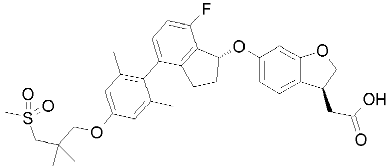

The title compound is prepared from methyl 2-((S)-6-((R)-4-(4-(2,2-dimethyl-3-(methylsulfonyl)propoxy)-2,6-dimethylphenyl)-7-fluoro-2,3-dihydro-1H-inden-1-yloxy)-2,3-dihydrobenzofuran-3-yl)acetate following a procedure analogous to that described in example 10. The mixture is stirred for 3 hours at 50°C. The product is purified by HPLC on reversed phase. LC (method 7): t_{R} = 1.14 min; Mass spectrum (ESI⁺): m/z = 597 [M+H]⁺.

### Example 112

### 2-((3S)-6-((1R)-7-Fluoro-4-(4-(3-hydroxy-3-methylcyclopentyloxy)-2,6-dimethylphenyl)-2,3-dihydro-1H-inden-1-yloxy)-2,3-dihydrobenzofuran-3-yl)acetic acid (diastereomer 1)

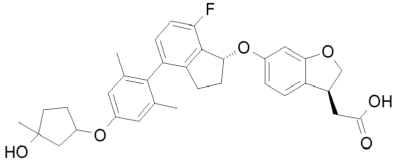

The title compound is prepared from methyl 2-((3S)-6-((1 R)-7-fluoro-4-(4-(3-hydroxy-3-methylcyclopentyloxy)-2,6-dimethylphenyl)-2,3-dihydro-1H-inden-1-yloxy)-2,3-dihydrobenzofuran-3-yl)acetate (diastereomer 1) following a procedure analogous to that described in example 1. LC (method 8): t_{R} = 0.54 min; Mass spectrum (ESI⁺): m/z = 547 [M+H]⁺.

### Example 113

### 2-((3S)-6-((1R)-7-Fluoro-4-(4-(3-hydroxy-3-methylcyclopentyloxy)-2,6-dimethylphenyl)-2,3-dihydro-1H-inden-1-yloxy)-2,3-dihydrobenzofuran-3-yl)acetic acid (diastereomer 2)

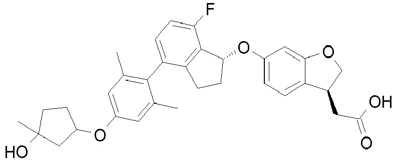

The title compound is prepared from methyl 2-((3S)-6-((1 R)-7-fluoro-4-(4-(3-hydroxy-3-methylcyclopentyloxy)-2,6-dimethylphenyl)-2,3-dihydro-1H-inden-1-yloxy)-2,3-dihydrobenzofuran-3-yl)acetate (diastereomer 2) following a procedure analogous to that described in example 1. LC (method 8): t_{R} = 0.56 min; Mass spectrum (ESI⁺): m/z = 547 [M+H]⁺.

### Example 114

### 2-((S)-6-((S)-7-Fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid

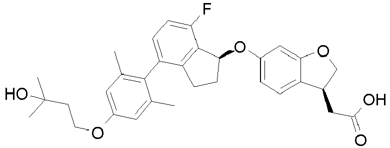

1 M aqueous NaOH solution (39 µL) is added to a solution of methyl 2-((3S)-6-(7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate (48 mg) in tetrahydrofurane (2 mL). The mixture is stirred at room temperature for 12 hours. The mixture is diluted with water and neutralized with 4 M aqueous HCl solution (41 µL). The resulting mixture is extracted with ethyl acatete, and the combined extracts are washed with brine and dried (MgSO₄). After evaporation of the solvents the diastereomers are separated by SFC on chiral phase (column: Daicel ADH, 5 µm, 250 mm x 10 mm; eluent: scCO₂/(methanol+0.2% diethylamine) 80:20, 10 mL/min):
2-((S)-6-((R)-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid (diastereomer 1): t_{R} = 22.1 min; Yield: 15 mg; Mass spectrum (ESI⁺): m/z = 535 [M+H]⁺.
2-((S)-6-((S)-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid (diastereomer 2): t_{R} = 31.1 min; Yield: 10 mg; Mass spectrum (ESI⁺): m/z = 535 [M+H]⁺.

### Example 115

### 2-((S)-6-((R)-5-Cyano-4-(2,6-dimethyl-4-((1-methyl-2-oxo-1,2-dihydropyridin-4-yl)methoxy)phenyl)-2,3-dihydro-1H-inden-1-yloxy)-2,3-dihydrobenzofuran-3-yl)acetic acid

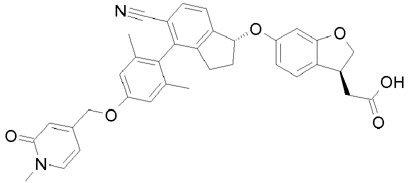

The title compound is prepared from methyl 2-((S)-6-((R)-5-cyano-4-(2,6-dimethyl-4-((1-methyl-2-oxo-1,2-dihydropyridin-4-yl)methoxy)phenyl)-2,3-dihydro-1H-inden-1-yloxy)-2,3-dihydrobenzofuran-3-yl)acetate following a procedure analogous to that described in example 10. The mixture is stirred for 2 days at room temperature and for 2 hours at 50°C. LC (method 9): t_{R} = 1.01 min; Mass spectrum (ESI⁺): m/z = 577 [M+H]⁺.

### Example 116

### 2-((S)-6-((R)-5-Cyano-4-(2,6-dimethyl-4-((tetrahydro-2H-pyran-4-yl)methoxy)phenyl)-2,3-dihydro-1H-inden-1-yloxy)-2,3-dihydrobenzofuran-3-yl)acetic acid

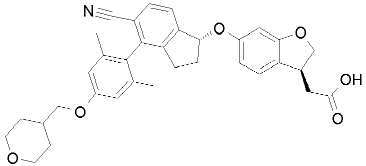

The title compound is prepared from methyl -((S)-6-((R)-5-cyano-4-(2,6-dimethyl-4-((tetrahydro-2H-pyran-4-yl)methoxy)phenyl)-2,3-dihydro-1H-inden-1-yloxy)-2,3-dihydrobenzofuran-3-yl)acetate following a procedure analogous to that described in example 10. The mixture is stirred for 2 days at room temperature. LC (method 9): t_{R} = 1.14 min; Mass spectrum (ESI⁺): m/z = 554 [M+H]⁺.

### Example 117

### 2-((S)-6-((R)-4-(2,6-Dimethyl-4-(tetrahydro-2H-pyran-4-yl)phenyl)-7-fluoro-2,3 dihydro-1H-inden-1-yloxy)-2,3-dihydrobenzofuran-3-yl)acetic acid

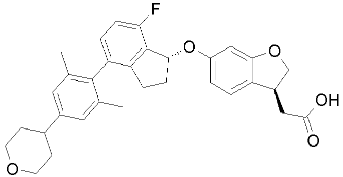

The title compound is prepared from methyl 2-((S)-6-((R)-4-(2,6-dimethyl-4-(tetrahydro-2H-pyran-4-yl)phenyl)-7-fluoro-2,3-dihydro-1H-inden-1-yloxy)-2,3-dihydrobenzofuran-3-yl)acetate following a procedure analogous to that described in example 1. LC (method 26): t_{R} = 0.79 min; Mass spectrum (ESI⁺): m/z = 517 [M+H]⁺.

### Example 118

### 2-((S)-6-((R)-4-(2,6-Dimethyl-4-(6-oxo-3,6-dihydro-2H-pyran-4-yl)phenyl)-7-fluoro-2,3-dihydro-1H-inden-1-yloxy)-2,3-dihydrobenzofuran-3-yl)acetic acid

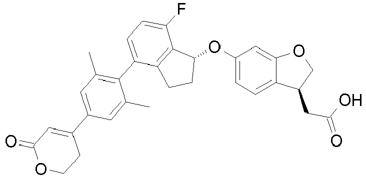

The title compound is prepared from methyl 2-((S)-6-((R)-4-(2,6-dimethyl-4-(6-oxo-3,6-dihydro-2H-pyran-4-yl)phenyl)-7-fluoro-2,3-dihydro-1H-inden-1-yloxy)-2,3-dihydro benzofuran-3-yl)acetate following a procedure analogous to that described in example 1. The product is purified by HPLC on reversed phase. LC (method 9): t_{R} = 0.96 min; Mass spectrum (ESI⁺): m/z = 529 [M+H]⁺.

### Example 119

### 2-((S)-6-((R)-5-Cyano-4-(2,6-dimethyl-4-(pyrimidin-5-yl)phenyl)-2,3-dihydro-1H-inden-1-yloxy)-2,3-dihydrobenzofuran-3-yl)acetic acid

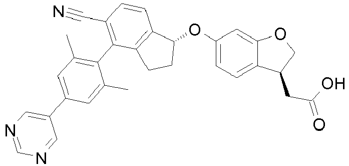

The title compound is prepared from methyl 2-((S)-6-((R)-5-cyano-4-(2,6-dimethyl-4-(pyrimidin-5-yl)phenyl)-2,3-dihydro-1H-inden-1-yloxy)-2,3-dihydrobenzofuran-3-yl)acetate following a procedure analogous to that described in example 10. The mixture is stirred for 12 hours at 50°C. LC (method 9): t_{R} = 1.04 min; Mass spectrum (ESI⁺): m/z = 518 [M+H]⁺.

### Example 120

### 2-((S)-6-((R)-5-Cyano-4-(2,6-dimethyl-4-(1-methyl-2-oxo-1,2-dihydropyridin-4-yl)lphenyl)-2,3-dihydro-1H-inden-1-yloxy)-2,3-dihydrobenzofuran-3-yl)acetic acid

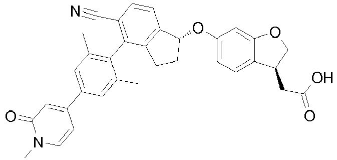

The title compound is prepared from methyl 2-((S)-6-((R)-5-cyano-4-(2,6-dimethyl-4-(1-methyl-2-oxo-1,2-dihydropyridin-4-yl)phenyl)-2,3-dihydro-1H-inden-1-yloxy)-2,3-dihydrobenzofuran-3-yl)acetate following a procedure analogous to that described in example 1. The mixture is stirred for 4 hours at 50°C. The product is purified by HPLC on reversed phase. LC (method 9): t_{R} = 1.00 min; Mass spectrum (ESI⁺): m/z = 547 [M+H]⁺.

### Example 121

### 2-((S)-6-((R)-5-Chloro-4-(2,6-dimethyl-4-((3-methyloxetan-3-yl)methoxy)phenyl)-2,3-dihydro-1H-inden-1-yloxy)-2,3-dihydrobenzofuran-3-yl)acetic acid

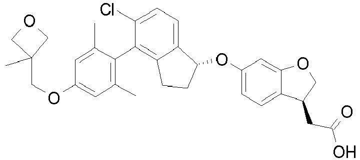

The title compound is prepared from methyl 2-((S)-6-((R)-5-chloro-4-(2,6-dimethyl-4-((3-methyloxetan-3-yl)methoxy)phenyl)-2,3-dihydro-1H-inden-1-yloxy)-2,3-dihydrobenzofuran-3-yl)acetate following a procedure analogous to that described in example 10. LC (method 8): t_{R} = 0.71 min; Mass spectrum (ESI⁺): m/z = 549 [M+H]⁺.

### Example 122

### 2-((S)-6-((R)-4-(2,6-Bis(methoxymethyl)-4-((3-methyloxetan-3-yl)methoxy)phenyl)-7-fluoro-2,3-dihydro-1H-inden-1-yloxy)-2,3-dihydrobenzofuran-3-yl)acetic acid

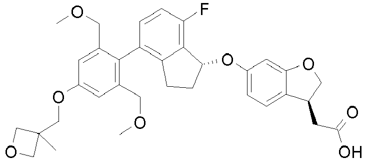

The title compound is prepared from methyl 2-((S)-6-((R)-4-(2,6-bis(methoxymethyl)-4-((3-methyloxetan-3-yl)methoxy)phenyl)-7-fluoro-2,3-dihydro-1H-inden-1-yloxy)-2,3-dihydrobenzofuran-3-yl)acetate following a procedure analogous to that described in example 10. LC (method 8): t_{R} = 0.43 min; Mass spectrum (ESI⁺): m/z = 593 [M+H]⁺.

### Example 123

### 2-((S)-6-((R)-7-Fluoro-4-(4-(1-(2-hydroxy-2-methylpropyl)-1H-pyrazol-4-yl)-2,6-dimethylphenyl)-2,3-dihydro-1H-inden-1-yloxy)-2,3-dihydrobenzofuran-3-yl)acetic acid

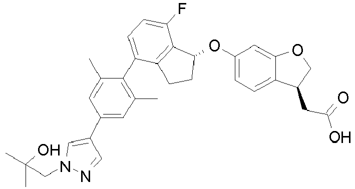

The title compound is prepared from methyl 2-((S)-6-((R)-7-fluoro-4-(4-(1-(2-hydroxy-2-methylpropyl)-1H-pyrazol-4-yl)-2,6-dimethylphenyl)-2,3-dihydro-1H-inden-1-yloxy)-2,3-dihydrobenzofuran-3-yl)acetate following a procedure analogous to that described in example 10. LC (method 11): t_{R} = 1.13 min; Mass spectrum (ESI⁺): m/z = 571 [M+H]⁺.

### Example 124

### 2-((S)-6-((R)-4-(2,6-Dimethyl-4-(1-methyl-2-oxo-1,2-dihydropyridin-4-yl)phenyl)-7-fluoro-2,3-dihydro-1H-inden-1-yloxy)-2,3-dihydrobenzofuran-3-yl)acetic acid

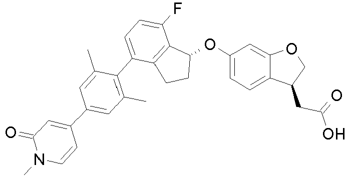

The title compound is prepared from methyl 2-((S)-6-((R)-4-(2,6-dimethyl-4-(1-methyl-2-oxo-1,2-dihydropyridin-4-yl)phenyl)-7-fluoro-2,3-dihydro-1H-inden-1-yloxy)-2,3-dihydrobenzofuran-3-yl)acetate following a procedure analogous to that described in example 10. LC (method 11): t_{R} = 1.09 min; Mass spectrum (ESI⁺): m/z = 540 [M+H]⁺.

### Example 125

### 2-((S)-6-((R)-4-(2,6-Dimethyl-4-(1-methyl-6-oxo-1,6-dihydropyridin-3-yl)phenyl)-7-fluoro-2,3-dihydro-1H-inden-1-yloxy-2,3-dihydrobenzofuran-3-yl)acetic acid

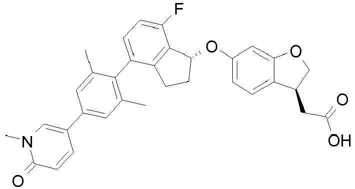

The title compound is prepared from methyl 2-((S)-6-((R)-4-(2,6-dimethyl-4-(1-methyl-6-oxo-1,6-dihydropyridin-3-yl)phenyl)-7-fluoro-2,3-dihydro-1H-inden-1-yloxy)-2,3-dihydrobenzofuran-3-yl)acetate following a procedure analogous to that described in example 10. LC (method 11): t_{R} = 1.09 min; Mass spectrum (ESI⁺): m/z = 540 [M+H]⁺.

### Example 126

### 2-((S)-6-((R)-4-(2,6-Dimethyl-4-(1H-1,2,4-triazol-1-yl)phenyl)-7-fluoro-2,3-dihydro-1H-inden-1-yloxy)-2,3-dihydrobenzofuran-3-yl)acetic acid

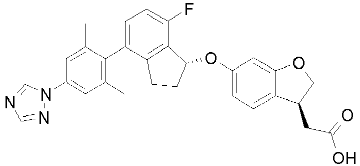

The title compound is prepared from methyl 2-((S)-6-((R)-4-(2,6-dimethyl-4-(1 H-1,2,4-triazol-1-yl)phenyl)-7-fluoro-2,3-dihydro-1H-inden-1-yloxy)-2,3-dihydrobenzofuran-3-yl)acetate following a procedure analogous to that described in example 10. LC (method 7): t_{R} = 1.06 min; Mass spectrum (ESI⁺): m/z = 500 [M+H]⁺.

### Example 127

### 2-((3S)-6-((1R)-4-(2,6-Dimethyl-4-(tetrahydrofuran-3-yl)lphenyl)-7-fluoro-2,3-dihydro-1H-inden-1-yloxy-2,3-dihydrobenzofuran-3-yl)acetic acid

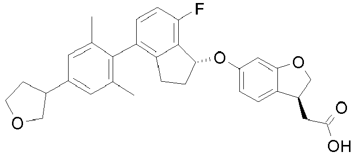

The title compound is prepared from 2-((S)-6-((R)-4-(4-(2,5-dihydrofuran-3-yl)-2,6-dimethylphenyl)-7-fluoro-2,3-dihydro-1H-inden-1-yloxy)-2,3-dihydrobenzofuran-3-yl)acetic acid by hydrogenation in the presence of palladium (10 % on carbon) in ethyl acetate at room temperature. LC (method 9): t_{R} = 1.17 min; Mass spectrum (ESI⁺): m/z = 503 [M+H]⁺.

### Example 128

### 2-((S)-6-((R)-5-Chloro-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetic acid

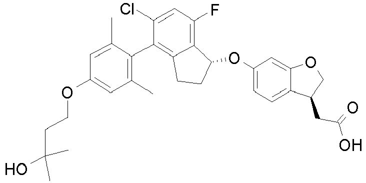

The title compound is prepared from methyl 2-((S)-6-((R)-5-chloro-7-fluoro-4-(4-(3-hydroxy-3-methylbutoxy)-2,6-dimethylphenyl)-2,3-dihydro-1H-inden-1-yloxy)-2,3-dihydrobenzofuran-3-yl)acetate following a procedure analogous to that described in example 10. LC (method 9): t_{R} = 1.17 min; Mass spectrum (ESI⁻): m/z = 567 [M-H]⁻.

### Example 129

### 2-((S)-6-((R)-5-Chloro-4-(2,6-dimethyl-4-((R)-tetrahydrofuran-3-yloxy)phenyl)-7-fluoro-2,3-dihydro-1H-inden-1-yloxy)-2,3-dihydrobenzofuran-3-yl)acetic acid

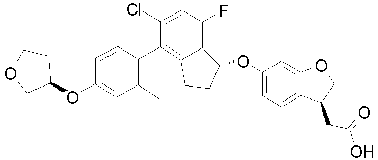

The title compound is prepared from methyl 2-((S)-6-((R)-5-chloro-4-(2,6-dimethyl-4-((R)-tetrahydrofuran-3-yloxy)phenyl)-7-fluoro-2,3-dihydro-1H-inden-1-yloxy)-2,3-dihydrobenzofuran-3-yl)acetate following a procedure analogous to that described in example 10. LC (method 9): t_{R} = 1.18 min; Mass spectrum (ESI⁻): m/z = 551 [M-H]⁻.

### Example 130*

### 2-((3S)-6-((1R)-4-(2-(Pyrrolidin-1-yl)pyridin-3-yl)-2,3-dihydro-1H-inden-1-yloxy)-2,3-dihydrobenzofuran-3-yl)acetic acid

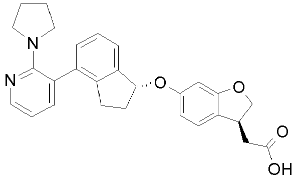

The title compound is prepared from methyl 2-((3S)-6-((1R)-4-(2-(pyrrolidin-1-yl)pyridin-3-yl)-2,3-dihydro-1H-inden-1-yloxy)-2,3-dihydrobenzofuran-3-yl)acetate following a procedure analogous to that described in example 10. LC (method 7): t_{R} = 0.87 min; Mass spectrum (ESI⁺): m/z = 457 [M+H]⁺.

### Example 131

### 2-((S)-6-((R)-4-(2,6-Dimethyl-4-(1-methyl-6-oxo-1,6-dihydropyrimidin-4-yl)phenyl)-7-fluoro-2,3-dihydro-1H-inden-1-yloxy)-2,3-dihydrobenzofuran-3-yl)acetic acid

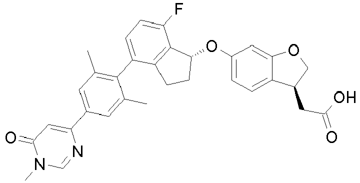

The title compound is prepared from methyl 2-((S)-6-((R)-5-cyano-4-(2,6-dimethyl-4-(1-methyl-2-oxo-1,2-dihydropyridin-4-yl)phenyl)-2,3-dihydro-1H-inden-1-yloxy)-2,3-dihydrobenzofuran-3-yl)acetate following a procedure analogous to that described in example 1. The mixture is stirred for 1 hour at 40°C. The product is purified by HPLC on reversed phase. LC (method 11): t_{R} = 1.09 min; Mass spectrum (ESI⁺): m/z = 541 [M+H]⁺.

### Example 132

### 2-((S)-6-((R)-4-(2,6-Dimethyl-4-(1-methyl-2-oxo-1,2-dihydropyrimidin-5-yl)phenyl)-7-fluoro-2,3-dihydro-1H-inden-1-yloxy)-2,3-dihydrobenzofuran-3-yl)acetic acid

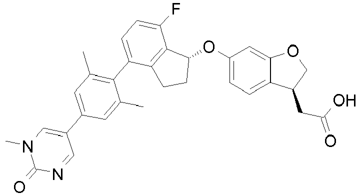

The title compound is prepared from methyl 2-((S)-6-((R)-4-(2,6-dimethyl-4-(1-methyl-2-oxo-1,2-dihydropyrimidin-5-yl)phenyl)-7-fluoro-2,3-dihydro-1H-inden-1-yloxy)-2,3-dihydrobenzofuran-3-yl)acetate following a procedure analogous to that described in example 1. The mixture is stirred for 2 hours at 40°C. LC (method 15): t_{R} = 1.05 min; Mass spectrum (ESI⁺): m/z = 541 [M+H]⁺.

### Example 133

### 2-((S)-6-((R)-4-(2,6-Dimethyl-4-(pyridazin-4-yl)phenyl)-7-fluoro-2,3-dihydro-1H-inden-1-yloxy)-2,3-dihydrobenzofuran-3-yl)acetic acid

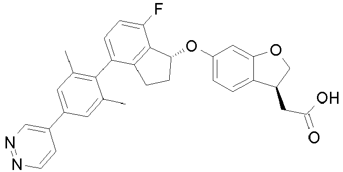

The title compound is prepared from methyl 2-((S)-6-((R)-4-(2,6-dimethyl-4-pyridazin-4-yl)phenyl)-7-fluoro-2,3-dihydro-1H-inden-1-yloxy)-2,3-dihydrobenzofuran-3-yl)acetate following a procedure analogous to that described in example 1. The mixture is stirred for 4 hours at 40°C. LC (method 15): t_{R} = 1.10 min; Mass spectrum (ESI⁺): m/z = 511 [M+H]⁺.

### Example 134

### 2-((S)-6-((R)-4-(2,6-Dimethyl-4-(pyrimidin-4-yl)phenyl)-7-fluoro-2,3-dihydro-1H-inden-1-yloxy)-2,3-dihydrobenzofuran-3-yl)acetic acid

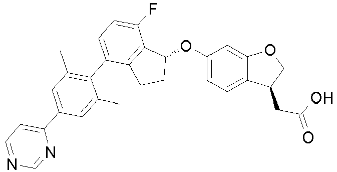

The title compound is prepared from methyl 2-((S)-6-((R)-4-(2,6-dimethyl-4-(pyrimidin-4-yl)phenyl)-7-fluoro-2,3-dihydro-1H-inden-1-yloxy)-2,3-dihydrobenzofuran-3-yl)acetate following a procedure analogous to that described in example 1. The mixture is stirred for 2 hours at 40°C. LC (method 15): t_{R} = 1.18 min; Mass spectrum (ESI⁺): m/z = 511 [M+H]⁺.

### Example 135

### 2-((S)-6-((R)-4-(2,6-Dimethyl-4-(1-methyl-6-oxo-1,6-dihydropyridazin-4-yl)phenyl)-7-fluoro-2,3-dihydro-1H-inden-1-yloxy)-2,3-dihydrobenzofuran-3-yl)acetic acid

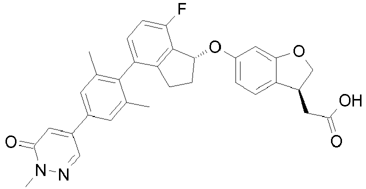

The title compound is prepared from methyl 2-((S)-6-((R)-4-(2,6-dimethyl-4-(1-methyl-6-oxo-1,6-dihydropyridazin-4-yl)phenyl)-7-fluoro-2,3-dihydro-1H-inden-1-yloxy)-2,3-dihydrobenzofuran-3-yl)acetate following a procedure analogous to that described in example 1. The mixture is stirred for 2 days at room temperature. LC (method 14): t_{R} = 0.82 min; Mass spectrum (ESI⁺): m/z = 541 [M+H]⁺.

### Example 136

### Methyl 2-((S)-6-((R)-7-fluoro-4-(4-(2-methoxypyrimidin-4-yl)-2,6-dimethylphenyl)-2,3-dihydro-1H-inden-1-yloxy)-2,3-dihydrobenzofuran-3-yl)acetate

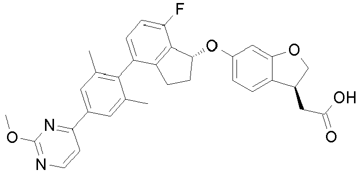

The title compound is prepared from methyl 2-((S)-6-((R)-7-fluoro-4-(4-(2-methoxypyrimidin-4-yl)-2,6-dimethylphenyl)-2,3-dihydro-1H-inden-1-yloxy)-2,3-dihydrobenzofuran-3-yl)acetate following a procedure analogous to that described in example 1. The mixture is stirred for 4 hours at room temperature. LC (method 8): t_{R} = 0.60 min; Mass spectrum (ESI⁺): m/z = 541 [M+H]⁺.

### Example 137

### 2-((S)-6-((R)-4-(2,6-Dimethyl-4-(1H-tetrazol-5-yl)phenyl)-7-fluoro-2,3-dihydro-1H-inden-1-yloxy)-2,3-dihydrobenzofuran-3-yl)acetic acid

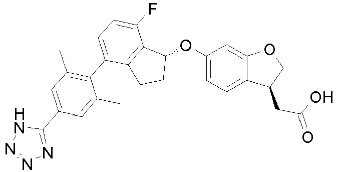

The title compound is prepared from methyl 2-((S)-6-((R)-4-(2,6-dimethyl-4-(1 H-tetrazol-5-yl)phenyl)-7-fluoro-2,3-dihydro-1H-inden-1-yloxy)-2,3-dihydrobenzofuran-3-yl)acetate following a procedure analogous to that described in example 1. The mixture is stirred for 2 hours at 40°C. The product is purified by HPLC on reversed phase. LC (method 11): t_{R} = 1.05 min; Mass spectrum (ESI⁺): m/z = 501 [M+H]⁺.

### Example 138

### 2-((S)-6-((R)-4-(2,6-Dimethyl-4-(2-methyl-2H-tetrazol-5-yl)phenyl)-7-fluoro-2,3-dihydro-1H-inden-1-yloxy)-2,3-dihydrobenzofuran-3-yl)acetic acid

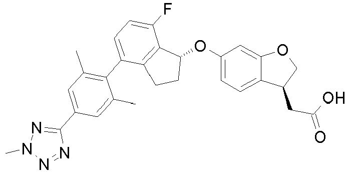

The title compound is prepared from methyl 2-((S)-6-((R)-4-(2,6-dimethyl-4-(2-methyl-2H-tetrazol-5-yl)phenyl)-7-fluoro-2,3-dihydro-1H-inden-1-yloxy)-2,3-dihydrobenzofur an-3-yl)acetate following a procedure analogous to that described in example 1. The mixture is stirred for 3 hours at 40°C. LC (method 11): t_{R} = 1.17 min; Mass spectrum (ESI⁺): m/z = 515 [M+H]⁺.

### Example 139

### 2-((S)-6-((R)-4-(2,6-Dimethyl-4-(1-methyl-1H-tetrazol-5-yl)phenyl)-7-fluoro-2,3-dihydro-1H-inden-1-yloxy)-2,3-dihydrobenzofuran-3-yl)acetic acid

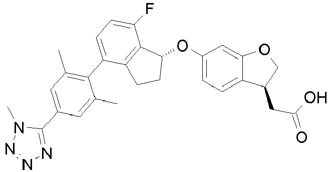

The title compound is prepared from methyl 2-((S)-6-((R)-4-(2,6-dimethyl-4-(1-methyl-1H-tetrazol-5-yl)phenyl)-7-fluoro-2,3-dihydro-1H-inden-1-yloxy)-2,3-dihydrobenzofuran-3-yl)acetate following a procedure analogous to that described in example 1. The mixture is stirred for 2 hours at 40°C. The product is purified by HPLC on reversed phase. LC (method 11): t_{R} = 1.10 min; Mass spectrum (ESI⁺): m/z = 515 [M+H]⁺.

### Example 140

### 2-((S)-6-((R)-4-(2,6-Dimethyl-4-(1-methyl-2-oxo-1,2-dihydropyrimidin-4-yl)phenyl)-7-fluoro-2,3-dihydro-1H-inden-1-yloxy)-2,3-dihydrobenzofuran-3-yl)acetic acid

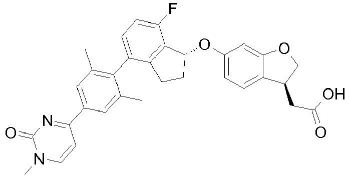

The title compound is prepared from methyl 2-((S)-6-((R)-4-(2,6-dimethyl-4-(1-methyl-2-oxo-1,2-dihydropyrimidin-4-yl)phenyl)-7-fluoro-2,3-dihydro-1H-inden-1-yloxy)-2,3-dihydrobenzofuran-3-yl)acetate following a procedure analogous to that described in example 1. The mixture is stirred for 6 hours at room temperature. LC (method 11): t_{R} = 1.01 min; Mass spectrum (ESI⁺): m/z = 541 [M+H]⁺.

### Example 141

### 2-((S)-6-((R)-7-Fluoro-4-(4-(2-(2-hydroxy-2-methylpropyl)-2H-tetrazol-5-yl)-2,6-dimethyliphenyl)-2,3-dihydro-1H-inden-1-yloxy)-2,3-dihydrobenzofuran-3-yl)acetic acid

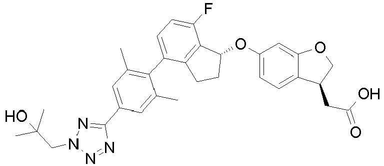

The title compound is prepared from methyl 2-((S)-6-((R)-7-fluoro-4-(4-(2-(2-hydroxy-2-methylpropyl)-2H-tetrazol-5-yl)-2,6-dimethylphenyl)-2,3-dihydro-1H-inden-1-yloxy)-2,3-dihydrobenzofuran-3-yl)acetate following a procedure analogous to that described in example 1. The mixture is stirred for 2 hours at 40°C. LC (method 11): t_{R} = 1.15 min; Mass spectrum (ESI⁺): m/z = 573 [M+H]⁺.

### Example 142

### 2-((S)-6-((R)-7-Fluoro-4-(4-(2-(1-hydroxy-2-methylpropan-2-yl)-2H-tetrazol-5-yl)-2,6-dimethylphenyl)-2,3-dihydro-1H-inden-1-yloxy)-2,3-dihydrobenzofuran-3-yl)acetic acid

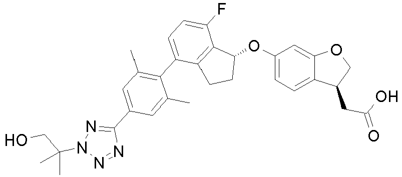

The title compound is prepared from methyl 2-((S)-6-((R)-7-fluoro-4-(4-(2-(1-hydroxy-2-methylpropan-2-yl)-2H-tetrazol-5-yl)-2,6-dimethylphenyl)-2,3-dihydro-1H-inden-1-yloxy)-2,3-dihydrobenzofuran-3-yl)acetate following a procedure analogous to that described in example 1. The mixture is stirred for 2 hours at 40°C. LC (method 11): t_{R} = 1.16 min; Mass spectrum (ESI⁺): m/z = 573 [M+H]⁺.

### Example 143*

### 2-((3S)-6-((1R)-4-(2-(Piperidin-1-yl)pyridin-3-yl)-2,3-dihydro-1H-inden-1-yloxy)-2,3-dihydrobenzofuran-3-yl)acetic acid

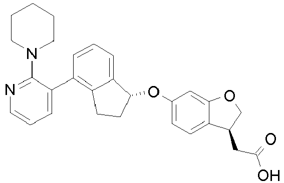

The title compound is prepared from methyl 2-((3S)-6-((1R)-4-(2-(piperidin-1-yl)pyridin-3-yl)-2,3-dihydro-1H-inden-1-yloxy)-2,3-dihydrobenzofuran-3-yl)acetate following a procedure analogous to that described in example 10. LC (method 7): t_{R} = 0.90 min; Mass spectrum (ESI⁺): m/z = 471 [M+H]⁺.

### Example 144*

### 2-((3S)-6-((1R)-4-(2-Phenoxypyridin-3-yl)-2,3-dihydro-1H-inden-1-yloxy)-2,3-dihydrobenzofuran-3-yl)acetic acid

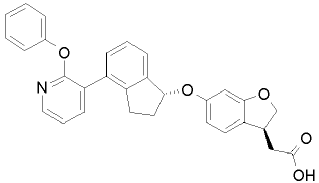

The title compound is prepared from methyl 2-((3S)-6-((1 R)-4-(2-phenoxypyridin-3-yl)-2,3-dihydro-1H-inden-1-yloxy)-2,3-dihydrobenzofuran-3-yl)acetate following a procedure analogous to that described in example 10. LC (method 7): t_{R} = 1.12 min; Mass spectrum (ESI⁺): m/z = 480 [M+H]⁺.

### Example 145*

### 2-((3S)-6-((1R)-4-(2-(Neopentyloxy)pyridin-3-yl)-2,3-dihydro-1H-inden-1-yloxy)-2,3-dihydrobenzofuran-3-yl)acetic acid

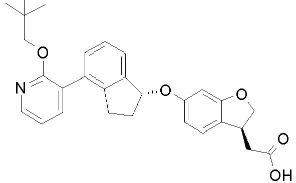

The title compound is prepared from methyl 2-((3S)-6-((1 R)-4-(2-(neopentyloxy)pyridin-3-yl)-2,3-dihydro-1H-inden-1-yloxy)-2,3-dihydrobenzofuran-3-yl)acetate following a procedure analogous to that described in example 1. The product is purified by HPLC on reversed phase. LC (method 15): t_{R} = 1.26 min; Mass spectrum (ESI⁺): m/z = 492 [M+H]⁺.

### Example 146

### 2-((S)-6-((R)-4-(2,6-Dimethyl-4-(1-methyl-6-oxo-1,6-dihydropyridazin-3-yl)phenyl)-7-fluoro-2,3-dihydro-1H-inden-1-yloxy)-2,3-dihydrobenzofuran-3-yl)acetic acid

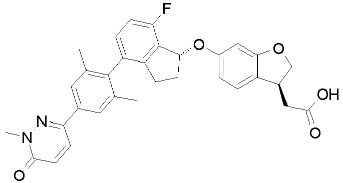

The title compound is prepared from methyl 2-((S)-6-((R)-4-(2,6-dimethyl-4-(1-methyl-6-oxo-1,6-dihydropyridazin-3-yl)phenyl)-7-fluoro-2,3-dihydro-1 H-inden-1-yloxy)-2,3-dihydrobenzofuran-3-yl)acetate following a procedure analogous to that described in example 1. LC (method 7): t_{R} = 1.09 min; Mass spectrum (ESI⁺): m/z = 541 [M+H]⁺.

### Example 147

### 2-((3S)-6-((1R)-4-(2-(Dimethylcarbamoyl)phenyl)-7-fluoro-2,3-dihydro-1H-inden-1-yloxy)-2,3-dihydrobenzofuran-3-yl)acetic acid

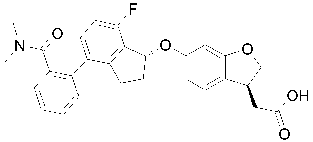

The title compound is prepared from methyl 2-((3S)-6-((1 R)-4-(2-(dimethylcarbamoyl)phenyl)-7-fluoro-2,3-dihydro-1H-inden-1-yloxy)-2,3-dihydrobenzofuran-3-yl)acetate following a procedure analogous to that described in example 1. The product is purified by HPLC on reversed phase. LC (method 9): t_{R} = 1.01 min; Mass spectrum (ESI⁺): m/z = 476 [M+H]⁺.

### Example 148

### 2-((3S)-6-((1R)-7-Fluoro-4-(3-fluoro-2-(1-methyl-1H-tetrazol-5-yl)phenyl)-2,3-dihydro-1H-inden-1-yloxy)-2,3-dihydrobenzofuran-3-yl)acetic acid

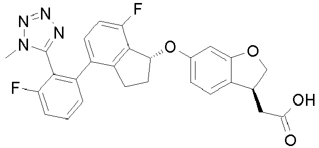

The title compound is prepared from methyl 2-((3S)-6-((1S)-7-fluoro-4-(3-fluoro-2-(1-methyl-1 H-tetrazol-5-yl)phenyl)-2,3-dihydro-1H-inden-1-yloxy)-2,3-dihydrobenzofuran-3-yl)acetate following a procedure analogous to that described in example 1. The product is purified by HPLC on reversed phase. LC (method 9): t_{R} = 1.01 min; Mass spectrum (ESI⁺): m/z = 505 [M+H]⁺.

### Example 149

### {(S)-6-[(R)-4-(2,6-Dimethyl-4-pyridin-4-yl-phenyl)-7-fluoro-indan-1-yloxyl-2,3-dihydrobenzofuran-3-yl}-acetic acid

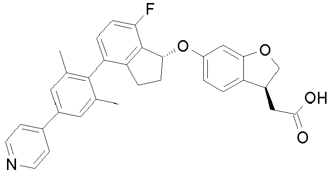

The title compound is prepared from {(*S*)-6-[(*R*)-4-(2,6-dimethyl-4-pyridin-4-yl-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 0.98 min; Mass spectrum (ESI⁺): m/z = 510 [M+H]⁺.

### Example 150

### {(S)-6-[(R)-7-Fluoro-4-(2-furan-3-yl-pyridin-3-yl)-indan-1-yloxy]-2,3-dihydrobenzofuran-3-yl}-acetic acid

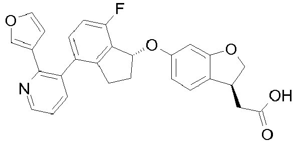

The title compound is prepared from {(*S*)-6-[(*R*)-7-fluoro-4-(2-furan-3-yl-pyridin-3-yl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.04 min; Mass spectrum (ESI⁺): m/z = 472 [M+H]⁺.

### Example 151

### {(S)-6-(R)-7-Fluoro-4-(2-phenl-pyridin-3-yl)-indan-1-yloxy]-2,3-dihydro-benzofuran-3-yl}-acetic acid

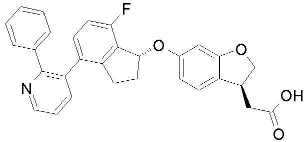

The title compound is prepared from {(*S*)-6-[(*R*)-7-fluoro-4-(2-phenyl-pyridin-3-yl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.04 min; Mass spectrum (ESI⁺): m/z = 482 [M+H]⁺.

### Example 152

### {(S)-6-[(R)-4-(2.6-Dimethyl-4-(2-methyl-pyridin-4-yl)-phenyl)-7-fluoro-indan-1-yloxy]-2,3-dihydro-benzofuran-3-yl}-acetic acid

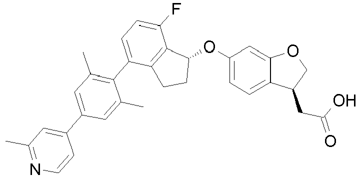

The methyl ester of the title compound is prepared from {(*S*)-6-[(*R*)-7-fluoro-4-(4,4,5,5-tetramethyl-[1,3,2]dioxaborolan-2-yl)-indan-1-yloxy]-2,3-dihydro-benzofuran-3-yl}-acetic acid methyl ester and 4-(4-bromo-3,5-dimethyl-phenyl)-2-methyl-pyridine following a procedure analogous to that described in Step 5 of Intermediate 12. Saponification of the methyl ester following a procedure analogous to that described for Example 10 gives the title compound; if the title compound does not precipitate from the aqueous solution, the crude title compound is purified by HPLC. LC (method 9): t_{R} = 0.95 min; Mass spectrum (ESI⁺): m/z = 524 [M+H]⁺.

### Example 153

### {(S)-6-[(R)-7-Fluoro-4-[2-(1,3,5-trimethyl-pyrazol-4-yl)-pyridin-3-yl]-indan-1-yloxy]-2,3-dihydro-benzofuran-3-yl}-acetic acid

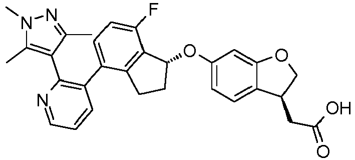

The methyl ester of the title compound is prepared from {(*S*)-6-[(*R*)-4-(2-bromo-pyridin-3-yl)-7-fluoro-indan-1-yloxy]-2,3-dihydro-benzofuran-3-yl}-acetic acid methyl ester and 1,3,5-trimethyl-4-(4,4,5,5-tetramethyl-[1,3,2]dioxaborolan-2-yl)-pyrazole following a procedure analogous to that described in Step 5 of Intermediate 12. Saponification of the methyl ester, {(*S*)-6-[(*R*)-7-fluoro- 4-[2-(1,3,5-trimethyl-1H-pyrazol-4-yl)-pyridin-3-yl]-indan-1-yloxy]-2,3-dihydro-benzofuran-3-yl}-acetic acid methyl ester, following a procedure analogous to that described for Example 10 gives the title compound; if the title compound does not precipitate from the aqueous solution, the crude title compound is purified by HPLC. LC (method 9): t_{R} = 0.88 min; Mass spectrum (ESI⁺): m/z = 514 [M+H]⁺.

### Example 154

### {(S)-6-[(R)-4-(2,6-Dimethyl-4-(1,3,5-trimethyl-pyrazol-4-yl)-phenyl)-7-fluoro-indan-1-yloxy]-2,3-dihydro-benzofuran-3-yl}-acetic acid

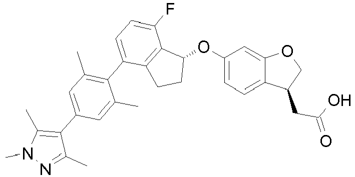

The title compound is prepared from {(*S*)-6-[(*R*)-7-fluoro-4-(2,6-dimethyl-4-(1,3,5-trimethyl-pyrazol-4-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.13 min; Mass spectrum (ESI⁺): m/z = 541 [M+H]⁺.

### Example 155

### {(S)-6-[(R)-7-Fluoro-4-(2-tetrahydropyran-4-yl-pyridin-3-yl)-indan-1-yloxy]-2,3-dihydro-benzofuran-3-yl}-acetic acid

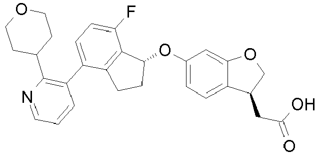

A mixture of {(*S*)-6-[(*R*)-7-fluoro-4-(2-(3,6-dihydro-pyran-4-yl)-pyridin-3-yl)-indan-1-yloxy]-2,3-dihydro-benzofuran-3-yl}-acetic acid (30 mg), 10% palladium on carbon (10 mg) and methanol (3 mL) is shaken under hydrogen atmosphere (3 bar) at room temperature for 12 h. The mixture is filtered over Celite and the filtrate is concentrated to give the title compound. LC (method 9): t_{R} = 0.96 min; Mass spectrum (ESI⁺): m/z = 490 [M+H]⁺.

### Example 156

### {(S)-6-[(R)-4-(2,6-Dimethyl-4-(5-methyl-pyridin-3-yl)-phenyl)-7-fluoro-indan-1-yloxy]-2,3-dihydro-benzofuran-3-yl}-acetic acid

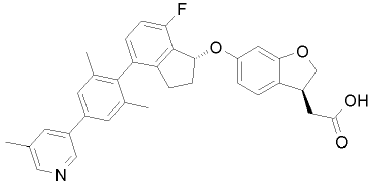

The title compound is prepared from {(*S*)-6-[(*R*)-4-(2,6-dimethyl-4-(5-methyl-pyridin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.06 min; Mass spectrum (ESI⁺): m/z = 524 [M+H]⁺.

### Example 157

### {(S)-6-[(R)-4-(2,6-Dimethyl-4-pyridin-3-yl-phenyl)-7-fluoro-indan-1-yloxy]-2,3-dihydrobenzofuran-3-yl}-acetic acid

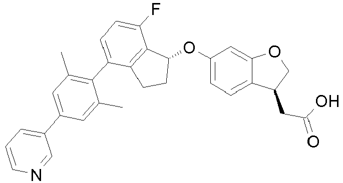

The title compound is prepared from {(*S*)-6-[(*R*)-4-(2,6-dimethyl-4-pyridin-3-yl-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.06 min; Mass spectrum (ESI⁺): m/z = 510 [M+H]⁺.

### Example 158

### {(S)-6-[(R)-4-(2,6-Dimethyl-4-(5-methyl-pyrimidin-2-yl)-phenyl)-7-fluoro-indan-1-yloxy]-2,3-dihydro-benzofuran-3-yl}-acetic acid

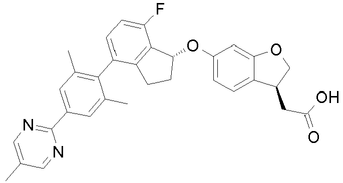

The title compound is prepared from {(*S*)-6-[(*R*)-4-(2,6-dimethyl-4-(5-methyl-pyrimidin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 1.19 min; Mass spectrum (ESI⁻): m/z = 523 [M-H]⁻.

### Example 159

### {(S)-6-[(R)-4-(2,6-Dimethyl-4-(5-methyl-pyrazin-2-yl)-phenyl)-7-fluoro-indan-1-yloxy]-2,3-dihydro-benzofuran-3-yl}-acetic acid

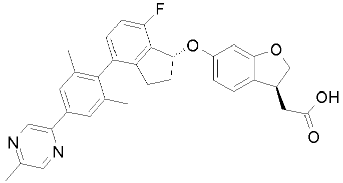

The title compound is prepared from {(*S*)-6-[(*R*)-4-(2,6-dimethyl-4-(5-methyl-pyrazin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 1.05 min; Mass spectrum (ESI⁺): m/z = 525 [M+H]⁺.

### Example 160

### {(S)-6-[(R)-4-(2,6-Dimethyl-4-(2,6-dimethyl-pyrimidin-4-yl)-phenyl)-7-fluoro-indan-1-yloxy]-2,3-dihydro-benzofuran-3-yl}-acetic acid

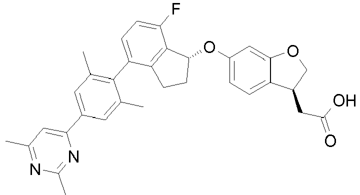

The title compound is prepared from {(*S*)-6-[(*R*)-4-(2,6-dimethyl-4-(2,6-dimethyl-pyrimidin-4-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 1.15 min; Mass spectrum (ESI⁻): m/z = 537 [M-H]⁻.

### Example 161

### {(S)-6-[(R)-4-(2,6-Dimethyl-4-(2-methoxy-pyridin-4-yl)-phenyl)-7-fluoro-indan-1-yloxy]-2,3-dihydro-benzofuran-3-yl}-acetic acid

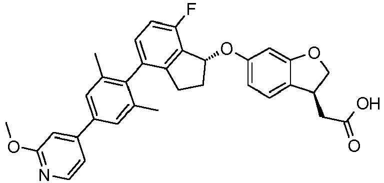

The title compound is prepared from {(*S*)-6-[(*R*)-4-(2,6-dimethyl-4-(2-methoxy-pyridin-4-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.23 min; Mass spectrum (ESI⁺): m/z = 540 [M+H]⁺.

### Example 162

### {(S)-6-[(R)-4-(2,6-Dimethyl-4-(6-methyl-pyridin-3-yl)-phenyl)-7-fluoro-indan-1-yloxy]-2,3-dihydro-benzofuran-3-yl}-acetic acid

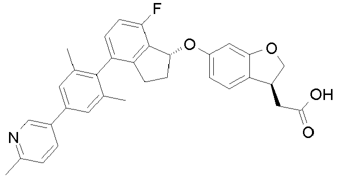

The title compound is prepared from {(*S*)-6-[(*R*)-4-(2,6-dimethyl-4-(6-methyl-pyridin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.84 min; Mass spectrum (ESI⁺): m/z = 524 [M+H]⁺.

### Example 163

### {(S)-6-[(R)-4-(2,6-Dimethyl-4-(6-methyl-pyrazin-2-yl)-phenyl)-7-fluoro-indan-1-yloxy]-2,3-dihydro-benzofuran-3-yl}-acetic acid

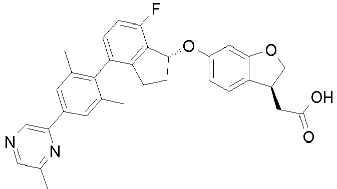

The title compound is prepared from {(*S*)-6-[(*R*)-4-(2,6-dimethyl-4-(6-methyl-pyrazin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.18 min; Mass spectrum (ESI⁺): m/z = 525 [M+H]⁺.

### Example 164

### {(S)-6-[(R)-4-(2,6-Dimethyl-4-(4-methyl-pyrimidin-2-yl)-phenyl)-7-fluoro-indan-1-yloxy]-2,3-dihydro-benzofuran-3-yl}-acetic acid

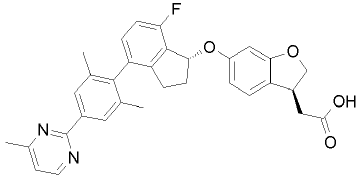

The title compound is prepared from {(*S*)-6-[(*R*)-4-(2,6-dimethyl-4-(4-methyl-pyrimidin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.20 min; Mass spectrum (ESI⁻): m/z = 523 [M-H]⁻.

### Example 165

### {(S)-6-[(R)-4-(2,6-Dimethyl-4-(6-methyl-pyridazin-3-yl)-phenyl)-7-fluoro-indan-1-yloxy]-2,3-dihydro-benzofuran-3-yl}-acetic acid

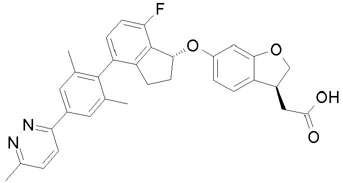

The title compound is prepared from {(*S*)-6-[(*R*)-4-(2,6-dimethyl-4-(6-methyl-pyridazin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.08 min; Mass spectrum (ESI⁺): m/z = 525 [M+H]⁺.

### Example 166

### {(S)-6-[(R)-4-(2,6-Dimethyl-4-(2,6-dimethyl-pyridin-4-yl)-phenyl)-7-fluoro-indan-1-yloxy]-2,3-dihydro-benzofuran-3-yl}-acetic acid

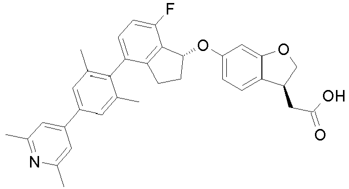

The title compound is prepared from {(*S*)-6-[(*R*)-4-(2,6-dimethyl-4-(2,6-dimethyl-pyridin-4-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.99 min; Mass spectrum (ESI⁺): m/z = 538 [M+H]⁺.

### Example 167

### {(S)-6-[(R)-4-(2,6-Dimethyl-4-pyrazin-2-yl-phenyl)-7-fluoro-indan-1-yloxy]-2,3-dihydrobenzofuran-3-yl}-acetic acid

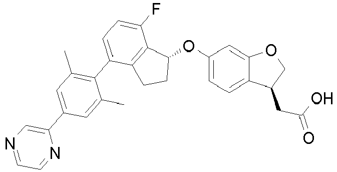

The title compound is prepared from {(*S*)-6-[(*R*)-4-(2,6-dimethyl-4-pyrazin-2-yl-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 1.14 min; Mass spectrum (ESI⁺): m/z = 511 [M+H]⁺.

### Example 168

### {(S)-6-(R)-4-(2,6-Dimethyl-4-(5-cyclopropyl-pyrazin-2-yl)-phenyl)-7-fluoro-indan-1-yloxy]-2,3-dihydro-benzofuran-3-yl}-acetic acid

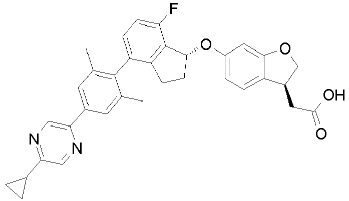

The title compound is prepared from {(*S*)-6-[(*R*)-4-(2,6-dimethyl-4-(5-cyclopropyl-pyrazin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.27 min; Mass spectrum (ESI⁺): m/z = 551 [M+H]⁺.

### Example 169

### {(S)-6-[(R)-4-(2-(2,6-Dimethyl-phenyl)-pyridin-3-yl)-7-fluoro-indan-1-yloxy]-2,3-dihydro-benzofuran-3-yl}-acetic acid

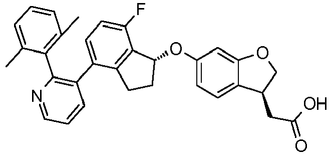

The title compound is prepared from {(*S*)-6-[(*R*)-4-(2-(2,6-dimethyl-phenyl)-pyridin-3-yl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.80 min; Mass spectrum (ESI⁺): m/z = 510 [M+H]⁺.

### Example 170

### {(S)-6-[(R)-4-(2,6-Dimethyl-4-(6-ethyl-pyridazin-3-yl)-phenyl)-7-fluoro-indan-1-yloxy]-2,3-dihydro-benzofuran-3-yl}-acetic acid

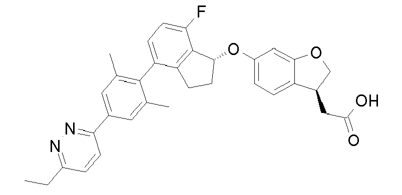

The title compound is prepared from {(*S*)-6-[(*R*)-4-(2,6-dimethyl-4-(6-ethyl-pyridazin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.06 min; Mass spectrum (ESI⁺): m/z = 539 [M+H]⁺.

### Example 171

### {(S)-6-[(R)-4-(2,6-Dimethyl-4-(6-methoxy-pyridin-3-yl)-phenyl)-7-fluoro-indan-1-yloxy-]-2,3-dihydro-benzofuran-3-yl}-acetic acid

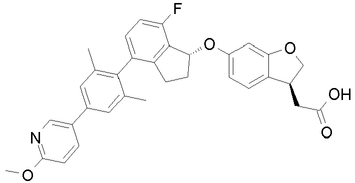

The title compound is prepared from {(*S*)-6-[(*R*)-4-(2,6-dimethyl-4-(6-methoxy-pyridin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.24 min; Mass spectrum (ESI⁺): m/z = 540 [M+H]⁺.

### Example 172

### {(S)-6-[(R)-7-Fluoro-4-(2-[1,4]oxazepan-4-yl-pyridin-3-yl)-indan-1-yloxy]-2,3-dihydrobenzofuran-3-yl}-acetic acid

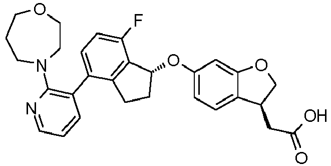

The title compound is prepared from {(*S*)-6-[(*R*)-7-fluoro-4-(2-fluoro-pyridin-3-yl)-indan-1-yloxy]-2,3-dihydro-benzofuran-3-yl}-acetic acid methyl ester and [1,4]oxazepane following a procedure analogous to that described for Example 173. LC (method 9): t_{R} = 0.87 min; Mass spectrum (ESI⁺): m/z = 505 [M+H]⁺.

### Example 173

### {(S)-6-[(R)-7-Fluoro-4-(2-morpholin-4-yl-pyridin-3-yl)-indan-1-yloxy]-2,3-dihydrobenzofuran-3-yl}-acetic acid

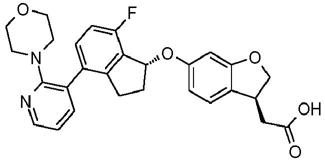

A mixture of {(*S*)-6-[(*R*)-7-fluoro-4-(2-fluoro-pyridin-3-yl)-indan-1-yloxy]-2,3-dihydro-benzofuran-3-yl}-acetic acid methyl ester (0.10 g), morpholine (0.10 g) and N-methylpyrrolidone (2 mL) is stirred at 180 °C for 48 h. After cooling to room temperature, 2 N aqueous NaOH solution (0.5 mL) is added, and the resulting mixture is stirred at 40 °C for 2 h. The mixture is neutralized with 1 N aqueous HCl solution and concentrated. The residue is chromatographed on reversed phase (HPLC; acetonitrile/water) to give the title compound. LC (method 7): t_{R} = 0.89 min; Mass spectrum (ESI⁺): m/z = 491 [M+H]⁺.

### Example 174

### {(S)-6-[(R)-4-(2,6-Dimethyl-4-(5-methoxy-pyridazin-3-yl)-phenyl)-7-fluoro-indan-1-yloxy]-2,3-dihydro-benzofuran-3-yl}-acetic acid

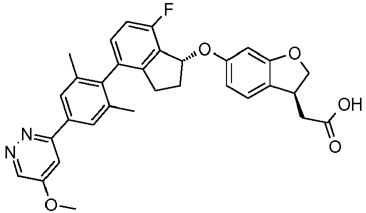

The title compound is prepared from {(*S*)-6-[(*R*)-4-(2,6-dimethyl-4-(5-methoxy-pyridazin-3-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.10 min; Mass spectrum (ESI⁺): m/z = 541 [M+H]⁺.

### Example 175

### {(S)-6-[(R)-4-(2,6-Dimethyl-4-(6-methyl-pyridazin-4-yl)-phenyl)-7-fluoro-indan-1-yloxy]-2,3-dihvdro-benzofuran-3-yl}-acetic acid

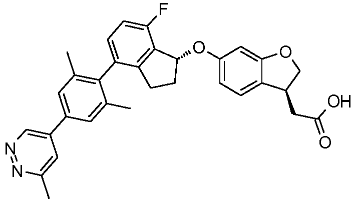

The title compound is prepared from {(*S*)-6-[(*R*)-4-(2,6-dimethyl-4-(6-methyl-pyridazin-4-yl)-phenyl)-7-fluoro-indan-1-yloxy]-2,3-dihydro-benzofuran-3-yl}-acetic acid methyl ester following a procedure analogous to that described for Example 10. LC (method 11): t_{R} = 1.00 min; Mass spectrum (ESI⁺): m/z = 525 [M+H]⁺.

### Example 176

### {(S)-6-[(R)-4-(2,6-Dimethyl-4-(1,2-dimethyl-imidazol-4-yl)-phenyl)-7-fluoro-indan-1-yloxy]-2,3-dihydro-benzofuran-3-yl}-acetic acid

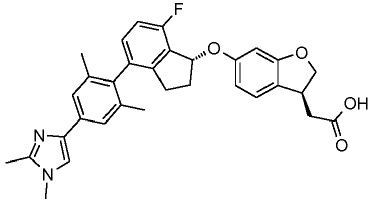

The title compound is prepared from {(*S*)-6-[(*R*)-7-fluoro-4-(2,6-dimethyl-4-(1,2-dimethyl-imidazol-4-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 0.88 min; Mass spectrum (ESI⁺): m/z = 527 [M+H]⁺.

### Example 177

### {(S)-6-[(R)-4-(2,6-Dimethyl-4-thiazol-2-yl-phenyl)-7-fluoro-indan-1-yloxy]-2,3-dihydro-benzofuran-3-yl}-acetic acid

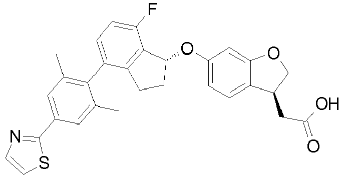

The title compound is prepared from {(*S*)-6-[(*R*)-7-fluoro-4-(2,6-dimethyl-4-thiazol-2-yl-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 1.21 min; Mass spectrum (ESI⁺): m/z = 516 [M+H]⁺.

### Example 178

### {(S)-6-[(R)-4-(2,6-Dimethyl-4-(1-methyl-imidazol-4-yl)-phenyl)-7-fluoro-indan-1-yloxy]-2,3-dihydro-benzofuran-3-yl}-acetic acid

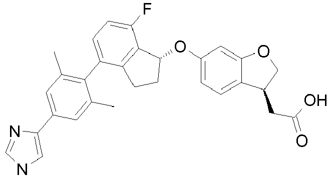

The title compound is prepared from {(*S*)-6-[(*R*)-7-fluoro-4-(2,6-dimethyl-4-(1-methyl-imidazol-4-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 9): t_{R} = 0.89 min; Mass spectrum (ESI⁺): m/z = 513 [M+H]⁺.

### Example 179

### {(S)-6-[(R)-4-(2,6-Dimethyl-4-(5-methyl-[1,2,4]oxadiazol-3-yl)-phenyl)-7-fluoro-indan-1-yloxy]-2,3-dihydro-benzofuran-3-yl)-acetic acid

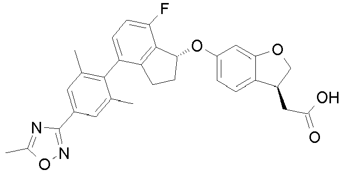

The title compound is prepared from {(*S*)-6-[(*R*)-7-fluoro-4-(2,6-dimethyl-4-(5-methyl-[1,2,4]oxadiazol-3-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.20 min; Mass spectrum (ESI⁺): m/z = 515 [M+H]⁺.

### Example 180

### {(S)-6-[(R)-4-(2,6-Dimethyl-4-(5-methoxy-pyridin-2-yl)-phenyl)-7-fluoro-indan-1-yloxy]-2,3-dihydro-benzofuran-3-yl}-acetic acid

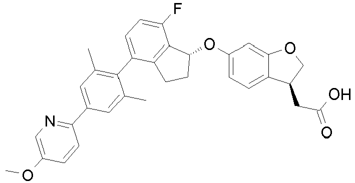

The title compound is prepared from {(*S*)-6-[(*R*)-4-(2,6-dimethyl-4-(5-methoxy-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 8): t_{R} = 0.79 min; Mass spectrum (ESI⁺): m/z = 540 [M+H]⁺.

### Example 181

### {(S)-6-[(R)-4-(2,6-Dimethyl-4-(5-[2-hydroxy-prop-2-yl}-[1,2,4]oxadiazol-3-yl)-phenyl)-7-fluoro-indan-1-yloxy]-2,3-dihydro-benzofuran-3-yl}-acetic acid

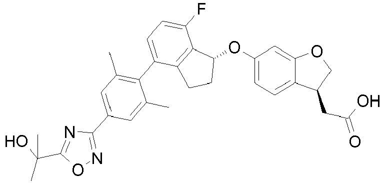

The title compound is prepared from {(*S*)-6-[(*R*)-7-fluoro-4-(2,6-dimethyl-4-(5-[2-hydroxy-prop-2-yl]-[1,2,4]oxadiazol-3-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 11): t_{R} = 1.16 min; Mass spectrum (ESI⁺): m/z = 559 [M+H]⁺.

### Example 182

### {(S)-6-[(R)-4-(2,6-Dimethyl-4-pyridin-2-yl-phenyl)-7-fluoro-indan-1-yloxyl-2,3-dihydro-benzofuran-3-yl}-acetic acid

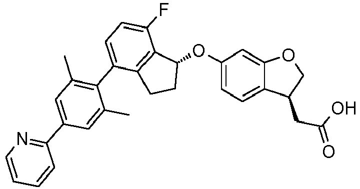

The title compound is prepared from {(*S*)-6-[(*R*)-4-(2,6-dimethyl-4-pyridin-2-yl-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.99 min; Mass spectrum (ESI⁺): m/z = 510 [M+H]⁺.

### Example 183

### {(S)-6-[(R)-4-(2,6-Dimethyl-4-(3-methyl-pyridin-2-yl)-phenyl)-7-fluoro-indan-1-yloxy]-2,3-dihydro-benzofuran-3-yl}-acetic acid

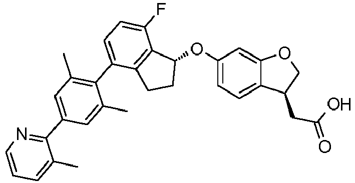

The title compound is prepared from {(*S*)-6-[(*R*)-4-(2,6-dimethyl-4-(3-methyl-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.97 min; Mass spectrum (ESI⁺): m/z = 524 [M+H]⁺.

### Example 184

### {(S)-6-[(R)-4-(2,6-Dimethyl-4-(6-methyl-pyridin-2-yl)-phenyl)-7-fluoro-indan-1-yloxy]-2,3-dihydro-benzofuran-3-yl}-acetic acid

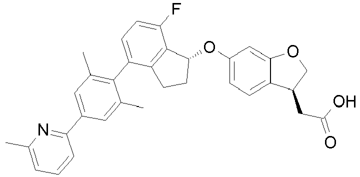

The title compound is prepared from {(*S*)-6-[(*R*)-4-(2,6-dimethyl-4-(6-methyl-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.99 min; Mass spectrum (ESI⁺): m/z = 524 [M+H]⁺.

### Example 185

### {(S)-6-[(R)-4-(2,6-Dimethyl-4-(4-methyl-pyridin-2-yl)-phenyl)-7-fluoro-indan-1-yloxy]-2,3-dihydro-benzofuran-3-yl}-acetic acid

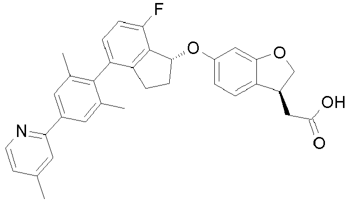

The title compound is prepared from {(*S*)-6-[(*R*)-4-(2,6-dimethyl-4-(4-methyl-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.98 min; Mass spectrum (ESI⁺): m/z = 524 [M+H]⁺.

### Example 186

### {(S)-6-[(R)-4-(2,6-Dimethyl-4-(4,6-dimethyl-pyridin-2-yl)-phenyl)-7-fluoro-indan-1-yloxy]-2,3-dihydro-benzofuran-3-yl}-acetic acid

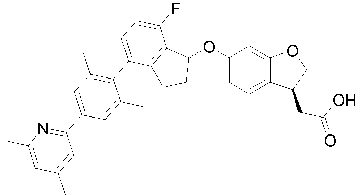

The title compound is prepared from {(*S*)-6-[(*R*)-4-(2,6-dimethyl-4-(4,6-dimethyl-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.99 min; Mass spectrum (ESI⁺): m/z = 538 [M+H]⁺.

### Example 187

### {(S)-6-[(R)-4-(2,6-Dimethyl-4-(1,4-dimethyl-imidazol-2-yl)-phenyl)-7-fluoro-indan-1-yloxy]-2,3-dihydro-benzofuran-3-yl}-acetic acid

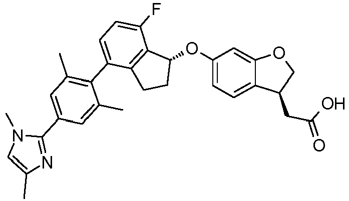

The title compound is prepared from {(*S*)-6-[(*R*)-7-fluoro-4-(2,6-dimethyl-4-(1,4-dimethyl-imidazol-2-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.95 min; Mass spectrum (ESI⁺): m/z = 527 [M+H]⁺.

### Example 188

### {(S)-6-[(R)-4-(2,6-Dimethyl-4-(5-methyl-pyridin-2-yl)-phenyl)-7-fluoro-indan-1-yloxy]-2,3-dihydro-benzofuran-3-yl}-acetic acid

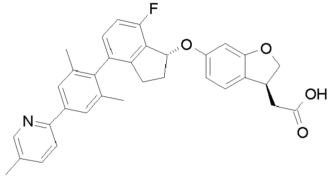

The title compound is prepared from {(*S*)-6-[(*R*)-4-(2,6-dimethyl-4-(5-methyl-pyridin-2-yl)-phenyl)-7-fluoro-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.99 min; Mass spectrum (ESI⁺): m/z = 524 [M+H]⁺.

### Example 189

### {(S)-6-[(R)-4-(2,6-Dimethyl-4-(1-methyl-imidazol-2-yl)-phenyl)-7-fluoro-indan-1-yloxy]-2,3-dihydro-benzofuran-3-yl}-acetic acid

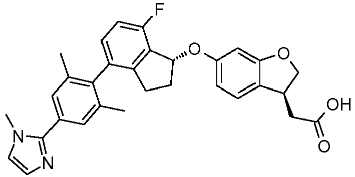

The title compound is prepared from {(*S*)-6-[(*R*)-7-fluoro-4-(2,6-dimethyl-4-(1-methyl-imidazol-2-yl)-phenyl)-indan-1-yloxy]-2,3-dihydro-benzofuran-3-yl}-acetic acid methyl ester following a procedure analogous to that described for Example 10; if the title compound does not precipitate from the aqueous solution, the crude title compound is purified by HPLC. LC (method 7): t_{R} = 0.94 min; Mass spectrum (ESI⁺): m/z = 513 [M+H]⁺.

**Saponification of the esters in the following table is performed in analogy to example 71**

| **Example No** | **Structure** | **Name** | | |
|---|---|---|---|---|
| **191** | 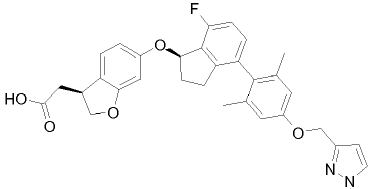 | 2-((S)-6-((R)-4-(2,6-dimethyl-4-((1-methyl-1H-pyrazol-3-yl)methoxy)phenyl)-7-fluoro-2,3-dihydro-1H-inden-1-yloxy)-2,3-dihydrobenzofuran-3-yl)acetic acid | | |
| **192** | 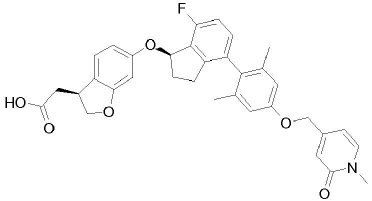 | 2-((S)-6-((R)-4-(2,6-dimethyl-4-((1-methyl-2-oxo-1,2-dihydropyridin-4-yl)methoxy)phenyl)-7-fluoro-2,3-dihydro-1H-inden-1-yloxy)-2,3-dihydrobenzofuran-3-yl)acetic acid | | |
| **193** | 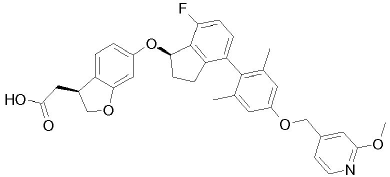 | 2-((S)-6-((R)-7-fluoro-4-(4-((2-methoxypyridin-4-yl)methoxy)-2,6-dimethylphenyl)-2,3-dihydro-1H-inden-1-yloxy)-2,3-dihydrobenzofuran-3-yl)acetic acid | | |
| **194** | 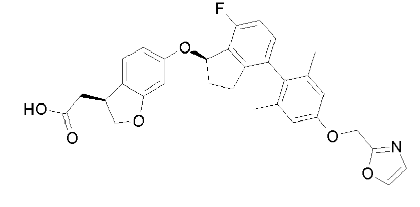 | 2-((S)-6-((R)-4-(2,6-dimethyl-4-(oxazol-2-ylmethoxy)phenyl)-7-fluoro-2,3-dihydro-1H-inden-1-yloxy)-2,3-dihydrobenzofuran-3-yl)acetic acid | | |
| **195** | 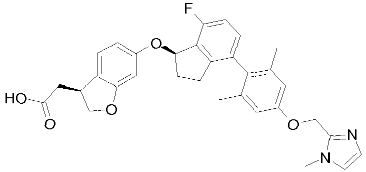 | 2-((S)-6-((R)-4-(2,6-dimethyl-4-((1-methyl-1H-imidazol-2-yl)methoxy)phenyl)-7-fluoro-2,3-dihydro-1H-inden-1-yloxy)-2,3-dihydrobenzofuran-3-yl)acetic acid | | |
| **196** | 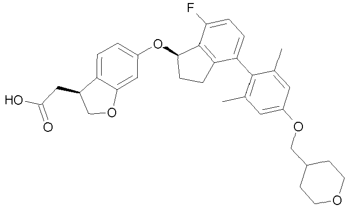 | 2-((S)-6-((R)-4-(2,6-dimethyl-4-((tetrahydro-2H-pyran-4-yl)methoxy)phenyl)-7-fluoro-2,3-dihydro-1H-inden-1-yloxy)-2,3-dihydrobenzofuran-3-yl)acetic acid | | |
| **197** | 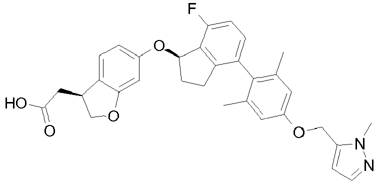 | 2-((S)-6-((R)-4-(2,6-dimethyl-4-((1-methyl-1H-pyrazol-5-yl)methoxy)phenyl)-7-fluoro-2,3-dihydro-1H-inden-1-yloxy)-2,3-dihydrobenzofuran-3-yl)acetic acid | | |
| **198** | 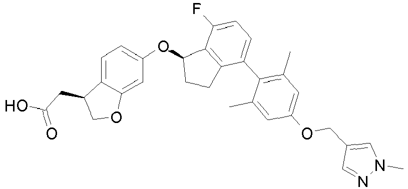 | 2-((S)-6-((R)-4-(2,6-dimethyl-4-((1-methyl-1H-pyrazol-4-yl)methoxy)phenyl)-7-fluoro-2,3-dihydro-1H-inden-1-yloxy)-2,3-dihydrobenzofuran-3-yl)acetic acid | | |

| **Example No** | **Structure** | **t_{R} [Min]** | **LC method No** | **(ESI⁺): m/z** |
|---|---|---|---|---|
| **191** | 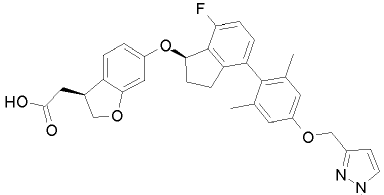 | 0.69 | 14 | 543 [M+H]⁺. |
| **192** | 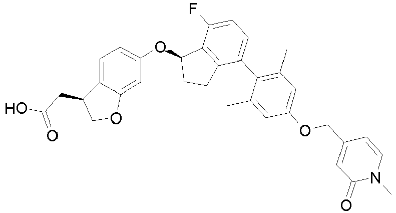 | 0.66 | 14 | 570 [M+H]⁺. |
| **193** | 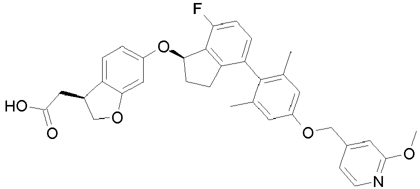 | 0.74 | 14 | 570 [M+H]⁺. |
| **194** | 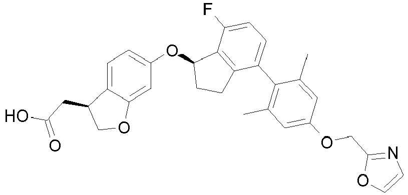 | 0.68 | 14 | 530 [M+H]⁺. |
| **195** | 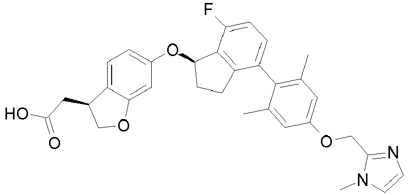 | 0.66 | 14 | 543 [M+H]⁺. |
| **196** | 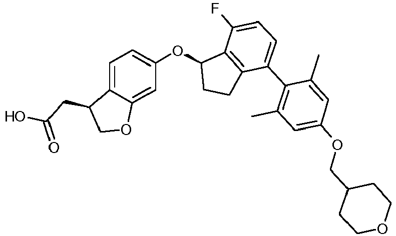 | 0.74 | 14 | 547 [M+H]⁺. |
| **197** | 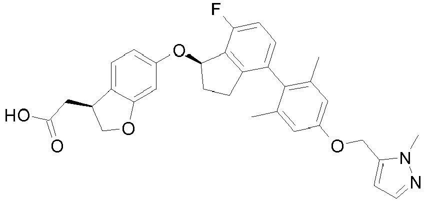 | 1.04 | 11 | 543 [M+H]⁺. |
| **198** | 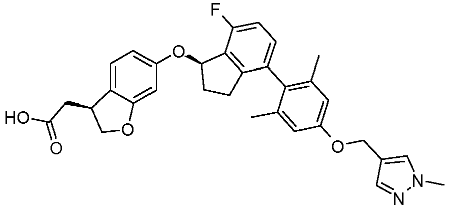 | 1.87 | 23 | 543 [M+H]⁺. |

## Claims

1. A compound of formula (I) 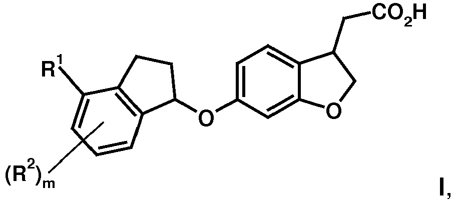 wherein
R¹ is selected from a group consisting of a phenyl ring, a tetrazolyl ring, and a 5- or 6-membered heteroaromatic ring which contains 1, 2, or 3 heteroatoms independently of each other selected from =N-, -NH-, -O- and -S-; wherein optionally a second ring is annulated to said phenyl or heteroaromatic ring,
wherein said second ring is 5- or 6-membered, unsaturated or aromatic and may contain 1, 2, or 3 heteroatoms independently of each other selected from =N-,-NH-, -O- and -S- with the proviso that only up to two of the heteroatoms are O and S and no O-O, S-S, and S-O bond is formed, and wherein in said second ring independently of the presence of heteroatoms 1 or 2 CH₂ groups may be replaced by -C(=O)-, -S(=O)- or -S(=O)₂-, and
wherein said phenyl ring, tetrazolyl ring, heteroaromatic ring, annulated phenyl ring, and annulated heteroaromatic ring are substituted with one group R³; and
wherein each of the phenyl ring, tetrazolyl ring, heteroaromatic ring, annulated phenyl ring, and annulated heteroaromatic ring is optionally additionally substituted with 1 to 4 groups independently selected from R⁴; and
wherein in said heteroaromatic ring and/or said second ring the H-atom in one or more NH groups, if present, is replaced by R^{N} or R³;
R² is selected from a group consisting of F, Cl, Br, H₂FC-, F₃C-, NC-, and F₃C-O-;
R³ is selected from a group consisting of
C₁₋₆-alkyl, C₂₋₆-alkenyl, C₂₋₆-alkinyl, C₃₋₆-cycloalkyl-, C₁₋₄-alkyl-NH-, (C₁₋₄-alkyl)₂N-, C₁₋₆-alkyl-O-, C₃₋₆-cycloalkyl-O-, C₁₋₄-alkyl-S-, C₁₋₄-alkyl-S(=O)-, and C₁₋₄-alkyl-S(=O)₂,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from C₁₋₄-alkyl-C(=O)-, heterocyclyl-C(=O)-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, HO₂C-, C₁₋₄-alkyl-O-C(=O)-, C₃₋₆-cycloalkyl-O-C(=O)-, heterocyclyl-O-C(=O)-, -NHR^{N}, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}- , heterocyclyl-S(=O)₂NR^{N}-, phenyl-S(=O)₂NR^{N}-, heteroaryl-S(=O)₂NR^{N}-, heterocyclyl-O-, phenyl-O-, heteroaryl-O-, C₃₋₆-cycloalkyl-S-, heterocyclyl-S-, phenyl-S-, heteroaryl-S-, C₃₋₆-cycloalkyl-S(=O)-, heterocyclyl-S(=O)-, phenyl-S(=O)-, heteroaryl-S(=O)-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups mentioned is optionally substituted with 1 to 3 groups independently selected from R⁵ and optionally substituted with 1 or more F atoms; and
wherein each phenyl and heteroaryl group is optionally substituted with 1 to 5 substituents independently selected from R⁶;
wherein heterocyclyl is selected from
a cyclobutyl group wherein 1 CH₂ group is replaced by -NH- or -O-,
a saturated or partially unsaturated C₅₋₇-cycloalkyl group wherein 1 CH₂ group is replaced by -C(=O)-, -NH-, -O-, -S(=O)- or -S(=O)₂- and/or 1 CH group by N;
a saturated or partially unsaturated C₅₋₇-cycloalkyl group wherein 1 CH₂ group is replaced by -NH- or -O-, a second CH₂ group is replaced by -NH-,-C(=O)-, -S(=O)- or -S(=O)₂- and/or 1 CH group is replaced by N; and
a saturated or partially unsaturated C₅₋₇-cycloalkyl group wherein 2 CH₂ groups are replaced by -NH- or 1 CH₂ group by -NH- and the other by -O-and a third CH₂ group is replaced by -C(=O)-, -S(=O)- or -S(=O)₂- and/or 1 CH group by N;
wherein heteroaryl is selected from
a tetrazolyl ring, and a 5- or 6-membered heteroaromatic ring which contains 1, 2, or 3 heteroatoms independently of each other selected from =N-, -NH-, -O-, and -S-, wherein in heteroaromatic groups containing a -HC=N- unit this group is optionally replaced by -NH-C(=O)-;
wherein in heteroaryl and heterocyclyl rings with one or more NH groups each of them is replaced by NR^{N} or NR⁵ ;
with the proviso that R³ in total cannot be C₁₋₄-alkyl-, C₁₋₄-alkyl-O-C₁₋₄-alkyl, HO-C₁₋₄-alkyl, C₃₋₄-alkenyl, C₃₋₄-alkinyl, H₂N-, C₁₋₄-alkyl-NH-, (C₁₋₄-alkyl)₂N-, C₁₋₄-alkyl-O-, C₁₋₄-alkyl-S-, C₁₋₄-alkyl-S(=O)-, and C₁₋₄-alkyl-S(=O)₂;
R⁴ is selected from a group consisting of F, Cl, Br, I, CN, -OH, C₁₋₄-alkyl, C₃₋₆-cycloalkyl-, HO-C₁₋₄-alkyl, C₁₋₄-alkyl-O-C₁₋₄-alkyl, -NR^{N}H, C₁₋₄-alkyl-NR^{N}-, C₁₋₄-alkyl-O-, C₃₋₆-cycloalkyl-O-, C₁₋₄-alkyl-O-C₁₋₄-alkyl-O-, C₁₋₄-alkyl-S-, C₁₋₄-alkyl-S(=O)-, and C₁₋₄-alkyl-S(=O)₂-,
wherein any alkyl and cycloalkyl group or sub-group within the groups mentioned is optionally substituted with 1 or more F atoms;
R⁵ is selected from a group consisting of Cl, Br, I, C₁₋₄-alkyl-, CN, C₃₋₆-cycloalkyl-, heterocyclyl-C(=O)-, H₂N-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, HO-C(=O)-, C₁₋₄-alkyl-O-C(=O)-, -NHR^{N}, C₁₋₄-alkyl-NR^{N}-, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, -OH, C₁₋₄-alkyl-O-, C₁₋₄-alkyl-O-C₁₋₄-alkyl-O-, C₃₋₆-cycloalkyl-O-, heterocyclyl-O-, phenyl-O-, heteroaryl-O-, C₁₋₄-alkyl-S-, C₃₋₆-cycloalkyl-S-, heterocyclyl-S-, phenyl-S-, heteroaryl-S-, C₁₋₄-alkyl-S(=O)-, C₃₋₆-cycloalkyl-S(=O)-, heterocyclyl-S(=O)-, phenyl-S(=O)-, heteroaryl-S(=O)-, C₁₋₄-alkyl-S(=O)₂-, C₃₋₆-cycloalkyl-S(=O)₂-, heterocyclyl-S(=O)₂-, phenyl-S(=O)₂-, heteroaryl-S(=O)₂-, H₂N-S(=O)₂-, C₁₋₄-alkyl-NR^{N}-S(=O)₂-, heterocyclyl, phenyl, and heteroaryl,
wherein any alkyl, cycloalkyl and heterocyclyl group or sub-group within the groups mentioned is optionally substituted with 1 or more F atoms and optionally substituted with 1 or 2 groups independently selected from H₃C-, HO-, H₃C-O-, and -CN;
wherein heterocyclyl is selected from
a cyclobutyl group wherein 1 CH₂ group is replaced by -NR^{N}- or -O-,
a saturated or partially unsaturated C₅₋₇-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₇-cycloalkyl group wherein 1 CH₂ group is replaced by -NR^{N}- or -O-, a second CH₂ group is replaced by -NR^{N}-, -C(=O)-, -S(=O)- or -S(=O)₂- and/or 1 CH group is replaced by N; and
a saturated or partially unsaturated C₅₋₇-cycloalkyl group wherein 2 CH₂ groups are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from
a tetrazolyl ring, and a 5- or 6-membered heteroaromatic ring which contains 1, 2, or 3 heteroatoms independently of each other selected from =N-, -NH-,-O-, and -S-, wherein in heteroaromatic groups containing a -HC=N- unit this group is optionally replaced by -NR^{N}-C(=O)-, and wherein in heteroaromatic rings with one ore more NH groups each of them is replaced by NR^{N}, and each heteroaryl group is optionally substituted with 1 to 3 substituents independently selected from F, Cl, -CH₃, -CN, and -O-CH₃;
R⁶ is selected from a group consisting of F, Cl, Br, I, CN, C₁₋₄-alkyl, C₃₋₆-cycloalkyl-, HO-C₁₋₄-alkyl-, C₁₋₄-alkyl-O-C₁₋₄-alkyl-, R^{N}HN-, C₁₋₄-alkyl-O-, -S(=O)-C₁₋₄-alkyl, and S(=O)₂-C₁₋₄-alkyl,
wherein any alkyl and cycloalkyl group or sub-group within the groups mentioned is optionally substituted with one or more F atoms;
R^{N} is independently of each other selected from a group consisting of H, C₁₋₄-alkyl, C₁₋₄-alkyl-C(=O)-, C₁₋₄-alkyl-NH-C(=O)-, C₁₋₄-alkyl-N(C₁₋₄-alkyl)-C(=O)-, C₁₋₄-alkyl-O-C(=O)-, and C₁₋₄-alkyl-S(=O)₂-; and
m is 1 or 2;
wherein in any definition mentioned hereinbefore and if not specified otherwise, any alkyl group or sub-group may be straight-chained or branched,
or a salt thereof.

2. A compound according to claim 1, wherein
R¹ is selected from a group consisting of a phenyl ring, a tetrazolyl ring, a 5-membered heteroaromatic ring which contains 2 or 3 heteroatoms independently of each other selected from =N-, -NH-, -O- and -S- with the proviso that not more than one heteroatom is -O- or -S-, and a 6-membered heteroaromatic ring which contains 1 or 2 =N- atoms;
wherein optionally a second ring is annulated to said phenyl ring and 5- and 6-membered heteroaromatic rings, wherein said second ring is 5- or 6-membered,
unsaturated or aromatic and may contain 1 or 2 heteroatoms independently of each other selected from =N-, -NH-, -O- and -S- with the proviso that no O-O, S-S, and SO bond is formed, and wherein in said second ring independently of the presence of heteroatoms 1 or 2 -CH₂- groups may be replaced by -C(=O)- or -S(=O)₂-, and
wherein in said heteroaromatic ring and/or said second ring the H-atom in one or more NH groups, if present, is replaced by R^{N} or R³, and
wherein each of said phenyl ring, tetrazolyl ring, heteroaromatic rings, annulated phenyl ring, and annulated heteroaromatic rings is substituted with one group R³ and optionally additionally substituted with 1 or 2 substituents independently selected from R⁴.
R² is selected from a group consisting ofF, Cl, Br, H₂FC-, F₃C-, NC-, and F₃C-O-;
R³ is selected from a group consisting of
C₁₋₄-alkyl, C₃₋₆-cycloalkyl-, C₁₋₄-alkyl-O-, C₃₋₆-cycloalkyl-O-, C₁₋₄-alkyl-S(=O)-, and C₁₋₄-alkyl-S(=O)₂-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from C₁₋₄-alkyl-C(=O)-, heterocyclyl-C(=O)-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, phenyl-NR^{N}-C(=O)-, heteroaryl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, phenyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, phenyl-S(=O)₂NR^{N}-, heteroaryl-S(=O)₂NR^{N}-, heterocyclyl-O-, phenyl-O-, heteroaryl-O-, C₃₋₆-cycloalkyl-S(=O)-, heterocyclyl-S(=O)-, phenyl-S(=O)-, heteroaryl-S(=O)-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups mentioned is optionally substituted with 1 to 3 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-,
a saturated or mono-unsaturated C₅₋₆-cycloalkyl group wherein 1 CH₂ group is replaced by -C(=O)-, -NH-, -O-, -S(=O)- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)-, -S(=O)- or -S(=O)₂- and/or 1 CH group is replaced by N; and
a saturated or mono-unsaturated C₅₋₆-cycloalkyl group wherein 2 CH₂ groups are replaced by -NH- or 1 CH₂ group by -NH- and the other by -O- and a third CH₂ group is replaced by -C(=O)-, -S(=O)- or -S(=O)₂- and/or 1 CH group by N;
wherein heteroaryl is selected from
a tetrazolyl ring, a pyridin-2-onyl ring, a 5-membered heteroaromatic ring which contains 1, 2, or 3 heteroatoms independently of each other selected from =N-, -NH-, O, and S, and a 6-membered heteroaromatic ring which contains 1 or 2 =N- atom,
and wherein in heteroaryl and heterocyclyl rings with one ore more NH groups each of them is replaced by NR^{N} or NR⁵,
with the proviso that R³ in total cannot be C₁₋₄-alkyl-, C₁₋₄-alkyl-O-C₁₋₄-alkyl, HO-C₁₋₄-alkyl, C₁₋₄-alkyl-O-, C₁₋₄-alkyl-S(=O)-, and C₁₋₄-alkyl-S(=O)₂,
R⁴ is selected from a group consisting of F, Cl, Br, CN, C₁₋₃-alkyl, C₃₋₄-cycloalkyl-, HO-C₁₋₃-alkyl, C₁₋₃-alkyl-O-C₁₋₃-ₐlkyl, -NR^{N}H, C₁₋₄-alkyl-O-, C₃₋₅-cycloalkyl-O-, H₃C-S(=O)-, H₃C-S(=O)₂-, wherein any alkyl and cycloalkyl group is optionally substituted with 1 or more F atoms,
R⁵ is selected from a group consisting of Cl, C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₃₋₆-cycloalkyl-NR^{N}-C(=O)-, heterocyclyl-NR^{N}-C(=O)-, heteroaryl-NR^{N}-C(=O)-, -NH₂, C₁₋₄-alkyl-NR^{N}-, C₁₋₄-alkyl-C(=O)NR^{N}-, C₃₋₆-cycloalkyl-C(=O)NR^{N}-, heterocyclyl-C(=O)NR^{N}-, heteroaryl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃-₆-cycloalkyl-O-, heterocyclyl-O-, phenyl-O-, heteroaryl-O-, C₁₋₄-alkyl-S(=O)-, C₃-₆-cycloalkyl-S(=O)-, C₁₋₄-alkyl-S(=O)₂-, C₃₋₆-cycloalkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, HO-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}-, or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a tetrazolyl ring, a pyridin-2-onyl ring, a 5-membered heteroaromatic ring which contains 1, 2, or 3 heteroatoms independently of each other selected from =N-, -NH-, -O-, and -S-, and a 6-membered heteroaromatic ring which contains 1 or 2 =N- atoms, and wherein in heteroaromatic rings with one ore more NH groups each of them is replaced by NR^{N}, and each heteroaryl group is optionally substituted with 1 to 3 substituents independently selected from F, Cl, -CH₃, -CN, and -O-CH₃,
R⁶ is selected from a group consisting of F, Cl, -CN, C₁₋₃-alkyl, cyclopropyl, HO-C₁₋₃-alkyl-, H₃C-O-C₁₋₃-alkyl-, H₃C-O-, -S(=O)CH₃, and -S(=O)₂-CH₃, wherein any alkyl and cycloalkyl group or sub-group within the groups mentioned is optionally substituted with one or more F atoms,
R^{N} is H, C₁₋₃-alkyl, H₃C-C(=O)-, or C₁₋₃-alkyl-S(=O)₂-,
m is 1, or 2,
or a salt thereof.

3. A compound according to claim 2, wherein
R¹ is selected from a group consisting of a phenyl ring, a tetrazolyl ring, a 5-membered heteroaromatic ring which contains 2 or 3 heteroatoms independently of each other selected from =N-, -NH-, -O- and -S-, and a 6-membered heteroaromatic ring which contains 1 or 2 =N- atoms;
wherein in said 5-membered heteroaromatic ring the H-atom in one or more NH groups is replaced with R^{N} or R³, and
wherein each of said phenyl ring, tetrazolyl ring, and heteroaromatic rings is substituted with one group R³ and optionally additionally substituted with 1 or 2 substituents independently selected from R⁴,
or a salt thereof.

4. A compound according to claim 2, wherein
R¹ is selected from a group consisting of a phenyl ring, a 5-membered heteroaromatic ring which contains 2 or 3 heteroatoms independently of each other selected from =N-, -NH-, -O- and -S-, and a 6-membered heteroaromatic ring which contains 1 or 2 =N- atoms, wherein a second 5- or 6-membered, unsaturated or aromatic ring is annulated to said phenyl ring and 5- and 6-membered heteroaromatic rings, which may contain 1 or 2 heteroatoms independently of each other selected from =N-, -NH-, -O- and -S- with the proviso that no O-O, S-S, and SO bond is formed, and wherein in said second ring 1 or 2 -CH₂- groups may be replaced by -C(=O)- or -S(=O)₂-, and
wherein in said heteroaromatic rings and said second rings the H-atom in one or more NH groups, if present, is replaced by R^{N} or R³, and
wherein each annulated phenyl ring and annulated heteroaromatic ring is substituted with one group R³ and optionally additionally substituted with 1 or 2 substituents independently selected from R⁴,
or a salt thereof.

5. A compound according to claim 1, wherein
R¹ is selected from the group consisting of 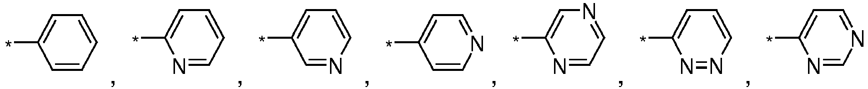 and 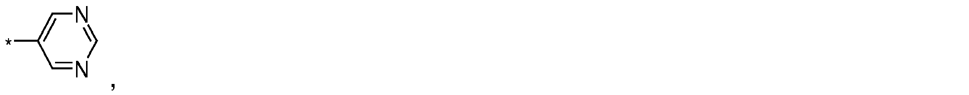 wherein each group is substituted with one group R³ and optionally additionally substituted with 1 or 2 groups independently selected from R⁴;
R² is selected from the group consisting of F, Cl, Br, H₂FC-, F₃C-, NC-, and F₃C-O-;
R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 to 3 groups independently selected from R⁵ and optionally substituted with 1 or more F atoms;
or from H₃C-C(=O)-, heterocyclyl-C(=O)-, H₂N-C(=O)-, C₁₋₃-alkyl-NR^{N}-C(=O)-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, heterocyclyl-O-, phenyl-O-, heteroaryl-O-, heterocyclyl-S(=O)₂-, H₂N-S(=O)₂-, heterocyclyl, phenyl, and heteroaryl,
wherein each alkyl, cycloalkyl, and heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 to 3 substituents independently selected from R⁶;
wherein heterocyclyl is selected from
a cyclobutyl group wherein 1 CH₂ group is replaced by -NH- or -O-;
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -C(=O)- or S(=O)₂-and/or 1 CH group is replaced by N; and
wherein heteroaryl is selected from a pyridin-2-onyl ring, a 5-membered heteroaromatic ring which contains 1, 2, or 3 heteroatoms independently of each other selected from =N-, -NH-, O, and S, and a 6-membered heteroaromatic ring which contains 1 or 2 =N- atoms,
and wherein in heteroaryl and heterocyclyl rings with one ore more NH groups each of them is replaced by NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁴ is selected from the group consisting of F, Cl, CN, -CH₃, -CF₃, isopropyl, cyclopropyl, H₃C-O-CH₂-, H₃C-O-, and F₃C-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃-₆-cycloalkyl-O-, heterocyclyl-O-, C₁₋₄-alkyl-S(=O)-, C₁₋₄-alkyl-S(=O)₂-, heterocyclyl, and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, H₃C-O-, and - CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}-, or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore moreNH groups each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, Cl, -CN, -CH₃, -CF₃, -OCH₃, -S(=O)CH₃, and -S(=O)₂-CH₃;
R^{N} is selected from the group consisting of H, H₃C-, H₃C-C(=O)-, and C₁₋₃-alkyl-S(=O)₂-; and
m is 1 or 2,
or a salt thereof.

6. A compound according to claim 1, wherein
R¹ is 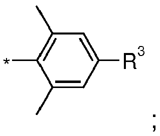 R² is selected from the group consisting of F, Cl, F₃C-, and NC-;
R³ is selected from the group consisting of C₁₋₄-alkyl, C₃₋₆-cycloalkyl-, C₁₋₄-alkyl-O-, and C₃₋₆-cycloalkyl-O-,
wherein each alkyl and cycloalkyl group and each alkyl and cycloalkyl sub-group within the groups mentioned is substituted with 1 group selected from R⁵ and optionally substituted with 1 or 2 H₃C- group;
or from H₃C-C(=O)-, heterocyclyl-C(=O)-, H₂N-C(=O)-, H₃C-NR^{N}-C(=O)-, heterocyclyl-O-, heteroaryl-O-, H₂N-S(=O)₂-, heterocyclyl, phenyl, and heteroaryl,
wherein each heterocyclyl group or sub-group within the groups mentioned is optionally substituted with 1 or 2 groups independently selected from R⁵ and optionally substituted with 1 or more F atoms; and
wherein each phenyl and heteroaryl group is optionally substituted with 1 or 2 substituents independently selected from R⁶;
wherein heterocyclyl is selected from
a cyclobutyl group wherein 1 CH₂ group is replaced by -NH- or -O-;
a saturated or mono-unsaturated C₅₋₆-cycloalkyl group wherein 1 CH₂ group is replaced by -C(=O)-, -NH-, -O- or -S(=O)₂- and/or 1 CH group by N;
a saturated or mono-unsaturated C₅₋₆-cycloalkyl group wherein 1 CH₂ group is replaced by -NH- or -O-, a second CH₂ group is replaced by -NH-, -C(=O)- or - S(=O)₂- and/or 1 CH group is replaced by N; and
wherein heteroaryl is selected from a 5-membered heteroaromatic ring which contains 1 or 2 heteroatoms independently of each other selected from =N-, -NH-, -O-, and -S-, and a 6-membered heteroaromatic ring which contains 1 or 2 =N-atoms, and wherein in heteroaryl and heterocyclyl rings with one ore more NH group each of them is replaced with NR^{N} or NR⁵;
with the proviso that R³ in total cannot be C₁₋₄-alkyl-, C₁₋₄-alkyl-O-C₁₋₄-alkyl, HO-C₁₋₄-alkyl, and C₁₋₄-alkyl-O-;
R⁵ is selected from the group consisting of C₁₋₄-alkyl-, -CN, C₃₋₆-cycloalkyl-, heterocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃-₆-cycloalkyl-O-, heterocyclyl-O-, C₁₋₄-alkyl-S(=O)-, C₁₋₄-alkyl-S(=O)₂-, heterocyclyl,
and heteroaryl,
wherein any alkyl, cycloalkyl, and heterocyclyl group or sub-group within the groups mentioned is optionally substituted with 1 or more F atoms and optionally substituted with 1 or 2 groups independently selected from H₃C-, H₃C-O-, and -CN,
wherein heterocyclyl is selected from
a cyclobutyl group wherein 1 CH₂ group is replaced by -NR^{N}- or -O-;
a saturated or partially unsaturated C₅₋₆-cycloalkyl group wherein 1 CH₂ group is replaced by -C(=O)-, -NR^{N}-, -O-, -S(=O)- or -S(=O)₂- and/or 1 CH group by N;
a saturated or partially unsaturated C₅₋₆-cycloalkyl group wherein 1 CH₂ group is replaced by -NR^{N}-, or -O-, a second CH₂ group is replaced by -NR^{N}-, -C(=O)-, -S(=O)- or -S(=O)₂- and/or 1 CH group is replaced by N; and
a saturated or partially unsaturated C₅₋₆-cycloalkyl group wherein 2 CH₂ group are replaced by -NR^{N}- or 1 CH₂ group by -NR^{N}- and the other by -O-, and a third CH₂ group is replaced by -C(=O)-, -S(=O)- or -S(=O)₂- and/or 1 CH group by N;
and wherein heteroaryl is selected from a pyridin-2-onyl ring, a 5-membered heteroaromatic ring which contains 1 or 2 heteroatoms independently of each other selected from =N-, -NH-, -O-, and -S-, and a 6-membered heteroaromatic ring which contains 1 or 2 =N- atoms, and wherein in heteroaromatic rings with one ore more NH group each of them is replaced by NR^{N}, and each heteroaryl group is optionally substituted with 1 or 2 substituents independently selected from F, Cl, -CH₃, -CN, and -O-CH₃;
R⁶ is selected from the group consisting of F, -CH₃, CN, and -OCH₃;
R^{N} is selected from the group consisting of H, H₃C-, H₃C-C(=O)-, and C₁₋₃-alkyl-S(=O)₂-; and
m is 1 or 2;
or a salt thereof.

7. A compound according to any one of claims 1-6, wherein R² is CF₃ and m is 1, or a salt thereof.

8. A compound according to claim 1, wherein said compound is selected from the group consisting of: 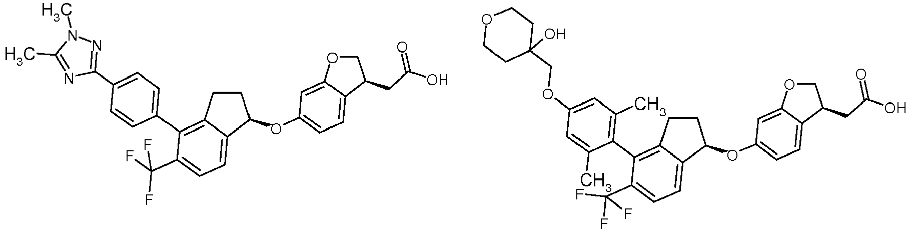 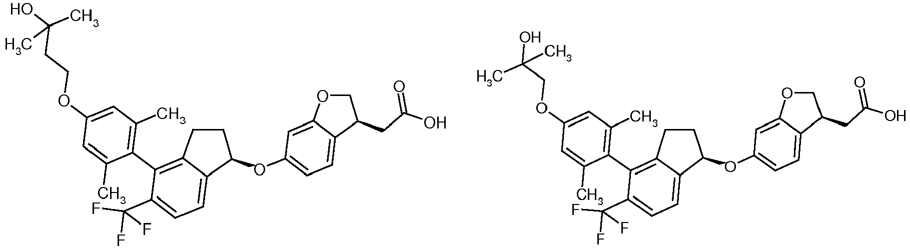 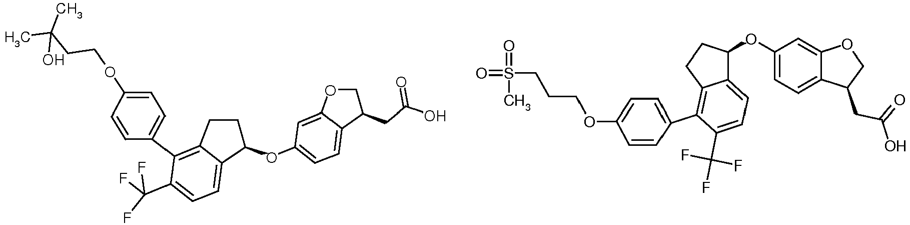 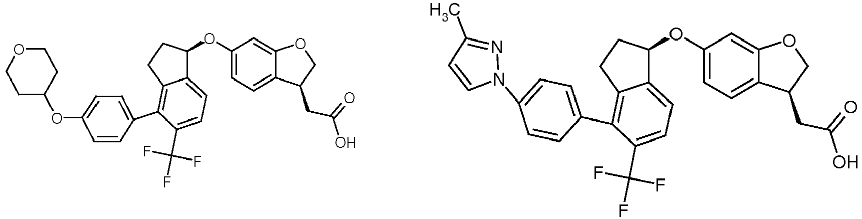 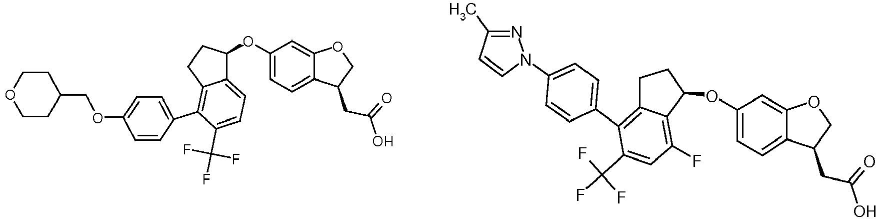 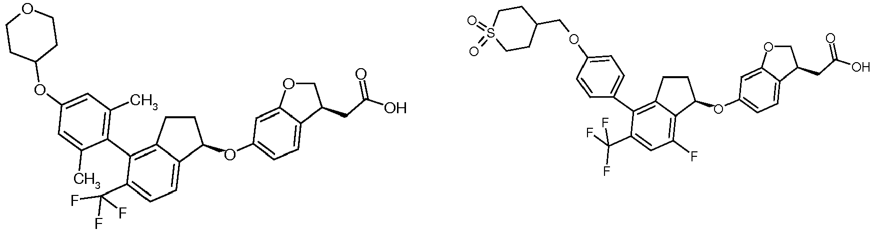 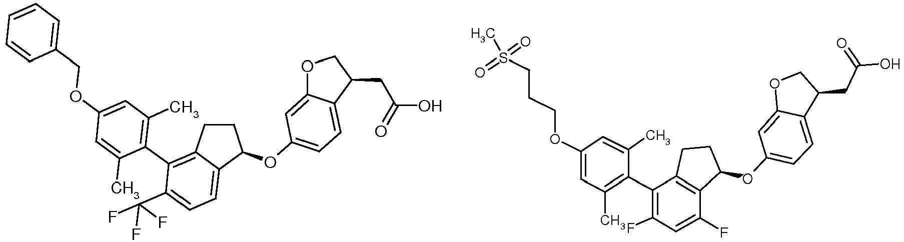 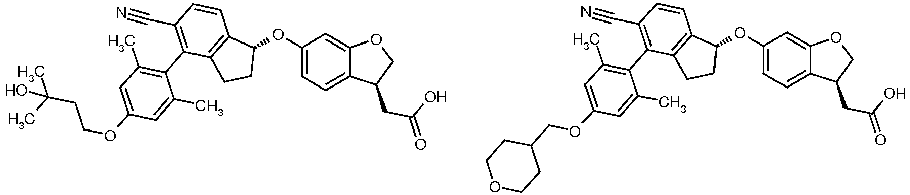 or a salt thereof.

9. A pharmaceutically acceptable salt of a compound according to one or more of the claims 1 to 8.

10. A pharmaceutical composition comprising one or more compounds according to one or more of the claims 1 to 8 or one or more pharmaceutically acceptable salts thereof, optionally together with one or more inert carriers and/or diluents.

11. A compound according to one or more of the claims 1 to 8 or a pharmaceutically acceptable salt thereof for use as a medicament.

12. A compound according to one or more of the claims 1 to 8 or a pharmaceutically acceptable salt thereof for use in the prophylaxis and/or therapy of metabolic diseases and conditions associated with the disease

13. A compound for use according to claim 12, wherein the metabolic diseases or conditions associated with the disease are selected from diabetes, insulin resistance, obesity, cardiovascular disease and dyslipidemia.

14. A compound for use according to claim 12, wherein the metabolic disease is type 2 diabetes mellitus.

15. A pharmaceutical composition comprising one or more compounds according to one or more of the claims 1 to 8 or one or more pharmaceutically acceptable salts thereof and one or more additional therapeutic agents, optionally together with one or more inert carriers and/or diluents.

16. A pharmaceutical composition according to claim 15 wherein the additional therapeutic agents are selected from the group consisting of antidiabetic agents, agents for the treatment of overweight and/or obesity and agents for the treatment of high blood pressure, heart failure and/or atherosclerosis

## Patentansprüche

1. Verbindung der Formel (I) 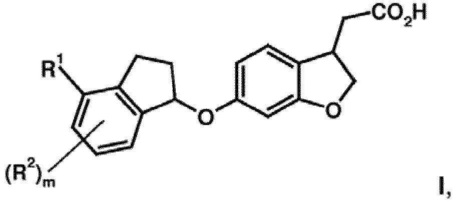 wobei
R¹ ausgewählt ist aus einer Gruppe, bestehend aus einem Phenylring, einem Tetrazolylring und einem 5- oder 6-gliedrigen heteroaromatischen Ring, der 1, 2 oder 3 Heteroatome enthält, unabhängig voneinander ausgewählt aus =N-, -NH-, -O- und -S-;
wobei gegebenenfalls ein zweiter Ring mit dem Phenyl- oder heteroaromatischen Ring kondensiert ist,
wobei der zweite Ring 5- oder 6-gliedrig ist, ungesättigt oder aromatisch ist und 1, 2 oder 3 Heteroatome enthalten kann, unabhängig voneinander ausgewählt aus =N-, -NH-, -O- und -S-, mit der Maßgabe, dass nur bis zu zwei der Heteroatome O und S sind und keine O-O-, S-S-, und S-O-Bindung gebildet wird, und wobei in dem zweiten Ring unabhängig von der Gegenwart von Heteroatomen 1 oder 2 CH₂-Gruppen ersetzt sein können durch -C(=O)-, -S(=O)- oder -S(=O)₂-, und
wobei der Phenylring, Tetrazolylring, heteroaromatische Ring, kondensierte Phenylring und kondensierte heteroaromatische Ring mit einer Gruppe R³ substituiert ist; und
wobei jeder Phenylring, Tetrazolylring, heteroaromatische Ring, kondensierte Phenyl-ring und kondensierte heteroaromatische Ring gegebenenfalls zusätzlich mit 1 bis 4 Gruppen substituiert ist, unabhängig ausgewählt aus R⁴; und
wobei in dem heteroaromatischen Ring und/oder dem zweiten Ring das H-Atom in einer oder mehreren NH-Gruppen, wenn vorhanden, durch R^{N} oder R³ ersetzt ist;
R² ist ausgewählt aus einer Gruppe, bestehend aus F, Cl, Br, H₂FC-, F₃C-, NC- und F₃C-O-;
R³ ist ausgewählt aus einer Gruppe, bestehend aus
C₁₋₆-Alkyl, C₂₋₆-Alkenyl, C₂₋₆-Alkinyl, C₃₋₆-Cycloalkyl-, C₁₋₄-Alkyl-NH-, (C₁₋₄-Alkyl)₂N-, C₁₋₆-Alkyl-O-, C₃₋₆-Cycloalkyl-O-, C₁₋₄-Alkyl-S-, C₁₋₄-Alkyl-S(=O)- und C₁₋₄-Alkyl-S(=O)₂,
wobei jede Alkyl- und Cycloalkylgruppe und jede Alkyl- und Cycloalkyluntergruppe in den erwähnten Gruppen mit 1 bis 3 Gruppen substituiert ist, unabhängig ausgewählt aus R⁵, und gegebenenfalls mit 1 oder mehr F-Atomen substituiert ist;
oder aus C₁₋₄-Alkyl-C(=O)-, Heterocyclyl-C(=O)-, HNR^{N}-C(=O)-, C₁₋₄-Alkyl-NR^{N}-C(=O)-, C₃₋₆-Cycloalkyl-NR^{N}-C(=O)-, Heterocyclyl-NR^{N}-C(=O)-, Phenyl-NR^{N}-C(=O)-, Heteroaryl-NR^{N}-C(=O)-, HO₂C-, C₁₋₄-Alkyl-O-C(=O)-, C₃₋₆-Cycloalkyl-O-C(=O)-, Heterocyclyl-O-C(=O)-, -NHR^{N}, C₁₋₄-Alkyl-C(=O)NR^{N}-, C₃₋₆-Cycloalkyl-C(=O)NR^{N}-, Heterocyclyl-C(=O)NR^{N}-, Phenyl-C(=O)NR^{N}-, Heteroaryl-C(=O)NR^{N}-, C₁₋₄-Alkyl-S(=)₂ONR^{N}-, C₃₋₆-Cycloalkyl-S(=O)₂NR^{N}-, Heterocyclyl-S(=O)₂NR^{N}-, Phenyl-S(=O)₂NR^{N}-, Heteroaryl-S(=O)₂NR^{N}-, Heterocyclyl-O-, Phenyl-O-, Heteroaryl-O-, C₃₋₆-Cycloalkyl-S-, Heterocyclyl-S-, Phenyl-S-, Heteroaryl-S-, C₃₋₆-Cycloalkyl-S(=O)-, Heterocyclyl-S(=O)-, Phenyl-S(=O)-, Heteroaryl-S(=O)-, C₃₋₆-Cycloalkyl-S(=O)₂-, Heterocyclyl-S(=O)₂-, Phenyl-S(=O)₂-, Heteroaryl-S(=O)₂-, HNR^{N}-S(=O)2-, C₁₋₄-Alkyl-NR^{N}-S(=O)₂-, Heterocyclyl, Phenyl und Heteroaryl,
wobei jede Alkyl-, Cycloalkyl- und Heterocyclylgruppe oder -untergruppe in den genannten Gruppen gegebenenfalls mit 1 bis 3 Gruppen substituiert ist, unabhängig ausgewählt aus R⁵, und gegebenenfalls mit 1 oder mehr F-Atomen substituiert ist; und
wobei jede Phenyl- und Heteroarylgruppe gegebenenfalls mit 1 bis 5 Substituenten substituiert ist, unabhängig ausgewählt aus R⁶;
wobei Heterocyclyl ausgewählt ist aus
einer Cyclobutylgruppe, wobei 1 CH₂-Gruppe durch -NH- oder -O- ersetzt ist, einer gesättigten oder teilweise ungesättigten C₅₋₇-Cycloalkylgruppe, wobei 1 CH₂-Gruppe durch -C(=O)-, -NH-, -O-, -S(=O)- oder -S(=O)₂- ersetzt ist und/oder 1 CH-Gruppe durch N ersetzt ist;
einer gesättigten oder teilweise ungesättigten C₅₋₇-Cycloalkylgruppe, wobei 1 CH₂-Gruppe durch -NH- oder -O- ersetzt ist, eine zweite CH₂-Gruppe durch -NH-, -C(=O)-, -S(=O)- oder -S(=O)₂- ersetzt ist und/oder 1 CH-Gruppe durch N ersetzt ist; und
einer gesättigten oder teilweise ungesättigten C₅₋₇-Cycloalkylgruppe, wobei 2 CH₂ Gruppen durch -NH- ersetzt sind oder 1 CH₂-Gruppe durch -NH- und die andere durch -O- ersetzt ist und eine dritte CH₂-Gruppe durch -C(=O)-, -S(=O)-oder -S(=O)₂- ersetzt ist und/oder 1 CH-Gruppe durch N ersetzt ist;
wobei Heteroaryl ausgewählt ist aus
einem Tetrazolyhing und einem 5- oder 6-gliedrigen heteroaromatischen Ring, der 1, 2 oder 3 Heteroatome enthält, unabhängig voneinander ausgewählt aus =N-, -NH-, -O- und -S-, wobei in heteroaromatischen Gruppen, die eine -HC=N-Einheit enthalten, diese Gruppe gegebenenfalls durch -NH-C(=O)- ersetzt ist;
wobei in Heteroaryl- und Heterocyclylringen mit ein oder mehreren NH-Gruppen jede von diesen durch NR^{N} oder NR⁵ ersetzt ist;
mit der Maßgabe, dass R³ insgesamt nicht C₁₋₄-Alkyl-, C₁₋₄-Alkyl-O-C₁₋₄-alkyl, HO-C₁₋₄-Alkyl, C₃₋₄-Alkenyl, C₃₋₄-Alkinyl, H₂N-, C₁₋₄-Alkyl-NH-, (C₁₋₄-Alkyl)₂N-, C₁₋₄-Alkyl-O-, C₁₋₄-Alk_{yl}-S-, C₁₋₄-Alkyl1-S(=O)- und C₁₋₄-Alkyl-S(=O)₂ sein kann;
R⁴ ist ausgewählt aus einer Gruppe, bestehend aus F, Cl, Br, I, CN, -OH, C₁₋₄-Alkyl, C₃₋₆-Cycloalkyl-, HO-C₁₋₄-Alkyl, C₁₋₄-Alkyl-O-C₁₋₄-alkyl, -NR^{N}H, C₁₋₄-Alkyl-NR^{N}-, C₁₋₄-Alkyl-O-, C₃₋₆-Cycloalkyl-O-, C₁₋₄-Alkyl-O-C₁₋₄-alkyl-O-, C₁₋₄-Alkyl-S-, C₁₋₄-Alkyl-S(=O)- und C₁₋₄-Alkyl-S(=O)₂-,
wobei jede Alkyl- und Cycloalkylgruppe oder -untergruppe in den genannten Gruppen gegebenenfalls mit 1 oder mehr F-Atomen substituiert ist;
R⁵ ist ausgewählt aus einer Gruppe, bestehend aus Cl, Br, I, C₁₋₄-Alkyl-, CN, C₃₋₆-Cycloalkyl-, Heterocyclyl-C(=O)-, H₂N-C(=O)-, C₁₋₄-Alkyl-NR^{N}-C(=O)-, C₃₋₆-Cycloalkyl-NR^{N}-C(=O)-, Heterocyclyl-NR^{N}-C(=O)-, Phenyl-NR^{N}-C(=O)-, Heteroaryl-NR^{N}-C(=O)-, HO-C(=O)-, C₁₋₄-Alkyl-O-C(=O)-, -NHR^{N}, C₁₋₄-Alkyl-NR^{N}-, C₁₋₄-Alkyl-C(=O)NR^{N}-, C₃₋₆-Cycloalkyl-C(=O)NR^{N}-, Heterocyclyl-C(=O)NR^{N}-, Phenyl-C(=O)NR^{N}-, Heteroaryl-C(=O)NR^{N}-, C₁₋₄-Alkyl-S(=O)₂NR^{N}-, C₃₋₆-Cycloalkyl-S(=O)₂NR^{N}-, Heterocyclyl-S(=0)₂NR^{N}-, Phenyl-S(=O)₂NR^{N}-, Heteroaryl-S(=O)₂NR^{N}-, -OH, C₁₋₄-Alkyl-O-, C₁₋₄-Alkyl-O-C₁₋₄-alkyl-O-, C₃₋₆-Cycloalkyl-O-, Heterocyclyl-O-, Phenyl-O-, Heteroaryl-O-, C₁₋₄-Alkyl-S-, C₃₋₆-Cycloalkyl-S-, Heterocyclyl-S-, Phenyl-S-, Heteroaryl-S-, C₁₋₄-Alkyl-S(=O)-, C₃₋₆-Cycloalkyl-S(=O)-, Heterocyclyl-S(=O)-, Phenyl-S(=O)-, Heteroaryl-S(=O)-, C₁₋₄-Alkyl-S(=O)₂-, C₃₋₆-Cycloalkyl-S(=O)₂-, Heterocyclyl-S(=O)₂-, Phenyl-S(=O)₂-, Heteroaryl-S(=O)₂-, H₂N-S(=O)₂-, C₁₋₄-Alkyl-NR-S(=O)₂-, Heterocyclyl, Phenyl und Heteroaryl,
wobei jede Alkyl-, Cycloalkyl- und Heterocyclylgruppe oder -untergruppe in den genannten Gruppen gegebenenfalls mit 1 oder mehr F-Atomen substituiert ist und gegebenenfalls mit 1 oder 2 Gruppen substituiert ist, unabhängig ausgewählt aus H₃C-, HO-, H₃C-O- und -CN;
wobei Heterocyclyl ausgewählt ist aus
einer Cyclobutylgruppe, wobei 1 CH₂-Gruppe durch -NR^{N}- oder -O- ersetzt ist, einer gesättigten oder teilweise ungesättigten C₅₋₇-Cycloalkylgruppe, wobei 1 CH₂-Gruppe durch -C(=O)-, -NR^{N}-, -O-, -S(=O)- oder -S(=O)₂- ersetzt ist und/oder 1 CH-Gruppe durch N ersetzt ist;
einer gesättigten oder teilweise ungesättigten C₅₋₇-Cycloalkylgruppe, wobei 1 CH₂-Gruppe durch -NR^{N}- oder -O- ersetzt ist, eine zweite CH₂-Gruppe durch -NR^{N}-, -C(=O)-, -S(=O)- oder -S(=O)₂- ersetzt ist und/oder 1 CH-Gruppe durch N ersetzt ist; und
einer gesättigten oder teilweise ungesättigten C₅₋₇-Cycloalkylgruppe, wobei 2 CH₂ Gruppen durch -NR^{N}- ersetzt sind oder 1 CH₂-Gruppe durch -NR^{N}- und die andere durch -O- ersetzt ist, und eine dritte CH₂-Gruppe durch -C(=O)-, -S(=O)- oder -S(=O)₂- ersetzt ist und/oder 1 CH-Gruppe durch N ersetzt ist;
und wobei Heteroaryl ausgewählt ist aus
einem Tetrazolylring und einem 5- oder 6-gliedrige heteroaromatischen Ring, der 1, 2 oder 3 Heteroatome enthält, unabhängig voneinander ausgewählt aus =N-, -NH-, -O- und -S-, wobei in heteroaromatischen Gruppen, die eine -HC=N-Einheit enthalten, diese Gruppe gegebenenfalls durch -NR^{N}-C(=O)- ersetzt ist, und wobei in heteroaromatischen Ringen mit ein oder mehr NH-Gruppen jede von diesen durch NR^{N} ersetzt ist, und jede Heteroarylgruppe gegebenenfalls mit 1 bis 3 Substituenten substituiert ist, unabhängig ausgewählt aus F, Cl, -CH₃, -CN und -O-CH₃;
R⁶ ist ausgewählt aus einer Gruppe, bestehend aus F, Cl, Br, I, CN, C₁₋₄-Alkyl, C₃₋₆-Cycloalkyl-, HO-C₁₋₄-Alkyl-, C₁₋₄-Alkyl-O-C₁₋₄-alkyl-, R^{N}HN-, C₁₋₄-Alkyl-O-, S(=O)- C₁₋₄-Alkyl und S(=O)₂-C₁₋₄-Alkyl,
wobei jede Alkyl- und Cycloalkylgruppe oder -untergruppe in den genannten Gruppen gegebenenfalls mit ein oder mehr F-Atomen substituiert ist;
R^{N} ist unabhängig voneinander ausgewählt aus einer Gruppe, bestehend aus H, C₁₋₄-Alkyl, C₁₋₄-Alkyl-C(=O)-, C₁₋₄-Alkyl-NH-C(=O)-, C₁₋₄-Alkyl-N(C₁₋₄-alkyl)-C(=O)-, C₁₋₄-Alkyl-O-C(=O)- und C₁₋₄-Alkyl-S(=O)₂-; und
m ist 1 oder 2;
wobei in jeder hier zuvor genannten Definition und sofern nicht anders angegeben, jede Alkylgruppe oder -untergruppe geradkettig oder verzweigt sein kann,
oder ein Salz hiervon.

2. Verbindung nach Anspruch 1, wobei
R¹ ausgewählt ist aus einer Gruppe, bestehend aus einem Phenylring, einem Tetrazolylring, einem 5-gliedrigen heteroaromatischen Ring, der 2 oder 3 Heteroatome enthält, unabhängig voneinander ausgewählt aus =N-, -NH-, -O- und -S-, mit der Maßgabe, dass nicht mehr als ein Heteroatom -O- oder -S- darstellt, und einem 6-gliedrigen heteroaromatischen Ring, der 1 oder 2 =N-Atome enthält;
wobei gegebenenfalls ein zweiter Ring mit dem Phenylring und den 5- und 6-gliedrigen heteroaromatischen Ringen kondensiert ist, wobei der zweite Ring 5- oder 6-gliedrig ist, ungesättigt oder aromatisch ist und 1 oder 2 Heteroatome enthalten kann, unabhängig voneinander ausgewählt aus =N-, -NH-, -O- und -S-, mit der Maßgabe, dass keine O-O-, S-S- und S-O-Bindung gebildet wird, und wobei in dem zweiten Ring, unabhängig von der Gegenwart von Heteroatomen, 1 oder 2 -CH₂-Gruppen durch -C(=O)- oder -S(=O)₂- ersetzt sein können, und
wobei in dem heteroaromatischen Ring und/oder dem zweiten Ring das H-Atom in ein oder mehreren NH-Gruppen, sofern vorhanden, durch R^{N} oder R³ ersetzt ist, und
wobei jeder Phenylring, Tetrazolylring, heteroaromatische Ring, kondensierte Phenylring und kondensierte heteroaromatische Ring mit einer Gruppe R³ substituiert ist, und gegebenenfalls zusätzlich mit 1 oder 2 Substituenten substituiert ist, unabhängig ausgewählt aus R⁴;
R² ist ausgewählt aus einer Gruppe, bestehend aus F, Cl, Br, H₂FC-, F₃C-, NC- und F₃C-O-;
R³ ist ausgewählt aus einer Gruppe, bestehend aus
C₁₋₄-Alkyl, C₃₋₆-Cycloalkyl-, C₁₋₄-Alkyl-O-, C₃₋₆-Cycloalkyl-O-, C₁₋₄-Alkyl-S(=O)- und C₁-₄-Alkyl-S(=O)₂-,
wobei jede Alkyl- und Cycloalkylgruppe und jede Alkyl- und Cycloalkyluntergruppe in den genannten Gruppen mit 1 bis 3 Gruppen substituiert ist, unabhängig ausgewählt aus R⁵ und gegebenenfalls mit 1 oder mehr F-Atomen substituiert ist;
oder aus C₁₋₄-Alkyl-C(=O)-, Heterocyclyl-C(=O)-, HNR^{N}-C(=O)-, C₁₋₄-Alkyl-NR^{N}-C(=O)-, C₃₋₆-Cycloalkyl-NR^{N}-C(=O)-, Heterocyclyl-NR^{N}-C(=O)-, Phenyl-NR^{N}-C(=O)-, Heteroaryl-NR^{N}-C(=O)-, C₁₋₄-Alkyl-C(=O)NR^{N}-, C₃₋₆-Cycloalkyl-C(=O)NR^{N}-, Heterocyclyl-C(=O)NR^{N}-, Phenyl-C(=O)NR^{N}-, Heteroaryl-C(=O)NR^{N}-, C₁₋₄-Alkyl-S(=O)₂NR^{N}-, C₃₋₆-Cycloalkyl-S(=O)₂NR^{N}-, Heterocyclyl-S(=O)₂NR^{N}-, Phenyl-S(=O)₂NR^{N}-, Heteroaryl-S(=O)₂NR^{N}-, Heterocyclyl-O-, Phenyl-O-, Heteroaryl-O-, C₃₋₆-Cycloalkyl-S(=O)-, Heterocyclyl-S(=O)-, Phenyl-S(=O)-, Heteroaryl-S(=O)-, C₃₋₆-Cycloalkyl-S(=O)₂-, Heterocyclyl-S(=O)₂-, Phenyl-S(=O)₂-, Heteroaryl-S(=O)₂-, HNR^{N}-S(=O)₂-, C₁₋₄-Alkyl-NR^{N}-S(=O)₂-, Heterocyclyl, Phenyl und Heteroaryl,
wobei jede Alkyl-, Cycloalkyl- und Heterocyclylgruppe oder -untergruppe in den genannten Gruppen gegebenenfalls mit 1 bis 3 Gruppen substituiert ist, unabhängig ausgewählt aus R⁵ und gegebenenfalls mit 1 oder mehr F-Atomen substituiert ist; und
wobei jede Phenyl- und Heteroarylgruppe gegebenenfalls mit 1 bis 3 Substituenten substituiert ist, unabhängig ausgewählt aus R⁶;
wobei Heterocyclyl ausgewählt ist aus
einer Cyclobutylgruppe, wobei 1 CH₂-Gruppe durch -NH- oder -O- ersetzt ist,
einer gesättigten oder einfach-ungesättigten C₅₋₆-Cycloalkylgruppe, wobei 1 CH₂-Gruppe durch -C(=O)-, -NH-, -O-, -S(=O)- oder -S(=O)₂- ersetzt ist und/oder 1 CH-Gruppe durch N ersetzt ist;
einer gesättigten oder einfach-ungesättigten C₅₋₆-Cycloalkylgruppe, wobei 1 CH₂-Gruppe durch -NH- oder -O- ersetzt ist, eine zweite CH₂-Gruppe durch -NH-, -S(=O)- oder -S(=O)₂- ersetzt ist und/oder 1 CH-Gruppe durch N ersetzt ist; und
einer gesättigten oder einfach-ungesättigten C₅₋₆-Cycloalkylgruppe, wobei 2 CH₂-Gruppen durch -NH- ersetzt sind oder 1 CH₂-Gruppe durch -NH- ersetzt ist und die andere durch -O- und eine dritte CH₂-Gruppe durch -C(=O)-, -S(=O)- oder -S(=O)₂- ersetzt ist und/oder 1 CH-Gruppe durch N ersetzt ist;
wobei Heteroaryl ausgewählt ist aus
einem Tetrazolylring, einem Pyridin-2-onylring, einem 5-gliedrigen heteroaromatischen Ring, der 1, 2 oder 3 Heteroatome enthält, unabhängig voneinander ausgewählt aus =N-, -NH-, O und S, und einem 6-gliedrigen heteroaromatischen Ring, der 1 oder 2 =N-Atome enthält,
und wobei in Heteroaryl- und Heterocyclylringen mit ein oder mehreren NH-Gruppen jede von diesen durch NR^{N} oder NR⁵ ersetzt ist,
mit der Maßgabe, dass R³ insgesamt nicht C₁₋₄-Alkyl-, C₁-₄-Alkyl-O-C₁₋₄-alkyl, HO-C₁₋₄-Alkyl, C₁₋₄-Alkyl-O-, C₁₋₄-Alkyl-S(=O)- und C₁₋₄-Alkyl-S(=O)₂ darstellt,
R⁴ ist ausgewählt aus einer Gruppe, bestehend aus F, Cl, Br, CN, C₁₋₃-Alkyl, C₃₋₄-Cycloalkyl-, HO-C₁₋₃-Alkyl, C₁₋₃-Alkyl-O-C₁₋₃-alkyl, -NR^{N}H, C₁₋₄-Alkyl-O-, C₃₋₅-Cycloalkyl-O-, H₃C-S(=O)-, H₃C-S(=O)₂-, wobei jede Alkyl- und Cycloalkylgruppe gegebenenfalls mit 1 oder mehr F-Atomen substituiert ist,
R⁵ ist ausgewählt aus einer Gruppe, bestehend aus Cl, C₁₋₄-Alkyl-, -CN, C₃₋₆-Cycloalkyl-, Heterocyclyl-C(=O)-, H₂N-C(=O)-, C₁₋₄-Alkyl-NR-C(=O)-, C₃₋₆-Cycloalkyl-NR^{N}-C(=O)-, Heterocyclyl-NR^{N}-C(=O)-, Heteroaryl-NR^{N}-C(=O)-, -NH₂, C₁₋₄-Alkyl-NR^{N}-, C₁₋₄-Alkyl-C(=O)NR^{N}-, C₃₋₆-Cycloalkyl-C(=O)NR^{N}-, Heterocyclyl-C(=O)NR^{N}-, Heteroaryl-C(=O)NR^{N}-, C₁₋₄-Alkyl-S(=O)₂NR^{N}-, -OH, C₁₋₄-Alkyl-O-, C₁₋₄-Alkyl-O-C₁₋₄-alkyl-O-, C₃₋₆-Cycloalkyl-O-, Heterocyclyl-O-, Phenyl-O-, Heteroaryl-O-, C₁₋₄-Alkyl-S(=O)-, C₃₋₆-Cycloalkyl-S(=O)-, C₁₋₄-Alkyl-S(=O)₂-, C₃₋₆-Cycloalkyl-S(=O)₂-, Heterocyclyl und Heteroaryl,
wobei jede Alkyl-, Cycloalkyl- und Heterocyclylgruppe oder -untergruppe in den genannten Gruppen gegebenenfalls mit 1 oder mehr F-Atomen substituiert ist und gegebenenfalls mit 1 oder 2 Gruppen substituiert ist; unabhängig ausgewählt aus H₃C-, HO-, H₃C-O-und -CN,
wobei Heterocyclyl ausgewählt ist aus
einer Cyclobutylgruppe, wobei 1 CH₂-Gruppe durch -NR^{N}- oder -O- ersetzt ist;
einer gesättigten oder teilweise ungesättigten C₅₋₆-Cycloalkylgruppe, wobei 1 CH₂-Gruppe durch -C(=O)-, -NR^{N}-, -O-, -S(=O)- oder -S(=O)₂- ersetzt ist und/oder 1 CH-Gruppe durch N ersetzt ist;
einer gesättigten oder teilweise ungesättigten C₅₋₆-Cycloalkylgruppe, wobei 1 CH₂-Gruppe durch -NR^{N}- oder -O- ersetzt ist, eine zweite CH₂-Gruppe durch -NR^{N}-, -C(=O)-, -S(=O)- oder -S(=O)₂- ersetzt ist und/oder 1 CH-Gruppe durch N ersetzt ist; und
einer gesättigten oder teilweise ungesättigten C₅₋₆-Cycloalkylgruppe, wobei 2 CH₂-Gruppen durch -NR^{N}- ersetzt sind oder 1 CH₂-Gruppe durch -NR^{N}- ersetzt ist und die andere durch -O-, und eine dritte CH₂-Gruppe durch -C(=O)-, -S(=O)- oder -S(=O)₂-ersetzt ist und/oder 1 CH-Gruppe durch N ersetzt ist;
und wobei Heteroaryl ausgewählt ist aus einem Tetrazolylring, einem Pyridin-2-onylring, einem 5-gliedrigen heteroaromatischen Ring, der 1, 2 oder 3 Heteroatome enthält, unabhängig voneinander ausgewählt aus =N-, -NH-, -O- und -S-, und einem 6-gliedrigen heteroaromatischen Ring, der 1 oder 2 =N-Atome enthält, und wobei in heteroaromatischen Ringen mit ein oder mehreren NH-Gruppen jede von diesen durch NR^{N} ersetzt ist, und jede Heteroarylgruppe ist gegebenenfalls mit 1 bis 3 Substituenten substituiert, unabhängig ausgewählt aus F, Cl, -CH₃, -CN und -O-CH₃,
R⁶ ist ausgewählt aus einer Gruppe, bestehend aus F, Cl, -CN, C₁₋₃-Alkyl, Cyclopropyl, HO-C₁₋₃-Alkyl-, H₃C-O-C₁₋₃-Alkyl-, H₃C-O-, -S(=O)CH₃ und -S(=O)₂-CH₃, wobei jede Alkyl- und Cycloalkylgruppe oder -untergruppe in den genannten Gruppen gegebenenfalls mit einem oder mehreren F-Atomen substituiert ist,
R^{N} ist H, C₁₋₃-Alkyl, H₃C-C(=O)- oder C₁₋₃-Alkyl-S(=O)₂-,
m ist 1 oder 2,
oder ein Salz hiervon.

3. Verbindung nach Anspruch 2, wobei
R¹ ausgewählt ist aus einer Gruppe, bestehend aus einem Phenylring, einem Tetrazolylring, einem 5-gliedrigen heteroaromatischen Ring, der 2 oder 3 Heteroatome enthält, unabhängig voneinander ausgewählt aus =N-, -NH-, -O- und -S-, und einem 6-gliedrigen heteroaromatischen Ring, der 1 oder 2 =N-Atome enthält;
wobei in dem 5-gliedrigen heteroaromatischen Ring das H-Atom in einer oder mehreren NH-Gruppen durch R^{N} oder R³ ersetzt ist, und
wobei jeder Phenylring, Tetrazolylring und heteroaromatische Ring mit einer Gruppe R³ substituiert ist und gegebenenfalls zusätzlich mit 1 oder 2 Substituenten substituiert ist, unabhängig ausgewählt aus R⁴,
oder ein Salz hiervon.

4. Verbindung nach Anspruch 2, wobei
R¹ ausgewählt ist aus einer Gruppe, bestehend aus einem Phenylring, einem 5-gliedrigen heteroaromatischen Ring, der 2 oder 3 Heteroatome enthält, unabhängig voneinander ausgewählt aus =N-, -NH-, -O- und -S-, und einem 6-gliedrigen heteroaromatischen Ring, der 1 oder 2 =N-Atome enthält, wobei ein zweiter 5- oder 6-gliedriger, ungesättigter oder aromatischer Ring an den Phenylring und den 5- und 6-gliedrigen heteroaromatischen Ring kondensiert ist, der 1 oder 2 Heteroatome enthalten kann, unabhängig voneinander ausgewählt aus =N-, -NH-, -O- und -S-, mit der Maßgabe, dass keine O-O-, S-S- und S-O-Bindung gebildet wird, und wobei in dem zweiten Ring 1 oder 2 -CH₂-Gruppen durch -C(=O)- oder -S(=O)₂-ersetzt sein können, und
wobei in den heteroaromatischen Ringen und den zweiten Ringen das H-Atom in einer oder mehreren NH-Gruppen, sofern vorhanden, durch R^{N} oder R³ ersetzt ist, und
wobei jeder kondensierte Phenylring und kondensierte heteroaromatische Ring mit einer Gruppe R³ substituiert ist und gegebenenfalls zusätzlich mit 1 oder 2 Substituenten substituiert ist, unabhängig ausgewählt aus R⁴,
oder ein Salz hiervon.

5. Verbindung nach Anspruch 1, wobei
R¹ ausgewählt ist aus der Gruppe, bestehend aus 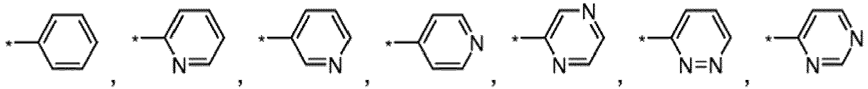 und 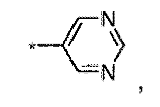 wobei jede Gruppe mit einer Gruppe R³ substituiert ist und gegebenenfalls zusätzlich mit 1 oder 2 Gruppen substituiert ist, unabhängig ausgewählt aus R⁴;
R² ist ausgewählt aus der Gruppe, bestehend aus F, Cl, Br, H₂FC-, F₃C-, NC- und F₃C-O-;
R³ ist ausgewählt aus der Gruppe, bestehend aus C₁₋₄-Alkyl, C₃₋₆-Cycloalkyl-, C₁₋₄-Alkyl-O- und C₃₋₆-Cycloalkyl-O-,
wobei jede Alkyl- und Cycloalkylgruppe und jede Alkyl- und Cycloalkyluntergruppe in den genannten Gruppen mit 1 bis 3 Gruppen substituiert ist, unabhängig ausgewählt aus R⁵ und gegebenenfalls mit 1 oder mehr F-Atomen substituiert ist;
oder aus H₃C-C(=O)-, Heterocyclyl-C(=O)-, H₂N-C(=O)-, C₁₋₃-Alkyl-NR^{N}-C(=O)-, C₁₋₄-Alkyl-C(=O)NR^{N}-, C₃₋₆-Cycloalkyl-C(=O)NR^{N}-, Heterocyclyl-C(=O)NR^{N}-, C₁₋₄-Alkyl-S(=O)₂NR^{N}-, Heterocyclyl-O-, Phenyl-O-, Heteroaryl-O-, Heterocyclyl-S(=O)₂-, H₂N-S(=O)₂-, Heterocyclyl, Phenyl und Heteroaryl,
wobei jede Alkyl-, Cycloalkyl- und Heterocyclylgruppe oder -untergruppe in den genannten Gruppen gegebenenfalls mit 1 oder 2 Gruppen substituiert ist, unabhängig ausgewählt aus R⁵ und gegebenenfalls mit 1 oder mehr F-Atomen substituiert ist; und
wobei jede Phenyl- und Heteroarylgruppe gegebenenfalls mit 1 bis 3 Substituenten substituiert ist, unabhängig ausgewählt aus R⁶;
wobei Heterocyclyl ausgewählt ist aus
einer Cyclobutylgruppe, wobei 1 CH₂-Gruppe durch -NH- oder -O- ersetzt ist;
einer gesättigten oder einfach-ungesättigten C₅₋₆-Cycloalkylgruppe, wobei 1 CH₂-Gruppe durch -C(=O)-, -NH-, -O- oder -S(=O)₂- ersetzt ist und/oder 1 CH-Gruppe durch N ersetzt ist;
einer gesättigten oder einfach-ungesättigten C₅₋₆-Cycloalkylgruppe, wobei 1 CH₂-Gruppe durch -NH- oder -O- ersetzt ist, eine zweite CH₂-Gruppe durch -C(=O)- oder - S(=O)₂- ersetzt ist und/oder 1 CH-Gruppe durch N ersetzt ist; und
wobei Heteroaryl ausgewählt ist aus einem Pyridin-2-onylring, einem 5-gliedrigen heteroaromatischen Ring, der 1, 2 oder 3 Heteroatome enthält, unabhängig voneinander ausgewählt aus =N-, -NH-, O und S, und einem 6-gliedrigen heteroaromatischen Ring, der 1 oder 2 =N-Atome enthält,
und wobei in Heteroaryl- und Heterocyclylringen mit ein oder mehreren NH-Gruppen jede von diesen durch NR^{N} oder NR⁵ ersetzt ist;
mit der Maßgabe, dass R³ insgesamt nicht C₁₋₄-Alkyl-, C₁₋₄-Alkyl-O-C₁₋₄-alkyl, HO-C₁₋₄-Alkyl und C₁₋₄-Alkyl-O- sein kann;
R⁴ ist ausgewählt aus der Gruppe, bestehend aus F, Cl, CN, -CH₃, -CF₃, Isopropyl, Cyclopropyl, H₃C-O-CH₂-, H₃C-O- und F₃C-O-;
R⁵ ist ausgewählt aus der Gruppe, bestehend aus C₁₋₄-Alkyl-, -CN, C₃₋₆-Cycloalkyl-, Heterocyclyl-C(=O)-, H₂N-C(=O)-, C₁₋₄-Alkyl-NR^{N}-C(=O)-, C₁₋₄-Alkyl-NR^{N}-, C₁₋₄-Alkyl-C(=O)NR^{N}-, C₁₋₄-Alkyl-S(=O)₂NR^{N}-, -OH, C₁₋₄-Alkyl-O-, C₁₋₄-Alkyl-O-C₁₋₄-alkyl-O-, C₃-₆-Cycloalkyl-O-, Heterocyclyl-O-, C₁₋₄-Alkyl-S(=O)-, C₁₋₄-Alkyl-S(=O)₂-, Heterocyclyl und Heteroaryl,
wobei jede Alkyl-, Cycloalkyl- und Heterocyclylgruppe oder -untergruppe in den genannten Gruppen gegebenenfalls mit 1 oder mehr F-Atomen substituiert ist und gegebenenfalls mit 1 oder 2 Gruppen substituiert ist, unabhängig ausgewählt aus H₃C-, H₃C-O- und -CN,
wobei Heterocyclyl ausgewählt ist aus
einer Cyclobutylgruppe, wobei 1 CH₂-Gruppe durch -NR^{N}- oder -O- ersetzt ist;
einer gesättigten oder teilweise ungesättigten C₅-₆-Cycloalkylgruppe, wobei 1 CH₂-Gruppe durch -C(=O)-, -NR^{N}-, -O-, -S(=O)- oder -S(=O)₂- ersetzt ist und/oder 1 CH-Gruppe durch N ersetzt ist;
einer gesättigten oder teilweise ungesättigten C₅₋₆-Cycloalkylgruppe, wobei 1 CH₂-Gruppe durch -NR^{N}- oder -O- ersetzt ist, eine zweite CH₂-Gruppe durch -NR^{N}-, -C(=O)-, -S(=O)- oder -S(=O)₂- ersetzt ist und/oder 1 CH-Gruppe durch N ersetzt ist; und
einer gesättigten oder teilweise ungesättigten C₅-₆-Cycloalkylgruppe, wobei 2 CH₂-Gruppe durch -NR^{N}- ersetzt sind oder 1 CH₂-Gruppe durch -NR^{N}- ersetzt ist und die andere durch -O- ersetzt ist, und eine dritte CH₂-Gruppe durch -C(=O)-, -S(=O)- oder -S(=O)₂- ersetzt ist und/oder 1 CH-Gruppe durch N ersetzt ist;
und wobei Heteroaryl ausgewählt ist aus einem Pyridin-2-onylring, einem 5-gliedrigen heteroaromatischen Ring, der 1 oder 2 Heteroatome enthält, unabhängig voneinander ausgewählt aus =N-, -NH-, -O- und -S-, und einem 6-gliedrigen heteroaromatischen Ring, der 1 oder 2 =N-Atome enthält, und wobei in heteroaromatischen Ringen mit einer oder mehreren NH-Gruppen jede von diesen durch NR^{N} ersetzt ist, und jede Heteroarylgruppe gegebenenfalls mit 1 oder 2 Substituenten substituiert ist, unabhängig ausgewählt aus F, Cl, -CH₃, -CN und -O-CH₃;
R⁶ ist ausgewählt aus der Gruppe, bestehend aus F, Cl, -CN, -CH₃, -CF₃, -OCH₃, -OCF₃, -S(=O)CH₃ und -S(=O)₂-CH₃;
R^{N} ist ausgewählt aus der Gruppe, bestehend aus H, H₃C-, H₃C-C(=O)- und C₁₋₃-Alkyl-S(=O)₂-; und
m ist 1 oder 2,
oder ein Salz hiervon.

6. Verbindung nach Anspruch 1, wobei
R¹ 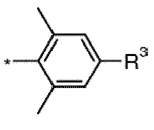 darstellt;
R² ist ausgewählt aus der Gruppe, bestehend aus F, Cl, F₃C- und NC-;
R³ ist ausgewählt aus der Gruppe, bestehend aus C₁₋₄-Alkyl, C₃₋₆-Cycloalkyl-, C₁₋₄-Alkyl-O-und C₃₋₆-Cycloalkyl-O-,
wobei jede Alkyl- und Cycloalkylgruppe und jede Alkyl- und Cycloalkyluntergruppe in den genannten Gruppen mit 1 Gruppe substituiert ist, ausgewählt aus R⁵, und gegebenenfalls mit 1 oder 2 H₃C-Gruppen substituiert ist;
oder aus H₃C-C(=O)-, Heterocyclyl-C(=O)-, H₂N-C(=O)-, H₃C-NR^{N}-C(=O)-, Heterocyclyl-O-, Heteroaryl-O-, H₂N-S(=O)₂-, Heterocyclyl, Phenyl und Heteroaryl,
wobei jede Heterocyclylgruppe oder -untergruppe in den genannten Gruppen gegebenenfalls mit 1 oder 2 Gruppen substituiert ist, unabhängig ausgewählt aus R⁵, und gegebenenfalls mit 1 oder mehr F-Atomen substituiert ist; und
wobei jede Phenyl- und Heteroarylgruppe gegebenenfalls mit 1 oder 2 Substituenten substituiert ist, unabhängig ausgewählt aus R⁶;
wobei Heterocyclyl ausgewählt ist aus
einer Cyclobutylgruppe, wobei 1 CH₂-Gruppe durch -NH- oder -O- ersetzt ist;
einer gesättigten oder einfach-ungesättigten C₅₋₆-Cycloalkylgruppe, wobei 1 CH₂-Gruppe durch -C(=O)-, -NH-, -O- oder -S(=O)₂- ersetzt ist und/oder 1 CH-Gruppe durch N ersetzt ist;
einer gesättigten oder einfach-ungesättigten C₅₋₆-Cycloalkylgruppe, wobei 1 CH₂-Gruppe durch -NH- oder -O- ersetzt ist, eine zweite CH₂-Gruppe durch -NH-, -C(=O)- oder -S(=O)₂- ersetzt ist und/oder 1 CH-Gruppe durch N ersetzt ist; und
wobei Heteroaryl ausgewählt ist aus einem 5-gliedrigen heteroaromatischen Ring, der 1 oder 2 Heteroatome enthält, unabhängig voneinander ausgewählt aus =N-, -NH-, -O- und -S-, und einem 6-gliedrigen heteroaromatischen Ring, der 1 oder 2 =N-Atome enthält, und wobei in Heteroaryl- und Heterocyclylringen mit einer oder mehreren NH-Gruppen jede von diesen durch NR^{N} oder NR⁵ ersetzt ist;
mit der Maßgabe, dass R³ insgesamt nicht C₁₋₄-Alkyl-, C₁₋₄-Alkyl-O-C₁₋₄-alkyl, HO-C₁₋₄-Alkyl und C₁₋₄-Alkyl-O- sein kann;
R⁵ ist ausgewählt aus der Gruppe, bestehend aus C₁₋₄-Alkyl-, -CN, C₃₋₆-Cycloalkyl-, Heterocyclyl-C(=O)-, H₂N-C(=O)-, C₁₋₄-Alkyl-NR^{N}-C(=O)-, C₁₋₄-Alkyl-NR^{N}-, C₁₋₄-Alkyl-C(=O)NR^{N}-, C₁₋₄-Alkyl-S(=O)₂NR^{N}-, -OH, C₁₋₄-Alkyl-O-, C₁₋₄-Alkyl-O-C₁₋₄-alkyl-O-, C₃-₆-Cycloalkyl-O-, Heterocyclyl-O-, C₁₋₄-Alkyl-S(=O)-, C₁₋₄-Alkyl-S(=O)₂-, Heterocyclyl und Heteroaryl,
wobei jede Alkyl-, Cycloalkyl- und Heterocyclylgruppe oder -untergruppe in den genannten Gruppen gegebenenfalls mit 1 oder mehr F-Atomen substituiert ist und gegebenenfalls mit 1 oder 2 Gruppen substituiert ist, unabhängig ausgewählt aus H₃C-, H₃C-O- und -CN,
wobei Heterocyclyl ausgewählt ist aus
einer Cyclobutylgruppe, wobei 1 CH₂-Gruppe durch -NR^{N}- oder -O- ersetzt ist;
einer gesättigten oder teilweise ungesättigten C₅₋₆-Cycloalkylgruppe, wobei 1 CH₂-Gruppe durch -C(=O)-, -NR^{N}-, -O-, -S(=O)- oder -S(=O)₂- ersetzt ist und/oder 1 CH-Gruppe durch N ersetzt ist;
einer gesättigten oder teilweise ungesättigten C₅₋₆-Cycloalkylgruppe, wobei 1 CH₂-Gruppe durch -NR^{N}- oder -O- ersetzt ist, eine zweite CH₂-Gruppe durch -NR^{N}-, -C(=O)-, -S(=O)- oder -S(=O)₂- ersetzt ist und/oder 1 CH-Gruppe durch N ersetzt ist; und
einer gesättigten oder teilweise ungesättigten C₅₋₆-Cycloalkylgruppe, wobei 2 CH₂-Gruppen durch -NR^{N}- ersetzt sind oder 1 CH₂-Gruppe durch -NR^{N}- ersetzt ist und die andere durch -O- ersetzt ist, und eine dritte CH₂-Gruppe durch -C(=O)-, -S(=O)- oder -S(=O)₂- ersetzt ist und/oder 1 CH-Gruppe durch N ersetzt ist;
und wobei Heteroaryl ausgewählt ist aus einem Pyridin-2-onylring, einem 5-gliedrigen heteroaromatischen Ring, der 1 oder 2 Heteroatome enthält, unabhängig voneinander ausgewählt aus =N-, -NH-, -O- und -S-, und einem 6-gliedrigen heteroaromatischen Ring, der 1 oder 2 =N-Atome enthält, und wobei in heteroaromatischen Ringen mit einer oder mehreren NH-Gruppen jede von diesen durch NR^{N} ersetzt ist, und jede Heteroarylgruppe ist gegebenenfalls mit 1 oder 2 Substituenten substituiert, unabhängig ausgewählt aus F, Cl, -CH₃, -CN und -O-CH₃;
R⁶ ist ausgewählt aus der Gruppe, bestehend aus F, -CH₃, CN und -OCH₃;
R^{N} ist ausgewählt aus der Gruppe, bestehend aus H, H₃C-, H₃C-C(=O)- und C₁₋₃-Alkyl-S(=O)₂-; und
m ist 1 oder 2;
oder ein Salz hiervon.

7. Verbindung nach irgendeinem der Ansprüche 1-6, wobei R² CF₃ darstellt und m 1 ist, oder ein Salz hiervon.

8. Verbindung nach Anspruch 1, wobei die Verbindung ausgewählt ist aus der Gruppe, bestehend aus: 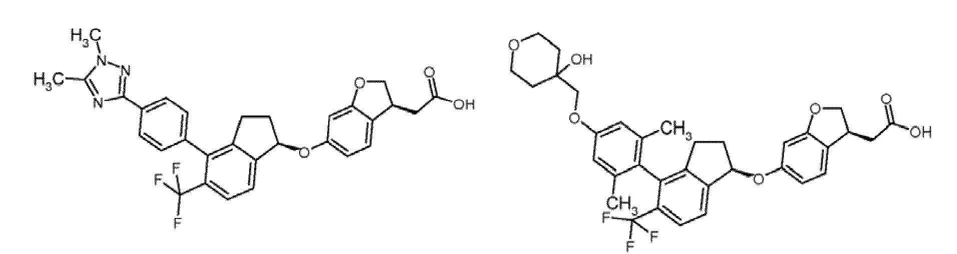 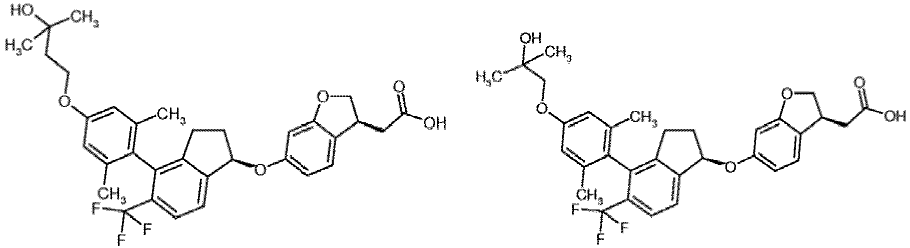 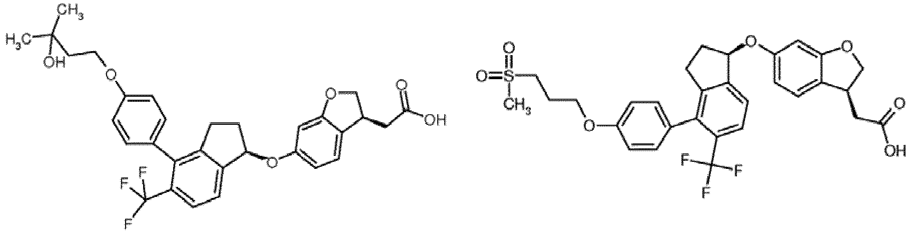 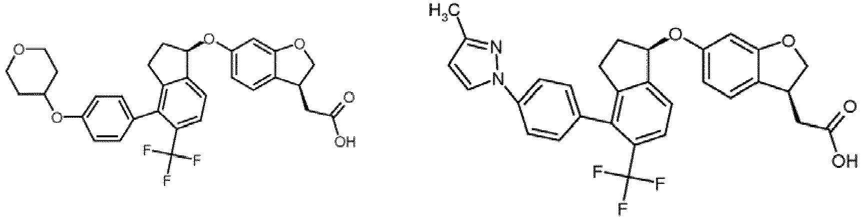 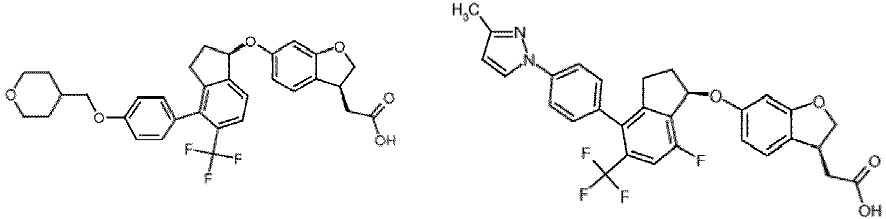 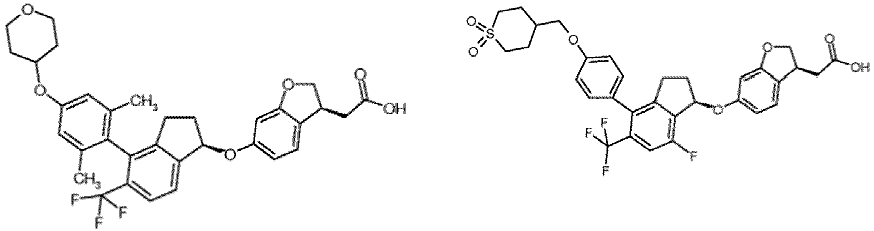 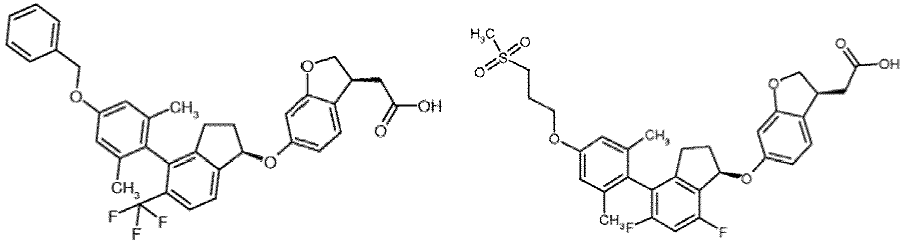 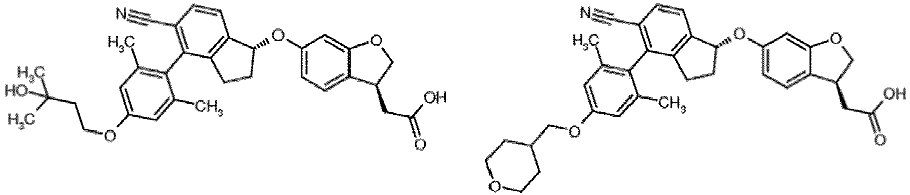 oder ein Salz hiervon.

9. Pharmazeutisch akzeptables bzw. annehmbares Salz einer Verbindung nach einem oder mehreren der Ansprüche 1 bis 8.

10. Pharmazeutische Zusammensetzung, umfassend eine oder mehrere Verbindungen nach einem oder mehreren der Ansprüche 1 bis 8 oder ein oder mehrere pharmazeutisch akzeptable bzw. annehmbare Salze hiervon, gegebenenfalls zusammen mit einem oder mehreren inerten Trägern und/oder Verdünnungsmitteln.

11. Verbindung nach einem oder mehreren der Ansprüche 1 bis 8 oder ein pharmazeutisch akzeptables bzw. annehmbares Salz hiervon zur Verwendung als Medikament.

12. Verbindung nach einem oder mehreren der Ansprüche 1 bis 8 oder ein pharmazeutisch akzeptables bzw. annehmbares Salz hiervon zur Verwendung in der Prophylaxe bzw. Vorbeugung und/oder Therapie von metabolischen Erkrankungen bzw. Stoffwechselerkrankungen und mit den Erkrankungen in Zusammenhang stehende Zustände.

13. Verbindung zur Verwendung nach Anspruch 12, wobei die metabolischen Erkrankungen oder mit den Erkrankungen in Zusammenhang stehende Zustände ausgewählt sind aus Diabetes, Insulinresistenz, Adipositas, kardiovaskulärer Erkrankung und Dyslipidämie.

14. Verbindung zur Verwendung nach Anspruch 12, wobei die metabolische Erkrankung Diabetes mellitus Typ 2 darstellt.

15. Pharmazeutische Zusammensetzung, umfassend eine oder mehrere Verbindungen nach einem oder mehreren der Ansprüche 1 bis 8 oder ein oder mehrere pharmazeutisch akzeptable bzw. annehmbare Salze hiervon und ein oder mehrere zusätzliche therapeutische Mittel, gegebenenfalls zusammen mit einem oder mehreren inerten Trägern und/oder Verdünnungsmitteln.

16. Pharmazeutische Zusammensetzung nach Anspruch 15 wobei die zusätzlichen therapeutischen Mittel ausgewählt sind aus der Gruppe, bestehend aus Antidiabetika, Mitteln zur Behandlung von Übergewicht und/oder Adipositas und Mittel zur Behandlung von Bluthochdruck, Herzinsuffizienz bzw. Herzschwäche und/oder Arteriosklerose.

## Revendications

1. Composé de formule (I) 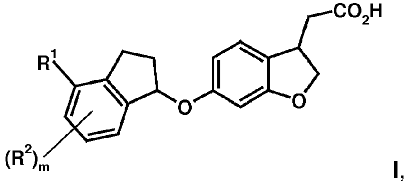 dans lequel
R¹ est sélectionné dans un groupe consistant en un cycle phényle, un cycle tétrazolyle, et un cycle hétéroaromatique à 5 ou 6 chaînons qui contient 1, 2 ou 3 hétéroatomes sélectionnés indépendamment les uns des autres parmi =N-, -NH-, -O- et -S- ;
dans lequel, éventuellement, un second cycle est condensé audit cycle phényle ou hétéroaromatique,
dans lequel ledit second cycle est un cycle à 5 ou 6 chaînons, insaturé ou aromatique, et peut contenir 1, 2 ou 3 hétéroatomes sélectionnés indépendamment les uns des autres parmi =N-, -NH-, -O- et -S- à condition que seulement au maximum deux des hétéroatomes soient 0 et S et qu'aucune liaison 0-0, S-S et S-0 ne se forme, et dans lequel, dans ledit second cycle, indépendamment de la présence des hétéroatomes, 1 ou 2 groupes CH₂ peuvent être remplacés par -C(=O)-, -S(=O)- ou -S(=O)₂-, et
dans lequel ledit cycle phényle, ledit cycle tétrazolyle, ledit cycle hétéroaromatique, ledit cycle phényle condensé et ledit cycle hétéroaromatique condensé sont substitués par un groupe R³ ; et
dans lequel chacun du cycle phényle, du cycle tétrazolyle, du cycle hétéroaromatique, du cycle phényle condensé et du cycle hétéroaromatique condensé est éventuellement en outre substitué par 1 à 4 groupes indépendamment sélectionnés parmi R⁴ ; et
dans lequel, dans ledit cycle hétéroaromatique et/ou ledit second cycle, l'atome H dans un ou plusieurs groupes NH, si présent, est remplacé par R^{N} ou R³ ;
R² est sélectionné dans un groupe consistant en F, Cl, Br, H₂FC-, F₃C-, NC-, et F₃C-O- ;
R³ est sélectionné dans un groupe consistant en les groupes C₁₋₆-alkyle, C₂₋₆-alcényle, C₂₋₆-alcynyle, C₃-₆-cycloalkyl-, C₁₋₄-alkyl-NH-, (C₁₋₄-alkyl)₂N-, C₁₋₆-alkyl-O-, C₃₋₆-cycloalkyl-O-, C₁₋₄-alkyl-S-, C₁₋₄-alkyl-S (=O) -, et C₁₋₄-alkyl-S (=O)₂,
dans lequel chaque groupe alkyle et cycloalkyle et chaque sous-groupe alkyle et cycloalkyle au sein des groupes mentionnés est substitué par 1 à 3 groupes indépendamment sélectionnés parmi R⁵ et éventuellement substitué par 1 ou plusieurs atomes F ;
ou parmi les groupes C₁₋₄-alkyl-C(=O)-, hétérocyclyl-C (=O) -, HNR^{N}-C (=O) -, C₁₋₄-alkyl-NR^{N}-C(=O)-, C₃₋₆-cycloalkyl-NR^{N}-C(=O)-, hétérocyclyl-NR^{N}-C(=O)-, phényl-NR^{N}-C (=O) -, hétéroaryl-NR^{N}-C(=O) -, HO₂C-, C₁₋₄-alkyl-O-C(=O) -, C₃₋₆-cycloalkyl-O-C(=O)-, hétérocyclyl-O-C(=O)-, -NHR^{N}, C₁₋₄-alkyl-C(=O)NR^{N}-, C₃₋₆-cycloalkyl-C(=O)NR^{N}-, hétérocyclyl-C(=O)-NR^{N}-, phényl-C(=O)NR^{N}-, hétéroaryl-C(=O) NR^{N}-, C₁₋₄-alkyl-S (=O) ₂-NR^{N}-, C₃-₆-cycloalkyl-S (=O) ₂NR^{N}-, hétérocyclyl-S-(=O)₂NR^{N}-, phényl-S(=O)₂NR^{N}-, hétéroaryl-S(=O)₂NR^{N}-, hétérocyclyl-O-, phényl-O-, hétéroaryl-O-, C₃₋₆-cycloalkyl-S-, hétérocyclyl-S-, phényl-S-, hétéroaryl-S-, C₃₋₆-cycloalkyl-S(=O)-, hétérocyclyl-S(=O)-, phényl-S(=O)-, hétéroaryl-S(=O)-, C₃₋₆-cycloalkyl-S(=O)₂-, hétérocyclyl-S(=O)₂-_{,} phényl-S(=O) ₂-, hétéroaryl-S(=O)₂-, HNR^{N}-S (=0) ₂-, C₁₋₄-alkyl-NR^{N}-S(=O)₂-, hétérocyclyle, phényle et hétéroaryle, dans lequel chaque groupe ou sous-groupe alkyle, cycloalkyle et hétérocyclyle au sein des groupes mentionnés est éventuellement substitué par 1 à 3 groupes indépendamment sélectionnés parmi R⁵ et éventuellement substitué par 1 ou plusieurs atomes F ; et
dans lequel chaque groupe phényle et hétéroaryle est éventuellement substitué par 1 à 5 substituants indépendamment sélectionnés parmi R⁶ ;
dans lequel le cycle hétérocyclyle est sélectionné parmi un groupe cyclobutyle dans lequel 1 groupe CH₂ est remplacé par -NH- ou -0-,
un groupe C₅₋₇-cycloalkyle saturé ou partiellement insaturé dans lequel 1 groupe CH₂ est remplacé par -C(=O)-, -NH-, -O-, -S(=O)- ou -S(=O)₂- et/ou 1 groupe CH par N ; un groupe C₅₋₇-cycloalkyle saturé ou partiellement insaturé dans lequel 1 groupe CH₂ est remplacé par -NH-ou -0-, un second groupe CH₂ est remplacé par -NH-, -C- (=O) -, -S(=O)- ou -S(=O)₂- et/ou 1 groupe CH est remplacé par N ; et
un groupe C₅₋₇-cycloalkyle saturé ou partiellement insaturé dans lequel 2 groupes CH₂ sont remplacés par -NH- ou 1 groupe CH₂ par -NH- et l'autre par -0- et un troisième groupe CH₂ est remplacé par -C(=O)-, -S(=O)- ou -S(=O)₂- et/ou 1 groupe CH par N ;
dans lequel le cycle hétéroaryle est sélectionné parmi un cycle tétrazolyle et un cycle hétéroaromatique à 5 ou 6 chaînons qui contient 1, 2 ou 3 hétéroatomes sélectionnés indépendamment les uns des autres parmi =N-, -NH-, -0- et -S-, dans lequel, dans les groupes hétéroaromatiques contenant un motif -HC=N-, ce groupe est éventuellement remplacé par -NH-C(=O)- ;
dans lequel, dans les cycles hétéroaryle et hétérocyclyle contenant un ou plusieurs groupes NH, chacun d'entre eux est remplacé par NR^{N} ou NR⁵ ;
à condition que R³ au total ne puisse pas être un groupe C₁₋₄-alkyle, C₁₋₄-alkyl-O-C₁₋₄-alkyle, HO-C₁₋₄-alkyle, C₃₋₄-alcényle, C₃₋₄-alcynyle, H₂N-, C₁₋₄-alkyl-NH-, (C₁₋₄-alkyl) ₂N-, C₁₋₄-alkyl-O-, C₁₋₄-alkyl-S-, C₁₋₄-alkyl-S (=O) - et C₁₋₄-alkyl-S(=O)₂- ;
R⁴ est sélectionné dans un groupe consistant en F, Cl, Br, I, CN, -OH, les groupes C₁₋₄-alkyle, C₃₋₆-cycloalkyle, HO-C₁₋₄-alkyle, C₁₋₄-alkyl-O-C₁₋₄-alkyle, -NR^{N}H, C₁₋₄-alkyl-NR^{N}-, C₁₋₄-alkyl-O-, C₃₋₆-cycloalkyl-O-, C₁₋₄-alkyl-O-C₁₋₄-alkyl-O-, C₁₋₄-alkyl-S-, C₁₋₄-alkyl-S (=O) - et C₁₋₄-alkyl-S(=O)₂-,
dans lequel tout groupe ou sous-groupe alkyle et cycloalkyle au sein des groupes mentionnés est éventuellement substitué par 1 ou plusieurs atomes F ;
R⁵ est sélectionné dans un groupe consistant en Cl, Br, I, les groupes C₁₋₄-alkyl-, CN, C₃-6-cycloalkyl-, hétérocyclyl-C(=O)-, H₂N-C(=O) -, C₁₋₄-alkyl-NR^{N}-C(=O)-, C₃₋₆-cycloalkyl-NR^{N}-C(=O)-, hétérocyclyl-NR^{N}-C(=O) -, phényl-NR^{N}-C (=O) -, hétéroaryl-NR^{N}-C (=O) -, HO-C (=O) -, C₁-₄-alkyl-O-C(=O) -, -NHR^{N}, C₁₋₄-alkyl-NR^{N}-, C₁₋₄-alkyl-C (=O) NR^{N}-, C₃₋₆-cycloalkyl-C(=O)NR^{N}-, hétérocyclyl-C(=O)NR^{N}-, phényl-C(=O) NR^{N}-, hétéroaryl-C (=O) NR^{N}-, C₁₋₄-alkyl-S(=O)₂NR^{N}-, C₃₋₆-cycloalkyl-S(=O)₂NR^{N}-, hétérocyclyl-S(=O)₂NR^{N}-, phényl-S(=O)₂NR^{N}-, hétéroaryl-S (=O) ₂NR^{N}-, -OH, C₁₋₄-alkyl-O-, C₁₋₄-alkyl-O-C₁₋₄-alkyl-O-, C₃₋₆-cycloalkyl-O-, hétérocyclyl-O-, phényl-O-, hétéroaryl-O-, C₁₋₄-alkyl-S-, C₃₋₆-cycloalkyl-S-, hétérocyclyl-S-, phényl-S-, hétéroaryl-S-, C₁₋₄-alkyl-S(=O)-, C₃₋₆-cycloalkyl-S(=O)-, hétérocyclyl-S(=O)-, phényl-S(=O)-, hétéroaryl-S(=O)-, C₁₋₄-alkyl-S(=O)2-, C₃₋₆-cycloalkyl-S(=O)₂-, hétérocyclyl-S(=O)₂-, phényl-S(=O)₂-, hétéroaryl-S(=O)₂-, H₂N-S(=O)₂-, C₁₋₄-alkyl-NR^{N}-S(=O)₂-, hétérocyclyle, phényle et hétéroaryle,
dans lequel tout groupe ou sous-groupe alkyle, cycloalkyle et hétérocyclyle au sein des groupes mentionnés est éventuellement substitué par 1 ou plusieurs atomes F et éventuellement substitué par 1 ou 2 groupes indépendamment sélectionnés parmi H₃C-, HO-, H₃C-O-et -CN ;
dans lequel le cycle hétérocyclyle est sélectionné parmi
un groupe cyclobutyle dans lequel 1 groupe CH₂ est remplacé par -NR^{N}- ou -O-,
un groupe C₅₋₇-cycloalkyle saturé ou partiellement insaturé dans lequel 1 groupe CH₂ est remplacé par -C(=O)-, -NR^{N}-, -O-, -S(=O)- ou -S(=O)₂- et/ou 1 groupe CH par N ;
un groupe C₅₋₇-cycloalkyle saturé ou partiellement insaturé dans lequel 1 groupe CH₂ est remplacé par -NR^{N}- ou -O-, un second groupe CH₂ est remplacé par -NR^{N}-, -C-(=O)-, -S(=O)- ou -S(=O)₂- et/ou 1 groupe CH est remplacé par N ; et
un groupe C₅₋₇-cycloalkyle saturé ou partiellement insaturé dans lequel 2 groupes CH₂ sont remplacés par -NR^{N}- ou 1 groupe CH₂ par -NR^{N}- et l'autre par -0- et un troisième groupe CH₂ est remplacé par -C(=O)-, -S(=O)- ou -S(=O)₂- et/ou 1 groupe CH par N ;
et dans lequel le cycle hétéroaryle est sélectionné parmi
un cycle tétrazolyle, et un cycle hétéroaromatique à 5 ou 6 chaînons qui contient 1, 2 ou 3 hétéroatomes sélectionnés indépendamment les uns des autres parmi =N-, -NH-, -0- et -S-, dans lequel, dans les groupes hétéroaromatiques contenant un motif -HC=N-, ce groupe est éventuellement remplacé par -NR^{N}-C(=O)-, et dans lequel, dans les cycles hétéroaromatiques contenant un ou plusieurs groupes NH, chacun d'entre eux est remplacé par NR^{N}, et chaque groupe hétéroaryle est éventuellement substitué par 1 à 3 substituants indépendamment sélectionnés parmi F, Cl, -CH₃, -CN et -O-CH₃ ;
R⁶ est sélectionné dans un groupe consistant en F, Cl, Br, I, CN, les groupes C₁₋₄-alkyle, C₃₋₆-cycloalkyl-, HO-C₁₋₄-alkyl-, C₁₋₄-alkyl-O-C₁₋₄-alkyl-, R^{N}HN-, C₁₋₄-alkyl-O-, -S(=O)-C₁₋₄-alkyle et S(=O)₂-C₁₋₄-alkyle,
dans lequel tout groupe ou sous-groupe alkyle et cycloalkyle au sein des groupes mentionnés est éventuellement substitué par un ou plusieurs atomes F ; R^{N} est sélectionné indépendamment les uns des autres dans un groupe consistant en H, les groupes C₁₋₄-alkyle, C₁₋₄-alkyl-C(=O)-, C₁₋₄-alkyl-NH-C(=O)-, C₁₋₄-alkyl-N (C₁₋₄-alkyl)-C(=O)-, C₁₋₄-alkyl-O-C(=O) - et C₁₋₄-alkyl-S(=O)₂- ; et
m vaut 1 ou 2 ;
dans lequel dans toute définition mentionnée ci-dessus et sauf spécification contraire, tout groupe ou sous-groupe alkyle peut être linéaire ou ramifié,
ou un sel de celui-ci.

2. Composé selon la revendication 1, dans lequel
R¹ est sélectionné dans un groupe consistant en un cycle phényle, un cycle tétrazolyle, un cycle hétéroaromatique à 5 chaînons qui contient 2 ou 3 hétéroatomes sélectionnés indépendamment les uns des autres parmi =N-, -NH-, -0- et -S- à condition que pas plus d'un hétéroatome soit -0- ou -S-, et un cycle hétéroaromatique à 6 chaînons qui contient 1 ou 2 atomes =N- ;
dans lequel, éventuellement, un second cycle est condensé audit cycle phényle et auxdits cycles hétéroaromatiques à 5 et 6 chaînons, dans lequel ledit second cycle est un cycle à 5 ou 6 chaînons, insaturé ou aromatique, et peut contenir 1 ou 2 hétéroatomes sélectionnés indépendamment les uns des autres parmi =N-, -NH-, -O- et -S- à condition qu'aucune liaison 0-0, S-S et S-O ne se forme, et dans lequel, dans ledit second cycle, indépendamment de la présence des hétéroatomes, 1 ou 2 groupes -CH₂- peuvent être remplacés par -C(=O)- ou -S(=O)₂-, et
dans lequel, dans ledit cycle hétéroaromatique et/ou ledit second cycle, l'atome H dans un ou plusieurs groupes NH, si présent, est remplacé par R^{N} ou R³, et dans lequel chacun desdits cycle phényle, cycle tétrazolyle, cycles hétéroaromatiques, cycle phényl condensé, et cycles hétéroaromatiques condensés est substitué par un groupe R³ et éventuellement substitué en outre par 1 ou 2 substituants indépendamment sélectionnés parmi R⁴ ;
R² est sélectionné dans un groupe consistant en F, Cl, Br, H₂FC-, F₃C-, NC-, et F₃C-O- ;
R³ est sélectionné dans un groupe consistant en les groupes C₁₋₄-alkyle, C₃₋₆-cycloalkyl-, C₁₋₄-alkyl-O-, C₃₋₆-cycloalkyl-O-, C₁₋₄-alkyl-S(=O) - et C₁₋₄-alkyl-S(=O)₂-,
dans lequel chaque groupe alkyle et cycloalkyle et chaque sous-groupe alkyle et cycloalkyle au sein des groupes mentionnés est substitué par 1 à 3 groupes indépendamment sélectionnés parmi R⁵ et éventuellement substitué par 1 ou plusieurs atomes F ;
ou parmi les groupes C₁₋₄-alkyl-C(=O)-, hétérocyclyl-C(=O) -, HNR^{N}-C (=O) -, C₁₋₄-alkyl-NR^{N}-C(=O) -, C₃₋₆-cycloalkyl-NR^{N}-C(=O)-, hétérocyclyl-NR^{N}-C(=O) -, phényl-NR^{N}-C (=O) -, hétéroaryl-NR^{N}-C(=O)-, C₁₋₄-alkyl-C(=O)NR^{N}-, C₃₋₆-cycloalkyl-C(=O)NR^{N}-, hétérocyclyl-C(=O)NR^{N}-, phényl-C(=O)NR^{N}-, hétéroaryl-C(=O)NR^{N}-, C₁₋₄-alkyl-S(=O)₂-NR^{N}-, C₃₋₆-cycloalkyl-S (=O)₂NR^{N}-, hétérocyclyl-S(=O)₂NR^{N}-, phényl-S(=O)₂NR^{N}-, hétéroaryl-S(=O)₂NR^{N}-, hétérocyclyl-O-, phényl-O-, hétéroaryl-O-, C₃₋₆-cycloalkyl-S(=O)-, hétérocyclyl-S(=O)-, phényl-S(=O)-, hétéroaryl-S(=O)-, C₃₋₆-cycloalkyl-S(=O)₂-, hétérocyclyl-S(=O)₂-, phényl-S(=O)₂-, hétéroaryl-S(=O)₂-, HNR^{N}-S(=O)₂-, C₁₋₄-alkyl-NR^{N}-S(=O)₂-, hétérocyclyle, phényle et hétéroaryle,
dans lequel chaque groupe ou sous-groupe alkyle, cycloalkyle et hétérocyclyle au sein des groupes mentionnés est éventuellement substitué par 1 à 3 groupes indépendamment sélectionnés parmi R⁵ et éventuellement substitué par 1 ou plusieurs atomes F ; et
dans lequel chaque groupe phényle et hétéroaryle est éventuellement substitué par 1 à 3 substituants sélectionnés indépendamment parmi R⁶ ;
dans lequel le cycle hétérocyclyle est sélectionné parmi
un groupe cyclobutyle dans lequel 1 groupe CH₂ est remplacé par -NH- ou -0-,
un groupe C₅₋₆-cycloalkyle saturé ou mono-insaturé dans lequel 1 groupe CH₂ est remplacé par -C(=O)-, -NH-, -0-, -S(=O)- ou -S(=O)₂- et/ou 1 groupe CH par N ;
un groupe C₅₋₆-cycloalkyle saturé ou mono-insaturé dans lequel 1 groupe CH₂ est remplacé par -NH- ou -0-, un second groupe CH₂ est remplacé par -NH-, -C(=O)-, -S(=O)- ou -S(=O)₂- et/ou 1 groupe CH est remplacé par N ; et
un groupe C₅₋₆-cycloalkyle saturé ou mono-insaturé dans lequel 2 groupes CH₂ sont remplacés par -NH- ou 1 groupe CH₂ par -NH- et l'autre par -0- et un troisième groupe CH₂ est remplacé par -C(=O) -, -S(=O) - ou -S(=O)₂- et/ou 1 groupe CH par N ;
dans lequel le cycle hétéroaryle est sélectionné parmi un cycle tétrazolyle, un cycle pyridin-2-onyle, un cycle hétéroaromatique à 5 chaînons qui contient 1, 2 ou 3 hétéroatomes sélectionnés indépendamment les uns des autres parmi =N-, -NH-, -0- et -S-, et un cycle hétéroaromatique à 6 chaînons qui contient 1 ou 2 atomes =N-,
et dans lequel, dans les cycles hétéroaryle et hétérocyclyle avec un ou plusieurs groupes NH, chacun d'entre eux est remplacé par NR^{N} ou NR⁵,
à condition que R³ au total ne puisse pas être un groupe C₁₋₄-alkyl-, C₁₋₄-alkyl-O-C₁₋₄-alkyle, HO-C₁₋₄-alkyle, C₁₋₄-alkyl-O-, C₁₋₄-alkyl-S(=O) -, et C₁₋₄-alkyl-S(=O)₂,
R⁴ est sélectionné dans un groupe consistant en F, Cl, Br, CN, les groupes C₁₋₃-alkyle, C₃₋₄-cycloalkyl-, HO-C₁-₃-alkyle, C₁₋₃-alkyl-O-C₁₋₃-alkyle, -NR^{N}H, C₁₋₄-alkyl-O-, C₃₋₅-cycloalkyl-O-, H₃C-S (=O) -, H₃C-S(=O)₂-, dans lequel tout groupe alkyle et cycloalkyle est éventuellement substitué par 1 ou plusieurs atomes F,
R⁵ est sélectionné dans un groupe consistant en Cl, les groupes C₁₋₄-alkyl-, -CN, C₃₋₆-cycloalkyl-, hétérocyclyl-C(=O)-, H₂N-C(=O)-, C₁₋₄-alkyl-NR^{N}-C(=O)-, C₃₋₆-cycloalkyl-NR^{N}-C(=O) -, hétérocyclyl-NR^{N}-C(=O) -, hétéroaryl-NR^{N}-C(=O) -, -NH₂, C₁₋₄-alkyl-NR^{N}- C₁₋₄-alkyl-C(=O)NR^{N}-, C₃₋₆-cycloalkyl-C(=O)NR^{N}- , hétérocyclyl-C(=O)NR^{N}-, hétéroaryl-C(=O)NR^{N}-, C₁₋₄-alkyl-S (=0) ₂NR^{N}-, -OH, C₁₋₄-alkyl-O-, C₁₋₄-alkyl-O-C₁₋₄-alkyl-O-, C₃₋₆-cycloalkyl-O-, hétérocyclyl-O-, phényl-O-, hétéroaryl-O-, C₁₋₄-alkyl-S(=O)-, C₃₋₆-cycloalkyl-S(=O)-, C₁₋₄-alkyl-S(=O)₂-, C₃₋₆-cycloalkyl-S(=O)₂-, hétérocyclyle, et hétéroaryle,
dans lequel tout groupe ou sous-groupe alkyle, cycloalkyle et hétérocyclyle au sein des groupes mentionnés est éventuellement substitué par 1 ou plusieurs atomes F et éventuellement substitué par 1 ou 2 groupes indépendamment sélectionnés parmi H₃C-, HO-, H₃C-O- et -CN ;
dans lequel le cycle hétérocyclyle est sélectionné parmi
un groupe cyclobutyle dans lequel 1 groupe CH₂ est remplacé par -NR^{N}- ou -O-,
un groupe C₅₋₆-cycloalkyle saturé ou partiellement insaturé dans lequel 1 groupe CH₂ est remplacé par -C(=O)-, -NR^{N}-, -O-, -S(=O)- ou -S(=O)₂- et/ou 1 groupe CH par N ;
un groupe C₅₋₆-cycloalkyle saturé ou partiellement insaturé dans lequel 1 groupe CH₂ est remplacé par -NR^{N}- ou -O-, un second groupe CH₂ est remplacé par -NR^{N}-, -C-(=O)-, -S(=O)- ou -S(=O)₂- et/ou 1 groupe CH est remplacé par N ; et
un groupe C₅₋₆-cycloalkyle saturé ou partiellement insaturé dans lequel 2 groupes CH₂ sont remplacés par -NR^{N}- ou 1 groupe CH₂ par -NR^{N}- et l'autre par -0- et un troisième groupe CH₂ est remplacé par -C(=O)-, -S(=O)-ou -S(=O)₂- et/ou 1 groupe CH par N ;
et dans lequel le cycle hétéroaryle est sélectionné parmi un cycle tétrazolyle, un cycle pyridin-2-onyle, un cycle hétéroaromatique à 5 chaînons qui contient 1, 2 ou 3 hétéroatomes sélectionnés indépendamment les uns des autres parmi =N-, -NH-, -0- et -S-, et un cycle hétéroaromatique à 6 chaînons qui contient 1 ou 2 atomes =N-, et dans lequel dans les cycles hétéroaromatiques avec un ou plusieurs groupes NH, chacun d'entre eux est remplacé par NR^{N}, et chaque groupe hétéroaryle est éventuellement substitué par 1 à 3 substituants indépendamment sélectionnés parmi F, Cl, -CH₃, -CN et -O-CH₃,
R⁶ est sélectionné dans un groupe consistant en F, Cl, -CN, les groupes C₁₋₃-alkyle, cyclopropyle, HO-C₁₋₃-alkyl-, H₃C-O-C₁₋₃-alkyl-, H₃C-O-, -S(=O)CH₃ et S(=O)₂-CH₃, dans lequel tout groupe ou sous-groupe alkyle et cycloalkyle au sein des groupes mentionnés est éventuellement substitué par un ou plusieurs atomes F ;
R^{N} est H, un groupe C₁₋₃-alkyle, H₃C-C(=O) -, ou C₁₋₃-alkyl-S(=O)₂- ;
m vaut 1 ou 2 ;
ou un sel de celui-ci.

3. Composé selon la revendication 2, dans lequel R¹ est sélectionné dans un groupe consistant en un cycle phényle, un cycle tétrazolyle, un cycle hétéroaromatique à 5 chaînons qui contient 2 ou 3 hétéroatomes sélectionnés indépendamment les uns des autres parmi =N-, -NH-, -O- et -S- et un cycle hétéroaromatique à 6 chaînons qui contient 1 ou 2 atomes =N- ;
dans lequel dans ledit cycle hétéroaromatique à 5 chaînons, l'atome H dans un ou plusieurs groupes NH est remplacé par R^{N} ou R³, et
dans lequel chacun desdits cycle phényle, cycle tétrazolyle et cycles hétéroaromatiques est substitué par un groupe R³ et éventuellement substitué en outre par 1 ou 2 substituants indépendamment sélectionnés parmi R⁴,
ou un sel de celui-ci.

4. Composé selon la revendication 2, dans lequel
R¹ est sélectionné dans un groupe consistant en un cycle phényle, un cycle hétéroaromatique à 5 chaînons qui contient 2 ou 3 hétéroatomes sélectionnés indépendamment les uns des autres parmi =N-, -NH-, -O-et -S-, et un cycle hétéroaromatique à 6 chaînons qui contient 1 ou 2 atomes =N-, dans lequel un second cycle à 5 ou 6 chaînons, insaturé ou aromatique est condensé audit cycle phényle et auxdits cycles hétéroaromatiques à 5 et 6 chaînons, qui peuvent contenir 1 ou 2 hétéroatomes sélectionnés indépendamment les uns des autres parmi =N-, -NH-, -0- et -S- à condition qu'aucune liaison 0-0, S-S et S-O ne se forme, et dans lequel, dans ledit second cycle, 1 ou 2 groupes -CH₂-peuvent être remplacés par -C(=O)- ou -S(=O)₂-, et
dans lequel, dans lesdits cycles hétéroaromatiques et lesdits seconds cycles, l'atome H dans un ou plusieurs groupes NH, si présent, est remplacé par R^{N} ou R³, et dans lequel chacun dudit cycle phényle condensé et dudit cycle hétéroaromatique condensé est substitué par un groupe R³ et éventuellement substitué en outre par 1 ou 2 substituants indépendamment sélectionnés parmi R⁴, ou un sel de celui-ci.

5. Composé selon la revendication 1, dans lequel R¹ est sélectionné dans le groupe consistant en 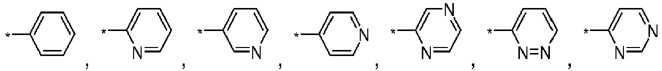 et 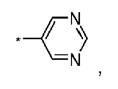 dans lequel chaque groupe est substitué par un groupe R³ et éventuellement en outre substitué par 1 ou 2 groupes indépendamment sélectionnés parmi R⁴ ;
R² est sélectionné dans le groupe consistant en F, Cl, Br, H₂FC-, F₃C-, NC-, et F₃C-O- ;
R³ est sélectionné dans le groupe consistant en les groupes C₁₋₄-alkyle, C₃₋₆-cycloalkyl-, C₁₋₄-alkyl-O-, et C₃₋₆-cycloalkyl-O- ,
dans lequel chaque groupe alkyle et cycloalkyle et chaque sous-groupe alkyle et cycloalkyle au sein des groupes mentionnés est substitué par 1 à 3 groupes indépendamment sélectionnés parmi R⁵ et éventuellement substitué par 1 ou plusieurs atomes F ;
ou parmi les groupes H₃C-C(=O) -, hétérocyclyl-C(=O)-, H₂N-C (=O) -, C₁₋₃-alkyl-NR^{N}-C (=O) -, C₁₋₄-alkyl-C(=O)NR^{N}-, C₃₋₆-cycloalkyl-C(=O)NR^{N}-, hétérocyclyl-C(=O)-NR^{N}-, C₁₋₄-alkyl-S(=O)₂-NR^{N}-, hétérocyclyl-O-, phényl-O-, hétéroaryl-O-, hétérocyclyl-S(=O)₂-, H₂N-S(=O)₂-, hétérocyclyle, phényle et hétéroaryle,
dans lequel chaque groupe ou sous-groupe alkyle, cycloalkyle et hétérocyclyle au sein des groupes mentionnés est éventuellement substitué par 1 ou 2 groupes indépendamment sélectionnés parmi R⁵ et éventuellement substitué par 1 ou plusieurs atomes F ; et
dans lequel chaque groupe phényle et hétéroaryle est éventuellement substitué par 1 à 3 substituants indépendamment sélectionnés parmi R⁶ ;
dans lequel le cycle hétérocyclyle est sélectionné parmi
un groupe cyclobutyle dans lequel 1 groupe CH₂ est remplacé par -NH- ou -0-,
un groupe C₅₋₆-cycloalkyle saturé ou mono-insaturé dans lequel 1 groupe CH₂ est remplacé par -C(=O)-, -NH-, -0-, ou -S(=O)₂- et/ou 1 groupe CH par N ;
un groupe C₅₋₆-cycloalkyle saturé ou mono-insaturé dans lequel 1 groupe CH₂ est remplacé par -NH- ou -0-, un second groupe CH₂ est remplacé par -C-(=O)- ou -S(=O)₂- et/ou 1 groupe CH est remplacé par N ; et
dans lequel le cycle hétéroaryle est sélectionné parmi un cycle pyridin-2-onyle, un cycle hétéroaromatique à 5 chaînons qui contient 1, 2 ou 3 hétéroatomes sélectionnés indépendamment les uns des autres parmi =N-, -NH-, -0- et -S-, et un cycle hétéroaromatique à 6 chaînons qui contient 1 ou 2 atomes =N-,
et dans lequel, dans les cycles hétéroaryle et hétérocyclyle avec un ou plusieurs groupes NH, chacun d'entre eux est remplacé par NR^{N} ou NR⁵ ;
à condition que R³ au total ne puisse pas être un groupe C₁₋₄-alkyle, C₁₋₄-alkyl-O-C₁₋₄-alkyle, HO-C₁₋₄-alkyle et C₁₋₄-alkyl-O- ;
R⁴ est sélectionné dans le groupe consistant en F, Cl, CN, -CH₃, -CF₃, les groupes isopropyle, cyclopropyle, H₃C-O-CH₂, H₃C-O- et F₃C-O- ;
R⁵ est sélectionné dans le groupe consistant en les groupes C₁₋₄-alkyl-, -CN, C₃₋₆-cycloalkyl-, hétérocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, hétérocyclyl-O-, C₁₋₄-alkyl-S(=O)-, C₁₋₄-alkyl-S(=O)₂-, hétérocyclyle, et hétéroaryle,
dans lequel tout groupe ou sous-groupe alkyle, cycloalkyle et hétérocyclyle au sein des groupes mentionnés est éventuellement substitué par 1 ou plusieurs atomes F et éventuellement substitué par 1 ou 2 groupes indépendamment sélectionnés parmi H₃C-, H₃C-0- et -CN,
dans lequel le cycle hétérocyclyle est sélectionné parmi
un groupe cyclobutyle dans lequel 1 groupe CH₂ est remplacé par -NR^{N}- ou -O- ;
un groupe C₅₋₆-cycloalkyle saturé ou partiellement insaturé dans lequel 1 groupe CH₂ est remplacé par -C(=O)-, -NR^{N}-, -O-, -S(=O)- ou -S(=O)₂- et/ou 1 groupe CH par N ;
un groupe C₅₋₆-cycloalkyle saturé ou partiellement insaturé dans lequel 1 groupe CH₂ est remplacé par -NR^{N}- ou -O-, un second groupe CH₂ est remplacé par -NR^{N}-, -C-(=O)-, -S(=O)- ou -S(=O)₂- et/ou 1 groupe CH est remplacé par N ; et
un groupe C₅₋₆-cycloalkyle saturé ou partiellement insaturé dans lequel 2 groupes CH₂ sont remplacés par -NR^{N}- ou 1 groupe CH₂ par -NR^{N}- et l'autre par -0- et un troisième groupe CH₂ est remplacé par -C(=O)-, -S(=O)-ou -S(=O)₂- et/ou 1 groupe CH par N ;
et dans lequel le cycle hétéroaryle est sélectionné parmi un cycle pyridin-2-onyle, un cycle hétéroaromatique à 5 chaînons qui contient 1 ou 2 hétéroatomes sélectionnés indépendamment les uns des autres parmi =N-, -NH-, -0- et -S-, et un cycle hétéroaromatique à 6 chaînons qui contient 1 ou 2 atomes =N-, et dans lequel, dans les cycles hétéroaromatiques avec un ou plusieurs groupes NH, chacun d'entre eux est remplacé par NR^{N}, et chaque groupe hétéroaryle est éventuellement substitué par 1 ou 2 substituants indépendamment sélectionnés parmi F, Cl, -CH₃, -CN et -O-CH₃ ;
R⁶ est sélectionné dans le groupe consistant en F, Cl, -CN, -CH₃, -CF₃, -OCH₃, -OCF₃, -S (=O) CH₃ et -S(=O)₂-CH₃ ;
R^{N} est sélectionné dans le groupe consistant en H, H₃C-, H₃C-C(=O)-, et C₁₋₃-alkyl-S(=O)₂- ; et
m vaut 1 ou 2 ;
ou un sel de celui-ci.

6. Composé selon la revendication 1, dans lequel
R¹ est 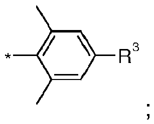 R² est sélectionné dans le groupe consistant en F, Cl, F₃C- et NC- ;
R³ est sélectionné dans le groupe consistant en les groupes C₁₋₄-alkyle, C₃₋₆-cycloalkyl-, C₁₋₄-alkyl-O-, et C₃₋₆-cycloalkyl-O-,
dans lequel chaque groupe alkyle et cycloalkyle et chaque sous-groupe alkyle et cycloalkyle au sein des groupes mentionnés est substitué par 1 groupe sélectionné parmi R⁵ et éventuellement substitué par 1 ou 2 groupes H₃C- ;
ou parmi les groupes H₃C-C(=O)-, hétérocyclyl-C(=O)-, H₂N-C (=O) -, H₃C-NR^{N}-C(=O)-, hétérocyclyl-O-, hétéroaryl-O-, H₂N-S(=O)₂-, hétérocyclyle, phényle et hétéroaryle, dans lequel chaque groupe ou sous-groupe hétérocyclyle au sein des groupes mentionnés est éventuellement substitué par 1 ou 2 groupes indépendamment sélectionnés parmi R⁵ et éventuellement substitué par 1 ou plusieurs atomes F ; et
dans lequel chaque groupe phényle et hétéroaryle est éventuellement substitué par 1 ou 2 substituants indépendamment sélectionnés parmi R⁶ ; dans lequel le cycle hétérocyclyle est sélectionné parmi
un groupe cyclobutyle dans lequel 1 groupe CH₂ est remplacé par -NH- ou -0-,
un groupe C₅₋₆-cycloalkyle saturé ou mono-insaturé dans lequel 1 groupe CH₂ est remplacé par -C(=O)-, -NH-, -0-, ou -S(=O)₂- et/ou 1 groupe CH par N ;
un groupe C₅₋₆-cycloalkyle saturé ou mono-insaturé dans lequel 1 groupe CH₂ est remplacé par -NH- ou -0-, un second groupe CH₂ est remplacé par -NH-, -C-(=0)- ou -S(=O)₂- et/ou 1 groupe CH est remplacé par N ; et
dans lequel le cycle hétéroaryle est sélectionné parmi un cycle hétéroaromatique à 5 chaînons qui contient 1 ou 2 hétéroatomes sélectionnés indépendamment les uns des autres parmi =N-, -NH-, -0- et -S-, et un cycle hétéroaromatique à 6 chaînons qui contient 1 ou 2 atomes =N-, et dans lequel, dans les cycles hétéroaryle et hétérocyclyle avec un ou plusieurs groupes NH, chacun d'entre eux est remplacé par NR^{N} ou NR⁵ ;
à condition que R³ au total ne puisse pas être un groupe C₁₋₄-alkyl-, C₁₋₄-alkyl-O-C₁₋₄-alkyle, HO-C₁₋₄-alkyle et C₁₋₄-alkyl-O- ;
R⁵ est sélectionné dans le groupe consistant en les groupes C₁₋₄-alkyl-, -CN, C₃₋₆-cycloalkyl-, hétérocyclyl-C(=O)-, H₂N-C(=O)-, C₁₋₄-alkyl-NR^{N}-C(=O)-, C₁₋₄-alkyl-NR^{N}-, C₁₋₄-alkyl-C(=O)NR^{N}-, C₁₋₄-alkyl-S(=O)₂NR^{N}-, -OH, C₁₋₄-alkyl-O-, C₁₋₄-alkyl-O-C₁₋₄-alkyl-O-, C₃₋₆-cycloalkyl-O-, hétérocyclyl-O-, C₁₋₄-alkyl-S(=O)-, hétérocyclyle, et hétéroaryle,
dans lequel tout groupe ou sous-groupe alkyle, cycloalkyle et hétérocyclyle au sein des groupes mentionnés est éventuellement substitué par 1 ou
plusieurs atomes F et éventuellement substitué par 1 ou 2 groupes indépendamment sélectionnés parmi H₃C-, H₃C-0- et -CN,
dans lequel le cycle hétérocyclyle est sélectionné parmi
un groupe cyclobutyle dans lequel 1 groupe CH₂ est remplacé par -NR^{N}- ou -O-,
un groupe C₅₋₆-cycloalkyle saturé ou partiellement insaturé dans lequel 1 groupe CH₂ est remplacé par -C(=O)-, -NR^{N}-, -O-, -S(=O)- ou -S(=O)₂- et/ou 1 groupe CH par N ;
un groupe C₅₋₆-cycloalkyle saturé ou partiellement insaturé dans lequel 1 groupe CH₂ est remplacé par -NR^{N}- ou -O-, un second groupe CH₂ est remplacé par -NR^{N}-, -C-(=O)-, -S(=O)- ou -S(=O)₂- et/ou 1 groupe CH est remplacé par N ; et
un groupe C₅₋₆-cycloalkyle saturé ou partiellement insaturé dans lequel 2 groupes CH₂ sont remplacés par -NR^{N}- ou 1 groupe CH₂ par -NR^{N}- et l'autre par -0- et un troisième groupe CH₂ est remplacé par -C(=O)-, -S(=O)-ou -S(=O)₂- et/ou 1 groupe CH par N ;
et dans lequel le cycle hétéroaryle est sélectionné parmi un cycle pyridin-2-onyle, un cycle hétéroaromatique à 5 chaînons qui contient 1 ou 2 hétéroatomes sélectionnés indépendamment les uns des autres parmi =N-, -NH-, -0- et -S-, et un cycle hétéroaromatique à 6 chaînons qui contient 1 ou 2 atomes =N-, et dans lequel, dans les cycles hétéroaromatiques avec un ou plusieurs groupes NH, chacun d'entre eux est remplacé par NR^{N}, et chaque groupe hétéroaryle est éventuellement substitué par 1 ou 2 substituants indépendamment sélectionnés parmi F, Cl, -CH₃, -CN et -O-CH₃ ;
R⁶ est sélectionné dans le groupe consistant en F, -CH₃, CN et -OCH₃ ;
R^{N} est sélectionné dans le groupe consistant en H, H₃C-, H₃C-C(=O) -, et C₁₋₃-alkyl-S(=O)₂- ; et
m vaut 1 ou 2 ;
ou un sel de celui-ci.

7. Composé selon l'une quelconque des revendications 1 à 6, dans lequel R² est CF₃ et m est 1, ou un sel de celui-ci.

8. Composé selon la revendication 1, dans lequel ledit composé est sélectionné dans le groupe consistant en : 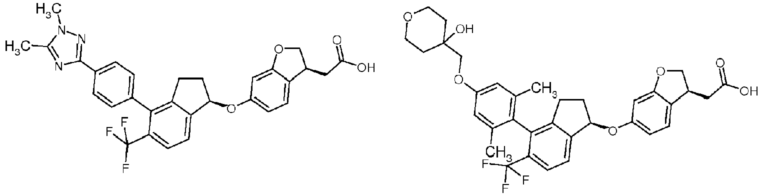 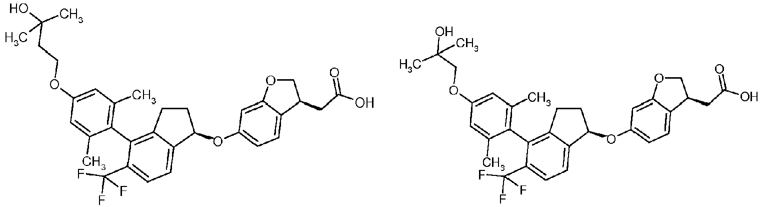 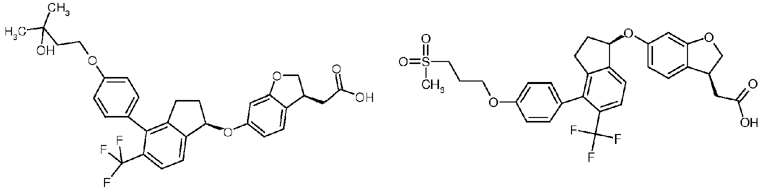 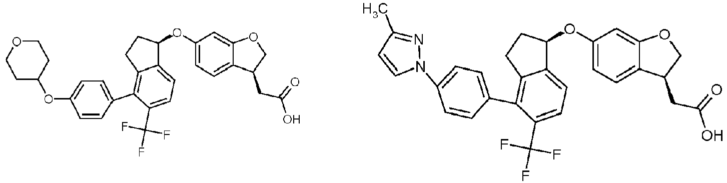 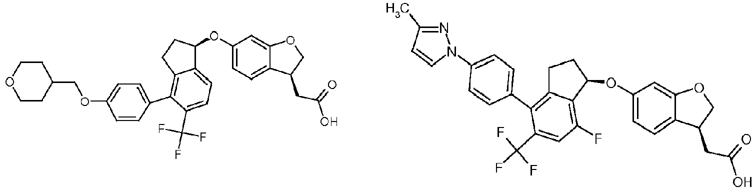 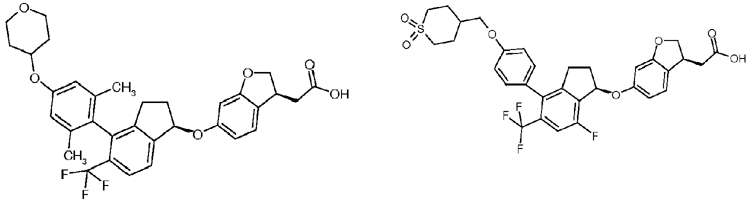 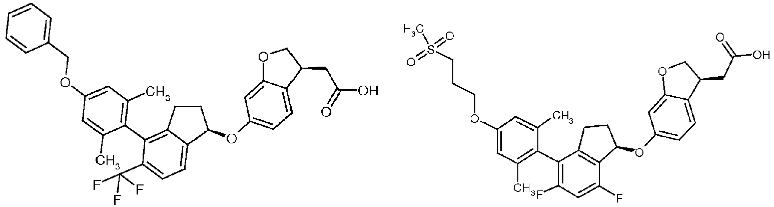 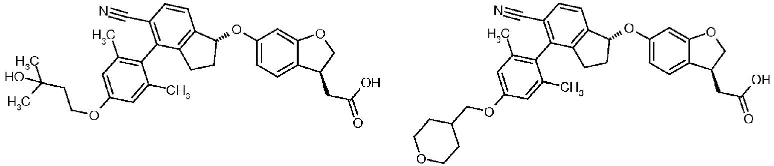 ou un sel de ceux-ci.

9. Sel pharmaceutiquement acceptable d'un composé selon au moins l'une des revendications 1 à 8.

10. Composition pharmaceutique comprenant un ou plusieurs composés selon au moins l'une des revendications 1 à 8 ou un ou plusieurs sels pharmaceutiquement acceptables de ceux-ci, éventuellement conjointement à un ou plusieurs véhicules et/ou diluants inertes.

11. Composé selon au moins l'une des revendications 1 à 8, ou un sel pharmaceutiquement acceptable de celui-ci, pour son utilisation en tant que médicament.

12. Composé selon au moins l'une des revendications 1 à 8 ou un sel pharmaceutiquement acceptable de celui-ci, pour son utilisation dans la prophylaxie et/ou le traitement de maladies métaboliques et d'affections associées à la maladie.

13. Composé pour son utilisation selon la revendication 12, dans lequel les maladies métaboliques ou les affections associées à la maladie sont sélectionnées parmi le diabète, la résistance à l'insuline, l'obésité, une maladie cardiovasculaire et la dyslipidémie.

14. Composé pour son utilisation selon la revendication 12, dans lequel la maladie métabolique est le diabète sucré de type 2.

15. Composition pharmaceutique comprenant un ou plusieurs composés selon au moins l'une des revendications 1 à 8 ou un ou plusieurs sels pharmaceutiquement acceptables de ceux-ci et un ou plusieurs agents thérapeutiques supplémentaires, éventuellement conjointement à un ou plusieurs véhicules et/ou diluants inertes.

16. Composition pharmaceutique selon la revendication 15, dans laquelle les agents thérapeutiques supplémentaires sont sélectionnés dans le groupe consistant en les agents antidiabétiques, les agents destinés au traitement du surpoids et/ou de l'obésité et les agents destinés au traitement de l'hypertension artérielle, de l'insuffisance cardiaque et/ou de l'athérosclérose.
